# EUROPEAN PATENT APPLICATION

(11) **EP 3 434 779 A1**
(43) Date of publication of application: **30.01.2019**
(21) Application number: 18175809.5
(22) Date of filing: 13.09.2012
(51) Int. Cl.: C12N 15/82

(54) **METHODS AND COMPOSITIONS FOR WEED CONTROL**

(30) Priority: 13.09.2011 US 201161534097 P
(62) Divisional of application: 12831166.9
(71) Applicant: Monsanto Technology LLC, Saint Louis, MO 63167 (US)
(72) Inventor: ADER, Daniel, St. Louis, MO 63167 (US); FINNESSY, John, J., St. Louis, MO 63167 (US); LI, Zhaolong, St. Louis, MO 63167 (US); SHAH, Ronak, Hasmukh, St. Louis, MO 63167 (US); TAO, Nengbing, St. Louis, MO 63167 (US); TAYLOR, Christina, Marie, St. Louis, MO 63167 (US); TAYLOR, Jennifer, Chou, St. Louis, MO 63167 (US)
(74) Representative: Uexküll & Stolberg

(57) **Abstract**

Novel compositions for use to enhance weed control. Specifically, methods and compositions that modulate dihydropteroate synthase in weed species. The present invention also provides for combinations of compositions and methods that enhance weed control.

<110>	Ader, Daniel
	Finnessy, John J.
	Li, Zhaolong
	Shah, Ronak Hasmukh
	Tao, Nengbing
	Taylor, Christina Marie
	Taylor, Jennifer Chou

<120>	Methods and Compositions for Weed Control

<130>	40-21(58641)

<150>	61/534097
<151>	2011-09-13

<160>	1222

<210>	1
<211>	672
<212>	DNA
<213>	Amaranthus hybridus

<400>	1

agatttgtga gcgccctgta gcagctgata gggatcctgc taccgttgct tcaataactg	60

ctggagtttt aggtggtgca aacattgtaa gagtacataa tgtgagggat aaccttgatg	120

ctgtcaagtt atgtgatgcc atactcggaa aaactgatta actgcttatt tgtaccacct	180

tgtgaatgac gtctagtgga aagttcgatt gggatattga tgaagtccgt tagtttgctc	240

gaaagtagag ctttagcccg taaggttata gttcttacag atgtagaatc attggctatt	300

tgcctattcc tgcttagaag atcatagcat tgatccagac tttggattcc atgtagaatt	360

atgttgcgac tcatggtcga attggccctt tgaggaattg ttgaagttcc ttgatctatg	420

gttcaatcgg ctcggttcga tgaaggttgg actgtgtcga gacaagatgg gtcaaagaga	480

ccaatggaca agaagtcgac cttgggaaag cttgatcaag ttacttggac gagcagatag	540

cagcaggact ctggcgagca ttagacatac atatcatttg ttttggagtc agatctgcaa	600

tcaatcagtt gcaatggaca aaccatatac ggttgttagt tgttactgtc actgcttgag	660

ttgaattaga tc	672

<210>	2
<211>	458
<212>	DNA
<213>	Amaranthus lividus

<400>	2

aagggaataa ggtatggtcc taggccaatt gacttggaca tactatttta tgggaaattt	60

agggtgagct ctgagagcct cactatcccc catgaaagga tatgggaaag accatttgtg	120

atggcaccat tgattgattg tattgggtct gatgtagaaa atgacactat ttgtacatgg	180

cattcattat caaatttttt gggtggaatc tttgaagcat ggggtaaact cggtggaagt 240

tccctaatcg ggaaggatgg aatgaaaagg gttttgcccg ttggaaatcg cttatgggat 300

tggtctcata aaacctctgt aatgggagtc ttgaacttga ctcctgatag ctttagtgat 360

ggcggaagtt ttcaatctgt cgatactgcg gttgctaagg ttcgtcagat gatctcagat 420

ggggcagata taattgacat cggggcacaa tcaaccag 458

<210> 3
<211> 5590
<212> DNA
<213> Amaranthus palmeri

<400> 3

ggtgtttatt tgggcatggc cttatgtgtg atgttggtaa attttgtggc ttcgtccact 60

aatctgatgt atcggatctg ttaaagggag ccctctcatc ctatgcttat caatgacttt 120

ttcatatgcc tatcaaacag atcagattgg atcgggttcg agttcaggtc atatataaac 180

atgtcagcaa acccttgacc catacccaac ccatttagtt aattggcttg aaaatcacaa 240

ccttaattcg accagcgaca gatcaggttg acctgatttc gacccactta acccatttat 300

aatttttttt tcgatttaag ttttttaaca ttgattaaat ctaatttttt aggctttaat 360

tttaatttta tgtttttttg tttaatttgt atttactatg aaaaataaga aactattata 420

tgaactgagt ttagcttaca cttattaatt taactcgaaa ttaacacatt taattaaatg 480

gttatatagg gttttttagt tttaaaattt caacctgaac ctaatctatt taataaaaag 540

atcagatctg atcaacccat ataattaatt gggtcaaaat ctcaactcaa acccatttat 600

ttcgaattaa attcagatca aattagcagg tcggatcaac tagcgctaat ttttcctctc 660

aagttttttc atatgatgcc ccttgttttc tttgaataaa attcaccaat ttaaaatccc 720

tgcccttgat ccgttggtat tggtatttgg taaagcacat atggtaaatt ctactccatt 780

gttatctgta gttttggttc taaaccccaa acaaatccta ctcctagcac cactaatcct 840

cattactttc tgtttgctgt ctttctctac attcctacta aattaagacc tagatgcagt 900

gttattctgt tcgaggttgc cgaaaacggt acaatagttg cctaatttgc aattcatgtt 960

tgttcttgcg ttttctacat tgtttactct attggaacaa aggttgaact ttcagccttt 1020

cagggagtat tttgattttt ctgtcaaatt accttgccag atttatttgg ctattttgat 1080

tgtgtgtatt tctttgtccc ttgtttccat ggggctaaac tccttttggt taatcttaaa 1140

catttcttct aattttgaat tgcagtggta tgaagtgttg tttaatatac aatcaatttt 1200

tctgaactgg gttaatgaag agaagcattg tagacatgag gaatgtgttc aagcatctca 1260

aaacaaccaa attaatccca aatgggatct tcaactgtaa tagaggtaaa tcacatatat 1320

tctcatttaa tggttattgt tttattttaa ggatgttatt atgttgttat caatgataga 1380

aataagagaa gcttgagcat tcaaatgtat ctatcactca ctggttgtgg acatctaaaa 1440
tatggaccaa gagttttctt catttcttgt accagaattt gcaaatgtat gattaaagtt 1500
taggttggag ttgcaataaa atttcattac cccgacgcta ttaagtggct cacactgagg 1560
cggggtttga gggtcggatg gtgcaacctt caggagaggt tctaagcatt ttcaacatgt 1620
tcaaccgcag agggtcccgt aaaattggag gcttcaatat taaattatgt agtatgtgtt 1680
aagtgtttaa agatataaaa atgttaatga atacataatt tttaaaattg cacaggcttt 1740
tcaaagaaat ttttgatttt gctccgccca tggcaacctt tggcaaatct ggatagaatt 1800
agagaaattt acttttatgt atgtataatc tgatgaatga atgaatgaac aatgaagttc 1860
atttctgagt caatgaccta tatactatcg aggtttcgtt aaataggata tataaataaa 1920
attagagaca tcttttatcc ctaatgttgt ctgttttctt ttctgggcga gactgttttt 1980
ggagaaaaac gttatgttga atgcttgatt tactcagaac tgatgcaaac agatggataa 2040
aaaatggtgt tttcctagtg tttatgtttc ataaaaggat ttcaaattcc aatcgtatga 2100
agtttgaaat actgcttttt gtttttgtag cttcgtgttt tgctttcctt cactcttcac 2160
cagggaacgt tgttgaagtt tgttccaaga agcaagaagt cgtaattgca atcgggagca 2220
atgttggtga cagattagaa aatttcaacc aagctctgca acaaatgaag aaattaggca 2280
tagacatcac aaggcatggt tgtttatatg agacggaacc tgcatacgtg actgatcaac 2340
cgaagtttct taactctgct ttaagaggct ttacaagact tggacctcat gaattattag 2400
gggtattgaa gaaaattgag aaggatatgg gtagaaccaa gggaataagg tatggtccta 2460
ggccaattga cttggacata ctattttatg ggaagtttag ggtgagctct gagagcctca 2520
ctatccccca tgaaaggata tgggaaagac catttgtgat ggcaccattg attgattgta 2580
ttgggtctga tgtagaaaat gacactattt gtacgtggca ttcattatca aatttttcgg 2640
gtggaatctt tgaagcatgg ggtaaactcg gtggaagctc cctaatcggg aaggatggaa 2700
tgaaaagggt tttgcccgtt ggaaatctct tatgggattg gtctcataaa acctctgtta 2760
tgggagtctt gaacttgact cctgatagct ttagtgatgg cggaagtttt caatctgtca 2820
atactgcggt tgctaaggtt cgtcagatga tctcagatgg ggcagatata attgacatcg 2880
gggcacaatc aaccagaccc atggcaacta gaatttcggc tgaagaagag ctagcaagag 2940
tagtacctgt attagaagct gtcaaggatt tgatcgagga agaaggaaga atcttgtcag 3000
tggatacgtt ttattctaaa gttgctttgg aggccgtcaa gaagggggca cacattgtga 3060
atgatgtctc tagtggtaaa ctcgattccg agatgtttaa tgttgttgcg gaccttaaag 3120
ttccttatat agcaatgcac atgcgaggag atccgacttc aatgcaaaac tctgagaact 3180
tgacctacaa tgatgtttgt aagcaagtgg cttcggagtt gagttctagg gtcatagatg 3240

cagaattatc gggaattcct gcttggagga tagttattga tcccggcatc ggattttcta 3300
agaatacgaa tcaaaatttg gaaattctta gtggtttaca aaagatacgg gaagagatag 3360
ctaagaagag tttggcggtg gctcattgcc ccttgctaat tggaccttca agaaagaggt 3420
ttctgggcga gatttgtgag cgccctgtag cagctgacag ggatcctgct accattgctt 3480
ctataactgc tggagtttta ggtggtgcaa acattgtaag agtacataat gttagggata 3540
accttgatgc tgtcaagtta tgtgatgcca tactcggaaa aactgattaa ctgcttgttt 3600
gtaccacctt gtgaatgatg tctagtggaa ggttcgattg ggattatgat gaagtccgtt 3660
agtttgctcg aaagaagagc tttagcccgt aaggttatag ttcttacaga tgtagaatca 3720
ttggctattt gcctatttct gcttagaaga tcatagcatt gatccagact ttggattcca 3780
tgtagaatta tgttgcgact catggtcgaa ttggcccttt gaggaattgt tgaagttcct 3840
tgagtctatg gttcaatcgg ctcggttcga tgaaggttgg actgtgtcga gacaagatgg 3900
gtcaaagaga ccaatggaca agaagtcgac cttgggcaag cttgatcaag ttacttggac 3960
gagcagataa cagcaggctc tggcgagcat tagacataca tatcatttgt tttttgaagt 4020
cagatctgca atcaatcagt tgcaatggac agaccatata cggttgttag ttgttcctgt 4080
cactctttga gttgaattag atcatgcaaa cactgtaaga gtcttgattt tgtgaagcta 4140
tgtgatactg tacttctcaa aatgaaatct ttgatatgat gttagcttca agagttgttt 4200
ttccccccct cacattctgt tctatgttgt attgttgtgt gctttttgga attgtattcc 4260
tgtaattaga attattcctg caaaatggta ttcctttata tcatcaaagg agtttttgta 4320
cttctaatca ttgatcttta gatgcaaaac tgaattaata caagtgttct tgatgaaggc 4380
ttattgaaga actcactttt tatttatgtc taaacttgtt aaagatgttc tgcttccgcc 4440
cttgccacgg gatgtcggga ttgactaagg gtggtaatcg agggattcct atgcttgaat 4500
tgactcgttt aagattttgc gttgatttta agtcaaatgt tttgctaagc ataaactatt 4560
ctagctcgac ttgaataatg aagttttcga gtcaagtccg agctgttctg taagtttatg 4620
tatcttgaag cttcttagtt cttacttctt atccttattt gtttgacaca ttgaaccgcg 4680
ttgatgcttg cttgactcaa cccgttaaag tcttgaattc atcacgaatt cgagtcgagc 4740
aagtttaaac ccaacattaa ctaagctctc gaggcttaat gaggcagggg tagctcaagt 4800
ttgttattca agcaacttac catagtattc ttttaaaagg catatagtca gttagttatt 4860
tgttaatttc agtagaacga aagaagtaca ttattaaact tggccattga tgcttgagct 4920
agagagcttc ggttgtgatc gaaacgaatc ctgatttcgc tcttgatctt tgatgtgaag 4980
tgacaaggcc ttcataaaac tcctttattc ttcagcagtg catacaaaaa tcatgtgtgc 5040
ccagctccta gctcacatac cgcgtttttc ccctaatcta gtgtggtcaa ataaaaacag 5100
caagatgtgt tgatgtcatg tgcaatagta attatcaggt gaagtaatgc aatatgtagc 5160

cagtcacctc atatcaaaaa aatgttggga ggaaaacgta ttcgttcatc actaaacctg 5220

tgtttataca tctaactgat cgatcagggt ttcaaaatcc aaattaatac aatggcattt 5280

gacgaagata tccagaacac atcccccttg tttctgctag ataatcaact attctgattc 5340

tgctgtaata cctcagcttc ggccctagcc atgggatcgt cctctttttc tccttcacgc 5400

tcctcggcca gtttctttct gtgcagttct tcagcagctt ctattgaagc tttgttctcg 5460

gcctgaagac gctgcatttc ctcttccatt tgctgcctct caaaccatgt atcagcatcc 5520

gcccaaactg cagacaagta tacaaaagtt aaataacgaa agtcttcaaa actgcagatc 5580

cagtgaacaa 5590

<210> 4
<211> 5515
<212> DNA
<213> Amaranthus palmeri

<400> 4

cggatctgtt aaagggagcc ctctcatcct atgcttatca atgacttttt catatgccta 60

tcaaacagat cagattggat cgggttcgag ttcaggtcat atataaacat gtcagcaaac 120

ccttgaccca tacccaaccc atttagttaa ttggcttgaa aatcacaacc ttaattcgac 180

cagcgacaga tcaggttgac ctgatttcga cccacttaac ccatttataa tttttttttc 240

gatttaagtt ttttaacatt gattaaatct aattttttag gctttaattt taatttatgt 300

ttttttgttt aatttgtatt tactatgaaa aataagaaac tattatatga actgagttta 360

gcttacactt attgatttaa ctcgaaatta acacatttaa ttaaatggtt atatagggtt 420

ttttagtttt aaaatttcaa cctgaaccta atctatttaa taaaaagatc agatctgatc 480

aacccatata attaattggg tcaaaatctc aactcaaacc catttatttc gaattaaatt 540

cagatcaaat tagcaggtcg gatcaactag cgctaatttt tcctctcaag ttttttcata 600

tgatgcccct tgttttcttt gaataaaatt caccaattta aaatccctgc ccttgatccg 660

ttggtattgg tatttggtaa agcacatatg gtaaattcta ctccattgtt atctgtagtt 720

ttggttctaa accccaaaca aatcctactc ctagcaccac taatcctcat tactttctgt 780

ttgctgtctt tctctacatt cctactaaat taagacctag atgcagtgtt attctgttcg 840

aggttgccga aaacggtaca atagttgcct aatttgcaat tcatgtttgt tcttgcgttt 900

tctacattgt ttactctatt ggaacaaagg ttgaactttc agcctttcag ggagtatttt 960

gatttttctg tcaaattacc ttgccagatt tatttggcta ttttgattgt gtgtatttct 1020

ttgtcccttg tttccatggg gctaaactcc ttttggttaa tcttaaacat ttcttctaat 1080

tttgaattgc agtggtatga agtgttgttt aatatacaat caatttttct gaactgggtt 1140

aatgaagaga agcattgtag acatgaggaa tgtgttcaag catctcaaaa caaccaaatt 1200

aatcccaaat gggatcttca actgtaatag aggtaaatca catatattct catttaatgg 1260

ttattgtttt attttaagga tgttattatg ttgttatcaa tgatagaaat aagagaagct 1320

tgagcattca aatttatcta tcactcactg gttgtggaca tctaaaatat ggaccaagag 1380

ttttcttcat tttttgtacc agaagctgca aatgtatgat taaagtttag gttggagttg 1440

caataaaatt ttattacccc gacgctatta agtggctcac actgaggcgg ggtttgaggg 1500

tcagatggtg caaccttcag aagaggttct aagcattttc aacatgttca accgcatagg 1560

gtcccgtaaa attggaggct tcaatattaa attatgtagt atgtgttatg tgtttaaaga 1620

tataaatatg ttaatgaata cataattttt aaattgcaca gggccttcaa agaaattttc 1680

gattttgctc tgcccctggc aacctttggc aaatttggat agaattagag aagtttactt 1740

ttatgtgtgt ataatatgat gaatgaatga gtgaacaatg aagttcattt ctgagtcaat 1800

gacctatata ctatcgaggt ttcgttaaaa aggatatata aataaaatta gagacatctt 1860

ttatccctaa tgttgtctgt tttcttttct gggcgagact gtttttggag aaaaacgtta 1920

tgttgaatgc ttgatttact cagaactgat gcaaacagat ggataaaaat ggtgttttcc 1980

tattgtttat gtttcgataa caggtttcaa attccactcg tatgaagttt gatatactgc 2040

tatgtgtttt tgtagcttcg tgttttgctt tccttcactc atcaccagtg aatgatgttg 2100

aagtttgttc caagaagcaa gaagtcgtaa ttgcaatcgg gagcaatgtt ggtgacagat 2160

tagaaaattt caaccaagct ctgcaacaaa tgaagaaatt aggcatagac atcacaaggc 2220

atgggtgttt atatgagacg gaacctgcat acgtgactga tcaaccgaag tttcttaact 2280

ctgctttaag aggctttaca aaacttgggc ctcatgaatt attaggggta ttaaagaaaa 2340

ttgagaagga tatgggtaga accaagggaa taaggtatgg tcctaggcca attgacttgg 2400

acatactatt ttatgggaag tttagggtga gctctgagag cctcactatc ccccatgaaa 2460

ggatatggga aagaccattt gtgatggcac cattgattga ttgtattggg tctgatgtag 2520

aaaatgacac tatttgtacg tggcattcat tatcaaattt ttcgggtgga atctttgaag 2580

catggggtaa actcggtgga agttccctaa tcgggaagga tggaatgaaa agggttttgc 2640

ccgttggaaa tcgcttatgg gattggtctc ataaaacctc tgttatggga gtcttgaact 2700

tgactcctga tagctttagt gatggcggaa gttttcaatc tgtcgatact gcggttgcta 2760

aggttcgtca gatgatctca gatggggcag atataattga catcggggca caatcaacca 2820

gacccatggc aactagaatt tcggctgaag aagagctaga aagagtagta cctgtattgg 2880

aagctgtcaa ggatttgatc gaggaagaag gaagaatctt gtcagtggat acgttttatt 2940

ctaaagttgc ttcggaggcc gtcaagaagg gggcacacat tgtgaatgat gtctctagtg 3000

ggaaactcga ttccgagatg tttaatgttg ttgcggacct taaagttcct tatatagcaa 3060

tgcacatgcg aggagatccg acttcaatgc aaaactctga gaacttgacc tacaatgatg 3120
tttgtaagca agtggctttg gagttgagtt ctagggtcat agatgcagaa ttatcgggaa 3180
ttcctgcttg gaggatagtt attgatcccg gcatcggatt ttctaagaat acgaatcaaa 3240
atttggaaat tcttagtggt ttacaaaaga tacgggaaga gatagctaag aagagtttgg 3300
cggtggctca ttgccccttg ctaattggac cttcaagaaa gaggtttctg ggcgagattt 3360
gtgagcgccc tgtagcagct gacagggatc ctgctaccat tgcttctata actgctggag 3420
ttttaggtgg tgcaaacatt gtaagagtac ataatgtgag ggataacctt gatgctgtca 3480
agttatgtga tgccatactc ggaaaaactg attaactgct tgtttgtacc accttgtgaa 3540
tgatgtctag tggaaggttc gattgggatt atgatgaagt ccgttagttt gctcgaaaga 3600
agagctttag cccgtaaggt tatagttctt acagatgtag aatcattggc tatttgccta 3660
tttctgctta gaagatcata gcattgatcc agactttgga ttccatgtag aattatgttg 3720
cgactcatgg tcgaattggc cctttgagga attgttgaag ttccttgagt ctatggttca 3780
atcggctcgg ttcgatgaag gttggactgt gtcgagacaa gatgggtcaa agagaccaat 3840
ggacaagaag tcgaccttgg gcaagcttga tcaagttact tggacaagca gataacagca 3900
ggctctggcg agcattagac atatcatttg ttttttggag tcagatctgc aatcaatcag 3960
ttgcaatgga caaaccatat acggttgtta gttgttactg tcactgtttg agttgaatta 4020
gatcatgcaa acactgtagg agtcttgatt atgtgaagct atgtgacact gtacttctca 4080
aaatgaaatc tttgatatga tgttagtttc aagagttgta attcccccct cacatttctg 4140
ttctatgttg tattgttgtg tgctttttgg aattgtattc ctgcaataga attattcccg 4200
caaaatggta ttcctttata tcatcaaagg agtttttgta cttctaatca ttgatcttta 4260
gatgcaaaac tgaattaata caagtattct tgatgaaggc ttattgaaga actcactttt 4320
tatttatgtc taaacttgtt aaagatgttc tgcttccgcc cttgccacgg gatgtcggga 4380
ttgactaggg gtggtaatcg agggattcct atgcttgaat tgactcgttt aagattttgc 4440
gttgatttta agtcaaacgt tttgctaagc ataaactatt ctagctcgac ttgaataatg 4500
aagttttcga gtcaagtccg agctgttcta taagtttacg tatcttgaag cttcttagtt 4560
cttacttctt atcctttatt ttttgacaca ttgaaccgca ttgatgcttg cttgactcaa 4620
ctcgttaaag tcttgagttc atcacgaatt cgagtcgagc aagtttaaac ccaacattaa 4680
ctaagctctc gaggcttaat gaggcagggg tagctcaagt ttgttattca agcaacttac 4740
catagtattc ttttaaaagg catatagtca gttagttatt tgttaatttc agtagaacga 4800
aagaagtaca ttattaaact tggccattga tgcttgagct agagagtttc ggttgtgatc 4860
gaaacgaatc ctgatttcgc tcttgatctt tgatgtgaag tgacaaggcc ttcataaaac 4920

tcctttattc ttcagcagtg catacaaaaa tcatgtgtgc ccagctccta gctcacatac 4980

cgcgtttttc ccctaatcta gtgtggttaa ataaaaacag caagatgtgt tgatgtcatg 5040

tgcaatagta attatcaggt gaagtaattc aatatgtagc cagtcacctc atatcaaaaa 5100

aatgttggga ggaaaacgta ttcgttcatc actaaacctg tttttataca tctaactgat 5160

cgatcagggt ttcaaaatcc aaattaatac aatggcattt gacgaagata tccagaacac 5220

atcccccttg tttctgctag ataatcaact attctgattc tgctgtaata cctcagcttc 5280

ggccctagcc atgggatcgt cctctttttc tccttcacgc tcctcggcca gtttctttct 5340

gtgcagttct tcagcagctt ctattgaagc tttgttctcg gcctgaagac gctgcatttc 5400

ctcttccatt tgctgcctct caaaccatgt atcagcatcc gcccaaactg cagacaagta 5460

tacaaaagtt aaataacgaa agtcttcaaa actgcagatc cagtgaacaa agtca 5515

<210> 5
<211> 1431
<212> DNA
<213> Amaranthus palmeri

<400> 5

gctttccttc actcatcacc agtgaatgat gttgaagttt gttccaagaa gcaagaagtc 60

gtaattgcaa tcgggagcaa tgttggtgac agattagaaa atttcaacca agctctgcaa 120

caaatgaaga aattaggcat agacatcaca aggcatgggt gtttatatga gacggaacct 180

gcatacgtga ctgatcaacc gaagtttctt aactctgctt taagaggctt tacaaaactt 240

gggcctcatg aattattagg ggtattaaag aaaattgaga aggatatggg tagaaccaag 300

ggaataaggt atggtcctag gccaattgac ttggacatac tattttatgg gaagtttagg 360

gtgagctctg agagcctcac tatcccccat gaaaggatat gggaaagacc atttgtgatg 420

gcaccattga ttgattgtat tgggtctgat gtagaaaatg acactatttg tacgtggcat 480

tcattatcaa atttttcggg tggaatcttt gaagcatggg gtaaactcgg tggaagttcc 540

ctaatcggga aggatggaat gaaaagggtt ttgcccgttg gaaatcgctt atgggattgg 600

tctcataaaa cctctgttat gggagtcttg aacttgactc ctgatagctt tagtgatggc 660

ggaagttttc aatctgtcga tactgcggtt gctaaggttc gtcagatgat ctcagatggg 720

gcagatataa ttgacatcgg ggcacaatca accagaccca tggcaactag aatttcggct 780

gaagaagagc tagaaagagt agtacctgta ttggaagctg tcaaggattt gatcgaggaa 840

gaaggaagaa tcttgtcagt ggatacgttt tattctaaag ttgcttcgga ggccgtcaag 900

aagggggcac acattgtgaa tgatgtctct agtgggaaac tcgattccga gatgtttaat 960

gttgttgcgg accttaaagt tccttatata gcaatgcaca tgcgaggaga tccgacttca 1020

atgcaaaact ctgagaactt gacctacaat gatgtttgta agcaagtggc tttggagttg 1080

agttctaggg tcatagatgc agaattatcg ggaattcctg cttggaggat agttattgat 1140

cccggcatcg gattttctaa gaatacgaat caaaatttgg aaattcttag tggtttacaa 1200

aagatacggg aagagatagc taagaagagt ttggcggtgg ctcattgccc cttgctaatt 1260

ggaccttcaa gaaagaggtt tctgggcgag atttgtgagc gccctgtagc agctgacagg 1320

gatcctgcta ccattgcttc tataactgct ggagttttag gtggtgcaaa cattgtaaga 1380

gtacataatg tgagggataa ccttgatgct gtcaagttat gtgatgccat a 1431

<210> 6
<211> 1431
<212> DNA
<213> Amaranthus palmeri

<400> 6

gctttccttc actcttcacc agggaacgtt gttgaagttt gttccaagaa gcaagaagtc 60

gtaattgcaa tcgggagcaa tgttggtgac agattagaaa atttcaacca agctctgcaa 120

caaatgaaga aattaggcat agacatcaca aggcatggtt gtttatatga gacggaacct 180

gcatacgtga ctgatcaacc gaagtttctt aactctgctt taagaggctt tacaagactt 240

ggacctcatg aattattagg ggtattgaag aaaattgaga aggatatggg tagaaccaag 300

ggaataaggt atggtcctag gccaattgac ttggacatac tattttatgg gaagtttagg 360

gtgagctctg agagcctcac tatcccccat gaaaggatat gggaaagacc atttgtgatg 420

gcaccattga ttgattgtat tgggtctgat gtagaaaatg acactatttg tacgtggcat 480

tcattatcaa atttttcggg tggaatcttt gaagcatggg gtaaactcgg tggaagctcc 540

ctaatcggga aggatggaat gaaaagggtt ttgcccgttg gaaatctctt atgggattgg 600

tctcataaaa cctctgttat gggagtcttg aacttgactc ctgatagctt tagtgatggc 660

ggaagttttc aatctgtcaa tactgcggtt gctaaggttc gtcagatgat ctcagatggg 720

gcagatataa ttgacatcgg ggcacaatca accagaccca tggcaactag aatttcggct 780

gaagaagagc tagcaagagt agtacctgta ttagaagctg tcaaggattt gatcgaggaa 840

gaaggaagaa tcttgtcagt ggatacgttt tattctaaag ttgctttgga ggccgtcaag 900

aagggggcac acattgtgaa tgatgtctct agtggtaaac tcgattccga gatgtttaat 960

gttgttgcgg accttaaagt tccttatata gcaatgcaca tgcgaggaga tccgacttca 1020

atgcaaaact ctgagaactt gacctacaat gatgtttgta agcaagtggc ttcggagttg 1080

agttctaggg tcatagatgc agaattatcg ggaattcctg cttggaggat agttattgat 1140

cccggcatcg gattttctaa gaatacgaat caaaatttgg aaattcttag tggtttacaa 1200

aagatacggg aagagatagc taagaagagt ttggcggtgg ctcattgccc cttgctaatt 1260

ggaccttcaa gaaagaggtt tctgggcgag atttgtgagc gccctgtagc agctgacagg 1320

gatcctgcta ccattgcttc tataactgct ggagttttag gtggtgcaaa cattgtaaga 1380

gtacataatg ttagggataa ccttgatgct gtcaagttat gtgatgccat a 1431

<210> 7
<211> 1163
<212> DNA
<213> Amaranthus palmeri

<400> 7

gaagctgtca aggatttgat cgaggaagaa ggaagaatct tgtcagtgga tacgttttat 60

tctaaagttg cttcggaggc cgtcaagaag ggggcacaca ttgtgaatga tgtctctagt 120

gggaaactcg attccgagat gtttaatgtt gttgcggacc ttaaagttcc ttatatagca 180

atgcacatgc gaggagatcc gacttcaatg caaaactctg agaacttgac ctacaatgat 240

gtttgtaagc aagtggcttt ggagttgagt tctagggtca tagatgcaga attatcggga 300

attcctgctt ggaggatagt tattgatccc ggcatcggat tttctaagaa tacgaatcaa 360

aatttggaaa ttcttagtgg tttacaaaag atacgggaag agatagctaa gaagagtttg 420

gcggtggctc attgcccctt gctaattgga ccttcaagaa agaggtttct gggcgagatt 480

tgtgagcgcc ctgtagcagc tgacagggat cctgctacca ttgcttctat aactgctgga 540

gttttaggtg gtgcaaacat tgtaagagta cataatgtta gggataacct tgatgctgtc 600

aagttatgtg atgccatact cggaaaaact gattaactgc ttgtttgtac caccttgtga 660

atgatgtcta gtggaaggtt cgattgggat tatgatgaag tccgttagtt tgctcgaaag 720

tagagcttta gcccgtaagg ctatagttct tacagatgta gaatcattgg ctatttgcct 780

atttctgctt agaagatcat agcattgatc cagactttgg attccatgta gaattatgtt 840

gcgactcatg gtcgaattgg ccctttgagg aattgttgaa gttccttgag tctatggttc 900

aatcggctcg gttcgatgaa ggttggactg tgtcgagaca agatgggtca aagagaccaa 960

tggacaagaa gtcgaccttg ggcaagcttg atcaagttac ttggacaagc agataacagc 1020

aggctctggc gagcattaga catatcattt gttttttgga gtcagatctg caatcaatca 1080

gttgcaatgg acaaaccata tacggttgtt agttgttact gtcactgttt gagttgaatt 1140

agatcatgca aacactgtag gag 1163

<210> 8
<211> 1431
<212> DNA
<213> Amaranthus rudis

<400> 8

gcgtaccttc actcatcacc agtggacgat gttgaacttt gttctaagaa gcaagaagtc 60

ataattgcaa tcgggagcaa tgttggtgac agattagaaa attttaacca agctctgcaa 120

caaatgaaga aattaggcat agacatcaca aggcatggtt gtttatatga gacagaaccc 180

gcatacgtga ctgatcaacc gaagtttctt aactctgctt taagaggctt tacaagactt 240

gggcctcatg aattattagg ggtattgaag aaaattgaga agaatatggg tagaaccaag 300

ggaataaggt atggtcctag gccaattgac ttggacatac tattttatgg gaagtttagg 360

gtgagctctg agagcctcac tattccccat gaaaggatat gggaaagacc atttgtgatg 420

gcaccattaa ttgattgtat tgggtctggt gtagaaaatg acactatttg tacatggcat 480

tcgttatcaa atttttcggg tggaatcttt gaagcatggg ataaactcgg tggaagttcc 540

ctaatcggga aggatggaat gaaaagggtt ttgcccgttg ggaaccgctt atgggattgg 600

tctcataaaa cctctgtaat gggagtcttg aacttgactc ctgatagctt tagtgatgga 660

ggaagttttc aatctgtcga tactgcggtt gctaaggttc gtcagatgat atcagatggg 720

gccgacataa ttgacattgg ggcacagtca acaagaccca tggcaactag gatttcggct 780

gaagaagagc tagaaagagt tgtacctgta ttggaagctg ttaaggatgt gatcgaggaa 840

gaaggaagaa tcttatcggt ggatactttt tattcaaaag ttgcttctga ggccgtcaag 900

aagggggcac acattgtgaa tgatgtctct agtgggaaat tcgatcctga gatgttcaat 960

gttgttgcgg accttaaagt tccttatata gcaatgcaca tgcgaggaga tccgacttca 1020

atgcaaaatt ctgagaactt gacgtacagt gatgtttgta agcaagtggc ttcggagttg 1080

agctctaggg tcatagatgc agaattatcg ggaattcctg cttggagaat agttattgat 1140

cccggcattg gattttctaa gaatacgaag caaaatttgg aaattcttac tggtttacaa 1200

aagatacggc tagagatagc taagaagagt ttggcggtgg ctcattgccc cttgctaatt 1260

ggaccttcac gaaagaggtt tctgggtgag atttgtaatc gccctgtagc agctgatagg 1320

gatcctgcta ccgtcgcttc tatcactgct ggagttttag gtggtgcaaa cattgtaaga 1380

gtacataatg tgagggataa ccttgatgca gtcaagttat gtgatgccat a 1431

<210> 9
<211> 1059
<212> DNA
<213> Amaranthus rudis

<400> 9

tgtttcgata aaaggatttc aaatcccaat cgtatgaagt ttgaaataca actatgtgtt 60

tttgcagctt tgttttttgc gtaccttcac tcatcaccag tggacgatgt tgaactttgt 120

tctaagaagc aagaagtcat aattgcaatc gggagcaatg ttggtgacag attagaaaat 180

tttaaccaag ctctgcaaca aatgaagaaa ttaggcatag acatcacaag gcatggttgt 240

ttatatgaga cagaacccgc atacgtgact gatcaaccga agtttcttaa ctctgcttta 300

agaggcttta caagacttgg gcctcatgaa ttattagggg tattgaagaa aattgagaag 360

aatatgggta gaaccaaggg aataaggtat ggtcctaggc caattgactt ggacatacta 420

ttttatggga agtttagggt gagctctgag agcctcacta ttccccatga aaggatatgg 480

gaaagaccat ttgtgatggc accattaatt gattgtattg ggtctggtgt agaaaatgac 540

actatttgta catggcattc gttatcaaat ttttcgggtg gaatctttga agcatgggat 600

aaactcggtg gaagttccct aatcgggaag gatggaatga aaagggtttt gcccgttggg 660

aaccgcttat gggattggtc tcataaaacc tctgtaatgg gagtcttgaa cttgactcct 720

gatagcttta gtgatggagg aagttttcaa tctgtcgata ctgcggttgc taaggttcgt 780

cagatgatat cagatggggc cgacataatt gacattgggg cacagtcaac aagacccatg 840

gcaactagga tttcggctga agaagagcta gaaagagttg tacctgtatt ggaagctgtt 900

aaggatgtga tcgaggaaga aggaagaatc ttatcggtgg atacttttta ttcaaaagtt 960

gcttctgagg ccgtcaagaa gggggcacac attgtgaatg atgtctctag tgggaaattc 1020

gatcctgaga tgttcaatgt tgttgcggac cttaaagtt 1059

<210> 10
<211> 792
<212> DNA
<213> Amaranthus rudis

<400> 10

aatgggagtc ttgaacttga ctcctgatag ctttagtgat ggcggaaggt ttcaatctgt 60

cgatactgcg gttgctaagg ttcgtcagat gatctctgac ggggcggaca taattgacat 120

tggggcgcag tcaacaagac ccatggcaac taggatttcg gctgaagaag agctagaaag 180

agtcgtacct gtattagaag ctgtcaagga tttgatcgag gaagaaggaa gaatcttgtc 240

agtggatacg ttttattcta aagttgcttc ggaggccgtc aagaaggggg cacacattgt 300

gaatgatgtc tctagtggga aactcgatcc cgagatgttt aatgttgttg cggaccttaa 360

agttccttat atagcaatgc acatgcgagg agatccgact tcaatgcaaa attctgagaa 420

cttgacgtac aatgatgtgt gtaagcaagt ggcttcggag ttgagctcta gggtcgtaga 480

tgcagaatta tcgggaattc ctgcttggag gatggttatt gatcccggca ttggattttc 540

taagaatacg aagcaaaatt tggaaattct tagtggttta caaaagatac ggcaagagat 600

agctaagaag agtttggcgg tggctcattg ccccttgcta attggacctt caagaaagag 660

gtttctgggc gagatttgca atcgccctgt agcagctgat agggatcctg ctaccgttgc 720

ttctatcact gctggagttt taggtggtgc aaacattgta agagtacata atgtgaggga 780

taaccttgat gc 792

<210>	11
<211>	764
<212>	DNA
<213>	Amaranthus rudis

<400>	11

tggcaactag gatttcggct gaagaagagc tagaaagagt cgtacctgta ttagaagctg	60

tcaaggattt gatcgaggaa gaaggaagaa tcttgtcagt ggatacgttt tattctaaag	120

ttgcttcgga ggccgtcaag aagggggcac acattgtgaa tgatgtttct agtgggaaac	180

tcgatcccga gatgtttaat gttgttgcgg accttaaagt tccttatata gcaatgcaca	240

tgcgaggaga tccgacttca atgcaaaatt ctgagaactt gacgtacaat gatgtgtgta	300

agcaagtggc ttcggagttg agctctaggg tcgtagatgc agaattatcg ggaattcctg	360

cttggaggat ggttattgat cccggcattg gattttctaa gaatacgaag caaaatttgg	420

aaattcttag tggtttacaa aagatacggc aagagatagc taagaagagt ttggcggtgg	480

ctcattgccc cttgctaatt ggaccttcaa gaaagaggtt tctgggcgag atttgcaatc	540

gccctgtagc agctgatagg gatcctgcta ccgttgcttc tatcactgct ggagttttag	600

gtggtgcaaa cattgtaaga gtacataatg tgagggataa ccttgatgca gtcaagttat	660

gtgatgccat actcagaaaa acggattagc cgattttttt ggattccaga gccgatgttc	720

cagattttcc tttgctttca tgtgccgaag attcttagtt tagg	764

<210>	12
<211>	755
<212>	DNA
<213>	Amaranthus rudis

<400>	12

gtaatgggag tcttgaactt gactcctgat agctttagtg atggcggaag gtttcaatct	60

gtcgatactg cggttgctaa ggttcgtcag atgatctctg acggggcgga cataattgac	120

attggggcgc agtcaacaag acccttggca actaggattt cggctgaaga agagctagaa	180

agagtcgtac ctgtattaga agctgtcaag gatttgatcg aggaagaagg aagaatcttg	240

tcagtggata cgttttattc taaagttgct tcggaggccg tcaagaaggg ggcacacatt	300

gtgaatgatg tttctagtgg gaaactcgat cccgagatgt ttaatgttgt tgcggacctt	360

aaagttcctt atatagcaat gcacatgcga ggagatccga cttcaatgca aaattctgag	420

aacttgacgt acagtgatgt ttgtaagcaa gtggcttcgg agttgagctc tagggtcata	480

gatgcagaat tatcgggaat tcctgcttgg agaatagtta ttgatcccgg cattggattt	540

tctaagaata cgaagcaaaa tttggaaatt cttactggtt tacaaaagat acggctagag	600

atagctaaga agagtttggc ggtggctcat tgccccttgc taattggacc ttcacgaaag 660

aggtttctgg gtgagatttg taatcgccct gtagcagctg atagggatcc tgctaccgtc 720

gcttctatca ctgctggagt tttaggtggt gcaaa 755

<210> 13
<211> 602
<212> DNA
<213> Amaranthus rudis

<400> 13

aggatttcgg ctgaagaaga gctagaaaga gttgtacctg tattggaagc tgttaaggat 60

gtgatcgagg aagaaggaag aatcttatcg gtggatacgt tttattctaa agttgcttcg 120

gaggccgtca agaagggggc acacattgtg aatgatgttt ctagtgggaa actcgatccc 180

gagatgttta atgttgttgc ggaccttaaa gttccttata tagcaatgca catgcgagga 240

gatccgactt caatgcaaaa ttctgagaac ttgacgtaca atgatgtgtg taagcaagtg 300

gcttcggagt tgagctctag ggtcgtagat gcagaattat cgggaattcc tgcttggagg 360

atggttattg atcccggcat tggattttct aagaatacga agcaaaattt ggaaattctt 420

agtggtttac aaaagatacg gcaagagata gctaagaaga gtttggcggt ggctcattgc 480

cccttgctaa ttggaccttc aagaaagaag gtttctgggc gagatttgca atcgccctgt 540

agcagctgat agggatcctg ctaccgttgc ttctatcact gctggagttt taggtggtgc 600

aa 602

<210> 14
<211> 584
<212> DNA
<213> Amaranthus rudis

<400> 14

attagaggca tcttttatcc ctaatgctgt ccgttttctt ttctgggtga gactattttg 60

gaagaaaaaa atttatgttc aatgtttaat ttactcagaa ttgatgcaaa cagatggata 120

aaaatggtgt tttcttatgg tttatgtttc aataaaagga tttcaaattc taatcatatg 180

aagtttgaaa tactatgtgt ttttgtagct tcttgtattg ctttccttca ctcatcacca 240

gtgaacgatg ttgaagtttg ttccaagaag caagaagtcg taattgcaat cgggagcaat 300

gttggtgaca gattagaaaa tttcaacgaa gctctgcaac aaatgaagaa attaggcata 360

gacatcacaa ggcatggttg tttatacgag acggaacctg cttacgtgac tgatcagccg 420

aagtttctta actctgcttt aagaggcttt acaagacttg ggcctcatga attattaggg 480

gtattgaaga gaatcgagaa ggatatgggc agaaccaagg gaataaggta tggtcctagg 540

ccaattgact tggacatact attttatggg aagtttaggg tgag 584

<210>	15
<211>	570
<212>	DNA
<213>	Amaranthus rudis

<400>	15

tgatttactc agaattgatg caaacaaatg gataaaaatg gtgttttcct atggtttatg	60

tttcgataaa aggatttcaa atcccaatcg tatgaagttt gaaatacaac tatgtgtttt	120

tgtagcttct tgtattgctt tccttcactc atcaccagtg aacgatgttg aagtttgttc	180

caagaagcaa gaagtcgtaa ttgcaatcgg gagcaatgtt ggtgacagat tagaaaattt	240

caacgaagct ctgcaacaaa tgaagaaatt aggcatagac atcacaaggc atggttgttt	300

atacgagacg gaacctgctt acgtgactga tcagccgaag tttcttaact ctgctttaag	360

aggctttaca agacttgggc ctcatgaatt attaggggta ttgaagagaa tcgagaagga	420

tatgggcaga accaagggaa taaggtatgg tcctaggcca attgacttgg acatactatt	480

ttatgggaag tttagggtga gctctgagag cctcactatt ccccatgaaa ggatatggga	540

aagaccattt gtgatggcac cattaattga	570

<210>	16
<211>	411
<212>	DNA
<213>	Amaranthus rudis

<400>	16

ctgagagcct cactattccc catgaaagga tatgggaaag accatttgtg atggcaccat	60

taattgattg tattgggtct gatgtagaaa atgacactat ttgtacatgg cattcgttat	120

caaatttttc gggtggaata tttgaagcat gggataaact cggaggaagt tccttaatcg	180

ggaaggatgg aatgaaaagg gttttgcccg ttgggaaccg tttatgggat tggtctcata	240

aaacctcagt aatgggagtc ttgaacttga ctcctgatag ctttagtgat ggcggaaggt	300

ttcaatctgt cgatactgcg gttgctaagg ttcgtcagat gatctctgac ggggcggaca	360

taattgacat tggggcgcag tcaacaagac ccatggcaac taggatttcg g	411

<210>	17
<211>	260
<212>	DNA
<213>	Amaranthus rudis

<400>	17

cgctactgct gcagctgtca ccgctgcaat tttgaatggt gctaacatag taaggtaca	60

taatgtagga tatagttcag atgctgcaaa gttttgtgat gcacctatac tcattttacc	120

catctcgctc ctaatggacc ccagcccctt aataacgtca atgttgtgcc cacatttctt 180

ggagaaccca atgccaggat caagaactat cctccacaat ggaatcccag atgggtctgc 240

ttctcacctt tgcatatagc 260

<210> 18
<211> 1504
<212> DNA
<213> Amaranthus viridis

<400> 18

tctgtataac ctaaagattg aaacttttga agatattttt gaagaagtac tctttggctt 60

tttgatccag aaaatcatat tggtatgaag tgttgtttaa tatacaatca atttttctga 120

actgggttat taaagagaag cattgtagac atgaggaatg tgttcaagca tctcaaaaca 180

accaaattaa tcccaaatgg gatcttcaac tgtaatagag cttcgtgttt tgctttcctt 240

cactcatcac cggtgaacga tgttgaagtt tgttccaaga agcaagaagt cgtaattgca 300

atcgggagca atgttggtga cagattagaa aatttcaacc aagctctgca acaaatgaag 360

aaattaggca tagacatcac aaggcatggg tgtttatatg agacggaacc tgcatacgtg 420

actgatcaac cgaagtttct taactctgct ttaagaggct ttacaaaact tgggcctcat 480

gaattattag gggttttaaa gaaaattgag aaggatatgg gtagaaccaa gggaataagg 540

tatggtccta ggccaattga cttggacata ctattttatg ggaaatttag ggtgagctct 600

gagagcctca ctatccccca tgaaaggata tgggaaagac catttgtgat ggcaccattg 660

attgattgta ttgggtctga tgtagaaaat gacactattt gtacatggca ttcattatca 720

aattttttgg gtggaatctt tgaagcatgg ggtaaactcg gtggaagttc cctaatcggg 780

aaggatggaa tgaaaagggt tttgcccgtt ggaaatcgct tatgggattg gtctcataaa 840

acctctgtaa tgggagtctt gaacttgact cctgatagct ttagtgatgg cggaagtttt 900

caatctgtcg atactgcggt tgctaaggtt cgtcagatga tctcagatgg ggcagatata 960

attgacatcg gggcacaatc aaccagaccc atggcaacta ggatttcggc tgaagaagag 1020

ctagaaagag tagtacctgt attggaagct gtcaaggatt tgatcgagga agaaggaaga 1080

atcttgtcag tggatacgtt ttattctaaa gttgcttcgg aggccgtcaa gaagggggca 1140

cacattgtga atgatgtctc tagtgggaaa ctcgattccg agatgtttaa tgttgttgcg 1200

gaccttaaag ttccttatat agcaatgcac atgcgaggag atccgacttc aatgcaaaat 1260

tctgagaact tgacgtacaa tgatgtttgt aagcaagtgg ctacggagtt gagctctagg 1320

gtcatagatg cagaattatc gggaattcct gcttggagga tagttattga tcccggcatt 1380

ggattttcta agaatacgaa tcaaaatttg gaaattcttc gtggtttaca aaagatacgg 1440

gaagagatag ctaagaagag tttggcagtg gctcattgcc ccttgctaat tggaccttca 1500

agaa                                                              1504

<210>    19
<211>    655
<212>    DNA
<213>    Amaranthus viridis

<400>    19

gcagctgata gggatcctgc taccgttgct tctataactg ctggagtttt aggtggtgca    60

aacattgtaa gagtacataa tgtgagggat aaccttgatg ctgtcaagtt atgtgatgcc   120

atactcggaa aaactgatta actgcttatt tgtaccacct tgtgaatgac gtctagtgga   180

aagttcgatt gggatatgat gaagtccgtt agtttgctcg aaagtagagc tttagcccgt   240

aaggttatag ttcttacaga tgtagaatca ttggctattt gcctattttt gcttagaaga   300

tcatagcatt gatccagact ttggattcca tctagaatta tgttgcgact catggtcgaa   360

ttggcccttt gaggaattgt tgaagttcct tgatctatgg ttcaatcggc ttggttcgat   420

gaaggttgga ctgtgtcgag acaagatggg tcaaagagac caatggacaa gaagtcgacc   480

ttgggaaagc ttgatcaagt tacttggacg agcagatagc agcaggactc tggcgagcat   540

tagacataca tatcatttgt ttttggagtc agatctgcaa tcaatcagtt gcaatggaca   600

aaccatatac ggttgttagt tgttactgtc actgcttgag ttgaattaga tcatg        655

<210>    20
<211>    1413
<212>    DNA
<213>    Ambrosia trifida

<400>    20

tcgttctttc atttctcttc ggatgcttcg atacaagtcc attctcaaga acaagaagta    60

gtaattgctt taggtagcaa tgtgggtgat aggcttaata actttaatga agccttaagt   120

caaatgaaaa aatcaggcat agaaataaca agacatgcat gtttatacga aaccgagcca   180

gcttacgtga ctgatcaacc tcttttcctc aattctgcca tcagaggcgt tacaaagctg   240

ggcccacatg agctactatc ggccctcaag aaaattgaaa aagaaatggg ccgaaccaaa   300

ggtattagat acggcccgcg acccattgac ttagatatac tgttttatgg taaacacaga   360

gttaactcgg aaattctcac tgttccacat gaaagaatct tcgagaggcc atttgttatg   420

gctccgttag ttgacttatt ggggtcggaa attgacaatg atacggttct atgctggcat   480

tctttctcaa aaagaggact tttgaatctt tgggaaaaat taggtggcga atctttgata   540

ggcaaagatg ggttaagaag agttttacca gtcaataatc ggttatggga ttggtcaaag   600

aaaacctctg tcatgggaat tttgaacttg actcccgata gttttagcga cggaggaaag   660

tttgacactg tggggtccgc tatatctcgt gttagggcca tgatatctga aggggcagac 720

ataattgatc tgggagctca atcgacacgt ccaatggcaa ccaagatttc agtccaagaa 780

gaactagata ggttaatccc tgttctcgaa aagattcttg aattacccga aattgaagga 840

aaactgctct ctatcgacac gttttactca gaagttgctt tagaagcgat taagaaaggg 900

gctcatataa tcaatgatgt atcgggtgga aatctagatt ctgatatgtt tcgggtagtt 960

gctaatcttg atgttccata tattgctatg cacatgagag gggacccatc cacaatgcaa 1020

aacagtgaga atttgaagta tgatgatgtt tgtaaagaag ttggtgatga gttgtatgag 1080

cgtgtaagag ctgcagagtt atgcggtgtg cctgcatgga ggatcattct tgacccaggg 1140

atcgggtttt caaagaaaac cgaagataat ttggatattt tgatgggatt aaagagaatt 1200

aggagtgaga ttgcacggaa gagcttaggg gtgtcacgtg cacctttgtt aatcggtcct 1260

tcaagaaaga gatttttggg ggagatttgt ggtcgggctt ctgctgtcga gagagatcca 1320

gcgactgttg ctgctgttac atgtgcggtt ttgggtggtg ctaatgttgt tcgcgttcat 1380

aacgttggag ataatgcgga tgctgtaaag ctt 1413

<210> 21
<211> 1328
<212> DNA
<213> Ambrosia trifida

<400> 21

aatgaagcct taagtcaaat gaaaaaatca ggcatagaaa taacaagaca tgcatgttta 60

tacgaaaccg agccagctta cgtgactgat caacctcttt tcctcaattc tgccatcaga 120

ggcgttacaa agctgggccc acatgagcta ctatcggccc tcaagaaaat tgaaaaagaa 180

atgggccgaa ccaaaggtat tagatacggc ccgcgaccca ttgacttaga tatactgttt 240

tatggtaaac acagagttaa ctcggaaatt ctcactgttc cacatgaaag aatcttcgag 300

aggccatttg ttatggctcc gttagttgac ttattggggt cggaaattga caatgatacg 360

gttctatgct ggcattcttt ctcaaaaaga ggactttttg aatcttggga aaaattaggt 420

ggcgaatctt tgataggcaa agatgggtta agaagagttt taccagtcaa taatcggtta 480

tgggattggt caaagaaaac ctctgtcatg ggaattttga acttgactcc cgatagtttt 540

agcgacggag gaaagtttga cactgtgggg tccgctatat ctcgtgttag ggccatgata 600

tctgaagggg cagacataat tgatctggga gctcaatcga cacgtccaat ggcaaccaag 660

atttcagtcc aagaagaact agataggtta atccctgttc tcgaaaagat tcttgaatta 720

cccgaaattg aaggaaaact gctctctatc gacacgtttt actcagaagt tgctttagaa 780

gcgattaaga aaggggctca tataatcaat gatgtatcgg gtggaaatct agattctgat 840

atgtttcggg tagttgctaa tcttgatgtt ccatatattg ctatgcacat gagaggggac 900

ccatccacaa tgcaaaacag tgagaatttg aagtatgatg atgtttgtaa agaagttggt 960

gatgagttgt atgagcgtgt aagagctgca gagttatgcg gtgtgcctgc atggaggatc 1020

attcttgacc cagggatcgg gttttcaaag aaaaccgaag ataatttgga tattttgatg 1080

ggattaaaga gaattaggag tgagattgca cggaagagct taggggtgtc acgtgcacct 1140

ttgttaatcg gtccttcaag aaagagattt ttgggggaga tttgtggtcg ggcttctgct 1200

gtcgagagag atccagcgac tgttgctgct gttacatgtg cggttttggg tggtgctaat 1260

gttgttcgcg ttcataacgt tggagataat gcggatgctg taaagctttg tgattcaatg 1320

ttgaaccg 1328

<210> 22
<211> 486
<212> DNA
<213> Ambrosia trifida

<400> 22

gccttaagtc aaatgaagaa atcaggcata gagataacaa gacatgcatg tttatacaaa 60

accgagccag cttatgtaac tgatcaacct ctttttctta attctgccat cagaggcgtt 120

acaaagctgg gcccacatga actactatcg gccctcaaga aaatcgaaaa agaattgggc 180

cgaaccaaag gtattagata cggcccgcga cccattgact tagatatact gttttatggt 240

aaacacagaa ttaactcgga aattctcact gttccacatg aaagaatctt cgagaggcca 300

tttgttatgg ctccgttagt tgacttattg gggtcggata ttgacaatga tacggttcta 360

tgctggcatt ctttctcaaa aagaggactt tttgaatctt gggaaaaatt aggtggcgaa 420

tctttgatag gcaaagatgg gttaagaaga gttttaccgg tcaataatcg gttatgggat 480

tggtca 486

<210> 23
<211> 486
<212> DNA
<213> Ambrosia trifida

<400> 23

gccttaagtc aaatgaagaa atcaggcata gagataacaa gacatgcatg tttatacaaa 60

accgagccag cttatgtaac tgatcaacct ctttttctta attctgccat cagaggcgtt 120

acaaagctgg gcccacatga actactatcg gccctcaaga aaatcgaaaa agaattgggc 180

cgaaccaaag gtattagata cggcccgcga cccattgact tagatatact gttttatggt 240

aaacacagaa ttaactcgga aattctcact gttccacatg aaagaatctt cgagaggcca 300

tttgttatgg ctccgttagt tgacttattg gggtcggata ttgacaatga tacggttcta 360

tgctggcatt ctttctcaaa aagaggactt tttgaatctt gggaaaaatt aggtggcgaa 420

tctttgatag gcaaagatgg gttaagaaga gttttaccgg tcaataatcg gttatgggat 480

tggtca 486

<210> 24
<211> 410
<212> DNA
<213> Ambrosia trifida

<400> 24

gacccatcca caatgcaaaa cagtgagaat ttgaagtata atgatgtttg taaagaagtt 60

ggtgatgagt tgtatgagcg tgtaagagct gcagagttat gcggtgtgcc tgcatggagg 120

atcattcttg acccagggat cgggttttca aagaaaaccg aagataattt ggatattttg 180

atgggattaa agagaattag gagtgagatt gcccggaaga gcttaggggt gtcacgtgca 240

cctttgttaa tcggtccttc aagaaagaga tttttgggag agatttgtgg tcgggcttct 300

gctggtgaga gagatccaac aactgttgct gctgttacat gtgcggtttt gggtggtgct 360

aatgttgttc gcgttcataa tgttggagat gatgctgatg ctgtaaagct 410

<210> 25
<211> 381
<212> DNA
<213> Ambrosia trifida

<400> 25

ttcgtgtatc tcatgatctc tgtataatgt taaacttatt atagttcaat ataagtcact 60

gtgtgatcta atttcgtata caattacttt atttgtgttg attctgatcc taaatattgc 120

taatagtttg tgcttaacta tatatcccgt taaacatcac atttacttta tccctagaag 180

atttgggcaa gtttttacaa atttatggcg acgttattgt aattttttaa atcattttcg 240

tgcagcatct ttttcgttct ttcatttctc ttcggatgct tcgatacaag tccattctca 300

agaacaagaa gtagtaattg ctttaggtag caatgtgggt gataggctta ataactttaa 360

tgaagcctta agtcaaatga a 381

<210> 26
<211> 304
<212> DNA
<213> Ambrosia trifida

<400> 26

gacccatcca caatgcaaaa cagtgagaat ttgaagtata atgatgtttg taaagaagtt 60

ggtgatgagt tgtatgagcg tgtaagagct gcagagttat gcggtgtgcc tgcatggagg 120

atcattcttg acccagggat cgggttttca aagaaaaccg aagataattt ggatattttg 180

atgggattaa agagaattag gagtgagatt gcccggaaga gcttaggggt gtcacgtgca 240

cctttgttaa tcggtccttc aagaaagaga tttttgggag agatttgtgg tcgggcttct 300

gctg 304

<210> 27
<211> 255
<212> DNA
<213> Ambrosia trifida

<400> 27

gatttgtggt cgggcttctg ctggtgagag agatccaaca actgttgctg ctgttacatg 60

tgcggttttg ggtggtgcta atgttgttcg cgttcataat gttggagatg atgctgatgc 120

tgtaaagctt tgtgattcaa tgttgaaccg ggttggaaga acgtaacagc tttttgaaat 180

ttgattgtgg gttttggtcc gggtggtggt gctcgggtgt ggtttgggtt ttggttcgag 240

tgggtggttg ctggt 255

<210> 28
<211> 201
<212> DNA
<213> Ambrosia trifida

<400> 28

acctcatcac ctccacaaat caccaccacc catctccacc ctccaccacc acccacttca 60

ttctccccac ccaccgtatc tcatcaccat catacctggt ggattgcagt ttgactctgt 120

ggggtccgct atatctcgtg ttaggaccat gatatctgaa ggggcggaca taattgatct 180

gggagctcaa tcgacacgtc c 201

<210> 29
<211> 101
<212> DNA
<213> Ambrosia trifida

<400> 29

ggattgcagt ttgactctgt ggggtccgct atatctcgtg ttaggaccat gatatctgaa 60

ggggcggaca taattgatct gggagctcaa tcgacacgtc c 101

<210> 30
<211> 5446
<212> DNA
<213> Conyza canadensis

<400> 30

tttcttcttc tagttggagc tcttttacgt ggtgtcttct tttccgagtc atcttcttcc 60

ccatcagatg gattttctgc agcctttttt ctacgaactg ctgtaatagc aaatgtacta 120

cgcaatctaa catctgggaa ttgtacaaag tttattgaag atagtgaagg cagattcgac 180

tgccacctac acatataaga aaccattaac ttctatactt aactcacaat ataaaacatc 240

atttgcatgt tcgtcaaaaa caataccatt tcttatcttt gctcaatgga tatgaaatct 300

gccaatacaa gattattcat ttgacctcaa tctagcatct gatgtctatt caaggtatcg 360

tttataatga agattattgt acttcaaaat actttcttgt gtagtggtat caaattttga 420

gttaaagtta ctccatttga cctcaaaagc caaataatga gcaacaaaca acacaatttc 480

acctccctta taccctgcga ctatatttta tcttcaaatc aatccatttc catgtttatc 540

ctctccatac agctacataa taagttttag ggaaaaaaca aaactaaact aaactaagtt 600

tacagaattc ccatttctta tcctaaaatt tacaagcaat tcaacatata ttctttattt 660

acaactataa atagcatatg ggtatgttgg tatatagata tagatataga tatacatatg 720

tagatgtgta ttatatgtgt gtaaatgggg agaaaaaagg aaacatatat tacctgtgta 780

aagaacaaaa ttgagtaaaa gaaggcaaag aagccatttc taaccactaa aatcttgggc 840

tttcaagaat cccacttcaa agctaccatt tcacatacca aacccccctt tctatgggtt 900

taaagatgag ggctttacat ttatctcttt tacaatccaa attttccaaa accgaaataa 960

tgtacgacat tcggtgcaca aggtacaaac agaaaaaact acttttgtat tttagatctc 1020

aagaaaggag tggccttttt ggtaataaca tactttattc aattgccact taccttcaag 1080

aaattaacaa aattacccac aattaaaaaa gggtttccca gtttcactca cacttattca 1140

agattgatgc aaaataaacc cataaataag aaggttaaaa tagtgttaaa gccttaaagg 1200

tgcttgcttt ttgtgttttt tgtgatgcct ataaaaagat tcttattgtt ttcaaatttt 1260

tttatgtcaa gttttcgttt ttaacagttt atatagttat ataatttgca gaaagccata 1320

aagctctgaa cttgaatgtt ttatagcagc tctttgcagt aatatcttgt agaggtaaac 1380

ctgttaaccc atatttatta atttgtacat aaaattgttg aattgaatag agctaccact 1440

gggacttgtg tagtcaaact gtagagcatc cctgtaatgc tggaagcttt atgacctaaa 1500

aggatttgta gacttgttat ctgtggattg gtataaagat tttcaagttg ttattttgaa 1560

actttgaata gtatattatg ctcattactg aatgatacaa tagggagtcg cgaatgttag 1620

aattgataag aattaggatg aagtttgtgt ttgaacgttc cttctgttta taatcttcat 1680

gtcacttcca tagtaccata accctagggc aacacgagcc tttagattat gaatgggcac 1740

acccaattgt ttagaccttg tataaatgaa gtgaatatca ttgttataag cattctggtg 1800

gtggttttgc agatttacct ttctctatct aatttgaagg tttgttgatt aaggtgatac 1860

aaatgagtgt tttcaagcag ctaatagctt caagattagt acgcactcat gtctgtggat 1920

cttatagagg tacacttcct cgaacattca tatagcgatg ttattgttaa gttttgaatc 1980

tgtagcaaca ttatatataa ttttttttgc attgttttcc tgcagcatcc ttttcactct 2040

ttcattcgtc ctcggatact tcaatacaag tgcattctcc agaacaagaa gtagtaattg 2100

ctttaggtag caatgtgggt aatagactta ataactttaa tgaagcctta tcccagatga 2160

agaaatcggg gatagaaata acaaggcatg cttgtctata cgaaactgaa ccagcttatg 2220

tgacggacca gcctcttttt ctcaactctg ccgttagagc cactacaaag cttggccctc 2280

atgagctact gtccattctc aagaaaattg aaaaggaaat gggtcgaacc aaagggctta 2340

ggtacggccc acgacccatt gacctagata tattgttcta tggtaaatgc aaagttaact 2400

ctgatattct aactgttcct catgaaagaa tctttgagag gccattcgtt atggctccat 2460

tagttgactt gttaggatca gatgtagaca atgatacggt tctatgctgg cattcttttt 2520

caaaaaatgg gctttttgga tcttgggaaa cattaggtgg tgaatcttcc ataggaaaag 2580

atggtttaag aagggtttta cctgtcaatg atcgtttatg ggattggtca aagaaaactt 2640

ccgtcatggg tattttgaat ataactcctg atagttttag tgatggaggg aagtttgatt 2700

ccatggggtc cgctttatct cgtgttcaga ccatgatatc tgaaggggtt gacataattg 2760

atctaggagc tcaatccaca cgcccaatgg cgaccaagat ctcagtcgaa gaggaactag 2820

ataggctaat acccgttctt gaaaagattc ttgaattacc tgaaattgaa ggaaagttgt 2880

tgtctgtgga cacattttac tcggaagttg cttcagaggc aatcaagaaa ggggctcata 2940

tggtcaatga tgtatcgggt ggaatgttag attctgatat gcttcatgtt gtggctgatc 3000

taaatgttcc atatatcact atgcacatga gaggggaccc atccacaatg caaaacagtg 3060

agaatttgaa gtatgatgat gttgtcaaag aagttgggga ggaattgtat gaacgtgtaa 3120

ggaatgcaga gttatgtggt gttcccgcat ggaggatggt tcttgatcca gggatcgggt 3180

tttcaaagaa aaccgaagat aatttggaga tattgatggg actaaagagg tttagaagtg 3240

agattggaca aaagagctta ggggtgtctc gtgcaccttt gttaatcgga ccttcaagaa 3300

aaaggttttt gggtgagatt tgtggtcggc cttctgctgt tgagagagat ccagggactg 3360

tagctgctgt taccagtgcg attttgggtg gtgccaatat cgttagggtt cataacattg 3420

gacataatgt ggatgctgtt aagctttgtg attcaatgtt ggatcgagct ggtagatctt 3480

agtagcttaa agttatttga catcttgttt tttattcaat tttctggaca ttactttttg 3540

tatttctgtc tggcaaatga ttttacaggg atatcctaga ggctaaattg tatattacat 3600

tcttgatttt ctcacaaagt tttacttttt cagtttttct tgttcagttt tgtttttgaa 3660

aatttgctag atcagattaa atcgttgctt tatgatcttc gtgtcttttt tgggacgctg 3720

cgagtttcat ttagtggtat tgtaaactcc ttttaaagtt tgtacacact tggtatttat 3780

gcttctagtt ttcgtctatg tctgtcctat attgtgtata gtatgtttaa agttcataaa 3840

aagaggataa acgaatagca cgtaaatctt ataattcgat tccacagagt gcaagttatg 3900

ctttcctaaa tgaaagcaag agtctagaca gtttggtcaa cgttaacatg agactattgt 3960

agaatacaac accacaagaa accaaaactg tcttgtgcat ttctctcttt tccaggttta 4020

aaactcaata ccaagacttg atatcaacta ttattctgct tcaatgattc agcttcagct 4080

atagccatag gatcatcttt ggtgtcaggt tcatccttct caccatctcg ttcctctgcc 4140

agtttttttc tgtgcaattc ctctgcaact tgctttgatg cttggtcttc tttttgaagg 4200

cgttgcatct cttcttcttg ttgctgtcgt tcaaaccatg tatccgcatc ggcccaaact 4260

gtaatttaga gccaaaacaa catgattaga atgcagattc cagaagccaa attcaaactg 4320

taagaaaaaa aatgtagtga tgatttaact caaaacaact cacacagctc caaaatggat 4380

gggggttcaa attagaggat agcctaatag atagaagctt ctaattatgg ggaaaaaaaa 4440

acgatcatgg caatgtatta cacaatctaa agcaaccact atgaataata aagataaatc 4500

tttccgagag tccacatgcc ttgatatttc ataagatagg ctttttcaga aatcagaatc 4560

ttcttggcaa atataacact aaaatcagtc taatgcatgt tattaagtaa atctttattt 4620

gtccatagta taatgccata gattatcgtc aattatccac atcgtcatta aatatcaagt 4680

aaccacatta tgaattgact tgtttaatca aagttcatct tacttcttca tagatgaaaa 4740

agagctatgc aaatacagaa cacatcagtt gttctaacat ataattactg ctagctgtag 4800

gaagaaacca agatgaaact tatctaggat tcaaattcat ggggatctgt tatttagatg 4860

aatttagaga aataagaaaa taagttgagt gaagtcagtt tcgaggttcc aaatttgtct 4920

ttattagttg agctaacaat attaacctct gaagcatgta tagtgtactt gtatgaacca 4980

cttaaggata taaatgaagc caaatcagtt tcattcgcta attgagcaaa tgcacgttct 5040

gtttgttagt attgaaattt ctttacagga tttctttcaa ctaaaagcat cacatgtccg 5100

actctcggtt tgatatttgt gacaagtcta attggttcat cagatgtact tatggctaaa 5160

tttcgaaact tgaaacaata agagcttagc actcttaacc ataaagggtt gctgttaatc 5220

ttacttgttc ttgaaagcaa ttccacagtg aagcatttgt taattgggaa gttttgcaat 5280

gtttctaaag cttcgatgat attttatatt ttcaaacatt tttcatcaca tgtgttaaca 5340

aaagccataa gaataccggg gtaatcaact aaagtctatt taaagttgca tgctcgagag 5400

caagaaaaaa ccatcaaatt tacataaact gaagggcaaa tgtacg 5446

<210> 31
<211> 5446
<212> DNA
<213> Conyza canadensis

<400> 31

ttttcttctt ctagttggag ctcttttacg tggtgtcttc ttttccgagt catcttcttc 60

cccatcagat ggattttctg cagccttttt tctacgaact gctgtaatag caaatgtact 120
acgcaatcta acatctggga attgtacaaa gtttattgaa gatagtgaag gcagattcga 180
ctgccaccta cacatataag aaaccattaa cttctatact taactcacaa tataaaacat 240
catttgcatg ttcgtcaaaa acaataccat ttcttatctt tgctcaatgg atatgaaatc 300
tgccaataca agattattca tttgacctca atctagcatc tgatgtctat tcaaggtatc 360
gtttataatg aagattattg tacttcaaaa tactttcttg tgtagtggta tcaaattttg 420
agttaaagtt actccatttg acctcaaaag ccaaataatg agcaacaaac aacacaattt 480
cacctccctt ataccctgcg actatatttt atcttcaaat caatccattt ccatgtttat 540
cctctccata cagctacata ataagtttta gggaaaaaac aaaactaaac taaactaagt 600
ttacagaatt cccatttctt atcctaaaat ttacaagcaa ttcaacatat attctttatt 660
tacaactata aatagcatat gggtatgttg gtatatagat atagatatag atatacatat 720
gtagatgtgt attatatgtg tgtaaatggg gagaaaaaag gaaacatata ttacctgtgt 780
aaagaacaaa attgagtaaa agaaggcaaa gaagccattt ctaaccacta aaatcttggg 840
ctttcaagaa tcccacttca aagctaccat ttcacatacc aaacccccct ttctatgggt 900
ttaaagatga gggctttaca tttatctctt ttacaatcca aattttccaa aaccgaaata 960
atgtacgaca ttcggtgcac aaggtacaaa cagaaaaaac tacttttgta ttttagatct 1020
caagaaagga gtggcctttt tggtaataac atactttatt caattgccac ttaccttcaa 1080
gaaattaaca aaattaccca caattaaaaa agggtttccc agtttcactc acacttattc 1140
aagattgatg caaaataaac ccataaataa gaaggttaaa atagtgttaa agccttaaag 1200
gtgcttgctt tttgtgtttt ttgtgatgcc tataaaaaga ttcttattgt tttcaaattt 1260
ttttatgtca agttttcgtt tttaacagtt tatatagtta tataatttgc agaaagccat 1320
aaagctctga acttgaatgt tttatagcag ctctttgcag taatatcttg tagaggtaaa 1380
cctgttaacc catatttatt aatttgtaca taaaattgtt gaattgaata gagctaccac 1440
tgggacttgt gtagtcaaac tgtagagcat ccctgtaatg ctggaagctt tatgacctaa 1500
aaggatttgt agacttgtta tctgtggatt ggtataaaga ttttcaagtt gttattttga 1560
aactttgaat agtatattat gctcattact gaatgataca atagggagtc gcgaatgtta 1620
gaattgataa gaattaggat gaagtttgtg tttgaacgtt ccttctgttt ataatcttca 1680
tgtcacttcc atagtaccat aaccctaggg caacacgagc ctttagatta tgaatgggca 1740
cacccaattg tttagacctt gtataaatga agtgaatatc attgttataa gcattctggt 1800
ggtggttttg cagatttacc tttctctatc taatttgaag gtttgttgat taaggtgata 1860
caaatgagtg ttttcaagca gctaatagct tcaagattag tacgcactca tgtctgtgga 1920

tcttatagag gtacacttcc tcgaacatta tatagcgatg ttattgttaa gttttgaatc 1980
tgtagcaaca ttatatataa ttttttttgc attgttttcc tgcagcatcc ttttcactct 2040
ttcattcgtc ctcggatact tcaatacaag tgcattctcc agaacaagaa gtagtaattg 2100
ctttaggtag caatgtgggt aatagactta ataactttaa tgaagcctta tcccagatga 2160
agaaatcggg gatagaaata acaaggcatg cttgtctata cgaaactgaa ccagcttatg 2220
tgacggacca gcctcttttt ctcaactctg ccgttagagc cactacaaag cttggccctc 2280
atgagctact gtccattctc aagaaaattg aaaaggaaat gggtcgaacc aaaggcttta 2340
ggtacggccc acgacccatt gacctagata tattgttcta tggtaaatgc aaagttaact 2400
ctgatattct aactgttcct catgaaagaa tctttgagag gccattcgtt atggctccat 2460
tagttgactt gttaggatca gatgtagaca atgatacggt tctatgctgg cattcttttt 2520
caaaaaatgg gctttttgga tcttgggaaa cattaggtgg tgaatcttcc ataggaaaag 2580
atggtttaag aagggtttta cctgtcaatg atcgtttatg ggattggtca aagaaaactt 2640
ccgtcatggg tattttgaat ataactcctg atagttttag tgatggaggg aagtttgatt 2700
ccatggggtc cgctttatct cgtgttcaga ccatgatatc tgaaggggtt gacataattg 2760
atctaggagc tcaatccaca cgcccaatgg cgaccaagat ctcagtcgaa gaggaactag 2820
ataggctaat acccgttctt gaaaagattc ttgaattacc tgaaattgaa ggaaagttgt 2880
tgtctgtgga cacattttac tcggaagttg cttcagaggc aatcaagaaa ggggctcata 2940
tggtcaatga tgtatcgggt ggaatgttag attctgatat gcttcatgtt gtggctgatc 3000
taaatgttcc atatatcact atgcacatga gaggggaccc atccacaatg caaaacagtg 3060
agaatttgaa gtatgatgat gttgtcaaag aagttgggga ggaattgtat gaacgtgtaa 3120
ggaatgcaga gttatgtggt gttcccgcat ggaggatggt tcttgatcca gggatcgggt 3180
tttcaaagaa aaccgaagat aatttggaga tattgatggg actaaagagg tttagaagtg 3240
agattggaca aaagagctta ggggtgtctc gtgcaccttt gttaatcgga ccttcaagaa 3300
aaaggttttt gggtgagatt tgtggtcggc cttctgctgt tgagagagat ccagggactg 3360
tagctgctgt taccagtgcg attttgggtg gtgccaatat cgttagggtt cataacattg 3420
gacataatgt ggatgctgtt aagctttgtg attcaatgtt ggatcgagct ggtagatctt 3480
agtagcttaa agttatttga catcttgttt tttattcaat tttctggaca ttactttttg 3540
tatttctgtc tggcaaatga ttttacaggg atatcctaga ggctaaattg tatattacat 3600
tcttgatttt ctcacaaagt tttacttttt cagtttttct tgttcagttt tgtttttgaa 3660
aatttgctag atcagattaa atcgttgctt tatgatcttc gtgtcttttt tgggacgctg 3720
cgagtttcat ttagtggtat tgtaaactcc ttttaaagtt tgtacacact tggtatttat 3780
gcttctagtt ttcgtctatg tctgtcctat attgtgtata gtatgtttaa agttcataaa 3840

aagaggataa acgaatagca cgtaaatctt ataattcgat tccacagagt gcaagttatg 3900

ctttcctaaa tgaaagcaag agtctagaca gtttggtcaa cgttaacatg agactattgt 3960

agaatacaac accacaagaa accaaaactg tcttgtgcat ttctctcttt tccaggttta 4020

aaactcaata ccaagacttg atatcaacta ttattctgct tcaatgattc agcttcagct 4080

atagccatag gatcatcttt ggtgtcaggt tcatccttct caccatctcg ttcctctgcc 4140

agtttttttc tgtgcaattc ctctgcaact tgctttgatg cttggtcttc tttttgaagg 4200

cgttgcatct cttcttcttg ttgctgtcgt tcaaaccatg tatccgcatc ggcccaaact 4260

gtaatttaga gccaaaacaa catgattaga atgcagattc cagaagccaa attcaaactg 4320

taagaaaaaa aatgtagtga tgatttaact caaaacaact cacacagctc caaaatggat 4380

gggggttcaa attagaggat agcctaatag atagaagctt ctaattatgg ggaaaaaaaa 4440

acgatcatgg caatgtatta cacaatctaa agcaaccact atgaataata aagataaatc 4500

tttccgagag tccacatgcc ttgatatttc ataagatagg ctttttcaga aatcagaatc 4560

ttcttggcaa atataacact aaaatcagtc taatgcatgt tattaagtaa atctttattt 4620

gtccatagta taatgccata gattatcgtc aattatccac atcgtcatta aatatcaagt 4680

aaccacatta tgaattgact tgtttaatca aagttcatct tacttcttca tagatgaaaa 4740

agagctatgc aaatacagaa cacatcagtt gttctaacat ataattactg ctagctgtag 4800

gaagaaacca agatgaaact tatctaggat tcaaattcat ggggatctgt tatttagatg 4860

aatttagaga aataagaaaa taagttgagt gaagtcagtt tcgaggttcc aaatttgtct 4920

ttattagttg agctaacaat attaacctct gaagcatgta tagtgtactt gtatgaacca 4980

cttaaggata taaatgaagc caaatcagtt tcattcgcta attgagcaaa tgcacgttct 5040

gtttgttagt attgaaattt ctttacagga tttctttcaa ctaaaagcat cacatgtccg 5100

actctcggtt tgatatttgt gacaagtcta attggttcat cagatgtact tatggctaaa 5160

tttcgaaact tgaaacaata agagcttagc actcttaacc ataaagggtt gctgttaatc 5220

ttacttgttc ttgaaagcaa ttccacagtg aagcatttgt taattgggaa gttttgcaat 5280

gtttctaaag cttcgatgat attttatatt ttcaaacatt tttcatcaca tgtgttaaca 5340

aaagccataa gaataccggg gtaatcaact aaagtctatt taaagttgca tgctcgagat 5400

caagaaaaaa ccatcaaatt tacataaact gaagggcaaa tgtacg 5446

<210> 32
<211> 1401
<212> DNA
<213> Conyza canadensis

<400> 32

tcgtcctcgg atacttcaat acaagtgcat tctccagaac aagaagtagt aattgcttta 60

ggtagcaatg tgggtaatag acttaataac tttaatgaag ccttatccca gatgaagaaa 120

tcggggatag aaataacaag gcatgcttgt ctatacgaaa ctgaaccagc ttatgtgacg 180

gaccagcctc tttttctcaa ctctgccgtt agagccacta caaagcttgg ccctcatgag 240

ctactgtcca ttctcaagaa aattgaaaag gaaatgggtc gaaccaaagg gcttaggtac 300

ggcccacgac ccattgacct agatatattg ttctatggta aatgcaaagt taactctgat 360

attctaactg ttcctcatga aagaatcttt gagaggccat tcgttatggc tccattagtt 420

gacttgttag gatcagatgt agacaatgat acggttctat gctggcattc tttttcaaaa 480

aatgggcttt ttggatcttg ggaaacatta ggtggtgaat cttccatagg aaaagatggt 540

ttaagaaggg ttttacctgt caatgatcgt ttatgggatt ggtcaaagaa aacttccgtc 600

atgggtattt tgaatataac tcctgatagt tttagtgatg gagggaagtt tgattccatg 660

gggtccgctt tatctcgtgt tcagaccatg atatctgaag gggttgacat aattgatcta 720

ggagctcaat ccacacgccc aatggcgacc aagatctcag tcgaagagga actagatagg 780

ctaatacccg ttcttgaaaa gattcttgaa ttacctgaaa ttgaaggaaa gttgttgtct 840

gtggacacat tttactcgga agttgcttca gaggcaatca agaaaggggc tcatatggtc 900

aatgatgtat cgggtggaat gttagattct gatatgcttc atgttgtggc tgatctaaat 960

gttccatata tcactatgca catgagaggg gacccatcca caatgcaaaa cagtgagaat 1020

ttgaagtatg atgatgttgt caaagaagtt ggggaggaat tgtatgaacg tgtaaggaat 1080

gcagagttat gtggtgttcc cgcatggagg atggttcttg atccagggat cgggttttca 1140

aagaaaaccg aagataattt ggagatattg atgggactaa agaggtttag aagtgagatt 1200

ggacaaaaga gcttaggggt gtctcgtgca cctttgttaa tcggaccttc aagaaaaagg 1260

tttttgggtg agatttgtgg tcggccttct gctgttgaga gagatccagg gactgtagct 1320

gctgttacca gtgcgatttt gggtggtgcc aatatcgtta gggttcataa cattggacat 1380

aatgtggatg ctgttaagct t 1401

<210> 33
<211> 1262
<212> DNA
<213> Conyza canadensis

<400> 33

gaaataacaa ggcatgcttg tctatacgaa actgaaccag cttatgtgac ggaccagcct 60

ctttttctca actctgccgt tagagccact acaaagcttg gccctcatga gctactgtcc 120

attctcaaga aaattgaaaa ggaaatgggt cgaaccaaag ggcttaggta cggcccacga 180

cccattgacc tagatatatt gttctatggt aaatgcaaag ttaactctga tattctaact 240

gttcctcatg aaagaatctt tgagaggcca ttcgttatgg ctccattagt tgacttgtta 300

ggatcagatg tagacaatga tacggttcta tgctggcatt ctttttcaaa aaatgggctt 360

tttggatctt gggaaacatt aggtggtgaa tcttccatag gaaaagatgg tttaagaagg 420

gttttacctg tcaatgatcg tttatgggat tggtcaaaga aaacttccgt catgggtatt 480

ttgaatataa ctcctgatag ttttagtgat ggagggaagt ttgattccat ggggtccgct 540

ttatctcgtg ttcagaccat gatatctgaa ggggttgaca taattgatct aggagctcaa 600

tccacacgcc caatggcgac caagatctca gtcgaagagg aactagatag gctaataccc 660

gttcttgaaa agattcttga attacctgaa attgaaggaa agttgttgtc tgtggacaca 720

ttttactcgg aagttgcttc agaggcaatc aagaaagggg ctcatatggt caatgatgta 780

tcgggtggaa tgttagattc tgatatgctt catgttgtgg ctgatctaaa tgttccatat 840

atcactatgc acatgagagg ggacccatcc acaatgcaaa acagtgagaa tttgaagtat 900

gatgatgttg tcaaagaagt tggggaggaa ttgtatgaac gtgtaaggaa tgcagagtta 960

tgtggtgttc ccgcatggag gatggttctt gatccaggga tcgggttttc aaagaaaacc 1020

gaagataatt tggagatatt gatgggacta aagaggttta gaagtgagat tggacaaaag 1080

agcttagggg tgtctcgtgc acctttgtta atcggacctt caagaaaaag gtttttgggt 1140

gagatttgtg gtcggccttc tgctgttgag agagatccag ggactgtagc tgctgttacc 1200

agtgcgattt tgggtggtgc caatatcgtt agggttcata acattggaca taatgtggat 1260

gc 1262

<210> 34
<211> 4643
<212> DNA
<213> Euphorbia heterophylla

<400> 34

tttgtatatt aaaacaataa aaatgatcca gattaataaa tagaaaaagt attaataacc 60

cgaaaatatc aaatatcaaa atataatgaa acatgaacac gaaatcacaa taaagaagtt 120

ggtatcacaa acacaaagac acccttaaag ctcctatgtt tgtggagagt tgtaagtcga 180

ctaatttcag attgcaaaag ttaaaaatgg ccaacttttt tagctatgag gttagattag 240

atacgagaat gccttggtga aaagctggtc actgtttctt cattcgcgtg gcaattaacc 300

cggcataatc gatacccaat tcacggacaa ggtcaaaggt atcagcttta ctcaaccctt 360

cgctttgata ctaaattgct gattcttatt ctgattatgc tttatttcgt tacttcattt 420

tattgtctat gcacataagc tgttcgacaa aaagcctgaa tgaaactgtg agtgctggta 480

cgcggttaca atactcctga ttatgtccaa tgattatttg ggtactttaa taccctaatt 540

tgtgaagttc actagcgaaa gggtagtttc ttccccggtt atttatcatt ttatgaatcc 600

ccaagtcttt gaagtacagc tgattaaaat gcaagtactc ataagttgtc attgtatgtt 660

tgaagtcctt acatttttgg tattttacag aatttgtact gaagaagcat aaattcatac 720

ctagttaggc ttccttgttc ctaatatgct tctcctcaag cggctggtgt ccactaaaca 780

agggttcaat aatgccatca accgttttgg aggtaatata gttatgccta tgatgcttta 840

agtcgctttt catcgatttg ttacattacc aggctaagat tctgcaattg aagttagaaa 900

tcgacgagtc ttgtcttgaa aagtatggat tttgttttgt caccctgggg ccggttcaag 960

attgctttcc caggttgctg tcttggcatc acgtcctaat ccaaaaccct aaggtgatgg 1020

gttaatgggt cccttcgcag ttatatatca ttcacttaac tcattaactt ccatgtggat 1080

aatcaactcc acacttgccc caacactgta taaacgtaga ttttgacctt tggaaatcat 1140

tagctggaca aatcctaact aattagtatc aagagccaaa atcttttgac caagaccaaa 1200

atatgttgtt tcattgttgc agcattgtca tcatcctttc tccataccga accaaatagt 1260

tcggtggaag ttcattctca agagcaagaa gtagtaattg cattaggaag caatgtggga 1320

gatagagttc ataatttcaa ccaagcattg caattaatga aaagttccgg cattaacata 1380

actagacatg gttgtttgta ccagacagca cctgcttatg tcactgacca acctcacttt 1440

ctcaactcag cagttcgagc tttcacgaaa ctcggacccc acgagttatt aggagtttta 1500

aagcagatcg aaaaggactt gggccgtacc aaaggattag ggtacggacc aaggccaatc 1560

gatttggata tacttttcta tggaaagttc cggattaatt ccgatacact tattgttcct 1620

catgagagaa tatgggagag accctttgtg atggccccat tggtggattt actcggctcg 1680

gaaatcgaga atgacacggt cgccagctgg cattccttgt ccggcggtct ttttgaatca 1740

tgggaaaagt tgggcggcga aagcctgatc gggaaggacg gaatgaaaag agttaccccg 1800

atcggaaacc atttatggga ttggtcggaa aagacttctg taatgggaat tatcaattta 1860

acccccgata gtttcagcga tgggggcaag ttgacaacta ttgattctat agtttctaag 1920

gttcgctcga tgatttccga aggggcggat attgtcgatt ttggtgctca atcgacacgc 1980

cctatggcta aaaggatatc cccgcaagag gaaatggata ggctaatccc tgtattggaa 2040

gcggttgtga aaatacccga gatgaccgaa aagctcatat cggtcgacac attttactcg 2100

gaagtcgcct tagaagcggt ccaaagagga gcaaatctcg taaatgacgt atctgggggg 2160

caattagatc caaaaatgac ggaaaatgtc gctaatctcg aggtaccata tatcttaatg 2220

cacatgaggg gggaccccac cacaatgcag aacactcaga atctaaaata cgataatgtt 2280

tgtaaagagg ttgcctttga gttgttttcg agggttaaag aagccgagat gtccggaatt 2340

ccagcctggc gaatgtttat tgaccctgga attgggtttt cgaagaacac gaatcagaat 2400

ctggaaatcc tgatgggaat tccaaggatc cgggccgaga ttgggaggaa aagcgtgggt 2460

atttctcgtg ggccgattct tattgggcct tcgagaaaga ggtttttggg cgaaatttgc 2520

gaacgccctg aagcaactga aagagacccg gcaactgttg cttctgttac tactgggatt 2580

ctgggagggg caaatattgt tagagttcat aatgttaggg ataatgtgga tgctgtgaag 2640

ctatgtgact cgatgatgaa actaaagagg tctacttaaa aattatcggg tatagttcga 2700

tttctttctt tccgaatctt ttgtttgact ttcagattgt ctttgattgg caaaacagtt 2760

tcggctgcgg ttactgtttg ctgttgcagt ttaccgttgt tgtttctaat tgtgatttta 2820

agttattgtt gttagttgat ttgtacttag tgcttggtga aaatataatt cacggttgcg 2880

gttggaggta taaattacta aaatagacat attttaaata aattagaagt tatattgaag 2940

ggtatttagg ataaataaaa gtccaaacca caaaaaaaaa aaaccgtaaa actattgatt 3000

tggtgaggtt tggaaaagga gataaatccc ttatgaaaag caactccata tatgttttga 3060

aacttaacca aacacttgta tacctccttt tccttttcaa acctcaaaag acctttttat 3120

agtaagctct tttgaagtat atacgctatt gtttccgaat cattttggac ttttcgtcga 3180

aatcaacaat actgttagct agtcatattt caagatattt gggatgtaaa gatcaaggta 3240

gttgttgttg tattctgttc tcatcagcaa aatttgttat aactccacca agatttaatt 3300

ttttccaatc tgaaaatgtc ttatgttata gtaagaagtg agatgagttt atgtctttgt 3360

gaaaagattt tgcccaatta atgcaaaaat tttgtgacaa gacaagaggg gtgaaagaga 3420

caagcaacaa gagaaagcag tgaaaggggc aaatgttagg aaactgttag ctagaaaagg 3480

gtagagggtg gcctacttta ccaatttgag cattaaaaaa gcacatggct atgtatttta 3540

gaccaaagtc cctattgttg gcagatgaac taagcaacca tatggttcat taactaacta 3600

actagttatt gcaaatttga ctgttccaat tcatcaaagg actctcccca tagaccattc 3660

aacacttggg ttctagccct ccctctcacc ctcagacaac ccttgtgaac ctcattttca 3720

tcattggtcc ccttttttgt taggactaca cttgtctcta taaaatctct tctagccatg 3780

tgttatgtgt agactttttc ggtttgagga ttgggtcggg ttcgggctgg gtctcgtcgg 3840

gctttttact gcaaacatca aactagggtt ctaaacccat acttcagtac tctggttcgg 3900

attgaacttg cgagatcatt ctaaattcaa tttacggctc atagattata tttgagagtt 3960

ttgagttttg ggtggattag attgaaaacg actatttttt gttgtataaa tatatctaaa 4020

aagttatata attacattct ttattggaat tcatcaatat atataagaat ttgactttgt 4080

tgtagccatt ttgaaagttc gataagctac tcccttcgtc tagtttcaat agttcattta 4140

aaagtttcat taattctgtt ttcaattgtc aatttaaatt ttcataatat ttttcaccat 4200

ttttttttcc aatttgccct tgtatttatt gagaaagaga ttatgttatg atgaaagtat 4260

aagttgctaa tcaagaagaa agagatttta atgtaattat ttaaataata aattcctacc 4320

attttaattg tgtgacaatt ggttataatg attccgttgc atcgacaact aaaactagat 4380

agaaggatca aaatataaaa ttttaacata attaatacat ataatatata aatgtataat 4440

agatacaatt attattcctc gaattaaaat tggctaatta tataataata taatccaacc 4500

tataaaaact tttgagcttt taagcttgag atgagctttg taaaaatgat tgtacaagcc 4560

cgaccatgaa agagagccaa gtagggtcaa atgcataccc cggcttgaat ataaattcaa 4620

tatatgttct ttcaaatctt gcc 4643

<210> 35
<211> 4556
<212> DNA
<213> Euphorbia heterophylla

<400> 35

aaatataaat ataaatataa aaataaaaat aaattataat aatataaaat ataaaaatta 60

atttaaacca tgttttaaaa atagtgttat gaaaaactcc gcttgggcgc cgtctagata 120

gtttacttag gctgattttt aaaacaaaac cccccgctta acccgcctaa atcggtcaat 180

tgtttatatc cttatgacta cttaaaatcg cagcggtgtt atggtgaatt atcatgtttt 240

aatgagctta tgaaatgcaa taataaaaaa ataaccatta ataatttgat atacatttat 300

cgggaaaaat gaaataataa aaaataaaaa aatatcaaaa aaatctataa aatatttgta 360

cctttttcaa ttagtaccca ttttgtatat taaaacaata aaaatgatcc agattaataa 420

atagaaaaag tattaataac ccgaaaatat caaatatcaa aatataatga aacatgaaca 480

cgaaaacaca ataaagaagt tggtatcaca aacacaaaga cacccttaaa gctcctatgt 540

ttgcggagag ttgtaagtcg actaatttca gattgaaaaa gttaaaaatg gccaactttt 600

ttagctatga ggttagatta gatacgagaa tgcctttgtg aaaagctggt cactatttct 660

tcattcgcgt ggcaattaac ccggcataat cgatagccaa ttcacggaca aggtcaaagg 720

tatcagcttt actcaaccct tcgctttgat actaaattgc tgattcttat tctgattatg 780

ctttatttcg ttacttcatt ttattgtcta tgcacataag ctgttcgaca aaaagcctga 840

atgaaactgt gagtgctggt acgcggttac aatactcctg attatgtcca atgattattt 900

gggtacttta ataccctaat ttgtgaagtt cactagcgaa agggtagttt cttccccggt 960

tatttatcat tttatgaatc cccaagtctt tgaagtacag ctgattaaaa tgcaagtact 1020

cataagttgt cattgtatgt ttgaaatcct tacgttgttg gtattttaca gaatttgtac 1080

tgaagaagca taaattcata cctagttagg cttccttgtt cctaatatgc ttctcctcaa 1140

gcggctggtg tccactaaac aagggttcaa taatgccatc aaccgttttg gaggtaatat 1200

agttatgcct atgatgcttt aagtcgcttt tcatcgattt gttacattac caggctaaga 1260

ttctgcaatt gaagttagaa atcgacgagt cttatcttga aaagtatgga ttttgttttg 1320

tcaccctggg gccagttcaa gattgctttc ccaggttgct gtctcggcac cacatcctaa 1380

tccaaaaccc taaggtgatg ggttaatggg tcccttcgca gttatatttc attcacttaa 1440

ctcattagct tccatgtgga taatcaactc cacacttgcc ccaaaactgt ataaacgtag 1500

attttgacct ttggaaatca ttagctggac aaatcctaac taattagtat caagagccaa 1560

aatcttttga ccaagaccaa atatgttgtt tcattgttgc agcattgtca tcatcctttc 1620

tccataccga accaaatagt tcggtggaag ttcattctca agagcaagaa gtagtaattg 1680

cattaggaag caatgtggga gatagagttc ataatttcaa ccaagcattg cgattaatga 1740

aaagttccgg cattaacgta actagacatg gttgtttgta ccagacagca cctgcttatg 1800

tcactgacca acctcacttt ctcaactcag cagttcgagc tttcacgaaa ctcggacccc 1860

acgagttatt aggagtttta aagcagatcg aaaaggactt gggccgtacc aaagggatta 1920

ggtacggacc aaggccaatc gatttggata tacttttcta tggaaagttc cggattaatt 1980

ccgatacact tattgttcct catgagagaa tatgggagag accctttgtg atggccccat 2040

tggtggattt actcggctcg gaaatcgaga atgacacggt cgccagctgg cattccttgt 2100

ccggcggtct ttttgaatca tgggaaaagt tgggcggcga aagcctgatc gggaaggacg 2160

gaatgaaaag agttaccccg atcggaaacc atttatggga ttggtcggaa aagacttctg 2220

taatgggaat tatcaattta acccccgata gtttcagcga tgggggcaag ttgacaacta 2280

ttgattctat agtttctaag gttcgctcga tgatttccga aggggcggat attgtcgatt 2340

ttggtgctca atcgacacgc cctatggcta aaaggatatc cccgcaagag gaaatggata 2400

ggctaatccc tgtattggaa gcggttgtga aaatacctga gatgaccgga aagctcatat 2460

cggtcgacac attttactcg gaagtcgcct tagaagcggt ccaaagagga gcaaatctcg 2520

taaatgacgt atctgggggg caattagatc caaaaatgac ggaaaatgtc gctaatctcg 2580

aggtaccata tatcttaatg cacatgaggg gggaccccac cacaatgcag aacactcaga 2640

atctaaaata cgataatgtt tgtaaagagg ttgcctttga gttgttttcg agggttaaag 2700

aagccgagat gtccggaatt ccagcctggc gaatgattat tgaccctgga attgggtttt 2760

cgaagaacac gaatcagaat ctggaaatcc tgatgggaat tccaaggatc cgggccgaga 2820

ttgggaggga aagcgtgggt atttctcgtg ggccgattct tattgggcct tcgagaaaga 2880

ggtttttggg cgaaatttgc gaacgcccta aagcaactga aagagacccg gcaactgttg 2940

cttctgttac tactgggatt ctgggagggg caaatattgt tagagttcat aatgttaggg 3000

ataatgtgga tgctgtgaag ctatgcgact cgatgatgaa actaaagagg tctacttaaa 3060

aagtatcggg tatagttcga tttctttctt tccgaatctt ttgtttgact ttcagattgt 3120

ctttgattgg caaaacagtt tcggctgcgg ttactgttgt tgtttgttgt tgcagtttag 3180

tgttgttgtt tctaattgtg attttaagtt attgttgtta gctgatttgt acttattgtt 3240

tggtgaaaat ataattcacg gttgcggttg gaggtataaa ttactaaaat agacatattt 3300

taaataaatt ataagttata ttgaagggta tttaggataa ataaaaatcc aaaccacaaa 3360

aaaaaacccg taaaactatt gatttggtga ggtttggaaa aggagataaa tcccttataa 3420

aaaaaacaac ttcatatatg ttttgaaact taaccaaaca cttgtatacc tccttttcct 3480

tttcaaacct caaaagacct ttttatagta agctcttttg aagtctatac gctattgttt 3540

ccgaatcatt ttggactttt catcgaaatc aacaatactg ctagctagtc atatttcaag 3600

atatttggga tgtaaagatc aaggtagttg ttgttgtatt ctgttctcat cagcaaaatt 3660

tgttataact ccaccaagat ttaatttttt ccaatctgaa aatgtcttat gttatagtaa 3720

gaagtgagat gagtttatgt ctttgtgaaa agattttgcc caattaatgc aaaaattttg 3780

tgacaagaca agaggggtga aagagacaag caacaagaga aagcagtgaa aggggcaaat 3840

gttaggaaac tgttagctag aaaagggtag agggtggcct actttaccaa tttgagcatt 3900

aaaaaagcac atggctatgt attttagacc aaagtcccta ttgttggcag atgaactaag 3960

caaccatatg gttcattaac taactaacta gttattgcaa atttgactgt tccaattcat 4020

caaaggactc tccccataga ccattcaaca cttgggttct agccctccct ctcaccctca 4080

gacaaccctt gtgaacctca ttttcatcat tggtcccctt ttttgttagg actacacttg 4140

tctctataaa atctcttcta gccatgtgtt atgtgtagac tttttcggtt tgaggattgg 4200

gtcgggttcg ggctgggtct cgtcggactt tttactgcaa acatcaaact agggttctaa 4260

acccatactt cagtactctg gttcggattg aacttgcgag atcattctaa attcaattta 4320

cggctcatag attatatttg agagttttga gttttgggtg gattagattg aaaacgacta 4380

ttttttgttg tataaatata tctaaaaagt tatataatta cattctttat tggaattcat 4440

caatatatat aagaatttga ctttgttgta gccattttga aagttcgata agctactccc 4500

ttcgtctagt ttcaatagtt catttaaaag tttcattaat tctgttttca attgtc 4556

<210> 36
<211> 1425
<212> DNA
<213> Euphorbia heterophylla

<400> 36

gcagcattgt catcatcctt tctccatacc gaaccaaata gttcggtgga agttcattct 60

caagagcaag aagtagtaat tgcattagga agcaatgtgg gagatagagt tcataatttc 120

aaccaagcat tgcgattaat gaaaagttcc ggcattaacg taactagaca tggttgtttg 180

taccagacag cacctgctta tgtcactgac caacctcact ttctcaactc agcagttcga 240

gctttcacga aactcggacc ccacgagtta ttaggagttt taaagcagat cgaaaaggac 300

ttgggccgta ccaaagggat taggtacgga ccaaggccaa tcgatttgga tatacttttc 360

tatggaaagt tccggattaa ttccgataca cttattgttc ctcatgagag aatatgggag 420

agaccctttg tgatggcccc attggtggat ttactcggct cggaaatcga gaatgacacg 480

gtcgccagct ggcattcctt gtccggcggt ctttttgaat catgggaaaa gttgggcggc 540

gaaagcctga tcgggaagga cggaatgaaa agagttaccc cgatcggaaa ccatttatgg 600

gattggtcgg aaaagacttc tgtaatggga attatcaatt taacccccga tagtttcagc 660

gatgggggca agttgacaac tattgattct atagtttcta aggttcgctc gatgatttcc 720

gaaggggcgg atattgtcga ttttggtgct caatcgacac gccctatggc taaaaggata 780

tccccgcaag aggaaatgga taggctaatc cctgtattgg aagcggttgt gaaaatacct 840

gagatgaccg gaaagctcat atcggtcgac acattttact cggaagtcgc cttagaagcg 900

gtccaaagag gagcaaatct cgtaaatgac gtatctgggg ggcaattaga tccaaaaatg 960

acggaaaatg tcgctaatct cgaggtacca tatatcttaa tgcacatgag gggggacccc 1020

accacaatgc agaacactca gaatctaaaa tacgataatg tttgtaaaga ggttgccttt 1080

gagttgtttt cgagggttaa agaagccgag atgtccggaa ttccagcctg gcgaatgatt 1140

attgaccctg gaattgggtt ttcgaagaac acgaatcaga atctggaaat cctgatggga 1200

attccaagga tccgggccga gattgggagg gaaagcgtgg gtatttctcg tgggccgatt 1260

cttattgggc cttcgagaaa gaggtttttg ggcgaaattt gcgaacgccc taaagcaact 1320

gaaagagacc cggcaactgt tgcttctgtt actactggga ttctgggagg ggcaaatatt 1380

gttagagttc ataatgttag ggataatgtg gatgctgtga agcta 1425

<210> 37
<211> 1425
<212> DNA
<213> Euphorbia heterophylla

<400> 37

gcagcattgt catcatcctt tctccatacc gaaccaaata gttcggtgga agttcattct 60

caagagcaag aagtagtaat tgcattagga agcaatgtgg gagatagagt tcataatttc 120

aaccaagcat tgcaattaat gaaaagttcc ggcattaaca taactagaca tggttgtttg 180

taccagacag cacctgctta tgtcactgac caacctcact ttctcaactc agcagttcga 240

gctttcacga aactcggacc ccacgagtta ttaggagttt taaagcagat cgaaaaggac 300

ttgggccgta ccaaagggat taggtacgga ccaaggccaa tcgatttgga tatacttttc 360

tatggaaagt tccggattaa ttccgataca cttattgttc ctcatgagag aatatgggag 420

agaccctttg tgatggcccc attggtggat ttactcggct cggaaatcga gaatgacacg 480

gtcgccagct ggcattcctt gtccggcggt ctttttgaat catgggaaaa gttgggcggc 540

gaaagcctga tcgggaagga cggaatgaaa agagttaccc cgatcggaaa ccatttatgg 600

gattggtcgg aaaagacttc tgtaatggga attatcaatt taacccccga tagtttcagc 660

gatgggggca agttgacaac tattgattct atagtttcta aggttcgctc gatgatttcc 720

gaaggggcgg atattgtcga ttttggtgct caatcgacac gccctatggc taaaaggata 780

tccccgcaag aggaaatgga taggctaatc cctgtattgg aagcggttgt gaaaataccc 840

gagatgaccg aaaagctcat atcggtcgac acatttactc cggaagtcgc cttagaagcg 900

gtccaaagag gagcaaatct cgtaaatgac gtatctgggg ggcaattaga tccaaaaatg 960

acggaaaatg tcgctaatct cgaggtacca tatatcttaa tgcacatgag gggggacccc 1020

accacaatgc agaacactca gaatctaaaa tacgataatg tttgtaaaga ggttgccttt 1080

gagttgtttt cgagggttaa agaagccgag atgtccggaa ttccagcctg gcgaatgttt 1140

attgaccctg gaattgggtt ttcgaagaac acgaatcaga atctggaaat cctgatggga 1200

attccaagga tccgggccga gattgggagg aaaagcgtgg gtatttctcg tgggccgatt 1260

cttattgggc cttcgagaaa gaggtttttg ggcgaaattt gcgaacgccc tgaagcaact 1320

gaaagagacc cggcaactgt tgcttctgtt actactggga ttctgggagg ggcaaatatt 1380

gttagagttc ataatgttag ggataatgtg gatgctgtga agcta 1425

<210> 38
<211> 681
<212> DNA
<213> Euphorbia heterophylla

<400> 38

ttaaagcaga tcgaaaagga cttgggccgt accaaaggga ttaggtacgg accaaggcca 60

atcgatttgg atatactttt tctatggaaa gttccggatt aattccgata cacttattgt 120

tcctcatgag agaatatggg agagaccctt tgtgatggcc ccattggtgg atttactcgg 180

ctcggaaatc gagaatgaca cggtcgccag ctggcattcc ttgtccggcg gtctttttga 240

atcatgggaa aagttgggcg gcgaaagcct gatcgggaag gacggaatga aaagagttac 300

cccgatcgga aaccatttat gggattggtc ggaaaagact tctgtaatgg gaattatcaa 360

tttaaccccc gatagtttca gcgatggggg caagttgaca actattgatt ctatagtttc 420

taaggttcgc tcgatgattt ccgaaggggc ggatattgtc gattttggtg ctcaatcgac 480

acgccctatg gctaaaagga tatccccgca agaggaaatg gataggctaa tccctgtatt 540

ggaagcggtt gtgaaaatac ccgagatgac cgaaaagctc atatcggtcg acacatttac 600

ctcggaagtc gccttagaag cggtccaaag aggagcgaat ctcgtaaatg acgtatctgg 660

ggggcaatta gatccaaaaa t 681

<210>	39
<211>	444
<212>	DNA
<213>	Euphorbia heterophylla

<400>	39

tctggggagt gatggttgga atagatgctt attcctggca gagatcgaaa tgctccattt	60

tagtttcggg ttttgttaaa atggggattt gttaaattgt ttcaatggat aattcactgt	120

tgtattagac ttgatattgt tagcagacac tgatttttat gtattcgtga actgaaaagg	180

ttgattttct tccatgtttt gtctgattta tgatggaact tcatttctta gtggttaatg	240

ttggtatttc cttctgttgt ttgatttgta tctgtaattg gtaccatgtt atatgcaatc	300

ttttaaaaaa tgttttaaaa agcgctatac gtggcagctg agactgttga gacttgaaaa	360

cgcacgggca tgtgcggggt ttaggcggat ttgtattttt tattttttaa aatatttttt	420

aatatataaa aatataaata taaa	444

<210>	40
<211>	379
<212>	DNA
<213>	Euphorbia heterophylla

<400>	40

gttatgatga aagtataagt tgctaatcaa gaagaaagag attttaatgt aattatttaa	60

ataataaatt cctaccattt taattgtgtg acaattggtt ataatgattc cgttgcatcg	120

acaactaaaa ctagatagaa ggatcaaaat ataaaatttt aacataatta atacatataa	180

tatataaatg tataatagat acaattatta ttcctcgaat taaaattggc taattatata	240

ataatataat ccaacctata aaaacttttg agcttttaag cttgagatga gctttgtaaa	300

aatgattgta caagcccgac catgaaagag agccaagtag ggtcaaatgc ataccctggc	360

ttgaatataa attcaatat	379

<210>	41
<211>	4031
<212>	DNA
<213>	Digitaria sanguinalis

<400>	41

aaatcgtaaa atttataact aacatgttcc ttcatgaatc caaaagccaa gtaaacaggt	60

ccatgagtgc ctttgtgaga ttgcttcgaa tagaatatac ctacggctct taatttcaga	120

ttctcttagg tagtgtttag gtagcgtttg gaagtagata tttaggcttg gaattaggaa	180

ttggcattga gatctacgaa tacctgccgt ttggatgtct aagaatttgg aattggaatt	240

gcagcccaat accaaccggc attccttggc ctctcatctg cctgccctca gtatagatag 300

agcttatctc cttcatcctc gcggagtggt ggcaagcggc ggcgagatcc ggaccacatg 360

gggtgtggga gcgcggtggc agagtggtgg caagcgatgg cgacggcacc gtgccgccgt 420

gccacattgg cacggcgacg gacgagtttg gatggtgtca gtacagacca ctgcccaatt 480

ctatcctttt gtgcatccaa acataaaatc tgaattagga tgctcttttg attctacaag 540

gcaatgtaat gaccatccaa acaacagatt ctgaattgga gtcaattcaa tgttacgatt 600

gtattggcat cggacctaat tcaatgtcat gccctctagt atctacattc aaacggagcg 660

gaaaaatcta cctccgtccc aagattccgg cccaagatcc cgactcttaa tacttttttc 720

atctttcgct gaaatttcag cccaagatcc cggcccgcct ttttgcctcc acgctgggcc 780

tggtgggacg cggccccttc acgagagtcg agaggggaaa gcggaaaggg aaacaaaaaa 840

actcaacgaa tcgccgccgc cgccttgccc tgcagcgaac aattaagggc cccgtacctc 900

tctctgcccg ttcttccgct gcactccgca tctgagcgga cgggtgggga caccgccgac 960

ggcgccgggg tggcgcctcc gtctgtgctc caaccaaggc agtgggtgct ggttgctgcc 1020

aatcgaccgg ccgggcatgc tctgaccatc gagaaaatct tcgggtaaca caacgttgtt 1080

catttcgttt gtatcgtggc atagttgatt tccctaactt ttaggatgcg tttcgattca 1140

ttgtatctgt ataccaaaac agagttcgat aacttagatt gaacggtagg cttcagcatc 1200

taaggcagca ggtgcaggtc tattctatca ctctgaaaat tagttttaac tgtctgatga 1260

taggaggatt agctaatgtt gttatatgag gaggtttctt gctgactaga gagctatttg 1320

accacctgat agattgcctc gttagcaact tagcattatg acgacgatgg cagcgagcca 1380

tttcatgtag aaaacgaaca tgagctgaga agcttaatcg tataagcttc aattggcttc 1440

ctggctgggg aggtttgcta gagaacttct gtattttgtt cattcattta atctgatgta 1500

tgttggcagc tctcctgtca ggtacagaaa tttagacggc cctgaaacca agcagggcaa 1560

cctcaccaat aggggggcag tcgcaccaca aaggttgctt gcctccagtg gggcatctcc 1620

tctccttaca ttgagaggtt tatttgacgt ctaagatatc tgaactcctg cctccagtta 1680

gagaaaaggc ccttgttcat acctcaaatt tgtctttgct cttctattga ccaggtgtga 1740

tggagtgatg cttgcgtgaa atatcctaca aggctacaat ttatttgaag catttgcttt 1800

cttaattatc atatgttcaa cgatgctcct ccatgctaag gagtccctta agaagatgta 1860

tcctgttgct atgaactact ttggagggct cctcccatcg cattcgtttt caggtactgt 1920

tcaaatcttt tcttttcaaa tcccctatca tgcgatcaag agcagctcta agcttctaac 1980

tcaccccttt gcatttctaa acagtgtctg atcttgttca tccgctgagg ttcccaaaca 2040

gagctcctag acatgctatt ccattcaggg ctcgttcgtt tacgcgatgt tcgcttgagg 2100

gatgttcaac tgaccaagag attgtgattg ctatggaaag caacgtgggt gatggagtca 2160

gtacatttga cagggcatta caaatgatga aaagctcagg cgtgaacatc actaggcatg 2220
cctgtctata tgagactgcc cctgcttatg tgaccgatca gccacggttc ctgaactctg 2280
ccattcgagg tacgactagg ctgggacctc acgagttgct taagaaacta aaggaaattg 2340
agaaggatat aggacgcact ggtggaataa ggtatggccc aagaccaatc gatctagata 2400
tacttctgta cggcaattcc cagattgata ctgaggctct aattgtgcca catgaacgca 2460
tccaggagag gccatttgtt ctagcacctc ttgttgacct tctaggtgca tctggcaatg 2520
atagtgtcga aacaagctgg cactctcttt caaagtgcag tggtggtttc tttgaattat 2580
ggaataaact tgggggtgaa tctataattg gaacagaaag tattaaaagg gtattacctg 2640
ttgggaatca tttgtttgat tggtgtgaga gaaccctcgt catgggggtc cttaatctaa 2700
caccagacag ctttagtgat ggaggtaagt ttcaacaagt ggaagctgct atttctcagg 2760
ctaaattatt aatttcagaa ggtgcagata tcatagatat tggtgctcaa tctaccaggc 2820
cttttgcaaa gagattatct ccaaacgaag aaattgaaag attggttcct gttctggatg 2880
tgattacgaa aattcctgaa atggagggaa agttgctctc agtggataca ttctatgcag 2940
aagttgcatg tgaagctgtg aaaagaggag ttcacatgat caatgatgta tctggtggac 3000
agcttgaccc caaaattctt aaagttgctg ctgaactcaa agttccgtat gttgcaatgc 3060
acatgagggg agatccatca acaatgcaaa gtgaacaaaa tttacagtat gacgatgtct 3120
gcaaggaagt tgcttcagag ctatatgcac aggtgagaga agcagagtta tctgggattc 3180
cattgtggag gatagttcta gatccaggca ttgggttctc caagaaatcc aaacataacc 3240
ttgaagtaat tatgggattg gaatccatta ggagggagat gagtaaaatg agtattggtg 3300
cttcacatgt gccaatatta ctgggaccct ctaggaaaag atttttgggt gaaatatgca 3360
atcgtgaaaa tccagttgag agagatgttg ctactgttgc agctgtgacg gcgggtattt 3420
tgaatggtgc taacatagta agggtacata atgcaggata cagttcagat gctgcaaagt 3480
tttgtgatgc attgaataag agaagaagaa tggaagactg aaccagctga tcaaaaagat 3540
accgagctct gattttattc gagaaaatgg tgatgcagga tagttactct gctgctcaat 3600
gggattctca tattacatca tttgtggagt attgtttttg taataaataa accagggatg 3660
acgtttttcc tgtgtcatct cctatctagt ttctagatac ttcaatgaag catggttcaa 3720
ctcagatcac ttggaaagag tagtcaagga ataaggttac agaatcagtt agtaattaca 3780
gattcttacc tcttgtggtc ctattttaga tgattttcca gaacattttc tttccaattt 3840
aataatatca gaaggcccta atttgtgtgt atcttttcta gtccagacat atttgataag 3900
ttggggtttt acctgcttct ccctttacct ttgtattttg cactttgttt gagctgtgca 3960
tcattgtatt agaggaagtg tattttgtac gttggtatac ccaacgagga aacctcatgt 4020

tttgcaagtg c                                                          4031

<210>    42
<211>    1428
<212>    DNA
<213>    Digitaria sanguinalis

<400>    42

gctcgttcgt ttacgcgatg ttcgcttgag ggatgttcaa ctgaccaaga gattgtgatt    60

gctatgggaa gcaacgtggg tgatggagtc agtacatttg acagggcatt acaaatgatg   120

aaaagctcag gcgtgaacat cactaggcat gcctgtctat atgagactgc ccctgcttat   180

gtgaccgatc agccacggtt cctgaactct gccattcgag gtacgactag gctgggacct   240

cacgagttgc ttaagaaact aaaggaaatt gagaaggata taggacgcac tggtggaata   300

aggtatggcc caagaccaat cgatctagat atacttctgt acggcaattc ccagattgat   360

actgaggctc taattgtgcc acatgaacgc atccaggaga ggccatttgt tctagcacct   420

cttgttgacc ttctaggtgc atctggcaat gatagtgtcg aaacaagctg gcactctctt   480

tcaaagtgca gtggtggttt ctttgaatta tggaataaac ttgggggtga atctataatt   540

ggaacagaaa gtattaaaag ggtattacct gttgggaatc atttgtttga ttggtgtgag   600

agaaccctcg tcatgggggt ccttaatcta acaccagaca gctttagtga tggaggtaag   660

tttcaacaag tggaagctgc tatttctcag gctaaattat taatttcaga aggtgcagat   720

atcatagata ttggtgctca atctaccagg ccttttgcaa agagattatc tccaaacgaa   780

gaaattgaaa gattggttcc tgttctggat gtgattacga aaattcctga aatggaggga   840

aagttgctct cagtggatac attctatgca gaagttgcat gtgaagctgt gaaaagagga   900

gttcacatga tcaatgatgt atctggtgga cagcttgacc ccaaaattct taaagttgct   960

gctgaactca aagttccgta tgttgcaatg cacatgaggg gagatccatc aacaatgcaa  1020

agtgaacaaa atttacagta tgacgatgtc tgcaaggaag ttgcttcaga gctatatgca  1080

caggtgagag aagcagagtt atctgggatt ccattgtgga ggatagttct agatccaggc  1140

attgggttct ccaagaaatc caaacataac cttgaagtaa ttatgggatt ggaatccatt  1200

aggagggaga tgagtaaaat gagtattggt gcttcacatg tgccaatatt actgggaccc  1260

tctaggaaaa gatttttggg tgaaatatgc aatcgtgaaa atccagttga gagagatgtt  1320

gctactgttg cagctgtgac ggcgggtatt ttgaatggtg ctaacatagt aagggtacat  1380

aatgcaggat acagttcaga tgctgcaaag ttttgtgatg cattgaat              1428

<210>    43
<211>    4007
<212>    DNA
<213>    Kochia scoparia

<400>      43

gaatgtgctt aggcggctgc aatcagccag tttgatttgg aagggaattt ccaagtatga    60

tagaggtaaa gcacagttgt ttcatttttt gactatatat ttaatttggg aatctgtact   120

gtttcatttt gtaactttat tttgatcctg agttattgaa tctgtcatgt ctcattttga   180

gacggtattt tgaatctgag tctggatatg aaattatgaa taggccatag gtatttataa   240

atatagggtt taagttgatg atctggatgc tatcagagct ctattatgca ggattacaaa   300

tcataatttc attgtgaaat ttacttcttt atgtgtattc acttgaagat tcataatttt   360

gatgtattca taaatttgat gtgccctaaa cagaaagagt cagcatgtgc tgaaagtata   420

agggggaaaa ccaagatgta taaatctttt ccttgatgag ttgcttatat gattgatgtc   480

atgattctaa aatgccttta cctaattcaa atggatttta aagattacgt atgtactttt   540

gcagcttcaa gtcttgcttt tttgcactca tcaccagaaa ctactattga ggtttgttct   600

aaagagcatg aagttgtaat tgctctaggg agcaatgtag gagacagatt agataacttt   660

aaccaagctc tgcagctaat gaagaaatta ggagtaaata tcacaaggca tggctgctta   720

tacgagacag atcctgccta tgtgactgat caaccaaagt ttcttaactc tgcagtgagg   780

ggcttcacaa aacttgggcc tctagaacta ttagggatgc tgaagaaaat tgagaaggat   840

atgggtcgaa ctaatggaat aagatatggc cctaggccaa ttgacttaga cattcttttt   900

tatgggaagt ttagggtaag ctctgagaaa cttacagttc cgcatgaaag gatatgggaa   960

agaccctttg tgatggcacc actaattgat ataattggat cggatgtaga aaatgacact  1020

gttgctgcat ggcattcatt atcaaaattt tctggtggac tatttgaagc gtgggataaa  1080

cttggtggag attcactcat tgggaaggac ggaatgagta gggttttgcc agttgggaac  1140

cacttgtggg attggtcgtg caaaacctct gtaatgggag tcttgaattt gactccggac  1200

agctttagtg atggaggaaa gtttctacct gtagaaaatg cagtttctca ggttcgtcaa  1260

atgatctcag aaggtgctga cataattgat attggagcgc aatcaacaag gcccatggca  1320

actaggattt ctgctgagga agagctggaa agactagtcc ctgttttaga aggtgtcaag  1380

gatgttatcg aggaagaagg aagaatgtta tcagtggata cattttactc aaaagttgct  1440

tctgaagctg tcaataaggg agcacatatg gtgaatgatg tttcgagtgg aaagctagat  1500

cctgagatgt tcaatgttgt tgcagggctt aaagtgcctt atatagcaat gcacatgcga  1560

ggtgatcctt cttcaatgca aaatgctgat aatttgacat acaatgatgt ttgtaaggag  1620

gtggctttag agttgagctc taggattaca gatgcagaat tatcaggtat tcctgcttgg  1680

aggatagtta ttgatcctgg cattggattt tccaagaata caaagcaaaa tttggaaatt  1740

ctcacagggt tgaaaagaat tcggcaagaa atagcaaaaa agagtttggc tgtggctcat  1800

ggtcccttac tgattggacc ttcaagaaag aggtttttgg gtgagatctg caatcgtcct 1860
gtagcatctg atagagatcc ggcaactgtt gcttctatca ccgctggagt tttgggtggt 1920
gcaaacattg taagagtaca taatgttcgg gatagccttg atggtgtcaa gctatgtgat 1980
gcaatgcttg aatatcagta taactgaaga ttcttagatt cttagattct tagattctaa 2040
gagttgattt tatagattat tgtctttgtt gtgtgccttc ggcagatatg aataatatga 2100
ttgtaatttt gttgcttatg attttcttcc tacctttctt ttatcataga atttagatta 2160
aaaaatgtac ttgaatttca taaatggtga aataaagttt gtcggtgctt catgcaatac 2220
gacttttcta gtttgcattg ctctatcata ggggctgtaa gcagattagc ccagtcaatt 2280
cacattagat ttagacggcc ggtgcaaaaa cagcataatg ttgctaacgc tgtttataca 2340
tttaaactat atgatctcta agagcaaaat acaaaataat acaatgtgtt tcatgaagat 2400
gtccaatcat ttctccctca aaaaaagatc gtgatgaaca atcaggtact ttgattttgt 2460
tgtagaacct cagcttcagc cctagccatg ggatcatctt tttctccttc tcgctcctca 2520
gccaatttct ttctgtgcat ctcctctgca gcttctattg aagctttgtg ctcagcctgg 2580
agacgctgca tttcctcttc catctgctgt ctttcgaacc atgtatcagc atccgcccaa 2640
actgcagaaa taatttaaac aagttaatca tacggagtgt gcagcatggc aaattggcaa 2700
taagatggtg caagtaacaa gctcaaatga tccaccatag ccaaattcct aaccattgga 2760
ctgtgtcata tactcatatc agctagtaga aacattctca ccataaccct aactattcga 2820
ggataagtga taccattcaa caatagtaaa gatcaaatca ggtattacta gacctatcaa 2880
agtaatacgg agtagttgtt attgttacag agttaggaag aatcctattt tcccatttct 2940
cctatcaatc ctaaactttt gtgactgaac tatcgacata tagtgtgaag aacatgaatg 3000
gcagaagtca caactctgat tcctgagtcc tgactataaa ctattggatg actgtgatac 3060
atcattagtc ctttaagcat tgagttactc tattctacat agttaaatta tatagattcc 3120
aagatccgaa tccatgaatt atcaattacc ataaattgat cttcttcctt tgagttttgt 3180
caacagttcc tactccgaaa tatgtcttaa atctagaagc aatagacact tatcaatgat 3240
acagcaatag acgaattcat ctacccaagg cagcatcaac aacagctgcc gtttgcgata 3300
atcgaaacta tcatagtgtc cttttcacta ttagtgcaga tgttatctag ggtctaaacc 3360
tctatgtttc tacattcctg gaaaatctta ttctgtactg cataatcctc tcacaaaaaa 3420
ttcgccaatg aatgacaaat tagctatcca gaattgcaga ttgattgaat caaattgatc 3480
atggcagcag cacaattgca gcagttatca attgaagcac aatattacct ctcaatcata 3540
acaatcaaag cacagaaatt ccaatcaata actccacaat atagtatcaa tctctaactc 3600
agaaactaaa cagaagcaaa aacccattca atcaaagcaa cagtaaaatt agggtttgaa 3660
ttagaaagaa agagagggaa gagaaacata ctgggttgcg cagcccatcc ctgttcacca 3720

cccttaatct tctcgggaag agcgtaattg acagtgagaa cacgaccgta aagctcagca 3780

ccatccatgt tatccatggc agaagaagcg tcgtctctct ccaagaaagt gacgaaaccg 3840

aaagaacgat gcttgttggt ggccaaatca agaggggttt tgacgtcttt gatttctcca 3900

aaagggatga aggcagcatg aaggattgat tcgttgactt cttctgctaa tcctcctacg 3960

tataatgtgt tcttctgcat tccttgttga ttcgtcgcca tttttcg 4007

<210> 44
<211> 3505
<212> DNA
<213> Kochia scoparia

<400> 44

taattcaaat ggattttaaa gattacgtat gtacttttgc agcttcaagt cttgcttttt 60

tgcactcatc accagaaact actattgagg tttgttctaa agagcatgaa gttgtaattg 120

ctctagggag caatgtagga gacagattag ataactttaa ccaagctctg cagctaatga 180

agaaattagg agtaaatatc acaaggcatg gctgcttata cgagacagat cctgcctatg 240

tgactgatca accaaagttt cttaactctg cagtgagggg cttcacaaaa cttgggcctc 300

tagaactatt agggatgctg aagaaaattg agaaggatat gggtcgaact aatggaataa 360

gatatggccc taggccaatt gacttagaca ttctttttta tgggaagttt agggtaagct 420

ctgagaaact tacagttccg catgaaagga tatgggaaag accctttgtg atggcaccac 480

taattgatat aattggatcg gatgtagaaa atgacactgt tgctgcatgg cattcattat 540

caaaattttc tggtggacta tttgaagcgt gggataaact tggtggagat tcactcattg 600

ggaaggacgg aatgagtagg gttttgccag ttgggaacca cttgtgggat tggtcgtgca 660

aaacctctgt aatgggagtc ttgaatttga ctccggacag ctttagtgat ggaggaaagt 720

ttctacctgt agaaaatgca gtttctcagg ttcgtcaaat gatctcagaa ggtgctgaca 780

taattgatat tggagcgcaa tcaacaaggc ccatggcaac taggatttct gctgaggaag 840

agctggaaag actagtccct gttttagaag gtgtcaagga tgttatcgag gaagaaggaa 900

gaatgttatc agtggataca ttttactcaa aagttgcttc tgaagctgtc aataagggag 960

cacatatggt gaatgatgtt tcgagtggaa agctagatcc tgagatgttc aatgttgttg 1020

cagggcttaa agtgccttat atagcaatgc acatgcgagg tgatccttct tcaatgcaaa 1080

atgctgataa tttgacatac aatgatgttt gtaaggaggt ggctttagag ttgagctcta 1140

ggattacaga tgcagaatta tcaggtattc ctgcttggag gatagttatt gatcctggca 1200

ttggattttc caagaataca aagcaaaatt tggaaattct cacagggttg aaaagaattc 1260

ggcaagaaat agcaaaaaag agtttggctg tggctcatgg tcccttactg attggacctt 1320

caagaaagag gtttttgggt gagatctgca atcgtcctgt agcatctgat agagatccgg 1380
caactgttgc ttctatcacc gctggagttt tgggtggtgc aaacattgta agagtacata 1440
atgttcggga tagccttgat ggtgtcaagc tatgtgatgc aatgcttgaa tatcagtata 1500
actgaagatt cttagattct tagattctta gattctaaga gttgatttta tagattattg 1560
tctttgttgt gtgccttcgg cagatatgaa taatatgatt gtaattttgt tgcttatgat 1620
tttcttccta cctttctttt atcatagaat ttagattaaa aaatgtactt gaatttcata 1680
aatggtgaaa taaagtttgt cggtgcttca tgcaatacga cttttctagt ttgcattgct 1740
ctatcatagg ggctgtaagc agattagccc agtcaattca cattagattt agacggccgg 1800
tgcaaaaaca gcataatgtt gctaacgctg tttatacatt taaactatat gatctctaag 1860
agcaaaatac aaaataatac aatgtgtttc atgaagatgt ccaatcattt ctccctcaaa 1920
aaaagatcgt gatgaacaat caggtacttt gattttgttg tagaacctca gcttcagccc 1980
tagccatggg atcatctttt tctccttctc gctcctcagc caatttcttt ctgtgcatct 2040
cctctgcagc ttctattgaa gctttgtgct cagcctggag acgctgcatt tcctcttcca 2100
tctgctgtct ttcgaaccat gtatcagcat ccgcccaaac tgcagaaata atttaaacaa 2160
gttaatcata cggagtgtgc agcatggcaa attggcaata agatggtgca agtaacaagc 2220
tcaaatgatc caccatagcc aaattcctaa ccattggact gtgtcatata ctcatatcag 2280
ctagtagaaa cattctcacc ataaccctaa ctattcgagg ataagtgata ccattcaaca 2340
atagtaaaga tcaaatcagg tattactaga cctatcaaag taatacggag tagttgttat 2400
tgttacagag ttaggaagaa tcctattttc ccatttctcc tatcaatcct aaacttttgt 2460
gactgaacta tcgacatata gtgtgaagaa catgaatggc agaagtcaca actctgattc 2520
ctgagtcctg actataaact attggatgac tgtgatacat cattagtcct ttaagcattg 2580
agttactcta ttctacatag ttaaattata tagattccaa gatccgaatc catgaattat 2640
caattaccat aaattgatct tcttcctttg agttttgtca acagttccta ctccgaaata 2700
tgtcttaaat ctagaagcaa tagacactta tcaatgatac agcaatagac gaattcatct 2760
acccaaggca gcatcaacaa cagctgccgt ttgcgataat cgaaactatc atagtgtcct 2820
tttcactatt agtgcagatg ttatctaggg tctaaacctc tatgtttcta cattcctgga 2880
aaatcttatt ctgtactgca taatcctctc acaaaaaatt cgccaatgaa tgacaaatta 2940
gctatccaga attgcagatt gattgaatca aattgatcat ggcagcagca caattgcagc 3000
agttatcaat tgaagcacaa tattacctct caatcataac aatcaaagca cagaaattcc 3060
aatcaataac tccacaatat agtatcaatc tctaactcag aaactaaaca gaagcaaaaa 3120
cccattcaat caaagcaaca gtaaaattag ggtttgaatt agaaagaaag agagggaaga 3180
gaaacatact gggttgcgca gcccatccct gttcaccacc cttaatcttc tcgggaagag 3240

cgtaattgac agtgagaaca cgaccgtaaa gctcagcacc atccatgtta tccatggcag 3300

aagaagcgtc gtctctctcc aagaaagtga cgaaaccgaa agaacgatgc ttgttggtgg 3360

ccaaatcaag aggggttttg acgtctttga tttctccaaa agggatgaag gcagcatgaa 3420

ggattgattc gttgacttct tctgctaatc ctcctacgta taatgtgttc ttctgcattc 3480

cttgttgatt cgtcgccatt tttcg 3505

<210> 45
<211> 1446
<212> DNA
<213> Kochia scoparia

<400> 45

gcagcttcaa gtcttgcttt tttgcactca tcaccagaaa ctactattga ggtttgttct 60

aaagagcatg aagttgtaat tgctctaggg agcaatgtag gagacagatt agataacttt 120

aaccaagctc tgcagctaat gaagaaatta ggagtaaata tcacaaggca tggctgctta 180

tacgagacag atcctgccta tgtgactgat caaccaaagt ttcttaactc tgcagtgagg 240

ggcttcacaa aacttgggcc tctagaacta ttagggatgc tgaagaaaat tgagaaggat 300

atgggtcgaa ctaatggaat aagatatggc cctaggccaa ttgacttaga cattcttttt 360

tatgggaagt ttagggtaag ctctgagaaa cttacagttc cgcatgaaag gatatgggaa 420

agaccctttg tgatggcacc actaattgat ataattggat cggatgtaga aaatgacact 480

gttgctgcat ggcattcatt atcaaaattt tctggtggac tatttgaagc gtgggataaa 540

cttggtggag attcactcat tgggaaggac ggaatgagta gggttttgcc agttgggaac 600

cacttgtggg attggtcgtg caaaacctct gtaatgggag tcttgaattt gactccggac 660

agctttagtg atggaggaaa gtttctacct gtagaaaatg cagtttctca ggttcgtcaa 720

atgatctcag aaggtgctga cataattgat attggagcgc aatcaacaag gcccatggca 780

actaggattt ctgctgagga agagctggaa agactagtcc ctgttttaga aggtgtcaag 840

gatgttatcg aggaagaagg aagaatgtta tcagtggata cattttactc aaaagttgct 900

tctgaagctg tcaataaggg agcacatatg gtgaatgatg tttcgagtgg aaagctagat 960

cctgagatgt tcaatgttgt tgcagggctt aaagtgcctt atatagcaat gcacatgcga 1020

ggtgatcctt cttcaatgca aaatgctgat aatttgacat acaatgatgt ttgtaaggag 1080

gtggctttag agttgagctc taggattaca gatgcagaat tatcaggtat tcctgcttgg 1140

aggatagtta ttgatcctgg cattggattt tccaagaata caaagcaaaa tttggaaatt 1200

ctcacagggt tgaaaagaat tcggcaagaa atagcaaaaa agagtttggc tgtggctcat 1260

ggtcccttac tgattggacc ttcaagaaag aggtttttgg gtgagatctg caatcgtcct 1320

gtagcatctg atagagatcc ggcaactgtt gcttctatca ccgctggagt tttgggtggt 1380

gcaaacattg taagagtaca taatgttcgg gatagccttg atggtgtcaa gctatgtgat 1440

gcaatg 1446

<210> 46
<211> 1446
<212> DNA
<213> Kochia scoparia

<400> 46

gcagcttcaa gtcttgcttt tttgcactca tcaccagaaa ctactattga ggtttgttct 60

aaagagcatg aagttgtaat tgctctaggg agcaatgtag gagacagatt agataacttt 120

aaccaagctc tgcagctaat gaagaaatta ggagtaaata tcacaaggca tggctgctta 180

tacgagacag atcctgccta tgtgactgat caaccaaagt ttcttaactc tgcagtgagg 240

ggcttcacaa aacttgggcc tctagaacta ttagggatgc tgaagaaaat tgagaaggat 300

atgggtcgaa ctaatggaat aagatatggc cctaggccaa ttgacttaga cattcttttt 360

tatgggaagt ttagggtaag ctctgagaaa cttacagttc cgcatgaaag gatatgggaa 420

agaccctttg tgatggcacc actaattgat ataattggat cggatgtaga aaatgacact 480

gttgctgcat ggcattcatt atcaaaattt tctggtggac tatttgaagc gtgggataaa 540

cttggtggag attcactcat tgggaaggac ggaatgagta gggttttgcc agttgggaac 600

cacttgtggg attggtcgtg caaaacctct gtaatgggag tcttgaattt gactccggac 660

agctttagtg atggaggaaa gtttctacct gtagaaaatg cagtttctca ggttcgtcaa 720

atgatctcag aaggtgctga cataattgat attggagcgc aatcaacaag gcccatggca 780

actaggattt ctgctgagga agagctggaa agactagtcc ctgttttaga aggtgtcaag 840

gatgttatcg aggaagaagg aagaatgtta tcagtggata cattttactc aaaagttgct 900

tctgaagctg tcaataaggg agcacatatg gtgaatgatg tttcgagtgg aaagctagat 960

cctgagatgt tcaatgttgt tgcagggctt aaagtgcctt atatagcaat gcacatgcga 1020

ggtgatcctt cttcaatgca aaatgctgat aatttgacat acaatgatgt ttgtaaggag 1080

gtggctttag agttgagctc taggattaca gatgcagaat tatcaggtat tcctgcttgg 1140

aggatagtta ttgatcctgg cattggattt tccaagaata caaagcaaaa tttggaaatt 1200

ctcacagggt tgaaaagaat tcggcaagaa atagcaaaaa agagtttggc tgtggctcat 1260

ggtcccttac tgattggacc ttcaagaaag aggtttttgg gtgagatctg caatcgtcct 1320

gtagcatctg atagagatcc ggcaactgtt gcttctatca ccgctggagt tttgggtggt 1380

gcaaacattg taagagtaca taatgttcgg gatagccttg atggtgtcaa gctatgtgat 1440

gcaatg 1446

<210>	47
<211>	3920
<212>	DNA
<213>	Lolium multiflorum

<400>	47

acgaaagctt tgaactctca agtggtgcaa catagataga ttatatatat ttcgaatata 60

attcgaatga acataaaaat tacatataaa aactttaaaa caaacttaaa ctacttcttc 120

ttcctcgatg gccccgcctc atcgtcgggg cggcgacgct tccggctcgt cacctcctcg 180

tcggaggaag ttgggtcctc ctcctcgtca gtgtcctcag cctcttcgtc gtcctcctcc 240

tcttcctcgg cctcttcgtc ggcctgctcc ttggcctctt cgtcctcctc ctcgtcgtca 300

tcccatgcgt cctcctcctc gtcgtcatcc cattcgtcct tgctggccgg cgggggggaa 360

tcgtcgctgc tggacgatgc agctttatcc caccaatggc gccatccagg cggcttcccc 420

tcgctgtcgg tgtccgatgg ccaagagaga tcgctcatgg ttgacggagg atggtgagga 480

gagaacagat gaatggcgtc ggccgtcgta aaccgtatat gtaggtctct cgacgaagag 540

agcggcggtt gctcttccgc ggagttcgtg ctccattaca gcggttctcg catcgaggcc 600

acttcggccg ttcccgacga gtcgtttggg ctctccagac cactttgcga cggttcaatg 660

cgtcgagggc actccgacga ttgcccttcc cggtgactgc gccgtcgcta tgcacgcggt 720

gtgtgcgcaa ccatgggctc gcggctggga aaatgggcct ccccaggcca caaaatacat 780

ccatccggcg ataactaacg ccggatttcg gcctgggagc cccaacggct gggatgctct 840

tagcccgtgg aacacattcc cgaatcggta ccgacggtct catctctctc gccctgatct 900

atctctcttc cgcgatctgg aagggccgac ggcgtggcgg cgcaaccacg ggcgctgatg 960

ggcggcggcg cgcagtctgt taagcaggac gcctgcttct cgctccgtcc tccctctcca 1020

accgagcggc acgaggtaga cccggagacc ccaactccgt cctagcccag tggcgcccgt 1080

gaggcgtcat ctccggcgcc ctctccatct ccgtctgctt cgcacttgta tgcacatact 1140

cttcctcctt accccagttt cttggtcttg gcttgtttga ttacgatttc tagggttctt 1200

ggcatgctcg gtagttgcag atttgtcttc tgttgggtgt ttgtttccat ggccttggct 1260

gcatccatcg tttcgtatta tgtagttgtt tgtttccatg ccattgcttg tatccatcaa 1320

ttggaaaggg aagaaagggc tacagggata ttaggaatta gatcagtcct tcctagctga 1380

ttttctctga caggttgtta gtctcgcgca tgtggtggac ttcttgtgct tttggctagc 1440

tccaagtctg atgtgtagct gctttctgag attttccaca taaacgttgc tatggctagt 1500

aagttgtggc taactaaatc cttgggcaca agctagtaat ctgaatggca tatccgcccc 1560

catgccttta attgttgcct aacgatgggc taggttctct tatttattca tttttgttaa 1620

aagaagaact tgggcatgct aattcagcaa aatagaaaaa atatgtgttg tttctgtata 1680

tagaggttct tggttttccc ttgccgattc tgaccgattt gcgggttttt tttttttttg 1740

tctttcctca tcgtcaggca agaagagcaa gatgtgacag aggatttaag cagcagcaga 1800

actagacctg gctcctatgc tctaagatgc ttcgtgccaa ggacacattc aggaagatgt 1860

actcagctgc caagggctac tactgcggag ggctagcgtc tcctaatgcc ttctcaggta 1920

gcttgcccaa agttccccaa taataactag ggaacatctc ttttccacct ctttcacatt 1980

ccaaccaaag acagtcctaa ctcgtttccc ctctgaacat accagcattg gatcttcggc 2040

atcctctcag ctcctcaaga aaaccaccag gccacgctaa cctgttcagg gtccgttcat 2100

acacgcaatg cgccctcacc aacgattccg ccgaccagga ggtcgtgatt gccctgggca 2160

gcaacgtggg agacagggtc agcatgttcg acagggcgct gcggctgatg aggagctcgg 2220

gcatcaaggt caccaggcac gcctgcctgt acgagaccgc cccagcctac gtgaccgacc 2280

agccgcggtt cctcaactct gccgttaggg gcacgacgaa gctggggcct cacgagttgc 2340

tcaggaagct caaggagatc gagaaggata taggacgtac cgccggggta aggtacggcc 2400

cgaggccgat cgatctggac attcttctgt atggggactc ccggatcaag accgagtctc 2460

tgattgtgcc gcatgaacgc atccatgaga gaccgttcgt cttggcgcct cttgttgacc 2520

tcctgggttc gtcggctgag gacggtatgg agaaaagctg gcactctctc tcgaagtgca 2580

gtggcgggtt ctttgatttg tggaacaagc tcggcggtga atctgttgtt ggaacagaag 2640

gcattaaaag ggtcatgtct gttggaaata cgctgttgga ctggcgtcag aggacccttg 2700

ttatgggggt gcttaacctc acgccagaca gcttcagcga cggaggtaag tttcaagaag 2760

tggaagctgc catttcccag gccaggttgt tgatatcaga aggtgctgac ataattgata 2820

ttggtgccca gtccaccagg ccctttgcaa ggaggctatc cgcagaagaa gagcttgaga 2880

ggctggtccc tgttctggat gctatcatga aactcccaga gatggaaggg aagttgcttt 2940

cggtagacac gttctacgcg caagtcgctg ctgaagctgt aaaaagagga gccaccatga 3000

tcaatgatgt atctggtggg cagcttgacc caagtattct tcaagttgtt gctgaactgg 3060

gagttccgta tgttaccatg cacatgagag gcgatccatc aactatgcag aatgaacaga 3120

atctacagta tgatgatgtc tgcaaagaag ttgcttctga gttatacgcc cggttgagag 3180

cagcagagct ttctggaatt cctttgtgga ggattattct tgaccctggc attgggtttt 3240

ctaagaaatc cacacagaat attgaagtaa tcgcgggttt ggaatccatt agagaagaga 3300

tgggtaaaat gagcctaggt gcttcacatg tgccaatatt acttggaccc tcgaggaaaa 3360

gtttcctagg tgaaatatgt agtcgcgccg atccagttca gagagatgct gctactgctt 3420

ctgccgttac aattgcgatc ttgaatggcg ctaatatagt aagggtccat aatgttagat 3480

acaatgtgga tgctgcaaag gtctctgatg cattactcaa gtacagaaga aaataataga 3540

aagtatacag cccggataga agccatacca gttgccagtt ttgtgcaagg gaatgctgat 3600

gtggagctca aaccatatgg ggtgtatcat cacttaataa gatcaaagca tgcgtgaata 3660

actattacag tgaacaagag atatacagtt ttttcgtgcg cctcctcctt cgtagaatct 3720

tcaaagaagc aaaggtaaca cagacctagg cctctggggt tggtatatga tgtaaccagt 3780

tttgtgaggg tttaataatt atctaatagt gagacctaag agtaagacct ttataatata 3840

ctttgcttgg aatttcctac ttgagagcat catttggggc tacagagttg attttcgtct 3900

cacgcagttt tctgagggtt 3920

<210> 48
<211> 1467
<212> DNA
<213> Lolium multiflorum

<400> 48

agctcctcaa gaaaaccacc aggccacgct aacctgttca gggtccgttc atacacgcaa 60

tgcgccctca ccaacgattc cgccgaccag gaggtcgtga ttgccctggg cagcaacgtg 120

ggagacaggg tcagcatgtt cgacagggcg ctgcggctga tgaggagctc gggcatcaag 180

gtcaccaggc acgcctgcct gtacgagacc gccccagcct acgtgaccga ccagccgcgg 240

ttcctcaact ctgccgttag gggcacgacg aagctggggc ctcacgagtt gctcaggaag 300

ctcaaggaga tcgagaagga tataggacgt accgccgggg taaggtacgg cccgaggccg 360

atcgatctgg acattcttct gtatggggac tcccggatca agaccgagtc tctgattgtg 420

ccgcatgaac gcatccatga gagaccgttc gtcttggcgc ctcttgttga cctcctgggt 480

tcgtcggctg aggacggtat ggagaaaagc tggcactctc tctcgaagtg cagtggcggg 540

ttctttgatt tgtggaacaa gctcggcggt gaatctgttg ttggaacaga aggcattaaa 600

agggtcatgt ctgttggaaa tacgctgttg gactggcgtc agaggaccct tgttatgggg 660

gtgcttaacc tcacgccaga cagcttcagc gacggaggta agtttcaaga agtggaagct 720

gccatttccc aggccaggtt gttgatatca gaaggtgctg acataattga tattggtgcc 780

cagtccacca ggccctttgc aaggaggcta tccgcagaag aagagcttga gaggctggtc 840

cctgttctgg atgctatcat gaaactccca gagatggaag ggaagttgct ttcggtagac 900

acgttctacg cgcaagtcgc tgctgaagct gtaaaaagag gagccaccat gatcaatgat 960

gtatctggtg ggcagcttga cccaagtatt cttcaagttg ttgctgaact gggagttccg 1020

tatgttacca tgcacatgag aggcgatcca tcaactatgc agaatgaaca gaatctacag 1080

tatgatgatg tctgcaaaga agttgcttct gagttatacg cccggttgag agcagcagag 1140

ctttctggaa ttcctttgtg gaggattatt cttgaccctg gcattgggtt ttctaagaaa 1200

tccacacaga atattgaagt aatcgcgggt ttggaatcca ttagagaaga gatgggtaaa 1260

atgagcctag gtgcttcaca tgtgccaata ttacttggac cctcgaggaa aagtttccta 1320

ggtgaaatat gtagtcgcgc cgatccagtt cagagagatg ctgctactgc ttctgccgtt 1380

acaattgcga tcttgaatgg cgctaatata gtaagggtcc ataatgttag atacaatgtg 1440

gatgctgcaa aggtctctga tgcatta 1467

<210> 49
<211> 834
<212> DNA
<213> Lolium multiflorum

<400> 49

agggtcatgt ctgttggaaa tacgctgttg gattggcggg agaggaccct ggttatgggg 60

gtgcttaacc tcacgccaga cagctttagc gatggaggta agtttcaaga ggtggaagct 120

gccatttctc aggccaggct gttaatttca gaaggtgcag gcataattga tattggcgct 180

cagtccacca ggccctttgc aaggtggcta tctgcagaag aagagcttga gaggctggtc 240

cctgttctta atgctatcat gaaactcccc gagatggaag ggaagttgct ttcagtagat 300

acgttctacg cgcaagtcgc ggctgaagcc gtgaaaagag gggccaccat gatcaatgat 360

gtatctggtg ggcagcttga cccagatatt cttcaagttg ttgctgaatt gggagttccg 420

tatgttgcca tgcacatgag aggggatcca tcaactatgc agaatgagca gaatttacag 480

tatgatgatg tctgcaaaga agttgcttct gagttatact ctcggttgag agcagcagag 540

ttgtctggaa ttcctttgtg gaggattatt cttgaccctg gcattgggtt ttccaagaaa 600

tccacacaga atattgaagt aatcgcgggc ttggaatcca ttagagaaga gatgggtaaa 660

atgagtctag gtgcttcaca tgtgccaata ttacttggac cctccaggaa acgtttctta 720

gggcagatat gcaatcgcgc tgatccagtt gagagagatg cagctactgc tgcttctgtt 780

acagttggga tattgaatgg tgctaatatt gtaagggtcc ataatgttag atac 834

<210> 50
<211> 834
<212> DNA
<213> Lolium multiflorum

<400> 50

agggtcatgt ctgttggaaa tacgctgttg gattggcggg agaggaccct ggttatgggg 60

gtgcttaacc tcacgccaga cagctttagc gatggaggta agtttcaaga ggtggaagct 120

gccatttctc aggccaggct gttaatttca gaaggtgcag gcataattga tattggcgct 180

cagtccacca ggccctttgc aaggtggcta tctgcagaag aagagcttga gaggctggtc 240

cctgttctta atgctatcat gaaactcccc gagatggaag ggaagttgct ttcagtagat 300

acgttctacg cgcaagtcgc ggctgaagcc gtgaaaagag gggccaccat gatcaatgat 360

gtatctggtg ggcagcttga cccagatatt cttcaagttg ttgctgaatt gggagttccg 420

tatgttgcca tgcacatgag aggggatcca tcaactatgc agaatgagca gaatttacag 480

tatgatgatg tctgcaaaga agttgcttct gagttatact ctcggttgag agcagcagag 540

ttgtctggaa ttcctttgtg gaggattatt cttgaccctg gcattgggtt ttccaagaaa 600

tccacacaga atattgaagt aatcgcgggc ttggaatcca ttagagaaga gatgggtaaa 660

atgagtctag gtgcttcaca tgtgccaata ttacttggac cctccaggaa acgtttctta 720

gggcagatat gcaatcgcgc tgatccagtt gagagagatg cagctactgc tgcttctgtt 780

acagttggga tattgaatgg tgctaatatt gtaagggtcc ataatgttag atac 834

<210> 51
<211> 212
<212> DNA
<213> Lolium multiflorum

<400> 51

aggatatagg gcgcaccgcc gggataaggt acggtccgag gccgatcgat ctggacattc 60

ttctgtatgg ggattcccgg atcaagaccg agtctctgac tgtgccgcat gaacgcatcc 120

atgagagacc gttcgtcttg gcgcctcttg ttgacctcct gggttcatcg ggtgaggatg 180

gtatggagag aagatggcac tctctctcga ag 212

<210> 52
<211> 210
<212> DNA
<213> Lolium multiflorum

<400> 52

gatatagggc gcaccgccgg gataaggtac ggtccgaggc cgatcgatct ggacattctt 60

ctgtatgggg attcccggat caagaccgag tctctgactg tgccgcatga acgcatccat 120

gagagaccgt tcgtcttggc gcctcttgtt gacctcctgg gttcatcggg tgaggatggt 180

atggagagaa gatggcactc tctctcgaag 210

<210> 53
<211> 201
<212> DNA
<213> Lolium multiflorum

<400> 53

cgctaacctg ttcagggtcc gttcgtacat gcaatgcgcg gtcaccaacg attccgccga 60

ccaagagatc gtgattgccc tgggcagcaa cgtgggagac agggtcagca cgttcgacag 120

ggcgctgcgg ctgatgagga gctcgggcat caggatcacc aggcacgcct gcctgtacga 180

gaccgcccct gcttacgtga c 201

<210> 54
<211> 150
<212> DNA
<213> Lolium multiflorum

<400> 54

gattccgccg accaagagat cgtgattgcc ctgggcagca acgtgggaga cagggtcagc 60

acgttcgaca gggcgctgcg gctgatgagg agctcgggca tcaggatcac caggcacgcc 120

tgcctgtacg agaccgcccc tgcttacgtg 150

<210> 55
<211> 200
<212> DNA
<213> Amaranthus hybridus

<400> 55

agatttgtga gcgccctgta gcagctgata gggatcctgc taccgttgct tcaataactg 60

ctggagtttt aggtggtgca aacattgtaa gagtacataa tgtgagggat aaccttgatg 120

ctgtcaagtt atgtgatgcc atactcggaa aaactgatta actgcttatt tgtaccacct 180

tgtgaatgac gtctagtgga 200

<210> 56
<211> 200
<212> DNA
<213> Amaranthus hybridus

<400> 56

tccactagac gtcattcaca aggtggtaca aataagcagt taatcagttt ttccgagtat 60

ggcatcacat aacttgacag catcaaggtt atccctcaca ttatgtactc ttacaatgtt 120

tgcaccacct aaaactccag cagttattga agcaacggta gcaggatccc tatcagctgc 180

tacagggcgc tcacaaatct 200

<210> 57
<211> 200
<212> DNA
<213> Amaranthus hybridus

<400> 57

caccttgtga atgacgtcta gtggaaagtt cgattgggat attgatgaag tccgttagtt 60

tgctcgaaag tagagcttta gcccgtaagg ttatagttct tacagatgta gaatcattgg 120

ctatttgcct attcctgctt agaagatcat agcattgatc cagactttgg attccatgta 180

gaattatgtt gcgactcatg 200

<210> 58
<211> 200
<212> DNA
<213> Amaranthus hybridus

<400> 58

catgagtcgc aacataattc tacatggaat ccaaagtctg gatcaatgct atgatcttct 60

aagcaggaat aggcaaatag ccaatgattc tacatctgta agaactataa ccttacgggc 120

taaagctcta ctttcgagca aactaacgga cttcatcaat atcccaatcg aactttccac 180

tagacgtcat tcacaaggtg 200

<210> 59
<211> 200
<212> DNA
<213> Amaranthus hybridus

<400> 59

atgtagaatt atgttgcgac tcatggtcga attggccctt tgaggaattg ttgaagttcc 60

ttgatctatg gttcaatcgg ctcggttcga tgaaggttgg actgtgtcga gacaagatgg 120

gtcaaagaga ccaatggaca agaagtcgac cttgggaaag cttgatcaag ttacttggac 180

gagcagatag cagcaggact 200

<210> 60
<211> 200
<212> DNA
<213> Amaranthus hybridus

<400> 60

agtcctgctg ctatctgctc gtccaagtaa cttgatcaag ctttcccaag gtcgacttct 60

tgtccattgg tctctttgac ccatcttgtc tcgacacagt ccaaccttca tcgaaccgag 120

ccgattgaac catagatcaa ggaacttcaa caattcctca aagggccaat tcgaccatga 180

gtcgcaacat aattctacat 200

<210> 61
<211> 200
<212> DNA
<213> Amaranthus lividus

<400> 61

aagggaataa ggtatggtcc taggccaatt gacttggaca tactatttta tgggaaattt 60

agggtgagct ctgagagcct cactatcccc catgaaagga tatgggaaag accatttgtg 120

atggcaccat tgattgattg tattgggtct gatgtagaaa atgacactat ttgtacatgg 180

cattcattat caaatttttt 200

<210> 62
<211> 200
<212> DNA
<213> Amaranthus lividus

<400> 62

aaaaaatttg ataatgaatg ccatgtacaa atagtgtcat tttctacatc agacccaata 60

caatcaatca atggtgccat cacaaatggt ctttcccata tcctttcatg ggggatagtg 120

aggctctcag agctcaccct aaatttccca taaaatagta tgtccaagtc aattggccta 180

ggaccatacc ttattccctt 200

<210> 63
<211> 200
<212> DNA
<213> Amaranthus lividus

<400> 63

catggcattc attatcaaat tttttgggtg gaatctttga agcatggggt aaactcggtg 60

gaagttccct aatcgggaag gatggaatga aaagggtttt gcccgttgga aatcgcttat 120

gggattggtc tcataaaacc tctgtaatgg gagtcttgaa cttgactcct gatagcttta 180

gtgatggcgg aagttttcaa 200

<210> 64
<211> 200
<212> DNA
<213> Amaranthus lividus

<400> 64

ttgaaaactt ccgccatcac taaagctatc aggagtcaag ttcaagactc ccattacaga 60

ggttttatga gaccaatccc ataagcgatt tccaacgggc aaaacccttt tcattccatc 120

cttcccgatt agggaacttc caccgagttt accccatgct tcaaagattc cacccaaaaa 180

atttgataat gaatgccatg 200

<210> 65
<211> 200
<212> DNA
<213> Amaranthus palmeri

<400> 65

cggatctgtt aaagggagcc ctctcatcct atgcttatca atgacttttt catatgccta 60

tcaaacagat cagattggat cgggttcgag ttcaggtcat atataaacat gtcagcaaac 120

ccttgaccca tacccaaccc atttagttaa ttggcttgaa aatcacaacc ttaattcgac 180

cagcgacaga tcaggttgac 200

<210> 66
<211> 200
<212> DNA
<213> Amaranthus palmeri

<400> 66

gtcaacctga tctgtcgctg gtcgaattaa ggttgtgatt ttcaagccaa ttaactaaat 60

gggttgggta tgggtcaagg gtttgctgac atgtttatat atgacctgaa ctcgaacccg 120

atccaatctg atctgtttga taggcatatg aaaaagtcat tgataagcat aggatgagag 180

ggctcccttt aacagatccg 200

<210> 67
<211> 200
<212> DNA
<213> Amaranthus palmeri

<400> 67

tcgaccagcg acagatcagg ttgacctgat ttcgacccac ttaacccatt tataattttt 60

ttttcgattt aagtttttta acattgatta aatctaattt tttaggcttt aattttaatt 120

tatgtttttt tgtttaattt gtatttacta tgaaaaataa gaaactatta tatgaactga 180

gtttagctta cacttattga 200

<210> 68
<211> 200
<212> DNA
<213> Amaranthus palmeri

<400> 68

tcaataagtg taagctaaac tcagttcata taatagtttc ttatttttca tagtaaatac 60

aaattaaaca aaaaaacata aattaaaatt aaagcctaaa aaattagatt taatcaatgt 120

taaaaaactt aaatcgaaaa aaaaattata aatgggttaa gtgggtcgaa atcaggtcaa 180

cctgatctgt cgctggtcga 200

<210> 69
<211> 200
<212> DNA
<213> Amaranthus palmeri

<400> 69

actgagttta gcttacactt attgatttaa ctcgaaatta acacatttaa ttaaatggtt 60

atatagggtt ttttagtttt aaaatttcaa cctgaaccta atctatttaa taaaaagatc 120

agatctgatc aacccatata attaattggg tcaaaatctc aactcaaacc catttatttc 180

gaattaaatt cagatcaaat 200

<210> 70
<211> 200
<212> DNA
<213> Amaranthus palmeri

<400> 70

atttgatctg aatttaattc gaaataaatg ggtttgagtt gagattttga cccaattaat 60

tatatgggtt gatcagatct gatcttttta ttaaatagat taggttcagg ttgaaatttt 120

aaaactaaaa aaccctatat aaccatttaa ttaaatgtgt taatttcgag ttaaatcaat 180

aagtgtaagc taaactcagt 200

<210> 71
<211> 200
<212> DNA
<213> Amaranthus palmeri

<400> 71

atttcgaatt aaattcagat caaattagca ggtcggatca actagcgcta atttttcctc 60

tcaagttttt tcatatgatg ccccttgttt tctttgaata aaattcacca atttaaaatc 120

cctgcccttg atccgttggt attggtattt ggtaaagcac atatggtaaa ttctactcca 180

ttgttatctg tagttttggt 200

<210> 72
<211> 200
<212> DNA
<213> Amaranthus palmeri

<400> 72

accaaaacta cagataacaa tggagtagaa tttaccatat gtgctttacc aaataccaat 60

accaacggat caagggcagg gattttaaat tggtgaattt tattcaaaga aaacaagggg 120

catcatatga aaaaacttga gaggaaaaat tagcgctagt tgatccgacc tgctaatttg 180

atctgaattt aattcgaaat 200

<210> 73
<211> 200
<212> DNA
<213> Amaranthus palmeri

<400> 73

ctccattgtt atctgtagtt ttggttctaa accccaaaca aatcctactc ctagcaccac 60

taatcctcat tactttctgt ttgctgtctt tctctacatt cctactaaat taagacctag 120

atgcagtgtt attctgttcg aggttgccga aaacggtaca atagttgcct aatttgcaat 180

tcatgtttgt tcttgcgttt 200

<210> 74
<211> 200
<212> DNA
<213> Amaranthus palmeri

<400> 74

aaacgcaaga acaaacatga attgcaaatt aggcaactat tgtaccgttt tcggcaacct 60

cgaacagaat aacactgcat ctaggtctta atttagtagg aatgtagaga aagacagcaa 120

acagaaagta atgaggatta gtggtgctag gagtaggatt tgtttggggt ttagaaccaa 180

aactacagat aacaatggag 200

<210> 75
<211> 200
<212> DNA
<213> Amaranthus palmeri

<400> 75

gcaattcatg tttgttcttg cgttttctac attgtttact ctattggaac aaaggttgaa 60

ctttcagcct ttcagggagt attttgattt ttctgtcaaa ttaccttgcc agatttattt 120

ggctattttg attgtgtgta tttctttgtc ccttgtttcc atggggctaa actccttttg 180

gttaatctta aacatttctt 200

<210> 76
<211> 200
<212> DNA
<213> Amaranthus palmeri

<400> 76

aagaaatgtt taagattaac caaaaggagt ttagccccat ggaaacaagg gacaaagaaa 60

tacacacaat caaaatagcc aaataaatct ggcaaggtaa tttgacagaa aaatcaaaat 120

actccctgaa aggctgaaag ttcaaccttt gttccaatag agtaaacaat gtagaaaacg 180

caagaacaaa catgaattgc 200

<210> 77
<211> 200
<212> DNA
<213> Amaranthus palmeri

<400> 77

ttttggttaa tcttaaacat ttcttctaat tttgaattgc agtggtatga agtgttgttt 60

aatatacaat caatttttct gaactgggtt aatgaagaga agcattgtag acatgaggaa 120

tgtgttcaag catctcaaaa caaccaaatt aatcccaaat gggatcttca actgtaatag 180

aggtaaatca catatattct 200

<210> 78
<211> 200
<212> DNA
<213> Amaranthus palmeri

<400> 78

agaatatatg tgatttacct ctattacagt tgaagatccc atttgggatt aatttggttg 60

ttttgagatg cttgaacaca ttcctcatgt ctacaatgct tctcttcatt aacccagttc 120

agaaaaattg attgtatatt aaacaacact tcataccact gcaattcaaa attagaagaa 180

atgtttaaga ttaaccaaaa 200

<210> 79
<211> 200
<212> DNA
<213> Amaranthus palmeri

<400> 79

aatagaggta aatcacatat attctcattt aatggttatt gttttatttt aaggatgtta 60

ttatgttgtt atcaatgata gaaataagag aagcttgagc attcaaattt atctatcact 120

cactggttgt ggacatctaa aatatggacc aagagttttc ttcatttttt gtaccagaag 180

ctgcaaatgt atgattaaag 200

<210> 80
<211> 200
<212> DNA
<213> Amaranthus palmeri

<400> 80

ctttaatcat acatttgcag cttctggtac aaaaaatgaa gaaaactctt ggtccatatt 60

ttagatgtcc acaaccagtg agtgatagat aaatttgaat gctcaagctt ctcttatttc 120

tatcattgat aacaacataa taacatcctt aaaataaaac aataaccatt aaatgagaat 180

atatgtgatt tacctctatt 200

<210> 81
<211> 200
<212> DNA
<213> Amaranthus palmeri

<400> 81

agaagctgca aatgtatgat taaagtttag gttggagttg caataaaatt ttattacccc 60

gacgctatta agtggctcac actgaggcgg ggtttgaggg tcagatggtg caaccttcag 120

aagaggttct aagcattttc aacatgttca accgcatagg gtcccgtaaa attggaggct 180

tcaatattaa attatgtagt 200

<210> 82
<211> 200
<212> DNA
<213> Amaranthus palmeri

<400> 82

actacataat ttaatattga agcctccaat tttacgggac cctatgcggt tgaacatgtt 60

gaaaatgctt agaacctctt ctgaaggttg caccatctga ccctcaaacc ccgcctcagt 120

gtgagccact taatagcgtc ggggtaataa aattttattg caactccaac ctaaacttta 180

atcatacatt tgcagcttct 200

<210> 83
<211> 200
<212> DNA
<213> Amaranthus palmeri

<400> 83

aggcttcaat attaaattat gtagtatgtg ttatgtgttt aaagatataa atatgttaat 60

gaatacataa tttttaaatt gcacagggcc ttcaaagaaa ttttcgattt tgctctgccc 120

ctggcaacct ttggcaaatt tggatagaat tagagaagtt tacttttatg tgtgtataat 180

atgatgaatg aatgagtgaa 200

<210> 84
<211> 200
<212> DNA
<213> Amaranthus palmeri

<400> 84

ttcactcatt cattcatcat attatacaca cataaaagta aacttctcta attctatcca 60

aatttgccaa aggttgccag gggcagagca aaatcgaaaa tttctttgaa ggccctgtgc 120

aatttaaaaa ttatgtattc attaacatat ttatatcttt aaacacataa cacatactac 180

ataatttaat attgaagcct 200

<210> 85
<211> 200
<212> DNA
<213> Amaranthus palmeri

<400>       85

ataatatgat gaatgaatga gtgaacaatg aagttcattt ctgagtcaat gacctatata    60

ctatcgaggt ttcgttaaaa aggatatata aataaaatta gagacatctt ttatccctaa   120

tgttgtctgt tttcttttct gggcgagact gtttttggag aaaaacgtta tgttgaatgc   180

ttgatttact cagaactgat                                               200


<210>       86
<211>       200
<212>       DNA
<213>       Amaranthus palmeri

<400>       86

atcagttctg agtaaatcaa gcattcaaca taacgttttt ctccaaaaac agtctcgccc    60

agaaaagaaa acagacaaca ttagggataa aagatgtctc taattttatt tatatatcct   120

ttttaacgaa acctcgatag tatataggtc attgactcag aaatgaactt cattgttcac   180

tcattcattc atcatattat                                               200


<210>       87
<211>       200
<212>       DNA
<213>       Amaranthus palmeri

<400>       87

aatgcttgat ttactcagaa ctgatgcaaa cagatggata aaaatggtgt tttcctattg    60

tttatgtttc gataacaggt ttcaaattcc actcgtatga agtttgatat actgctatgt   120

gtttttgtag cttcgtgttt tgctttcctt cactcatcac cagtgaatga tgttgaagtt   180

tgttccaaga agcaagaagt                                               200


<210>       88
<211>       200
<212>       DNA
<213>       Amaranthus palmeri

<400>       88

acttcttgct tcttggaaca aacttcaaca tcattcactg gtgatgagtg aaggaaagca    60

aaacacgaag ctacaaaaac acatagcagt atatcaaact tcatacgagt ggaatttgaa   120

acctgttatc gaaacataaa caataggaaa acaccatttt tatccatctg tttgcatcag   180

ttctgagtaa atcaagcatt                                               200


<210>       89
<211>       200
<212>       DNA
<213>       Amaranthus palmeri

<400>       89

aagtttgttc caagaagcaa gaagtcgtaa ttgcaatcgg gagcaatgtt ggtgacagat    60

tagaaaattt caaccaagct ctgcaacaaa tgaagaaatt aggcatagac atcacaaggc   120

atgggtgttt atatgagacg gaacctgcat acgtgactga tcaaccgaag tttcttaact   180

ctgctttaag aggctttaca                                               200


<210>       90
<211>       200
<212>       DNA
<213>       Amaranthus palmeri

<400>       90

tgtaaagcct cttaaagcag agttaagaaa cttcggttga tcagtcacgt atgcaggttc    60

cgtctcatat aaacacccat gccttgtgat gtctatgcct aatttcttca tttgttgcag   120

agcttggttg aaattttcta atctgtcacc aacattgctc ccgattgcaa ttacgacttc   180

ttgcttcttg gaacaaactt                                               200


<210>       91
<211>       200
<212>       DNA
<213>       Amaranthus palmeri

<400>       91
   taactctgct ttaagaggct ttacaaaact tgggcctcat gaattattag gggtattaaa    60
   gaaaattgag aaggatatgg gtagaaccaa gggaataagg tatggtccta ggccaattga   120
   cttggacata ctattttatg ggaagtttag ggtgagctct gagagcctca ctatccccca   180
   tgaaaggata tgggaaagac                                               200


<210>       92
<211>       200
<212>       DNA
<213>       Amaranthus palmeri

<400>       92

gtctttccca tatcctttca tgggggatag tgaggctctc agagctcacc ctaaacttcc    60

cataaaatag tatgtccaag tcaattggcc taggaccata ccttattccc ttggttctac   120

ccatatcctt ctcaattttc tttaataccc ctaataattc atgaggccca agttttgtaa   180

agcctcttaa agcagagtta                                               200


<210>       93
<211>       200
<212>       DNA

<213>       Amaranthus palmeri

<400>       93

ccccatgaaa ggatatggga aagaccattt gtgatggcac cattgattga ttgtattggg    60

tctgatgtag aaaatgacac tatttgtacg tggcattcat tatcaaattt ttcgggtgga   120

atctttgaag catggggtaa actcggtgga agttccctaa tcgggaagga tggaatgaaa   180

agggttttgc ccgttggaaa                                               200

<210>       94
<211>       200
<212>       DNA
<213>       Amaranthus palmeri

<400>       94

tttccaacgg gcaaaaccct tttcattcca tccttcccga ttagggaact tccaccgagt    60

ttaccccatg cttcaaagat tccacccgaa aaatttgata atgaatgcca cgtacaaata   120

gtgtcatttt ctacatcaga cccaatacaa tcaatcaatg gtgccatcac aaatggtctt   180

tcccatatcc tttcatgggg                                               200

<210>       95
<211>       200
<212>       DNA
<213>       Amaranthus palmeri

<400>       95

tgaaaagggt tttgcccgtt ggaaatcgct tatgggattg gtctcataaa acctctgtta    60

tgggagtctt gaacttgact cctgatagct ttagtgatgg cggaagtttt caatctgtcg   120

atactgcggt tgctaaggtt cgtcagatga tctcagatgg ggcagatata attgacatcg   180

gggcacaatc aaccagaccc                                               200

<210>       96
<211>       200
<212>       DNA
<213>       Amaranthus palmeri

<400>       96

gggtctggtt gattgtgccc cgatgtcaat tatatctgcc ccatctgaga tcatctgacg    60

aaccttagca accgcagtat cgacagattg aaaacttccg ccatcactaa agctatcagg   120

agtcaagttc aagactccca taacagaggt tttatgagac caatcccata agcgatttcc   180

aacgggcaaa acccttttca                                               200

<210>       97
<211>       200

<212>       DNA
<213>       Amaranthus palmeri

<400>       97

catcggggca caatcaacca gacccatggc aactagaatt tcggctgaag aagagctaga    60

aagagtagta cctgtattgg aagctgtcaa ggatttgatc gaggaagaag gaagaatctt   120

gtcagtggat acgttttatt ctaaagttgc ttcggaggcc gtcaagaagg gggcacacat   180

tgtgaatgat gtctctagtg                                               200


<210>       98
<211>       200
<212>       DNA
<213>       Amaranthus palmeri

<400>       98

cactagagac atcattcaca atgtgtgccc ccttcttgac ggcctccgaa gcaactttag    60

aataaaacgt atccactgac aagattcttc cttcttcctc gatcaaatcc ttgacagctt   120

ccaatacagg tactactctt tctagctctt cttcagccga aattctagtt gccatgggtc   180

tggttgattg tgccccgatg                                               200


<210>       99
<211>       200
<212>       DNA
<213>       Amaranthus palmeri

<400>       99

cacattgtga atgatgtctc tagtgggaaa ctcgattccg agatgtttaa tgttgttgcg    60

gaccttaaag ttccttatat agcaatgcac atgcgaggag atccgacttc aatgcaaaac   120

tctgagaact tgacctacaa tgatgtttgt aagcaagtgg ctttggagtt gagttctagg   180

gtcatagatg cagaattatc                                               200


<210>       100
<211>       200
<212>       DNA
<213>       Amaranthus palmeri

<400>       100

gataattctg catctatgac cctagaactc aactccaaag ccacttgctt acaaacatca    60

ttgtaggtca agttctcaga gttttgcatt gaagtcggat ctcctcgcat gtgcattgct   120

atataaggaa ctttaaggtc cgcaacaaca ttaaacatct cggaatcgag tttcccacta   180

gagacatcat tcacaatgtg                                               200


<210>       101

<211>	200
<212>	DNA
<213>	Amaranthus palmeri

<400>	101

ctagggtcat agatgcagaa ttatcgggaa ttcctgcttg gaggatagtt attgatcccg	60

gcatcggatt ttctaagaat acgaatcaaa atttggaaat tcttagtggt ttacaaaaga	120

tacgggaaga gatagctaag aagagtttgg cggtggctca ttgccccttg ctaattggac	180

cttcaagaaa gaggtttctg	200

<210>	102
<211>	200
<212>	DNA
<213>	Amaranthus palmeri

<400>	102

cagaaacctc tttcttgaag gtccaattag caaggggcaa tgagccaccg ccaaactctt	60

cttagctatc tcttcccgta tcttttgtaa accactaaga atttccaaat tttgattcgt	120

attcttagaa aatccgatgc cgggatcaat aactatcctc caagcaggaa ttcccgataa	180

ttctgcatct atgaccctag	200

<210>	103
<211>	200
<212>	DNA
<213>	Amaranthus palmeri

<400>	103

tggaccttca agaaagaggt ttctgggcga gatttgtgag cgccctgtag cagctgacag	60

ggatcctgct accattgctt ctataactgc tggagtttta ggtggtgcaa acattgtaag	120

agtacataat gtgagggata accttgatgc tgtcaagtta tgtgatgcca tactcggaaa	180

aactgattaa ctgcttgttt	200

<210>	104
<211>	200
<212>	DNA
<213>	Amaranthus palmeri

<400>	104

aaacaagcag ttaatcagtt tttccgagta tggcatcaca taacttgaca gcatcaaggt	60

tatccctcac attatgtact cttacaatgt ttgcaccacc taaaactcca gcagttatag	120

aagcaatggt agcaggatcc ctgtcagctg ctacagggcg ctcacaaatc tcgcccagaa	180

acctctttct tgaaggtcca	200

<210>	105
<211>	200
<212>	DNA
<213>	Amaranthus palmeri

<400>	105

ggaaaaactg attaactgct tgtttgtacc accttgtgaa tgatgtctag tggaaggttc	60

gattgggatt atgatgaagt ccgttagttt gctcgaaaga agagctttag cccgtaaggt	120

tatagttctt acagatgtag aatcattggc tatttgccta tttctgctta gaagatcata	180

gcattgatcc agactttgga	200

<210>	106
<211>	200
<212>	DNA
<213>	Amaranthus palmeri

<400>	106

tccaaagtct ggatcaatgc tatgatcttc taagcagaaa taggcaaata gccaatgatt	60

ctacatctgt aagaactata accttacggg ctaaagctct tctttcgagc aaactaacgg	120

acttcatcat aatcccaatc gaaccttcca ctagacatca ttcacaaggt ggtacaaaca	180

agcagttaat cagtttttcc	200

<210>	107
<211>	200
<212>	DNA
<213>	Amaranthus palmeri

<400>	107

tcatagcatt gatccagact ttggattcca tgtagaatta tgttgcgact catggtcgaa	60

ttggcccttt gaggaattgt tgaagttcct tgagtctatg gttcaatcgg ctcggttcga	120

tgaaggttgg actgtgtcga gacaagatgg gtcaaagaga ccaatggaca agaagtcgac	180

cttgggcaag cttgatcaag	200

<210>	108
<211>	200
<212>	DNA
<213>	Amaranthus palmeri

<400>	108

cttgatcaag cttgcccaag gtcgacttct tgtccattgg tctctttgac ccatcttgtc	60

tcgacacagt ccaaccttca tcgaaccgag ccgattgaac catagactca aggaacttca	120

acaattcctc aaagggccaa ttcgaccatg agtcgcaaca taattctaca tggaatccaa	180

agtctggatc aatgctatga	200

<210>	109
<211>	200
<212>	DNA
<213>	Amaranthus palmeri

<400>	109

tcgaccttgg gcaagcttga tcaagttact tggacaagca gataacagca ggctctggcg	60

agcattagac atatcatttg ttttttggag tcagatctgc aatcaatcag ttgcaatgga	120

caaaccatat acggttgtta gttgttactg tcactgtttg agttgaatta gatcatgcaa	180

acactgtagg agtcttgatt	200

<210>	110
<211>	200
<212>	DNA
<213>	Amaranthus palmeri

<400>	110

aatcaagact cctacagtgt ttgcatgatc taattcaact caaacagtga cagtaacaac	60

taacaaccgt atatggtttg tccattgcaa ctgattgatt gcagatctga ctccaaaaaa	120

caaatgatat gtctaatgct cgccagagcc tgctgttatc tgcttgtcca agtaacttga	180

tcaagcttgc ccaaggtcga	200

<210>	111
<211>	200
<212>	DNA
<213>	Amaranthus palmeri

<400>	111

tgcaaacact gtaggagtct tgattatgtg aagctatgtg acactgtact tctcaaaatg	60

aaatctttga tatgatgtta gtttcaagag ttgtaattcc cccctcacat ttctgttcta	120

tgttgtattg ttgtgtgctt tttggaattg tattcctgca atagaattat tcccgcaaaa	180

tggtattcct ttatatcatc	200

<210>	112
<211>	200
<212>	DNA
<213>	Amaranthus palmeri

<400>	112

gatgatataa aggaatacca ttttgcggga ataattctat tgcaggaata caattccaaa	60

aagcacacaa caatacaaca tagaacagaa atgtgagggg ggaattacaa ctcttgaaac	120

taacatcata tcaaagattt cattttgaga agtacagtgt cacatagctt cacataatca	180

agactcctac agtgtttgca	200

<210>	113
<211>	200
<212>	DNA
<213>	Amaranthus palmeri

<400>	113

caaaatggta ttcctttata tcatcaaagg agtttttgta cttctaatca ttgatcttta	60

gatgcaaaac tgaattaata caagtattct tgatgaaggc ttattgaaga actcactttt	120

tatttatgtc taaacttgtt aaagatgttc tgcttccgcc cttgccacgg gatgtcggga	180

ttgactaggg gtggtaatcg	200

<210>	114
<211>	200
<212>	DNA
<213>	Amaranthus palmeri

<400>	114

cgattaccac ccctagtcaa tcccgacatc ccgtggcaag ggcggaagca gaacatcttt	60

aacaagttta gacataaata aaaagtgagt tcttcaataa gccttcatca agaatacttg	120

tattaattca gttttgcatc taaagatcaa tgattagaag tacaaaaact cctttgatga	180

tataaaggaa taccattttg	200

<210>	115
<211>	200
<212>	DNA
<213>	Amaranthus palmeri

<400>	115

cgggattgac taggggtggt aatcgaggga ttcctatgct tgaattgact cgtttaagat	60

tttgcgttga ttttaagtca aacgttttgc taagcataaa ctattctagc tcgacttgaa	120

taatgaagtt ttcgagtcaa gtccgagctg ttctataagt ttacgtatct tgaagcttct	180

tagttcttac ttcttatcct	200

<210>	116
<211>	200
<212>	DNA
<213>	Amaranthus palmeri

<400>	116

aggataagaa gtaagaacta agaagcttca agatacgtaa acttatagaa cagctcggac	60

ttgactcgaa aacttcatta ttcaagtcga gctagaatag tttatgctta gcaaaacgtt	120

tgacttaaaa tcaacgcaaa atcttaaacg agtcaattca agcataggaa tccctcgatt	180

accaccccta gtcaatcccg 200

<210> 117
<211> 200
<212> DNA
<213> Amaranthus palmeri

<400> 117

cttcttagtt cttacttctt atcctttatt ttttgacaca ttgaaccgca ttgatgcttg 60

cttgactcaa ctcgttaaag tcttgagttc atcacgaatt cgagtcgagc aagtttaaac 120

ccaacattaa ctaagctctc gaggcttaat gaggcagggg tagctcaagt ttgttattca 180

agcaacttac catagtattc 200

<210> 118
<211> 200
<212> DNA
<213> Amaranthus palmeri

<400> 118

gaatactatg gtaagttgct tgaataacaa acttgagcta cccctgcctc attaagcctc 60

gagagcttag ttaatgttgg gtttaaactt gctcgactcg aattcgtgat gaactcaaga 120

ctttaacgag ttgagtcaag caagcatcaa tgcggttcaa tgtgtcaaaa aataaaggat 180

aagaagtaag aactaagaag 200

<210> 119
<211> 200
<212> DNA
<213> Amaranthus palmeri

<400> 119

attcaagcaa cttaccatag tattctttta aaaggcatat agtcagttag ttatttgtta 60

atttcagtag aacgaaagaa gtacattatt aaacttggcc attgatgctt gagctagaga 120

gtttcggttg tgatcgaaac gaatcctgat ttcgctcttg atctttgatg tgaagtgaca 180

aggccttcat aaaactcctt 200

<210> 120
<211> 200
<212> DNA
<213> Amaranthus palmeri

<400> 120

aaggagtttt atgaaggcct tgtcacttca catcaaagat caagagcgaa atcaggattc 60

gtttcgatca caaccgaaac tctctagctc aagcatcaat ggccaagttt aataatgtac 120

ttctttcgtt ctactgaaat taacaaataa ctaactgact atatgccttt taaaagaata 180

ctatggtaag ttgcttgaat 200

<210> 121
<211> 200
<212> DNA
<213> Amaranthus palmeri

<400> 121

tgacaaggcc ttcataaaac tcctttattc ttcagcagtg catacaaaaa tcatgtgtgc 60

ccagctccta gctcacatac cgcgtttttc ccctaatcta gtgtggttaa ataaaaacag 120

caagatgtgt tgatgtcatg tgcaatagta attatcaggt gaagtaattc aatatgtagc 180

cagtcacctc atatcaaaaa 200

<210> 122
<211> 200
<212> DNA
<213> Amaranthus palmeri

<400> 122

tttttgatat gaggtgactg gctacatatt gaattacttc acctgataat tactattgca 60

catgacatca acacatcttg ctgtttttat ttaaccacac tagattaggg gaaaaacgcg 120

gtatgtgagc taggagctgg gcacacatga tttttgtatg cactgctgaa gaataaagga 180

gttttatgaa ggccttgtca 200

<210> 123
<211> 200
<212> DNA
<213> Amaranthus palmeri

<400> 123

gtagccagtc acctcatatc aaaaaaatgt tgggaggaaa acgtattcgt tcatcactaa 60

acctgttttt atacatctaa ctgatcgatc agggtttcaa aatccaaatt aatacaatgg 120

catttgacga agatatccag aacacatccc ccttgtttct gctagataat caactattct 180

gattctgctg taatacctca 200

<210> 124
<211> 200
<212> DNA
<213> Amaranthus palmeri

<400> 124

tgaggtatta cagcagaatc agaatagttg attatctagc agaaacaagg gggatgtgtt 60

ctggatatct tcgtcaaatg ccattgtatt aatttggatt ttgaaaccct gatcgatcag 120

ttagatgtat aaaaacaggt ttagtgatga acgaatacgt tttcctccca acattttttt 180

gatatgaggt gactggctac 200

<210> 125
<211> 200
<212> DNA
<213> Amaranthus palmeri

<400> 125

attctgattc tgctgtaata cctcagcttc ggccctagcc atgggatcgt cctctttttc 60

tccttcacgc tcctcggcca gtttctttct gtgcagttct tcagcagctt ctattgaagc 120

tttgttctcg gcctgaagac gctgcatttc ctcttccatt tgctgcctct caaaccatgt 180

atcagcatcc gcccaaactg 200

<210> 126
<211> 200
<212> DNA
<213> Amaranthus palmeri

<400> 126

cagtttgggc ggatgctgat acatggtttg agaggcagca aatggaagag gaaatgcagc 60

gtcttcaggc cgagaacaaa gcttcaatag aagctgctga agaactgcac agaaagaaac 120

tggccgagga gcgtgaagga gaaaaagagg acgatcccat ggctagggcc gaagctgagg 180

tattacagca gaatcagaat 200

<210> 127
<211> 200
<212> DNA
<213> Amaranthus palmeri

<400> 127

gaagctgtca aggatttgat cgaggaagaa ggaagaatct tgtcagtgga tacgttttat 60

tctaaagttg cttcggaggc cgtcaagaag ggggcacaca ttgtgaatga tgtctctagt 120

gggaaactcg attccgagat gtttaatgtt gttgcggacc ttaaagttcc ttatatagca 180

atgcacatgc gaggagatcc 200

<210> 128
<211> 200
<212> DNA
<213> Amaranthus palmeri

<400> 128

ggatctcctc gcatgtgcat tgctatataa ggaactttaa ggtccgcaac aacattaaac 60

atctcggaat cgagtttccc actagagaca tcattcacaa tgtgtgcccc cttcttgacg 120

gcctccgaag caactttaga ataaaacgta tccactgaca agattcttcc ttcttcctcg 180

atcaaatcct tgacagcttc 200

<210> 129
<211> 200
<212> DNA
<213> Amaranthus palmeri

<400> 129

tagcaatgca catgcgagga gatccgactt caatgcaaaa ctctgagaac ttgacctaca 60

atgatgtttg taagcaagtg gctttggagt tgagttctag ggtcatagat gcagaattat 120

cgggaattcc tgcttggagg atagttattg atcccggcat cggattttct aagaatacga 180

atcaaaattt ggaaattctt 200

<210> 130
<211> 200
<212> DNA
<213> Amaranthus palmeri

<400> 130

aagaatttcc aaattttgat tcgtattctt agaaaatccg atgccgggat caataactat 60

cctccaagca ggaattcccg ataattctgc atctatgacc ctagaactca actccaaagc 120

cacttgctta caaacatcat tgtaggtcaa gttctcagag ttttgcattg aagtcggatc 180

tcctcgcatg tgcattgcta 200

<210> 131
<211> 200
<212> DNA
<213> Amaranthus palmeri

<400> 131

tacgaatcaa aatttggaaa ttcttagtgg tttacaaaag atacgggaag agatagctaa 60

gaagagtttg gcggtggctc attgcccctt gctaattgga ccttcaagaa agaggtttct 120

gggcgagatt tgtgagcgcc ctgtagcagc tgacagggat cctgctacca ttgcttctat 180

aactgctgga gttttaggtg 200

<210> 132
<211> 200
<212> DNA
<213> Amaranthus palmeri

<400> 132

cacctaaaac tccagcagtt atagaagcaa tggtagcagg atccctgtca gctgctacag 60

ggcgctcaca aatctcgccc agaaacctct ttcttgaagg tccaattagc aaggggcaat 120

gagccaccgc caaactcttc ttagctatct cttcccgtat cttttgtaaa ccactaagaa 180

tttccaaatt ttgattcgta 200

<210> 133
<211> 200
<212> DNA
<213> Amaranthus palmeri

<400> 133

tctataactg ctggagtttt aggtggtgca aacattgtaa gagtacataa tgttagggat 60

aaccttgatg ctgtcaagtt atgtgatgcc atactcggaa aaactgatta actgcttgtt 120

tgtaccacct tgtgaatgat gtctagtgga aggttcgatt gggattatga tgaagtccgt 180

tagtttgctc gaaagtagag 200

<210> 134
<211> 200
<212> DNA
<213> Amaranthus palmeri

<400> 134

ctctactttc gagcaaacta acggacttca tcataatccc aatcgaacct tccactagac 60

atcattcaca aggtggtaca aacaagcagt taatcagttt ttccgagtat ggcatcacat 120

aacttgacag catcaaggtt atccctaaca ttatgtactc ttacaatgtt tgcaccacct 180

aaaactccag cagttataga 200

<210> 135
<211> 200
<212> DNA
<213> Amaranthus palmeri

<400> 135

tccgttagtt tgctcgaaag tagagcttta gcccgtaagg ctatagttct tacagatgta 60

gaatcattgg ctatttgcct atttctgctt agaagatcat agcattgatc cagactttgg 120

attccatgta gaattatgtt gcgactcatg gtcgaattgg ccctttgagg aattgttgaa 180

gttccttgag tctatggttc 200

<210> 136
<211> 200
<212> DNA
<213> Amaranthus palmeri

<400> 136

gaaccataga ctcaaggaac ttcaacaatt cctcaaaggg ccaattcgac catgagtcgc 60

aacataattc tacatggaat ccaaagtctg gatcaatgct atgatcttct aagcagaaat 120

aggcaaatag ccaatgattc tacatctgta agaactatag ccttacgggc taaagctcta 180

ctttcgagca aactaacgga 200

<210> 137
<211> 200
<212> DNA
<213> Amaranthus palmeri

<400> 137

ttgaagttcc ttgagtctat ggttcaatcg gctcggttcg atgaaggttg gactgtgtcg 60

agacaagatg ggtcaaagag accaatggac aagaagtcga ccttgggcaa gcttgatcaa 120

gttacttgga caagcagata acagcaggct ctggcgagca ttagacatat catttgtttt 180

ttggagtcag atctgcaatc 200

<210> 138
<211> 200
<212> DNA
<213> Amaranthus palmeri

<400> 138

gattgcagat ctgactccaa aaaacaaatg atatgtctaa tgctcgccag agcctgctgt 60

tatctgcttg tccaagtaac ttgatcaagc ttgcccaagg tcgacttctt gtccattggt 120

ctctttgacc catcttgtct cgacacagtc caaccttcat cgaaccgagc cgattgaacc 180

atagactcaa ggaacttcaa 200

<210> 139
<211> 200
<212> DNA
<213> Amaranthus palmeri

<400> 139

gctttccttc actcatcacc agtgaatgat gttgaagttt gttccaagaa gcaagaagtc 60

gtaattgcaa tcgggagcaa tgttggtgac agattagaaa atttcaacca agctctgcaa 120

caaatgaaga aattaggcat agacatcaca aggcatgggt gtttatatga gacggaacct 180

gcatacgtga ctgatcaacc 200

<210> 140
<211> 200
<212> DNA
<213> Amaranthus palmeri

<400> 140

ggttgatcag tcacgtatgc aggttccgtc tcatataaac acccatgcct tgtgatgtct 60

atgcctaatt tcttcatttg ttgcagagct tggttgaaat tttctaatct gtcaccaaca 120

ttgctcccga ttgcaattac gacttcttgc ttcttggaac aaacttcaac atcattcact 180

ggtgatgagt gaaggaaagc 200

<210> 141
<211> 200
<212> DNA
<213> Amaranthus palmeri

<400> 141

aacctgcata cgtgactgat caaccgaagt ttcttaactc tgctttaaga ggctttacaa 60

aacttgggcc tcatgaatta ttaggggtat taaagaaaat tgagaaggat atgggtagaa 120

ccaagggaat aaggtatggt cctaggccaa ttgacttgga catactattt tatgggaagt 180

ttagggtgag ctctgagagc 200

<210> 142
<211> 200
<212> DNA
<213> Amaranthus palmeri

<400> 142

gctctcagag ctcaccctaa acttcccata aaatagtatg tccaagtcaa ttggcctagg 60

accatacctt attcccttgg ttctacccat atccttctca attttcttta atacccctaa 120

taattcatga ggcccaagtt ttgtaaagcc tcttaaagca gagttaagaa acttcggttg 180

atcagtcacg tatgcaggtt 200

<210> 143
<211> 200
<212> DNA
<213> Amaranthus palmeri

<400> 143

gaagtttagg gtgagctctg agagcctcac tatcccccat gaaaggatat gggaaagacc 60

atttgtgatg gcaccattga ttgattgtat tgggtctgat gtagaaaatg acactatttg 120

tacgtggcat tcattatcaa atttttcggg tggaatcttt gaagcatggg gtaaactcgg 180

tggaagttcc ctaatcggga 200

<210> 144
<211> 200
<212> DNA
<213> Amaranthus palmeri

<400> 144

tcccgattag ggaacttcca ccgagtttac cccatgcttc aaagattcca cccgaaaaat 60

ttgataatga atgccacgta caaatagtgt cattttctac atcagaccca atacaatcaa 120

tcaatggtgc catcacaaat ggtctttccc atatcctttc atgggggata gtgaggctct 180

cagagctcac cctaaacttc 200

<210> 145
<211> 200
<212> DNA
<213> Amaranthus palmeri

<400> 145

ctcggtggaa gttccctaat cgggaaggat ggaatgaaaa gggttttgcc cgttggaaat 60

cgcttatggg attggtctca taaaacctct gttatgggag tcttgaactt gactcctgat 120

agctttagtg atggcggaag ttttcaatct gtcgatactg cggttgctaa ggttcgtcag 180

atgatctcag atggggcaga 200

<210> 146
<211> 200
<212> DNA
<213> Amaranthus palmeri

<400> 146

tctgccccat ctgagatcat ctgacgaacc ttagcaaccg cagtatcgac agattgaaaa 60

cttccgccat cactaaagct atcaggagtc aagttcaaga ctcccataac agaggtttta 120

tgagaccaat cccataagcg atttccaacg ggcaaaaccc ttttcattcc atccttcccg 180

attagggaac ttccaccgag 200

<210> 147
<211> 200
<212> DNA
<213> Amaranthus palmeri

<400> 147

gtcagatgat ctcagatggg gcagatataa ttgacatcgg ggcacaatca accagaccca 60

tggcaactag aatttcggct gaagaagagc tagaaagagt agtacctgta ttggaagctg 120

tcaaggattt gatcgaggaa gaaggaagaa tcttgtcagt ggatacgttt tattctaaag 180

ttgcttcgga ggccgtcaag 200

<210> 148
<211> 200
<212> DNA
<213> Amaranthus palmeri

<400>       148

cttgacggcc tccgaagcaa ctttagaata aaacgtatcc actgacaaga ttcttccttc    60

ttcctcgatc aaatccttga cagcttccaa tacaggtact actctttcta gctcttcttc   120

agccgaaatt ctagttgcca tgggtctggt tgattgtgcc ccgatgtcaa ttatatctgc   180

cccatctgag atcatctgac                                               200


<210>       149
<211>       200
<212>       DNA
<213>       Amaranthus palmeri

<400>       149

taaagttgct tcggaggccg tcaagaaggg ggcacacatt gtgaatgatg tctctagtgg    60

gaaactcgat tccgagatgt ttaatgttgt tgcggacctt aaagttcctt atatagcaat   120

gcacatgcga ggagatccga cttcaatgca aaactctgag aacttgacct acaatgatgt   180

ttgtaagcaa gtggctttgg                                               200


<210>       150
<211>       200
<212>       DNA
<213>       Amaranthus palmeri

<400>       150

ccaaagccac ttgcttacaa acatcattgt aggtcaagtt ctcagagttt tgcattgaag    60

tcggatctcc tcgcatgtgc attgctatat aaggaacttt aaggtccgca acaacattaa   120

acatctcgga atcgagtttc ccactagaga catcattcac aatgtgtgcc cccttcttga   180

cggcctccga agcaacttta                                               200


<210>       151
<211>       200
<212>       DNA
<213>       Amaranthus palmeri

<400>       151

gatgtttgta agcaagtggc tttggagttg agttctaggg tcatagatgc agaattatcg    60

ggaattcctg cttggaggat agttattgat cccggcatcg gattttctaa gaatacgaat   120

caaaatttgg aaattcttag tggtttacaa aagatacggg aagagatagc taagaagagt   180

ttggcggtgg ctcattgccc                                               200


<210>       152
<211>       200
<212>       DNA
<213>       Amaranthus palmeri

<400>       152

gggcaatgag ccaccgccaa actcttctta gctatctctt cccgtatctt ttgtaaacca    60

ctaagaattt ccaaattttg attcgtattc ttagaaaatc cgatgccggg atcaataact   120

atcctccaag caggaattcc cgataattct gcatctatga ccctagaact caactccaaa   180

gccacttgct tacaaacatc                                                200


<210>       153
<211>       200
<212>       DNA
<213>       Amaranthus palmeri

<400>       153

agagtttggc ggtggctcat tgccccttgc taattggacc ttcaagaaag aggtttctgg    60

gcgagatttg tgagcgccct gtagcagctg acagggatcc tgctaccatt gcttctataa   120

ctgctggagt tttaggtggt gcaaacattg taagagtaca taatgtgagg gataaccttg   180

atgctgtcaa gttatgtgat                                                200


<210>       154
<211>       200
<212>       DNA
<213>       Amaranthus palmeri

<400>       154

atcacataac ttgacagcat caaggttatc cctcacatta tgtactctta caatgtttgc    60

accacctaaa actccagcag ttatagaagc aatggtagca ggatccctgt cagctgctac   120

agggcgctca caaatctcgc ccagaaacct ctttcttgaa ggtccaatta gcaaggggca   180

atgagccacc gccaaactct                                                200


<210>       155
<211>       200
<212>       DNA
<213>       Amaranthus palmeri

<400>       155

gctttccttc actcttcacc agggaacgtt gttgaagttt gttccaagaa gcaagaagtc    60

gtaattgcaa tcgggagcaa tgttggtgac agattagaaa atttcaacca agctctgcaa   120

caaatgaaga aattaggcat agacatcaca aggcatggtt gtttatatga gacggaacct   180

gcatacgtga ctgatcaacc                                                200


<210>       156
<211>       200
<212>       DNA

<213>       Amaranthus palmeri

<400>       156

ggttgatcag tcacgtatgc aggttccgtc tcatataaac aaccatgcct tgtgatgtct    60

atgcctaatt tcttcatttg ttgcagagct tggttgaaat tttctaatct gtcaccaaca   120

ttgctcccga ttgcaattac gacttcttgc ttcttggaac aaacttcaac aacgttccct   180

ggtgaagagt gaaggaaagc                                               200


<210>       157
<211>       200
<212>       DNA
<213>       Amaranthus palmeri

<400>       157

aacctgcata cgtgactgat caaccgaagt ttcttaactc tgctttaaga ggctttacaa    60

gacttggacc tcatgaatta ttaggggtat tgaagaaaat tgagaaggat atgggtagaa   120

ccaagggaat aaggtatggt cctaggccaa ttgacttgga catactattt tatgggaagt   180

ttagggtgag ctctgagagc                                               200


<210>       158
<211>       200
<212>       DNA
<213>       Amaranthus palmeri

<400>       158

gctctcagag ctcaccctaa acttcccata aaatagtatg tccaagtcaa ttggcctagg    60

accatacctt attcccttgg ttctacccat atccttctca attttcttca atacccctaa   120

taattcatga ggtccaagtc ttgtaaagcc tcttaaagca gagttaagaa acttcggttg   180

atcagtcacg tatgcaggtt                                               200


<210>       159
<211>       200
<212>       DNA
<213>       Amaranthus palmeri

<400>       159

gaagtttagg gtgagctctg agagcctcac tatcccccat gaaaggatat gggaaagacc    60

atttgtgatg gcaccattga ttgattgtat tgggtctgat gtagaaaatg acactatttg   120

tacgtggcat tcattatcaa atttttcggg tggaatcttt gaagcatggg gtaaactcgg   180

tggaagctcc ctaatcggga                                               200


<210>       160
<211>       200

<212>       DNA
<213>       Amaranthus palmeri

<400>       160

tcccgattag ggagcttcca ccgagtttac cccatgcttc aaagattcca cccgaaaaat    60

ttgataatga atgccacgta caaatagtgt cattttctac atcagaccca atacaatcaa   120

tcaatggtgc catcacaaat ggtctttccc atatcctttc atgggggata gtgaggctct   180

cagagctcac cctaaacttc                                               200


<210>       161
<211>       200
<212>       DNA
<213>       Amaranthus palmeri

<400>       161

ctcggtggaa gctccctaat cgggaaggat ggaatgaaaa gggttttgcc cgttggaaat    60

ctcttatggg attggtctca taaaacctct gttatgggag tcttgaactt gactcctgat   120

agctttagtg atggcggaag ttttcaatct gtcaatactg cggttgctaa ggttcgtcag   180

atgatctcag atggggcaga                                               200


<210>       162
<211>       200
<212>       DNA
<213>       Amaranthus palmeri

<400>       162

tctgccccat ctgagatcat ctgacgaacc ttagcaaccg cagtattgac agattgaaaa    60

cttccgccat cactaaagct atcaggagtc aagttcaaga ctcccataac agaggtttta   120

tgagaccaat cccataagag atttccaacg ggcaaaaccc ttttcattcc atccttcccg   180

attagggagc ttccaccgag                                               200


<210>       163
<211>       200
<212>       DNA
<213>       Amaranthus palmeri

<400>       163

gtcagatgat ctcagatggg gcagatataa ttgacatcgg ggcacaatca accagaccca    60

tggcaactag aatttcggct gaagaagagc tagcaagagt agtacctgta ttagaagctg   120

tcaaggattt gatcgaggaa gaaggaagaa tcttgtcagt ggatacgttt tattctaaag   180

ttgctttgga ggccgtcaag                                               200


<210>       164

<211>	200
<212>	DNA
<213>	Amaranthus palmeri

<400>	164

cttgacggcc tccaaagcaa ctttagaata aaacgtatcc actgacaaga ttcttccttc	60

ttcctcgatc aaatccttga cagcttctaa tacaggtact actcttgcta gctcttcttc	120

agccgaaatt ctagttgcca tgggtctggt tgattgtgcc ccgatgtcaa ttatatctgc	180

cccatctgag atcatctgac	200

<210>	165
<211>	200
<212>	DNA
<213>	Amaranthus palmeri

<400>	165

taaagttgct ttggaggccg tcaagaaggg ggcacacatt gtgaatgatg tctctagtgg	60

taaactcgat tccgagatgt ttaatgttgt tgcggacctt aaagttcctt atatagcaat	120

gcacatgcga ggagatccga cttcaatgca aaactctgag aacttgacct acaatgatgt	180

ttgtaagcaa gtggcttcgg	200

<210>	166
<211>	200
<212>	DNA
<213>	Amaranthus palmeri

<400>	166

ccgaagccac ttgcttacaa acatcattgt aggtcaagtt ctcagagttt tgcattgaag	60

tcggatctcc tcgcatgtgc attgctatat aaggaacttt aaggtccgca acaacattaa	120

acatctcgga atcgagttta ccactagaga catcattcac aatgtgtgcc cccttcttga	180

cggcctccaa agcaacttta	200

<210>	167
<211>	200
<212>	DNA
<213>	Amaranthus palmeri

<400>	167

gatgtttgta agcaagtggc ttcggagttg agttctaggg tcatagatgc agaattatcg	60

ggaattcctg cttggaggat agttattgat cccggcatcg gattttctaa gaatacgaat	120

caaaatttgg aaattcttag tggtttacaa aagatacggg aagagatagc taagaagagt	180

ttggcggtgg ctcattgccc	200

<210>	168
<211>	200
<212>	DNA
<213>	Amaranthus palmeri

<400>	168

gggcaatgag ccaccgccaa actcttctta gctatctctt cccgtatctt ttgtaaacca	60

ctaagaattt ccaaattttg attcgtattc ttagaaaatc cgatgccggg atcaataact	120

atcctccaag caggaattcc cgataattct gcatctatga ccctagaact caactccgaa	180

gccacttgct tacaaacatc	200

<210>	169
<211>	200
<212>	DNA
<213>	Amaranthus palmeri

<400>	169

agagtttggc ggtggctcat tgccccttgc taattggacc ttcaagaaag aggtttctgg	60

gcgagatttg tgagcgccct gtagcagctg acagggatcc tgctaccatt gcttctataa	120

ctgctggagt tttaggtggt gcaaacattg taagagtaca taatgttagg gataaccttg	180

atgctgtcaa gttatgtgat	200

<210>	170
<211>	200
<212>	DNA
<213>	Amaranthus palmeri

<400>	170

atcacataac ttgacagcat caaggttatc cctaacatta tgtactctta caatgtttgc	60

accacctaaa actccagcag ttatagaagc aatggtagca ggatccctgt cagctgctac	120

agggcgctca caaatctcgc ccagaaacct ctttcttgaa ggtccaatta gcaaggggca	180

atgagccacc gccaaactct	200

<210>	171
<211>	200
<212>	DNA
<213>	Amaranthus palmeri

<400>	171

ggtgtttatt tgggcatggc cttatgtgtg atgttggtaa attttgtggc ttcgtccact	60

aatctgatgt atcggatctg ttaaagggag ccctctcatc ctatgcttat caatgacttt	120

ttcatatgcc tatcaaacag atcagattgg atcgggttcg agttcaggtc atatataaac	180

atgtcagcaa acccttgacc	200

<210>	172
<211>	200
<212>	DNA
<213>	Amaranthus palmeri

<400>	172

ggtcaagggt ttgctgacat gtttatatat gacctgaact cgaacccgat ccaatctgat	60

ctgtttgata ggcatatgaa aaagtcattg ataagcatag gatgagaggg ctccctttaa	120

cagatccgat acatcagatt agtggacgaa gccacaaaat ttaccaacat cacacataag	180

gccatgccca aataaacacc	200

<210>	173
<211>	200
<212>	DNA
<213>	Amaranthus palmeri

<400>	173

taaacatgtc agcaaaccct tgacccatac ccaacccatt tagttaattg gcttgaaaat	60

cacaacctta attcgaccag cgacagatca ggttgacctg atttcgaccc acttaaccca	120

tttataattt ttttttcgat ttaagttttt taacattgat taaatctaat tttttaggct	180

ttaattttaa ttttatgttt	200

<210>	174
<211>	200
<212>	DNA
<213>	Amaranthus palmeri

<400>	174

aaacataaaa ttaaaattaa agcctaaaaa attagattta atcaatgtta aaaaacttaa	60

atcgaaaaaa aaattataaa tgggttaagt gggtcgaaat caggtcaacc tgatctgtcg	120

ctggtcgaat taaggttgtg attttcaagc caattaacta aatgggttgg gtatgggtca	180

agggtttgct gacatgttta	200

<210>	175
<211>	200
<212>	DNA
<213>	Amaranthus palmeri

<400>	175

aggctttaat tttaatttta tgtttttttg tttaatttgt atttactatg aaaaataaga	60

aactattata tgaactgagt ttagcttaca cttattaatt taactcgaaa ttaacacatt	120

taattaaatg gttatatagg gttttttagt tttaaaattt caacctgaac ctaatctatt	180

taataaaaag atcagatctg	200

<210>	176
<211>	200
<212>	DNA
<213>	Amaranthus palmeri

<400>	176

cagatctgat ctttttatta aatagattag gttcaggttg aaattttaaa actaaaaaac	60

cctatataac catttaatta aatgtgttaa tttcgagtta aattaataag tgtaagctaa	120

actcagttca tataatagtt tcttattttt catagtaaat acaaattaaa caaaaaaaca	180

taaaattaaa attaaagcct	200

<210>	177
<211>	200
<212>	DNA
<213>	Amaranthus palmeri

<400>	177

ctatttaata aaaagatcag atctgatcaa cccatataat taattgggtc aaaatctcaa	60

ctcaaaccca tttatttcga attaaattca gatcaaatta gcaggtcgga tcaactagcg	120

ctaatttttc ctctcaagtt ttttcatatg atgccccttg ttttctttga ataaaattca	180

ccaatttaaa atccctgccc	200

<210>	178
<211>	200
<212>	DNA
<213>	Amaranthus palmeri

<400>	178

gggcagggat tttaaattgg tgaattttat tcaaagaaaa caaggggcat catatgaaaa	60

aacttgagag gaaaaattag cgctagttga tccgacctgc taatttgatc tgaatttaat	120

tcgaaataaa tgggtttgag ttgagatttt gacccaatta attatatggg ttgatcagat	180

ctgatctttt tattaaatag	200

<210>	179
<211>	200
<212>	DNA
<213>	Amaranthus palmeri

<400>	179

attcaccaat ttaaaatccc tgcccttgat ccgttggtat tggtatttgg taaagcacat	60

atggtaaatt ctactccatt gttatctgta gttttggttc taaaccccaa acaaatccta	120

ctcctagcac cactaatcct cattactttc tgtttgctgt ctttctctac attcctacta	180

aattaagacc tagatgcagt 200

<210> 180
<211> 200
<212> DNA
<213> Amaranthus palmeri

<400> 180

actgcatcta ggtcttaatt tagtaggaat gtagagaaag acagcaaaca gaaagtaatg 60

aggattagtg gtgctaggag taggatttgt ttggggttta gaaccaaaac tacagataac 120

aatggagtag aatttaccat atgtgcttta ccaaatacca ataccaacgg atcaagggca 180

gggattttaa attggtgaat 200

<210> 181
<211> 200
<212> DNA
<213> Amaranthus palmeri

<400> 181

tactaaatta agacctagat gcagtgttat tctgttcgag gttgccgaaa acggtacaat 60

agttgcctaa tttgcaattc atgtttgttc ttgcgttttc tacattgttt actctattgg 120

aacaaaggtt gaactttcag cctttcaggg agtattttga tttttctgtc aaattacctt 180

gccagattta tttggctatt 200

<210> 182
<211> 200
<212> DNA
<213> Amaranthus palmeri

<400> 182

aatagccaaa taaatctggc aaggtaattt gacagaaaaa tcaaaatact ccctgaaagg 60

ctgaaagttc aacctttgtt ccaatagagt aaacaatgta gaaaacgcaa gaacaaacat 120

gaattgcaaa ttaggcaact attgtaccgt tttcggcaac ctcgaacaga ataacactgc 180

atctaggtct taatttagta 200

<210> 183
<211> 200
<212> DNA
<213> Amaranthus palmeri

<400> 183

accttgccag atttatttgg ctattttgat tgtgtgtatt tctttgtccc ttgtttccat 60

ggggctaaac tccttttggt taatcttaaa catttcttct aattttgaat tgcagtggta 120

tgaagtgttg tttaatatac aatcaatttt tctgaactgg gttaatgaag agaagcattg 180

tagacatgag gaatgtgttc 200

<210> 184
<211> 200
<212> DNA
<213> Amaranthus palmeri

<400> 184

gaacacattc ctcatgtcta caatgcttct cttcattaac ccagttcaga aaaattgatt 60

gtatattaaa caacacttca taccactgca attcaaaatt agaagaaatg tttaagatta 120

accaaaagga gtttagcccc atggaaacaa gggacaaaga aatacacaca atcaaaatag 180

ccaaataaat ctggcaaggt 200

<210> 185
<211> 200
<212> DNA
<213> Amaranthus palmeri

<400> 185

cattgtagac atgaggaatg tgttcaagca tctcaaaaca accaaattaa tcccaaatgg 60

gatcttcaac tgtaatagag gtaaatcaca tatattctca tttaatggtt attgttttat 120

tttaaggatg ttattatgtt gttatcaatg atagaaataa gagaagcttg agcattcaaa 180

tgtatctatc actcactggt 200

<210> 186
<211> 200
<212> DNA
<213> Amaranthus palmeri

<400> 186

accagtgagt gatagataca tttgaatgct caagcttctc ttatttctat cattgataac 60

aacataataa catccttaaa ataaaacaat aaccattaaa tgagaatata tgtgatttac 120

ctctattaca gttgaagatc ccatttggga ttaatttggt tgttttgaga tgcttgaaca 180

cattcctcat gtctacaatg 200

<210> 187
<211> 200
<212> DNA
<213> Amaranthus palmeri

<400> 187

tcaaatgtat ctatcactca ctggttgtgg acatctaaaa tatggaccaa gagttttctt 60

catttcttgt accagaattt gcaaatgtat gattaaagtt taggttggag ttgcaataaa 120

atttcattac cccgacgcta ttaagtggct cacactgagg cggggtttga gggtcggatg 180

gtgcaacctt caggagaggt 200

<210> 188
<211> 200
<212> DNA
<213> Amaranthus palmeri

<400> 188

acctctcctg aaggttgcac catccgaccc tcaaaccccg cctcagtgtg agccacttaa 60

tagcgtcggg gtaatgaaat tttattgcaa ctccaaccta aactttaatc atacatttgc 120

aaattctggt acaagaaatg aagaaaactc ttggtccata ttttagatgt ccacaaccag 180

tgagtgatag atacatttga 200

<210> 189
<211> 200
<212> DNA
<213> Amaranthus palmeri

<400> 189

ggatggtgca accttcagga gaggttctaa gcattttcaa catgttcaac cgcagagggt 60

cccgtaaaat tggaggcttc aatattaaat tatgtagtat gtgttaagtg tttaaagata 120

taaaaatgtt aatgaataca taatttttaa aattgcacag ggctttcaaa gaaatttttg 180

attttgctcc gcccatggca 200

<210> 190
<211> 200
<212> DNA
<213> Amaranthus palmeri

<400> 190

tgccatgggc ggagcaaaat caaaaatttc tttgaaagcc ctgtgcaatt ttaaaaatta 60

tgtattcatt aacattttta tatctttaaa cacttaacac atactacata atttaatatt 120

gaagcctcca attttacggg accctctgcg gttgaacatg ttgaaaatgc ttagaacctc 180

tcctgaaggt tgcaccatcc 200

<210> 191
<211> 200
<212> DNA
<213> Amaranthus palmeri

<400> 191

ttttgatttt gctccgccca tggcaacctt tggcaaatct ggatagaatt agagaaattt 60

acttttatgt atgtataatc tgatgaatga atgaatgaac aatgaagttc atttctgagt 120

caatgaccta tatactatcg aggtttcgtt aaataggata tataaataaa attagagaca 180

tcttttatcc ctaatgttgt 200

<210> 192
<211> 200
<212> DNA
<213> Amaranthus palmeri

<400> 192

acaacattag ggataaaaga tgtctctaat tttatttata tatcctattt aacgaaacct 60

cgatagtata taggtcattg actcagaaat gaacttcatt gttcattcat tcattcatca 120

gattatacat acataaaagt aaatttctct aattctatcc agatttgcca aaggttgcca 180

tgggcggagc aaaatcaaaa 200

<210> 193
<211> 200
<212> DNA
<213> Amaranthus palmeri

<400> 193

agacatcttt tatccctaat gttgtctgtt ttcttttctg ggcgagactg tttttggaga 60

aaaacgttat gttgaatgct tgatttactc agaactgatg caaacagatg gataaaaaat 120

ggtgttttcc tagtgtttat gtttcataaa aggatttcaa attccaatcg tatgaagttt 180

gaaatactgc tttttgtttt 200

<210> 194
<211> 200
<212> DNA
<213> Amaranthus palmeri

<400> 194

aaaacaaaaa gcagtatttc aaacttcata cgattggaat ttgaaatcct tttatgaaac 60

ataaacacta ggaaaacacc attttttatc catctgtttg catcagttct gagtaaatca 120

agcattcaac ataacgtttt tctccaaaaa cagtctcgcc cagaaaagaa aacagacaac 180

attagggata aaagatgtct 200

<210> 195
<211> 200
<212> DNA
<213> Amaranthus palmeri

<400> 195

agtttgaaat actgcttttt gtttttgtag cttcgtgttt tgctttcctt cactcttcac 60

cagggaacgt tgttgaagtt tgttccaaga agcaagaagt cgtaattgca atcgggagca 120

atgttggtga cagattagaa aatttcaacc aagctctgca acaaatgaag aaattaggca 180

tagacatcac aaggcatggt 200

<210> 196
<211> 200
<212> DNA
<213> Amaranthus palmeri

<400> 196

accatgcctt gtgatgtcta tgcctaattt cttcatttgt tgcagagctt ggttgaaatt 60

ttctaatctg tcaccaacat tgctcccgat tgcaattacg acttcttgct tcttggaaca 120

aacttcaaca acgttccctg gtgaagagtg aaggaaagca aaacacgaag ctacaaaaac 180

aaaaagcagt atttcaaact 200

<210> 197
<211> 200
<212> DNA
<213> Amaranthus palmeri

<400> 197

aggcatagac atcacaaggc atggttgttt atatgagacg gaacctgcat acgtgactga 60

tcaaccgaag tttcttaact ctgctttaag aggctttaca agacttggac ctcatgaatt 120

attaggggta ttgaagaaaa ttgagaagga tatgggtaga accaagggaa taaggtatgg 180

tcctaggcca attgacttgg 200

<210> 198
<211> 200
<212> DNA
<213> Amaranthus palmeri

<400> 198

ccaagtcaat tggcctagga ccatacctta ttcccttggt tctacccata tccttctcaa 60

ttttcttcaa tacccctaat aattcatgag gtccaagtct tgtaaagcct cttaaagcag 120

agttaagaaa cttcggttga tcagtcacgt atgcaggttc cgtctcatat aaacaaccat 180

gccttgtgat gtctatgcct 200

<210> 199
<211> 200
<212> DNA
<213> Amaranthus palmeri

<400> 199

tatggtccta ggccaattga cttggacata ctattttatg ggaagtttag ggtgagctct 60

gagagcctca ctatccccca tgaaaggata tgggaaagac catttgtgat ggcaccattg 120

attgattgta ttgggtctga tgtagaaaat gacactattt gtacgtggca ttcattatca 180

aatttttcgg gtggaatctt 200

<210> 200
<211> 200
<212> DNA
<213> Amaranthus palmeri

<400> 200

aagattccac ccgaaaaatt tgataatgaa tgccacgtac aaatagtgtc attttctaca 60

tcagacccaa tacaatcaat caatggtgcc atcacaaatg gtctttccca tatcctttca 120

tgggggatag tgaggctctc agagctcacc ctaaacttcc cataaaatag tatgtccaag 180

tcaattggcc taggaccata 200

<210> 201
<211> 200
<212> DNA
<213> Amaranthus palmeri

<400> 201

tatcaaattt ttcgggtgga atctttgaag catggggtaa actcggtgga agctccctaa 60

tcgggaagga tggaatgaaa agggttttgc ccgttggaaa tctcttatgg gattggtctc 120

ataaaacctc tgttatggga gtcttgaact tgactcctga tagctttagt gatggcggaa 180

gttttcaatc tgtcaatact 200

<210> 202
<211> 200
<212> DNA
<213> Amaranthus palmeri

<400> 202

agtattgaca gattgaaaac ttccgccatc actaaagcta tcaggagtca agttcaagac 60

tcccataaca gaggttttat gagaccaatc ccataagaga tttccaacgg gcaaaaccct 120

tttcattcca tccttcccga ttagggagct tccaccgagt ttaccccatg cttcaaagat 180

tccacccgaa aaatttgata 200

<210> 203
<211> 200
<212> DNA
<213> Amaranthus palmeri

<400> 203

cggaagtttt caatctgtca atactgcggt tgctaaggtt cgtcagatga tctcagatgg 60

ggcagatata attgacatcg gggcacaatc aaccagaccc atggcaacta gaatttcggc 120

tgaagaagag ctagcaagag tagtacctgt attagaagct gtcaaggatt tgatcgagga 180

agaaggaaga atcttgtcag 200

<210> 204
<211> 200
<212> DNA
<213> Amaranthus palmeri

<400> 204

ctgacaagat tcttccttct tcctcgatca aatccttgac agcttctaat acaggtacta 60

ctcttgctag ctcttcttca gccgaaattc tagttgccat gggtctggtt gattgtgccc 120

cgatgtcaat tatatctgcc ccatctgaga tcatctgacg aaccttagca accgcagtat 180

tgacagattg aaaacttccg 200

<210> 205
<211> 200
<212> DNA
<213> Amaranthus palmeri

<400> 205

gaggaagaag gaagaatctt gtcagtggat acgttttatt ctaaagttgc tttggaggcc 60

gtcaagaagg gggcacacat tgtgaatgat gtctctagtg gtaaactcga ttccgagatg 120

tttaatgttg ttgcggacct taaagttcct tatatagcaa tgcacatgcg aggagatccg 180

acttcaatgc aaaactctga 200

<210> 206
<211> 200
<212> DNA
<213> Amaranthus palmeri

<400> 206

tcagagtttt gcattgaagt cggatctcct cgcatgtgca ttgctatata aggaacttta 60

aggtccgcaa caacattaaa catctcggaa tcgagtttac cactagagac atcattcaca 120

atgtgtgccc ccttcttgac ggcctccaaa gcaactttag aataaaacgt atccactgac 180

aagattcttc cttcttcctc 200

<210> 207
<211> 200
<212> DNA
<213> Amaranthus palmeri

<400> 207

atccgacttc aatgcaaaac tctgagaact tgacctacaa tgatgtttgt aagcaagtgg 60

cttcggagtt gagttctagg gtcatagatg cagaattatc gggaattcct gcttggagga 120

tagttattga tcccggcatc ggattttcta agaatacgaa tcaaaatttg gaaattctta 180

gtggtttaca aaagatacgg 200

<210> 208
<211> 200
<212> DNA
<213> Amaranthus palmeri

<400> 208

ccgtatcttt tgtaaaccac taagaatttc caaattttga ttcgtattct tagaaaatcc 60

gatgccggga tcaataacta tcctccaagc aggaattccc gataattctg catctatgac 120

cctagaactc aactccgaag ccacttgctt acaaacatca ttgtaggtca agttctcaga 180

gttttgcatt gaagtcggat 200

<210> 209
<211> 200
<212> DNA
<213> Amaranthus palmeri

<400> 209

tcttagtggt ttacaaaaga tacgggaaga gatagctaag aagagtttgg cggtggctca 60

ttgccccttg ctaattggac cttcaagaaa gaggtttctg ggcgagattt gtgagcgccc 120

tgtagcagct gacagggatc ctgctaccat tgcttctata actgctggag ttttaggtgg 180

tgcaaacatt gtaagagtac 200

<210> 210
<211> 200
<212> DNA
<213> Amaranthus palmeri

<400> 210

gtactcttac aatgtttgca ccacctaaaa ctccagcagt tatagaagca atggtagcag 60

gatccctgtc agctgctaca gggcgctcac aaatctcgcc cagaaacctc tttcttgaag 120

gtccaattag caaggggcaa tgagccaccg ccaaactctt cttagctatc tcttcccgta 180

tcttttgtaa accactaaga 200

<210> 211
<211> 200
<212> DNA
<213> Amaranthus palmeri

<400>       211

ggtggtgcaa acattgtaag agtacataat gttagggata accttgatgc tgtcaagtta    60

tgtgatgcca tactcggaaa aactgattaa ctgcttgttt gtaccacctt gtgaatgatg   120

tctagtggaa ggttcgattg ggattatgat gaagtccgtt agtttgctcg aaagaagagc   180

tttagcccgt aaggttatag                                               200


<210>       212
<211>       200
<212>       DNA
<213>       Amaranthus palmeri

<400>       212

ctataacctt acgggctaaa gctcttcttt cgagcaaact aacggacttc atcataatcc    60

caatcgaacc ttccactaga catcattcac aaggtggtac aaacaagcag ttaatcagtt   120

tttccgagta tggcatcaca taacttgaca gcatcaaggt tatccctaac attatgtact   180

cttacaatgt ttgcaccacc                                               200


<210>       213
<211>       200
<212>       DNA
<213>       Amaranthus palmeri

<400>       213

agagctttag cccgtaaggt tatagttctt acagatgtag aatcattggc tatttgccta    60

tttctgctta gaagatcata gcattgatcc agactttgga ttccatgtag aattatgttg   120

cgactcatgg tcgaattggc cctttgagga attgttgaag ttccttgagt ctatggttca   180

atcggctcgg ttcgatgaag                                               200


<210>       214
<211>       200
<212>       DNA
<213>       Amaranthus palmeri

<400>       214

cttcatcgaa ccgagccgat tgaaccatag actcaaggaa cttcaacaat tcctcaaagg    60

gccaattcga ccatgagtcg caacataatt ctacatggaa tccaaagtct ggatcaatgc   120

tatgatcttc taagcagaaa taggcaaata gccaatgatt ctacatctgt aagaactata   180

accttacggg ctaaagctct                                               200


<210>       215
<211>       200
<212>       DNA
<213>       Amaranthus palmeri

<400>       215

gttcaatcgg ctcggttcga tgaaggttgg actgtgtcga gacaagatgg gtcaaagaga    60

ccaatggaca agaagtcgac cttgggcaag cttgatcaag ttacttggac gagcagataa   120

cagcaggctc tggcgagcat tagacataca tatcatttgt tttttgaagt cagatctgca   180

atcaatcagt tgcaatggac                                               200


<210>       216
<211>       200
<212>       DNA
<213>       Amaranthus palmeri

<400>       216

gtccattgca actgattgat tgcagatctg acttcaaaaa acaaatgata tgtatgtcta    60

atgctcgcca gagcctgctg ttatctgctc gtccaagtaa cttgatcaag cttgcccaag   120

gtcgacttct tgtccattgg tctctttgac ccatcttgtc tcgacacagt ccaaccttca   180

tcgaaccgag ccgattgaac                                               200


<210>       217
<211>       200
<212>       DNA
<213>       Amaranthus palmeri

<400>       217

ctgcaatcaa tcagttgcaa tggacagacc atatacggtt gttagttgtt cctgtcactc    60

tttgagttga attagatcat gcaaacactg taagagtctt gattttgtga agctatgtga   120

tactgtactt ctcaaaatga aatctttgat atgatgttag cttcaagagt tgtttttccc   180

cccctcacat tctgttctat                                               200


<210>       218
<211>       200
<212>       DNA
<213>       Amaranthus palmeri

<400>       218

atagaacaga atgtgagggg gggaaaaaca actcttgaag ctaacatcat atcaaagatt    60

tcattttgag aagtacagta tcacatagct tcacaaaatc aagactctta cagtgtttgc   120

atgatctaat tcaactcaaa gagtgacagg aacaactaac aaccgtatat ggtctgtcca   180

ttgcaactga ttgattgcag                                               200


<210>       219
<211>       200
<212>       DNA

<213>       Amaranthus palmeri

<400>       219

ttccccccct cacattctgt tctatgttgt attgttgtgt gctttttgga attgtattcc    60

tgtaattaga attattcctg caaaatggta ttcctttata tcatcaaagg agtttttgta   120

cttctaatca ttgatcttta gatgcaaaac tgaattaata caagtgttct tgatgaaggc   180

ttattgaaga actcactttt                                               200


<210>       220
<211>       200
<212>       DNA
<213>       Amaranthus palmeri

<400>       220

aaaagtgagt tcttcaataa gccttcatca agaacacttg tattaattca gttttgcatc    60

taaagatcaa tgattagaag tacaaaaact cctttgatga tataaaggaa taccattttg   120

caggaataat tctaattaca ggaatacaat tccaaaaagc acacaacaat acaacataga   180

acagaatgtg aggggggaa                                                200


<210>       221
<211>       200
<212>       DNA
<213>       Amaranthus palmeri

<400>       221

aaggcttatt gaagaactca ctttttattt atgtctaaac ttgttaaaga tgttctgctt    60

ccgcccttgc cacgggatgt cgggattgac taagggtggt aatcgaggga ttcctatgct   120

tgaattgact cgtttaagat tttgcgttga ttttaagtca aatgttttgc taagcataaa   180

ctattctagc tcgacttgaa                                               200


<210>       222
<211>       200
<212>       DNA
<213>       Amaranthus palmeri

<400>       222

ttcaagtcga gctagaatag tttatgctta gcaaaacatt tgacttaaaa tcaacgcaaa    60

atcttaaacg agtcaattca agcataggaa tccctcgatt accaccctta gtcaatcccg   120

acatcccgtg gcaagggcgg aagcagaaca tctttaacaa gtttagacat aaataaaaag   180

tgagttcttc aataagcctt                                               200


<210>       223
<211>       200

<212>       DNA
<213>       Amaranthus palmeri

<400>       223

ataaactatt ctagctcgac ttgaataatg aagttttcga gtcaagtccg agctgttctg    60

taagtttatg tatcttgaag cttcttagtt cttacttctt atccttattt gtttgacaca   120

ttgaaccgcg ttgatgcttg cttgactcaa cccgttaaag tcttgaattc atcacgaatt   180

cgagtcgagc aagtttaaac                                                200


<210>       224
<211>       200
<212>       DNA
<213>       Amaranthus palmeri

<400>       224

gtttaaactt gctcgactcg aattcgtgat gaattcaaga ctttaacggg ttgagtcaag    60

caagcatcaa cgcggttcaa tgtgtcaaac aaataaggat aagaagtaag aactaagaag   120

cttcaagata cataaactta cagaacagct cggacttgac tcgaaaactt cattattcaa   180

gtcgagctag aatagtttat                                                200


<210>       225
<211>       200
<212>       DNA
<213>       Amaranthus palmeri

<400>       225

gaattcgagt cgagcaagtt taaacccaac attaactaag ctctcgaggc ttaatgaggc    60

aggggtagct caagtttgtt attcaagcaa cttaccatag tattctttta aaaggcatat   120

agtcagttag ttatttgtta atttcagtag aacgaaagaa gtacattatt aaacttggcc   180

attgatgctt gagctagaga                                                200


<210>       226
<211>       200
<212>       DNA
<213>       Amaranthus palmeri

<400>       226

tctctagctc aagcatcaat ggccaagttt aataatgtac ttctttcgtt ctactgaaat    60

taacaaataa ctaactgact atatgccttt taaaagaata ctatggtaag ttgcttgaat   120

aacaaacttg agctacccct gcctcattaa gcctcgagag cttagttaat gttgggttta   180

aacttgctcg actcgaattc                                                200


<210>       227

<211>       200
<212>       DNA
<213>       Amaranthus palmeri

<400>       227

tggccattga tgcttgagct agagagcttc ggttgtgatc gaaacgaatc ctgatttcgc    60

tcttgatctt tgatgtgaag tgacaaggcc ttcataaaac tcctttattc ttcagcagtg   120

catacaaaaa tcatgtgtgc ccagctccta gctcacatac cgcgtttttc ccctaatcta   180

gtgtggtcaa ataaaaacag                                               200


<210>       228
<211>       200
<212>       DNA
<213>       Amaranthus palmeri

<400>       228

ctgtttttat ttgaccacac tagattaggg gaaaaacgcg gtatgtgagc taggagctgg    60

gcacacatga tttttgtatg cactgctgaa gaataaagga gttttatgaa ggccttgtca   120

cttcacatca aagatcaaga gcgaaatcag gattcgtttc gatcacaacc gaagctctct   180

agctcaagca tcaatggcca                                               200


<210>       229
<211>       200
<212>       DNA
<213>       Amaranthus palmeri

<400>       229

atctagtgtg gtcaaataaa aacagcaaga tgtgttgatg tcatgtgcaa tagtaattat    60

caggtgaagt aatgcaatat gtagccagtc acctcatatc aaaaaaatgt tgggaggaaa   120

acgtattcgt tcatcactaa acctgtgttt atacatctaa ctgatcgatc agggtttcaa   180

aatccaaatt aatacaatgg                                               200


<210>       230
<211>       200
<212>       DNA
<213>       Amaranthus palmeri

<400>       230

ccattgtatt aatttggatt ttgaaaccct gatcgatcag ttagatgtat aaacacaggt    60

ttagtgatga acgaatacgt tttcctccca acattttttt gatatgaggt gactggctac   120

atattgcatt acttcacctg ataattacta ttgcacatga catcaacaca tcttgctgtt   180

tttatttgac cacactagat                                               200

<210>	231
<211>	200
<212>	DNA
<213>	Amaranthus palmeri

<400>	231

ttcaaaatcc aaattaatac aatggcattt gacgaagata tccagaacac atcccccttg	60

tttctgctag ataatcaact attctgattc tgctgtaata cctcagcttc ggccctagcc	120

atgggatcgt cctctttttc tccttcacgc tcctcggcca gtttctttct gtgcagttct	180

tcagcagctt ctattgaagc	200

<210>	232
<211>	200
<212>	DNA
<213>	Amaranthus palmeri

<400>	232

gcttcaatag aagctgctga agaactgcac agaaagaaac tggccgagga gcgtgaagga	60

gaaaaagagg acgatcccat ggctagggcc gaagctgagg tattacagca gaatcagaat	120

agttgattat ctagcagaaa caagggggat gtgttctgga tatcttcgtc aaatgccatt	180

gtattaattt ggattttgaa	200

<210>	233
<211>	200
<212>	DNA
<213>	Amaranthus rudis

<400>	233

aatgggagtc ttgaacttga ctcctgatag ctttagtgat ggcggaaggt ttcaatctgt	60

cgatactgcg gttgctaagg ttcgtcagat gatctctgac ggggcggaca taattgacat	120

tggggcgcag tcaacaagac ccatggcaac taggatttcg gctgaagaag agctagaaag	180

agtcgtacct gtattagaag	200

<210>	234
<211>	200
<212>	DNA
<213>	Amaranthus rudis

<400>	234

cttctaatac aggtacgact ctttctagct cttcttcagc cgaaatccta gttgccatgg	60

gtcttgttga ctgcgcccca atgtcaatta tgtccgcccc gtcagagatc atctgacgaa	120

ccttagcaac cgcagtatcg acagattgaa accttccgcc atcactaaag ctatcaggag	180

tcaagttcaa gactcccatt	200

<210>	235
<211>	200
<212>	DNA
<213>	Amaranthus rudis

<400>	235

gaaagagtcg tacctgtatt agaagctgtc aaggatttga tcgaggaaga aggaagaatc	60

ttgtcagtgg atacgtttta ttctaaagtt gcttcggagg ccgtcaagaa gggggcacac	120

attgtgaatg atgtctctag tgggaaactc gatcccgaga tgtttaatgt tgttgcggac	180

cttaaagttc cttatatagc	200

<210>	236
<211>	200
<212>	DNA
<213>	Amaranthus rudis

<400>	236

gctatataag gaactttaag gtccgcaaca acattaaaca tctcgggatc gagtttccca	60

ctagagacat cattcacaat gtgtgccccc ttcttgacgg cctccgaagc aactttagaa	120

taaaacgtat ccactgacaa gattcttcct tcttcctcga tcaaatcctt gacagcttct	180

aatacaggta cgactctttc	200

<210>	237
<211>	200
<212>	DNA
<213>	Amaranthus rudis

<400>	237

cggaccttaa agttccttat atagcaatgc acatgcgagg agatccgact tcaatgcaaa	60

attctgagaa cttgacgtac aatgatgtgt gtaagcaagt ggcttcggag ttgagctcta	120

gggtcgtaga tgcagaatta tcgggaattc ctgcttggag gatggttatt gatcccggca	180

ttggattttc taagaatacg	200

<210>	238
<211>	200
<212>	DNA
<213>	Amaranthus rudis

<400>	238

cgtattctta gaaaatccaa tgccgggatc aataaccatc ctccaagcag gaattcccga	60

taattctgca tctacgaccc tagagctcaa ctccgaagcc acttgcttac acacatcatt	120

gtacgtcaag ttctcagaat tttgcattga agtcggatct cctcgcatgt gcattgctat	180

ataaggaact ttaaggtccg	200

<210>	239
<211>	200
<212>	DNA
<213>	Amaranthus rudis

<400>	239

cggcattgga ttttctaaga atacgaagca aaatttggaa attcttagtg gtttacaaaa	60

gatacggcaa gagatagcta agaagagttt ggcggtggct cattgcccct tgctaattgg	120

accttcaaga aagaggtttc tgggcgagat ttgcaatcgc cctgtagcag ctgataggga	180

tcctgctacc gttgcttcta	200

<210>	240
<211>	200
<212>	DNA
<213>	Amaranthus rudis

<400>	240

tagaagcaac ggtagcagga tccctatcag ctgctacagg gcgattgcaa atctcgccca	60

gaaacctctt tcttgaaggt ccaattagca aggggcaatg agccaccgcc aaactcttct	120

tagctatctc ttgccgtatc ttttgtaaac cactaagaat ttccaaattt tgcttcgtat	180

tcttagaaaa tccaatgccg	200

<210>	241
<211>	200
<212>	DNA
<213>	Amaranthus rudis

<400>	241

aggatttcgg ctgaagaaga gctagaaaga gttgtacctg tattggaagc tgttaaggat	60

gtgatcgagg aagaaggaag aatcttatcg gtggatacgt tttattctaa agttgcttcg	120

gaggccgtca agaagggggc acacattgtg aatgatgttt ctagtgggaa actcgatccc	180

gagatgttta atgttgttgc	200

<210>	242
<211>	200
<212>	DNA
<213>	Amaranthus rudis

<400>	242

gcaacaacat taaacatctc gggatcgagt ttcccactag aaacatcatt cacaatgtgt	60

gcccccttct tgacggcctc cgaagcaact ttagaataaa acgtatccac cgataagatt	120

cttccttctt cctcgatcac atccttaaca gcttccaata caggtacaac tctttctagc	180

tcttcttcag ccgaaatcct 200

<210> 243
<211> 200
<212> DNA
<213> Amaranthus rudis

<400> 243

atcccgagat gtttaatgtt gttgcggacc ttaaagttcc ttatatagca atgcacatgc 60

gaggagatcc gacttcaatg caaaattctg agaacttgac gtacaatgat gtgtgtaagc 120

aagtggcttc ggagttgagc tctagggtcg tagatgcaga attatcggga attcctgctt 180

ggaggatggt tattgatccc 200

<210> 244
<211> 200
<212> DNA
<213> Amaranthus rudis

<400> 244

gggatcaata accatcctcc aagcaggaat tcccgataat tctgcatcta cgaccctaga 60

gctcaactcc gaagccactt gcttacacac atcattgtac gtcaagttct cagaattttg 120

cattgaagtc ggatctcctc gcatgtgcat tgctatataa ggaactttaa ggtccgcaac 180

aacattaaac atctcgggat 200

<210> 245
<211> 200
<212> DNA
<213> Amaranthus rudis

<400> 245

tgcttggagg atggttattg atcccggcat tggattttct aagaatacga agcaaaattt 60

ggaaattctt agtggtttac aaaagatacg gcaagagata gctaagaaga gtttggcggt 120

ggctcattgc cccttgctaa ttggaccttc aagaaagaag gtttctgggc gagatttgca 180

atcgccctgt agcagctgat 200

<210> 246
<211> 200
<212> DNA
<213> Amaranthus rudis

<400> 246

atcagctgct acagggcgat tgcaaatctc gcccagaaac cttctttctt gaaggtccaa 60

ttagcaaggg gcaatgagcc accgccaaac tcttcttagc tatctcttgc cgtatctttt 120

gtaaaccact aagaatttcc aaattttgct tcgtattctt agaaaatcca atgccgggat 180

caataaccat cctccaagca                                              200

<210>   247
<211>   200
<212>   DNA
<213>   Amaranthus rudis

<400>   247

attagaggca tcttttatcc ctaatgctgt ccgttttctt ttctgggtga gactattttg    60

gaagaaaaaa atttatgttc aatgtttaat ttactcagaa ttgatgcaaa cagatggata   120

aaaatggtgt tttcttatgg tttatgtttc aataaaagga tttcaaattc taatcatatg   180

aagtttgaaa tactatgtgt                                              200

<210>   248
<211>   200
<212>   DNA
<213>   Amaranthus rudis

<400>   248

acacatagta tttcaaactt catatgatta gaatttgaaa tccttttatt gaaacataaa    60

ccataagaaa acaccatttt tatccatctg tttgcatcaa ttctgagtaa attaaacatt   120

gaacataaat ttttttcttc caaaatagtc tcacccagaa aagaaaacgg acagcattag   180

ggataaaaga tgcctctaat                                              200

<210>   249
<211>   200
<212>   DNA
<213>   Amaranthus rudis

<400>   249

atatgaagtt tgaaatacta tgtgtttttg tagcttcttg tattgctttc cttcactcat    60

caccagtgaa cgatgttgaa gtttgttcca agaagcaaga agtcgtaatt gcaatcggga   120

gcaatgttgg tgacagatta gaaaatttca acgaagctct gcaacaaatg aagaaattag   180

gcatagacat cacaaggcat                                              200

<210>   250
<211>   200
<212>   DNA
<213>   Amaranthus rudis

<400>   250

atgccttgtg atgtctatgc ctaatttctt catttgttgc agagcttcgt tgaaattttc    60

taatctgtca ccaacattgc tcccgattgc aattacgact tcttgcttct tggaacaaac   120

ttcaacatcg ttcactggtg atgagtgaag gaaagcaata caagaagcta caaaaacaca 180

tagtatttca aacttcatat 200

<210> 251
<211> 200
<212> DNA
<213> Amaranthus rudis

<400> 251

attaggcata gacatcacaa ggcatggttg tttatacgag acggaacctg cttacgtgac 60

tgatcagccg aagtttctta actctgcttt aagaggcttt acaagacttg ggcctcatga 120

attattaggg gtattgaaga gaatcgagaa ggatatgggc agaaccaagg gaataaggta 180

tggtcctagg ccaattgact 200

<210> 252
<211> 200
<212> DNA
<213> Amaranthus rudis

<400> 252

agtcaattgg cctaggacca taccttattc ccttggttct gcccatatcc ttctcgattc 60

tcttcaatac ccctaataat tcatgaggcc caagtcttgt aaagcctctt aaagcagagt 120

taagaaactt cggctgatca gtcacgtaag caggttccgt ctcgtataaa caaccatgcc 180

ttgtgatgtc tatgcctaat 200

<210> 253
<211> 200
<212> DNA
<213> Amaranthus rudis

<400> 253

cgctactgct gcagctgtca ccgctgcaat tttgaatggt gctaacatag taagggtaca 60

taatgtagga tatagttcag atgctgcaaa gttttgtgat gcacctatac tcattttacc 120

catctcgctc ctaatggacc ccagcccctt aataacgtca atgttgtgcc cacatttctt 180

ggagaaccca atgccaggat 200

<210> 254
<211> 200
<212> DNA
<213> Amaranthus rudis

<400> 254

atcctggcat tgggttctcc aagaaatgtg ggcacaacat tgacgttatt aaggggctgg 60

ggtccattag gagcgagatg ggtaaaatga gtataggtgc atcacaaaac tttgcagcat 120

ctgaactata tcctacatta tgtaccctta ctatgttagc accattcaaa attgcagcgg 180

tgacagctgc agcagtagcg 200

<210> 255
<211> 200
<212> DNA
<213> Amaranthus rudis

<400> 255

ctgagagcct cactattccc catgaaagga tatgggaaag accatttgtg atggcaccat 60

taattgattg tattgggtct gatgtagaaa atgacactat ttgtacatgg cattcgttat 120

caaatttttc gggtggaata tttgaagcat gggataaact cggaggaagt tccttaatcg 180

ggaaggatgg aatgaaaagg 200

<210> 256
<211> 200
<212> DNA
<213> Amaranthus rudis

<400> 256

ccttttcatt ccatccttcc cgattaagga acttcctccg agtttatccc atgcttcaaa 60

tattccaccc gaaaaatttg ataacgaatg ccatgtacaa atagtgtcat tttctacatc 120

agacccaata caatcaatta atggtgccat cacaaatggt ctttcccata tcctttcatg 180

gggaatagtg aggctctcag 200

<210> 257
<211> 200
<212> DNA
<213> Amaranthus rudis

<400> 257

aatcgggaag gatggaatga aaagggtttt gcccgttggg aaccgtttat gggattggtc 60

tcataaaacc tcagtaatgg gagtcttgaa cttgactcct gatagcttta gtgatggcgg 120

aaggtttcaa tctgtcgata ctgcggttgc taaggttcgt cagatgatct ctgacggggc 180

ggacataatt gacattgggg 200

<210> 258
<211> 200
<212> DNA
<213> Amaranthus rudis

<400> 258

ccccaatgtc aattatgtcc gccccgtcag agatcatctg acgaacctta gcaaccgcag 60

tatcgacaga ttgaaacctt ccgccatcac taaagctatc aggagtcaag ttcaagactc 120

ccattactga ggttttatga gaccaatccc ataaacggtt cccaacgggc aaaacccttt 180

tcattccatc cttcccgatt 200

<210> 259
<211> 200
<212> DNA
<213> Amaranthus rudis

<400> 259

gcgtaccttc actcatcacc agtggacgat gttgaacttt gttctaagaa gcaagaagtc 60

ataattgcaa tcgggagcaa tgttggtgac agattagaaa attttaacca agctctgcaa 120

caaatgaaga aattaggcat agacatcaca aggcatggtt gtttatatga gacagaaccc 180

gcatacgtga ctgatcaacc 200

<210> 260
<211> 200
<212> DNA
<213> Amaranthus rudis

<400> 260

ggttgatcag tcacgtatgc gggttctgtc tcatataaac aaccatgcct tgtgatgtct 60

atgcctaatt tcttcatttg ttgcagagct tggttaaaat tttctaatct gtcaccaaca 120

ttgctcccga ttgcaattat gacttcttgc ttcttagaac aaagttcaac atcgtccact 180

ggtgatgagt gaaggtacgc 200

<210> 261
<211> 200
<212> DNA
<213> Amaranthus rudis

<400> 261

aacccgcata cgtgactgat caaccgaagt ttcttaactc tgctttaaga ggctttacaa 60

gacttgggcc tcatgaatta ttaggggtat tgaagaaaat tgagaagaat atgggtagaa 120

ccaagggaat aaggtatggt cctaggccaa ttgacttgga catactattt tatgggaagt 180

ttagggtgag ctctgagagc 200

<210> 262
<211> 200
<212> DNA
<213> Amaranthus rudis

<400> 262

gctctcagag ctcaccctaa acttcccata aaatagtatg tccaagtcaa ttggcctagg 60

accatacctt attcccttgg ttctacccat attcttctca attttcttca atacccctaa 120

taattcatga ggcccaagtc ttgtaaagcc tcttaaagca gagttaagaa acttcggttg 180

atcagtcacg tatgcgggtt 200

<210> 263
<211> 200
<212> DNA
<213> Amaranthus rudis

<400> 263

gaagtttagg gtgagctctg agagcctcac tattccccat gaaaggatat gggaaagacc 60

atttgtgatg gcaccattaa ttgattgtat tgggtctggt gtagaaaatg acactatttg 120

tacatggcat tcgttatcaa atttttcggg tggaatcttt gaagcatggg ataaactcgg 180

tggaagttcc ctaatcggga 200

<210> 264
<211> 200
<212> DNA
<213> Amaranthus rudis

<400> 264

tcccgattag ggaacttcca ccgagtttat cccatgcttc aaagattcca cccgaaaaat 60

ttgataacga atgccatgta caaatagtgt cattttctac accagaccca atacaatcaa 120

ttaatggtgc catcacaaat ggtctttccc atatcctttc atggggaata gtgaggctct 180

cagagctcac cctaaacttc 200

<210> 265
<211> 200
<212> DNA
<213> Amaranthus rudis

<400> 265

ctcggtggaa gttccctaat cgggaaggat ggaatgaaaa gggttttgcc cgttgggaac 60

cgcttatggg attggtctca taaaacctct gtaatgggag tcttgaactt gactcctgat 120

agctttagtg atggaggaag ttttcaatct gtcgatactg cggttgctaa ggttcgtcag 180

atgatatcag atggggccga 200

<210> 266
<211> 200
<212> DNA
<213> Amaranthus rudis

<400> 266

tcggccccat ctgatatcat ctgacgaacc ttagcaaccg cagtatcgac agattgaaaa 60

cttcctccat cactaaagct atcaggagtc aagttcaaga ctcccattac agaggtttta 120

tgagaccaat cccataagcg gttcccaacg ggcaaaaccc ttttcattcc atccttcccg 180

attagggaac ttccaccgag 200

<210> 267
<211> 200
<212> DNA
<213> Amaranthus rudis

<400> 267

gtcagatgat atcagatggg gccgacataa ttgacattgg ggcacagtca acaagaccca 60

tggcaactag gatttcggct gaagaagagc tagaaagagt tgtacctgta ttggaagctg 120

ttaaggatgt gatcgaggaa gaaggaagaa tcttatcggt ggatactttt tattcaaaag 180

ttgcttctga ggccgtcaag 200

<210> 268
<211> 200
<212> DNA
<213> Amaranthus rudis

<400> 268

cttgacggcc tcagaagcaa cttttgaata aaaagtatcc accgataaga ttcttccttc 60

ttcctcgatc acatccttaa cagcttccaa tacaggtaca actctttcta gctcttcttc 120

agccgaaatc ctagttgcca tgggtcttgt tgactgtgcc ccaatgtcaa ttatgtcggc 180

cccatctgat atcatctgac 200

<210> 269
<211> 200
<212> DNA
<213> Amaranthus rudis

<400> 269

aaaagttgct tctgaggccg tcaagaaggg ggcacacatt gtgaatgatg tctctagtgg 60

gaaattcgat cctgagatgt tcaatgttgt tgcggacctt aaagttcctt atatagcaat 120

gcacatgcga ggagatccga cttcaatgca aaattctgag aacttgacgt acagtgatgt 180

ttgtaagcaa gtggcttcgg 200

<210> 270
<211> 200
<212> DNA
<213> Amaranthus rudis

<400> 270

ccgaagccac ttgcttacaa acatcactgt acgtcaagtt ctcagaattt tgcattgaag 60

tcggatctcc tcgcatgtgc attgctatat aaggaacttt aaggtccgca acaacattga 120

acatctcagg atcgaatttc ccactagaga catcattcac aatgtgtgcc cccttcttga 180

cggcctcaga agcaactttt 200

<210> 271
<211> 200
<212> DNA
<213> Amaranthus rudis

<400> 271

gatgtttgta agcaagtggc ttcggagttg agctctaggg tcatagatgc agaattatcg 60

ggaattcctg cttggagaat agttattgat cccggcattg gattttctaa gaatacgaag 120

caaaatttgg aaattcttac tggtttacaa aagatacggc tagagatagc taagaagagt 180

ttggcggtgg ctcattgccc 200

<210> 272
<211> 200
<212> DNA
<213> Amaranthus rudis

<400> 272

gggcaatgag ccaccgccaa actcttctta gctatctcta gccgtatctt ttgtaaacca 60

gtaagaattt ccaaattttg cttcgtattc ttagaaaatc caatgccggg atcaataact 120

attctccaag caggaattcc cgataattct gcatctatga ccctagagct caactccgaa 180

gccacttgct tacaaacatc 200

<210> 273
<211> 200
<212> DNA
<213> Amaranthus rudis

<400> 273

agagtttggc ggtggctcat tgccccttgc taattggacc ttcacgaaag aggtttctgg 60

gtgagatttg taatcgccct gtagcagctg atagggatcc tgctaccgtc gcttctatca 120

ctgctggagt tttaggtggt gcaaacattg taagagtaca taatgtgagg gataaccttg 180

atgcagtcaa gttatgtgat 200

<210> 274
<211> 200
<212> DNA
<213> Amaranthus rudis

<400>       274

atcacataac ttgactgcat caaggttatc cctcacatta tgtactctta caatgtttgc    60

accacctaaa actccagcag tgatagaagc gacggtagca ggatccctat cagctgctac   120

agggcgatta caaatctcac ccagaaacct ctttcgtgaa ggtccaatta gcaaggggca   180

atgagccacc gccaaactct                                               200


<210>       275
<211>       200
<212>       DNA
<213>       Amaranthus rudis

<400>       275

gtaatgggag tcttgaactt gactcctgat agctttagtg atggcggaag gtttcaatct    60

gtcgatactg cggttgctaa ggttcgtcag atgatctctg acggggcgga cataattgac   120

attggggcgc agtcaacaag acccttggca actaggattt cggctgaaga agagctagaa   180

agagtcgtac ctgtattaga                                               200


<210>       276
<211>       200
<212>       DNA
<213>       Amaranthus rudis

<400>       276

tctaatacag gtacgactct ttctagctct tcttcagccg aaatcctagt tgccaagggt    60

cttgttgact gcgccccaat gtcaattatg tccgccccgt cagagatcat ctgacgaacc   120

ttagcaaccg cagtatcgac agattgaaac cttccgccat cactaaagct atcaggagtc   180

aagttcaaga ctcccattac                                               200


<210>       277
<211>       200
<212>       DNA
<213>       Amaranthus rudis

<400>       277

tagaaagagt cgtacctgta ttagaagctg tcaaggattt gatcgaggaa gaaggaagaa    60

tcttgtcagt ggatacgttt tattctaaag ttgcttcgga ggccgtcaag aagggggcac    120

acattgtgaa tgatgtttct agtgggaaac tcgatcccga gatgtttaat gttgttgcgg   180

accttaaagt tccttatata                                               200


<210>       278
<211>       200
<212>       DNA
<213>       Amaranthus rudis

<400>       278

tatataagga actttaaggt ccgcaacaac attaaacatc tcgggatcga gtttcccact    60

agaaacatca ttcacaatgt gtgccccctt cttgacggcc tccgaagcaa ctttagaata   120

aaacgtatcc actgacaaga ttcttccttc ttcctcgatc aaatccttga cagcttctaa   180

tacaggtacg actctttcta                                               200


<210>       279
<211>       200
<212>       DNA
<213>       Amaranthus rudis

<400>       279

tgcggacctt aaagttcctt atatagcaat gcacatgcga ggagatccga cttcaatgca    60

aaattctgag aacttgacgt acagtgatgt ttgtaagcaa gtggcttcgg agttgagctc   120

tagggtcata gatgcagaat tatcgggaat tcctgcttgg agaatagtta ttgatcccgg   180

cattggattt tctaagaata                                               200


<210>       280
<211>       200
<212>       DNA
<213>       Amaranthus rudis

<400>       280

tattcttaga aaatccaatg ccgggatcaa taactattct ccaagcagga attcccgata    60

attctgcatc tatgacccta gagctcaact ccgaagccac ttgcttacaa acatcactgt   120

acgtcaagtt ctcagaattt tgcattgaag tcggatctcc tcgcatgtgc attgctatat   180

aaggaacttt aaggtccgca                                               200


<210>       281
<211>       200
<212>       DNA
<213>       Amaranthus rudis

<400>       281

cccggcattg gattttctaa gaatacgaag caaaatttgg aaattcttac tggtttacaa    60

aagatacggc tagagatagc taagaagagt ttggcggtgg ctcattgccc cttgctaatt   120

ggaccttcac gaaagaggtt tctgggtgag atttgtaatc gccctgtagc agctgatagg   180

gatcctgcta ccgtcgcttc                                               200


<210>       282
<211>       200
<212>       DNA

<213>       Amaranthus rudis

<400>       282

gaagcgacgg tagcaggatc cctatcagct gctacagggc gattacaaat ctcacccaga    60

aacctctttc gtgaaggtcc aattagcaag gggcaatgag ccaccgccaa actcttctta   120

gctatctcta gccgtatctt ttgtaaacca gtaagaattt ccaaattttg cttcgtattc   180

ttagaaaatc caatgccggg                                               200


<210>       283
<211>       200
<212>       DNA
<213>       Amaranthus rudis

<400>       283

tgatttactc agaattgatg caaacaaatg gataaaaatg gtgttttcct atggtttatg    60

tttcgataaa aggatttcaa atcccaatcg tatgaagttt gaaatacaac tatgtgtttt   120

tgtagcttct tgtattgctt tccttcactc atcaccagtg aacgatgttg aagtttgttc   180

caagaagcaa gaagtcgtaa                                               200


<210>       284
<211>       200
<212>       DNA
<213>       Amaranthus rudis

<400>       284

ttacgacttc ttgcttcttg gaacaaactt caacatcgtt cactggtgat gagtgaagga    60

aagcaataca agaagctaca aaaacacata gttgtatttc aaacttcata cgattgggat   120

ttgaaatcct tttatcgaaa cataaaccat aggaaaacac catttttatc catttgtttg   180

catcaattct gagtaaatca                                               200


<210>       285
<211>       200
<212>       DNA
<213>       Amaranthus rudis

<400>       285

tgttccaaga agcaagaagt cgtaattgca atcgggagca atgttggtga cagattagaa    60

aatttcaacg aagctctgca acaaatgaag aaattaggca tagacatcac aaggcatggt   120

tgtttatacg agacggaacc tgcttacgtg actgatcagc cgaagtttct taactctgct   180

ttaagaggct ttacaagact                                               200


<210>       286
<211>       200

<212>       DNA
<213>       Amaranthus rudis

<400>       286

agtcttgtaa agcctcttaa agcagagtta agaaacttcg gctgatcagt cacgtaagca    60

ggttccgtct cgtataaaca accatgcctt gtgatgtcta tgcctaattt cttcatttgt   120

tgcagagctt cgttgaaatt ttctaatctg tcaccaacat tgctcccgat tgcaattacg   180

acttcttgct tcttggaaca                                               200


<210>       287
<211>       200
<212>       DNA
<213>       Amaranthus rudis

<400>       287

ctgctttaag aggctttaca agacttgggc ctcatgaatt attaggggta ttgaagagaa    60

tcgagaagga tatgggcaga accaagggaa taaggtatgg tcctaggcca attgacttgg   120

acatactatt ttatgggaag tttagggtga gctctgagag cctcactatt ccccatgaaa   180

ggatatggga aagaccattt                                               200


<210>       288
<211>       200
<212>       DNA
<213>       Amaranthus rudis

<400>       288

aaatggtctt tcccatatcc tttcatgggg aatagtgagg ctctcagagc tcaccctaaa    60

cttcccataa aatagtatgt ccaagtcaat tggcctagga ccatacctta ttcccttggt   120

tctgcccata tccttctcga ttctcttcaa tacccctaat aattcatgag gcccaagtct   180

tgtaaagcct cttaaagcag                                               200


<210>       289
<211>       200
<212>       DNA
<213>       Amaranthus rudis

<400>       289

tggcaactag gatttcggct gaagaagagc tagaaagagt cgtacctgta ttagaagctg    60

tcaaggattt gatcgaggaa gaaggaagaa tcttgtcagt ggatacgttt tattctaaag   120

ttgcttcgga ggccgtcaag aagggggcac acattgtgaa tgatgtttct agtgggaaac   180

tcgatcccga gatgtttaat                                               200


<210>       290

<211>	200
<212>	DNA
<213>	Amaranthus rudis

<400>	290

attaaacatc tcgggatcga gtttcccact agaaacatca ttcacaatgt gtgccccctt	60

cttgacggcc tccgaagcaa ctttagaata aaacgtatcc actgacaaga ttcttccttc	120

ttcctcgatc aaatccttga cagcttctaa tacaggtacg actctttcta gctcttcttc	180

agccgaaatc ctagttgcca	200

<210>	291
<211>	200
<212>	DNA
<213>	Amaranthus rudis

<400>	291

gaaactcgat cccgagatgt ttaatgttgt tgcggacctt aaagttcctt atatagcaat	60

gcacatgcga ggagatccga cttcaatgca aaattctgag aacttgacgt acaatgatgt	120

gtgtaagcaa gtggcttcgg agttgagctc tagggtcgta gatgcagaat tatcgggaat	180

tcctgcttgg aggatggtta	200

<210>	292
<211>	200
<212>	DNA
<213>	Amaranthus rudis

<400>	292

taaccatcct ccaagcagga attcccgata attctgcatc tacgacccta gagctcaact	60

ccgaagccac ttgcttacac acatcattgt acgtcaagtt ctcagaattt tgcattgaag	120

tcggatctcc tcgcatgtgc attgctatat aaggaacttt aaggtccgca acaacattaa	180

acatctcggg atcgagtttc	200

<210>	293
<211>	200
<212>	DNA
<213>	Amaranthus rudis

<400>	293

ggaattcctg cttggaggat ggttattgat cccggcattg gattttctaa gaatacgaag	60

caaaatttgg aaattcttag tggtttacaa aagatacggc aagagatagc taagaagagt	120

ttggcggtgg ctcattgccc cttgctaatt ggaccttcaa gaaagaggtt tctgggcgag	180

atttgcaatc gccctgtagc	200

<210>	294
<211>	200
<212>	DNA
<213>	Amaranthus rudis

<400>	294

gctacagggc gattgcaaat ctcgcccaga aacctctttc ttgaaggtcc aattagcaag	60

gggcaatgag ccaccgccaa actcttctta gctatctctt gccgtatctt ttgtaaacca	120

ctaagaattt ccaaattttg cttcgtattc ttagaaaatc caatgccggg atcaataacc	180

atcctccaag caggaattcc	200

<210>	295
<211>	200
<212>	DNA
<213>	Amaranthus rudis

<400>	295

gcgagatttg caatcgccct gtagcagctg atagggatcc tgctaccgtt gcttctatca	60

ctgctggagt tttaggtggt gcaaacattg taagagtaca taatgtgagg gataaccttg	120

atgcagtcaa gttatgtgat gccatactca gaaaaacgga ttagccgatt tttttggatt	180

ccagagccga tgttccagat	200

<210>	296
<211>	200
<212>	DNA
<213>	Amaranthus rudis

<400>	296

atctggaaca tcggctctgg aatccaaaaa aatcggctaa tccgtttttc tgagtatggc	60

atcacataac ttgactgcat caaggttatc cctcacatta tgtactctta caatgtttgc	120

accacctaaa actccagcag tgatagaagc aacggtagca ggatccctat cagctgctac	180

agggcgattg caaatctcgc	200

<210>	297
<211>	200
<212>	DNA
<213>	Amaranthus rudis

<400>	297

tgtttcgata aaaggatttc aaatcccaat cgtatgaagt ttgaaataca actatgtgtt	60

tttgcagctt tgttttttgc gtaccttcac tcatcaccag tggacgatgt tgaactttgt	120

tctaagaagc aagaagtcat aattgcaatc gggagcaatg ttggtgacag attagaaaat	180

tttaaccaag ctctgcaaca	200

<210>	298
<211>	200
<212>	DNA
<213>	Amaranthus rudis

<400>	298

tgttgcagag cttggttaaa attttctaat ctgtcaccaa cattgctccc gattgcaatt	60

atgacttctt gcttcttaga acaaagttca acatcgtcca ctggtgatga gtgaaggtac	120

gcaaaaaaca aagctgcaaa aacacatagt tgtatttcaa acttcatacg attgggattt	180

gaaatccttt tatcgaaaca	200

<210>	299
<211>	200
<212>	DNA
<213>	Amaranthus rudis

<400>	299

aaaattttaa ccaagctctg caacaaatga agaaattagg catagacatc acaaggcatg	60

gttgtttata tgagacagaa cccgcatacg tgactgatca accgaagttt cttaactctg	120

ctttaagagg ctttacaaga cttgggcctc atgaattatt aggggtattg aagaaaattg	180

agaagaatat gggtagaacc	200

<210>	300
<211>	200
<212>	DNA
<213>	Amaranthus rudis

<400>	300

ggttctaccc atattcttct caattttctt caatacccct aataattcat gaggcccaag	60

tcttgtaaag cctcttaaag cagagttaag aaacttcggt tgatcagtca cgtatgcggg	120

ttctgtctca tataaacaac catgccttgt gatgtctatg cctaatttct tcatttgttg	180

cagagcttgg ttaaaatttt	200

<210>	301
<211>	200
<212>	DNA
<213>	Amaranthus rudis

<400>	301

aattgagaag aatatgggta gaaccaaggg aataaggtat ggtcctaggc caattgactt	60

ggacatacta ttttatggga agtttagggt gagctctgag agcctcacta ttccccatga	120

aaggatatgg gaaagaccat ttgtgatggc accattaatt gattgtattg ggtctggtgt	180

agaaaatgac actatttgta	200

<210>	302
<211>	200
<212>	DNA
<213>	Amaranthus rudis

<400>	302

tacaaatagt gtcattttct acaccagacc caatacaatc aattaatggt gccatcacaa	60

atggtctttc ccatatcctt tcatggggaa tagtgaggct ctcagagctc accctaaact	120

tcccataaaa tagtatgtcc aagtcaattg gcctaggacc ataccttatt cccttggttc	180

tacccatatt cttctcaatt	200

<210>	303
<211>	200
<212>	DNA
<213>	Amaranthus rudis

<400>	303

ggtgtagaaa atgacactat ttgtacatgg cattcgttat caaatttttc gggtggaatc	60

tttgaagcat gggataaact cggtggaagt tccctaatcg ggaaggatgg aatgaaaagg	120

gttttgcccg ttgggaaccg cttatgggat tggtctcata aaacctctgt aatgggagtc	180

ttgaacttga ctcctgatag	200

<210>	304
<211>	200
<212>	DNA
<213>	Amaranthus rudis

<400>	304

ctatcaggag tcaagttcaa gactcccatt acagaggttt tatgagacca atcccataag	60

cggttcccaa cgggcaaaac ccttttcatt ccatccttcc cgattaggga acttccaccg	120

agtttatccc atgcttcaaa gattccaccc gaaaaatttg ataacgaatg ccatgtacaa	180

atagtgtcat tttctacacc	200

<210>	305
<211>	200
<212>	DNA
<213>	Amaranthus rudis

<400>	305

gagtcttgaa cttgactcct gatagcttta gtgatggagg aagttttcaa tctgtcgata	60

ctgcggttgc taaggttcgt cagatgatat cagatggggc cgacataatt gacattgggg	120

cacagtcaac aagacccatg gcaactagga tttcggctga agaagagcta gaaagagttg	180

tacctgtatt ggaagctgtt 200

<210> 306
<211> 200
<212> DNA
<213> Amaranthus rudis

<400> 306

aacagcttcc aatacaggta caactctttc tagctcttct tcagccgaaa tcctagttgc 60

catgggtctt gttgactgtg ccccaatgtc aattatgtcg gccccatctg atatcatctg 120

acgaacctta gcaaccgcag tatcgacaga ttgaaaactt cctccatcac taaagctatc 180

aggagtcaag ttcaagactc 200

<210> 307
<211> 200
<212> DNA
<213> Amaranthus viridis

<400> 307

gcagctgata gggatcctgc taccgttgct tctataactg ctggagtttt aggtggtgca 60

aacattgtaa gagtacataa tgtgagggat aaccttgatg ctgtcaagtt atgtgatgcc 120

atactcggaa aaactgatta actgcttatt tgtaccacct tgtgaatgac gtctagtgga 180

aagttcgatt gggatatgat 200

<210> 308
<211> 200
<212> DNA
<213> Amaranthus viridis

<400> 308

atcatatccc aatcgaactt tccactagac gtcattcaca aggtggtaca aataagcagt 60

taatcagttt ttccgagtat ggcatcacat aacttgacag catcaaggtt atccctcaca 120

ttatgtactc ttacaatgtt tgcaccacct aaaactccag cagttataga agcaacggta 180

gcaggatccc tatcagctgc 200

<210> 309
<211> 200
<212> DNA
<213> Amaranthus viridis

<400> 309

gtggaaagtt cgattgggat atgatgaagt ccgttagttt gctcgaaagt agagctttag 60

cccgtaaggt tatagttctt acagatgtag aatcattggc tatttgccta tttttgctta 120

gaagatcata gcattgatcc agactttgga ttccatctag aattatgttg cgactcatgg 180

tcgaattggc cctttgagga                                                200

<210>    310
<211>    200
<212>    DNA
<213>    Amaranthus viridis

<400>    310

tcctcaaagg gccaattcga ccatgagtcg caacataatt ctagatggaa tccaaagtct    60

ggatcaatgc tatgatcttc taagcaaaaa taggcaaata gccaatgatt ctacatctgt   120

aagaactata accttacggg ctaaagctct actttcgagc aaactaacgg acttcatcat   180

atcccaatcg aactttccac                                                200

<210>    311
<211>    200
<212>    DNA
<213>    Amaranthus viridis

<400>    311

catggtcgaa ttggcccttt gaggaattgt tgaagttcct tgatctatgg ttcaatcggc    60

ttggttcgat gaaggttgga ctgtgtcgag acaagatggg tcaaagagac caatggacaa   120

gaagtcgacc ttgggaaagc ttgatcaagt tacttggacg agcagatagc agcaggactc   180

tggcgagcat tagacataca                                                200

<210>    312
<211>    200
<212>    DNA
<213>    Amaranthus viridis

<400>    312

tgtatgtcta atgctcgcca gagtcctgct gctatctgct cgtccaagta acttgatcaa    60

gctttcccaa ggtcgacttc ttgtccattg gtctctttga cccatcttgt ctcgacacag   120

tccaaccttc atcgaaccaa gccgattgaa ccatagatca aggaacttca acaattcctc   180

aaagggccaa ttcgaccatg                                                200

<210>    313
<211>    200
<212>    DNA
<213>    Amaranthus viridis

<400>    313

tctgtataac ctaaagattg aaacttttga agatattttt gaagaagtac tctttggctt    60

tttgatccag aaaatcatat tggtatgaag tgttgtttaa tatacaatca atttttctga   120

actgggttat taaagagaag cattgtagac atgaggaatg tgttcaagca tctcaaaaca 180

accaaattaa tcccaaatgg 200

<210> 314
<211> 200
<212> DNA
<213> Amaranthus viridis

<400> 314

ccatttggga ttaatttggt tgttttgaga tgcttgaaca cattcctcat gtctacaatg 60

cttctcttta ataacccagt tcagaaaaat tgattgtata ttaaacaaca cttcatacca 120

atatgatttt ctggatcaaa aagccaaaga gtacttcttc aaaaatatct tcaaaagttt 180

caatctttag gttatacaga 200

<210> 315
<211> 200
<212> DNA
<213> Amaranthus viridis

<400> 315

aaacaaccaa attaatccca aatgggatct tcaactgtaa tagagcttcg tgttttgctt 60

tccttcactc atcaccggtg aacgatgttg aagtttgttc caagaagcaa gaagtcgtaa 120

ttgcaatcgg gagcaatgtt ggtgacagat tagaaaattt caaccaagct ctgcaacaaa 180

tgaagaaatt aggcatagac 200

<210> 316
<211> 200
<212> DNA
<213> Amaranthus viridis

<400> 316

gtctatgcct aatttcttca tttgttgcag agcttggttg aaattttcta atctgtcacc 60

aacattgctc ccgattgcaa ttacgacttc ttgcttcttg gaacaaactt caacatcgtt 120

caccggtgat gagtgaagga aagcaaaaca cgaagctcta ttacagttga agatcccatt 180

tgggattaat ttggttgttt 200

<210> 317
<211> 200
<212> DNA
<213> Amaranthus viridis

<400> 317

acaaatgaag aaattaggca tagacatcac aaggcatggg tgtttatatg agacggaacc 60

tgcatacgtg actgatcaac cgaagtttct taactctgct ttaagaggct ttacaaaact 120

tgggcctcat gaattattag gggttttaaa gaaaattgag aaggatatgg gtagaaccaa 180

gggaataagg tatggtccta 200

<210> 318
<211> 200
<212> DNA
<213> Amaranthus viridis

<400> 318

taggaccata ccttattccc ttggttctac ccatatcctt ctcaattttc tttaaaaccc 60

ctaataattc atgaggccca agttttgtaa agcctcttaa agcagagtta agaaacttcg 120

gttgatcagt cacgtatgca ggttccgtct catataaaca cccatgcctt gtgatgtcta 180

tgcctaattt cttcatttgt 200

<210> 319
<211> 200
<212> DNA
<213> Amaranthus viridis

<400> 319

accaagggaa taaggtatgg tcctaggcca attgacttgg acatactatt ttatgggaaa 60

tttagggtga gctctgagag cctcactatc ccccatgaaa ggatatggga aagaccattt 120

gtgatggcac cattgattga ttgtattggg tctgatgtag aaaatgacac tatttgtaca 180

tggcattcat tatcaaattt 200

<210> 320
<211> 200
<212> DNA
<213> Amaranthus viridis

<400> 320

aaatttgata atgaatgcca tgtacaaata gtgtcatttt ctacatcaga cccaatacaa 60

tcaatcaatg gtgccatcac aaatggtctt tcccatatcc tttcatgggg gatagtgagg 120

ctctcagagc tcaccctaaa tttcccataa aatagtatgt ccaagtcaat tggcctagga 180

ccatacctta ttcccttggt 200

<210> 321
<211> 200
<212> DNA
<213> Amaranthus viridis

<400> 321

gtacatggca ttcattatca aatttttggg gtggaatctt tgaagcatgg ggtaaactcg 60

gtggaagttc cctaatcggg aaggatggaa tgaaaagggt tttgcccgtt ggaaatcgct 120

tatgggattg gtctcataaa acctctgtaa tgggagtctt gaacttgact cctgatagct 180

ttagtgatgg cggaagtttt 200

<210> 322
<211> 200
<212> DNA
<213> Amaranthus viridis

<400> 322

aaaacttccg ccatcactaa agctatcagg agtcaagttc aagactccca ttacagaggt 60

tttatgagac caatcccata agcgatttcc aacgggcaaa acccttttca ttccatcctt 120

cccgattagg gaacttccac cgagtttacc ccatgcttca aagattccac ccaaaaaatt 180

tgataatgaa tgccatgtac 200

<210> 323
<211> 200
<212> DNA
<213> Amaranthus viridis

<400> 323

tagctttagt gatggcggaa gttttcaatc tgtcgatact gcggttgcta aggttcgtca 60

gatgatctca gatggggcag atataattga catcggggca caatcaacca gacccatggc 120

aactaggatt tcggctgaag aagagctaga aagagtagta cctgtattgg aagctgtcaa 180

ggatttgatc gaggaagaag 200

<210> 324
<211> 200
<212> DNA
<213> Amaranthus viridis

<400> 324

cttcttcctc gatcaaatcc ttgacagctt ccaatacagg tactactctt tctagctctt 60

cttcagccga aatcctagtt gccatgggtc tggttgattg tgccccgatg tcaattatat 120

ctgccccatc tgagatcatc tgacgaacct tagcaaccgc agtatcgaca gattgaaaac 180

ttccgccatc actaaagcta 200

<210> 325
<211> 200
<212> DNA
<213> Amaranthus viridis

<400> 325

gtcaaggatt tgatcgagga agaaggaaga atcttgtcag tggatacgtt ttattctaaa 60

gttgcttcgg aggccgtcaa gaagggggca cacattgtga atgatgtctc tagtgggaaa 120

ctcgattccg agatgtttaa tgttgttgcg gaccttaaag ttccttatat agcaatgcac 180

atgcgaggag atccgacttc 200

<210> 326
<211> 200
<212> DNA
<213> Amaranthus viridis

<400> 326

gaagtcggat ctcctcgcat gtgcattgct atataaggaa ctttaaggtc cgcaacaaca 60

ttaaacatct cggaatcgag tttcccacta gagacatcat tcacaatgtg tgcccccttc 120

ttgacggcct ccgaagcaac tttagaataa aacgtatcca ctgacaagat tcttccttct 180

tcctcgatca aatccttgac 200

<210> 327
<211> 200
<212> DNA
<213> Amaranthus viridis

<400> 327

tgcacatgcg aggagatccg acttcaatgc aaaattctga gaacttgacg tacaatgatg 60

tttgtaagca agtggctacg gagttgagct ctagggtcat agatgcagaa ttatcgggaa 120

ttcctgcttg gaggatagtt attgatcccg gcattggatt ttctaagaat acgaatcaaa 180

atttggaaat tcttcgtggt 200

<210> 328
<211> 200
<212> DNA
<213> Amaranthus viridis

<400> 328

accacgaaga atttccaaat tttgattcgt attcttagaa aatccaatgc cgggatcaat 60

aactatcctc caagcaggaa ttcccgataa ttctgcatct atgaccctag agctcaactc 120

cgtagccact tgcttacaaa catcattgta cgtcaagttc tcagaatttt gcattgaagt 180

cggatctcct cgcatgtgca 200

<210> 329
<211> 200
<212> DNA
<213> Ambrosia trifida

<400> 329

aatgaagcct taagtcaaat gaaaaaatca ggcatagaaa taacaagaca tgcatgttta 60

tacgaaaccg agccagctta cgtgactgat caacctcttt tcctcaattc tgccatcaga 120

ggcgttacaa agctgggccc acatgagcta ctatcggccc tcaagaaaat tgaaaaagaa 180

atgggccgaa ccaaaggtat 200

<210> 330
<211> 200
<212> DNA
<213> Ambrosia trifida

<400> 330

atacctttgg ttcggcccat ttctttttca attttcttga gggccgatag tagctcatgt 60

gggcccagct ttgtaacgcc tctgatggca gaattgagga aaagaggttg atcagtcacg 120

taagctggct cggtttcgta taaacatgca tgtcttgtta tttctatgcc tgattttttc 180

atttgactta aggcttcatt 200

<210> 331
<211> 200
<212> DNA
<213> Ambrosia trifida

<400> 331

aagaaatggg ccgaaccaaa ggtattagat acggcccgcg acccattgac ttagatatac 60

tgttttatgg taaacacaga gttaactcgg aaattctcac tgttccacat gaaagaatct 120

tcgagaggcc atttgttatg gctccgttag ttgacttatt ggggtcggaa attgacaatg 180

atacggttct atgctggcat 200

<210> 332
<211> 200
<212> DNA
<213> Ambrosia trifida

<400> 332

atgccagcat agaaccgtat cattgtcaat ttccgacccc aataagtcaa ctaacggagc 60

cataacaaat ggcctctcga agattctttc atgtggaaca gtgagaattt ccgagttaac 120

tctgtgttta ccataaaaca gtatatctaa gtcaatgggt cgcgggccgt atctaatacc 180

tttggttcgg cccatttctt 200

<210> 333
<211> 200
<212> DNA
<213> Ambrosia trifida

<400> 333

caatgatacg gttctatgct ggcattcttt ctcaaaaaga ggactttttg aatcttggga 60

aaaattaggt ggcgaatctt tgataggcaa agatgggtta agaagagttt taccagtcaa 120

taatcggtta tgggattggt caaagaaaac ctctgtcatg ggaattttga acttgactcc 180

cgatagtttt agcgacggag 200

<210> 334
<211> 200
<212> DNA
<213> Ambrosia trifida

<400> 334

ctccgtcgct aaaactatcg ggagtcaagt tcaaaattcc catgacagag gttttctttg 60

accaatccca taaccgatta ttgactggta aaactcttct taacccatct ttgcctatca 120

aagattcgcc acctaatttt tcccaagatt caaaaagtcc tctttttgag aaagaatgcc 180

agcatagaac cgtatcattg 200

<210> 335
<211> 200
<212> DNA
<213> Ambrosia trifida

<400> 335

actcccgata gttttagcga cggaggaaag tttgacactg tggggtccgc tatatctcgt 60

gttagggcca tgatatctga aggggcagac ataattgatc tgggagctca atcgacacgt 120

ccaatggcaa ccaagatttc agtccaagaa gaactagata ggttaatccc tgttctcgaa 180

aagattcttg aattacccga 200

<210> 336
<211> 200
<212> DNA
<213> Ambrosia trifida

<400> 336

tcgggtaatt caagaatctt ttcgagaaca gggattaacc tatctagttc ttcttggact 60

gaaatcttgg ttgccattgg acgtgtcgat tgagctccca gatcaattat gtctgcccct 120

tcagatatca tggccctaac acgagatata gcggacccca cagtgtcaaa ctttcctccg 180

tcgctaaaac tatcgggagt 200

<210> 337
<211> 200
<212> DNA
<213> Ambrosia trifida

<400>       337

tcgaaaagat tcttgaatta cccgaaattg aaggaaaact gctctctatc gacacgtttt    60

actcagaagt tgctttagaa gcgattaaga aaggggctca tataatcaat gatgtatcgg   120

gtggaaatct agattctgat atgtttcggg tagttgctaa tcttgatgtt ccatatattg   180

ctatgcacat gagaggggac                                               200

<210>       338
<211>       200
<212>       DNA
<213>       Ambrosia trifida

<400>       338

gtcccctctc atgtgcatag caatatatgg aacatcaaga ttagcaacta cccgaaacat    60

atcagaatct agatttccac ccgatacatc attgattata tgagcccctt tcttaatcgc   120

ttctaaagca acttctgagt aaaacgtgtc gatagagagc agttttcctt caatttcggg   180

taattcaaga atcttttcga                                               200

<210>       339
<211>       200
<212>       DNA
<213>       Ambrosia trifida

<400>       339

tattgctatg cacatgagag gggacccatc cacaatgcaa aacagtgaga atttgaagta    60

tgatgatgtt tgtaaagaag ttggtgatga gttgtatgag cgtgtaagag ctgcagagtt   120

atgcggtgtg cctgcatgga ggatcattct tgacccaggg atcgggtttt caaagaaaac   180

cgaagataat ttggatattt                                               200

<210>       340
<211>       200
<212>       DNA
<213>       Ambrosia trifida

<400>       340

aaatatccaa attatcttcg gttttctttg aaaacccgat ccctgggtca agaatgatcc    60

tccatgcagg cacaccgcat aactctgcag ctcttacacg ctcatacaac tcatcaccaa   120

cttctttaca aacatcatca tacttcaaat tctcactgtt ttgcattgtg gatgggtccc   180

ctctcatgtg catagcaata                                               200

<210>       341
<211>       200
<212>       DNA
<213>       Ambrosia trifida

<400>       341

aaaaccgaag ataatttgga tattttgatg ggattaaaga gaattaggag tgagattgca    60

cggaagagct taggggtgtc acgtgcacct ttgttaatcg gtccttcaag aaagagattt   120

ttgggggaga tttgtggtcg ggcttctgct gtcgagagag atccagcgac tgttgctgct   180

gttacatgtg cggttttggg                                                200

<210>       342
<211>       200
<212>       DNA
<213>       Ambrosia trifida

<400>       342

cccaaaaccg cacatgtaac agcagcaaca gtcgctggat ctctctcgac agcagaagcc    60

cgaccacaaa tctcccccaa aaatctcttt cttgaaggac cgattaacaa aggtgcacgt   120

gacaccccta agctcttccg tgcaatctca ctcctaattc tctttaatcc catcaaaata   180

tccaaattat cttcggtttt                                                200

<210>       343
<211>       200
<212>       DNA
<213>       Ambrosia trifida

<400>       343

acctcatcac ctccacaaat caccaccacc catctccacc ctccaccacc acccacttca    60

ttctccccac ccaccgtatc tcatcaccat catacctggt ggattgcagt ttgactctgt   120

ggggtccgct atatctcgtg ttaggaccat gatatctgaa ggggcggaca taattgatct   180

gggagctcaa tcgacacgtc                                                200

<210>       344
<211>       200
<212>       DNA
<213>       Ambrosia trifida

<400>       344

gacgtgtcga ttgagctccc agatcaatta tgtccgcccc ttcagatatc atggtcctaa    60

cacgagatat agcggacccc acagagtcaa actgcaatcc accaggtatg atggtgatga   120

gatacggtgg gtggggagaa tgaagtgggt ggtggtggag ggtggagatg ggtggtggtg   180

atttgtggag gtgatgaggt                                                200

<210>       345
<211>       200
<212>       DNA

<213>       Ambrosia trifida

<400>       345

gacccatcca caatgcaaaa cagtgagaat ttgaagtata atgatgtttg taaagaagtt    60

ggtgatgagt tgtatgagcg tgtaagagct gcagagttat gcggtgtgcc tgcatggagg   120

atcattcttg acccagggat cgggttttca aagaaaaccg aagataattt ggatattttg   180

atgggattaa agagaattag                                               200

<210>       346
<211>       200
<212>       DNA
<213>       Ambrosia trifida

<400>       346

ctaattctct ttaatcccat caaaatatcc aaattatctt cggttttctt tgaaaacccg    60

atccctgggt caagaatgat cctccatgca ggcacaccgc ataactctgc agctcttaca   120

cgctcataca actcatcacc aacttcttta caaacatcat tatacttcaa attctcactg   180

ttttgcattg tggatgggtc                                               200

<210>       347
<211>       200
<212>       DNA
<213>       Ambrosia trifida

<400>       347

gacccatcca caatgcaaaa cagtgagaat ttgaagtata atgatgtttg taaagaagtt    60

ggtgatgagt tgtatgagcg tgtaagagct gcagagttat gcggtgtgcc tgcatggagg   120

atcattcttg acccagggat cgggttttca aagaaaaccg aagataattt ggatattttg   180

atgggattaa agagaattag                                               200

<210>       348
<211>       200
<212>       DNA
<213>       Ambrosia trifida

<400>       348

ctaattctct ttaatcccat caaaatatcc aaattatctt cggttttctt tgaaaacccg    60

atccctgggt caagaatgat cctccatgca ggcacaccgc ataactctgc agctcttaca   120

cgctcataca actcatcacc aacttcttta caaacatcat tatacttcaa attctcactg   180

ttttgcattg tggatgggtc                                               200

<210>       349
<211>       200

<212>       DNA
<213>       Ambrosia trifida

<400>       349

ttttgatggg attaaagaga attaggagtg agattgcccg gaagagctta ggggtgtcac    60

gtgcaccttt gttaatcggt ccttcaagaa agagattttt gggagagatt tgtggtcggg   120

cttctgctgg tgagagagat ccaacaactg ttgctgctgt tacatgtgcg gttttgggtg   180

gtgctaatgt tgttcgcgtt                                               200


<210>       350
<211>       200
<212>       DNA
<213>       Ambrosia trifida

<400>       350

aacgcgaaca acattagcac cacccaaaac cgcacatgta acagcagcaa cagttgttgg    60

atctctctca ccagcagaag cccgaccaca aatctctccc aaaaatctct ttcttgaagg   120

accgattaac aaaggtgcac gtgacacccc taagctcttc cgggcaatct cactcctaat   180

tctctttaat cccatcaaaa                                               200


<210>       351
<211>       200
<212>       DNA
<213>       Ambrosia trifida

<400>       351

gatttgtggt cgggcttctg ctggtgagag agatccaaca actgttgctg ctgttacatg    60

tgcggttttg ggtggtgcta atgttgttcg cgttcataat gttggagatg atgctgatgc   120

tgtaaagctt tgtgattcaa tgttgaaccg ggttggaaga acgtaacagc tttttgaaat   180

ttgattgtgg gttttggtcc                                               200


<210>       352
<211>       200
<212>       DNA
<213>       Ambrosia trifida

<400>       352

ggaccaaaac ccacaatcaa atttcaaaaa gctgttacgt tcttccaacc cggttcaaca    60

ttgaatcaca aagctttaca gcatcagcat catctccaac attatgaacg cgaacaacat   120

tagcaccacc caaaaccgca catgtaacag cagcaacagt tgttggatct ctctcaccag   180

cagaagcccg accacaaatc                                               200


<210>       353

<211>	200
<212>	DNA
<213>	Ambrosia trifida

<400>	353

gccttaagtc aaatgaagaa atcaggcata gagataacaa gacatgcatg tttatacaaa	60

accgagccag cttatgtaac tgatcaacct ctttttctta attctgccat cagaggcgtt	120

acaaagctgg gcccacatga actactatcg gccctcaaga aaatcgaaaa agaattgggc	180

cgaaccaaag gtattagata	200


<210>	354
<211>	200
<212>	DNA
<213>	Ambrosia trifida

<400>	354

tatctaatac ctttggttcg gcccaattct ttttcgattt tcttgagggc cgatagtagt	60

tcatgtgggc ccagctttgt aacgcctctg atggcagaat taagaaaaag aggttgatca	120

gttacataag ctggctcggt tttgtataaa catgcatgtc ttgttatctc tatgcctgat	180

ttcttcattt gacttaaggc	200


<210>	355
<211>	200
<212>	DNA
<213>	Ambrosia trifida

<400>	355

tgggccgaac caaaggtatt agatacggcc cgcgacccat tgacttagat atactgtttt	60

atggtaaaca cagaattaac tcggaaattc tcactgttcc acatgaaaga atcttcgaga	120

ggccatttgt tatggctccg ttagttgact tattggggtc ggatattgac aatgatacgg	180

ttctatgctg gcattctttc	200


<210>	356
<211>	200
<212>	DNA
<213>	Ambrosia trifida

<400>	356

gaaagaatgc cagcatagaa ccgtatcatt gtcaatatcc gaccccaata agtcaactaa	60

cggagccata acaaatggcc tctcgaagat tctttcatgt ggaacagtga gaatttccga	120

gttaattctg tgtttaccat aaaacagtat atctaagtca atgggtcgcg ggccgtatct	180

aatacctttg gttcggccca	200

<210>	357
<211>	101
<212>	DNA
<213>	Ambrosia trifida

<400>	357

ggattgcagt ttgactctgt ggggtccgct atatctcgtg ttaggaccat gatatctgaa	60

ggggcggaca taattgatct gggagctcaa tcgacacgtc c	101


<210>	358
<211>	101
<212>	DNA
<213>	Ambrosia trifida

<400>	358

ggacgtgtcg attgagctcc cagatcaatt atgtccgccc cttcagatat catggtccta	60

acacgagata tagcggaccc cacagagtca aactgcaatc c	101


<210>	359
<211>	200
<212>	DNA
<213>	Ambrosia trifida

<400>	359

tcgttctttc atttctcttc ggatgcttcg atacaagtcc attctcaaga acaagaagta	60

gtaattgctt taggtagcaa tgtgggtgat aggcttaata actttaatga agccttaagt	120

caaatgaaaa aatcaggcat agaaataaca agacatgcat gtttatacga aaccgagcca	180

gcttacgtga ctgatcaacc	200


<210>	360
<211>	200
<212>	DNA
<213>	Ambrosia trifida

<400>	360

ggttgatcag tcacgtaagc tggctcggtt tcgtataaac atgcatgtct tgttatttct	60

atgcctgatt ttttcatttg acttaaggct tcattaaagt tattaagcct atcacccaca	120

ttgctaccta aagcaattac tacttcttgt tcttgagaat ggacttgtat cgaagcatcc	180

gaagagaaat gaaagaacga	200


<210>	361
<211>	200
<212>	DNA
<213>	Ambrosia trifida

<400>	361

agccagctta cgtgactgat caacctcttt tcctcaattc tgccatcaga ggcgttacaa 60

agctgggccc acatgagcta ctatcggccc tcaagaaaat tgaaaaagaa atgggccgaa 120

ccaaaggtat tagatacggc ccgcgaccca ttgacttaga tatactgttt tatggtaaac 180

acagagttaa ctcggaaatt 200

<210> 362
<211> 200
<212> DNA
<213> Ambrosia trifida

<400> 362

aatttccgag ttaactctgt gtttaccata aaacagtata tctaagtcaa tgggtcgcgg 60

gccgtatcta atacctttgg ttcggcccat ttctttttca attttcttga gggccgatag 120

tagctcatgt gggcccagct ttgtaacgcc tctgatggca gaattgagga aaagaggttg 180

atcagtcacg taagctggct 200

<210> 363
<211> 200
<212> DNA
<213> Ambrosia trifida

<400> 363
   taaacacaga gttaactcgg aaattctcac tgttccacat gaaagaatct tcgagaggcc 60
   atttgttatg gctccgttag ttgacttatt ggggtcggaa attgacaatg atacggttct 120
   atgctggcat tctttctcaa aaagaggact tttgaatctt tgggaaaaat taggtggcga 180
   atctttgata ggcaaagatg 200

<210> 364
<211> 200
<212> DNA
<213> Ambrosia trifida

<400> 364

catctttgcc tatcaaagat tcgccaccta atttttccca agattcaaaa agtcctcttt 60

ttgagaaaga atgccagcat agaaccgtat cattgtcaat ttccgacccc aataagtcaa 120

ctaacggagc cataacaaat ggcctctcga agattctttc atgtggaaca gtgagaattt 180

ccgagttaac tctgtgttta 200

<210> 365
<211> 200
<212> DNA
<213> Ambrosia trifida

<400> 365

ggcgaatctt tgataggcaa agatgggtta agaagagttt taccagtcaa taatcggtta 60

tgggattggt caaagaaaac ctctgtcatg ggaattttga acttgactcc cgatagtttt 120

agcgacggag gaaagtttga cactgtgggg tccgctatat ctcgtgttag ggccatgata 180

tctgaagggg cagacataat 200

<210> 366
<211> 200
<212> DNA
<213> Ambrosia trifida

<400> 366

attatgtctg ccccttcaga tatcatggcc ctaacacgag atatagcgga ccccacagtg 60

tcaaactttc ctccgtcgct aaaactatcg ggagtcaagt tcaaaattcc catgacagag 120

gttttctttg accaatccca taaccgatta ttgactggta aaactcttct taacccatct 180

ttgcctatca aagattcgcc 200

<210> 367
<211> 200
<212> DNA
<213> Ambrosia trifida

<400> 367

tgatatctga aggggcagac ataattgatc tgggagctca atcgacacgt ccaatggcaa 60

ccaagatttc agtccaagaa gaactagata ggttaatccc tgttctcgaa aagattcttg 120

aattacccga aattgaagga aaactgctct ctatcgacac gttttactca gaagttgctt 180

tagaagcgat taagaaaggg 200

<210> 368
<211> 200
<212> DNA
<213> Ambrosia trifida

<400> 368

ccctttctta atcgcttcta aagcaacttc tgagtaaaac gtgtcgatag agagcagttt 60

tccttcaatt tcgggtaatt caagaatctt ttcgagaaca gggattaacc tatctagttc 120

ttcttggact gaaatcttgg ttgccattgg acgtgtcgat tgagctccca gatcaattat 180

gtctgcccct tcagatatca 200

<210> 369
<211> 200
<212> DNA
<213> Ambrosia trifida

<400>       369

tgctttagaa gcgattaaga aaggggctca tataatcaat gatgtatcgg gtggaaatct    60

agattctgat atgtttcggg tagttgctaa tcttgatgtt ccatatattg ctatgcacat   120

gagaggggac ccatccacaa tgcaaaacag tgagaatttg aagtatgatg atgtttgtaa   180

agaagttggt gatgagttgt                                               200


<210>       370
<211>       200
<212>       DNA
<213>       Ambrosia trifida

<400>       370

acaactcatc accaacttct ttacaaacat catcatactt caaattctca ctgttttgca    60

ttgtggatgg gtcccctctc atgtgcatag caatatatgg aacatcaaga ttagcaacta   120

cccgaaacat atcagaatct agatttccac ccgatacatc attgattata tgagcccctt   180

tcttaatcgc ttctaaagca                                               200


<210>       371
<211>       200
<212>       DNA
<213>       Ambrosia trifida

<400>       371

tgtaaagaag ttggtgatga gttgtatgag cgtgtaagag ctgcagagtt atgcggtgtg    60

cctgcatgga ggatcattct tgacccaggg atcgggtttt caaagaaaac cgaagataat   120

ttggatattt tgatgggatt aaagagaatt aggagtgaga ttgcacggaa gagcttaggg   180

gtgtcacgtg cacctttgtt                                               200


<210>       372
<211>       200
<212>       DNA
<213>       Ambrosia trifida

<400>       372

aacaaaggtg cacgtgacac ccctaagctc ttccgtgcaa tctcactcct aattctcttt    60

aatcccatca aaatatccaa attatcttcg gttttctttg aaaacccgat ccctgggtca   120

agaatgatcc tccatgcagg cacaccgcat aactctgcag ctcttacacg ctcatacaac   180

tcatcaccaa cttctttaca                                               200


<210>       373
<211>       200
<212>       DNA
<213>       Ambrosia trifida

<400>       373

ttcgtgtatc tcatgatctc tgtataatgt taaacttatt atagttcaat ataagtcact    60

gtgtgatcta atttcgtata caattacttt atttgtgttg attctgatcc taaatattgc   120

taatagtttg tgcttaacta tatatcccgt taaacatcac atttacttta tccctagaag   180

atttgggcaa gtttttacaa                                               200


<210>       374
<211>       200
<212>       DNA
<213>       Ambrosia trifida

<400>       374

ttgtaaaaac ttgcccaaat cttctaggga taaagtaaat gtgatgttta acgggatata    60

tagttaagca caaactatta gcaatattta ggatcagaat caacacaaat aaagtaattg   120

tatacgaaat tagatcacac agtgacttat attgaactat aataagttta acattataca   180

gagatcatga gatacacgaa                                               200


<210>       375
<211>       200
<212>       DNA
<213>       Ambrosia trifida

<400>       375

agaagatttg ggcaagtttt tacaaattta tggcgacgtt attgtaattt tttaaatcat    60

tttcgtgcag catctttttc gttctttcat ttctcttcgg atgcttcgat acaagtccat   120

tctcaagaac aagaagtagt aattgcttta ggtagcaatg tgggtgatag gcttaataac   180

tttaatgaag ccttaagtca                                               200


<210>       376
<211>       200
<212>       DNA
<213>       Ambrosia trifida

<400>       376

tgacttaagg cttcattaaa gttattaagc ctatcaccca cattgctacc taaagcaatt    60

actacttctt gttcttgaga atggacttgt atcgaagcat ccgaagagaa atgaaagaac   120

gaaaaagatg ctgcacgaaa atgatttaaa aaattacaat aacgtcgcca taaatttgta   180

aaaacttgcc caaatcttct                                               200


<210>       377
<211>       200
<212>       DNA

<213>       Conyza canadensis

<400>       377

gaaataacaa ggcatgcttg tctatacgaa actgaaccag cttatgtgac ggaccagcct    60

ctttttctca actctgccgt tagagccact acaaagcttg gccctcatga gctactgtcc   120

attctcaaga aaattgaaaa ggaaatgggt cgaaccaaag ggcttaggta cggcccacga   180

cccattgacc tagatatatt                                               200


<210>       378
<211>       200
<212>       DNA
<213>       Conyza canadensis

<400>       378

aatatatcta ggtcaatggg tcgtgggccg tacctaagcc ctttggttcg acccatttcc    60

ttttcaattt tcttgagaat ggacagtagc tcatgagggc caagctttgt agtggctcta   120

acggcagagt tgagaaaaag aggctggtcc gtcacataag ctggttcagt ttcgtataga   180

caagcatgcc ttgttatttc                                               200


<210>       379
<211>       200
<212>       DNA
<213>       Conyza canadensis

<400>       379

cacgacccat tgacctagat atattgttct atggtaaatg caaagttaac tctgatattc    60

taactgttcc tcatgaaaga atctttgaga ggccattcgt tatggctcca ttagttgact   120

tgttaggatc agatgtagac aatgatacgg ttctatgctg gcattctttt tcaaaaaatg   180

ggctttttgg atcttgggaa                                               200


<210>       380
<211>       200
<212>       DNA
<213>       Conyza canadensis

<400>       380

ttcccaagat ccaaaaagcc cattttttga aaaagaatgc cagcatagaa ccgtatcatt    60

gtctacatct gatcctaaca agtcaactaa tggagccata acgaatggcc tctcaaagat   120

tctttcatga ggaacagtta gaatatcaga gttaactttg catttaccat agaacaatat   180

atctaggtca atgggtcgtg                                               200


<210>       381
<211>       200

<212>       DNA
<213>       Conyza canadensis

<400>       381

aaatgggctt tttggatctt gggaaacatt aggtggtgaa tcttccatag gaaaagatgg    60

tttaagaagg gttttacctg tcaatgatcg tttatgggat tggtcaaaga aaacttccgt   120

catgggtatt ttgaatataa ctcctgatag ttttagtgat ggagggaagt ttgattccat   180

ggggtccgct ttatctcgtg                                               200


<210>       382
<211>       200
<212>       DNA
<213>       Conyza canadensis

<400>       382

cacgagataa agcggacccc atggaatcaa acttccctcc atcactaaaa ctatcaggag    60

ttatattcaa aatacccatg acggaagttt tctttgacca atcccataaa cgatcattga   120

caggtaaaac ccttcttaaa ccatcttttc ctatggaaga ttcaccacct aatgtttccc   180

aagatccaaa aagcccattt                                               200


<210>       383
<211>       200
<212>       DNA
<213>       Conyza canadensis

<400>       383

tccatggggt ccgctttatc tcgtgttcag accatgatat ctgaaggggt tgacataatt    60

gatctaggag ctcaatccac acgcccaatg gcgaccaaga tctcagtcga agaggaacta   120

gataggctaa tacccgttct tgaaaagatt cttgaattac ctgaaattga aggaaagttg   180

ttgtctgtgg acacatttta                                               200


<210>       384
<211>       200
<212>       DNA
<213>       Conyza canadensis

<400>       384

taaaatgtgt ccacagacaa caactttcct tcaatttcag gtaattcaag aatcttttca    60

agaacgggta ttagcctatc tagttcctct tcgactgaga tcttggtcgc cattgggcgt   120

gtggattgag ctcctagatc aattatgtca accccttcag atatcatggt ctgaacacga   180

gataaagcgg accccatgga                                               200


<210>       385

<211>       200
<212>       DNA
<213>       Conyza canadensis

<400>       385

agttgttgtc tgtggacaca ttttactcgg aagttgcttc agaggcaatc aagaaagggg    60

ctcatatggt caatgatgta tcgggtggaa tgttagattc tgatatgctt catgttgtgg   120

ctgatctaaa tgttccatat atcactatgc acatgagagg ggacccatcc acaatgcaaa   180

acagtgagaa tttgaagtat                                                200


<210>       386
<211>       200
<212>       DNA
<213>       Conyza canadensis

<400>       386

atacttcaaa ttctcactgt tttgcattgt ggatgggtcc cctctcatgt gcatagtgat    60

atatggaaca tttagatcag ccacaacatg aagcatatca gaatctaaca ttccacccga   120

tacatcattg accatatgag cccctttctt gattgcctct gaagcaactt ccgagtaaaa   180

tgtgtccaca gacaacaact                                                200


<210>       387
<211>       200
<212>       DNA
<213>       Conyza canadensis

<400>       387

gcaaaacagt gagaatttga agtatgatga tgttgtcaaa gaagttgggg aggaattgta    60

tgaacgtgta aggaatgcag agttatgtgg tgttcccgca tggaggatgg ttcttgatcc   120

agggatcggg ttttcaaaga aaaccgaaga taatttggag atattgatgg gactaaagag   180

gtttagaagt gagattggac                                                200


<210>       388
<211>       200
<212>       DNA
<213>       Conyza canadensis

<400>       388

gtccaatctc acttctaaac ctctttagtc ccatcaatat ctccaaatta tcttcggttt    60

tctttgaaaa cccgatccct ggatcaagaa ccatcctcca tgcgggaaca ccacataact   120

ctgcattcct tacacgttca tacaattcct ccccaacttc tttgacaaca tcatcatact   180

tcaaattctc actgttttgc                                                200

<210>	389
<211>	200
<212>	DNA
<213>	Conyza canadensis

<400>	389

aagaggttta gaagtgagat tggacaaaag agcttagggg tgtctcgtgc acctttgtta	60

atcggacctt caagaaaaag gtttttgggt gagatttgtg gtcggccttc tgctgttgag	120

agagatccag ggactgtagc tgctgttacc agtgcgattt tgggtggtgc caatatcgtt	180

agggttcata acattggaca	200

<210>	390
<211>	200
<212>	DNA
<213>	Conyza canadensis

<400>	390

tgtccaatgt tatgaaccct aacgatattg gcaccaccca aaatcgcact ggtaacagca	60

gctacagtcc ctggatctct ctcaacagca gaaggccgac cacaaatctc acccaaaaac	120

ctttttcttg aaggtccgat taacaaaggt gcacgagaca cccctaagct cttttgtcca	180

atctcacttc taaacctctt	200

<210>	391
<211>	200
<212>	DNA
<213>	Conyza canadensis

<400>	391

tcgtcctcgg atacttcaat acaagtgcat tctccagaac aagaagtagt aattgcttta	60

ggtagcaatg tgggtaatag acttaataac tttaatgaag ccttatccca gatgaagaaa	120

tcggggatag aaataacaag gcatgcttgt ctatacgaaa ctgaaccagc ttatgtgacg	180

gaccagcctc tttttctcaa	200

<210>	392
<211>	200
<212>	DNA
<213>	Conyza canadensis

<400>	392

ttgagaaaaa gaggctggtc cgtcacataa gctggttcag tttcgtatag acaagcatgc	60

cttgttattt ctatccccga tttcttcatc tgggataagg cttcattaaa gttattaagt	120

ctattaccca cattgctacc taaagcaatt actacttctt gttctggaga atgcacttgt	180

attgaagtat ccgaggacga	200

<210>	393
<211>	200
<212>	DNA
<213>	Conyza canadensis

<400>	393

tgacggacca gcctcttttt ctcaactctg ccgttagagc cactacaaag cttggccctc	60

atgagctact gtccattctc aagaaaattg aaaaggaaat gggtcgaacc aaagggctta	120

ggtacggccc acgacccatt gacctagata tattgttcta tggtaaatgc aaagttaact	180

ctgatattct aactgttcct	200

<210>	394
<211>	200
<212>	DNA
<213>	Conyza canadensis

<400>	394

aggaacagtt agaatatcag agttaacttt gcatttacca tagaacaata tatctaggtc	60

aatgggtcgt gggccgtacc taagcccttt ggttcgaccc atttcctttt caattttctt	120

gagaatggac agtagctcat gagggccaag ctttgtagtg gctctaacgg cagagttgag	180

aaaaagaggc tggtccgtca	200

<210>	395
<211>	200
<212>	DNA
<213>	Conyza canadensis

<400>	395

taactctgat attctaactg ttcctcatga aagaatcttt gagaggccat tcgttatggc	60

tccattagtt gacttgttag gatcagatgt agacaatgat acggttctat gctggcattc	120

tttttcaaaa aatgggcttt ttggatcttg ggaaacatta ggtggtgaat cttccatagg	180

aaaagatggt ttaagaaggg	200

<210>	396
<211>	200
<212>	DNA
<213>	Conyza canadensis

<400>	396

cccttcttaa accatctttt cctatggaag attcaccacc taatgtttcc caagatccaa	60

aaagcccatt ttttgaaaaa gaatgccagc atagaaccgt atcattgtct acatctgatc	120

ctaacaagtc aactaatgga gccataacga atggcctctc aaagattctt tcatgaggaa	180

cagttagaat atcagagtta	200

<210>	397
<211>	200
<212>	DNA
<213>	Conyza canadensis

<400>	397

ataggaaaag atggtttaag aagggtttta cctgtcaatg atcgtttatg ggattggtca	60

aagaaaactt ccgtcatggg tattttgaat ataactcctg atagttttag tgatggaggg	120

aagtttgatt ccatggggtc cgctttatct cgtgttcaga ccatgatatc tgaaggggtt	180

gacataattg atctaggagc	200

<210>	398
<211>	200
<212>	DNA
<213>	Conyza canadensis

<400>	398

gctcctagat caattatgtc aaccccttca gatatcatgg tctgaacacg agataaagcg	60

gaccccatgg aatcaaactt ccctccatca ctaaaactat caggagttat attcaaaata	120

cccatgacgg aagttttctt tgaccaatcc cataaacgat cattgacagg taaaaccctt	180

cttaaaccat cttttcctat	200

<210>	399
<211>	200
<212>	DNA
<213>	Conyza canadensis

<400>	399

gggttgacat aattgatcta ggagctcaat ccacacgccc aatggcgacc aagatctcag	60

tcgaagagga actagatagg ctaatacccg ttcttgaaaa gattcttgaa ttacctgaaa	120

ttgaaggaaa gttgttgtct gtggacacat tttactcgga agttgcttca gaggcaatca	180

agaaaggggc tcatatggtc	200

<210>	400
<211>	200
<212>	DNA
<213>	Conyza canadensis

<400>	400

gaccatatga gcccctttct tgattgcctc tgaagcaact tccgagtaaa atgtgtccac	60

agacaacaac tttccttcaa tttcaggtaa ttcaagaatc ttttcaagaa cgggtattag	120

cctatctagt tcctcttcga ctgagatctt ggtcgccatt gggcgtgtgg attgagctcc	180

tagatcaatt atgtcaaccc 200

<210> 401
<211> 200
<212> DNA
<213> Conyza canadensis

<400> 401

aatcaagaaa ggggctcata tggtcaatga tgtatcgggt ggaatgttag attctgatat 60

gcttcatgtt gtggctgatc taaatgttcc atatatcact atgcacatga gaggggaccc 120

atccacaatg caaaacagtg agaatttgaa gtatgatgat gttgtcaaag aagttgggga 180

ggaattgtat gaacgtgtaa 200

<210> 402
<211> 200
<212> DNA
<213> Conyza canadensis

<400> 402

ttacacgttc atacaattcc tccccaactt ctttgacaac atcatcatac ttcaaattct 60

cactgttttg cattgtggat gggtcccctc tcatgtgcat agtgatatat ggaacattta 120

gatcagccac aacatgaagc atatcagaat ctaacattcc acccgataca tcattgacca 180

tatgagcccc tttcttgatt 200

<210> 403
<211> 200
<212> DNA
<213> Conyza canadensis

<400> 403

ggggaggaat tgtatgaacg tgtaaggaat gcagagttat gtggtgttcc cgcatggagg 60

atggttcttg atccagggat cgggttttca aagaaaaccg aagataattt ggagatattg 120

atgggactaa agaggtttag aagtgagatt ggacaaaaga gcttaggggt gtctcgtgca 180

cctttgttaa tcggaccttc 200

<210> 404
<211> 200
<212> DNA
<213> Conyza canadensis

<400> 404

gaaggtccga ttaacaaagg tgcacgagac acccctaagc tcttttgtcc aatctcactt 60

ctaaacctct ttagtcccat caatatctcc aaattatctt cggttttctt tgaaaacccg 120

atccctggat caagaaccat cctccatgcg ggaacaccac ataactctgc attccttaca 180

cgttcataca attcctcccc 200

<210> 405
<211> 200
<212> DNA
<213> Conyza canadensis

<400> 405

tttcttcttc tagttggagc tcttttacgt ggtgtcttct tttccgagtc atcttcttcc 60

ccatcagatg gattttctgc agcctttttt ctacgaactg ctgtaatagc aaatgtacta 120

cgcaatctaa catctgggaa ttgtacaaag tttattgaag atagtgaagg cagattcgac 180

tgccacctac acatataaga 200

<210> 406
<211> 200
<212> DNA
<213> Conyza canadensis

<400> 406

tcttatatgt gtaggtggca gtcgaatctg ccttcactat cttcaataaa ctttgtacaa 60

ttcccagatg ttagattgcg tagtacattt gctattacag cagttcgtag aaaaaaggct 120

gcagaaaatc catctgatgg ggaagaagat gactcggaaa agaagacacc acgtaaaaga 180

gctccaacta gaagaagaaa 200

<210> 407
<211> 200
<212> DNA
<213> Conyza canadensis

<400> 407

tcgactgcca cctacacata taagaaacca ttaacttcta tacttaactc acaatataaa 60

acatcatttg catgttcgtc aaaaacaata ccatttctta tctttgctca atggatatga 120

aatctgccaa tacaagatta ttcatttgac ctcaatctag catctgatgt ctattcaagg 180

tatcgtttat aatgaagatt 200

<210> 408
<211> 200
<212> DNA
<213> Conyza canadensis

<400> 408

aatcttcatt ataaacgata ccttgaatag acatcagatg ctagattgag gtcaaatgaa 60

taatcttgta ttggcagatt tcatatccat tgagcaaaga taagaaatgg tattgttttt 120

gacgaacatg caaatgatgt tttatattgt gagttaagta tagaagttaa tggtttctta 180

tatgtgtagg tggcagtcga 200

<210> 409
<211> 200
<212> DNA
<213> Conyza canadensis

<400> 409

caaggtatcg tttataatga agattattgt acttcaaaat actttcttgt gtagtggtat 60

caaattttga gttaaagtta ctccatttga cctcaaaagc caaataatga gcaacaaaca 120

acacaatttc acctccctta taccctgcga ctatatttta tcttcaaatc aatccatttc 180

catgtttatc ctctccatac 200

<210> 410
<211> 200
<212> DNA
<213> Conyza canadensis

<400> 410

gtatggagag gataaacatg gaaatggatt gatttgaaga taaaatatag tcgcagggta 60

taagggaggt gaaattgtgt tgtttgttgc tcattatttg gcttttgagg tcaaatggag 120

taactttaac tcaaaatttg ataccactac acaagaaagt attttgaagt acaataatct 180

tcattataaa cgataccttg 200

<210> 411
<211> 200
<212> DNA
<213> Conyza canadensis

<400> 411

atttccatgt ttatcctctc catacagcta cataataagt tttagggaaa aaacaaaact 60

aaactaaact aagtttacag aattcccatt tcttatccta aaatttacaa gcaattcaac 120

atatattctt tatttacaac tataaatagc atatgggtat gttggtatat agatatagat 180

atagatatac atatgtagat 200

<210> 412
<211> 200
<212> DNA
<213> Conyza canadensis

<400> 412

atctacatat gtatatctat atctatatct atataccaac atacccatat gctatttata 60

gttgtaaata aagaatatat gttgaattgc ttgtaaattt taggataaga aatgggaatt 120

ctgtaaactt agtttagttt agttttgttt tttccctaaa acttattatg tagctgtatg 180

gagaggataa acatggaaat 200

<210> 413
<211> 200
<212> DNA
<213> Conyza canadensis

<400> 413

tagatataga tatacatatg tagatgtgta ttatatgtgt gtaaatgggg agaaaaaagg 60

aaacatatat tacctgtgta aagaacaaaa ttgagtaaaa gaaggcaaag aagccatttc 120

taaccactaa aatcttgggc tttcaagaat cccacttcaa agctaccatt tcacatacca 180

aacccccctt tctatgggtt 200

<210> 414
<211> 200
<212> DNA
<213> Conyza canadensis

<400> 414

aacccataga aaggggggtt tggtatgtga aatggtagct ttgaagtggg attcttgaaa 60

gcccaagatt ttagtggtta gaaatggctt ctttgccttc ttttactcaa ttttgttctt 120

tacacaggta atatatgttt ccttttttct ccccatttac acacatataa tacacatcta 180

catatgtata tctatatcta 200

<210> 415
<211> 200
<212> DNA
<213> Conyza canadensis

<400> 415
   taccaaaccc ccctttctat gggtttaaag atgaggcttt tacatttatc tcttttacaa 60
   tccaaatttt ccaaaaccga aataatgtac gacattcggt gcacaaggta caaacagaaa 120
   aaactacttt tgtattttag atctcaagaa aggagtggcc tttttggtaa taacatactt 180
   tattcaattg ccacttacct 200

<210> 416
<211> 200
<212> DNA
<213> Conyza canadensis

<400> 416

aggtaagtgg caattgaata aagtatgtta ttaccaaaaa ggccactcct ttcttgagat 60

ctaaaataca aaagtagttt tttctgtttg taccttgtgc accgaatgtc gtacattatt 120

tcggttttgg aaaatttgga ttgtaaaaga gataaatgta aagccctcat ctttaaaccc 180

atagaaaggg gggtttggta 200

<210> 417
<211> 200
<212> DNA
<213> Conyza canadensis

<400> 417

tactttattc aattgccact taccttcaag aaattaacaa aattacccac aattaaaaaa 60

gggtttccca gtttcactca cacttattca agattgatgc aaaataaacc cataaataag 120

aaggttaaaa tagtgttaaa gccttaaagg tgcttgcttt ttgtgttttt tgtgatgcct 180

ataaaaagat tcttattgtt 200

<210> 418
<211> 200
<212> DNA
<213> Conyza canadensis

<400> 418

aacaataaga atctttttat aggcatcaca aaaaacacaa aaagcaagca cctttaaggc 60

tttaacacta ttttaacctt cttatttatg ggtttatttt gcatcaatct tgaataagtg 120

tgagtgaaac tgggaaaccc ttttttaatt gtgggtaatt ttgttaattt cttgaaggta 180

agtggcaatt gaataaagta 200

<210> 419
<211> 200
<212> DNA
<213> Conyza canadensis

<400> 419

tgcctataaa aagattctta ttgttttcaa atttttttat gtcaagtttt cgtttttaac 60

agtttatata gttatataat ttgcagaaag ccataaagct ctgaacttga atgttttata 120

gcagctcttt gcagtaatat cttgtagagg taaacctgtt aacccatatt tattaatttg 180

tacataaaat tgttgaattg 200

<210> 420
<211> 200
<212> DNA
<213> Conyza canadensis

<400> 420

caattcaaca attttatgta caaattaata aatatgggtt aacaggttta cctctacaag 60

atattactgc aaagagctgc tataaaacat tcaagttcag agctttatgg ctttctgcaa 120

attatataac tatataaact gttaaaaacg aaaacttgac ataaaaaaat ttgaaaacaa 180

taagaatctt tttataggca 200

<210> 421
<211> 200
<212> DNA
<213> Conyza canadensis

<400> 421

atttgtacat aaaattgttg aattgaatag agctaccact gggacttgtg tagtcaaact 60

gtagagcatc cctgtaatgc tggaagcttt atgacctaaa aggatttgta gacttgttat 120

ctgtggattg gtataaagat tttcaagttg ttattttgaa actttgaata gtatattatg 180

ctcattactg aatgatacaa 200

<210> 422
<211> 200
<212> DNA
<213> Conyza canadensis

<400> 422

ttgtatcatt cagtaatgag cataatatac tattcaaagt ttcaaaataa caacttgaaa 60

atctttatac caatccacag ataacaagtc tacaaatcct tttaggtcat aaagcttcca 120

gcattacagg gatgctctac agtttgacta cacaagtccc agtggtagct ctattcaatt 180

caacaatttt atgtacaaat 200

<210> 423
<211> 200
<212> DNA
<213> Conyza canadensis

<400> 423

ttatgctcat tactgaatga tacaataggg agtcgcgaat gttagaattg ataagaatta 60

ggatgaagtt tgtgtttgaa cgttccttct gtttataatc ttcatgtcac ttccatagta 120

ccataaccct agggcaacac gagcctttag attatgaatg ggcacaccca attgtttaga 180

ccttgtataa atgaagtgaa 200

<210> 424
<211> 200
<212> DNA
<213> Conyza canadensis

<400> 424

ttcacttcat ttatacaagg tctaaacaat tgggtgtgcc cattcataat ctaaaggctc 60

gtgttgccct agggttatgg tactatggaa gtgacatgaa gattataaac agaaggaacg 120

ttcaaacaca aacttcatcc taattcttat caattctaac attcgcgact ccctattgta 180

tcattcagta atgagcataa 200

<210> 425
<211> 200
<212> DNA
<213> Conyza canadensis

<400> 425

ttagaccttg tataaatgaa gtgaatatca ttgttataag cattctggtg gtggttttgc 60

agatttacct ttctctatct aatttgaagg tttgttgatt aaggtgatac aaatgagtgt 120

tttcaagcag ctaatagctt caagattagt acgcactcat gtctgtggat cttatagagg 180

tacacttcct cgaacattca 200

<210> 426
<211> 200
<212> DNA
<213> Conyza canadensis

<400> 426

tgaatgttcg aggaagtgta cctctataag atccacagac atgagtgcgt actaatcttg 60

aagctattag ctgcttgaaa acactcattt gtatcacctt aatcaacaaa ccttcaaatt 120

agatagagaa aggtaaatct gcaaaaccac caccagaatg cttataacaa tgatattcac 180

ttcatttata caaggtctaa 200

<210> 427
<211> 200
<212> DNA
<213> Conyza canadensis

<400> 427

agaggtacac ttcctcgaac attcatatag cgatgttatt gttaagtttt gaatctgtag 60

caacattata tataattttt tttgcattgt tttcctgcag catccttttc actctttcat 120

tcgtcctcgg atacttcaat acaagtgcat tctccagaac aagaagtagt aattgcttta 180

ggtagcaatg tgggtaatag 200

<210> 428
<211> 200
<212> DNA
<213> Conyza canadensis

<400> 428

ctattaccca cattgctacc taaagcaatt actacttctt gttctggaga atgcacttgt 60

attgaagtat ccgaggacga atgaaagagt gaaaaggatg ctgcaggaaa acaatgcaaa 120

aaaaattata tataatgttg ctacagattc aaaacttaac aataacatcg ctatatgaat 180

gttcgaggaa gtgtacctct 200

<210> 429
<211> 200
<212> DNA
<213> Conyza canadensis

<400> 429

ctttaggtag caatgtgggt aatagactta ataactttaa tgaagcctta tcccagatga 60

agaaatcggg gatagaaata acaaggcatg cttgtctata cgaaactgaa ccagcttatg 120

tgacggacca gcctcttttt ctcaactctg ccgttagagc cactacaaag cttggccctc 180

atgagctact gtccattctc 200

<210> 430
<211> 200
<212> DNA
<213> Conyza canadensis

<400> 430

gagaatggac agtagctcat gagggccaag ctttgtagtg gctctaacgg cagagttgag 60

aaaaagaggc tggtccgtca cataagctgg ttcagtttcg tatagacaag catgccttgt 120

tatttctatc cccgatttct tcatctggga taaggcttca ttaaagttat taagtctatt 180

acccacattg ctacctaaag 200

<210> 431
<211> 200
<212> DNA
<213> Conyza canadensis

<400> 431

ccctcatgag ctactgtcca ttctcaagaa aattgaaaag gaaatgggtc gaaccaaagg 60

gcttaggtac ggcccacgac ccattgacct agatatattg ttctatggta aatgcaaagt 120

taactctgat attctaactg ttcctcatga aagaatcttt gagaggccat tcgttatggc 180

tccattagtt gacttgttag 200

<210> 432
<211> 200
<212> DNA
<213> Conyza canadensis

<400>       432

ctaacaagtc aactaatgga gccataacga atggcctctc aaagattctt tcatgaggaa    60

cagttagaat atcagagtta actttgcatt taccatagaa caatatatct aggtcaatgg   120

gtcgtgggcc gtacctaagc cctttggttc gacccatttc cttttcaatt ttcttgagaa   180

tggacagtag ctcatgaggg                                               200


<210>       433
<211>       200
<212>       DNA
<213>       Conyza canadensis

<400>       433

atggctccat tagttgactt gttaggatca gatgtagaca atgatacggt tctatgctgg    60

cattcttttt caaaaaatgg gctttttgga tcttgggaaa cattaggtgg tgaatcttcc   120

ataggaaaag atggtttaag aagggtttta cctgtcaatg atcgtttatg ggattggtca   180

aagaaaactt ccgtcatggg                                               200


<210>       434
<211>       200
<212>       DNA
<213>       Conyza canadensis

<400>       434

cccatgacgg aagttttctt tgaccaatcc cataaacgat cattgacagg taaaaccctt    60

cttaaaccat cttttcctat ggaagattca ccacctaatg tttcccaaga tccaaaaagc   120

ccattttttg aaaaagaatg ccagcataga accgtatcat tgtctacatc tgatcctaac   180

aagtcaacta atggagccat                                               200


<210>       435
<211>       200
<212>       DNA
<213>       Conyza canadensis

<400>       435

ggtcaaagaa aacttccgtc atgggtattt tgaatataac tcctgatagt tttagtgatg    60

gagggaagtt tgattccatg gggtccgctt tatctcgtgt tcagaccatg atatctgaag   120

gggttgacat aattgatcta ggagctcaat ccacacgccc aatggcgacc aagatctcag   180

tcgaagagga actagatagg                                               200


<210>       436
<211>       200
<212>       DNA
<213>       Conyza canadensis

<400>       436

cctatctagt tcctcttcga ctgagatctt ggtcgccatt gggcgtgtgg attgagctcc    60

tagatcaatt atgtcaaccc cttcagatat catggtctga acacgagata aagcggaccc   120

catggaatca aacttccctc catcactaaa actatcagga gttatattca aaatacccat   180

gacggaagtt ttctttgacc                                                200


<210>       437
<211>       200
<212>       DNA
<213>       Conyza canadensis

<400>       437

ctcagtcgaa gaggaactag ataggctaat acccgttctt gaaaagattc ttgaattacc    60

tgaaattgaa ggaaagttgt tgtctgtgga cacattttac tcggaagttg cttcagaggc   120

aatcaagaaa ggggctcata tggtcaatga tgtatcgggt ggaatgttag attctgatat   180

gcttcatgtt gtggctgatc                                                200


<210>       438
<211>       200
<212>       DNA
<213>       Conyza canadensis

<400>       438

gatcagccac aacatgaagc atatcagaat ctaacattcc acccgataca tcattgacca    60

tatgagcccc tttcttgatt gcctctgaag caacttccga gtaaaatgtg tccacagaca   120

acaactttcc ttcaatttca ggtaattcaa gaatcttttc aagaacgggt attagcctat   180

ctagttcctc ttcgactgag                                                200


<210>       439
<211>       200
<212>       DNA
<213>       Conyza canadensis

<400>       439

gatatgcttc atgttgtggc tgatctaaat gttccatata tcactatgca catgagaggg    60

gacccatcca caatgcaaaa cagtgagaat ttgaagtatg atgatgttgt caaagaagtt   120

ggggaggaat tgtatgaacg tgtaaggaat gcagagttat gtggtgttcc cgcatggagg   180

atggttcttg atccagggat                                                200


<210>       440
<211>       200
<212>       DNA

<213>       Conyza canadensis

<400>       440

atccctggat caagaaccat cctccatgcg ggaacaccac ataactctgc attccttaca    60

cgttcataca attcctcccc aacttctttg acaacatcat catacttcaa attctcactg   120

ttttgcattg tggatgggtc ccctctcatg tgcatagtga tatatggaac atttagatca   180

gccacaacat gaagcatatc                                               200

<210>       441
<211>       200
<212>       DNA
<213>       Conyza canadensis

<400>       441

ggaggatggt tcttgatcca gggatcgggt tttcaaagaa aaccgaagat aatttggaga    60

tattgatggg actaaagagg tttagaagtg agattggaca aaagagctta ggggtgtctc   120

gtgcaccttt gttaatcgga ccttcaagaa aaaggttttt gggtgagatt tgtggtcggc   180

cttctgctgt tgagagagat                                               200

<210>       442
<211>       200
<212>       DNA
<213>       Conyza canadensis

<400>       442

atctctctca acagcagaag gccgaccaca aatctcaccc aaaaaccttt ttcttgaagg    60

tccgattaac aaaggtgcac gagacacccc taagctcttt tgtccaatct cacttctaaa   120

cctctttagt cccatcaata tctccaaatt atcttcggtt ttctttgaaa acccgatccc   180

tggatcaaga accatcctcc                                               200

<210>       443
<211>       200
<212>       DNA
<213>       Conyza canadensis

<400>       443
   tcggccttct gctgttgaga gagatccagg gactgtagct gctgttacca gtgcgatttt    60
   gggtggtgcc aatatcgtta gggttcataa cattggacat aatgtggatg ctgttaagct   120
   ttgtgattca atgttggatc gagctggtag atcttagtag cttaaagtta tttgacatct   180
   tgttttttat tcaattttct                                                200

<210>       444
<211>       200

<212>       DNA
<213>       Conyza canadensis

<400>       444

agaaaattga ataaaaaaca agatgtcaaa taactttaag ctactaagat ctaccagctc    60

gatccaacat tgaatcacaa agcttaacag catccacatt atgtccaatg ttatgaaccc   120

taacgatatt ggcaccaccc aaaatcgcac tggtaacagc agctacagtc cctggatctc   180

tctcaacagc agaaggccga                                               200


<210>       445
<211>       200
<212>       DNA
<213>       Conyza canadensis

<400>       445

catcttgttt tttattcaat tttctggaca ttactttttg tatttctgtc tggcaaatga    60

ttttacaggg atatcctaga ggctaaattg tatattacat tcttgatttt ctcacaaagt   120

tttacttttt cagtttttct tgttcagttt tgtttttgaa aatttgctag atcagattaa   180

atcgttgctt tatgatcttc                                               200


<210>       446
<211>       200
<212>       DNA
<213>       Conyza canadensis

<400>       446

gaagatcata aagcaacgat ttaatctgat ctagcaaatt ttcaaaaaca aaactgaaca    60

agaaaaactg aaaaagtaaa actttgtgag aaaatcaaga atgtaatata caatttagcc   120

tctaggatat ccctgtaaaa tcatttgcca gacagaaata caaaaagtaa tgtccagaaa   180

attgaataaa aaacaagatg                                               200


<210>       447
<211>       200
<212>       DNA
<213>       Conyza canadensis

<400>       447

attaaatcgt tgctttatga tcttcgtgtc ttttttggga cgctgcgagt ttcatttagt    60

ggtattgtaa actcctttta aagtttgtac acacttggta tttatgcttc tagttttcgt   120

ctatgtctgt cctatattgt gtatagtatg tttaaagttc ataaaaagag gataaacgaa   180

tagcacgtaa atcttataat                                               200


<210>       448

<211>	200
<212>	DNA
<213>	Conyza canadensis

<400>	448

attataagat ttacgtgcta ttcgtttatc ctctttttat gaactttaaa catactatac	60

acaatatagg acagacatag acgaaaacta gaagcataaa taccaagtgt gtacaaactt	120

taaaaggagt ttacaatacc actaaatgaa actcgcagcg tcccaaaaaa gacacgaaga	180

tcataaagca acgatttaat	200

<210>	449
<211>	200
<212>	DNA
<213>	Conyza canadensis

<400>	449

acgaatagca cgtaaatctt ataattcgat tccacagagt gcaagttatg ctttcctaaa	60

tgaaagcaag agtctagaca gtttggtcaa cgttaacatg agactattgt agaatacaac	120

accacaagaa accaaaactg tcttgtgcat ttctctcttt tccaggttta aaactcaata	180

ccaagacttg atatcaacta	200

<210>	450
<211>	200
<212>	DNA
<213>	Conyza canadensis

<400>	450

tagttgatat caagtcttgg tattgagttt taaacctgga aaagagagaa atgcacaaga	60

cagttttggt ttcttgtggt gttgtattct acaatagtct catgttaacg ttgaccaaac	120

tgtctagact cttgctttca tttaggaaag cataacttgc actctgtgga atcgaattat	180

aagatttacg tgctattcgt	200

<210>	451
<211>	200
<212>	DNA
<213>	Conyza canadensis

<400>	451

caataccaag acttgatatc aactattatt ctgcttcaat gattcagctt cagctatagc	60

cataggatca tctttggtgt caggttcatc cttctcacca tctcgttcct ctgccagttt	120

ttttctgtgc aattcctctg caacttgctt tgatgcttgg tcttcttttt gaaggcgttg	180

catctcttct tcttgttgct	200

<210>	452
<211>	200
<212>	DNA
<213>	Conyza canadensis

<400>	452

agcaacaaga agaagagatg caacgccttc aaaaagaaga ccaagcatca aagcaagttg	60

cagaggaatt gcacagaaaa aaactggcag aggaacgaga tggtgagaag gatgaacctg	120

acaccaaaga tgatcctatg gctatagctg aagctgaatc attgaagcag aataatagtt	180

gatatcaagt cttggtattg	200

<210>	453
<211>	200
<212>	DNA
<213>	Conyza canadensis

<400>	453

cgttgcatct cttcttcttg ttgctgtcgt tcaaaccatg tatccgcatc ggcccaaact	60

gtaatttaga gccaaaacaa catgattaga atgcagattc cagaagccaa attcaaactg	120

taagaaaaaa aatgtagtga tgatttaact caaaacaact cacacagctc caaaatggat	180

gggggttcaa attagaggat	200

<210>	454
<211>	200
<212>	DNA
<213>	Conyza canadensis

<400>	454

atcctctaat ttgaaccccc atccattttg gagctgtgtg agttgttttg agttaaatca	60

tcactacatt ttttttctta cagtttgaat ttggcttctg gaatctgcat tctaatcatg	120

ttgttttggc tctaaattac agtttgggcc gatgcggata catggtttga acgacagcaa	180

caagaagaag agatgcaacg	200

<210>	455
<211>	200
<212>	DNA
<213>	Conyza canadensis

<400>	455

tggatggggg ttcaaattag aggatagcct aatagataga agcttctaat tatggggaaa	60

aaaaaacgat catggcaatg tattacacaa tctaaagcaa ccactatgaa taataaagat	120

aaatctttcc gagagtccac atgccttgat atttcataag ataggctttt tcagaaatca	180

gaatcttctt ggcaaatata	200

<210>	456
<211>	200
<212>	DNA
<213>	Conyza canadensis

<400>	456

tatatttgcc aagaagattc tgatttctga aaaagcctat cttatgaaat atcaaggcat	60

gtggactctc ggaaagattt atctttatta ttcatagtgg ttgctttaga ttgtgtaata	120

cattgccatg atcgtttttt tttccccata attagaagct tctatctatt aggctatcct	180

ctaatttgaa cccccatcca	200

<210>	457
<211>	200
<212>	DNA
<213>	Conyza canadensis

<400>	457

aatcagaatc ttcttggcaa atataacact aaaatcagtc taatgcatgt tattaagtaa	60

atctttattt gtccatagta taatgccata gattatcgtc aattatccac atcgtcatta	120

aatatcaagt aaccacatta tgaattgact tgtttaatca aagttcatct tacttcttca	180

tagatgaaaa agagctatgc	200

<210>	458
<211>	200
<212>	DNA
<213>	Conyza canadensis

<400>	458

gcatagctct ttttcatcta tgaagaagta agatgaactt tgattaaaca agtcaattca	60

taatgtggtt acttgatatt taatgacgat gtggataatt gacgataatc tatggcatta	120

tactatggac aaataaagat ttacttaata acatgcatta gactgatttt agtgttatat	180

ttgccaagaa gattctgatt	200

<210>	459
<211>	200
<212>	DNA
<213>	Conyza canadensis

<400>	459

cttcatagat gaaaaagagc tatgcaaata cagaacacat cagttgttct aacatataat	60

tactgctagc tgtaggaaga aaccaagatg aaacttatct aggattcaaa ttcatgggga	120

tctgttattt agatgaattt agagaaataa gaaaataagt tgagtgaagt cagtttcgag	180

gttccaaatt tgtctttatt	200

<210>	460
<211>	200
<212>	DNA
<213>	Conyza canadensis

<400>	460

aataaagaca aatttggaac ctcgaaactg acttcactca acttattttc ttatttctct	60

aaattcatct aaataacaga tccccatgaa tttgaatcct agataagttt catcttggtt	120

tcttcctaca gctagcagta attatatgtt agaacaactg atgtgttctg tatttgcata	180

gctctttttc atctatgaag	200

<210>	461
<211>	200
<212>	DNA
<213>	Conyza canadensis

<400>	461

tcgaggttcc aaatttgtct ttattagttg agctaacaat attaacctct gaagcatgta	60

tagtgtactt gtatgaacca cttaaggata taaatgaagc caaatcagtt tcattcgcta	120

attgagcaaa tgcacgttct gtttgttagt attgaaattt ctttacagga tttctttcaa	180

ctaaaagcat cacatgtccg	200

<210>	462
<211>	200
<212>	DNA
<213>	Conyza canadensis

<400>	462

cggacatgtg atgcttttag ttgaaagaaa tcctgtaaag aaatttcaat actaacaaac	60

agaacgtgca tttgctcaat tagcgaatga aactgatttg gcttcattta tatccttaag	120

tggttcatac aagtacacta tacatgcttc agaggttaat attgttagct caactaataa	180

agacaaattt ggaacctcga	200

<210>	463
<211>	200
<212>	DNA
<213>	Conyza canadensis

<400>	463

ttcaactaaa agcatcacat gtccgactct cggtttgata tttgtgacaa gtctaattgg	60

ttcatcagat gtacttatgg ctaaatttcg aaacttgaaa caataagagc ttagcactct	120

taaccataaa gggttgctgt taatcttact tgttcttgaa agcaattcca cagtgaagca	180

tttgttaatt gggaagtttt 200

<210> 464
<211> 200
<212> DNA
<213> Conyza canadensis

<400> 464

aaaacttccc aattaacaaa tgcttcactg tggaattgct ttcaagaaca agtaagatta 60

acagcaaccc tttatggtta agagtgctaa gctcttattg tttcaagttt cgaaatttag 120

ccataagtac atctgatgaa ccaattagac ttgtcacaaa tatcaaaccg agagtcggac 180

atgtgatgct tttagttgaa 200

<210> 465
<211> 200
<212> DNA
<213> Conyza canadensis

<400> 465

ttttcttctt ctagttggag ctcttttacg tggtgtcttc ttttccgagt catcttcttc 60

cccatcagat ggattttctg cagccttttt tctacgaact gctgtaatag caaatgtact 120

acgcaatcta acatctggga attgtacaaa gtttattgaa gatagtgaag gcagattcga 180

ctgccaccta cacatataag 200

<210> 466
<211> 200
<212> DNA
<213> Conyza canadensis

<400> 466

cttatatgtg taggtggcag tcgaatctgc cttcactatc ttcaataaac tttgtacaat 60

tcccagatgt tagattgcgt agtacatttg ctattacagc agttcgtaga aaaaaggctg 120

cagaaaatcc atctgatggg gaagaagatg actcggaaaa gaagacacca cgtaaaagag 180

ctccaactag aagaagaaaa 200

<210> 467
<211> 200
<212> DNA
<213> Conyza canadensis

<400> 467

ttcgactgcc acctacacat ataagaaacc attaacttct atacttaact cacaatataa 60

aacatcattt gcatgttcgt caaaaacaat accatttctt atctttgctc aatggatatg 120

aaatctgcca atacaagatt attcatttga cctcaatcta gcatctgatg tctattcaag 180

gtatcgttta taatgaagat 200

<210> 468
<211> 200
<212> DNA
<213> Conyza canadensis

<400> 468

atcttcatta taaacgatac cttgaataga catcagatgc tagattgagg tcaaatgaat 60

aatcttgtat tggcagattt catatccatt gagcaaagat aagaaatggt attgtttttg 120

acgaacatgc aaatgatgtt ttatattgtg agttaagtat agaagttaat ggtttcttat 180

atgtgtaggt ggcagtcgaa 200

<210> 469
<211> 200
<212> DNA
<213> Conyza canadensis

<400> 469

tcaaggtatc gtttataatg aagattattg tacttcaaaa tactttcttg tgtagtggta 60

tcaaattttg agttaaagtt actccatttg acctcaaaag ccaaataatg agcaacaaac 120

aacacaattt cacctccctt ataccctgcg actatatttt atcttcaaat caatccattt 180

ccatgtttat cctctccata 200

<210> 470
<211> 200
<212> DNA
<213> Conyza canadensis

<400> 470

tatggagagg ataaacatgg aaatggattg atttgaagat aaaatatagt cgcagggtat 60

aagggaggtg aaattgtgtt gtttgttgct cattatttgg cttttgaggt caaatggagt 120

aactttaact caaaatttga taccactaca caagaaagta ttttgaagta caataatctt 180

cattataaac gataccttga 200

<210> 471
<211> 200
<212> DNA
<213> Conyza canadensis

<400> 471

catttccatg tttatcctct ccatacagct acataataag ttttagggaa aaaacaaaac 60

taaactaaac taagtttaca gaattcccat ttcttatcct aaaatttaca agcaattcaa 120

catatattct ttatttacaa ctataaatag catatgggta tgttggtata tagatataga 180

tatagatata catatgtaga 200

<210> 472
<211> 200
<212> DNA
<213> Conyza canadensis

<400> 472

tctacatatg tatatctata tctatatcta tataccaaca tacccatatg ctatttatag 60

ttgtaaataa agaatatatg ttgaattgct tgtaaatttt aggataagaa atgggaattc 120

tgtaaactta gtttagttta gttttgtttt ttccctaaaa cttattatgt agctgtatgg 180

agaggataaa catggaaatg 200

<210> 473
<211> 200
<212> DNA
<213> Conyza canadensis

<400> 473
   atagatatag atatacatat gtagatgtgt attatatgtg tgtaaatggg gagaaaaaag 60
   gaaacatata ttacctgtgt aaagaacaaa attgagtaaa agaaggcaaa gaagccattt 120
   ctaaccacta aaatcttggg ctttcaagaa tcccacttca aagctaccat ttcacatacc 180
   aaacccccct ttctatgggt 200

<210> 474
<211> 200
<212> DNA
<213> Conyza canadensis

<400> 474

acccatagaa agggggtttt ggtatgtgaa atggtagctt tgaagtggga ttcttgaaag 60

cccaagattt tagtggttag aaatggcttc tttgccttct tttactcaat tttgttcttt 120

acacaggtaa tatatgtttc cttttttctc cccatttaca cacatataat acacatctac 180

atatgtatat ctatatctat 200

<210> 475
<211> 200
<212> DNA
<213> Conyza canadensis

<400> 475

ataccaaacc cccctttcta tgggtttaaa gatgagggct ttacatttat ctcttttaca 60

atccaaattt tccaaaaccg aaataatgta cgacattcgg tgcacaaggt acaaacagaa 120

aaaactactt ttgtatttta gatctcaaga aaggagtggc ctttttggta ataacatact 180

ttattcaatt gccacttacc 200

<210> 476
<211> 200
<212> DNA
<213> Conyza canadensis

<400> 476

ggtaagtggc aattgaataa agtatgttat taccaaaaag gccactcctt tcttgagatc 60

taaaatacaa aagtagtttt ttctgtttgt accttgtgca ccgaatgtcg tacattattt 120

cggttttgga aaatttggat tgtaaaagag ataaatgtaa agccctcatc tttaaaccca 180

tagaaagggg ggtttggtat 200

<210> 477
<211> 200
<212> DNA
<213> Conyza canadensis

<400> 477

atactttatt caattgccac ttaccttcaa gaaattaaca aaattaccca caattaaaaa 60

agggtttccc agtttcactc acacttattc aagattgatg caaaataaac ccataaataa 120

gaaggttaaa atagtgttaa agccttaaag gtgcttgctt tttgtgtttt ttgtgatgcc 180

tataaaaaga ttcttattgt 200

<210> 478
<211> 200
<212> DNA
<213> Conyza canadensis

<400> 478

acaataagaa tctttttata ggcatcacaa aaaacacaaa aagcaagcac ctttaaggct 60

ttaacactat tttaaccttc ttatttatgg gtttattttg catcaatctt gaataagtgt 120

gagtgaaact gggaaaccct tttttaattg tgggtaattt tgttaatttc ttgaaggtaa 180

gtggcaattg aataaagtat 200

<210> 479
<211> 200
<212> DNA
<213> Conyza canadensis

<400> 479

atgcctataa aaagattctt attgttttca aattttttta tgtcaagttt tcgtttttaa 60

cagtttatat agttatataa tttgcagaaa gccataaagc tctgaacttg aatgttttat 120

agcagctctt tgcagtaata tcttgtagag gtaaacctgt taacccatat ttattaattt 180

gtacataaaa ttgttgaatt 200

<210> 480
<211> 200
<212> DNA
<213> Conyza canadensis

<400> 480

aattcaacaa ttttatgtac aaattaataa atatgggtta acaggtttac ctctacaaga 60

tattactgca aagagctgct ataaaacatt caagttcaga gctttatggc tttctgcaaa 120

ttatataact atataaactg ttaaaaacga aaacttgaca taaaaaaatt tgaaaacaat 180

aagaatcttt ttataggcat 200

<210> 481
<211> 200
<212> DNA
<213> Conyza canadensis

<400> 481

aatttgtaca taaaattgtt gaattgaata gagctaccac tgggacttgt gtagtcaaac 60

tgtagagcat ccctgtaatg ctggaagctt tatgacctaa aaggatttgt agacttgtta 120

tctgtggatt ggtataaaga ttttcaagtt gttattttga aactttgaat agtatattat 180

gctcattact gaatgataca 200

<210> 482
<211> 200
<212> DNA
<213> Conyza canadensis

<400> 482

tgtatcattc agtaatgagc ataatatact attcaaagtt tcaaaataac aacttgaaaa 60

tctttatacc aatccacaga taacaagtct acaaatcctt ttaggtcata aagcttccag 120

cattacaggg atgctctaca gtttgactac acaagtccca gtggtagctc tattcaattc 180

aacaatttta tgtacaaatt 200

<210> 483
<211> 200
<212> DNA
<213> Conyza canadensis

<400> 483

attatgctca ttactgaatg atacaatagg gagtcgcgaa tgttagaatt gataagaatt 60

aggatgaagt ttgtgtttga acgttccttc tgtttataat cttcatgtca cttccatagt 120

accataaccc tagggcaaca cgagccttta gattatgaat gggcacaccc aattgtttag 180

accttgtata aatgaagtga 200

<210> 484
<211> 200
<212> DNA
<213> Conyza canadensis

<400> 484

tcacttcatt tatacaaggt ctaaacaatt gggtgtgccc attcataatc taaaggctcg 60

tgttgcccta gggttatggt actatggaag tgacatgaag attataaaca gaaggaacgt 120

tcaaacacaa acttcatcct aattcttatc aattctaaca ttcgcgactc cctattgtat 180

cattcagtaa tgagcataat 200

<210> 485
<211> 200
<212> DNA
<213> Conyza canadensis

<400> 485

tttagacctt gtataaatga agtgaatatc attgttataa gcattctggt ggtggttttg 60

cagatttacc tttctctatc taatttgaag gtttgttgat taaggtgata caaatgagtg 120

ttttcaagca gctaatagct tcaagattag tacgcactca tgtctgtgga tcttatagag 180

gtacacttcc tcgaacatta 200

<210> 486
<211> 200
<212> DNA
<213> Conyza canadensis

<400> 486

taatgttcga ggaagtgtac ctctataaga tccacagaca tgagtgcgta ctaatcttga 60

agctattagc tgcttgaaaa cactcatttg tatcacctta atcaacaaac cttcaaatta 120

gatagagaaa ggtaaatctg caaaaccacc accagaatgc ttataacaat gatattcact 180

tcatttatac aaggtctaaa 200

<210> 487
<211> 200
<212> DNA
<213> Conyza canadensis

<400> 487

tagaggtaca cttcctcgaa cattatatag cgatgttatt gttaagtttt gaatctgtag 60

caacattata tataattttt tttgcattgt tttcctgcag catccttttc actctttcat 120

tcgtcctcgg atacttcaat acaagtgcat tctccagaac aagaagtagt aattgcttta 180

ggtagcaatg tgggtaatag 200

<210> 488
<211> 200
<212> DNA
<213> Conyza canadensis

<400> 488

ctattaccca cattgctacc taaagcaatt actacttctt gttctggaga atgcacttgt 60

attgaagtat ccgaggacga atgaaagagt gaaaaggatg ctgcaggaaa acaatgcaaa 120

aaaaattata tataatgttg ctacagattc aaaacttaac aataacatcg ctatataatg 180

ttcgaggaag tgtacctcta 200

<210> 489
<211> 200
<212> DNA
<213> Conyza canadensis

<400> 489

ctttaggtag caatgtgggt aatagactta ataactttaa tgaagcctta tcccagatga 60

agaaatcggg gatagaaata acaaggcatg cttgtctata cgaaactgaa ccagcttatg 120

tgacggacca gcctcttttt ctcaactctg ccgttagagc cactacaaag cttggccctc 180

atgagctact gtccattctc 200

<210> 490
<211> 200
<212> DNA
<213> Conyza canadensis

<400> 490

gagaatggac agtagctcat gagggccaag ctttgtagtg gctctaacgg cagagttgag 60

aaaaagaggc tggtccgtca cataagctgg ttcagtttcg tatagacaag catgccttgt 120

tatttctatc cccgatttct tcatctggga taaggcttca ttaaagttat taagtctatt 180

acccacattg ctacctaaag 200

<210> 491
<211> 200
<212> DNA
<213> Conyza canadensis

<400> 491

ccctcatgag ctactgtcca ttctcaagaa aattgaaaag gaaatgggtc gaaccaaagg 60

gcttaggtac ggcccacgac ccattgacct agatatattg ttctatggta aatgcaaagt 120

taactctgat attctaactg ttcctcatga aagaatcttt gagaggccat tcgttatggc 180

tccattagtt gacttgttag 200

<210> 492
<211> 200
<212> DNA
<213> Conyza canadensis

<400> 492

ctaacaagtc aactaatgga gccataacga atggcctctc aaagattctt tcatgaggaa 60

cagttagaat atcagagtta actttgcatt taccatagaa caatatatct aggtcaatgg 120

gtcgtgggcc gtacctaagc cctttggttc gacccatttc cttttcaatt ttcttgagaa 180

tggacagtag ctcatgaggg 200

<210> 493
<211> 200
<212> DNA
<213> Conyza canadensis

<400> 493

atggctccat tagttgactt gttaggatca gatgtagaca atgatacggt tctatgctgg 60

cattcttttt caaaaaatgg gctttttgga tcttgggaaa cattaggtgg tgaatcttcc 120

ataggaaaag atggtttaag aagggtttta cctgtcaatg atcgtttatg ggattggtca 180

aagaaaactt ccgtcatggg 200

<210> 494
<211> 200
<212> DNA
<213> Conyza canadensis

<400> 494

cccatgacgg aagttttctt tgaccaatcc cataaacgat cattgacagg taaaaccctt 60

cttaaaccat cttttcctat ggaagattca ccacctaatg tttcccaaga tccaaaaagc 120

ccattttttg aaaaagaatg ccagcataga accgtatcat tgtctacatc tgatcctaac 180

aagtcaacta atggagccat 200

<210> 495
<211> 200
<212> DNA
<213> Conyza canadensis

<400>       495

ggtcaaagaa aacttccgtc atgggtattt tgaatataac tcctgatagt tttagtgatg    60

gagggaagtt tgattccatg gggtccgctt tatctcgtgt tcagaccatg atatctgaag   120

gggttgacat aattgatcta ggagctcaat ccacacgccc aatggcgacc aagatctcag   180

tcgaagagga actagatagg                                              200

<210>       496
<211>       200
<212>       DNA
<213>       Conyza canadensis

<400>       496

cctatctagt tcctcttcga ctgagatctt ggtcgccatt gggcgtgtgg attgagctcc    60

tagatcaatt atgtcaaccc cttcagatat catggtctga acacgagata aagcggaccc   120

catggaatca aacttccctc catcactaaa actatcagga gttatattca aaatacccat   180

gacggaagtt ttctttgacc                                              200

<210>       497
<211>       200
<212>       DNA
<213>       Conyza canadensis

<400>       497

ctcagtcgaa gaggaactag ataggctaat acccgttctt gaaaagattc ttgaattacc    60

tgaaattgaa ggaaagttgt tgtctgtgga cacattttac tcggaagttg cttcagaggc   120

aatcaagaaa ggggctcata tggtcaatga tgtatcgggt ggaatgttag attctgatat   180

gcttcatgtt gtggctgatc                                              200

<210>       498
<211>       200
<212>       DNA
<213>       Conyza canadensis

<400>       498

gatcagccac aacatgaagc atatcagaat ctaacattcc acccgataca tcattgacca    60

tatgagcccc tttcttgatt gcctctgaag caacttccga gtaaaatgtg tccacagaca   120

acaactttcc ttcaatttca ggtaattcaa gaatcttttc aagaacgggt attagcctat   180

ctagttcctc ttcgactgag                                              200

<210>       499
<211>       200
<212>       DNA
<213>       Conyza canadensis

<400>       499

gatatgcttc atgttgtggc tgatctaaat gttccatata tcactatgca catgagaggg    60

gacccatcca caatgcaaaa cagtgagaat ttgaagtatg atgatgttgt caaagaagtt   120

ggggaggaat tgtatgaacg tgtaaggaat gcagagttat gtggtgttcc cgcatggagg   180

atggttcttg atccagggat                                               200

<210>       500
<211>       200
<212>       DNA
<213>       Conyza canadensis

<400>       500

atccctggat caagaaccat cctccatgcg ggaacaccac ataactctgc attccttaca    60

cgttcataca attcctcccc aacttctttg acaacatcat catacttcaa attctcactg   120

ttttgcattg tggatgggtc ccctctcatg tgcatagtga tatatggaac atttagatca   180

gccacaacat gaagcatatc                                               200

<210>       501
<211>       200
<212>       DNA
<213>       Conyza canadensis

<400>       501

ggaggatggt tcttgatcca gggatcgggt tttcaaagaa aaccgaagat aatttggaga    60

tattgatggg actaaagagg tttagaagtg agattggaca aaagagctta ggggtgtctc   120

gtgcaccttt gttaatcgga ccttcaagaa aaaggttttt gggtgagatt tgtggtcggc   180

cttctgctgt tgagagagat                                               200

<210>       502
<211>       200
<212>       DNA
<213>       Conyza canadensis

<400>       502

atctctctca acagcagaag gccgaccaca aatctcaccc aaaaaccttt ttcttgaagg    60

tccgattaac aaaggtgcac gagacacccc taagctcttt tgtccaatct cacttctaaa   120

cctctttagt cccatcaata tctccaaatt atcttcggtt ttctttgaaa acccgatccc   180

tggatcaaga accatcctcc                                               200

<210>       503
<211>       200
<212>       DNA

<213>       Conyza canadensis

<400>       503

tcggccttct gctgttgaga gagatccagg gactgtagct gctgttacca gtgcgatttt    60

gggtggtgcc aatatcgtta gggttcataa cattggacat aatgtggatg ctgttaagct   120

ttgtgattca atgttggatc gagctggtag atcttagtag cttaaagtta tttgacatct   180

tgtttttat tcaattttct                                                200


<210>       504
<211>       200
<212>       DNA
<213>       Conyza canadensis

<400>       504

agaaaattga ataaaaaaca agatgtcaaa taactttaag ctactaagat ctaccagctc    60

gatccaacat tgaatcacaa agcttaacag catccacatt atgtccaatg ttatgaaccc   120

taacgatatt ggcaccaccc aaaatcgcac tggtaacagc agctacagtc cctggatctc   180

tctcaacagc agaaggccga                                               200


<210>       505
<211>       200
<212>       DNA
<213>       Conyza canadensis

<400>       505

catcttgttt tttattcaat tttctggaca ttactttttg tatttctgtc tggcaaatga    60

ttttacaggg atatcctaga ggctaaattg tatattacat tcttgatttt ctcacaaagt   120

tttacttttt cagtttttct tgttcagttt tgtttttgaa aatttgctag atcagattaa   180

atcgttgctt tatgatcttc                                               200


<210>       506
<211>       200
<212>       DNA
<213>       Conyza canadensis

<400>       506

gaagatcata aagcaacgat ttaatctgat ctagcaaatt ttcaaaaaca aaactgaaca    60

agaaaaactg aaaaagtaaa actttgtgag aaaatcaaga atgtaatata caatttagcc   120

tctaggatat ccctgtaaaa tcatttgcca gacagaaata caaaaagtaa tgtccagaaa   180

attgaataaa aaacaagatg                                               200


<210>       507
<211>       200

<212>       DNA
<213>       Conyza canadensis

<400>       507

attaaatcgt tgctttatga tcttcgtgtc ttttttggga cgctgcgagt ttcatttagt    60

ggtattgtaa actcctttta aagtttgtac acacttggta tttatgcttc tagttttcgt   120

ctatgtctgt cctatattgt gtatagtatg tttaaagttc ataaaaagag gataaacgaa   180

tagcacgtaa atcttataat                                               200


<210>       508
<211>       200
<212>       DNA
<213>       Conyza canadensis

<400>       508

attataagat ttacgtgcta ttcgtttatc ctctttttat gaactttaaa catactatac    60

acaatatagg acagacatag acgaaaacta gaagcataaa taccaagtgt gtacaaactt   120

taaaaggagt ttacaatacc actaaatgaa actcgcagcg tcccaaaaaa gacacgaaga   180

tcataaagca acgatttaat                                               200


<210>       509
<211>       200
<212>       DNA
<213>       Conyza canadensis

<400>       509

acgaatagca cgtaaatctt ataattcgat tccacagagt gcaagttatg ctttcctaaa    60

tgaaagcaag agtctagaca gtttggtcaa cgttaacatg agactattgt agaatacaac   120

accacaagaa accaaaactg tcttgtgcat ttctctcttt tccaggttta aaactcaata   180

ccaagacttg atatcaacta                                               200


<210>       510
<211>       200
<212>       DNA
<213>       Conyza canadensis

<400>       510

tagttgatat caagtcttgg tattgagttt taaacctgga aaagagagaa atgcacaaga    60

cagttttggt ttcttgtggt gttgtattct acaatagtct catgttaacg ttgaccaaac   120

tgtctagact cttgctttca tttaggaaag cataacttgc actctgtgga atcgaattat   180

aagatttacg tgctattcgt                                               200


<210>       511

<211>       200
<212>       DNA
<213>       Conyza canadensis

<400>       511

caataccaag acttgatatc aactattatt ctgcttcaat gattcagctt cagctatagc    60

cataggatca tctttggtgt caggttcatc cttctcacca tctcgttcct ctgccagttt   120

ttttctgtgc aattcctctg caacttgctt tgatgcttgg tcttcttttt gaaggcgttg   180

catctcttct tcttgttgct                                               200


<210>       512
<211>       200
<212>       DNA
<213>       Conyza canadensis

<400>       512

agcaacaaga agaagagatg caacgccttc aaaaagaaga ccaagcatca aagcaagttg    60

cagaggaatt gcacagaaaa aaactggcag aggaacgaga tggtgagaag gatgaacctg   120

acaccaaaga tgatcctatg gctatagctg aagctgaatc attgaagcag aataatagtt   180

gatatcaagt cttggtattg                                               200


<210>       513
<211>       200
<212>       DNA
<213>       Conyza canadensis

<400>       513

cgttgcatct cttcttcttg ttgctgtcgt tcaaaccatg tatccgcatc ggcccaaact    60

gtaatttaga gccaaaacaa catgattaga atgcagattc cagaagccaa attcaaactg   120

taagaaaaaa aatgtagtga tgatttaact caaaacaact cacacagctc caaaatggat   180

gggggttcaa attagaggat                                               200


<210>       514
<211>       200
<212>       DNA
<213>       Conyza canadensis

<400>       514

atcctctaat ttgaaccccc atccattttg gagctgtgtg agttgttttg agttaaatca    60

tcactacatt ttttttctta cagtttgaat ttggcttctg gaatctgcat tctaatcatg   120

ttgttttggc tctaaattac agtttgggcc gatgcggata catggtttga acgacagcaa   180

caagaagaag agatgcaacg                                               200

<210>	515
<211>	200
<212>	DNA
<213>	Conyza canadensis

<400>	515

tggatggggg ttcaaattag aggatagcct aatagataga agcttctaat tatggggaaa	60

aaaaaacgat catggcaatg tattacacaa tctaaagcaa ccactatgaa taataaagat	120

aaatctttcc gagagtccac atgccttgat atttcataag ataggctttt tcagaaatca	180

gaatcttctt ggcaaatata	200

<210>	516
<211>	200
<212>	DNA
<213>	Conyza canadensis

<400>	516

tatatttgcc aagaagattc tgatttctga aaaagcctat cttatgaaat atcaaggcat	60

gtggactctc ggaaagattt atctttatta ttcatagtgg ttgctttaga ttgtgtaata	120

cattgccatg atcgtttttt tttccccata attagaagct tctatctatt aggctatcct	180

ctaatttgaa cccccatcca	200

<210>	517
<211>	200
<212>	DNA
<213>	Conyza canadensis

<400>	517

aatcagaatc ttcttggcaa atataacact aaaatcagtc taatgcatgt tattaagtaa	60

atctttattt gtccatagta taatgccata gattatcgtc aattatccac atcgtcatta	120

aatatcaagt aaccacatta tgaattgact tgtttaatca aagttcatct tacttcttca	180

tagatgaaaa agagctatgc	200

<210>	518
<211>	200
<212>	DNA
<213>	Conyza canadensis

<400>	518

gcatagctct ttttcatcta tgaagaagta agatgaactt tgattaaaca agtcaattca	60

taatgtggtt acttgatatt taatgacgat gtggataatt gacgataatc tatggcatta	120

tactatggac aaataaagat ttacttaata acatgcatta gactgatttt agtgttatat	180

ttgccaagaa gattctgatt	200

<210>	519
<211>	200
<212>	DNA
<213>	Conyza canadensis

<400>	519

cttcatagat gaaaaagagc tatgcaaata cagaacacat cagttgttct aacatataat	60

tactgctagc tgtaggaaga aaccaagatg aaacttatct aggattcaaa ttcatgggga	120

tctgttattt agatgaattt agagaaataa gaaaataagt tgagtgaagt cagtttcgag	180

gttccaaatt tgtctttatt	200

<210>	520
<211>	200
<212>	DNA
<213>	Conyza canadensis

<400>	520

aataaagaca aatttggaac ctcgaaactg acttcactca acttattttc ttatttctct	60

aaattcatct aaataacaga tccccatgaa tttgaatcct agataagttt catcttggtt	120

tcttcctaca gctagcagta attatatgtt agaacaactg atgtgttctg tatttgcata	180

gctctttttc atctatgaag	200

<210>	521
<211>	200
<212>	DNA
<213>	Conyza canadensis

<400>	521

tcgaggttcc aaatttgtct ttattagttg agctaacaat attaacctct gaagcatgta	60

tagtgtactt gtatgaacca cttaaggata taaatgaagc caaatcagtt tcattcgcta	120

attgagcaaa tgcacgttct gtttgttagt attgaaattt ctttacagga tttctttcaa	180

ctaaaagcat cacatgtccg	200

<210>	522
<211>	200
<212>	DNA
<213>	Conyza canadensis

<400>	522

cggacatgtg atgcttttag ttgaaagaaa tcctgtaaag aaatttcaat actaacaaac	60

agaacgtgca tttgctcaat tagcgaatga aactgatttg gcttcattta tatccttaag	120

tggttcatac aagtacacta tacatgcttc agaggttaat attgttagct caactaataa	180

agacaaattt ggaacctcga	200

<210>	523
<211>	200
<212>	DNA
<213>	Conyza canadensis

<400>	523

ttcaactaaa agcatcacat gtccgactct cggtttgata tttgtgacaa gtctaattgg	60

ttcatcagat gtacttatgg ctaaatttcg aaacttgaaa caataagagc ttagcactct	120

taaccataaa gggttgctgt taatcttact tgttcttgaa agcaattcca cagtgaagca	180

tttgttaatt gggaagtttt	200

<210>	524
<211>	200
<212>	DNA
<213>	Conyza canadensis

<400>	524

aaaacttccc aattaacaaa tgcttcactg tggaattgct ttcaagaaca agtaagatta	60

acagcaaccc tttatggtta agagtgctaa gctcttattg tttcaagttt cgaaatttag	120

ccataagtac atctgatgaa ccaattagac ttgtcacaaa tatcaaaccg agagtcggac	180

atgtgatgct tttagttgaa	200

<210>	525
<211>	200
<212>	DNA
<213>	Digitaria sanguinalis

<400>	525

aaatcgtaaa atttataact aacatgttcc ttcatgaatc caaaagccaa gtaaacaggt	60

ccatgagtgc ctttgtgaga ttgcttcgaa tagaatatac ctacggctct taatttcaga	120

ttctcttagg tagtgtttag gtagcgtttg gaagtagata tttaggcttg gaattaggaa	180

ttggcattga gatctacgaa	200

<210>	526
<211>	200
<212>	DNA
<213>	Digitaria sanguinalis

<400>	526

ttcgtagatc tcaatgccaa ttcctaattc caagcctaaa tatctacttc caaacgctac	60

ctaaacacta cctaagagaa tctgaaatta agagccgtag gtatattcta ttcgaagcaa	120

tctcacaaag gcactcatgg acctgtttac ttggcttttg gattcatgaa ggaacatgtt	180

agttataaat tttacgattt                                              200

<210>    527
<211>    200
<212>    DNA
<213>    Digitaria sanguinalis

<400>    527

aggaattggc attgagatct acgaatacct gccgtttgga tgtctaagaa tttggaattg    60

gaattgcagc ccaataccaa ccggcattcc ttggcctctc atctgcctgc cctcagtata   120

gatagagctt atctccttca tcctcgcgga gtggtggcaa gcggcggcga gatccggacc   180

acatggggtg tgggagcgcg                                              200

<210>    528
<211>    200
<212>    DNA
<213>    Digitaria sanguinalis

<400>    528

cgcgctccca caccccatgt ggtccggatc tcgccgccgc ttgccaccac tccgcgagga    60

tgaaggagat aagctctatc tatactgagg gcaggcagat gagaggccaa ggaatgccgg   120

ttggtattgg gctgcaattc caattccaaa ttcttagaca tccaaacggc aggtattcgt   180

agatctcaat gccaattcct                                              200

<210>    529
<211>    200
<212>    DNA
<213>    Digitaria sanguinalis

<400>    529

ggaccacatg gggtgtggga gcgcggtggc agagtggtgg caagcgatgg cgacggcacc    60

gtgccgccgt gccacattgg cacggcgacg gacgagtttg gatggtgtca gtacagacca   120

ctgcccaatt ctatcctttt gtgcatccaa acataaaatc tgaattagga tgctcttttg   180

attctacaag gcaatgtaat                                              200

<210>    530
<211>    200
<212>    DNA
<213>    Digitaria sanguinalis

<400>    530

attacattgc cttgtagaat caaaagagca tcctaattca gattttatgt ttggatgcac    60

aaaaggatag aattgggcag tggtctgtac tgacaccatc caaactcgtc cgtcgccgtg   120

ccaatgtggc acggcggcac ggtgccgtcg ccatcgcttg ccaccactct gccaccgcgc   180

tcccacaccc catgtggtcc 200

<210> 531
<211> 200
<212> DNA
<213> Digitaria sanguinalis

<400> 531

ttttgattct acaaggcaat gtaatgacca tccaaacaac agattctgaa ttggagtcaa 60

ttcaatgtta cgattgtatt ggcatcggac ctaattcaat gtcatgccct ctagtatcta 120

cattcaaacg gagcggaaaa atctacctcc gtcccaagat tccggcccaa gatcccgact 180

cttaatactt ttttcatctt 200

<210> 532
<211> 200
<212> DNA
<213> Digitaria sanguinalis

<400> 532

aagatgaaaa aagtattaag agtcgggatc ttgggccgga atcttgggac ggaggtagat 60

ttttccgctc cgtttgaatg tagatactag agggcatgac attgaattag gtccgatgcc 120

aatacaatcg taacattgaa ttgactccaa ttcagaatct gttgtttgga tggtcattac 180

attgccttgt agaatcaaaa 200

<210> 533
<211> 200
<212> DNA
<213> Digitaria sanguinalis

<400> 533

cgactcttaa tacttttttc atctttcgct gaaatttcag cccaagatcc cggcccgcct 60

ttttgcctcc acgctgggcc tggtgggacg cggccccttc acgagagtcg agaggggaaa 120

gcggaaaggg aaacaaaaaa actcaacgaa tcgccgccgc cgccttgccc tgcagcgaac 180

aattaagggc cccgtacctc 200

<210> 534
<211> 200
<212> DNA
<213> Digitaria sanguinalis

<400> 534

gaggtacggg gcccttaatt gttcgctgca gggcaaggcg gcggcggcga ttcgttgagt 60

ttttttgttt ccctttccgc tttcccctct cgactctcgt gaaggggccg cgtcccacca 120

ggcccagcgt ggaggcaaaa aggcgggccg ggatcttggg ctgaaatttc agcgaaagat 180

gaaaaaagta ttaagagtcg 200

<210> 535
<211> 200
<212> DNA
<213> Digitaria sanguinalis

<400> 535

cgaacaatta agggccccgt acctctctct gcccgttctt ccgctgcact ccgcatctga 60

gcggacgggt ggggacaccg ccgacggcgc cggggtggcg cctccgtctg tgctccaacc 120

aaggcagtgg gtgctggttg ctgccaatcg accggccggg catgctctga ccatcgagaa 180

aatcttcggg taacacaacg 200

<210> 536
<211> 200
<212> DNA
<213> Digitaria sanguinalis

<400> 536

cgttgtgtta cccgaagatt ttctcgatgg tcagagcatg cccggccggt cgattggcag 60

caaccagcac ccactgcctt ggttggagca cagacggagg cgccaccccg gcgccgtcgg 120

cggtgtcccc acccgtccgc tcagatgcgg agtgcagcgg aagaacgggc agagagaggt 180

acggggccct taattgttcg 200

<210> 537
<211> 200
<212> DNA
<213> Digitaria sanguinalis

<400> 537

gagaaaatct tcgggtaaca caacgttgtt catttcgttt gtatcgtggc atagttgatt 60

tccctaactt ttaggatgcg tttcgattca ttgtatctgt ataccaaaac agagttcgat 120

aacttagatt gaacggtagg cttcagcatc taaggcagca ggtgcaggtc tattctatca 180

ctctgaaaat tagttttaac 200

<210> 538
<211> 200
<212> DNA
<213> Digitaria sanguinalis

<400> 538

gttaaaacta attttcagag tgatagaata gacctgcacc tgctgcctta gatgctgaag 60

cctaccgttc aatctaagtt atcgaactct gttttggtat acagatacaa tgaatcgaaa 120

cgcatcctaa aagttaggga aatcaactat gccacgatac aaacgaaatg aacaacgttg 180

tgttacccga agattttctc 200

<210> 539
<211> 200
<212> DNA
<213> Digitaria sanguinalis

<400> 539

tatcactctg aaaattagtt ttaactgtct gatgatagga ggattagcta atgttgttat 60

atgaggaggt ttcttgctga ctagagagct atttgaccac ctgatagatt gcctcgttag 120

caacttagca ttatgacgac gatggcagcg agccatttca tgtagaaaac gaacatgagc 180

tgagaagctt aatcgtataa 200

<210> 540
<211> 200
<212> DNA
<213> Digitaria sanguinalis

<400> 540

ttatacgatt aagcttctca gctcatgttc gttttctaca tgaaatggct cgctgccatc 60

gtcgtcataa tgctaagttg ctaacgaggc aatctatcag gtggtcaaat agctctctag 120

tcagcaagaa acctcctcat ataacaacat tagctaatcc tcctatcatc agacagttaa 180

aactaatttt cagagtgata 200

<210> 541
<211> 200
<212> DNA
<213> Digitaria sanguinalis

<400> 541

tgagctgaga agcttaatcg tataagcttc aattggcttc ctggctgggg aggtttgcta 60

gagaacttct gtattttgtt cattcattta atctgatgta tgttggcagc tctcctgtca 120

ggtacagaaa tttagacggc cctgaaacca agcagggcaa cctcaccaat aggggggcag 180

tcgcaccaca aaggttgctt 200

<210> 542
<211> 200
<212> DNA
<213> Digitaria sanguinalis

<400> 542

aagcaacctt tgtggtgcga ctgcccccct attggtgagg ttgccctgct tggtttcagg 60

gccgtctaaa tttctgtacc tgacaggaga gctgccaaca tacatcagat taaatgaatg 120

aacaaaatac agaagttctc tagcaaacct ccccagccag gaagccaatt gaagcttata 180

cgattaagct tctcagctca 200

<210> 543
<211> 200
<212> DNA
<213> Digitaria sanguinalis

<400> 543

ggcagtcgca ccacaaaggt tgcttgcctc cagtggggca tctcctctcc ttacattgag 60

aggtttattt gacgtctaag atatctgaac tcctgcctcc agttagagaa aaggcccttg 120

ttcatacctc aaatttgtct ttgctcttct attgaccagg tgtgatggag tgatgcttgc 180

gtgaaatatc ctacaaggct 200

<210> 544
<211> 200
<212> DNA
<213> Digitaria sanguinalis

<400> 544

agccttgtag gatatttcac gcaagcatca ctccatcaca cctggtcaat agaagagcaa 60

agacaaattt gaggtatgaa caagggcctt ttctctaact ggaggcagga gttcagatat 120

cttagacgtc aaataaacct ctcaatgtaa ggagaggaga tgccccactg gaggcaagca 180

acctttgtgg tgcgactgcc 200

<210> 545
<211> 200
<212> DNA
<213> Digitaria sanguinalis

<400> 545

cttgcgtgaa atatcctaca aggctacaat ttatttgaag catttgcttt cttaattatc 60

atatgttcaa cgatgctcct ccatgctaag gagtccctta agaagatgta tcctgttgct 120

atgaactact ttggagggct cctcccatcg cattcgtttt caggtactgt tcaaatcttt 180

tcttttcaaa tcccctatca 200

<210> 546
<211> 200
<212> DNA
<213> Digitaria sanguinalis

<400> 546

tgatagggga tttgaaaaga aaagatttga acagtacctg aaaacgaatg cgatgggagg 60

agccctccaa agtagttcat agcaacagga tacatcttct taagggactc cttagcatgg 120

aggagcatcg ttgaacatat gataattaag aaagcaaatg cttcaaataa attgtagcct 180

tgtaggatat ttcacgcaag 200

<210> 547
<211> 200
<212> DNA
<213> Digitaria sanguinalis

<400> 547

tcttttcttt tcaaatcccc tatcatgcga tcaagagcag ctctaagctt ctaactcacc 60

cctttgcatt tctaaacagt gtctgatctt gttcatccgc tgaggttccc aaacagagct 120

cctagacatg ctattccatt cagggctcgt tcgtttacgc gatgttcgct tgagggatgt 180

tcaactgacc aagagattgt 200

<210> 548
<211> 200
<212> DNA
<213> Digitaria sanguinalis

<400> 548

acaatctctt ggtcagttga acatccctca agcgaacatc gcgtaaacga acgagccctg 60

aatggaatag catgtctagg agctctgttt gggaacctca gcggatgaac aagatcagac 120

actgtttaga aatgcaaagg ggtgagttag aagcttagag ctgctcttga tcgcatgata 180

ggggatttga aaagaaaaga 200

<210> 549
<211> 200
<212> DNA
<213> Digitaria sanguinalis

<400> 549

gatgttcaac tgaccaagag attgtgattg ctatgggaag caacgtgggt gatggagtca 60

gtacatttga cagggcatta caaatgatga aaagctcagg cgtgaacatc actaggcatg 120

cctgtctata tgagactgcc cctgcttatg tgaccgatca gccacggttc ctgaactctg 180

ccattcgagg tacgactagg 200

<210> 550
<211> 200
<212> DNA
<213> Digitaria sanguinalis

<400> 550

cctagtcgta cctcgaatgg cagagttcag gaaccgtggc tgatcggtca cataagcagg 60

ggcagtctca tatagacagg catgcctagt gatgttcacg cctgagcttt tcatcatttg 120

taatgccctg tcaaatgtac tgactccatc acccacgttg cttcccatag caatcacaat 180

ctcttggtca gttgaacatc 200

<210> 551
<211> 200
<212> DNA
<213> Digitaria sanguinalis

<400> 551

ctctgccatt cgaggtacga ctaggctggg acctcacgag ttgcttaaga aactaaagga 60

aattgagaag gatataggac gcactggtgg aataaggtat ggcccaagac caatcgatct 120

agatatactt ctgtacggca attcccagat tgatactgag gctctaattg tgccacatga 180

acgcatccag gagaggccat 200

<210> 552
<211> 200
<212> DNA
<213> Digitaria sanguinalis

<400> 552

atggcctctc ctggatgcgt tcatgtggca caattagagc ctcagtatca atctgggaat 60

tgccgtacag aagtatatct agatcgattg gtcttgggcc ataccttatt ccaccagtgc 120

gtcctatatc cttctcaatt tcctttagtt tcttaagcaa ctcgtgaggt cccagcctag 180

tcgtacctcg aatggcagag 200

<210> 553
<211> 200
<212> DNA
<213> Digitaria sanguinalis

<400> 553

catgaacgca tccaggagag gccatttgtt ctagcacctc ttgttgacct tctaggtgca 60

tctggcaatg atagtgtcga aacaagctgg cactctcttt caaagtgcag tggtggtttc 120

tttgaattat ggaataaact tgggggtgaa tctataattg gaacagaaag tattaaaagg 180

gtattacctg ttgggaatca 200

<210> 554
<211> 200
<212> DNA
<213> Digitaria sanguinalis

<400> 554

tgattcccaa caggtaatac ccttttaata ctttctgttc caattataga ttcaccccca 60

agtttattcc ataattcaaa gaaaccacca ctgcactttg aaagagagtg ccagcttgtt 120

tcgacactat cattgccaga tgcacctaga aggtcaacaa gaggtgctag aacaaatggc 180

ctctcctgga tgcgttcatg 200

<210> 555
<211> 200
<212> DNA
<213> Digitaria sanguinalis

<400> 555

aaagggtatt acctgttggg aatcatttgt ttgattggtg tgagagaacc ctcgtcatgg 60

gggtccttaa tctaacacca gacagcttta gtgatggagg taagtttcaa caagtggaag 120

ctgctatttc tcaggctaaa ttattaattt cagaaggtgc agatatcata gatattggtg 180

ctcaatctac caggcctttt 200

<210> 556
<211> 200
<212> DNA
<213> Digitaria sanguinalis

<400> 556
   aaaaggcctg gtagattgag caccaatatc tatgatatct gcaccttctg aaattaataa 60
   tttagcctga gaaatagcag cttccacttg ttgaaactta cctccatcac taaagctgtc 120
   tggtgttaga ttaaggaccc ccatgacgag ggttctctca caccaatcaa acaaatgatt 180
   cccaacaggt aatacccttt 200

<210> 557
<211> 200
<212> DNA
<213> Digitaria sanguinalis

<400> 557

tggtgctcaa tctaccaggc cttttgcaaa gagattatct ccaaacgaag aaattgaaag 60

attggttcct gttctggatg tgattacgaa aattcctgaa atggagggaa agttgctctc 120

agtggataca ttctatgcag aagttgcatg tgaagctgtg aaaagaggag ttcacatgat 180

caatgatgta tctggtggac 200

<210> 558
<211> 200
<212> DNA
<213> Digitaria sanguinalis

<400>       558

gtccaccaga tacatcattg atcatgtgaa ctcctctttt cacagcttca catgcaactt    60

ctgcatagaa tgtatccact gagagcaact ttccctccat ttcaggaatt ttcgtaatca   120

catccagaac aggaaccaat ctttcaattt cttcgtttgg agataatctc tttgcaaaag   180

gcctggtaga ttgagcacca                                               200


<210>       559
<211>       200
<212>       DNA
<213>       Digitaria sanguinalis

<400>       559

atgatcaatg atgtatctgg tggacagctt gaccccaaaa ttcttaaagt tgctgctgaa    60

ctcaaagttc cgtatgttgc aatgcacatg aggggagatc catcaacaat gcaaagtgaa   120

caaaatttac agtatgacga tgtctgcaag gaagttgctt cagagctata tgcacaggtg   180

agagaagcag agttatctgg                                               200


<210>       560
<211>       200
<212>       DNA
<213>       Digitaria sanguinalis

<400>       560

ccagataact ctgcttctct cacctgtgca tatagctctg aagcaacttc cttgcagaca    60

tcgtcatact gtaaattttg ttcactttgc attgttgatg gatctcccct catgtgcatt   120

gcaacatacg gaactttgag ttcagcagca actttaagaa ttttggggtc aagctgtcca   180

ccagatacat cattgatcat                                               200


<210>       561
<211>       200
<212>       DNA
<213>       Digitaria sanguinalis

<400>       561

aggtgagaga agcagagtta tctgggattc cattgtggag gatagttcta gatccaggca    60

ttgggttctc caagaaatcc aaacataacc ttgaagtaat tatgggattg gaatccatta   120

ggagggagat gagtaaaatg agtattggtg cttcacatgt gccaatatta ctgggaccct   180

ctaggaaaag atttttgggt                                               200


<210>       562
<211>       200
<212>       DNA
<213>       Digitaria sanguinalis

<400>       562

acccaaaaat cttttcctag agggtcccag taatattggc acatgtgaag caccaatact    60

cattttactc atctccctcc taatggattc caatcccata attacttcaa ggttatgttt   120

ggatttcttg gagaacccaa tgcctggatc tagaactatc ctccacaatg gaatcccaga   180

taactctgct tctctcacct                                               200


<210>       563
<211>       200
<212>       DNA
<213>       Digitaria sanguinalis

<400>       563

accctctagg aaaagatttt tgggtgaaat atgcaatcgt gaaaatccag ttgagagaga    60

tgttgctact gttgcagctg tgacggcggg tattttgaat ggtgctaaca tagtaagggt   120

acataatgca ggatacagtt cagatgctgc aaagttttgt gatgcattga ataagagaag   180

aagaatggaa gactgaacca                                               200


<210>       564
<211>       200
<212>       DNA
<213>       Digitaria sanguinalis

<400>       564

tggttcagtc ttccattctt cttctcttat tcaatgcatc acaaaacttt gcagcatctg    60

aactgtatcc tgcattatgt acccttacta tgttagcacc attcaaaata cccgccgtca   120

cagctgcaac agtagcaaca tctctctcaa ctggattttc acgattgcat atttcaccca   180

aaaatctttt cctagagggt                                               200


<210>       565
<211>       200
<212>       DNA
<213>       Digitaria sanguinalis

<400>       565

agaagaagaa tggaagactg aaccagctga tcaaaaagat accgagctct gattttattc    60

gagaaaatgg tgatgcagga tagttactct gctgctcaat gggattctca tattacatca   120

tttgtggagt attgtttttg taataaataa accagggatg acgtttttcc tgtgtcatct   180

cctatctagt ttctagatac                                               200


<210>       566
<211>       200
<212>       DNA

<213>	Digitaria sanguinalis

<400>	566

gtatctagaa actagatagg agatgacaca ggaaaaacgt catccctggt ttatttatta	60

caaaaacaat actccacaaa tgatgtaata tgagaatccc attgagcagc agagtaacta	120

tcctgcatca ccattttctc gaataaaatc agagctcggt atctttttga tcagctggtt	180

cagtcttcca ttcttcttct	200

<210>	567
<211>	200
<212>	DNA
<213>	Digitaria sanguinalis

<400>	567

catctcctat ctagtttcta gatacttcaa tgaagcatgg ttcaactcag atcacttgga	60

aagagtagtc aaggaataag gttacagaat cagttagtaa ttacagattc ttacctcttg	120

tggtcctatt ttagatgatt ttccagaaca ttttctttcc aatttaataa tatcagaagg	180

gcctaatttg tgtgtatctt	200

<210>	568
<211>	200
<212>	DNA
<213>	Digitaria sanguinalis

<400>	568

aagatacaca caaattaggc ccttctgata ttattaaatt ggaaagaaaa tgttctggaa	60

aatcatctaa aataggacca caagaggtaa gaatctgtaa ttactaactg attctgtaac	120

cttattcctt gactactctt tccaagtgat ctgagttgaa ccatgcttca ttgaagtatc	180

tagaaactag ataggagatg	200

<210>	569
<211>	200
<212>	DNA
<213>	Digitaria sanguinalis

<400>	569

gctcgttcgt ttacgcgatg ttcgcttgag ggatgttcaa ctgaccaaga gattgtgatt	60

gctatgggaa gcaacgtggg tgatggagtc agtacatttg acagggcatt acaaatgatg	120

aaaagctcag gcgtgaacat cactaggcat gcctgtctat atgagactgc ccctgcttat	180

gtgaccgatc agccacggtt	200

<210>	570
<211>	200

<212>       DNA
<213>       Digitaria sanguinalis

<400>       570

aaccgtggct gatcggtcac ataagcaggg gcagtctcat atagacaggc atgcctagtg    60

atgttcacgc ctgagctttt catcatttgt aatgccctgt caaatgtact gactccatca   120

cccacgttgc ttcccatagc aatcacaatc tcttggtcag ttgaacatcc ctcaagcgaa   180

catcgcgtaa acgaacgagc                                               200


<210>       571
<211>       200
<212>       DNA
<213>       Digitaria sanguinalis

<400>       571

cttatgtgac cgatcagcca cggttcctga actctgccat tcgaggtacg actaggctgg    60

gacctcacga gttgcttaag aaactaaagg aaattgagaa ggatatagga cgcactggtg   120

gaataaggta tggcccaaga ccaatcgatc tagatatact tctgtacggc aattcccaga   180

ttgatactga ggctctaatt                                               200


<210>       572
<211>       200
<212>       DNA
<213>       Digitaria sanguinalis

<400>       572

aattagagcc tcagtatcaa tctgggaatt gccgtacaga agtatatcta gatcgattgg    60

tcttgggcca taccttattc caccagtgcg tcctatatcc ttctcaattt cctttagttt   120

cttaagcaac tcgtgaggtc ccagcctagt cgtacctcga atggcagagt tcaggaaccg   180

tggctgatcg gtcacataag                                               200


<210>       573
<211>       200
<212>       DNA
<213>       Digitaria sanguinalis

<400>       573

ccagattgat actgaggctc taattgtgcc acatgaacgc atccaggaga ggccatttgt    60

tctagcacct cttgttgacc ttctaggtgc atctggcaat gatagtgtcg aaacaagctg   120

gcactctctt tcaaagtgca gtggtggttt ctttgaatta tggaataaac ttgggggtga   180

atctataatt ggaacagaaa                                               200


<210>       574

<211>       200
<212>       DNA
<213>       Digitaria sanguinalis

<400>       574

tttctgttcc aattatagat tcacccccaa gtttattcca taattcaaag aaaccaccac    60

tgcactttga aagagagtgc cagcttgttt cgacactatc attgccagat gcacctagaa   120

ggtcaacaag aggtgctaga acaaatggcc tctcctggat gcgttcatgt ggcacaatta   180

gagcctcagt atcaatctgg                                               200


<210>       575
<211>       200
<212>       DNA
<213>       Digitaria sanguinalis

<400>       575

ggtgaatcta taattggaac agaaagtatt aaaagggtat tacctgttgg gaatcatttg    60

tttgattggt gtgagagaac cctcgtcatg gggtccttta atctaacacc agacagcttt   120

agtgatggag gtaagtttca acaagtggaa gctgctattt ctcaggctaa attattaatt   180

tcagaaggtg cagatatcat                                               200


<210>       576
<211>       200
<212>       DNA
<213>       Digitaria sanguinalis

<400>       576

atgatatctg caccttctga aattaataat ttagcctgag aaatagcagc ttccacttgt    60

tgaaacttac ctccatcact aaagctgtct ggtgttagat taaggacccc catgacgagg   120

gttctctcac accaatcaaa caaatgattc ccaacaggta ataccctttt aatactttct   180

gttccaatta tagattcacc                                               200


<210>       577
<211>       200
<212>       DNA
<213>       Digitaria sanguinalis

<400>       577

taatttcaga aggtgcagat atcatagata ttggtgctca atctaccagg ccttttgcaa    60

agagattatc tccaaacgaa gaaattgaaa gattggttcc tgttctggat gtgattacga   120

aaattcctga aatggaggga aagttgctct cagtggatac attctatgca gaagttgcat   180

gtgaagctgt gaaaagagga                                               200

<210>	578
<211>	200
<212>	DNA
<213>	Digitaria sanguinalis

<400>	578

tcctcttttc acagcttcac atgcaacttc tgcatagaat gtatccactg agagcaactt	60

tccctccatt tcaggaattt tcgtaatcac atccagaaca ggaaccaatc tttcaatttc	120

ttcgtttgga gataatctct ttgcaaaagg cctggtagat tgagcaccaa tatctatgat	180

atctgcacct tctgaaatta	200

<210>	579
<211>	200
<212>	DNA
<213>	Digitaria sanguinalis

<400>	579

tgcatgtgaa gctgtgaaaa gaggagttca catgatcaat gatgtatctg gtggacagct	60

tgaccccaaa attcttaaag ttgctgctga actcaaagtt ccgtatgttg caatgcacat	120

gaggggagat ccatcaacaa tgcaaagtga acaaaattta cagtatgacg atgtctgcaa	180

ggaagttgct tcagagctat	200

<210>	580
<211>	200
<212>	DNA
<213>	Digitaria sanguinalis

<400>	580

atagctctga agcaacttcc ttgcagacat cgtcatactg taaattttgt tcactttgca	60

ttgttgatgg atctcccctc atgtgcattg caacatacgg aactttgagt tcagcagcaa	120

ctttaagaat tttggggtca agctgtccac cagatacatc attgatcatg tgaactcctc	180

ttttcacagc ttcacatgca	200

<210>	581
<211>	200
<212>	DNA
<213>	Digitaria sanguinalis

<400>	581

tgcaaggaag ttgcttcaga gctatatgca caggtgagag aagcagagtt atctgggatt	60

ccattgtgga ggatagttct agatccaggc attgggttct ccaagaaatc caaacataac	120

cttgaagtaa ttatgggatt ggaatccatt aggagggaga tgagtaaaat gagtattggt	180

gcttcacatg tgccaatatt	200

<210>	582
<211>	200
<212>	DNA
<213>	Digitaria sanguinalis

<400>	582

aatattggca catgtgaagc accaatactc attttactca tctccctcct aatggattcc	60

aatcccataa ttacttcaag gttatgtttg gatttcttgg agaacccaat gcctggatct	120

agaactatcc tccacaatgg aatcccagat aactctgctt ctctcacctg tgcatatagc	180

tctgaagcaa cttccttgca	200

<210>	583
<211>	200
<212>	DNA
<213>	Digitaria sanguinalis

<400>	583

ttggtgcttc acatgtgcca atattactgg gaccctctag gaaaagattt ttgggtgaaa	60

tatgcaatcg tgaaaatcca gttgagagag atgttgctac tgttgcagct gtgacggcgg	120

gtattttgaa tggtgctaac atagtaaggg tacataatgc aggatacagt tcagatgctg	180

caaagttttg tgatgcattg	200

<210>	584
<211>	200
<212>	DNA
<213>	Digitaria sanguinalis

<400>	584

caatgcatca caaaactttg cagcatctga actgtatcct gcattatgta cccttactat	60

gttagcacca ttcaaaatac ccgccgtcac agctgcaaca gtagcaacat ctctctcaac	120

tggattttca cgattgcata tttcacccaa aaatcttttc ctagagggtc ccagtaatat	180

tggcacatgt gaagcaccaa	200

<210>	585
<211>	200
<212>	DNA
<213>	Euphorbia heterophylla

<400>	585

aaatataaat ataaatataa aaataaaaat aaattataat aatataaaat ataaaaatta	60

atttaaacca tgttttaaaa atagtgttat gaaaaactcc gcttgggcgc cgtctagata	120

gtttacttag gctgattttt aaaacaaaac cccccgctta acccgcctaa atcggtcaat	180

tgtttatatc cttatgacta	200

<210>	586
<211>	200
<212>	DNA
<213>	Euphorbia heterophylla

<400>	586

tagtcataag gatataaaca attgaccgat ttaggcgggt taagcggggg gttttgtttt	60

aaaaatcagc ctaagtaaac tatctagacg gcgcccaagc ggagtttttc ataacactat	120

ttttaaaaca tggtttaaat taatttttat attttatatt attataattt atttttattt	180

ttatatttat atttatattt	200


<210>	587
<211>	200
<212>	DNA
<213>	Euphorbia heterophylla

<400>	587

tcaattgttt atatccttat gactacttaa aatcgcagcg gtgttatggt gaattatcat	60

gttttaatga gcttatgaaa tgcaataata aaaaaataac cattaataat ttgatataca	120

tttatcggga aaaatgaaat aataaaaaat aaaaaaatat caaaaaaatc tataaaatat	180

ttgtaccttt ttcaattagt	200


<210>	588
<211>	200
<212>	DNA
<213>	Euphorbia heterophylla

<400>	588

actaattgaa aaaggtacaa atattttata gatttttttg atattttttt attttttatt	60

atttcatttt tcccgataaa tgtatatcaa attattaatg gttatttttt tattattgca	120

tttcataagc tcattaaaac atgataattc accataacac cgctgcgatt ttaagtagtc	180

ataaggatat aaacaattga	200


<210>	589
<211>	200
<212>	DNA
<213>	Euphorbia heterophylla

<400>	589

aatatttgta cctttttcaa ttagtaccca ttttgtatat taaaacaata aaaatgatcc	60

agattaataa aatagaaaag tattaataac ccgaaaatat caaatatcaa aatataatga	120

aacatgaaca cgaaaacaca ataaagaagt tggtatcaca aacacaaaga cacccttaaa	180

gctcctatgt ttgcggagag                                                200

<210>    590
<211>    200
<212>    DNA
<213>    Euphorbia heterophylla

<400>    590

ctctccgcaa acataggagc tttaagggtg tctttgtgtt tgtgatacca acttctttat    60

tgtgttttcg tgttcatgtt tcattatatt ttgatatttg atattttcgg gttattaata   120

ctttttctat ttattaatct ggatcatttt tattgtttta atatacaaaa tgggtactaa   180

ttgaaaaagg tacaaatatt                                                200

<210>    591
<211>    200
<212>    DNA
<213>    Euphorbia heterophylla

<400>    591

ttaaagctcc tatgtttgcg gagagttgta agtcgactaa tttcagattg aaaaagttaa    60

aaatggccaa cttttttagc tatgaggtta gattagatac gagaatgcct ttgtgaaaag   120

ctggtcacta tttcttcatt cgcgtggcaa ttaacccggc ataatcgata gccaattcac   180

ggacaaggtc aaaggtatca                                                200

<210>    592
<211>    200
<212>    DNA
<213>    Euphorbia heterophylla

<400>    592

tgataccttt gaccttgtcc gtgaattggc tatcgattat gccgggttaa ttgccacgcg    60

aatgaagaaa tagtgaccag cttttcacaa aggcattctc gtatctaatc taacctcata   120

gctaaaaaag ttggccattt ttaacttttt caatctgaaa ttagtcgact tacaactctc   180

cgcaaacata ggagctttaa                                                200

<210>    593
<211>    200
<212>    DNA
<213>    Euphorbia heterophylla

<400>    593

ttcacggaca aggtcaaagg tatcagcttt actcaaccct tcgctttgat actaaattgc    60

tgattcttat tctgattatg ctttatttcg ttacttcatt ttattgtcta tgcacataag   120

ctgttcgaca aaaagcctga atgaaactgt gagtgctggt acgcggttac aatactcctg   180

attatgtcca atgattattt                                                200

<210>   594
<211>   200
<212>   DNA
<213>   Euphorbia heterophylla

<400>   594

aaataatcat tggacataat caggagtatt gtaaccgcgt accagcactc acagtttcat    60

tcaggctttt tgtcgaacag cttatgtgca tagacaataa aatgaagtaa cgaaataaag   120

cataatcaga ataagaatca gcaatttagt atcaaagcga agggttgagt aaagctgata   180

cctttgacct tgtccgtgaa                                                200

<210>   595
<211>   200
<212>   DNA
<213>   Euphorbia heterophylla

<400>   595

tcctgattat gtccaatgat tatttgggta ctttaatacc ctaatttgtg aagttcacta    60

gcgaaagggt agtttcttcc ccggttattt atcattttat gaatccccaa gtctttgaag   120

tacagctgat taaaatgcaa gtactcataa gttgtcattg tatgtttgaa atccttacgt   180

tgttggtatt ttacagaatt                                                200

<210>   596
<211>   200
<212>   DNA
<213>   Euphorbia heterophylla

<400>   596

aattctgtaa aataccaaca acgtaaggat ttcaaacata caatgacaac ttatgagtac    60

ttgcatttta atcagctgta cttcaaagac ttggggattc ataaaatgat aaataaccgg   120

ggaagaaact accctttcgc tagtgaactt cacaaattag ggtattaaag tacccaaata   180

atcattggac ataatcagga                                                200

<210>   597
<211>   200
<212>   DNA
<213>   Euphorbia heterophylla

<400>   597

tacgttgttg gtattttaca gaatttgtac tgaagaagca taaattcata cctagttagg    60

cttccttgtt cctaatatgc ttctcctcaa gcggctggtg tccactaaac aagggttcaa   120

taatgccatc aaccgttttg gaggtaatat agttatgcct atgatgcttt aagtcgcttt 180

tcatcgattt gttacattac 200

<210> 598
<211> 200
<212> DNA
<213> Euphorbia heterophylla

<400> 598

gtaatgtaac aaatcgatga aaagcgactt aaagcatcat aggcataact atattacctc 60

caaaacggtt gatggcatta ttgaaccctt gtttagtgga caccagccgc ttgaggagaa 120

gcatattagg aacaaggaag cctaactagg tatgaattta tgcttcttca gtacaaattc 180

tgtaaaatac caacaacgta 200

<210> 599
<211> 200
<212> DNA
<213> Euphorbia heterophylla

<400> 599

gcttttcatc gatttgttac attaccaggc taagattctg caattgaagt tagaaatcga 60

cgagtcttat cttgaaaagt atggattttg ttttgtcacc ctggggccag ttcaagattg 120

ctttcccagg ttgctgtctc ggcaccacat cctaatccaa aaccctaagg tgatgggtta 180

atgggtccct tcgcagttat 200

<210> 600
<211> 200
<212> DNA
<213> Euphorbia heterophylla

<400> 600

ataactgcga agggacccat taacccatca ccttagggtt ttggattagg atgtggtgcc 60

gagacagcaa cctgggaaag caatcttgaa ctggccccag ggtgacaaaa caaaatccat 120

acttttcaag ataagactcg tcgatttcta acttcaattg cagaatctta gcctggtaat 180

gtaacaaatc gatgaaaagc 200

<210> 601
<211> 200
<212> DNA
<213> Euphorbia heterophylla

<400> 601

ggttaatggg tcccttcgca gttatatttc attcacttaa ctcattagct tccatgtgga 60

taatcaactc cacacttgcc ccaaaactgt ataaacgtag attttgacct ttggaaatca 120

ttagctggac aaatcctaac taattagtat caagagccaa aatcttttga ccaagaccaa 180

atatgttgtt tcattgttgc 200

<210> 602
<211> 200
<212> DNA
<213> Euphorbia heterophylla

<400> 602

gcaacaatga aacaacatat ttggtcttgg tcaaaagatt ttggctcttg atactaatta 60

gttaggattt gtccagctaa tgatttccaa aggtcaaaat ctacgtttat acagttttgg 120

ggcaagtgtg gagttgatta tccacatgga agctaatgag ttaagtgaat gaaatataac 180

tgcgaaggga cccattaacc 200

<210> 603
<211> 200
<212> DNA
<213> Euphorbia heterophylla

<400> 603

accaaatatg ttgtttcatt gttgcagcat tgtcatcatc ctttctccat accgaaccaa 60

atagttcggt ggaagttcat tctcaagagc aagaagtagt aattgcatta ggaagcaatg 120

tgggagatag agttcataat ttcaaccaag cattgcgatt aatgaaaagt tccggcatta 180

acgtaactag acatggttgt 200

<210> 604
<211> 200
<212> DNA
<213> Euphorbia heterophylla

<400> 604

acaaccatgt ctagttacgt taatgccgga acttttcatt aatcgcaatg cttggttgaa 60

attatgaact ctatctccca cattgcttcc taatgcaatt actacttctt gctcttgaga 120

atgaacttcc accgaactat ttggttcggt atggagaaag gatgatgaca atgctgcaac 180

aatgaaacaa catatttggt 200

<210> 605
<211> 200
<212> DNA
<213> Euphorbia heterophylla

<400> 605

cattaacgta actagacatg gttgtttgta ccagacagca cctgcttatg tcactgacca 60

acctcacttt ctcaactcag cagttcgagc tttcacgaaa ctcggacccc acgagttatt 120

aggagtttta aagcagatcg aaaaggactt gggccgtacc aaagggatta ggtacggacc 180

aaggccaatc gatttggata 200

<210> 606
<211> 200
<212> DNA
<213> Euphorbia heterophylla

<400> 606

tatccaaatc gattggcctt ggtccgtacc taatcccttt ggtacggccc aagtcctttt 60

cgatctgctt taaaactcct aataactcgt ggggtccgag tttcgtgaaa gctcgaactg 120

ctgagttgag aaagtgaggt tggtcagtga cataagcagg tgctgtctgg tacaaacaac 180

catgtctagt tacgttaatg 200

<210> 607
<211> 200
<212> DNA
<213> Euphorbia heterophylla

<400> 607

ggaccaaggc caatcgattt ggatatactt ttctatggaa agttccggat taattccgat 60

acacttattg ttcctcatga gagaatatgg gagagaccct ttgtgatggc cccattggtg 120

gatttactcg gctcggaaat cgagaatgac acggtcgcca gctggcattc cttgtccggc 180

ggtctttttg aatcatggga 200

<210> 608
<211> 200
<212> DNA
<213> Euphorbia heterophylla

<400> 608

tcccatgatt caaaaagacc gccggacaag gaatgccagc tggcgaccgt gtcattctcg 60

atttccgagc cgagtaaatc caccaatggg gccatcacaa agggtctctc ccatattctc 120

tcatgaggaa caataagtgt atcggaatta atccggaact ttccatagaa aagtatatcc 180

aaatcgattg gccttggtcc 200

<210> 609
<211> 200
<212> DNA
<213> Euphorbia heterophylla

<400> 609

ccggcggtct ttttgaatca tgggaaaagt tggcggcgaa aagcctgatc gggaaggacg 60

gaatgaaaag agttaccccg atcggaaacc atttatggga ttggtcggaa aagacttctg 120

taatgggaat tatcaattta acccccgata gtttcagcga tgggggcaag ttgacaacta 180

ttgattctat agtttctaag 200

<210> 610
<211> 200
<212> DNA
<213> Euphorbia heterophylla

<400> 610

cttagaaact atagaatcaa tagttgtcaa cttgccccca tcgctgaaac tatcgggggt 60

taaattgata attcccatta cagaagtctt ttccgaccaa tcccataaat ggtttccgat 120

cggggtaact cttttcattc cgtccttccc gatcaggctt tcgccgccca acttttccca 180

tgattcaaaa agaccgccgg 200

<210> 611
<211> 200
<212> DNA
<213> Euphorbia heterophylla

<400> 611

aactattgat tctatagttt ctaaggttcg ctcgatgatt tccgaagggg cggatattgt 60

cgattttggt gctcaatcga cacgccctat ggctaaaagg atatccccgc aagaggaaat 120

ggataggcta atccctgtat tggaagcggt tgtgaaaata cctgagatga ccggaaagct 180

catatcggtc gacacatttt 200

<210> 612
<211> 200
<212> DNA
<213> Euphorbia heterophylla

<400> 612

aaaatgtgtc gaccgatatg agctttccgg tcatctcagg tattttcaca accgcttcca 60

atacagggat tagcctatcc atttcctctt gcggggatat ccttttagcc atagggcgtg 120

tcgattgagc accaaaatcg acaatatccg ccccttcgga aatcatcgag cgaaccttag 180

aaactataga atcaatagtt 200

<210> 613
<211> 200
<212> DNA
<213> Euphorbia heterophylla

<400> 613

aagctcatat cggtcgacac attttactcg gaagtcgcct tagaagcggt ccaaagagga 60

gcaaatctcg taaatgacgt atctgggggg caattagatc caaaaatgac ggaaaatgtc 120

gctaatctcg aggtaccata tatcttaatg cacatgaggg gggaccccac cacaatgcag 180

aacactcaga atctaaaata 200

<210> 614
<211> 200
<212> DNA
<213> Euphorbia heterophylla

<400> 614

tattttagat tctgagtgtt ctgcattgtg gtggggtccc ccctcatgtg cattaagata 60

tatggtacct cgagattagc gacattttcc gtcatttttg gatctaattg ccccccagat 120

acgtcattta cgagatttgc tcctctttgg accgcttcta aggcgacttc cgagtaaaat 180

gtgtcgaccg atatgagctt 200

<210> 615
<211> 200
<212> DNA
<213> Euphorbia heterophylla

<400> 615

tgcagaacac tcagaatcta aaatacgata atgtttgtaa agaggttgcc tttgagttgt 60

tttcgagggt taaagaagcc gagatgtccg gaattccagc ctggcgaatg attattgacc 120

ctggaattgg gttttcgaag aacacgaatc agaatctgga aatcctgatg ggaattccaa 180

ggatccgggc cgagattggg 200

<210> 616
<211> 200
<212> DNA
<213> Euphorbia heterophylla

<400> 616

cccaatctcg gcccggatcc ttggaattcc catcaggatt tccagattct gattcgtgtt 60

cttcgaaaac ccaattccag ggtcaataat cattcgccag gctggaattc cggacatctc 120

ggcttcttta accctcgaaa acaactcaaa ggcaacctct ttacaaacat tatcgtattt 180

tagattctga gtgttctgca 200

<210> 617
<211> 200
<212> DNA
<213> Euphorbia heterophylla

<400> 617

tccaaggatc cgggccgaga ttgggaggga aagcgtgggt atttctcgtg ggccgattct 60

tattgggcct tcgagaaaga ggtttttggg cgaaatttgc gaacgcccta aagcaactga 120

aagagacccg gcaactgttg cttctgttac tactgggatt ctgggagggg caaatattgt 180

tagagttcat aatgttaggg 200

<210> 618
<211> 200
<212> DNA
<213> Euphorbia heterophylla

<400> 618

ccctaacatt atgaactcta acaatatttg cccctcccag aatcccagta gtaacagaag 60

caacagttgc cgggtctctt tcagttgctt tagggcgttc gcaaatttcg cccaaaaacc 120

tctttctcga aggcccaata agaatcggcc cacgagaaat acccacgctt tccctcccaa 180

tctcggcccg gatccttgga 200

<210> 619
<211> 200
<212> DNA
<213> Euphorbia heterophylla

<400> 619

attgttagag ttcataatgt tagggataat gtggatgctg tgaagctatg cgactcgatg 60

atgaaactaa agaggtctac ttaaaaagta tcgggtatag ttcgatttct ttctttccga 120

atcttttgtt tgactttcag attgtctttg attggcaaaa cagtttcggc tgcggttact 180

gttgttgttt gttgttgcag 200

<210> 620
<211> 200
<212> DNA
<213> Euphorbia heterophylla

<400> 620

ctgcaacaac aaacaacaac agtaaccgca gccgaaactg ttttgccaat caaagacaat 60

ctgaaagtca aacaaaagat tcggaaagaa agaaatcgaa ctatacccga tactttttaa 120

gtagacctct ttagtttcat catcgagtcg catagcttca cagcatccac attatcccta 180

acattatgaa ctctaacaat 200

<210> 621
<211> 200
<212> DNA
<213> Euphorbia heterophylla

<400>       621

ttactgttgt tgtttgttgt tgcagtttag tgttgttgtt tctaattgtg attttaagtt    60

attgttgtta gctgatttgt acttattgtt tggtgaaaat ataattcacg gttgcggttg   120

gaggtataaa ttactaaaat agacatattt taaataaatt ataagttata ttgaagggta   180

tttaggataa ataaaaatcc                                                200


<210>       622
<211>       200
<212>       DNA
<213>       Euphorbia heterophylla

<400>       622

ggatttttat ttatcctaaa tacccttcaa tataacttat aatttattta aaatatgtct    60

attttagtaa tttatacctc caaccgcaac cgtgaattat attttcacca aacaataagt   120

acaaatcagc taacaacaat aacttaaaat cacaattaga aacaacaaca ctaaactgca   180

acaacaaaca acaacagtaa                                                200


<210>       623
<211>       200
<212>       DNA
<213>       Euphorbia heterophylla

<400>       623

gggtatttag gataaataaa aatccaaacc acaaaaaaaa acccgtaaaa ctattgattt    60

ggtgaggttt ggaaaaggag ataaatccct tataaaaaaa acaacttcat atatgttttg   120

aaacttaacc aaacacttgt atacctcctt ttccttttca aacctcaaaa gaccttttta   180

tagtaagctc ttttgaagtc                                                200


<210>       624
<211>       200
<212>       DNA
<213>       Euphorbia heterophylla

<400>       624

gacttcaaaa gagcttacta taaaaaggtc ttttgaggtt tgaaaaggaa aaggaggtat    60

acaagtgttt ggttaagttt caaaacatat atgaagttgt tttttttata agggatttat   120

ctccttttcc aaacctcacc aaatcaatag ttttacgggt ttttttttgt ggtttggatt   180

tttatttatc ctaaataccc                                                200


<210>       625
<211>       200
<212>       DNA
<213>       Euphorbia heterophylla

<400>       625

ttttatagta agctcttttg aagtctatac gctattgttt ccgaatcatt ttggactttt    60

catcgaaatc aacaatactg ctagctagtc atatttcaag atatttggga tgtaaagatc   120

aaggtagttg ttgttgtatt ctgttctcat cagcaaaatt tgttataact ccaccaagat   180

ttaatttttt ccaatctgaa                                                200

<210>       626
<211>       200
<212>       DNA
<213>       Euphorbia heterophylla

<400>       626

ttcagattgg aaaaaattaa atcttggtgg agttataaca aattttgctg atgagaacag    60

aatacaacaa caactacctt gatctttaca tcccaaatat cttgaaatat gactagctag   120

cagtattgtt gatttcgatg aaaagtccaa aatgattcgg aaacaatagc gtatagactt   180

caaaagagct tactataaaa                                                200

<210>       627
<211>       200
<212>       DNA
<213>       Euphorbia heterophylla

<400>       627

aagatttaat tttttccaat ctgaaaatgt cttatgttat agtaagaagt gagatgagtt    60

tatgtctttg tgaaaagatt ttgcccaatt aatgcaaaaa ttttgtgaca agacaagagg   120

ggtgaaagag acaagcaaca agagaaagca gtgaaagggg caaatgttag gaaactgtta   180

gctagaaaag ggtagagggt                                                200

<210>       628
<211>       200
<212>       DNA
<213>       Euphorbia heterophylla

<400>       628

accctctacc cttttctagc taacagtttc ctaacatttg cccctttcac tgctttctct    60

tgttgcttgt ctctttcacc cctcttgtct tgtcacaaaa tttttgcatt aattgggcaa   120

aatcttttca caaagacata aactcatctc acttcttact ataacataag acattttcag   180

attggaaaaa attaaatctt                                                200

<210>       629
<211>       200
<212>       DNA

<213>       Euphorbia heterophylla

<400>       629

tgttagctag aaaagggtag agggtggcct actttaccaa tttgagcatt aaaaaagcac    60

atggctatgt attttagacc aaagtcccta ttgttggcag atgaactaag caaccatatg   120

gttcattaac taactaacta gttattgcaa atttgactgt tccaattcat caaaggactc   180

tccccataga ccattcaaca                                                200

<210>       630
<211>       200
<212>       DNA
<213>       Euphorbia heterophylla

<400>       630

tgttgaatgg tctatgggga gagtcctttg atgaattgga acagtcaaat ttgcaataac    60

tagttagtta gttaatgaac catatggttg cttagttcat ctgccaacaa tagggacttt   120

ggtctaaaat acatagccat gtgctttttt aatgctcaaa ttggtaaagt aggccaccct   180

ctaccctttt ctagctaaca                                                200

<210>       631
<211>       200
<212>       DNA
<213>       Euphorbia heterophylla

<400>       631

gactctcccc atagaccatt caacacttgg gttctagccc tccctctcac cctcagacaa    60

cccttgtgaa cctcattttc atcattggtc cccttttttg ttaggactac acttgtctct   120

ataaaatctc ttctagccat gtgttatgtg tagacttttt cggtttgagg attgggtcgg   180

gttcgggctg ggtctcgtcg                                                200

<210>       632
<211>       200
<212>       DNA
<213>       Euphorbia heterophylla

<400>       632

cgacgagacc cagcccgaac ccgacccaat cctcaaaccg aaaaagtcta cacataacac    60

atggctagaa gagattttat agagacaagt gtagtcctaa caaaaaaggg gaccaatgat   120

gaaaatgagg ttcacaaggg ttgtctgagg gtgagaggga gggctagaac ccaagtgttg   180

aatggtctat ggggagagtc                                                200

<210>       633
<211>       200

<212>       DNA
<213>       Euphorbia heterophylla

<400>       633

gtcgggttcg ggctgggtct cgtcggactt tttactgcaa acatcaaact agggttctaa    60

acccatactt cagtactctg gttcggattg aacttgcgag atcattctaa attcaattta   120

cggctcatag attatatttg agagttttga gttttgggtg gattagattg aaaacgacta   180

tttttttgttg tataaatata                                               200


<210>       634
<211>       200
<212>       DNA
<213>       Euphorbia heterophylla

<400>       634

tatatttata caacaaaaaa tagtcgtttt caatctaatc cacccaaaac tcaaaactct    60

caaatataat ctatgagccg taaattgaat ttagaatgat ctcgcaagtt caatccgaac   120

cagagtactg aagtatgggt ttagaaccct agtttgatgt ttgcagtaaa aagtccgacg   180

agacccagcc cgaacccgac                                               200


<210>       635
<211>       200
<212>       DNA
<213>       Euphorbia heterophylla

<400>       635

gcagcattgt catcatcctt tctccatacc gaaccaaata gttcggtgga agttcattct    60

caagagcaag aagtagtaat tgcattagga agcaatgtgg gagatagagt tcataatttc   120

aaccaagcat tgcaattaat gaaaagttcc ggcattaaca taactagaca tggttgtttg   180

taccagacag cacctgctta                                               200


<210>       636
<211>       200
<212>       DNA
<213>       Euphorbia heterophylla

<400>       636

taagcaggtg ctgtctggta caaacaacca tgtctagtta tgttaatgcc ggaacttttc    60

attaattgca atgcttggtt gaaattatga actctatctc ccacattgct tcctaatgca   120

attactactt cttgctcttg agaatgaact tccaccgaac tatttggttc ggtatggaga   180

aaggatgatg acaatgctgc                                               200


<210>       637

<211>       200
<212>       DNA
<213>       Euphorbia heterophylla

<400>       637

gtttgtacca gacagcacct gcttatgtca ctgaccaacc tcactttctc aactcagcag    60

ttcgagcttt cacgaaactc ggaccccacg agttattagg agttttaaag cagatcgaaa   120

aggacttggg ccgtaccaaa gggattaggt acggaccaag gccaatcgat ttggatatac   180

ttttctatgg aaagttccgg                                               200

<210>       638
<211>       200
<212>       DNA
<213>       Euphorbia heterophylla

<400>       638

ccggaacttt ccatagaaaa gtatatccaa atcgattggc cttggtccgt acctaatccc    60

tttggtacgg cccaagtcct tttcgatctg ctttaaaact cctaataact cgtggggtcc   120

gagtttcgtg aaagctcgaa ctgctgagtt gagaaagtga ggttggtcag tgacataagc   180

aggtgctgtc tggtacaaac                                               200

<210>       639
<211>       200
<212>       DNA
<213>       Euphorbia heterophylla

<400>       639

tatacttttc tatggaaagt tccggattaa ttccgataca cttattgttc ctcatgagag    60

aatatgggag agaccctttg tgatggcccc attggtggat ttactcggct cggaaatcga   120

gaatgacacg gtcgccagct ggcattcctt gtccggcggt ctttttgaat catgggaaaa   180

gttgggcggc gaaagcctga                                               200

<210>       640
<211>       200
<212>       DNA
<213>       Euphorbia heterophylla

<400>       640

tcaggctttc gccgcccaac ttttcccatg attcaaaaag accgccggac aaggaatgcc    60

agctggcgac cgtgtcattc tcgatttccg agccgagtaa atccaccaat ggggccatca   120

caaagggtct ctcccatatt ctctcatgag gaacaataag tgtatcggaa ttaatccgga   180

actttccata gaaaagtata                                               200

<210>	641
<211>	200
<212>	DNA
<213>	Euphorbia heterophylla

<400>	641

gaaaagttgg gcggcgaaag cctgatcggg aaggacggaa tgaaaagagt taccccgatc	60

ggaaaccatt tatgggattg gtcggaaaag acttctgtaa tgggaattat caatttaacc	120

cccgatagtt tcagcgatgg gggcaagttg acaactattg attctatagt ttctaaggtt	180

cgctcgatga tttccgaagg	200

<210>	642
<211>	200
<212>	DNA
<213>	Euphorbia heterophylla

<400>	642

ccttcggaaa tcatcgagcg aaccttagaa actatagaat caatagttgt caacttgccc	60

ccatcgctga aactatcggg ggttaaattg ataattccca ttacagaagt cttttccgac	120

caatcccata aatggtttcc gatcggggta actcttttca ttccgtcctt cccgatcagg	180

ctttcgccgc ccaacttttc	200

<210>	643
<211>	200
<212>	DNA
<213>	Euphorbia heterophylla

<400>	643

aggttcgctc gatgatttcc gaaggggcgg atattgtcga ttttggtgct caatcgacac	60

gccctatggc taaaaggata tccccgcaag aggaaatgga taggctaatc cctgtattgg	120

aagcggttgt gaaaataccc gagatgaccg aaaagctcat atcggtcgac acattttact	180

cggaagtcgc cttagaagcg	200

<210>	644
<211>	200
<212>	DNA
<213>	Euphorbia heterophylla

<400>	644

cgcttctaag gcgacttccg agtaaaatgt gtcgaccgat atgagctttt cggtcatctc	60

gggtattttc acaaccgctt ccaatacagg gattagccta tccatttcct cttgcgggga	120

tatcctttta gccatagggc gtgtcgattg agcaccaaaa tcgacaatat ccgccccttc	180

ggaaatcatc gagcgaacct	200

<210>	645
<211>	200
<212>	DNA
<213>	Euphorbia heterophylla

<400>	645

ttactcggaa gtcgccttag aagcggtcca aagaggagca aatctcgtaa atgacgtatc	60

tggggggcaa ttagatccaa aaatgacgga aaatgtcgct aatctcgagg taccatatat	120

cttaatgcac atgagggggg accccaccac aatgcagaac actcagaatc taaaatacga	180

taatgtttgt aaagaggttg	200

<210>	646
<211>	200
<212>	DNA
<213>	Euphorbia heterophylla

<400>	646

caacctcttt acaaacatta tcgtatttta gattctgagt gttctgcatt gtggtggggt	60

cccccctcat gtgcattaag atatatggta cctcgagatt agcgacattt tccgtcattt	120

ttggatctaa ttgcccccca gatacgtcat ttacgagatt tgctcctctt tggaccgctt	180

ctaaggcgac ttccgagtaa	200

<210>	647
<211>	200
<212>	DNA
<213>	Euphorbia heterophylla

<400>	647

tacgataatg tttgtaaaga ggttgccttt gagttgtttt cgagggttaa agaagccgag	60

atgtccggaa ttccagcctg gcgaatgttt attgaccctg gaattgggtt ttcgaagaac	120

acgaatcaga atctggaaat cctgatggga attccaagga tccgggccga gattgggagg	180

aaaagcgtgg gtatttctcg	200

<210>	648
<211>	200
<212>	DNA
<213>	Euphorbia heterophylla

<400>	648

cgagaaatac ccacgctttt cctcccaatc tcggcccgga tccttggaat tcccatcagg	60

atttccagat tctgattcgt gttcttcgaa aacccaattc cagggtcaat aaacattcgc	120

caggctggaa ttccggacat ctcggcttct ttaaccctcg aaaacaactc aaaggcaacc	180

tctttacaaa cattatcgta	200

<210>	649
<211>	200
<212>	DNA
<213>	Euphorbia heterophylla

<400>	649

ggaggaaaag cgtgggtatt tctcgtgggc cgattcttat tgggccttcg agaaagaggt	60

ttttgggcga aatttgcgaa cgccctgaag caactgaaag agacccggca actgttgctt	120

ctgttactac tgggattctg ggaggggcaa atattgttag agttcataat gttagggata	180

atgtggatgc tgtgaagcta	200

<210>	650
<211>	200
<212>	DNA
<213>	Euphorbia heterophylla

<400>	650

tagcttcaca gcatccacat tatccctaac attatgaact ctaacaatat ttgcccctcc	60

cagaatccca gtagtaacag aagcaacagt tgccgggtct ctttcagttg cttcagggcg	120

ttcgcaaatt tcgcccaaaa acctctttct cgaaggccca ataagaatcg gcccacgaga	180

aatacccacg cttttcctcc	200

<210>	651
<211>	200
<212>	DNA
<213>	Euphorbia heterophylla

<400>	651

gcagcattgt catcatcctt tctccatacc gaaccaaata gttcggtgga agttcattct	60

caagagcaag aagtagtaat tgcattagga agcaatgtgg gagatagagt tcataatttc	120

aaccaagcat tgcgattaat gaaaagttcc ggcattaacg taactagaca tggttgtttg	180

taccagacag cacctgctta	200

<210>	652
<211>	200
<212>	DNA
<213>	Euphorbia heterophylla

<400>	652

taagcaggtg ctgtctggta caaacaacca tgtctagtta cgttaatgcc ggaacttttc	60

attaatcgca atgcttggtt gaaattatga actctatctc ccacattgct tcctaatgca	120

attactactt cttgctcttg agaatgaact tccaccgaac tatttggttc ggtatggaga	180

aaggatgatg acaatgctgc 200

<210> 653
<211> 200
<212> DNA
<213> Euphorbia heterophylla

<400> 653

gtttgtacca gacagcacct gcttatgtca ctgaccaacc tcactttctc aactcagcag 60

ttcgagcttt cacgaaactc ggaccccacg agttattagg agttttaaag cagatcgaaa 120

aggacttggg ccgtaccaaa gggattaggt acggaccaag gccaatcgat ttggatatac 180

ttttctatgg aaagttccgg 200

<210> 654
<211> 200
<212> DNA
<213> Euphorbia heterophylla

<400> 654

ccggaacttt ccatagaaaa gtatatccaa atcgattggc cttggtccgt acctaatccc 60

tttggtacgg cccaagtcct tttcgatctg ctttaaaact cctaataact cgtggggtcc 120

gagtttcgtg aaagctcgaa ctgctgagtt gagaaagtga ggttggtcag tgacataagc 180

aggtgctgtc tggtacaaac 200

<210> 655
<211> 200
<212> DNA
<213> Euphorbia heterophylla

<400> 655

tatacttttc tatggaaagt tccggattaa ttccgataca cttattgttc ctcatgagag 60

aatatgggag agaccctttg tgatggcccc attggtggat ttactcggct cggaaatcga 120

gaatgacacg gtcgccagct ggcattcctt gtccggcggt ctttttgaat catgggaaaa 180

gttgggcggc gaaagcctga 200

<210> 656
<211> 200
<212> DNA
<213> Euphorbia heterophylla

<400> 656
   tcaggctttc gccgcccaac ttttcccatg attcaaaaag accgccggac aaggaatgcc 60
   agctggcgac cgtgtcattc tcgatttccg agccgagtaa atccaccaat ggggccatca 120
   caaagggtct ctcccatatt ctctcatgag gaacaataag tgtatcggaa ttaatccgga 180
   actttccata gaaaagtata                                              200

<210>   657
<211>   200
<212>   DNA
<213>   Euphorbia heterophylla

<400>   657

gaaaagttgg gcggcgaaag cctgatcggg aaggacggaa tgaaaagagt taccccgatc    60

ggaaaccatt tatgggattg gtcggaaaag acttctgtaa tgggaattat caatttaacc   120

cccgatagtt tcagcgatgg gggcaagttg acaactattg attctatagt ttctaaggtt   180

cgctcgatga tttccgaagg                                              200

<210>   658
<211>   200
<212>   DNA
<213>   Euphorbia heterophylla

<400>   658

ccttcggaaa tcatcgagcg aaccttagaa actatagaat caatagttgt caacttgccc    60

ccatcgctga aactatcggg ggttaaattg ataattccca ttacagaagt cttttccgac   120

caatcccata aatggtttcc gatcggggta actcttttca ttccgtcctt cccgatcagg   180

ctttcgccgc ccaacttttc                                              200

<210>   659
<211>   200
<212>   DNA
<213>   Euphorbia heterophylla

<400>   659

aggttcgctc gatgatttcc gaaggggcgg atattgtcga ttttggtgct caatcgacac    60

gccctatggc taaaaggata tccccgcaag aggaaatgga taggctaatc cctgtattgg   120

aagcggttgt gaaaatacct gagatgaccg gaaagctcat atcggtcgac acattttact   180

cggaagtcgc cttagaagcg                                              200

<210>   660
<211>   200
<212>   DNA
<213>   Euphorbia heterophylla

<400>   660

cgcttctaag gcgacttccg agtaaaatgt gtcgaccgat atgagctttc cggtcatctc    60

aggtattttc acaaccgctt ccaatacagg gattagccta tccatttcct cttgcgggga   120

tatcctttta gccatagggc gtgtcgattg agcaccaaaa tcgacaatat ccgccccttc 180

ggaaatcatc gagcgaacct 200

<210> 661
<211> 200
<212> DNA
<213> Euphorbia heterophylla

<400> 661

ttactcggaa gtcgccttag aagcggtcca aagaggagca aatctcgtaa atgacgtatc 60

tggggggcaa ttagatccaa aaatgacgga aaatgtcgct aatctcgagg taccatatat 120

cttaatgcac atgagggggg accccaccac aatgcagaac actcagaatc taaaatacga 180

taatgtttgt aaagaggttg 200

<210> 662
<211> 200
<212> DNA
<213> Euphorbia heterophylla

<400> 662

caacctcttt acaaacatta tcgtatttta gattctgagt gttctgcatt gtggtggggt 60

cccccctcat gtgcattaag atatatggta cctcgagatt agcgacattt tccgtcattt 120

ttggatctaa ttgcccccca gatacgtcat ttacgagatt tgctcctctt tggaccgctt 180

ctaaggcgac ttccgagtaa 200

<210> 663
<211> 200
<212> DNA
<213> Euphorbia heterophylla

<400> 663

tacgataatg tttgtaaaga ggttgccttt gagttgtttt cgagggttaa agaagccgag 60

atgtccggaa ttccagcctg gcgaatgatt attgaccctg gaattgggtt ttcgaagaac 120

acgaatcaga atctggaaat cctgatggga attccaagga tccgggccga gattgggagg 180

gaaagcgtgg gtatttctcg 200

<210> 664
<211> 200
<212> DNA
<213> Euphorbia heterophylla

<400> 664

cgagaaatac ccacgctttc cctcccaatc tcggcccgga tccttggaat tcccatcagg 60

atttccagat tctgattcgt gttcttcgaa aacccaattc cagggtcaat aatcattcgc 120

caggctggaa ttccggacat ctcggcttct ttaaccctcg aaaacaactc aaaggcaacc 180

tctttacaaa cattatcgta 200

<210> 665
<211> 200
<212> DNA
<213> Euphorbia heterophylla

<400> 665

ggagggaaag cgtgggtatt tctcgtgggc cgattcttat tgggccttcg agaaagaggt 60

ttttgggcga aatttgcgaa cgccctaaag caactgaaag agacccggca actgttgctt 120

ctgttactac tgggattctg ggaggggcaa atattgttag agttcataat gttagggata 180

atgtggatgc tgtgaagcta 200

<210> 666
<211> 200
<212> DNA
<213> Euphorbia heterophylla

<400> 666

tagcttcaca gcatccacat tatccctaac attatgaact ctaacaatat ttgcccctcc 60

cagaatccca gtagtaacag aagcaacagt tgccgggtct ctttcagttg ctttagggcg 120

ttcgcaaatt tcgcccaaaa acctctttct cgaaggccca ataagaatcg gcccacgaga 180

aatacccacg ctttccctcc 200

<210> 667
<211> 200
<212> DNA
<213> Euphorbia heterophylla

<400> 667

gttatgatga aagtataagt tgctaatcaa gaagaaagag attttaatgt aattatttaa 60

ataataaatt cctaccattt taattgtgtg acaattggtt ataatgattc cgttgcatcg 120

acaactaaaa ctagatagaa ggatcaaaat ataaaatttt aacataatta atacatataa 180

tatataaatg tataatagat 200

<210> 668
<211> 200
<212> DNA
<213> Euphorbia heterophylla

<400> 668

atctattata catttatata ttatatgtat taattatgtt aaaattttat attttgatcc 60

ttctatctag ttttagttgt cgatgcaacg gaatcattat aaccaattgt cacacaatta 120

aaatggtagg aatttattat ttaaataatt acattaaaat ctctttcttc ttgattagca 180

acttatactt tcatcataac 200

<210> 669
<211> 200
<212> DNA
<213> Euphorbia heterophylla

<400> 669

tataatatat aaatgtataa tagatacaat tattattcct cgaattaaaa ttggctaatt 60

atataataat ataatccaac ctataaaaac ttttgagctt ttaagcttga gatgagcttt 120

gtaaaaatga ttgtacaagc ccgaccatga aagagagcca agtagggtca aatgcatacc 180

ctggcttgaa tataaattca 200

<210> 670
<211> 200
<212> DNA
<213> Euphorbia heterophylla

<400> 670

tgaatttata ttcaagccag ggtatgcatt tgaccctact tggctctctt tcatggtcgg 60

gcttgtacaa tcatttttac aaagctcatc tcaagcttaa aagctcaaaa gtttttatag 120

gttggattat attattatat aattagccaa ttttaattcg aggaataata attgtatcta 180

ttatacattt atatattata 200

<210> 671
<211> 200
<212> DNA
<213> Euphorbia heterophylla

<400> 671

tctggggagt gatggttgga atagatgctt attcctggca gagatcgaaa tgctccattt 60

tagtttcggg ttttgttaaa atggggattt gttaaattgt ttcaatggat aattcactgt 120

tgtattagac ttgatattgt tagcagacac tgatttttat gtattcgtga actgaaaagg 180

ttgattttct tccatgtttt 200

<210> 672
<211> 200
<212> DNA
<213> Euphorbia heterophylla

<400> 672

aaaacatgga agaaaatcaa ccttttcagt tcacgaatac ataaaaatca gtgtctgcta 60

acaatatcaa gtctaataca acagtgaatt atccattgaa acaatttaac aaatccccat 120

tttaacaaaa cccgaaacta aaatggagca tttcgatctc tgccaggaat aagcatctat 180

tccaaccatc actccccaga 200

<210> 673
<211> 200
<212> DNA
<213> Euphorbia heterophylla

<400> 673

aaaggttgat tttcttccat gttttgtctg atttatgatg gaacttcatt tcttagtggt 60

taatgttggt atttccttct gttgtttgat ttgtatctgt aattggtacc atgttatatg 120

caatctttta aaaaatgttt taaaaagcgc tatacgtggc agctgagact gttgagactt 180

gaaaacgcac gggcatgtgc 200

<210> 674
<211> 200
<212> DNA
<213> Euphorbia heterophylla

<400> 674

gcacatgccc gtgcgttttc aagtctcaac agtctcagct gccacgtata gcgcttttta 60

aaacattttt taaaagattg catataacat ggtaccaatt acagatacaa atcaaacaac 120

agaaggaaat accaacatta accactaaga aatgaagttc catcataaat cagacaaaac 180

atggaagaaa atcaaccttt 200

<210> 675
<211> 200
<212> DNA
<213> Euphorbia heterophylla

<400> 675

ttaaagcaga tcgaaaagga cttgggccgt accaaaggga ttaggtacgg accaaggcca 60

atcgatttgg atatactttt tctatggaaa gttccggatt aattccgata cacttattgt 120

tcctcatgag agaatatggg agagaccctt tgtgatggcc ccattggtgg atttactcgg 180

ctcggaaatc gagaatgaca 200

<210> 676
<211> 200
<212> DNA
<213> Euphorbia heterophylla

<400> 676

tgtcattctc gatttccgag ccgagtaaat ccaccaatgg ggccatcaca aagggtctct 60

cccatattct ctcatgagga acaataagtg tatcggaatt aatccggaac tttccataga 120

aaaagtatat ccaaatcgat tggccttggt ccgtacctaa tccctttggt acggcccaag 180

tccttttcga tctgctttaa 200

<210> 677
<211> 200
<212> DNA
<213> Euphorbia heterophylla

<400> 677

ctcggctcgg aaatcgagaa tgacacggtc gccagctggc attccttgtc cggcggtctt 60

tttgaatcat gggaaaagtt gggcggcgaa agcctgatcg ggaaggacgg aatgaaaaga 120

gttaccccga tcggaaacca tttatgggat tggtcggaaa agacttctgt aatgggaatt 180

atcaatttaa cccccgatag 200

<210> 678
<211> 200
<212> DNA
<213> Euphorbia heterophylla

<400> 678

ctatcggggg ttaaattgat aattcccatt acagaagtct tttccgacca atcccataaa 60

tggtttccga tcggggtaac tcttttcatt ccgtccttcc cgatcaggct ttcgccgccc 120

aacttttccc atgattcaaa aagaccgccg gacaaggaat gccagctggc gaccgtgtca 180

ttctcgattt ccgagccgag 200

<210> 679
<211> 200
<212> DNA
<213> Euphorbia heterophylla

<400> 679

gaattatcaa tttaaccccc gatagtttca gcgatggggg caagttgaca actattgatt 60

ctatagtttc taaggttcgc tcgatgattt ccgaaggggc ggatattgtc gattttggtg 120

ctcaatcgac acgccctatg gctaaaagga tatccccgca agaggaaatg gataggctaa 180

tccctgtatt ggaagcggtt 200

<210> 680
<211> 200
<212> DNA
<213> Euphorbia heterophylla

<400> 680

aaccgcttcc aatacaggga ttagcctatc catttcctct tgcggggata tccttttagc   60

catagggcgt gtcgattgag caccaaaatc gacaatatcc gccccttcgg aaatcatcga  120

gcgaacctta gaaactatag aatcaatagt tgtcaacttg cccccatcgc tgaaactatc  180

gggggttaaa ttgataattc                                              200

<210>      681
<211>      200
<212>      DNA
<213>      Euphorbia heterophylla

<400>      681

tttgtatatt aaaacaataa aaatgatcca gattaataaa tagaaaaagt attaataacc   60

cgaaaatatc aaatatcaaa atataatgaa acatgaacac gaaatcacaa taaagaagtt  120

ggtatcacaa acacaaagac acccttaaag ctcctatgtt tgtggagagt tgtaagtcga  180

ctaatttcag attgcaaaag                                              200

<210>      682
<211>      200
<212>      DNA
<213>      Euphorbia heterophylla

<400>      682

cttttgcaat ctgaaattag tcgacttaca actctccaca aacataggag ctttaagggt   60

gtctttgtgt ttgtgatacc aacttcttta ttgtgatttc gtgttcatgt ttcattatat  120

tttgatattt gatattttcg ggttattaat actttttcta tttattaatc tggatcattt  180

ttattgtttt aatatacaaa                                              200

<210>      683
<211>      200
<212>      DNA
<213>      Euphorbia heterophylla

<400>      683

gtcgactaat ttcagattgc aaaagttaaa aatggccaac ttttttagct atgaggttag   60

attagatacg agaatgcctt ggtgaaaagc tggtcactgt ttcttcattc gcgtggcaat  120

taacccggca taatcgatac ccaattcacg gacaaggtca aaggtatcag ctttactcaa  180

cccttcgctt tgatactaaa                                              200

<210>      684
<211>      200
<212>      DNA
<213>      Euphorbia heterophylla

<400>       684

tttagtatca aagcgaaggg ttgagtaaag ctgatacctt tgaccttgtc cgtgaattgg    60

gtatcgatta tgccgggtta attgccacgc gaatgaagaa acagtgacca gcttttcacc   120

aaggcattct cgtatctaat ctaacctcat agctaaaaaa gttggccatt tttaactttt   180

gcaatctgaa attagtcgac                                               200


<210>       685
<211>       200
<212>       DNA
<213>       Euphorbia heterophylla

<400>       685

ctcaaccctt cgctttgata ctaaattgct gattcttatt ctgattatgc tttatttcgt    60

tacttcattt tattgtctat gcacataagc tgttcgacaa aaagcctgaa tgaaactgtg   120

agtgctggta cgcggttaca atactcctga ttatgtccaa tgattatttg ggtactttaa   180

taccctaatt tgtgaagttc                                               200


<210>       686
<211>       200
<212>       DNA
<213>       Euphorbia heterophylla

<400>       686

gaacttcaca aattagggta ttaaagtacc caaataatca ttggacataa tcaggagtat    60

tgtaaccgcg taccagcact cacagtttca ttcaggcttt ttgtcgaaca gcttatgtgc   120

atagacaata aaatgaagta acgaaataaa gcataatcag aataagaatc agcaatttag   180

tatcaaagcg aagggttgag                                               200


<210>       687
<211>       200
<212>       DNA
<213>       Euphorbia heterophylla

<400>       687

tttaataccc taatttgtga agttcactag cgaaagggta gtttcttccc cggttattta    60

tcattttatg aatccccaag tctttgaagt acagctgatt aaaatgcaag tactcataag   120

ttgtcattgt atgtttgaag tccttacatt tttggtattt tacagaattt gtactgaaga   180

agcataaatt catacctagt                                               200


<210>       688
<211>       200
<212>       DNA
<213>       Euphorbia heterophylla

<400>       688

actaggtatg aatttatgct tcttcagtac aaattctgta aaataccaaa aatgtaagga    60

cttcaaacat acaatgacaa cttatgagta cttgcatttt aatcagctgt acttcaaaga   120

cttggggatt cataaaatga taaataaccg gggaagaaac taccctttcg ctagtgaact   180

tcacaaatta gggtattaaa                                               200


<210>       689
<211>       200
<212>       DNA
<213>       Euphorbia heterophylla

<400>       689

gaagaagcat aaattcatac ctagttaggc ttccttgttc ctaatatgct tctcctcaag    60

cggctggtgt ccactaaaca agggttcaat aatgccatca accgttttgg aggtaatata   120

gttatgccta tgatgcttta agtcgctttt catcgatttg ttacattacc aggctaagat   180

tctgcaattg aagttagaaa                                               200


<210>       690
<211>       200
<212>       DNA
<213>       Euphorbia heterophylla

<400>       690

tttctaactt caattgcaga atcttagcct ggtaatgtaa caaatcgatg aaaagcgact    60

taaagcatca taggcataac tatattacct ccaaaacggt tgatggcatt attgaaccct   120

tgtttagtgg acaccagccg cttgaggaga agcatattag gaacaaggaa gcctaactag   180

gtatgaattt atgcttcttc                                               200


<210>       691
<211>       200
<212>       DNA
<213>       Euphorbia heterophylla

<400>       691

aagattctgc aattgaagtt agaaatcgac gagtcttgtc ttgaaaagta tggattttgt    60

tttgtcaccc tggggccggt tcaagattgc tttcccaggt tgctgtcttg gcatcacgtc   120

ctaatccaaa accctaaggt gatgggttaa tgggtccctt cgcagttata tatcattcac   180

ttaactcatt aacttccatg                                               200


<210>       692
<211>       200
<212>       DNA

<213>       Euphorbia heterophylla

<400>       692

catggaagtt aatgagttaa gtgaatgata tataactgcg aagggaccca ttaacccatc    60

accttagggt tttggattag gacgtgatgc caagacagca acctgggaaa gcaatcttga   120

accggcccca gggtgacaaa acaaaatcca tacttttcaa gacaagactc gtcgatttct   180

aacttcaatt gcagaatctt                                               200


<210>       693
<211>       200
<212>       DNA
<213>       Euphorbia heterophylla

<400>       693

ttcacttaac tcattaactt ccatgtggat aatcaactcc acacttgccc caacactgta    60

taaacgtaga ttttgacctt tggaaatcat tagctggaca aatcctaact aattagtatc   120

aagagccaaa atcttttgac caagaccaaa atatgttgtt tcattgttgc agcattgtca   180

tcatcctttc tccataccga                                               200


<210>       694
<211>       200
<212>       DNA
<213>       Euphorbia heterophylla

<400>       694

tcggtatgga gaaaggatga tgacaatgct gcaacaatga aacaacatat tttggtcttg    60

gtcaaaagat tttggctctt gatactaatt agttaggatt tgtccagcta atgatttcca   120

aaggtcaaaa tctacgttta tacagtgttg gggcaagtgt ggagttgatt atccacatgg   180

aagttaatga gttaagtgaa                                               200


<210>       695
<211>       200
<212>       DNA
<213>       Euphorbia heterophylla

<400>       695

tgtcatcatc ctttctccat accgaaccaa atagttcggt ggaagttcat tctcaagagc    60

aagaagtagt aattgcatta ggaagcaatg tgggagatag agttcataat ttcaaccaag   120

cattgcaatt aatgaaaagt tccggcatta acataactag acatggttgt ttgtaccaga   180

cagcacctgc ttatgtcact                                               200


<210>       696
<211>       200

<212>	DNA
<213>	Euphorbia heterophylla

<400>	696

agtgacataa gcaggtgctg tctggtacaa acaaccatgt ctagttatgt taatgccgga	60

acttttcatt aattgcaatg cttggttgaa attatgaact ctatctccca cattgcttcc	120

taatgcaatt actacttctt gctcttgaga atgaacttcc accgaactat ttggttcggt	180

atggagaaag gatgatgaca	200

<210>	697
<211>	200
<212>	DNA
<213>	Euphorbia heterophylla

<400>	697

ccagacagca cctgcttatg tcactgacca acctcacttt ctcaactcag cagttcgagc	60

tttcacgaaa ctcggacccc acgagttatt aggagtttta aagcagatcg aaaaggactt	120

gggccgtacc aaagggatta ggtacggacc aaggccaatc gatttggata tacttttcta	180

tggaaagttc cggattaatt	200

<210>	698
<211>	200
<212>	DNA
<213>	Euphorbia heterophylla

<400>	698

aattaatccg gaactttcca tagaaaagta tatccaaatc gattggcctt ggtccgtacc	60

taatcccttt ggtacggccc aagtcctttt cgatctgctt taaaactcct aataactcgt	120

ggggtccgag tttcgtgaaa gctcgaactg ctgagttgag aaagtgaggt tggtcagtga	180

cataagcagg tgctgtctgg	200

<210>	699
<211>	200
<212>	DNA
<213>	Euphorbia heterophylla

<400>	699

ttctatggaa agttccggat taattccgat acacttattg ttcctcatga gagaatatgg	60

gagagaccct ttgtgatggc cccattggtg gatttactcg gctcggaaat cgagaatgac	120

acggtcgcca gctggcattc cttgtccggc ggtctttttg aatcatggga aaagttgggc	180

ggcgaaagcc tgatcgggaa	200

<210>	700

<211>	200
<212>	DNA
<213>	Euphorbia heterophylla

<400>	700

ttcccgatca ggctttcgcc gcccaacttt tcccatgatt caaaaagacc gccggacaag	60

gaatgccagc tggcgaccgt gtcattctcg atttccgagc cgagtaaatc caccaatggg	120

gccatcacaa agggtctctc ccatattctc tcatgaggaa caataagtgt atcggaatta	180

atccggaact ttccatagaa	200

<210>	701
<211>	200
<212>	DNA
<213>	Euphorbia heterophylla

<400>	701

tgggcggcga aagcctgatc gggaaggacg gaatgaaaag agttaccccg atcggaaacc	60

atttatggga ttggtcggaa aagacttctg taatgggaat tatcaattta acccccgata	120

gtttcagcga tgggggcaag ttgacaacta ttgattctat agtttctaag gttcgctcga	180

tgatttccga aggggcggat	200

<210>	702
<211>	200
<212>	DNA
<213>	Euphorbia heterophylla

<400>	702

atccgcccct tcggaaatca tcgagcgaac cttagaaact atagaatcaa tagttgtcaa	60

cttgccccca tcgctgaaac tatcgggggt taaattgata attcccatta cagaagtctt	120

ttccgaccaa tcccataaat ggtttccgat cggggtaact cttttcattc cgtccttccc	180

gatcaggctt tcgccgccca	200

<210>	703
<211>	200
<212>	DNA
<213>	Euphorbia heterophylla

<400>	703

ctcgatgatt tccgaagggg cggatattgt cgattttggt gctcaatcga cacgccctat	60

ggctaaaagg atatccccgc aagaggaaat ggataggcta atccctgtat tggaagcggt	120

tgtgaaaata cccgagatga ccgaaaagct catatcggtc gacacatttt actcggaagt	180

cgccttagaa gcggtccaaa	200

<210>	704
<211>	200
<212>	DNA
<213>	Euphorbia heterophylla

<400>	704

tttggaccgc ttctaaggcg acttccgagt aaaatgtgtc gaccgatatg agcttttcgg	60

tcatctcggg tattttcaca accgcttcca atacagggat tagcctatcc atttcctctt	120

gcggggatat ccttttagcc atagggcgtg tcgattgagc accaaaatcg acaatatccg	180

ccccttcgga aatcatcgag	200

<210>	705
<211>	200
<212>	DNA
<213>	Euphorbia heterophylla

<400>	705

gaagtcgcct tagaagcggt ccaaagagga gcaaatctcg taaatgacgt atctgggggg	60

caattagatc caaaaatgac ggaaaatgtc gctaatctcg aggtaccata tatcttaatg	120

cacatgaggg gggaccccac cacaatgcag aacactcaga atctaaaata cgataatgtt	180

tgtaaagagg ttgcctttga	200

<210>	706
<211>	200
<212>	DNA
<213>	Euphorbia heterophylla

<400>	706

tcaaaggcaa cctctttaca aacattatcg tattttagat tctgagtgtt ctgcattgtg	60

gtggggtccc ccctcatgtg cattaagata tatggtacct cgagattagc gacattttcc	120

gtcatttttg gatctaattg ccccccagat acgtcattta cgagatttgc tcctctttgg	180

accgcttcta aggcgacttc	200

<210>	707
<211>	200
<212>	DNA
<213>	Euphorbia heterophylla

<400>	707

atgtttgtaa agaggttgcc tttgagttgt tttcgagggt taaagaagcc gagatgtccg	60

gaattccagc ctggcgaatg tttattgacc ctggaattgg gttttcgaag aacacgaatc	120

agaatctgga aatcctgatg ggaattccaa ggatccgggc cgagattggg aggaaaagcg	180

tgggtatttc tcgtgggccg	200

<210>	708
<211>	200
<212>	DNA
<213>	Euphorbia heterophylla

<400>	708

cggcccacga gaaataccca cgcttttcct cccaatctcg gcccggatcc ttggaattcc	60

catcaggatt tccagattct gattcgtgtt cttcgaaaac ccaattccag ggtcaataaa	120

cattcgccag gctggaattc cggacatctc ggcttcttta accctcgaaa acaactcaaa	180

ggcaacctct ttacaaacat	200

<210>	709
<211>	200
<212>	DNA
<213>	Euphorbia heterophylla

<400>	709

aagcgtgggt atttctcgtg ggccgattct tattgggcct tcgagaaaga ggtttttggg	60

cgaaatttgc gaacgccctg aagcaactga aagagacccg gcaactgttg cttctgttac	120

tactgggatt ctgggagggg caaatattgt tagagttcat aatgttaggg ataatgtgga	180

tgctgtgaag ctatgtgact	200

<210>	710
<211>	200
<212>	DNA
<213>	Euphorbia heterophylla

<400>	710

agtcacatag cttcacagca tccacattat ccctaacatt atgaactcta acaatatttg	60

cccctcccag aatcccagta gtaacagaag caacagttgc cgggtctctt tcagttgctt	120

cagggcgttc gcaaatttcg cccaaaaacc tctttctcga aggcccaata agaatcggcc	180

cacgagaaat acccacgctt	200

<210>	711
<211>	200
<212>	DNA
<213>	Euphorbia heterophylla

<400>	711

gtggatgctg tgaagctatg tgactcgatg atgaaactaa agaggtctac ttaaaaatta	60

tcgggtatag ttcgatttct ttctttccga atcttttgtt tgactttcag attgtctttg	120

attggcaaaa cagtttcggc tgcggttact gtttgctgtt gcagtttacc gttgttgttt	180

ctaattgtga ttttaagtta	200

<210>	712
<211>	200
<212>	DNA
<213>	Euphorbia heterophylla

<400>	712

taacttaaaa tcacaattag aaacaacaac ggtaaactgc aacagcaaac agtaaccgca	60

gccgaaactg ttttgccaat caaagacaat ctgaaagtca aacaaaagat tcggaaagaa	120

agaaatcgaa ctatacccga taatttttaa gtagacctct ttagtttcat catcgagtca	180

catagcttca cagcatccac	200

<210>	713
<211>	200
<212>	DNA
<213>	Euphorbia heterophylla

<400>	713

tgtttctaat tgtgatttta agttattgtt gttagttgat ttgtacttag tgcttggtga	60

aaatataatt cacggttgcg gttggaggta taaattacta aaatagacat attttaaata	120

aattagaagt tatattgaag ggtatttagg ataaataaaa gtccaaacca caaaaaaaaa	180

aaaccgtaaa actattgatt	200

<210>	714
<211>	200
<212>	DNA
<213>	Euphorbia heterophylla

<400>	714

aatcaatagt tttacggttt tttttttttg tggtttggac ttttatttat cctaaatacc	60

cttcaatata acttctaatt tatttaaaat atgtctattt tagtaattta tacctccaac	120

cgcaaccgtg aattatattt tcaccaagca ctaagtacaa atcaactaac aacaataact	180

taaaatcaca attagaaaca	200

<210>	715
<211>	200
<212>	DNA
<213>	Euphorbia heterophylla

<400>	715

aaaaaaaacc gtaaaactat tgatttggtg aggtttggaa aaggagataa atcccttatg	60

aaaagcaact ccatatatgt tttgaaactt aaccaaacac ttgtatacct ccttttcctt	120

ttcaaacctc aaaagacctt tttatagtaa gctcttttga agtatatacg ctattgtttc	180

cgaatcattt tggacttttc                                              200

<210>    716
<211>    200
<212>    DNA
<213>    Euphorbia heterophylla

<400>    716

gaaaagtcca aaatgattcg gaaacaatag cgtatatact tcaaaagagc ttactataaa    60

aaggtctttt gaggtttgaa aaggaaaagg aggtatacaa gtgtttggtt aagtttcaaa   120

acatatatgg agttgctttt cataagggat ttatctcctt ttccaaacct caccaaatca   180

atagttttac ggtttttttt                                              200

<210>    717
<211>    200
<212>    DNA
<213>    Euphorbia heterophylla

<400>    717

gtttccgaat cattttggac ttttcgtcga aatcaacaat actgttagct agtcatattt    60

caagatattt gggatgtaaa gatcaaggta gttgttgttg tattctgttc tcatcagcaa   120

aatttgttat aactccacca agatttaatt ttttccaatc tgaaaatgtc ttatgttata   180

gtaagaagtg agatgagttt                                              200

<210>    718
<211>    200
<212>    DNA
<213>    Euphorbia heterophylla

<400>    718

aaactcatct cacttcttac tataacataa gacattttca gattggaaaa aattaaatct    60

tggtggagtt ataacaaatt ttgctgatga gaacagaata caacaacaac taccttgatc   120

tttacatccc aaatatcttg aaatatgact agctaacagt attgttgatt tcgacgaaaa   180

gtccaaaatg attcggaaac                                              200

<210>    719
<211>    200
<212>    DNA
<213>    Euphorbia heterophylla

<400>    719

ttatagtaag aagtgagatg agtttatgtc tttgtgaaaa gattttgccc aattaatgca    60

aaaattttgt gacaagacaa gaggggtgaa agagacaagc aacaagagaa agcagtgaaa   120

ggggcaaatg ttaggaaact gttagctaga aaagggtaga gggtggccta ctttaccaat   180

ttgagcatta aaaaagcaca 200

<210> 720
<211> 200
<212> DNA
<213> Euphorbia heterophylla

<400> 720

tgtgcttttt taatgctcaa attggtaaag taggccaccc tctacccttt tctagctaac 60

agtttcctaa catttgcccc tttcactgct ttctcttgtt gcttgtctct ttcacccctc 120

ttgtcttgtc acaaaatttt tgcattaatt gggcaaaatc ttttcacaaa gacataaact 180

catctcactt cttactataa 200

<210> 721
<211> 200
<212> DNA
<213> Euphorbia heterophylla

<400> 721

ccaatttgag cattaaaaaa gcacatggct atgtatttta gaccaaagtc cctattgttg 60

gcagatgaac taagcaacca tatggttcat taactaacta actagttatt gcaaatttga 120

ctgttccaat tcatcaaagg actctcccca tagaccattc aacacttggg ttctagccct 180

ccctctcacc ctcagacaac 200

<210> 722
<211> 200
<212> DNA
<213> Euphorbia heterophylla

<400> 722

gttgtctgag ggtgagaggg agggctagaa cccaagtgtt gaatggtcta tggggagagt 60

cctttgatga attggaacag tcaaatttgc aataactagt tagttagtta atgaaccata 120

tggttgctta gttcatctgc caacaatagg gactttggtc taaaatacat agccatgtgc 180

ttttttaatg ctcaaattgg 200

<210> 723
<211> 200
<212> DNA
<213> Euphorbia heterophylla

<400> 723

gccctccctc tcaccctcag acaacccttg tgaacctcat tttcatcatt ggtccccttt 60

tttgttagga ctacacttgt ctctataaaa tctcttctag ccatgtgtta tgtgtagact 120

ttttcggttt gaggattggg tcgggttcgg gctgggtctc gtcgggcttt ttactgcaaa 180

catcaaacta gggttctaaa 200

<210> 724
<211> 200
<212> DNA
<213> Euphorbia heterophylla

<400> 724

tttagaaccc tagtttgatg tttgcagtaa aaagcccgac gagacccagc ccgaacccga 60

cccaatcctc aaaccgaaaa agtctacaca taacacatgg ctagaagaga ttttatagag 120

acaagtgtag tcctaacaaa aaaggggacc aatgatgaaa atgaggttca caagggttgt 180

ctgagggtga gagggagggc 200

<210> 725
<211> 200
<212> DNA
<213> Euphorbia heterophylla

<400> 725

gcaaacatca aactagggtt ctaaacccat acttcagtac tctggttcgg attgaacttg 60

cgagatcatt ctaaattcaa tttacggctc atagattata tttgagagtt ttgagttttg 120

ggtggattag attgaaaacg actatttttt gttgtataaa tatatctaaa aagttatata 180

attacattct ttattggaat 200

<210> 726
<211> 200
<212> DNA
<213> Euphorbia heterophylla

<400> 726

attccaataa agaatgtaat tatataactt tttagatata tttatacaac aaaaaatagt 60

cgttttcaat ctaatccacc caaaactcaa aactctcaaa tataatctat gagccgtaaa 120

ttgaatttag aatgatctcg caagttcaat ccgaaccaga gtactgaagt atgggtttag 180

aaccctagtt tgatgtttgc 200

<210> 727
<211> 200
<212> DNA
<213> Euphorbia heterophylla

<400> 727

atataattac attctttatt ggaattcatc aatatatata agaatttgac tttgttgtag 60

ccattttgaa agttcgataa gctactccct tcgtctagtt tcaatagttc atttaaaagt 120

ttcattaatt ctgttttcaa ttgtcaattt aaattttcat aatatttttc accatttttt 180

tttccaattt gcccttgtat 200

<210> 728
<211> 200
<212> DNA
<213> Euphorbia heterophylla

<400> 728

atacaagggc aaattggaaa aaaaaatggt gaaaaatatt atgaaaattt aaattgacaa 60

ttgaaaacag aattaatgaa acttttaaat gaactattga aactagacga agggagtagc 120

ttatcgaact ttcaaaatgg ctacaacaaa gtcaaattct tatatatatt gatgaattcc 180

aataaagaat gtaattatat 200

<210> 729
<211> 200
<212> DNA
<213> Euphorbia heterophylla

<400> 729

ttttttttcc aatttgccct tgtatttatt gagaaagaga ttatgttatg atgaaagtat 60

aagttgctaa tcaagaagaa agagatttta atgtaattat ttaaataata aattcctacc 120

attttaattg tgtgacaatt ggttataatg attccgttgc atcgacaact aaaactagat 180

agaaggatca aaatataaaa 200

<210> 730
<211> 200
<212> DNA
<213> Euphorbia heterophylla

<400> 730

ttttatattt tgatccttct atctagtttt agttgtcgat gcaacggaat cattataacc 60

aattgtcaca caattaaaat ggtaggaatt tattatttaa ataattacat taaaatctct 120

ttcttcttga ttagcaactt atactttcat cataacataa tctctttctc aataaataca 180

agggcaaatt ggaaaaaaaa 200

<210> 731
<211> 200
<212> DNA
<213> Euphorbia heterophylla

<400> 731

tagatagaag gatcaaaata taaaatttta acataattaa tacatataat atataaatgt 60

ataatagata caattattat tcctcgaatt aaaattggct aattatataa taatataatc 120

caacctataa aaacttttga gcttttaagc ttgagatgag ctttgtaaaa atgattgtac 180

aagcccgacc atgaaagaga 200

<210> 732
<211> 200
<212> DNA
<213> Euphorbia heterophylla

<400> 732

tctctttcat ggtcgggctt gtacaatcat ttttacaaag ctcatctcaa gcttaaaagc 60

tcaaaagttt ttataggttg gattatatta ttatataatt agccaatttt aattcgagga 120

ataataattg tatctattat acatttatat attatatgta ttaattatgt taaaatttta 180

tattttgatc cttctatcta 200

<210> 733
<211> 200
<212> DNA
<213> Kochia scoparia

<400> 733

gaatgtgctt aggcggctgc aatcagccag tttgatttgg aagggaattt ccaagtatga 60

tagaggtaaa gcacagttgt ttcatttttt gactatatat ttaatttggg aatctgtact 120

gtttcatttt gtaactttat tttgatcctg agttattgaa tctgtcatgt ctcattttga 180

gacggtattt tgaatctgag 200

<210> 734
<211> 200
<212> DNA
<213> Kochia scoparia

<400> 734

ctcagattca aaataccgtc tcaaaatgag acatgacaga ttcaataact caggatcaaa 60

ataaagttac aaaatgaaac agtacagatt cccaaattaa atatatagtc aaaaaatgaa 120

acaactgtgc tttacctcta tcatacttgg aaattccctt ccaaatcaaa ctggctgatt 180

gcagccgcct aagcacattc 200

<210> 735
<211> 200
<212> DNA
<213> Kochia scoparia

<400> 735

tttgagacgg tattttgaat ctgagtctgg atatgaaatt atgaataggc cataggtatt 60

tataaatata gggtttaagt tgatgatctg gatgctatca gagctctatt atgcaggatt 120

acaaatcata atttcattgt gaaatttact tctttatgtg tattcacttg aagattcata 180

attttgatgt attcataaat 200

<210> 736
<211> 200
<212> DNA
<213> Kochia scoparia

<400> 736

atttatgaat acatcaaaat tatgaatctt caagtgaata cacataaaga agtaaatttc 60

acaatgaaat tatgatttgt aatcctgcat aatagagctc tgatagcatc cagatcatca 120

acttaaaccc tatatttata aatacctatg gcctattcat aatttcatat ccagactcag 180

attcaaaata ccgtctcaaa 200

<210> 737
<211> 200
<212> DNA
<213> Kochia scoparia

<400> 737

tcataatttt gatgtattca taaatttgat gtgccctaaa cagaaagagt cagcatgtgc 60

tgaaagtata agggggaaaa ccaagatgta taaatctttt ccttgatgag ttgcttatat 120

gattgatgtc atgattctaa aatgccttta cctaattcaa atggatttta aagattacgt 180

atgtactttt gcagcttcaa 200

<210> 738
<211> 200
<212> DNA
<213> Kochia scoparia

<400> 738

ttgaagctgc aaaagtacat acgtaatctt taaaatccat ttgaattagg taaaggcatt 60

ttagaatcat gacatcaatc atataagcaa ctcatcaagg aaaagattta tacatcttgg 120

ttttccccct tatactttca gcacatgctg actctttctg tttagggcac atcaaattta 180

tgaatacatc aaaattatga 200

<210> 739
<211> 200
<212> DNA
<213> Kochia scoparia

<400> 739

tacgtatgta cttttgcagc ttcaagtctt gcttttttgc actcatcacc agaaactact 60

attgaggttt gttctaaaga gcatgaagtt gtaattgctc tagggagcaa tgtaggagac 120

agattagata actttaacca agctctgcag ctaatgaaga aattaggagt aaatatcaca 180

aggcatggct gcttatacga 200

<210> 740
<211> 200
<212> DNA
<213> Kochia scoparia

<400> 740

tcgtataagc agccatgcct tgtgatattt actcctaatt tcttcattag ctgcagagct 60

tggttaaagt tatctaatct gtctcctaca ttgctcccta gagcaattac aacttcatgc 120

tctttagaac aaacctcaat agtagtttct ggtgatgagt gcaaaaaagc aagacttgaa 180

gctgcaaaag tacatacgta 200

<210> 741
<211> 200
<212> DNA
<213> Kochia scoparia

<400> 741

tcacaaggca tggctgctta tacgagacag atcctgccta tgtgactgat caaccaaagt 60

ttcttaactc tgcagtgagg ggcttcacaa aacttgggcc tctagaacta ttagggatgc 120

tgaagaaaat tgagaaggat atgggtcgaa ctaatggaat aagatatggc cctaggccaa 180

ttgacttaga cattcttttt 200

<210> 742
<211> 200
<212> DNA
<213> Kochia scoparia

<400> 742

aaaaagaatg tctaagtcaa ttggcctagg gccatatctt attccattag ttcgacccat 60

atccttctca attttcttca gcatccctaa tagttctaga ggcccaagtt ttgtgaagcc 120

cctcactgca gagttaagaa actttggttg atcagtcaca taggcaggat ctgtctcgta 180

taagcagcca tgccttgtga 200

<210> 743
<211> 200
<212> DNA
<213> Kochia scoparia

<400> 743

gccaattgac ttagacattc ttttttatgg gaagtttagg gtaagctctg agaaacttac 60

agttccgcat gaaaggatat gggaaagacc ctttgtgatg gcaccactaa ttgatataat 120

tggatcggat gtagaaaatg acactgttgc tgcatggcat tcattatcaa aattttctgg 180

tggactattt gaagcgtggg 200

<210> 744
<211> 200
<212> DNA
<213> Kochia scoparia

<400> 744

cccacgcttc aaatagtcca ccagaaaatt ttgataatga atgccatgca gcaacagtgt 60

cattttctac atccgatcca attatatcaa ttagtggtgc catcacaaag ggtctttccc 120

atatcctttc atgcggaact gtaagtttct cagagcttac cctaaacttc ccataaaaaa 180

gaatgtctaa gtcaattggc 200

<210> 745
<211> 200
<212> DNA
<213> Kochia scoparia

<400> 745

tctggtggac tatttgaagc gtgggataaa cttggtggag attcactcat tgggaaggac 60

ggaatgagta gggttttgcc agttgggaac cacttgtggg attggtcgtg caaaacctct 120

gtaatgggag tcttgaattt gactccggac agctttagtg atggaggaaa gtttctacct 180

gtagaaaatg cagtttctca 200

<210> 746
<211> 200
<212> DNA
<213> Kochia scoparia

<400> 746

tgagaaactg cattttctac aggtagaaac tttcctccat cactaaagct gtccggagtc 60

aaattcaaga ctcccattac agaggttttg cacgaccaat cccacaagtg gttcccaact 120

ggcaaaaccc tactcattcc gtccttccca atgagtgaat ctccaccaag tttatcccac 180

gcttcaaata gtccaccaga 200

<210> 747
<211> 200
<212> DNA
<213> Kochia scoparia

<400>       747

tacctgtaga aaatgcagtt tctcaggttc gtcaaatgat ctcagaaggt gctgacataa    60

ttgatattgg agcgcaatca acaaggccca tggcaactag gatttctgct gaggaagagc   120

tggaaagact agtccctgtt ttagaaggtg tcaaggatgt tatcgaggaa gaaggaagaa   180

tgttatcagt ggatacattt                                               200


<210>       748
<211>       200
<212>       DNA
<213>       Kochia scoparia

<400>       748

aaatgtatcc actgataaca ttcttccttc ttcctcgata acatccttga caccttctaa    60

aacagggact agtctttcca gctcttcctc agcagaaatc ctagttgcca tgggccttgt   120

tgattgcgct ccaatatcaa ttatgtcagc accttctgag atcatttgac gaacctgaga   180

aactgcattt tctacaggta                                               200


<210>       749
<211>       200
<212>       DNA
<213>       Kochia scoparia

<400>       749

aagaatgtta tcagtggata cattttactc aaaagttgct tctgaagctg tcaataaggg    60

agcacatatg gtgaatgatg tttcgagtgg aaagctagat cctgagatgt tcaatgttgt   120

tgcagggctt aaagtgcctt atatagcaat gcacatgcga ggtgatcctt cttcaatgca   180

aaatgctgat aatttgacat                                               200


<210>       750
<211>       200
<212>       DNA
<213>       Kochia scoparia

<400>       750

atgtcaaatt atcagcattt tgcattgaag aaggatcacc tcgcatgtgc attgctatat    60

aaggcacttt aagccctgca acaacattga acatctcagg atctagcttt ccactcgaaa   120

catcattcac catatgtgct cccttattga cagcttcaga agcaactttt gagtaaaatg   180

tatccactga taacattctt                                               200


<210>       751
<211>       200
<212>       DNA
<213>       Kochia scoparia

<400>       751

atgcaaaatg ctgataattt gacatacaat gatgtttgta aggaggtggc tttagagttg    60

agctctagga ttacagatgc agaattatca ggtattcctg cttggaggat agttattgat   120

cctggcattg gattttccaa gaatacaaag caaaatttgg aaattctcac agggttgaaa   180

agaattcggc aagaaatagc                                                200

<210>       752
<211>       200
<212>       DNA
<213>       Kochia scoparia

<400>       752

gctatttctt gccgaattct tttcaaccct gtgagaattt ccaaattttg ctttgtattc    60

ttggaaaatc caatgccagg atcaataact atcctccaag caggaatacc tgataattct   120

gcatctgtaa tcctagagct caactctaaa gccacctcct tacaaacatc attgtatgtc   180

aaattatcag cattttgcat                                                200

<210>       753
<211>       200
<212>       DNA
<213>       Kochia scoparia

<400>       753

tgaaaagaat tcggcaagaa atagcaaaaa agagtttggc tgtggctcat ggtcccttac    60

tgattggacc ttcaagaaag aggtttttgg gtgagatctg caatcgtcct gtagcatctg   120

atagagatcc ggcaactgtt gcttctatca ccgctggagt tttgggtggt gcaaacattg   180

taagagtaca taatgttcgg                                                200

<210>       754
<211>       200
<212>       DNA
<213>       Kochia scoparia

<400>       754

ccgaacatta tgtactctta caatgtttgc accacccaaa actccagcgg tgatagaagc    60

aacagttgcc ggatctctat cagatgctac aggacgattg cagatctcac ccaaaaacct   120

ctttcttgaa ggtccaatca gtaagggacc atgagccaca gccaaactct tttttgctat   180

ttcttgccga attcttttca                                                200

<210>       755
<211>       200
<212>       DNA

<213>       Kochia scoparia

<400>       755

cattgtaaga gtacataatg ttcgggatag ccttgatggt gtcaagctat gtgatgcaat    60

gcttgaatat cagtataact gaagattctt agattcttag attcttagat tctaagagtt   120

gattttatag attattgtct ttgttgtgtg ccttcggcag atatgaataa tatgattgta   180

attttgttgc ttatgatttt                                               200


<210>       756
<211>       200
<212>       DNA
<213>       Kochia scoparia

<400>       756

aaaatcataa gcaacaaaat tacaatcata ttattcatat ctgccgaagg cacacaacaa    60

agacaataat ctataaaatc aactcttaga atctaagaat ctaagaatct aagaatcttc   120

agttatactg atattcaagc attgcatcac atagcttgac accatcaagg ctatcccgaa   180

cattatgtac tcttacaatg                                               200


<210>       757
<211>       200
<212>       DNA
<213>       Kochia scoparia

<400>       757

ttgtaatttt gttgcttatg attttcttcc tacctttctt ttatcataga atttagatta    60

aaaaatgtac ttgaatttca taaatggtga aataaagttt gtcggtgctt catgcaatac   120

gacttttcta gtttgcattg ctctatcata ggggctgtaa gcagattagc ccagtcaatt   180

cacattagat ttagacggcc                                               200


<210>       758
<211>       200
<212>       DNA
<213>       Kochia scoparia

<400>       758

ggccgtctaa atctaatgtg aattgactgg gctaatctgc ttacagcccc tatgatagag    60

caatgcaaac tagaaaagtc gtattgcatg aagcaccgac aaactttatt tcaccattta   120

tgaaattcaa gtacattttt taatctaaat tctatgataa aagaaaggta ggaagaaaat   180

cataagcaac aaaattacaa                                               200


<210>       759
<211>       200

<212>	DNA
<213>	Kochia scoparia

<400>	759

caattcacat tagatttaga cggccggtgc aaaaacagca taatgttgct aacgctgttt	60

atacatttaa actatatgat ctctaagagc aaaatacaaa ataatacaat gtgtttcatg	120

aagatgtcca atcatttctc cctcaaaaaa agatcgtgat gaacaatcag gtactttgat	180

tttgttgtag aacctcagct	200


<210>	760
<211>	200
<212>	DNA
<213>	Kochia scoparia

<400>	760

agctgaggtt ctacaacaaa atcaaagtac ctgattgttc atcacgatct ttttttgagg	60

gagaaatgat tggacatctt catgaaacac attgtattat tttgtatttt gctcttagag	120

atcatatagt ttaaatgtat aaacagcgtt agcaacatta tgctgttttt gcaccggccg	180

tctaaatcta atgtgaattg	200


<210>	761
<211>	200
<212>	DNA
<213>	Kochia scoparia

<400>	761

ttgattttgt tgtagaacct cagcttcagc cctagccatg ggatcatctt tttctccttc	60

tcgctcctca gccaatttct ttctgtgcat ctcctctgca gcttctattg aagctttgtg	120

ctcagcctgg agacgctgca tttcctcttc catctgctgt ctttcgaacc atgtatcagc	180

atccgcccaa actgcagaaa	200


<210>	762
<211>	200
<212>	DNA
<213>	Kochia scoparia

<400>	762

tttctgcagt ttgggcggat gctgatacat ggttcgaaag acagcagatg gaagaggaaa	60

tgcagcgtct ccaggctgag cacaaagctt caatagaagc tgcagaggag atgcacagaa	120

agaaattggc tgaggagcga gaaggagaaa aagatgatcc catggctagg gctgaagctg	180

aggttctaca acaaaatcaa	200


<210>	763

<211>	200
<212>	DNA
<213>	Kochia scoparia

<400>	763

tcagcatccg cccaaactgc agaaataatt taaacaagtt aatcatacgg agtgtgcagc	60

atggcaaatt ggcaataaga tggtgcaagt aacaagctca aatgatccac catagccaaa	120

ttcctaacca ttggactgtg tcatatactc atatcagcta gtagaaacat tctcaccata	180

accctaacta ttcgaggata	200


<210>	764
<211>	200
<212>	DNA
<213>	Kochia scoparia

<400>	764

tatcctcgaa tagttagggt tatggtgaga atgtttctac tagctgatat gagtatatga	60

cacagtccaa tggttaggaa tttggctatg gtggatcatt tgagcttgtt acttgcacca	120

tcttattgcc aatttgccat gctgcacact ccgtatgatt aacttgttta aattatttct	180

gcagtttggg cggatgctga	200


<210>	765
<211>	200
<212>	DNA
<213>	Kochia scoparia

<400>	765

ccataaccct aactattcga ggataagtga taccattcaa caatagtaaa gatcaaatca	60

ggtattacta gacctatcaa agtaatacgg agtagttgtt attgttacag agttaggaag	120

aatcctattt tcccatttct cctatcaatc ctaaactttt gtgactgaac tatcgacata	180

tagtgtgaag aacatgaatg	200


<210>	766
<211>	200
<212>	DNA
<213>	Kochia scoparia

<400>	766

cattcatgtt cttcacacta tatgtcgata gttcagtcac aaaagtttag gattgatagg	60

agaaatggga aaataggatt cttcctaact ctgtaacaat aacaactact ccgtattact	120

ttgataggtc tagtaatacc tgatttgatc tttactattg ttgaatggta tcacttatcc	180

tcgaatagtt agggttatgg	200

<210>	767
<211>	200
<212>	DNA
<213>	Kochia scoparia

<400>	767

acatatagtg tgaagaacat gaatggcaga agtcacaact ctgattcctg agtcctgact	60

ataaactatt ggatgactgt gatacatcat tagtccttta agcattgagt tactctattc	120

tacatagtta aattatatag attccaagat ccgaatccat gaattatcaa ttaccataaa	180

ttgatcttct tcctttgagt	200

<210>	768
<211>	200
<212>	DNA
<213>	Kochia scoparia

<400>	768

actcaaagga agaagatcaa tttatggtaa ttgataattc atggattcgg atcttggaat	60

ctatataatt taactatgta gaatagagta actcaatgct taaaggacta atgatgtatc	120

acagtcatcc aatagtttat agtcaggact caggaatcag agttgtgact tctgccattc	180

atgttcttca cactatatgt	200

<210>	769
<211>	200
<212>	DNA
<213>	Kochia scoparia

<400>	769

ataaattgat cttcttcctt tgagttttgt caacagttcc tactccgaaa tatgtcttaa	60

atctagaagc aatagacact tatcaatgat acagcaatag acgaattcat ctacccaagg	120

cagcatcaac aacagctgcc gtttgcgata atcgaaacta tcatagtgtc cttttcacta	180

ttagtgcaga tgttatctag	200

<210>	770
<211>	200
<212>	DNA
<213>	Kochia scoparia

<400>	770

ctagataaca tctgcactaa tagtgaaaag gacactatga tagtttcgat tatcgcaaac	60

ggcagctgtt gttgatgctg ccttgggtag atgaattcgt ctattgctgt atcattgata	120

agtgtctatt gcttctagat ttaagacata tttcggagta ggaactgttg acaaaactca	180

aaggaagaag atcaatttat	200

<210>	771
<211>	200
<212>	DNA
<213>	Kochia scoparia

<400>	771

cactattagt gcagatgtta tctagggtct aaacctctat gtttctacat tcctggaaaa	60

tcttattctg tactgcataa tcctctcaca aaaaattcgc caatgaatga caaattagct	120

atccagaatt gcagattgat tgaatcaaat tgatcatggc agcagcacaa ttgcagcagt	180

tatcaattga agcacaatat	200

<210>	772
<211>	200
<212>	DNA
<213>	Kochia scoparia

<400>	772

atattgtgct tcaattgata actgctgcaa ttgtgctgct gccatgatca atttgattca	60

atcaatctgc aattctggat agctaatttg tcattcattg gcgaattttt tgtgagagga	120

ttatgcagta cagaataaga ttttccagga atgtagaaac atagaggttt agaccctaga	180

taacatctgc actaatagtg	200

<210>	773
<211>	200
<212>	DNA
<213>	Kochia scoparia

<400>	773

gcagttatca attgaagcac aatattacct ctcaatcata acaatcaaag cacagaaatt	60

ccaatcaata actccacaat atagtatcaa tctctaactc agaaactaaa cagaagcaaa	120

aacccattca atcaaagcaa cagtaaaatt agggtttgaa ttagaaagaa agagagggaa	180

gagaaacata ctgggttgcg	200

<210>	774
<211>	200
<212>	DNA
<213>	Kochia scoparia

<400>	774

cgcaacccag tatgtttctc ttccctctct ttctttctaa ttcaaaccct aattttactg	60

ttgctttgat tgaatgggtt tttgcttctg tttagtttct gagttagaga ttgatactat	120

attgtggagt tattgattgg aatttctgtg ctttgattgt tatgattgag aggtaatatt	180

gtgcttcaat tgataactgc	200

<210>	775
<211>	200
<212>	DNA
<213>	Kochia scoparia

<400>	775

gggaagagaa acatactggg ttgcgcagcc catccctgtt caccaccctt aatcttctcg	60

ggaagagcgt aattgacagt gagaacacga ccgtaaagct cagcaccatc catgttatcc	120

atggcagaag aagcgtcgtc tctctccaag aaagtgacga aaccgaaaga acgatgcttg	180

ttggtggcca aatcaagagg	200

<210>	776
<211>	200
<212>	DNA
<213>	Kochia scoparia

<400>	776

cctcttgatt tggccaccaa caagcatcgt tctttcggtt tcgtcacttt cttggagaga	60

gacgacgctt cttctgccat ggataacatg gatggtgctg agctttacgg tcgtgttctc	120

actgtcaatt acgctcttcc cgagaagatt aagggtggtg aacagggatg ggctgcgcaa	180

cccagtatgt ttctcttccc	200

<210>	777
<211>	200
<212>	DNA
<213>	Kochia scoparia

<400>	777

gcagcttcaa gtcttgcttt tttgcactca tcaccagaaa ctactattga ggtttgttct	60

aaagagcatg aagttgtaat tgctctaggg agcaatgtag gagacagatt agataacttt	120

aaccaagctc tgcagctaat gaagaaatta ggagtaaata tcacaaggca tggctgctta	180

tacgagacag atcctgccta	200

<210>	778
<211>	200
<212>	DNA
<213>	Kochia scoparia

<400>	778

taggcaggat ctgtctcgta taagcagcca tgccttgtga tatttactcc taatttcttc	60

attagctgca gagcttggtt aaagttatct aatctgtctc ctacattgct ccctagagca	120

attacaactt catgctcttt agaacaaacc tcaatagtag tttctggtga tgagtgcaaa	180

aaagcaagac ttgaagctgc 200

<210> 779
<211> 200
<212> DNA
<213> Kochia scoparia

<400> 779
   gcttatacga gacagatcct gcctatgtga ctgatcaacc aaagtttctt aactctgcag 60
   tgaggggctt cacaaaactt gggcctctag aactattagg gatgctgaag aaaattgaga 120
   aggatatggg tcgaactaat ggaataagat atggccctag gccaattgac ttagacattc 180
   ttttttatgg gaagtttagg 200

<210> 780
<211> 200
<212> DNA
<213> Kochia scoparia

<400> 780

cctaaacttc ccataaaaaa gaatgtctaa gtcaattggc ctagggccat atcttattcc 60

attagttcga cccatatcct tctcaatttt cttcagcatc cctaatagtt ctagaggccc 120

aagttttgtg aagcccctca ctgcagagtt aagaaacttt ggttgatcag tcacataggc 180

aggatctgtc tcgtataagc 200

<210> 781
<211> 200
<212> DNA
<213> Kochia scoparia

<400> 781

cattcttttt tatgggaagt ttagggtaag ctctgagaaa cttacagttc cgcatgaaag 60

gatatgggaa agaccctttg tgatggcacc actaattgat ataattggat cggatgtaga 120

aaatgacact gttgctgcat ggcattcatt atcaaaattt tctggtggac tatttgaagc 180

gtgggataaa cttggtggag 200

<210> 782
<211> 200
<212> DNA
<213> Kochia scoparia

<400> 782

ctccaccaag tttatcccac gcttcaaata gtccaccaga aaattttgat aatgaatgcc 60

atgcagcaac agtgtcattt tctacatccg atccaattat atcaattagt ggtgccatca 120

caaagggtct ttcccatatc ctttcatgcg gaactgtaag tttctcagag cttaccctaa 180

acttcccata aaaaagaatg 200

<210> 783
<211> 200
<212> DNA
<213> Kochia scoparia

<400> 783

gaagcgtggg ataaacttgg tggagattca ctcattggga aggacggaat gagtagggtt 60

ttgccagttg ggaaccactt gtgggattgg tcgtgcaaaa cctctgtaat gggagtcttg 120

aatttgactc cggacagctt tagtgatgga ggaaagtttc tacctgtaga aaatgcagtt 180

tctcaggttc gtcaaatgat 200

<210> 784
<211> 200
<212> DNA
<213> Kochia scoparia

<400> 784

atcatttgac gaacctgaga aactgcattt tctacaggta gaaactttcc tccatcacta 60

aagctgtccg gagtcaaatt caagactccc attacagagg ttttgcacga ccaatcccac 120

aagtggttcc caactggcaa aaccctactc attccgtcct tcccaatgag tgaatctcca 180

ccaagtttat cccacgcttc 200

<210> 785
<211> 200
<212> DNA
<213> Kochia scoparia

<400> 785

cagtttctca ggttcgtcaa atgatctcag aaggtgctga cataattgat attggagcgc 60

aatcaacaag gcccatggca actaggattt ctgctgagga agagctggaa agactagtcc 120

ctgttttaga aggtgtcaag gatgttatcg aggaagaagg aagaatgtta tcagtggata 180

cattttactc aaaagttgct 200

<210> 786
<211> 200
<212> DNA
<213> Kochia scoparia

<400> 786

agcaactttt gagtaaaatg tatccactga taacattctt ccttcttcct cgataacatc 60

cttgacacct tctaaaacag ggactagtct ttccagctct tcctcagcag aaatcctagt 120

tgccatgggc cttgttgatt gcgctccaat atcaattatg tcagcacctt ctgagatcat 180

ttgacgaacc tgagaaactg 200

<210> 787
<211> 200
<212> DNA
<213> Kochia scoparia

<400> 787

ggatacattt tactcaaaag ttgcttctga agctgtcaat aagggagcac atatggtgaa 60

tgatgtttcg agtggaaagc tagatcctga gatgttcaat gttgttgcag ggcttaaagt 120

gccttatata gcaatgcaca tgcgaggtga tccttcttca atgcaaaatg ctgataattt 180

gacatacaat gatgtttgta 200

<210> 788
<211> 200
<212> DNA
<213> Kochia scoparia

<400> 788

tacaaacatc attgtatgtc aaattatcag cattttgcat tgaagaagga tcacctcgca 60

tgtgcattgc tatataaggc actttaagcc ctgcaacaac attgaacatc tcaggatcta 120

gctttccact cgaaacatca ttcaccatat gtgctccctt attgacagct tcagaagcaa 180

cttttgagta aaatgtatcc 200

<210> 789
<211> 200
<212> DNA
<213> Kochia scoparia

<400> 789

aatttgacat acaatgatgt ttgtaaggag gtggctttag agttgagctc taggattaca 60

gatgcagaat tatcaggtat tcctgcttgg aggatagtta ttgatcctgg cattggattt 120

tccaagaata caaagcaaaa tttggaaatt ctcacagggt tgaaaagaat tcggcaagaa 180

atagcaaaaa agagtttggc 200

<210> 790
<211> 200
<212> DNA
<213> Kochia scoparia

<400> 790

gccaaactct tttttgctat ttcttgccga attcttttca accctgtgag aatttccaaa 60

ttttgctttg tattcttgga aaatccaatg ccaggatcaa taactatcct ccaagcagga 120

atacctgata attctgcatc tgtaatccta gagctcaact ctaaagccac ctccttacaa 180

acatcattgt atgtcaaatt 200

<210> 791
<211> 200
<212> DNA
<213> Kochia scoparia

<400> 791

aagaaatagc aaaaaagagt ttggctgtgg ctcatggtcc cttactgatt ggaccttcaa 60

gaaagaggtt tttgggtgag atctgcaatc gtcctgtagc atctgataga gatccggcaa 120

ctgttgcttc tatcaccgct ggagttttgg gtggtgcaaa cattgtaaga gtacataatg 180

ttcgggatag ccttgatggt 200

<210> 792
<211> 200
<212> DNA
<213> Kochia scoparia

<400> 792

accatcaagg ctatcccgaa cattatgtac tcttacaatg tttgcaccac ccaaaactcc 60

agcggtgata gaagcaacag ttgccggatc tctatcagat gctacaggac gattgcagat 120

ctcacccaaa aacctctttc ttgaaggtcc aatcagtaag ggaccatgag ccacagccaa 180

actctttttt gctatttctt 200

<210> 793
<211> 200
<212> DNA
<213> Kochia scoparia

<400> 793

taattcaaat ggattttaaa gattacgtat gtacttttgc agcttcaagt cttgcttttt 60

tgcactcatc accagaaact actattgagg tttgttctaa agagcatgaa gttgtaattg 120

ctctagggag caatgtagga gacagattag ataactttaa ccaagctctg cagctaatga 180

agaaattagg agtaaatatc 200

<210> 794
<211> 200
<212> DNA
<213> Kochia scoparia

<400> 794

gatatttact cctaatttct tcattagctg cagagcttgg ttaaagttat ctaatctgtc 60

tcctacattg ctccctagag caattacaac ttcatgctct ttagaacaaa cctcaatagt 120

agtttctggt gatgagtgca aaaaagcaag acttgaagct gcaaaagtac atacgtaatc 180

tttaaaatcc atttgaatta 200

<210> 795
<211> 200
<212> DNA
<213> Kochia scoparia

<400> 795

aatgaagaaa ttaggagtaa atatcacaag gcatggctgc ttatacgaga cagatcctgc 60

ctatgtgact gatcaaccaa agtttcttaa ctctgcagtg aggggcttca caaaacttgg 120

gcctctagaa ctattaggga tgctgaagaa aattgagaag gatatgggtc gaactaatgg 180

aataagatat ggccctaggc 200

<210> 796
<211> 200
<212> DNA
<213> Kochia scoparia

<400> 796

gcctagggcc atatcttatt ccattagttc gacccatatc cttctcaatt ttcttcagca 60

tccctaatag ttctagaggc ccaagttttg tgaagcccct cactgcagag ttaagaaact 120

ttggttgatc agtcacatag gcaggatctg tctcgtataa gcagccatgc cttgtgatat 180

ttactcctaa tttcttcatt 200

<210> 797
<211> 200
<212> DNA
<213> Kochia scoparia

<400> 797

aatggaataa gatatggccc taggccaatt gacttagaca ttctttttta tgggaagttt 60

agggtaagct ctgagaaact tacagttccg catgaaagga tatgggaaag accctttgtg 120

atggcaccac taattgatat aattggatcg gatgtagaaa atgacactgt tgctgcatgg 180

cattcattat caaaattttc 200

<210> 798
<211> 200
<212> DNA
<213> Kochia scoparia

<400> 798

gaaaattttg ataatgaatg ccatgcagca acagtgtcat tttctacatc cgatccaatt 60

atatcaatta gtggtgccat cacaaagggt ctttcccata tcctttcatg cggaactgta 120

agtttctcag agcttaccct aaacttccca taaaaaagaa tgtctaagtc aattggccta 180

gggccatatc ttattccatt 200

<210> 799
<211> 200
<212> DNA
<213> Kochia scoparia

<400> 799

catggcattc attatcaaaa ttttctggtg gactatttga agcgtgggat aaacttggtg 60

gagattcact cattgggaag gacggaatga gtagggtttt gccagttggg aaccacttgt 120

gggattggtc gtgcaaaacc tctgtaatgg gagtcttgaa tttgactccg gacagcttta 180

gtgatggagg aaagtttcta 200

<210> 800
<211> 200
<212> DNA
<213> Kochia scoparia

<400> 800

tagaaacttt cctccatcac taaagctgtc cggagtcaaa ttcaagactc ccattacaga 60

ggttttgcac gaccaatccc acaagtggtt cccaactggc aaaaccctac tcattccgtc 120

cttcccaatg agtgaatctc caccaagttt atcccacgct tcaaatagtc caccagaaaa 180

ttttgataat gaatgccatg 200

<210> 801
<211> 200
<212> DNA
<213> Kochia scoparia

<400> 801

ctttagtgat ggaggaaagt ttctacctgt agaaaatgca gtttctcagg ttcgtcaaat 60

gatctcagaa ggtgctgaca taattgatat tggagcgcaa tcaacaaggc ccatggcaac 120

taggatttct gctgaggaag agctggaaag actagtccct gttttagaag gtgtcaagga 180

tgttatcgag gaagaaggaa 200

<210> 802
<211> 200
<212> DNA
<213> Kochia scoparia

<400> 802

ttccttcttc ctcgataaca tccttgacac cttctaaaac agggactagt ctttccagct 60

cttcctcagc agaaatccta gttgccatgg gccttgttga ttgcgctcca atatcaatta 120

tgtcagcacc ttctgagatc atttgacgaa cctgagaaac tgcattttct acaggtagaa 180

actttcctcc atcactaaag 200

<210> 803
<211> 200
<212> DNA
<213> Kochia scoparia

<400> 803

aaggatgtta tcgaggaaga aggaagaatg ttatcagtgg atacatttta ctcaaaagtt 60

gcttctgaag ctgtcaataa gggagcacat atggtgaatg atgtttcgag tggaaagcta 120

gatcctgaga tgttcaatgt tgttgcaggg cttaaagtgc cttatatagc aatgcacatg 180

cgaggtgatc cttcttcaat 200

<210> 804
<211> 200
<212> DNA
<213> Kochia scoparia

<400> 804

attgaagaag gatcacctcg catgtgcatt gctatataag gcactttaag ccctgcaaca 60

acattgaaca tctcaggatc tagctttcca ctcgaaacat cattcaccat atgtgctccc 120

ttattgacag cttcagaagc aacttttgag taaaatgtat ccactgataa cattcttcct 180

tcttcctcga taacatcctt 200

<210> 805
<211> 200
<212> DNA
<213> Kochia scoparia

<400> 805

acatgcgagg tgatccttct tcaatgcaaa atgctgataa tttgacatac aatgatgttt 60

gtaaggaggt ggctttagag ttgagctcta ggattacaga tgcagaatta tcaggtattc 120

ctgcttggag gatagttatt gatcctggca ttggattttc caagaataca aagcaaaatt 180

tggaaattct cacagggttg 200

<210> 806
<211> 200
<212> DNA
<213> Kochia scoparia

<400> 806

caaccctgtg agaatttcca aattttgctt tgtattcttg gaaaatccaa tgccaggatc 60

aataactatc ctccaagcag gaatacctga taattctgca tctgtaatcc tagagctcaa 120

ctctaaagcc acctccttac aaacatcatt gtatgtcaaa ttatcagcat tttgcattga 180

agaaggatca cctcgcatgt 200

<210> 807
<211> 200
<212> DNA
<213> Kochia scoparia

<400> 807

aaatttggaa attctcacag ggttgaaaag aattcggcaa gaaatagcaa aaaagagttt 60

ggctgtggct catggtccct tactgattgg accttcaaga aagaggtttt tgggtgagat 120

ctgcaatcgt cctgtagcat ctgatagaga tccggcaact gttgcttcta tcaccgctgg 180

agttttgggt ggtgcaaaca 200

<210> 808
<211> 200
<212> DNA
<213> Kochia scoparia

<400> 808

tgtttgcacc acccaaaact ccagcggtga tagaagcaac agttgccgga tctctatcag 60

atgctacagg acgattgcag atctcaccca aaaacctctt tcttgaaggt ccaatcagta 120

agggaccatg agccacagcc aaactctttt ttgctatttc ttgccgaatt cttttcaacc 180

ctgtgagaat ttccaaattt 200

<210> 809
<211> 200
<212> DNA
<213> Kochia scoparia

<400> 809

gctggagttt tgggtggtgc aaacattgta agagtacata atgttcggga tagccttgat 60

ggtgtcaagc tatgtgatgc aatgcttgaa tatcagtata actgaagatt cttagattct 120

tagattctta gattctaaga gttgatttta tagattattg tctttgttgt gtgccttcgg 180

cagatatgaa taatatgatt 200

<210> 810
<211> 200
<212> DNA
<213> Kochia scoparia

<400>       810

aatcatatta ttcatatctg ccgaaggcac acaacaaaga caataatcta taaaatcaac    60

tcttagaatc taagaatcta agaatctaag aatcttcagt tatactgata ttcaagcatt   120

gcatcacata gcttgacacc atcaaggcta tcccgaacat tatgtactct tacaatgttt   180

gcaccaccca aaactccagc                                               200


<210>       811
<211>       200
<212>       DNA
<213>       Kochia scoparia

<400>       811

ttcggcagat atgaataata tgattgtaat tttgttgctt atgattttct tcctaccttt    60

cttttatcat agaatttaga ttaaaaaatg tacttgaatt tcataaatgg tgaaataaag   120

tttgtcggtg cttcatgcaa tacgactttt ctagtttgca ttgctctatc ataggggctg   180

taagcagatt agcccagtca                                               200


<210>       812
<211>       200
<212>       DNA
<213>       Kochia scoparia

<400>       812

tgactgggct aatctgctta cagcccctat gatagagcaa tgcaaactag aaaagtcgta    60

ttgcatgaag caccgacaaa ctttatttca ccatttatga aattcaagta cattttttaa   120

tctaaattct atgataaaag aaaggtagga agaaaatcat aagcaacaaa attacaatca   180

tattattcat atctgccgaa                                               200


<210>       813
<211>       200
<212>       DNA
<213>       Kochia scoparia

<400>       813

ggctgtaagc agattagccc agtcaattca cattagattt agacggccgg tgcaaaaaca    60

gcataatgtt gctaacgctg tttatacatt taaactatat gatctctaag agcaaaatac   120

aaaataatac aatgtgtttc atgaagatgt ccaatcattt ctccctcaaa aaaagatcgt   180

gatgaacaat caggtacttt                                               200


<210>       814
<211>       200
<212>       DNA
<213>       Kochia scoparia

<400>       814

aaagtacctg attgttcatc acgatctttt tttgagggag aaatgattgg acatcttcat    60

gaaacacatt gtattatttt gtattttgct cttagagatc atatagttta aatgtataaa   120

cagcgttagc aacattatgc tgtttttgca ccggccgtct aaatctaatg tgaattgact   180

gggctaatct gcttacagcc                                               200

<210>       815
<211>       200
<212>       DNA
<213>       Kochia scoparia

<400>       815

atcgtgatga acaatcaggt actttgattt tgttgtagaa cctcagcttc agccctagcc    60

atgggatcat ctttttctcc ttctcgctcc tcagccaatt tctttctgtg catctcctct   120

gcagcttcta ttgaagcttt gtgctcagcc tggagacgct gcatttcctc ttccatctgc   180

tgtctttcga accatgtatc                                               200

<210>       816
<211>       200
<212>       DNA
<213>       Kochia scoparia

<400>       816

gatacatggt tcgaaagaca gcagatggaa gaggaaatgc agcgtctcca ggctgagcac    60

aaagcttcaa tagaagctgc agaggagatg cacagaaaga aattggctga ggagcgagaa   120

ggagaaaaag atgatcccat ggctagggct gaagctgagg ttctacaaca aaatcaaagt   180

acctgattgt tcatcacgat                                               200

<210>       817
<211>       200
<212>       DNA
<213>       Kochia scoparia

<400>       817

tctgctgtct ttcgaaccat gtatcagcat ccgcccaaac tgcagaaata atttaaacaa    60

gttaatcata cggagtgtgc agcatggcaa attggcaata agatggtgca agtaacaagc   120

tcaaatgatc caccatagcc aaattcctaa ccattggact gtgtcatata ctcatatcag   180

ctagtagaaa cattctcacc                                               200

<210>       818
<211>       200
<212>       DNA

<213>       Kochia scoparia

<400>       818

ggtgagaatg tttctactag ctgatatgag tatatgacac agtccaatgg ttaggaattt    60

ggctatggtg gatcatttga gcttgttact tgcaccatct tattgccaat ttgccatgct   120

gcacactccg tatgattaac ttgtttaaat tatttctgca gtttgggcgg atgctgatac   180

atggttcgaa agacagcaga                                               200

<210>       819
<211>       200
<212>       DNA
<213>       Kochia scoparia

<400>       819

atcagctagt agaaacattc tcaccataac cctaactatt cgaggataag tgataccatt    60

caacaatagt aaagatcaaa tcaggtatta ctagacctat caaagtaata cggagtagtt   120

gttattgtta cagagttagg aagaatccta ttttcccatt tctcctatca atcctaaact   180

tttgtgactg aactatcgac                                               200

<210>       820
<211>       200
<212>       DNA
<213>       Kochia scoparia

<400>       820

gtcgatagtt cagtcacaaa agtttaggat tgataggaga aatgggaaaa taggattctt    60

cctaactctg taacaataac aactactccg tattactttg ataggtctag taatacctga   120

tttgatcttt actattgttg aatggtatca cttatcctcg aatagttagg gttatggtga   180

gaatgtttct actagctgat                                               200

<210>       821
<211>       200
<212>       DNA
<213>       Kochia scoparia

<400>       821

aaacttttgt gactgaacta tcgacatata gtgtgaagaa catgaatggc agaagtcaca    60

actctgattc ctgagtcctg actataaact attggatgac tgtgatacat cattagtcct   120

ttaagcattg agttactcta ttctacatag ttaaattata tagattccaa gatccgaatc   180

catgaattat caattaccat                                               200

<210>       822
<211>       200

<212>       DNA
<213>       Kochia scoparia

<400>       822

atggtaattg ataattcatg gattcggatc ttggaatcta tataatttaa ctatgtagaa    60

tagagtaact caatgcttaa aggactaatg atgtatcaca gtcatccaat agtttatagt   120

caggactcag gaatcagagt tgtgacttct gccattcatg ttcttcacac tatatgtcga   180

tagttcagtc acaaaagttt                                                200


<210>       823
<211>       200
<212>       DNA
<213>       Kochia scoparia

<400>       823

gaatccatga attatcaatt accataaatt gatcttcttc ctttgagttt tgtcaacagt    60

tcctactccg aaatatgtct taaatctaga agcaatagac acttatcaat gatacagcaa   120

tagacgaatt catctaccca aggcagcatc aacaacagct gccgtttgcg ataatcgaaa   180

ctatcatagt gtccttttca                                                200


<210>       824
<211>       200
<212>       DNA
<213>       Kochia scoparia

<400>       824

tgaaaaggac actatgatag tttcgattat cgcaaacggc agctgttgtt gatgctgcct    60

tgggtagatg aattcgtcta ttgctgtatc attgataagt gtctattgct tctagattta   120

agacatattt cggagtagga actgttgaca aaactcaaag gaagaagatc aatttatggt   180

aattgataat tcatggattc                                                200


<210>       825
<211>       200
<212>       DNA
<213>       Kochia scoparia

<400>       825

cgaaactatc atagtgtcct tttcactatt agtgcagatg ttatctaggg tctaaacctc    60

tatgtttcta cattcctgga aaatcttatt ctgtactgca taatcctctc acaaaaaatt   120

cgccaatgaa tgacaaatta gctatccaga attgcagatt gattgaatca aattgatcat   180

ggcagcagca caattgcagc                                                200


<210>       826

<211>       200
<212>       DNA
<213>       Kochia scoparia

<400>       826

gctgcaattg tgctgctgcc atgatcaatt tgattcaatc aatctgcaat tctggatagc    60

taatttgtca ttcattggcg aattttttgt gagaggatta tgcagtacag aataagattt   120

tccaggaatg tagaaacata gaggtttaga ccctagataa catctgcact aatagtgaaa   180

aggacactat gatagtttcg                                                200


<210>       827
<211>       200
<212>       DNA
<213>       Kochia scoparia

<400>       827

atcatggcag cagcacaatt gcagcagtta tcaattgaag cacaatatta cctctcaatc    60

ataacaatca aagcacagaa attccaatca ataactccac aatatagtat caatctctaa   120

ctcagaaact aaacagaagc aaaaacccat tcaatcaaag caacagtaaa attagggttt   180

gaattagaaa gaaagagagg                                                200


<210>       828
<211>       200
<212>       DNA
<213>       Kochia scoparia

<400>       828

cctctctttc tttctaattc aaaccctaat tttactgttg ctttgattga atgggttttt    60

gcttctgttt agtttctgag ttagagattg atactatatt gtggagttat tgattggaat   120

ttctgtgctt tgattgttat gattgagagg taatattgtg cttcaattga taactgctgc   180

aattgtgctg ctgccatgat                                                200


<210>       829
<211>       200
<212>       DNA
<213>       Kochia scoparia

<400>       829

ggtttgaatt agaaagaaag agagggaaga gaaacatact gggttgcgca gcccatccct    60

gttcaccacc cttaatcttc tcgggaagag cgtaattgac agtgagaaca cgaccgtaaa   120

gctcagcacc atccatgtta tccatggcag aagaagcgtc gtctctctcc aagaaagtga   180

cgaaaccgaa agaacgatgc                                                200

<210>	830
<211>	200
<212>	DNA
<213>	Kochia scoparia

<400>	830

gcatcgttct ttcggtttcg tcactttctt ggagagagac gacgcttctt ctgccatgga	60

taacatggat ggtgctgagc tttacggtcg tgttctcact gtcaattacg ctcttcccga	120

gaagattaag ggtggtgaac agggatgggc tgcgcaaccc agtatgtttc tcttccctct	180

ctttctttct aattcaaacc	200

<210>	831
<211>	200
<212>	DNA
<213>	Lolium multiflorum

<400>	831

acgaaagctt tgaactctca agtggtgcaa catagataga ttatatatat ttcgaatata	60

attcgaatga acataaaaat tacatataaa aactttaaaa caaacttaaa ctacttcttc	120

ttcctcgatg gccccgcctc atcgtcgggg cggcgacgct tccggctcgt cacctcctcg	180

tcggaggaag ttgggtcctc	200

<210>	832
<211>	200
<212>	DNA
<213>	Lolium multiflorum

<400>	832

gaggacccaa cttcctccga cgaggaggtg acgagccgga agcgtcgccg ccccgacgat	60

gaggcggggc catcgaggaa gaagaagtag tttaagtttg ttttaaagtt tttatatgta	120

atttttatgt tcattcgaat tatattcgaa atatatataa tctatctatg ttgcaccact	180

tgagagttca aagctttcgt	200

<210>	833
<211>	200
<212>	DNA
<213>	Lolium multiflorum

<400>	833

cctcgtcgga ggaagttggg tcctcctcct cgtcagtgtc ctcagcctct tcgtcgtcct	60

cctcctcttc ctcggcctct tcgtcggcct gctccttggc ctcttcgtcc tcctcctcgt	120

cgtcatccca tgcgtcctcc tcctcgtcgt catcccattc gtccttgctg gccggcgggg	180

gggaatcgtc gctgctggac	200

<210>	834
<211>	200
<212>	DNA
<213>	Lolium multiflorum

<400>	834

gtccagcagc gacgattccc ccccgccggc cagcaaggac gaatgggatg acgacgagga	60

ggaggacgca tgggatgacg acgaggagga ggacgaagag gccaaggagc aggccgacga	120

agaggccgag gaagaggagg aggacgacga agaggctgag gacactgacg aggaggagga	180

cccaacttcc tccgacgagg	200

<210>	835
<211>	200
<212>	DNA
<213>	Lolium multiflorum

<400>	835

cgggggggaa tcgtcgctgc tggacgatgc agctttatcc caccaatggc gccatccagg	60

cggcttcccc tcgctgtcgg tgtccgatgg ccaagagaga tcgctcatgg ttgacggagg	120

atggtgagga gagaacagat gaatggcgtc ggccgtcgta aaccgtatat gtaggtctct	180

cgacgaagag agcggcggtt	200

<210>	836
<211>	200
<212>	DNA
<213>	Lolium multiflorum

<400>	836

aaccgccgct ctcttcgtcg agagacctac atatacggtt tacgacggcc gacgccattc	60

atctgttctc tcctcaccat cctccgtcaa ccatgagcga tctctcttgg ccatcggaca	120

ccgacagcga ggggaagccg cctggatggc gccattggtg ggataaagct gcatcgtcca	180

gcagcgacga ttcccccccg	200

<210>	837
<211>	200
<212>	DNA
<213>	Lolium multiflorum

<400>	837

tctctcgacg aagagagcgg cggttgctct tccgcggagt tcgtgctcca ttacagcggt	60

tctcgcatcg aggccacttc ggccgttccc gacgagtcgt ttgggctctc cagaccactt	120

tgcgacggtt caatgcgtcg agggcactcc gacgattgcc cttcccggtg actgcgccgt	180

cgctatgcac gcggtgtgtg	200

<210>	838
<211>	200
<212>	DNA
<213>	Lolium multiflorum

<400>	838

cacacaccgc gtgcatagcg acggcgcagt caccgggaag ggcaatcgtc ggagtgccct	60

cgacgcattg aaccgtcgca aagtggtctg gagagcccaa acgactcgtc gggaacggcc	120

gaagtggcct cgatgcgaga accgctgtaa tggagcacga actccgcgga agagcaaccg	180

ccgctctctt cgtcgagaga	200

<210>	839
<211>	200
<212>	DNA
<213>	Lolium multiflorum

<400>	839

gccgtcgcta tgcacgcggt gtgtgcgcaa ccatgggctc gcggctggga aaatgggcct	60

ccccaggcca caaaatacat ccatccggcg ataactaacg ccggatttcg gcctgggagc	120

cccaacggct gggatgctct tagcccgtgg aacacattcc cgaatcggta ccgacggtct	180

catctctctc gccctgatct	200

<210>	840
<211>	200
<212>	DNA
<213>	Lolium multiflorum

<400>	840

agatcagggc gagagagatg agaccgtcgg taccgattcg ggaatgtgtt ccacgggcta	60

agagcatccc agccgttggg gctcccaggc cgaaatccgg cgttagttat cgccggatgg	120

atgtattttg tggcctgggg aggcccattt tcccagccgc gagcccatgg ttgcgcacac	180

accgcgtgca tagcgacggc	200

<210>	841
<211>	200
<212>	DNA
<213>	Lolium multiflorum

<400>	841

ggtctcatct ctctcgccct gatctatctc tcttccgcga tctggaaggg ccgacggcgt	60

ggcggcgcaa ccacgggcgc tgatggcggc ggcgcgcagt ctgttaagca ggacgcctg	120

cttctcgctc cgtcctccct ctccaaccga gcggcacgag gtagacccgg agaccccaac	180

tccgtcctag cccagtggcg 200

<210> 842
<211> 200
<212> DNA
<213> Lolium multiflorum

<400> 842

cgccactggg ctaggacgga gttggggtct ccgggtctac ctcgtgccgc tcggttggag 60

agggaggacg gagcgagaag caggcgtcct gcttaacaga ctgcgcgccg ccgcccatca 120

gcgcccgtgg ttgcgccgcc acgccgtcgg cccttccaga tcgcggaaga gagatagatc 180

agggcgagag agatgagacc 200

<210> 843
<211> 200
<212> DNA
<213> Lolium multiflorum

<400> 843

ccaactccgt cctagcccag tggcgcccgt gaggcgtcat ctccggcgcc ctctccatct 60

ccgtctgctt cgcacttgta tgcacatact cttcctcctt accccagttt cttggtcttg 120

gcttgtttga ttacgatttc tagggttctt ggcatgctcg gtagttgcag atttgtcttc 180

tgttgggtgt ttgtttccat 200

<210> 844
<211> 200
<212> DNA
<213> Lolium multiflorum

<400> 844

atggaaacaa acacccaaca gaagacaaat ctgcaactac cgagcatgcc aagaacccta 60

gaaatcgtaa tcaaacaagc caagaccaag aaactggggt aaggaggaag agtatgtgca 120

tacaagtgcg aagcagacgg agatggagag ggcgccggag atgacgcctc acgggcgcca 180

ctgggctagg acggagttgg 200

<210> 845
<211> 200
<212> DNA
<213> Lolium multiflorum

<400> 845

tcttctgttg ggtgtttgtt tccatggcct tggctgcatc catcgtttcg tattatgtag 60

ttgtttgttt ccatgccatt gcttgtatcc atcaattgga aagggaagaa agggctacag 120

ggatattagg aattagatca gtccttccta gctgattttc tctgacaggt tgttagtctc 180

gcgcatgtgg tggacttctt                                                200

<210>   846
<211>   200
<212>   DNA
<213>   Lolium multiflorum

<400>   846

aagaagtcca ccacatgcgc gagactaaca acctgtcaga gaaaatcagc taggaaggac    60

tgatctaatt cctaatatcc ctgtagccct ttcttccctt tccaattgat ggatacaagc   120

aatggcatgg aaacaaacaa ctacataata cgaaacgatg gatgcagcca aggccatgga   180

aacaaacacc caacagaaga                                                200

<210>   847
<211>   200
<212>   DNA
<213>   Lolium multiflorum

<400>   847

gtctcgcgca tgtggtggac ttcttgtgct tttggctagc tccaagtctg atgtgtagct    60

gctttctgag attttccaca taaacgttgc tatggctagt aagttgtggc taactaaatc   120

cttgggcaca agctagtaat ctgaatggca tatccgcccc catgccttta attgttgcct   180

aacgatgggc taggttctct                                                200

<210>   848
<211>   200
<212>   DNA
<213>   Lolium multiflorum

<400>   848

agagaaccta gcccatcgtt aggcaacaat taaaggcatg ggggcggata tgccattcag    60

attactagct tgtgcccaag gatttagtta gccacaactt actagccata gcaacgttta   120

tgtggaaaat ctcagaaagc agctacacat cagacttgga gctagccaaa agcacaagaa   180

gtccaccaca tgcgcgagac                                                200

<210>   849
<211>   200
<212>   DNA
<213>   Lolium multiflorum

<400>   849

tgcctaacga tgggctaggt tctcttattt attcattttt gttaaaagaa gaacttgggc    60

atgctaattc agcaaaatag aaaaaatatg tgttgtttct gtatatagag gttcttggtt   120

ttcccttgcc gattctgacc gatttgcggg tttttttttt ttttgtcttt cctcatcgtc 180

aggcaagaag agcaagatgt 200

<210> 850
<211> 200
<212> DNA
<213> Lolium multiflorum

<400> 850

acatcttgct cttcttgcct gacgatgagg aaagacaaaa aaaaaaaaaa cccgcaaatc 60

ggtcagaatc ggcaagggaa aaccaagaac ctctatatac agaaacaaca catatttttt 120

ctattttgct gaattagcat gcccaagttc ttcttttaac aaaaatgaat aaataagaga 180

acctagccca tcgttaggca 200

<210> 851
<211> 200
<212> DNA
<213> Lolium multiflorum

<400> 851

tcgtcaggca agaagagcaa gatgtgacag aggatttaag cagcagcaga actagacctg 60

gctcctatgc tctaagatgc ttcgtgccaa ggacacattc aggaagatgt actcagctgc 120

caagggctac tactgcggag ggctagcgtc tcctaatgcc ttctcaggta gcttgcccaa 180

agttccccaa taataactag 200

<210> 852
<211> 200
<212> DNA
<213> Lolium multiflorum

<400> 852

ctagttatta ttggggaact ttgggcaagc tacctgagaa ggcattagga gacgctagcc 60

ctccgcagta gtagcccttg gcagctgagt acatcttcct gaatgtgtcc ttggcacgaa 120

gcatcttaga gcataggagc caggtctagt tctgctgctg cttaaatcct ctgtcacatc 180

ttgctcttct tgcctgacga 200

<210> 853
<211> 200
<212> DNA
<213> Lolium multiflorum

<400> 853

cccaaagttc cccaataata actagggaac atctcttttc cacctctttc acattccaac 60

caaagacagt cctaactcgt ttcccctctg aacataccag cattggatct tcggcatcct 120

ctcagctcct caagaaaacc accaggccac gctaacctgt tcagggtccg ttcatacacg 180

caatgcgccc tcaccaacga 200

<210> 854
<211> 200
<212> DNA
<213> Lolium multiflorum

<400> 854

tcgttggtga gggcgcattg cgtgtatgaa cggaccctga acaggttagc gtggcctggt 60

ggttttcttg aggagctgag aggatgccga agatccaatg ctggtatgtt cagaggggaa 120

acgagttagg actgtctttg gttggaatgt gaaagaggtg gaaaagagat gttccctagt 180

tattattggg gaactttggg 200

<210> 855
<211> 200
<212> DNA
<213> Lolium multiflorum

<400> 855

acacgcaatg cgccctcacc aacgattccg ccgaccagga ggtcgtgatt gccctgggca 60

gcaacgtggg agacagggtc agcatgttcg acagggcgct gcggctgatg aggagctcgg 120

gcatcaaggt caccaggcac gcctgcctgt acgagaccgc cccagcctac gtgaccgacc 180

agccgcggtt cctcaactct 200

<210> 856
<211> 200
<212> DNA
<213> Lolium multiflorum

<400> 856

agagttgagg aaccgcggct ggtcggtcac gtaggctggg gcggtctcgt acaggcaggc 60

gtgcctggtg accttgatgc ccgagctcct catcagccgc agcgccctgt cgaacatgct 120

gaccctgtct cccacgttgc tgcccagggc aatcacgacc tcctggtcgg cggaatcgtt 180

ggtgagggcg cattgcgtgt 200

<210> 857
<211> 200
<212> DNA
<213> Lolium multiflorum

<400> 857

cgaccagccg cggttcctca actctgccgt taggggcacg acgaagctgg ggcctcacga 60

gttgctcagg aagctcaagg agatcgagaa ggatatagga cgtaccgccg gggtaaggta 120

cggcccgagg ccgatcgatc tggacattct tctgtatggg gactcccgga tcaagaccga 180

gtctctgatt gtgccgcatg 200

<210> 858
<211> 200
<212> DNA
<213> Lolium multiflorum

<400> 858

catgcggcac aatcagagac tcggtcttga tccgggagtc cccatacaga agaatgtcca 60

gatcgatcgg cctcgggccg taccttaccc cggcggtacg tcctatatcc ttctcgatct 120

ccttgagctt cctgagcaac tcgtgaggcc ccagcttcgt cgtgccccta acggcagagt 180

tgaggaaccg cggctggtcg 200

<210> 859
<211> 200
<212> DNA
<213> Lolium multiflorum

<400> 859

accgagtctc tgattgtgcc gcatgaacgc atccatgaga gaccgttcgt cttggcgcct 60

cttgttgacc tcctgggttc gtcggctgag gacggtatgg agaaaagctg gcactctctc 120

tcgaagtgca gtggcgggtt ctttgatttg tggaacaagc tcggcggtga atctgttgtt 180

ggaacagaag gcattaaaag 200

<210> 860
<211> 200
<212> DNA
<213> Lolium multiflorum

<400> 860

cttttaatgc cttctgttcc aacaacagat tcaccgccga gcttgttcca caaatcaaag 60

aacccgccac tgcacttcga gagagagtgc cagcttttct ccataccgtc ctcagccgac 120

gaacccagga ggtcaacaag aggcgccaag acgaacggtc tctcatggat gcgttcatgc 180

ggcacaatca gagactcggt 200

<210> 861
<211> 200
<212> DNA
<213> Lolium multiflorum

<400> 861

ttgttggaac agaaggcatt aaaagggtca tgtctgttgg aaatacgctg ttggactggc 60

gtcagaggac ccttgttatg ggggtgctta acctcacgcc agacagcttc agcgacggag 120

gtaagtttca agaagtggaa gctgccattt cccaggccag gttgttgata tcagaaggtg 180

ctgacataat tgatattggt 200

<210> 862
<211> 200
<212> DNA
<213> Lolium multiflorum

<400> 862

accaatatca attatgtcag caccttctga tatcaacaac ctggcctggg aaatggcagc 60

ttccacttct tgaaacttac ctccgtcgct gaagctgtct ggcgtgaggt taagcacccc 120

cataacaagg gtcctctgac gccagtccaa cagcgtattt ccaacagaca tgaccctttt 180

aatgccttct gttccaacaa 200

<210> 863
<211> 200
<212> DNA
<213> Lolium multiflorum

<400> 863

aggtgctgac ataattgata ttggtgccca gtccaccagg ccctttgcaa ggaggctatc 60

cgcagaagaa gagcttgaga ggctggtccc tgttctggat gctatcatga aactcccaga 120

gatggaaggg aagttgcttt cggtagacac gttctacgcg caagtcgctg ctgaagctgt 180

aaaaagagga gccaccatga 200

<210> 864
<211> 200
<212> DNA
<213> Lolium multiflorum

<400> 864

tcatggtggc tcctcttttt acagcttcag cagcgacttg cgcgtagaac gtgtctaccg 60

aaagcaactt cccttccatc tctgggagtt tcatgatagc atccagaaca gggaccagcc 120

tctcaagctc ttcttctgcg gatagcctcc ttgcaaaggg cctggtggac tgggcaccaa 180

tatcaattat gtcagcacct 200

<210> 865
<211> 200
<212> DNA
<213> Lolium multiflorum

<400> 865

gctgtaaaaa gaggagccac catgatcaat gatgtatctg gtgggcagct tgacccaagt 60

attcttcaag ttgttgctga actgggagtt ccgtatgtta ccatgcacat gagaggcgat 120

ccatcaacta tgcagaatga acagaatcta cagtatgatg atgtctgcaa agaagttgct 180

tctgagttat acgcccggtt 200

<210> 866
<211> 200
<212> DNA
<213> Lolium multiflorum

<400> 866

aaccgggcgt ataactcaga agcaacttct ttgcagacat catcatactg tagattctgt 60

tcattctgca tagttgatgg atcgcctctc atgtgcatgg taacatacgg aactcccagt 120

tcagcaacaa cttgaagaat acttgggtca agctgcccac cagatacatc attgatcatg 180

gtggctcctc tttttacagc 200

<210> 867
<211> 200
<212> DNA
<213> Lolium multiflorum

<400> 867

ttgcttctga gttatacgcc cggttgagag cagcagagct ttctggaatt cctttgtgga 60

ggattattct tgaccctggc attgggtttt ctaagaaatc cacacagaat attgaagtaa 120

tcgcgggttt ggaatccatt agagaagaga tgggtaaaat gagcctaggt gcttcacatg 180

tgccaatatt acttggaccc 200

<210> 868
<211> 200
<212> DNA
<213> Lolium multiflorum

<400> 868

gggtccaagt aatattggca catgtgaagc acctaggctc attttaccca tctcttctct 60

aatggattcc aaacccgcga ttacttcaat attctgtgtg gatttcttag aaaacccaat 120

gccagggtca agaataatcc tccacaaagg aattccagaa agctctgctg ctctcaaccg 180

ggcgtataac tcagaagcaa 200

<210> 869
<211> 200
<212> DNA
<213> Lolium multiflorum

<400> 869

acatgtgcca atattacttg gaccctcgag gaaaagtttc ctaggtgaaa tatgtagtcg 60

cgccgatcca gttcagagag atgctgctac tgcttctgcc gttacaattg cgatcttgaa 120

tggcgctaat atagtaaggg tccataatgt tagatacaat gtggatgctg caaaggtctc 180

tgatgcatta ctcaagtaca 200

<210> 870
<211> 200
<212> DNA
<213> Lolium multiflorum

<400> 870

tgtacttgag taatgcatca gagacctttg cagcatccac attgtatcta acattatgga 60

cccttactat attagcgcca ttcaagatcg caattgtaac ggcagaagca gtagcagcat 120

ctctctgaac tggatcggcg cgactacata tttcacctag gaaacttttc ctcgagggtc 180

caagtaatat tggcacatgt 200

<210> 871
<211> 200
<212> DNA
<213> Lolium multiflorum

<400> 871

gtctctgatg cattactcaa gtacagaaga aaataataga aagtatacag cccggataga 60

agccatacca gttgccagtt ttgtgcaagg gaatgctgat gtggagctca aaccatatgg 120

ggtgtatcat cacttaataa gatcaaagca tgcgtgaata actattacag tgaacaagag 180

atatacagtt ttttcgtgcg 200

<210> 872
<211> 200
<212> DNA
<213> Lolium multiflorum

<400> 872

cgcacgaaaa aactgtatat ctcttgttca ctgtaatagt tattcacgca tgctttgatc 60

ttattaagtg atgatacacc ccatatggtt tgagctccac atcagcattc ccttgcacaa 120

aactggcaac tggtatggct tctatccggg ctgtatactt tctattattt tcttctgtac 180

ttgagtaatg catcagagac 200

<210> 873
<211> 200
<212> DNA
<213> Lolium multiflorum

<400>       873

aagagatata cagttttttc gtgcgcctcc tccttcgtag aatcttcaaa gaagcaaagg    60

taacacagac ctaggcctct ggggttggta tatgatgtaa ccagttttgt gagggtttaa   120

taattatcta atagtgagac ctaagagtaa gacctttata atatactttg cttggaattt   180

cctacttgag agcatcattt                                               200


<210>       874
<211>       200
<212>       DNA
<213>       Lolium multiflorum

<400>       874

aaatgatgct ctcaagtagg aaattccaag caaagtatat tataaaggtc ttactcttag    60

gtctcactat tagataatta ttaaaccctc acaaaactgg ttacatcata taccaacccc   120

agaggcctag gtctgtgtta cctttgcttc tttgaagatt ctacgaagga ggaggcgcac   180

gaaaaaactg tatatctctt                                               200


<210>       875
<211>       200
<212>       DNA
<213>       Lolium multiflorum

<400>       875

agctcctcaa gaaaaccacc aggccacgct aacctgttca gggtccgttc atacacgcaa    60

tgcgccctca ccaacgattc cgccgaccag gaggtcgtga ttgccctggg cagcaacgtg   120

ggagacaggg tcagcatgtt cgacagggcg ctgcggctga tgaggagctc gggcatcaag   180

gtcaccaggc acgcctgcct                                               200


<210>       876
<211>       200
<212>       DNA
<213>       Lolium multiflorum

<400>       876

aggcaggcgt gcctggtgac cttgatgccc gagctcctca tcagccgcag cgccctgtcg    60

aacatgctga ccctgtctcc cacgttgctg cccagggcaa tcacgacctc ctggtcggcg   120

gaatcgttgg tgagggcgca ttgcgtgtat gaacggaccc tgaacaggtt agcgtggcct   180

ggtggttttc ttgaggagct                                               200


<210>       877
<211>       200
<212>       DNA
<213>       Lolium multiflorum

<400>       877

tcaaggtcac caggcacgcc tgcctgtacg agaccgcccc agcctacgtg accgaccagc    60

cgcggttcct caactctgcc gttaggggca cgacgaagct ggggcctcac gagttgctca   120

ggaagctcaa ggagatcgag aaggatatag gacgtaccgc cggggtaagg tacggcccga   180

ggccgatcga tctggacatt                                               200


<210>       878
<211>       200
<212>       DNA
<213>       Lolium multiflorum

<400>       878

aatgtccaga tcgatcggcc tcgggccgta ccttaccccg gcggtacgtc ctatatcctt    60

ctcgatctcc ttgagcttcc tgagcaactc gtgaggcccc agcttcgtcg tgcccctaac   120

ggcagagttg aggaaccgcg gctggtcggt cacgtaggct ggggcggtct cgtacaggca   180

ggcgtgcctg gtgaccttga                                               200


<210>       879
<211>       200
<212>       DNA
<213>       Lolium multiflorum

<400>       879

cccgaggccg atcgatctgg acattcttct gtatggggac tcccggatca agaccgagtc    60

tctgattgtg ccgcatgaac gcatccatga gagaccgttc gtcttggcgc ctcttgttga   120

cctcctgggt tcgtcggctg aggacggtat ggagaaaagc tggcactctc tctcgaagtg   180

cagtggcggg ttctttgatt                                               200


<210>       880
<211>       200
<212>       DNA
<213>       Lolium multiflorum

<400>       880

aatcaaagaa cccgccactg cacttcgaga gagagtgcca gcttttctcc ataccgtcct    60

cagccgacga acccaggagg tcaacaagag gcgccaagac gaacggtctc tcatggatgc   120

gttcatgcgg cacaatcaga gactcggtct tgatccggga gtccccatac agaagaatgt   180

ccagatcgat cggcctcggg                                               200


<210>       881
<211>       200
<212>       DNA

<213>        Lolium multiflorum

<400>        881

aagtgcagtg gcgggttctt tgatttgtgg aacaagctcg gcggtgaatc tgttgttgga    60

acagaaggca ttaaaagggt catgtctgtt ggaaatacgc tgttggactg gcgtcagagg   120

acccttgtta tgggggtgct taacctcacg ccagacagct tcagcgacgg aggtaagttt   180

caagaagtgg aagctgccat                                               200


<210>        882
<211>        200
<212>        DNA
<213>        Lolium multiflorum

<400>        882

atggcagctt ccacttcttg aaacttacct ccgtcgctga agctgtctgg cgtgaggtta    60

agcaccccca taacaagggt cctctgacgc cagtccaaca gcgtatttcc aacagacatg   120

acccttttaa tgccttctgt tccaacaaca gattcaccgc cgagcttgtt ccacaaatca   180

aagaacccgc cactgcactt                                               200


<210>        883
<211>        200
<212>        DNA
<213>        Lolium multiflorum

<400>        883

agtttcaaga agtggaagct gccatttccc aggccaggtt gttgatatca gaaggtgctg    60

acataattga tattggtgcc cagtccacca ggccctttgc aaggaggcta tccgcagaag   120

aagagcttga gaggctggtc cctgttctgg atgctatcat gaaactccca gagatggaag   180

ggaagttgct ttcggtagac                                               200


<210>        884
<211>        200
<212>        DNA
<213>        Lolium multiflorum

<400>        884

gtctaccgaa agcaacttcc cttccatctc tgggagtttc atgatagcat ccagaacagg    60

gaccagcctc tcaagctctt cttctgcgga tagcctcctt gcaaagggcc tggtggactg   120

ggcaccaata tcaattatgt cagcaccttc tgatatcaac aacctggcct gggaaatggc   180

agcttccact tcttgaaact                                               200


<210>        885
<211>        200

<212>	DNA
<213>	Lolium multiflorum

<400>	885

ggaagggaag ttgctttcgg tagacacgtt ctacgcgcaa gtcgctgctg aagctgtaaa	60

aagaggagcc accatgatca atgatgtatc tggtgggcag cttgacccaa gtattcttca	120

agttgttgct gaactgggag ttccgtatgt taccatgcac atgagaggcg atccatcaac	180

tatgcagaat gaacagaatc	200


<210>	886
<211>	200
<212>	DNA
<213>	Lolium multiflorum

<400>	886

gattctgttc attctgcata gttgatggat cgcctctcat gtgcatggta acatacggaa	60

ctcccagttc agcaacaact tgaagaatac ttgggtcaag ctgcccacca gatacatcat	120

tgatcatggt ggctcctctt tttacagctt cagcagcgac ttgcgcgtag aacgtgtcta	180

ccgaaagcaa cttcccttcc	200


<210>	887
<211>	200
<212>	DNA
<213>	Lolium multiflorum

<400>	887

tcaactatgc agaatgaaca gaatctacag tatgatgatg tctgcaaaga agttgcttct	60

gagttatacg cccggttgag agcagcagag ctttctggaa ttcctttgtg gaggattatt	120

cttgaccctg gcattgggtt ttctaagaaa tccacacaga atattgaagt aatcgcgggt	180

ttggaatcca ttagagaaga	200


<210>	888
<211>	200
<212>	DNA
<213>	Lolium multiflorum

<400>	888

tcttctctaa tggattccaa acccgcgatt acttcaatat tctgtgtgga tttcttagaa	60

aacccaatgc cagggtcaag aataatcctc cacaaaggaa ttccagaaag ctctgctgct	120

ctcaaccggg cgtataactc agaagcaact tctttgcaga catcatcata ctgtagattc	180

tgttcattct gcatagttga	200


<210>	889

<211>	200
<212>	DNA
<213>	Lolium multiflorum

<400>	889

cgggtttgga atccattaga gaagagatgg gtaaaatgag cctaggtgct tcacatgtgc	60

caatattact tggaccctcg aggaaaagtt tcctaggtga aatatgtagt cgcgccgatc	120

cagttcagag agatgctgct actgcttctg ccgttacaat tgcgatcttg aatggcgcta	180

atatagtaag ggtccataat	200

<210>	890
<211>	200
<212>	DNA
<213>	Lolium multiflorum

<400>	890

attatggacc cttactatat tagcgccatt caagatcgca attgtaacgg cagaagcagt	60

agcagcatct ctctgaactg gatcggcgcg actacatatt tcacctagga aacttttcct	120

cgagggtcca agtaatattg gcacatgtga agcacctagg ctcattttac ccatctcttc	180

tctaatggat tccaaacccg	200

<210>	891
<211>	200
<212>	DNA
<213>	Lolium multiflorum

<400>	891

aggatatagg gcgcaccgcc gggataaggt acggtccgag gccgatcgat ctggacattc	60

ttctgtatgg ggattcccgg atcaagaccg agtctctgac tgtgccgcat gaacgcatcc	120

atgagagacc gttcgtcttg gcgcctcttg ttgacctcct gggttcatcg ggtgaggatg	180

gtatggagag aagatggcac	200

<210>	892
<211>	200
<212>	DNA
<213>	Lolium multiflorum

<400>	892

gtgccatctt ctctccatac catcctcacc cgatgaaccc aggaggtcaa caagaggcgc	60

caagacgaac ggtctctcat ggatgcgttc atgcggcaca gtcagagact cggtcttgat	120

ccgggaatcc ccatacagaa gaatgtccag atcgatcggc ctcggaccgt accttatccc	180

ggcggtgcgc cctatatcct	200

<210>	893
<211>	200
<212>	DNA
<213>	Lolium multiflorum

<400>	893

agggtcatgt ctgttggaaa tacgctgttg gattggcggg agaggaccct ggttatgggg	60

gtgcttaacc tcacgccaga cagctttagc gatggaggta agtttcaaga ggtggaagct	120

gccatttctc aggccaggct gttaatttca gaaggtgcag gcataattga tattggcgct	180

cagtccacca ggccctttgc	200

<210>	894
<211>	200
<212>	DNA
<213>	Lolium multiflorum

<400>	894

gcaaagggcc tggtggactg agcgccaata tcaattatgc ctgcaccttc tgaaattaac	60

agcctggcct gagaaatggc agcttccacc tcttgaaact tacctccatc gctaaagctg	120

tctggcgtga ggttaagcac ccccataacc agggtcctct cccgccaatc caacagcgta	180

tttccaacag acatgaccct	200

<210>	895
<211>	200
<212>	DNA
<213>	Lolium multiflorum

<400>	895

gcgctcagtc caccaggccc tttgcaaggt ggctatctgc agaagaagag cttgagaggc	60

tggtccctgt tcttaatgct atcatgaaac tccccgagat ggaagggaag ttgctttcag	120

tagatacgtt ctacgcgcaa gtcgcggctg aagccgtgaa aagaggggcc accatgatca	180

atgatgtatc tggtgggcag	200

<210>	896
<211>	200
<212>	DNA
<213>	Lolium multiflorum

<400>	896

ctgcccacca gatacatcat tgatcatggt ggcccctctt ttcacggctt cagccgcgac	60

ttgcgcgtag aacgtatcta ctgaaagcaa cttcccttcc atctcgggga gtttcatgat	120

agcattaaga acagggacca gcctctcaag ctcttcttct gcagatagcc accttgcaaa	180

gggcctggtg gactgagcgc	200

<210>	897
<211>	200
<212>	DNA
<213>	Lolium multiflorum

<400>	897

gatcaatgat gtatctggtg ggcagcttga cccagatatt cttcaagttg ttgctgaatt	60

gggagttccg tatgttgcca tgcacatgag aggggatcca tcaactatgc agaatgagca	120

gaatttacag tatgatgatg tctgcaaaga agttgcttct gagttatact ctcggttgag	180

agcagcagag ttgtctggaa	200

<210>	898
<211>	200
<212>	DNA
<213>	Lolium multiflorum

<400>	898

ttccagacaa ctctgctgct ctcaaccgag agtataactc agaagcaact tctttgcaga	60

catcatcata ctgtaaattc tgctcattct gcatagttga tggatcccct ctcatgtgca	120

tggcaacata cggaactccc aattcagcaa caacttgaag aatatctggg tcaagctgcc	180

caccagatac atcattgatc	200

<210>	899
<211>	200
<212>	DNA
<213>	Lolium multiflorum

<400>	899

ttgagagcag cagagttgtc tggaattcct ttgtggagga ttattcttga ccctggcatt	60

gggtttccaa agaaatccac acagaatatt gaagtaatcg cgggcttgga atccattaga	120

gaagagatgg gtaaaatgag tctaggtgct tcacatgtgc caatattact tggaccctcc	180

aggaaacgtt tcttagggca	200

<210>	900
<211>	200
<212>	DNA
<213>	Lolium multiflorum

<400>	900

tgccctaaga aacgtttcct ggagggtcca agtaatattg gcacatgtga agcacctaga	60

ctcattttac ccatctcttc tctaatggat tccaagcccg cgattacttc aatattctgt	120

gtggatttct ttggaaaccc aatgccaggg tcaagaataa tcctccacaa aggaattcca	180

gacaactctg ctgctctcaa	200

<210>	901
<211>	200
<212>	DNA
<213>	Lolium multiflorum

<400>	901

cgctaacctg ttcagggtcc gttcgtacat gcaatgcgcg gtcaccaacg attccgccga	60

ccaagagatc gtgattgccc tgggcagcaa cgtgggagac agggtcagca cgttcgacag	120

ggcgctgcgg ctgatgagga gctcgggcat caggatcacc aggcacgcct gcctgtacga	180

gaccgcccct gcttacgtga	200

<210>	902
<211>	200
<212>	DNA
<213>	Lolium multiflorum

<400>	902

tcacgtaagc aggggcggtc tcgtacaggc aggcgtgcct ggtgatcctg atgcccgagc	60

tcctcatcag ccgcagcgcc ctgtcgaacg tgctgaccct gtctcccacg ttgctgccca	120

gggcaatcac gatctcttgg tcggcggaat cgttggtgac cgcgcattgc atgtacgaac	180

ggaccctgaa caggttagcg	200

<210>	903
<211>	200
<212>	DNA
<213>	Lolium multiflorum

<400>	903

gatatagggc gcaccgccgg gataaggtac ggtccgaggc cgatcgatct ggacattctt	60

ctgtatgggg attcccggat caagaccgag tctctgactg tgccgcatga acgcatccat	120

gagagaccgt tcgtcttggc gcctcttgtt gacctcctgg gttcatcggg tgaggatggt	180

atggagagaa gatggcactc	200

<210>	904
<211>	200
<212>	DNA
<213>	Lolium multiflorum

<400>	904

gagtgccatc ttctctccat accatcctca cccgatgaac ccaggaggtc aacaagaggc	60

gccaagacga acggtctctc atggatgcgt tcatgcggca cagtcagaga ctcggtcttg	120

atccgggaat ccccatacag aagaatgtcc agatcgatcg gcctcggacc gtaccttatc	180

ccggcggtgc gccctatatc                                                200

<210>   905
<211>   150
<212>   DNA
<213>   Lolium multiflorum

<400>   905

gattccgccg accaagagat cgtgattgcc ctgggcagca acgtgggaga cagggtcagc    60

acgttcgaca gggcgctgcg gctgatgagg agctcgggca tcaggatcac caggcacgcc   120

tgcctgtacg agaccgcccc tgcttacgtg                                    150

<210>   906
<211>   150
<212>   DNA
<213>   Lolium multiflorum

<400>   906

cacgtaagca ggggcggtct cgtacaggca ggcgtgcctg gtgatcctga tgcccgagct    60

cctcatcagc cgcagcgccc tgtcgaacgt gctgaccctg tctcccacgt tgctgcccag   120

ggcaatcacg atctcttggt cggcggaatc                                    150

<210>   907
<211>   21
<212>   DNA
<213>   Unknown

<220>
<223>   Found in multiple species

<400>   907

ttatgtactc ttacaatgtt t                                              21

<210>   908
<211>   21
<212>   DNA
<213>   Unknown

<220>
<223>   Found in multiple species

<400>   908

tgtactctta caatgtttgc a                                              21

<210>   909
<211>   21
<212>   DNA
<213>   Unknown

<220>

<223>	Found in multiple species

<400>	909

tggtgcaaac attgtaagag t	21


<210>	910
<211>	21
<212>	DNA
<213>	Unknown

<220>
<223>	Found in multiple species

<400>	910

tgaaaggata tgggaaagac c	21


<210>	911
<211>	21
<212>	DNA
<213>	Unknown

<220>
<223>	Found in multiple species

<400>	911

tctttcccat atcctttcat g	21


<210>	912
<211>	21
<212>	DNA
<213>	Unknown

<220>
<223>	Found in multiple species

<400>	912

tcttacaatg tttgcaccac c	21


<210>	913
<211>	21
<212>	DNA
<213>	Unknown

<220>
<223>	Found in multiple species

<400>	913

tatgtactct tacaatgttt g	21


<210>	914
<211>	21
<212>	DNA
<213>	Unknown

<220>
<223>	Found in multiple species

<400>	914

tactcttaca atgtttgcac c	21


<210>	915
<211>	21
<212>	DNA
<213>	Unknown

<220>
<223>	Found in multiple species

<400>	915

gtggtgcaaa cattgtaaga g	21


<210>	916
<211>	21
<212>	DNA
<213>	Unknown

<220>
<223>	Found in multiple species

<400>	916

gtgcaaacat tgtaagagta c	21


<210>	917
<211>	21
<212>	DNA
<213>	Unknown

<220>
<223>	Found in multiple species

<400>	917

gtctttccca tatcctttca t	21


<210>	918
<211>	21
<212>	DNA
<213>	Unknown

<220>
<223>	Found in multiple species

<400>	918

gcaaacattg taagagtaca t	21


<210>	919
<211>	21

<212>	DNA
<213>	Unknown

<220>
<223>	Found in multiple species

<400>	919

cattgtaaga gtacataatg t	21


<210>	920
<211>	21
<212>	DNA
<213>	Unknown

<220>
<223>	Found in multiple species

<400>	920

cattatgtac tcttacaatg t	21


<210>	921
<211>	21
<212>	DNA
<213>	Unknown

<220>
<223>	Found in multiple species

<400>	921

attatgtact cttacaatgt t	21


<210>	922
<211>	21
<212>	DNA
<213>	Unknown

<220>
<223>	Found in multiple species

<400>	922

ttttctacat cagacccaat a	21


<210>	923
<211>	21
<212>	DNA
<213>	Unknown

<220>
<223>	Found in multiple species

<400>	923

ttttcattcc atccttcccg a	21

<210>	924
<211>	21
<212>	DNA
<213>	Unknown

<220>
<223>	Found in multiple species

<400>	924

ttttcaatct gtcgatactg c	21


<210>	925
<211>	21
<212>	DNA
<213>	Unknown

<220>
<223>	Found in multiple species

<400>	925

ttttatgaga ccaatcccat a	21


<210>	926
<211>	21
<212>	DNA
<213>	Unknown

<220>
<223>	Found in multiple species

<400>	926

ttttaggtgg tgcaaacatt g	21


<210>	927
<211>	21
<212>	DNA
<213>	Unknown

<220>
<223>	Found in multiple species

<400>	927

tttgcaccac ctaaaactcc a	21


<210>	928
<211>	21
<212>	DNA
<213>	Unknown

<220>
<223>	Found in multiple species

<400>	928

tttctacatc agacccaata c	21

<210> 929
<211> 21
<212> DNA
<213> Unknown

<220>
<223> Found in multiple species

<400> 929

tttcccatat cctttcatgg g 21


<210> 930
<211> 21
<212> DNA
<213> Unknown

<220>
<223> Found in multiple species

<400> 930

tttcattcca tccttcccga t 21


<210> 931
<211> 21
<212> DNA
<213> Unknown

<220>
<223> Found in multiple species

<400> 931

tttcaatctg tcgatactgc g 21


<210> 932
<211> 21
<212> DNA
<213> Unknown

<220>
<223> Found in multiple species

<400> 932

tttatgagac caatcccata a 21


<210> 933
<211> 21
<212> DNA
<213> Unknown

<220>
<223> Found in multiple species

<400> 933

tttaggtggt gcaaacattg t                                                21

<210>    934
<211>    21
<212>    DNA
<213>    Unknown

<220>
<223>    Found in multiple species

<400>    934

tttagggtga gctctgagag c                                                21

<210>    935
<211>    21
<212>    DNA
<213>    Unknown

<220>
<223>    Found in multiple species

<400>    935

ttgtattggg tctgatgtag a                                                21

<210>    936
<211>    21
<212>    DNA
<213>    Unknown

<220>
<223>    Found in multiple species

<400>    936

ttgtaagagt acataatgtg a                                                21

<210>    937
<211>    21
<212>    DNA
<213>    Unknown

<220>
<223>    Found in multiple species

<400>    937

ttggtctcat aaaacctctg t                                                21

<210>    938
<211>    21
<212>    DNA
<213>    Unknown

<220>
<223>    Found in multiple species

<400>       938

ttgggtctga tgtagaaaat g                                                21


<210>       939
<211>       21
<212>       DNA
<213>       Unknown

<220>
<223>       Found in multiple species

<400>       939

ttggcctagg accatacctt a                                                21


<210>       940
<211>       21
<212>       DNA
<213>       Unknown

<220>
<223>       Found in multiple species

<400>       940

ttggacatac tattttatgg g                                                21


<210>       941
<211>       21
<212>       DNA
<213>       Unknown

<220>
<223>       Found in multiple species

<400>       941

ttgctaaggt tcgtcagatg a                                                21


<210>       942
<211>       21
<212>       DNA
<213>       Unknown

<220>
<223>       Found in multiple species

<400>       942

ttgcaccacc taaaactcca g                                                21


<210>       943
<211>       21
<212>       DNA
<213>       Unknown

<220>
<223>	Found in multiple species

<400>	943

ttgattgtat tgggtctgat g	21


<210>	944
<211>	21
<212>	DNA
<213>	Unknown

<220>
<223>	Found in multiple species

<400>	944

ttgacttgga catactattt t	21


<210>	945
<211>	21
<212>	DNA
<213>	Unknown

<220>
<223>	Found in multiple species

<400>	945

ttgactcctg atagctttag t	21


<210>	946
<211>	21
<212>	DNA
<213>	Unknown

<220>
<223>	Found in multiple species

<400>	946

ttgaacttga ctcctgatag c	21


<210>	947
<211>	21
<212>	DNA
<213>	Unknown

<220>
<223>	Found in multiple species

<400>	947

ttctacatca gacccaatac a	21


<210>	948
<211>	21
<212>	DNA

<213>      Unknown

<220>
<223>      Found in multiple species

<400>      948

ttccgccatc actaaagcta t                                                21


<210>      949
<211>      21
<212>      DNA
<213>      Unknown

<220>
<223>      Found in multiple species

<400>      949

ttccctaatc gggaaggatg g                                                21


<210>      950
<211>      21
<212>      DNA
<213>      Unknown

<220>
<223>      Found in multiple species

<400>      950

ttcccgatta gggaacttcc a                                                21


<210>      951
<211>      21
<212>      DNA
<213>      Unknown

<220>
<223>      Found in multiple species

<400>      951

ttcccatatc ctttcatggg g                                                21


<210>      952
<211>      21
<212>      DNA
<213>      Unknown

<220>
<223>      Found in multiple species

<400>      952

ttcccataaa atagtatgtc c                                                21


<210>      953

<211>	21
<212>	DNA
<213>	Unknown

<220>
<223>	Found in multiple species

<400>	953

ttccatcctt cccgattagg g	21


<210>	954
<211>	21
<212>	DNA
<213>	Unknown

<220>
<223>	Found in multiple species

<400>	954

ttcattccat ccttcccgat t	21


<210>	955
<211>	21
<212>	DNA
<213>	Unknown

<220>
<223>	Found in multiple species

<400>	955

ttcaatctgt cgatactgcg g	21


<210>	956
<211>	21
<212>	DNA
<213>	Unknown

<220>
<223>	Found in multiple species

<400>	956

ttcaagactc ccattacaga g	21


<210>	957
<211>	21
<212>	DNA
<213>	Unknown

<220>
<223>	Found in multiple species

<400>	957

ttatgagacc aatcccataa g	21

<210>	958
<211>	21
<212>	DNA
<213>	Unknown

<220>
<223>	Found in multiple species

<400>	958

ttatccctca cattatgtac t	21


<210>	959
<211>	21
<212>	DNA
<213>	Unknown

<220>
<223>	Found in multiple species

<400>	959

ttatatagca atgcacatgc g	21


<210>	960
<211>	21
<212>	DNA
<213>	Unknown

<220>
<223>	Found in multiple species

<400>	960

ttaggtggtg caaacattgt a	21


<210>	961
<211>	21
<212>	DNA
<213>	Unknown

<220>
<223>	Found in multiple species

<400>	961

ttagggtgag ctctgagagc c	21


<210>	962
<211>	21
<212>	DNA
<213>	Unknown

<220>
<223>	Found in multiple species

<400>	962

ttagggaact tccaccgagt t                                                21

<210>    963
<211>    21
<212>    DNA
<213>    Unknown

<220>
<223>    Found in multiple species

<400>    963

ttagcaaccg cagtatcgac a                                                21

<210>    964
<211>    21
<212>    DNA
<213>    Unknown

<220>
<223>    Found in multiple species

<400>    964

ttacaatgtt tgcaccacct a                                                21

<210>    965
<211>    21
<212>    DNA
<213>    Unknown

<220>
<223>    Found in multiple species

<400>    965

tgtttgcacc acctaaaact c                                                21

<210>    966
<211>    21
<212>    DNA
<213>    Unknown

<220>
<223>    Found in multiple species

<400>    966

tgtgagggat aaccttgatg c                                                21

<210>    967
<211>    21
<212>    DNA
<213>    Unknown

<220>
<223>    Found in multiple species

<400>       967

tgtccaagtc aattggccta g                                                21


<210>       968
<211>       21
<212>       DNA
<213>       Unknown

<220>
<223>       Found in multiple species

<400>       968

tgtcattttc tacatcagac c                                                21


<210>       969
<211>       21
<212>       DNA
<213>       Unknown

<220>
<223>       Found in multiple species

<400>       969

tgtagaaaat gacactattt g                                                21


<210>       970
<211>       21
<212>       DNA
<213>       Unknown

<220>
<223>       Found in multiple species

<400>       970

tgtaagagta cataatgtga g                                                21


<210>       971
<211>       21
<212>       DNA
<213>       Unknown

<220>
<223>       Found in multiple species

<400>       971

tggtgccatc acaaatggtc t                                                21


<210>       972
<211>       21
<212>       DNA
<213>       Unknown

<220>

<223>      Found in multiple species

<400>      972

tggtctttcc catatccttt c                                                21


<210>      973
<211>      21
<212>      DNA
<213>      Unknown

<220>
<223>      Found in multiple species

<400>      973

tggtcctagg ccaattgact t                                                21


<210>      974
<211>      21
<212>      DNA
<213>      Unknown

<220>
<223>      Found in multiple species

<400>      974

tgggtctgat gtagaaaatg a                                                21


<210>      975
<211>      21
<212>      DNA
<213>      Unknown

<220>
<223>      Found in multiple species

<400>      975

tgggattggt ctcataaaac c                                                21


<210>      976
<211>      21
<212>      DNA
<213>      Unknown

<220>
<223>      Found in multiple species

<400>      976

tgggagtctt gaacttgact c                                                21


<210>      977
<211>      21
<212>      DNA
<213>      Unknown

<220>
<223>       Found in multiple species

<400>       977

tgggaaagac catttgtgat g                                                 21


<210>       978
<211>       21
<212>       DNA
<213>       Unknown

<220>
<223>       Found in multiple species

<400>       978

tggcctagga ccatacctta t                                                 21


<210>       979
<211>       21
<212>       DNA
<213>       Unknown

<220>
<223>       Found in multiple species

<400>       979

tggacatact attttatggg a                                                 21


<210>       980
<211>       21
<212>       DNA
<213>       Unknown

<220>
<223>       Found in multiple species

<400>       980

tggaatgaaa agggttttgc c                                                 21


<210>       981
<211>       21
<212>       DNA
<213>       Unknown

<220>
<223>       Found in multiple species

<400>       981

tgctggagtt ttaggtggtg c                                                 21


<210>       982
<211>       21

<212>	DNA
<213>	Unknown

<220>
<223>	Found in multiple species

<400>	982

tgctaaggtt cgtcagatga t	21


<210>	983
<211>	21
<212>	DNA
<213>	Unknown

<220>
<223>	Found in multiple species

<400>	983

tgcggttgct aaggttcgtc a	21


<210>	984
<211>	21
<212>	DNA
<213>	Unknown

<220>
<223>	Found in multiple species

<400>	984

tgccatcaca aatggtcttt c	21


<210>	985
<211>	21
<212>	DNA
<213>	Unknown

<220>
<223>	Found in multiple species

<400>	985

tgcaccacct aaaactccag c	21


<210>	986
<211>	21
<212>	DNA
<213>	Unknown

<220>
<223>	Found in multiple species

<400>	986

tgattgtatt gggtctgatg t	21

<210>	987
<211>	21
<212>	DNA
<213>	Unknown

<220>
<223>	Found in multiple species

<400>	987

tgatgtagaa aatgacacta t	21


<210>	988
<211>	21
<212>	DNA
<213>	Unknown

<220>
<223>	Found in multiple species

<400>	988

tgatagcttt agtgatggcg g	21


<210>	989
<211>	21
<212>	DNA
<213>	Unknown

<220>
<223>	Found in multiple species

<400>	989

tgaggctctc agagctcacc c	21


<210>	990
<211>	21
<212>	DNA
<213>	Unknown

<220>
<223>	Found in multiple species

<400>	990

tgagctctga gagcctcact a	21


<210>	991
<211>	21
<212>	DNA
<213>	Unknown

<220>
<223>	Found in multiple species

<400>	991

tgacttggac atactatttt a	21

<210>	992
<211>	21
<212>	DNA
<213>	Unknown

<220>
<223>	Found in multiple species

<400>	992

tgactcctga tagctttagt g	21


<210>	993
<211>	21
<212>	DNA
<213>	Unknown

<220>
<223>	Found in multiple species

<400>	993

tgaacttgac tcctgatagc t	21


<210>	994
<211>	21
<212>	DNA
<213>	Unknown

<220>
<223>	Found in multiple species

<400>	994

tgaaaagggt tttgcccgtt g	21


<210>	995
<211>	21
<212>	DNA
<213>	Unknown

<220>
<223>	Found in multiple species

<400>	995

tcttgaactt gactcctgat a	21


<210>	996
<211>	21
<212>	DNA
<213>	Unknown

<220>
<223>	Found in multiple species

<400>	996

tctgtcgata ctgcggttgc t                                                21

<210>    997
<211>    21
<212>    DNA
<213>    Unknown

<220>
<223>    Found in multiple species

<400>    997

tctgatgtag aaaatgacac t                                                21

<210>    998
<211>    21
<212>    DNA
<213>    Unknown

<220>
<223>    Found in multiple species

<400>    998

tctgacgaac cttagcaacc g                                                21

<210>    999
<211>    21
<212>    DNA
<213>    Unknown

<220>
<223>    Found in multiple species

<400>    999

tcggtggaag ttccctaatc g                                                21

<210>    1000
<211>    21
<212>    DNA
<213>    Unknown

<220>
<223>    Found in multiple species

<400>    1000

tcgcatgtgc attgctatat a                                                21

<210>    1001
<211>    21
<212>    DNA
<213>    Unknown

<220>
<223>    Found in multiple species

<400>       1001

tcgatactgc ggttgctaag g                                                 21


<210>       1002
<211>       21
<212>       DNA
<213>       Unknown

<220>
<223>       Found in multiple species

<400>       1002

tccttcccga ttagggaact t                                                 21


<210>       1003
<211>       21
<212>       DNA
<213>       Unknown

<220>
<223>       Found in multiple species

<400>       1003

tcctgatagc tttagtgatg g                                                 21


<210>       1004
<211>       21
<212>       DNA
<213>       Unknown

<220>
<223>       Found in multiple species

<400>       1004

tcctaggcca attgacttgg a                                                 21


<210>       1005
<211>       21
<212>       DNA
<213>       Unknown

<220>
<223>       Found in multiple species

<400>       1005

tccgccatca ctaaagctat c                                                 21


<210>       1006
<211>       21
<212>       DNA
<213>       Unknown

<220>
<223>	Found in multiple species

<400>	1006

tccctcacat tatgtactct t	21


<210>	1007
<211>	21
<212>	DNA
<213>	Unknown

<220>
<223>	Found in multiple species

<400>	1007

tccctaatcg ggaaggatgg a	21


<210>	1008
<211>	21
<212>	DNA
<213>	Unknown

<220>
<223>	Found in multiple species

<400>	1008

tcccgattag ggaacttcca c	21


<210>	1009
<211>	21
<212>	DNA
<213>	Unknown

<220>
<223>	Found in multiple species

<400>	1009

tcattccatc cttcccgatt a	21


<210>	1010
<211>	21
<212>	DNA
<213>	Unknown

<220>
<223>	Found in multiple species

<400>	1010

tcaggagtca agttcaagac t	21


<210>	1011
<211>	21
<212>	DNA

<213>      Unknown

<220>
<223>      Found in multiple species

<400>      1011

tcactaaagc tatcaggagt c                                                21


<210>      1012
<211>      21
<212>      DNA
<213>      Unknown

<220>
<223>      Found in multiple species

<400>      1012

tcacaaatgg tctttcccat a                                                21


<210>      1013
<211>      21
<212>      DNA
<213>      Unknown

<220>
<223>      Found in multiple species

<400>      1013

tcaattggcc taggaccata c                                                21


<210>      1014
<211>      21
<212>      DNA
<213>      Unknown

<220>
<223>      Found in multiple species

<400>      1014

tcaatctgtc gatactgcgg t                                                21


<210>      1015
<211>      21
<212>      DNA
<213>      Unknown

<220>
<223>      Found in multiple species

<400>      1015

tcaagttatg tgatgccata c                                                21


<210>      1016

<211>	21
<212>	DNA
<213>	Unknown

<220>
<223>	Found in multiple species

<400>	1016

tcaaggttat ccctcacatt a	21


<210>	1017
<211>	21
<212>	DNA
<213>	Unknown

<220>
<223>	Found in multiple species

<400>	1017

tcaagactcc cattacagag g	21


<210>	1018
<211>	21
<212>	DNA
<213>	Unknown

<220>
<223>	Found in multiple species

<400>	1018

tatgtccaag tcaattggcc t	21


<210>	1019
<211>	21
<212>	DNA
<213>	Unknown

<220>
<223>	Found in multiple species

<400>	1019

tatggtccta ggccaattga c	21


<210>	1020
<211>	21
<212>	DNA
<213>	Unknown

<220>
<223>	Found in multiple species

<400>	1020

tatggcatca cataacttga c	21

<210>	1021
<211>	21
<212>	DNA
<213>	Unknown

<220>
<223>	Found in multiple species

<400>	1021

tatgagacca atcccataag c	21


<210>	1022
<211>	21
<212>	DNA
<213>	Unknown

<220>
<223>	Found in multiple species

<400>	1022

tatcgacaga ttgaaaactt c	21


<210>	1023
<211>	21
<212>	DNA
<213>	Unknown

<220>
<223>	Found in multiple species

<400>	1023

tatccctcac attatgtact c	21


<210>	1024
<211>	21
<212>	DNA
<213>	Unknown

<220>
<223>	Found in multiple species

<400>	1024

tatagcaatg cacatgcgag g	21


<210>	1025
<211>	21
<212>	DNA
<213>	Unknown

<220>
<223>	Found in multiple species

<400>	1025

tagtgtcatt ttctacatca g 21

<210> 1026
<211> 21
<212> DNA
<213> Unknown

<220>
<223> Found in multiple species

<400> 1026

tagtatgtcc aagtcaattg g 21

<210> 1027
<211> 21
<212> DNA
<213> Unknown

<220>
<223> Found in multiple species

<400> 1027

tagggtgagc tctgagagcc t 21

<210> 1028
<211> 21
<212> DNA
<213> Unknown

<220>
<223> Found in multiple species

<400> 1028

tagggaactt ccaccgagtt t 21

<210> 1029
<211> 21
<212> DNA
<213> Unknown

<220>
<223> Found in multiple species

<400> 1029

taggccaatt gacttggaca t 21

<210> 1030
<211> 21
<212> DNA
<213> Unknown

<220>
<223> Found in multiple species

<400>       1030

taggaccata ccttattccc t                                                21


<210>       1031
<211>       21
<212>       DNA
<213>       Unknown

<220>
<223>       Found in multiple species

<400>       1031

tagctttagt gatggcggaa g                                                21


<210>       1032
<211>       21
<212>       DNA
<213>       Unknown

<220>
<223>       Found in multiple species

<400>       1032

tagcaaccgc agtatcgaca g                                                21


<210>       1033
<211>       21
<212>       DNA
<213>       Unknown

<220>
<223>       Found in multiple species

<400>       1033

tagaaaatga cactatttgt a                                                21


<210>       1034
<211>       21
<212>       DNA
<213>       Unknown

<220>
<223>       Found in multiple species

<400>       1034

tactgcggtt gctaaggttc g                                                21


<210>       1035
<211>       21
<212>       DNA
<213>       Unknown

<220>

<223>	Found in multiple species

<400>	1035

tacatcagac ccaatacaat c	21


<210>	1036
<211>	21
<212>	DNA
<213>	Unknown

<220>
<223>	Found in multiple species

<400>	1036

tacataatgt gagggataac c	21


<210>	1037
<211>	21
<212>	DNA
<213>	Unknown

<220>
<223>	Found in multiple species

<400>	1037

taaggttcgt cagatgatct c	21


<210>	1038
<211>	21
<212>	DNA
<213>	Unknown

<220>
<223>	Found in multiple species

<400>	1038

taagagtaca taatgtgagg g	21


<210>	1039
<211>	21
<212>	DNA
<213>	Unknown

<220>
<223>	Found in multiple species

<400>	1039

taaagctatc aggagtcaag t	21


<210>	1040
<211>	21
<212>	DNA
<213>	Unknown

<220>
<223>	Found in multiple species

<400>	1040

taaactcggt ggaagttccc t	21


<210>	1041
<211>	21
<212>	DNA
<213>	Unknown

<220>
<223>	Found in multiple species

<400>	1041

gttttcaatc tgtcgatact g	21


<210>	1042
<211>	21
<212>	DNA
<213>	Unknown

<220>
<223>	Found in multiple species

<400>	1042

gttttatgag accaatccca t	21


<210>	1043
<211>	21
<212>	DNA
<213>	Unknown

<220>
<223>	Found in multiple species

<400>	1043

gttttaggtg gtgcaaacat t	21


<210>	1044
<211>	21
<212>	DNA
<213>	Unknown

<220>
<223>	Found in multiple species

<400>	1044

gtttgcacca cctaaaactc c	21


<210>	1045
<211>	21

<212>	DNA
<213>	Unknown

<220>
<223>	Found in multiple species

<400>	1045

gttgctaagg ttcgtcagat g	21


<210>	1046
<211>	21
<212>	DNA
<213>	Unknown

<220>
<223>	Found in multiple species

<400>	1046

gttccctaat cgggaaggat g	21


<210>	1047
<211>	21
<212>	DNA
<213>	Unknown

<220>
<223>	Found in multiple species

<400>	1047

gttatccctc acattatgta c	21


<210>	1048
<211>	21
<212>	DNA
<213>	Unknown

<220>
<223>	Found in multiple species

<400>	1048

gtgtcatttt ctacatcaga c	21


<210>	1049
<211>	21
<212>	DNA
<213>	Unknown

<220>
<223>	Found in multiple species

<400>	1049

gtggaatctt tgaagcatgg g	21

<210>	1050
<211>	21
<212>	DNA
<213>	Unknown

<220>
<223>	Found in multiple species

<400>	1050

gtgccatcac aaatggtctt t	21


<210>	1051
<211>	21
<212>	DNA
<213>	Unknown

<220>
<223>	Found in multiple species

<400>	1051

gtgaggctct cagagctcac c	21


<210>	1052
<211>	21
<212>	DNA
<213>	Unknown

<220>
<223>	Found in multiple species

<400>	1052

gtgagctctg agagcctcac t	21


<210>	1053
<211>	21
<212>	DNA
<213>	Unknown

<220>
<223>	Found in multiple species

<400>	1053

gtcttgaact tgactcctga t	21


<210>	1054
<211>	21
<212>	DNA
<213>	Unknown

<220>
<223>	Found in multiple species

<400>	1054

gtcgatactg cggttgctaa g	21

<210>	1055
<211>	21
<212>	DNA
<213>	Unknown

<220>
<223>	Found in multiple species

<400>	1055

gtcctaggcc aattgacttg g	21


<210>	1056
<211>	21
<212>	DNA
<213>	Unknown

<220>
<223>	Found in multiple species

<400>	1056

gtccaagtca attggcctag g	21


<210>	1057
<211>	21
<212>	DNA
<213>	Unknown

<220>
<223>	Found in multiple species

<400>	1057

gtcattttct acatcagacc c	21


<210>	1058
<211>	21
<212>	DNA
<213>	Unknown

<220>
<223>	Found in multiple species

<400>	1058

gtatgtccaa gtcaattggc c	21


<210>	1059
<211>	21
<212>	DNA
<213>	Unknown

<220>
<223>	Found in multiple species

<400>	1059

gtatcgacag attgaaaact t                                                21

<210>    1060
<211>    21
<212>    DNA
<213>    Unknown

<220>
<223>    Found in multiple species

<400>    1060

gtagaaaatg acactatttg t                                                21

<210>    1061
<211>    21
<212>    DNA
<213>    Unknown

<220>
<223>    Found in multiple species

<400>    1061

gtacaaatag tgtcattttc t                                                21

<210>    1062
<211>    21
<212>    DNA
<213>    Unknown

<220>
<223>    Found in multiple species

<400>    1062

gtaagagtac ataatgtgag g                                                21

<210>    1063
<211>    21
<212>    DNA
<213>    Unknown

<220>
<223>    Found in multiple species

<400>    1063

ggttgctaag gttcgtcaga t                                                21

<210>    1064
<211>    21
<212>    DNA
<213>    Unknown

<220>
<223>    Found in multiple species

<400>       1064

ggtggaatct ttgaagcatg g                                                 21


<210>       1065
<211>       21
<212>       DNA
<213>       Unknown

<220>
<223>       Found in multiple species

<400>       1065

ggtggaagtt ccctaatcgg g                                                 21


<210>       1066
<211>       21
<212>       DNA
<213>       Unknown

<220>
<223>       Found in multiple species

<400>       1066

ggtgccatca caaatggtct t                                                 21


<210>       1067
<211>       21
<212>       DNA
<213>       Unknown

<220>
<223>       Found in multiple species

<400>       1067

ggtcctaggc caattgactt g                                                 21


<210>       1068
<211>       21
<212>       DNA
<213>       Unknown

<220>
<223>       Found in multiple species

<400>       1068

ggtatggtcc taggccaatt g                                                 21


<210>       1069
<211>       21
<212>       DNA
<213>       Unknown

<220>
<223>	Found in multiple species

<400>	1069

gggtggaatc tttgaagcat g	21


<210>	1070
<211>	21
<212>	DNA
<213>	Unknown

<220>
<223>	Found in multiple species

<400>	1070

gggcaaaacc cttttcattc c	21


<210>	1071
<211>	21
<212>	DNA
<213>	Unknown

<220>
<223>	Found in multiple species

<400>	1071

gggattggtc tcataaaacc t	21


<210>	1072
<211>	21
<212>	DNA
<213>	Unknown

<220>
<223>	Found in multiple species

<400>	1072

gggagtcttg aacttgactc c	21


<210>	1073
<211>	21
<212>	DNA
<213>	Unknown

<220>
<223>	Found in multiple species

<400>	1073

gggaataagg tatggtccta g	21


<210>	1074
<211>	21
<212>	DNA

<213>      Unknown

<220>
<223>      Found in multiple species

<400>      1074

gggaaggatg gaatgaaaag g                                                21


<210>      1075
<211>      21
<212>      DNA
<213>      Unknown

<220>
<223>      Found in multiple species

<400>      1075

gggaaagacc atttgtgatg g                                                21


<210>      1076
<211>      21
<212>      DNA
<213>      Unknown

<220>
<223>      Found in multiple species

<400>      1076
   ggcctaggac cataccttat t                                               21


<210>      1077
<211>      21
<212>      DNA
<213>      Unknown

<220>
<223>      Found in multiple species

<400>      1077

ggattggtct cataaaacct c                                                21


<210>      1078
<211>      21
<212>      DNA
<213>      Unknown

<220>
<223>      Found in multiple species

<400>      1078

ggatggaatg aaaagggttt t                                                21


<210>      1079

<211>	21
<212>	DNA
<213>	Unknown

<220>
<223>	Found in multiple species

<400>	1079

ggatatggga aagaccattt g	21


<210>	1080
<211>	21
<212>	DNA
<213>	Unknown

<220>
<223>	Found in multiple species

<400>	1080

ggagtcttga acttgactcc t	21


<210>	1081
<211>	21
<212>	DNA
<213>	Unknown

<220>
<223>	Found in multiple species

<400>	1081

ggaataaggt atggtcctag g	21


<210>	1082
<211>	21
<212>	DNA
<213>	Unknown

<220>
<223>	Found in multiple species

<400>	1082

ggaagttttc aatctgtcga t	21


<210>	1083
<211>	21
<212>	DNA
<213>	Unknown

<220>
<223>	Found in multiple species

<400>	1083

ggaagttccc taatcgggaa g	21

<210>	1084
<211>	21
<212>	DNA
<213>	Unknown

<220>
<223>	Found in multiple species

<400>	1084

ggaaggatgg aatgaaaagg g	21


<210>	1085
<211>	21
<212>	DNA
<213>	Unknown

<220>
<223>	Found in multiple species

<400>	1085

ggaaagacca tttgtgatgg c	21


<210>	1086
<211>	21
<212>	DNA
<213>	Unknown

<220>
<223>	Found in multiple species

<400>	1086

gctaaggttc gtcagatgat c	21


<210>	1087
<211>	21
<212>	DNA
<213>	Unknown

<220>
<223>	Found in multiple species

<400>	1087

gcggttgcta aggttcgtca g	21


<210>	1088
<211>	21
<212>	DNA
<213>	Unknown

<220>
<223>	Found in multiple species

<400>	1088

gcctaggacc ataccttatt c                                                21

<210>    1089
<211>    21
<212>    DNA
<213>    Unknown

<220>
<223>    Found in multiple species

<400>    1089

gccatcacta aagctatcag g                                                21

<210>    1090
<211>    21
<212>    DNA
<213>    Unknown

<220>
<223>    Found in multiple species

<400>    1090

gccaattgac ttggacatac t                                                21

<210>    1091
<211>    21
<212>    DNA
<213>    Unknown

<220>
<223>    Found in multiple species

<400>    1091

gcatgtgcat tgctatataa g                                                21

<210>    1092
<211>    21
<212>    DNA
<213>    Unknown

<220>
<223>    Found in multiple species

<400>    1092

gcaaccgcag tatcgacaga t                                                21

<210>    1093
<211>    21
<212>    DNA
<213>    Unknown

<220>
<223>    Found in multiple species

<400>       1093

gcaaaaccct tttcattcca t                                                21


<210>       1094
<211>       21
<212>       DNA
<213>       Unknown

<220>
<223>       Found in multiple species

<400>       1094

gattggtctc ataaaacctc t                                                21


<210>       1095
<211>       21
<212>       DNA
<213>       Unknown

<220>
<223>       Found in multiple species

<400>       1095

gattagggaa cttccaccga g                                                21


<210>       1096
<211>       21
<212>       DNA
<213>       Unknown

<220>
<223>       Found in multiple species

<400>       1096

gatgtagaaa atgacactat t                                                21


<210>       1097
<211>       21
<212>       DNA
<213>       Unknown

<220>
<223>       Found in multiple species

<400>       1097

gatggaatga aaagggtttt g                                                21


<210>       1098
<211>       21
<212>       DNA
<213>       Unknown

<220>

<223>       Found in multiple species

<400>       1098

gatatgggaa agaccatttg t                                                 21


<210>       1099
<211>       21
<212>       DNA
<213>       Unknown

<220>
<223>       Found in multiple species

<400>       1099

gatactgcgg ttgctaaggt t                                                 21


<210>       1100
<211>       21
<212>       DNA
<213>       Unknown

<220>
<223>       Found in multiple species

<400>       1100

gagtcttgaa cttgactcct g                                                 21


<210>       1101
<211>       21
<212>       DNA
<213>       Unknown

<220>
<223>       Found in multiple species

<400>       1101

gagtatggca tcacataact t                                                 21


<210>       1102
<211>       21
<212>       DNA
<213>       Unknown

<220>
<223>       Found in multiple species

<400>       1102

gaggctctca gagctcaccc t                                                 21


<210>       1103
<211>       21
<212>       DNA
<213>       Unknown

<220>
<223>	Found in multiple species

<400>	1103

gagctctgag agcctcacta t	21


<210>	1104
<211>	21
<212>	DNA
<213>	Unknown

<220>
<223>	Found in multiple species

<400>	1104

gacttggaca tactatttta t	21


<210>	1105
<211>	21
<212>	DNA
<213>	Unknown

<220>
<223>	Found in multiple species

<400>	1105

gactcccatt acagaggttt t	21


<210>	1106
<211>	21
<212>	DNA
<213>	Unknown

<220>
<223>	Found in multiple species

<400>	1106

gacgaacctt agcaaccgca g	21


<210>	1107
<211>	21
<212>	DNA
<213>	Unknown

<220>
<223>	Found in multiple species

<400>	1107

gaccatttgt gatggcacca t	21


<210>	1108
<211>	21

<212>	DNA
<213>	Unknown

<220>
<223>	Found in multiple species

<400>	1108

gaatgaaaag ggttttgccc g	21


<210>	1109
<211>	21
<212>	DNA
<213>	Unknown

<220>
<223>	Found in multiple species

<400>	1109

gaagttccct aatcgggaag g	21


<210>	1110
<211>	21
<212>	DNA
<213>	Unknown

<220>
<223>	Found in multiple species

<400>	1110

gaaggatgga atgaaaaggg t	21


<210>	1111
<211>	21
<212>	DNA
<213>	Unknown

<220>
<223>	Found in multiple species

<400>	1111

gaacttgact cctgatagct t	21


<210>	1112
<211>	21
<212>	DNA
<213>	Unknown

<220>
<223>	Found in multiple species

<400>	1112

gaaccttagc aaccgcagta t	21

<210> 1113
<211> 21
<212> DNA
<213> Unknown

<220>
<223> Found in multiple species

<400> 1113

gaaaagggtt ttgcccgttg g 21


<210> 1114
<211> 21
<212> DNA
<213> Unknown

<220>
<223> Found in multiple species

<400> 1114

cttttcattc catccttccc g 21


<210> 1115
<211> 21
<212> DNA
<213> Unknown

<220>
<223> Found in multiple species

<400> 1115

ctttcccata tcctttcatg g 21


<210> 1116
<211> 21
<212> DNA
<213> Unknown

<220>
<223> Found in multiple species

<400> 1116

cttggacata ctattttatg g 21


<210> 1117
<211> 21
<212> DNA
<213> Unknown

<220>
<223> Found in multiple species

<400> 1117

cttgactcct gatagcttta g 21

<210>	1118
<211>	21
<212>	DNA
<213>	Unknown

<220>
<223>	Found in multiple species

<400>	1118

cttgaacttg actcctgata g	21


<210>	1119
<211>	21
<212>	DNA
<213>	Unknown

<220>
<223>	Found in multiple species

<400>	1119

cttacaatgt ttgcaccacc t	21


<210>	1120
<211>	21
<212>	DNA
<213>	Unknown

<220>
<223>	Found in multiple species

<400>	1120

ctgctggagt tttaggtggt g	21


<210>	1121
<211>	21
<212>	DNA
<213>	Unknown

<220>
<223>	Found in multiple species

<400>	1121

ctgatagctt tagtgatggc g	21


<210>	1122
<211>	21
<212>	DNA
<213>	Unknown

<220>
<223>	Found in multiple species

<400>	1122

ctcgcatgtg cattgctata t                                                21

<210>    1123
<211>    21
<212>    DNA
<213>    Unknown

<220>
<223>    Found in multiple species

<400>    1123

ctcctgatag ctttagtgat g                                                21

<210>    1124
<211>    21
<212>    DNA
<213>    Unknown

<220>
<223>    Found in multiple species

<400>    1124

ctacatcaga cccaatacaa t                                                21

<210>    1125
<211>    21
<212>    DNA
<213>    Unknown

<220>
<223>    Found in multiple species

<400>    1125

ctaatcggga aggatggaat g                                                21

<210>    1126
<211>    21
<212>    DNA
<213>    Unknown

<220>
<223>    Found in multiple species

<400>    1126

ctaaggttcg tcagatgatc t                                                21

<210>    1127
<211>    21
<212>    DNA
<213>    Unknown

<220>
<223>    Found in multiple species

<400>       1127

cggtggaagt tccctaatcg g                                                21


<210>       1128
<211>       21
<212>       DNA
<213>       Unknown

<220>
<223>       Found in multiple species

<400>       1128

cgcttatggg attggtctca t                                                21


<210>       1129
<211>       21
<212>       DNA
<213>       Unknown

<220>
<223>       Found in multiple species

<400>       1129

cgatactgcg gttgctaagg t                                                21


<210>       1130
<211>       21
<212>       DNA
<213>       Unknown

<220>
<223>       Found in multiple species

<400>       1130

ccttatatag caatgcacat g                                                21


<210>       1131
<211>       21
<212>       DNA
<213>       Unknown

<220>
<223>       Found in multiple species

<400>       1131

cctaatcggg aaggatggaa t                                                21


<210>       1132
<211>       21
<212>       DNA
<213>       Unknown

<220>
<223>       Found in multiple species

<400>       1132

catcaaggtt atccctcaca t                                                21


<210>       1133
<211>       21
<212>       DNA
<213>       Unknown

<220>
<223>       Found in multiple species

<400>       1133

cataatgtga gggataacct t                                                21


<210>       1134
<211>       21
<212>       DNA
<213>       Unknown

<220>
<223>       Found in multiple species

<400>       1134

cataaaatag tatgtccaag t                                                21


<210>       1135
<211>       21
<212>       DNA
<213>       Unknown

<220>
<223>       Found in multiple species

<400>       1135

cagaggtttt atgagaccaa t                                                21


<210>       1136
<211>       21
<212>       DNA
<213>       Unknown

<220>
<223>       Found in multiple species

<400>       1136

cacattatgt actcttacaa t                                                21


<210>       1137
<211>       21
<212>       DNA

<213>	Unknown

<220>
<223>	Found in multiple species

<400>	1137

cacaaatggt ctttcccata t	21


<210>	1138
<211>	21
<212>	DNA
<213>	Unknown

<220>
<223>	Found in multiple species

<400>	1138

caattgactt ggacatacta t	21


<210>	1139
<211>	21
<212>	DNA
<213>	Unknown

<220>
<223>	Found in multiple species

<400>	1139

caatctgtcg atactgcggt t	21


<210>	1140
<211>	21
<212>	DNA
<213>	Unknown

<220>
<223>	Found in multiple species

<400>	1140

caagttatgt gatgccatac t	21


<210>	1141
<211>	21
<212>	DNA
<213>	Unknown

<220>
<223>	Found in multiple species

<400>	1141

caaggttatc cctcacatta t	21


<210>	1142

<211>	21
<212>	DNA
<213>	Unknown

<220>
<223>	Found in multiple species

<400>	1142

caagactccc attacagagg t	21


<210>	1143
<211>	21
<212>	DNA
<213>	Unknown

<220>
<223>	Found in multiple species

<400>	1143

caaccgcagt atcgacagat t	21


<210>	1144
<211>	21
<212>	DNA
<213>	Unknown

<220>
<223>	Found in multiple species

<400>	1144

attttctaca tcagacccaa t	21


<210>	1145
<211>	21
<212>	DNA
<213>	Unknown

<220>
<223>	Found in multiple species

<400>	1145

attggcctag gaccatacct t	21


<210>	1146
<211>	21
<212>	DNA
<213>	Unknown

<220>
<223>	Found in multiple species

<400>	1146

attgattgta ttgggtctga t	21

<210>	1147
<211>	21
<212>	DNA
<213>	Unknown

<220>
<223>	Found in multiple species

<400>	1147

attgacttgg acatactatt t	21


<210>	1148
<211>	21
<212>	DNA
<213>	Unknown

<220>
<223>	Found in multiple species

<400>	1148

attagggaac ttccaccgag t	21


<210>	1149
<211>	21
<212>	DNA
<213>	Unknown

<220>
<223>	Found in multiple species

<400>	1149

atgtttgcac cacctaaaac t	21


<210>	1150
<211>	21
<212>	DNA
<213>	Unknown

<220>
<223>	Found in multiple species

<400>	1150

atgtagaaaa tgacactatt t	21


<210>	1151
<211>	21
<212>	DNA
<213>	Unknown

<220>
<223>	Found in multiple species

<400>	1151

atggtctttc ccatatcctt t                                                21

<210>   1152
<211>   21
<212>   DNA
<213>   Unknown

<220>
<223>   Found in multiple species

<400>   1152

atggtcctag gccaattgac t                                                21

<210>   1153
<211>   21
<212>   DNA
<213>   Unknown

<220>
<223>   Found in multiple species

<400>   1153

atgggagtct tgaacttgac t                                                21

<210>   1154
<211>   21
<212>   DNA
<213>   Unknown

<220>
<223>   Found in multiple species

<400>   1154

atgggaaaga ccatttgtga t                                                21

<210>   1155
<211>   21
<212>   DNA
<213>   Unknown

<220>
<223>   Found in multiple species

<400>   1155

atgaaaaggg ttttgcccgt t                                                21

<210>   1156
<211>   21
<212>   DNA
<213>   Unknown

<220>
<223>   Found in multiple species

<400>       1156

atccttcccg attagggaac t                                                21


<210>       1157
<211>       21
<212>       DNA
<213>       Unknown

<220>
<223>       Found in multiple species

<400>       1157

atccctcaca ttatgtactc t                                                21


<210>       1158
<211>       21
<212>       DNA
<213>       Unknown

<220>
<223>       Found in multiple species

<400>       1158

atcactaaag ctatcaggag t                                                21


<210>       1159
<211>       21
<212>       DNA
<213>       Unknown

<220>
<223>       Found in multiple species

<400>       1159

atcaaggtta tccctcacat t                                                21


<210>       1160
<211>       21
<212>       DNA
<213>       Unknown

<220>
<223>       Found in multiple species

<400>       1160

agttttcaat ctgtcgatac t                                                21


<210>       1161
<211>       21
<212>       DNA
<213>       Unknown

<220>

<223>       Found in multiple species

<400>       1161

aggttatccc tcacattatg t                                                21


<210>       1162
<211>       21
<212>       DNA
<213>       Unknown

<220>
<223>       Found in multiple species

<400>       1162

aggtatggtc ctaggccaat t                                                21


<210>       1163
<211>       21
<212>       DNA
<213>       Unknown

<220>
<223>       Found in multiple species

<400>       1163

aggatggaat gaaaagggtt t                                                21


<210>       1164
<211>       21
<212>       DNA
<213>       Unknown

<220>
<223>       Found in multiple species

<400>       1164

aggatatggg aaagaccatt t                                                21


<210>       1165
<211>       21
<212>       DNA
<213>       Unknown

<220>
<223>       Found in multiple species

<400>       1165

aggaccatac cttattccct t                                                21


<210>       1166
<211>       21
<212>       DNA
<213>       Unknown

<220>
<223>	Found in multiple species

<400>	1166

agactcccat tacagaggtt t	21


<210>	1167
<211>	21
<212>	DNA
<213>	Unknown

<220>
<223>	Found in multiple species

<400>	1167

actgctggag ttttaggtgg t	21


<210>	1168
<211>	21
<212>	DNA
<213>	Unknown

<220>
<223>	Found in multiple species

<400>	1168

actgcggttg ctaaggttcg t	21


<210>	1169
<211>	21
<212>	DNA
<213>	Unknown

<220>
<223>	Found in multiple species

<400>	1169
   acgggcaaaa cccttttcat t	21


<210>	1170
<211>	21
<212>	DNA
<213>	Unknown

<220>
<223>	Found in multiple species

<400>	1170

accatttgtg atggcaccat t	21


<210>	1171
<211>	21

<212>	DNA
<213>	Unknown

<220>
<223>	Found in multiple species

<400>	1171

aattgacttg gacatactat t	21


<210>	1172
<211>	21
<212>	DNA
<213>	Unknown

<220>
<223>	Found in multiple species

<400>	1172

aatggtcttt cccatatcct t	21


<210>	1173
<211>	21
<212>	DNA
<213>	Unknown

<220>
<223>	Found in multiple species

<400>	1173

aaggatggaa tgaaaagggt t	21


<210>	1174
<211>	21
<212>	DNA
<213>	Unknown

<220>
<223>	Found in multiple species

<400>	1174

aagactccca ttacagaggt t	21


<210>	1175
<211>	21
<212>	DNA
<213>	Unknown

<220>
<223>	Found in multiple species

<400>	1175

aacttgactc ctgatagctt t	21

<210>	1176
<211>	25
<212>	DNA
<213>	Amaranthus palmeri

<400>	1176

ttttattcta aagttgcttc ggagg	25

<210>	1177
<211>	25
<212>	DNA
<213>	Amaranthus palmeri

<400>	1177

ccgtcaagaa gggggcacac attgt	25

<210>	1178
<211>	25
<212>	DNA
<213>	Amaranthus palmeri

<400>	1178

gaatgatgtc tctagtggga aactc	25

<210>	1179
<211>	25
<212>	DNA
<213>	Amaranthus palmeri

<400>	1179

gattccgaga tgtttaatgt tgttg	25

<210>	1180
<211>	25
<212>	DNA
<213>	Amaranthus palmeri

<400>	1180

cggaccttaa agttccttat atagc	25

<210>	1181
<211>	25
<212>	DNA
<213>	Amaranthus palmeri

<400>	1181

aatgcacatg cgaggagatc cgact	25

<210>	1182
<211>	25
<212>	DNA

<213>       Amaranthus palmeri

<400>       1182

tcaatgcaaa actctgagaa cttga                                             25


<210>       1183
<211>       25
<212>       DNA
<213>       Amaranthus palmeri

<400>       1183

cctacaatga tgtttgtaag caagt                                             25


<210>       1184
<211>       25
<212>       DNA
<213>       Amaranthus palmeri

<400>       1184

ggcttcggag ttgagttcta gggtc                                             25


<210>       1185
<211>       25
<212>       DNA
<213>       Amaranthus palmeri

<400>       1185

atagatgcag aattatcggg aattc                                             25


<210>       1186
<211>       25
<212>       DNA
<213>       Amaranthus palmeri

<400>       1186

ctgcttggag gatagttatt gatcc                                             25


<210>       1187
<211>       25
<212>       DNA
<213>       Amaranthus palmeri

<400>       1187

cggcatcgga ttttctaaga atacg                                             25


<210>       1188
<211>       25
<212>       DNA
<213>       Amaranthus palmeri

<400>       1188

aatcaaaatt tggaaattct tagtg 25

<210> 1189
<211> 25
<212> DNA
<213> Amaranthus palmeri

<400> 1189

gtttacaaaa gatacgggaa gagat 25

<210> 1190
<211> 25
<212> DNA
<213> Amaranthus palmeri

<400> 1190

agctaagaag agtttggcgg tggct 25

<210> 1191
<211> 25
<212> DNA
<213> Amaranthus palmeri

<400> 1191

cattgcccct tgctaattgg acctt 25

<210> 1192
<211> 25
<212> DNA
<213> Amaranthus palmeri

<400> 1192

caagaaagag gtttctgggc gagat 25

<210> 1193
<211> 25
<212> DNA
<213> Amaranthus palmeri

<400> 1193

ttgtgagcgc cctgtagcag ctgac 25

<210> 1194
<211> 25
<212> DNA
<213> Amaranthus palmeri

<400> 1194

agggatcctg ctaccattgc ttcta 25

<210>	1195
<211>	25
<212>	DNA
<213>	Amaranthus palmeri

<400>	1195

taactgctgg agttttaggt ggtgc	25

<210>	1196
<211>	25
<212>	DNA
<213>	Amaranthus palmeri

<400>	1196

aaacattgta agagtacata atgtt	25

<210>	1197
<211>	25
<212>	DNA
<213>	Amaranthus palmeri

<400>	1197

agggataacc ttgatgctgt caagt	25

<210>	1198
<211>	25
<212>	DNA
<213>	Amaranthus palmeri

<400>	1198

tatgtgatgc catactcgga aaaac	25

<210>	1199
<211>	25
<212>	DNA
<213>	Amaranthus palmeri

<400>	1199

tgattaactg cttgtttgta ccacc	25

<210>	1200
<211>	25
<212>	DNA
<213>	Amaranthus palmeri

<400>	1200

ttgtgaatga tgtctagtgg aaggt	25

<210>	1201
<211>	25

<212>	DNA
<213>	Amaranthus palmeri

<400>	1201

tcgattggga ttatgatgaa gtccg	25


<210>	1202
<211>	25
<212>	DNA
<213>	Amaranthus palmeri

<400>	1202

ttagtttgct cgaaagtaga gcttt	25


<210>	1203
<211>	25
<212>	DNA
<213>	Amaranthus palmeri

<400>	1203

agcccgtaag gctatagttc ttaca	25


<210>	1204
<211>	25
<212>	DNA
<213>	Amaranthus palmeri

<400>	1204

gatgtagaat cattggctat ttgcc	25


<210>	1205
<211>	25
<212>	DNA
<213>	Amaranthus palmeri

<400>	1205

tatttctgct tagaagatca tagca	25


<210>	1206
<211>	25
<212>	DNA
<213>	Amaranthus palmeri

<400>	1206

ttgatccaga ctttggattc catgt	25


<210>	1207
<211>	25
<212>	DNA
<213>	Amaranthus palmeri

<400>       1207

agaattatgt tgcgactcat ggtcg                                              25


<210>       1208
<211>       25
<212>       DNA
<213>       Amaranthus palmeri

<400>       1208

aattggccct ttgaggaatt gttga                                              25


<210>       1209
<211>       25
<212>       DNA
<213>       Amaranthus palmeri

<400>       1209

agttccttga gtctatggtt caatc                                              25


<210>       1210
<211>       25
<212>       DNA
<213>       Amaranthus palmeri

<400>       1210

ggctcggttc gatgaaggtt ggact                                              25


<210>       1211
<211>       25
<212>       DNA
<213>       Amaranthus palmeri

<400>       1211

gtgtcgagac aagatgggtc aaaga                                              25


<210>       1212
<211>       25
<212>       DNA
<213>       Amaranthus palmeri

<400>       1212

gaccaatgga caagaagtcg acctt                                              25


<210>       1213
<211>       25
<212>       DNA
<213>       Amaranthus palmeri

<400>       1213

gggcaagctt gatcgagtta cttgg                                              25

<210>	1214
<211>	25
<212>	DNA
<213>	Amaranthus palmeri

<400>	1214

acaagcagat aacagcaggc tctgg	25

<210>	1215
<211>	25
<212>	DNA
<213>	Amaranthus palmeri

<400>	1215

cgaccattag acatacatat cattt	25

<210>	1216
<211>	25
<212>	DNA
<213>	Amaranthus palmeri

<400>	1216

gtttttgaa gtcagatctg caatc	25

<210>	1217
<211>	25
<212>	DNA
<213>	Amaranthus palmeri

<400>	1217

aatcagttgc aatggacaaa ccata	25

<210>	1218
<211>	25
<212>	DNA
<213>	Amaranthus palmeri

<400>	1218

tacggttatt agttgttcct gtcac	25

<210>	1219
<211>	25
<212>	DNA
<213>	Amaranthus palmeri

<400>	1219

tgtttgagtt gaattagatc atgca	25

<210>	1220

<211>	25
<212>	DNA
<213>	Amaranthus palmeri

<400>	1220

aacattgtaa gaatcttgat catgt	25

<210>	1221
<211>	25
<212>	DNA
<213>	Amaranthus palmeri

<400>	1221

gaagctatgt gatactgtac ttctc	25

<210>	1222
<211>	25
<212>	DNA
<213>	Amaranthus palmeri

<400>	1222

aaaatgaaat ctttgatatg atgtt	25

## Description

This application claims benefit under 35USC § 119(e) of United States provisional application serial no. 61/534,097 filed 09/13/2011, herein incorporated by reference in it's entirety. The sequence listing that is contained in the file named "40_21(58641)B seq listing.txt", which is 513,100 bytes (measured in operating system MS-Windows) and was created on 4 Sept 2012, is filed herewith and incorporated herein by reference.

Table 1 is provided herewith as a part of this U.S. patent application via the USPTO's EFS system in file named "40_21(58641)Btable 1.doxc " which is 39,989 bytes in size (measured in MS-Windows®). Table 1 (file "40_21(58641)Btable1.doxc" comprises 54 sequences and is herein incorporated by reference in its entirety.

Table 2 is provided herewith as a part of this U.S. patent application via the USPTO's EFS system in file named "40_21(58641)Btable2.doxc" which is 107,841 bytes in size (measured in MS-Windows®). Table 2 (file "40_21(58641)Btable2.doxc" comprises 848 sequences and is herein incorporated by reference in its entirety.

Table 3 is provided herewith as a part of this U.S. patent application via the USPTO's EFS system in file named "40_21(58641)Btable3.doxc" which is 21,718 bytes in size (measured in MS-Windows®). Table 3 (file "40_21(58641)Btable3.doxc" comprises 269 sequences and is herein incorporated by reference in its entirety.

### FIELD

The methods and compositions generally relate to the field of weed management. More specifically, relate to 7,8-dihydropteroate synthase inhibitors (DHPS) genes in plants and compositions containing polynucleotide molecules for modulating and/or regulating their expression. Further provided are methods and compositions useful for weed control.

### BACKGROUND

Weeds are plants that compete with cultivated plants in an agronomic environment and cost farmers billions of dollars annually in crop losses and the expense of efforts to keep weeds under control. Weeds also serve as hosts for crop diseases and insect pests. The losses caused by weeds in agricultural production environments include decreases in crop yield, reduced crop quality, increased irrigation costs, increased harvesting costs, reduced land value, injury to livestock, and crop damage from insects and diseases harbored by the weeds. The principal means by which weeds cause these effects are: 1) competing with crop plants for water, nutrients, sunlight and other essentials for growth and development, 2) production of toxic or irritant chemicals that cause human or animal health problem, 3) production of immense quantities of seed or vegetative reproductive parts or both that contaminate agricultural products and perpetuate the species in agricultural lands, and 4) production on agricultural and nonagricultural lands of vast amounts of vegetation that must be disposed of. Herbicide tolerant weeds are a problem with nearly all herbicides in use, there is a need to effectively manage these weeds. There are over 365 weed biotypes currently identified as being herbicide resistant to one or more herbicides by the Herbicide Resistance Action Committee (HRAC), the North American Herbicide Resistance Action Committee (NAHRAC), and the Weed Science Society of America (WSSA).

The 7,8-dihydropteroate synthase inhibitors (DHPS) is an enzyme involved in folic acid synthesis which is needed for purine nucleotide biosynthesis. This enzyme is the target of herbicides that include the carbamate chemical family.

### BRIEF DESCRIPTION OF THE FIGURES

The following drawings form part of the present specification and are included to further demonstrate certain methods, compositions or results. They may be better understood by reference to one or more of these drawings in combination with the detailed description of specific embodiments presented herein. The invention can be more fully understood from the following description of the figures:
FIGURE 1. Treatment with oligonucleotide pools followed by Prowl (pendimethalin)

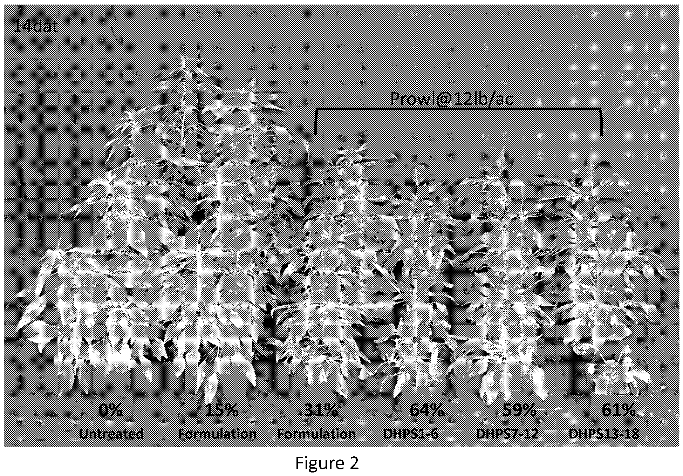

FIGURE 2. Treatment of Palmer Amaranth with 3 oligonucleotide pools followed by Prowl herbicide at 121b/ac rate

### SUMMARY

In one aspect, the invention provides a method of plant control comprising an external application to a plant or plant part a composition comprising a polynucleotide and a transfer agent, wherein the polynucleotide is essentially identical or essentially complementary to a DHPS (dihydropteroate synthase, DHP or DHPS) gene sequence or fragment thereof, or to the RNA transcript of said DHPS gene sequence or fragment thereof, wherein said DHPS gene sequence is selected from the group consisting of SEQ ID NO:1-54 or a polynucleotide fragment thereof. As a result of such application, the plant growth or development or reproductive ability is reduced or the plant is made more sensitive to a DHPS inhibitor herbicide relative to a plant not treated with said composition. In this manner, plants that have become resistant to the application of DHPS inhibitor containining herbicides are made more susceptible to the herbicidal effects the herbicides, thus potentiating the effect of the herbicides. The polynucleotide fragment is at least 18 contiguous nucleotides, at least 19 contiguous nucleotides, at least 20 contiguous nucleotides or at least 21 contiguous nucleotides in length and at least 85 percent identical to a DHPS gene sequence selected from the group consisting of SEQ ID NO:1-54 and the transfer agent comprises an organosilicone composition or compound. The polynucleotide fragment can be sense or anti-sense ssDNA or ssRNA, dsRNA, or dsDNA, or dsDNA/RNA hybrids. The composition can include various components that include more than one polynucleotide fragments, a DHPS inhibitor herbicide and/or other herbicides that enhance the plant control activity of the composition.

In another aspect, polynucleotide molecules and methods for modulating DHPS gene expression in a plant species are provided. The method reduces, represses or otherwise delays expression of a DHPS gene in a plant comprising an external application to such plant of a composition comprising a polynucleotide and a transfer agent, wherein the polynucleotide is essentially identical or essentially complementary to a DHPS gene sequence or fragment thereof, or to the RNA transcript of the DHPS gene sequence or fragment thereof, wherein the DHPS gene sequence is selected from the group consisting of SEQ ID NO:1-54 or a polynucleotide fragment thereof. The polynucleotide fragment is at least 18 contiguous nucleotides, at least 19 contiguous nucleotides, at least 20 contiguous nucleotides or at least 21 contiguous nucleotides in length and at least 85 percent identical to a DHPS gene sequence selected from the group consisting of SEQ ID NO:1-54 and the transfer agent is an organosilicone compound. The polynucleotide fragment can be sense or anti-sense ssDNA or ssRNA, dsRNA, or dsDNA, or dsDNA/RNA hybrids. Polynucleotide molecules comprising SEQ ID NOs 55-1175 are fragments of the DHPS gene.

In a further aspect, the polynucleotide molecule composition may be combined with other herbicidal compounds to provide additional control of unwanted plants in a field of cultivated plants.

In a further aspect, the polynucleotide molecule composition may be combined with any one or more additional agricultural chemicals, such as, insecticides, fungicides, nematocides, bactericides, acaricides, growth regulators, chemosterilants, semiochemicals, repellents, attractants, pheromones, feeding stimulants, biopesticides, microbial pesticides or other biologically active compounds to form a multi-component pesticide giving an even broader spectrum of agricultural protection.

### DETAILED DESCRIPTION

Provided are methods and compositions containing a polynucleotide that provide for regulation, repression or delay of 7,8-dihydropteroate synthase (DHPS) gene expression and enhanced control of weedy plant species amd importantly DHPS inhibitor resistant weed biotypes. Aspects of the method can be applied to manage various weedy plants in agronomic and other cultivated environments.

The following definitions and methods are provided to better define the present invention and to guide those of ordinary skill in the art. Unless otherwise noted, terms are to be understood according to conventional usage by those of ordinary skill in the relevant art. Where a term is provided in the singular, the inventors also contemplate aspects described by the plural of that term.

By "non-transcribable" polynucleotides is meant that the polynucleotides do not comprise a complete polymerase II transcription unit.

As used herein "solution" refers to homogeneous mixtures and non-homogeneous mixtures such as suspensions, colloids, micelles, and emulsions.

Weedy plants are plants that compete with cultivated plants, those of particular importance include, but are not limited to important invasive and noxious weeds and herbicide resistant biotypes in crop production, such as, *Amaranthus* species -*A. albus, A. blitoides, A. hybridus, A. palmeri, A. powellii, A. retroflexus, A. spinosus, A. tuberculatus,* and *A*. *viridis; Ambrosia* species - *A. trifida, A. artemisifolia; Lolium* species *-L. multiflorum, L. rigidium, L perenne; Digitaria* species *-D. insularis; Euphorbia* species *-E. heterophylla; Kochia species - K. scoparia; Sorghum* species *-S. halepense; Conyza* species -*C*. *bonariensis, C. canadensis, C. sumatrensis; Chloris* species -*C*. *truncate; Echinochola* species - *E. colona, E. crus-galli; Eleusine species -E. indica; Poa* species *-P. annua; Plantago* species *-P. lanceolata; Avena species* - *A. fatua; Chenopodium* species - *C. album; Setaria* species - *S*. *viridis, Abutilon theophrasti, Ipomoea* species, *Sesbania,* species, *Cassia* species, *Sida* species, *Brachiaria,* species and *Solanum* species.

Additional weedy plant species found in cultivated areas include *Alopecurus myosuroides, Avena sterilis, Avena sterilis ludoviciana, Brachiaria plantaginea, Bromus diandrus, Bromus rigidus, Cynosurus echinatus, Digitaria ciliaris, Digitaria ischaemum, Digitaria sanguinalis, Echinochloa oryzicola, Echinochloa phyllopogon, Eriochloa punctata, Hordeum glaucum, Hordeum leporinum, Ischaemum rugosum, Leptochloa chinensis, Lolium persicum,* , *Phalaris minor, Phalaris paradoxa, Rottboellia exalta, Setaria faberi, Setaria viridis var, robusta-alba schreiber, Setaria viridis var, robusta-purpurea, Snowdenia polystachea, Sorghum sudanese, Alisma plantago-aquatica, Amaranthus lividus, Amaranthus quitensis, Ammania auriculata, Ammania coccinea, Anthemis cotula, Apera spica-venti, Bacopa rotundifolia, Bidens pilosa, Bidens subalternans, Brassica tournefortii, Bromus tectorum, Camelina microcarpa, Chrysanthemum coronarium, Cuscuta campestris, Cyperus difformis, Damasonium minus, Descurainia sophia, Diplotaxis tenuifolia, Echium plantagineum, Elatine triandra var, pedicellata, Euphorbia heterophylla, Fallopia convolvulus, Fimbristylis miliacea, Galeopsis tetrahit, Galium spurium, Helianthus annuus, Iva xanthifolia, Ixophorus unisetus, Ipomoea indica, Ipomoea purpurea, Ipomoea sepiaria, Ipomoea aquatic, Ipomoea triloba, Lactuca serriola, Limnocharis flava, Limnophila erecta, Limnophila sessiliflora, Lindernia dubia, Lindernia dubia var, major, Lindernia micrantha, Lindernia procumbens, Mesembryanthemum crystallinum, Monochoria korsakowii, Monochoria vaginalis, Neslia paniculata, Papaver rhoeas, Parthenium hysterophorus, Pentzia suffruticosa, Phalaris minor, Raphanus raphanistrum, Raphanus sativus, Rapistrum rugosum, Rotala indica var, uliginosa, Sagittaria guyanensis, Sagittaria montevidensis, Sagittaria pygmaea, Salsola iberica, Scirpus juncoides var, ohwianus, Scirpus mucronatus, Setaria lutescens, Sida spinosa, Sinapis arvensis, Sisymbrium orientale, Sisymbrium thellungii, Solanum ptycanthum, Sonchus asper, Sonchus oleraceus, Sorghum bicolor, Stellaria media, Thlaspi arvense, Xanthium strumarium, Arctotheca calendula, Conyza sumatrensis, Crassocephalum crepidiodes, Cuphea carthagenenis, Epilobium adenocaulon, Erigeron philadelphicus, Landoltia punctata, Lepidium virginicum, Monochoria korsakowii, Solanum americanum, Solanum nigrum, Vulpia bromoides, Youngia japonica, Hydrilla verticillata, Carduus nutans, Carduus pycnocephalus, Centaurea solstitialis, Cirsium arvense, Commelina diffusa, Convolvulus arvensis, Daucus carota, Digitaria ischaemum, Echinochloa crus-pavonis, Fimbristylis miliacea, Galeopsis tetrahit, Galium spurium, Limnophila erecta, Matricaria perforate, Papaver rhoeas, Ranunculus acris, Soliva sessilis, Sphenoclea zeylanica, Stellaria media, Nassella trichotoma, Stipa neesiana, Agrostis stolonifera, Polygonum aviculare, Alopecurus japonicus, Beckmannia syzigachne, Bromus tectorum, Chloris inflate, Echinochloa erecta, Portulaca oleracea, and Senecio vulgaris.* It is believed that all plants contain an DHPS gene in their genome, which can be isolated and polynucleotides made according to the methods that are useful for regulating, suppressing or delaying the expression of the target DHPS gene in the plants and the growth or development of the treated plants.

Some cultivated plants may also be weedy plants when they occur in unwanted environments. For example, corn plants growing in a soybean field. Transgenic crops with one or more herbicide tolerances will need specialized methods of management to control weeds and volunteer crop plants.

A "trigger" or "trigger polynucleotide" is a polynucleotide molecule that is homologous or complementary to a target gene polynucleotide. The trigger polynucleotide molecules modulate expression of the target gene when topically applied to a plant surface with a transfer agent, whereby a plant treated with said composition has its growth or development or reproductive ability regulated, suppressed or delayed or said plant is more sensitive to a DHPS inhibitor herbicide or mitosis inhibitor herbicide as a result of said polynucleotide containing composition relative to a plant not treated with a composition containing the trigger molecule.

It is contemplated that the compositions will contain one or more polynucleotides and one or more herbicides that include but not limited to DHPS gene trigger polynucleotides and a DHPS inhibitor herbicide and anyone or more additional herbicide target gene trigger polynucleotides and the related herbicides and anyone or more additional essential gene trigger polynucleotides. Essential genes are genes in a plant that provide key enzymes or other proteins, for example, a biosynthetic enzyme, metabolizing enzyme, receptor, signal transduction protein, structural gene product, transcription factor, or transport protein; or regulating RNAs, such as, microRNAs, that are essential to the growth or survival of the organism or cell or involved in the normal growth and development of the plant (Meinke, et al., Trends Plant Sci. 2008 Sep;13(9):483-91). The suppression of an essential gene enhances the effect of a herbicide that affects the function of a gene product different than the suppressed essential gene. The compositions can include various trigger polynucleotides that modulate the expression of an essential gene other than DHPS.

Herbicides, for which transgenes for plant tolerance have been demonstrated, include but are not limited to: auxin-like herbicides, glyphosate, glufosinate, sulfonylureas, imidazolinones, bromoxynil, delapon, dicamba, cyclohezanedione, protoporphyrionogen oxidase inhibitors, 4-hydroxyphenyl-pyruvate-dioxygenase inhibitors herbicides. For example, transgenes and their polynucleotide molecules that encode proteins involved in herbicide tolerance are known in the art, and include, but are not limited to an 5-enolpyruvylshikimate-3-phosphate synthase (EPSPS), for example, as more fully described in U.S. Pat. Nos. 7,807,791 (SEQ ID NO:5); 6,248,876 B1; 5,627,061; 5,804,425; 5,633,435; 5,145,783; 4,971,908; 5,392,910; 5,188,642; 4,940,835; 5,866,775; 6,225,114 B1; 6,130,366; 5,390,667; 4,535,060; 4,769,061; 5,633,448; 5,510,471; U.S. Pat. No. RE36,449; U.S. Pat. Nos. RE37,287 E; and 5,491,288; tolerance to sulfonylurea and/or imidazolinone, for example, as described more fully in U.S. Pat. Nos. 5,605,011; 5,013,659; 5,141,870; 5,767,361; 5,739,180; 5,304,732; 4,761,373; 5,339,107; 5,928,937; and 5,378,824; and international publication WO 96/33270; tolerance to hydroxyphenylpyruvatedioxygenases inhibitiong herbicides in plants are described in U.S. Pat. Nos. 6,245,968 B1; 6,268,549, and 6,069,115; US Pat.Pub. 20110191897 and US7,392,379 SEQ ID NO:3; US7,935,869; US7,304,209, SEQ ID NO:1, 3,5 and 15; aryloxyalkanoate dioxygenase polynucleotides, which confer tolerance to 2,4-D and other phenoxy auxin herbicides as well as to aryloxyphenoxypropionate herbicides as described, for example, in WO2005/107437; US7,838,733 SEQ ID NO:5;) and dicamba-tolerance polynucleotides as described, for example, in Herman et al. (2005) J. Biol. Chem. 280: 24759-24767. Other examples of herbicide-tolerance traits include those conferred by polynucleotides encoding an exogenous phosphinothricin acetyltransferase, as described in U.S. Pat. Nos. 5,969,213; 5,489,520; 5,550,398; 5,874,265; 5,919,675; 5,561,236; 5,648,477; 5,639,024; 6,177,616; and 5,879,903. Plants containing an exogenous phosphinothricin acetyltransferase can exhibit improved tolerance to glufosinate herbicides, which inhibit the enzyme glutamine synthase. Additionally, herbicide-tolerance polynucleotides include those conferred by polynucleotides conferring altered protoporphyrinogen oxidase (protox) activity, as described in U.S. Pat. Nos. 6,288,306 B1; 6,282,837 B1; and 5,767,373; and WO 01/12825. Plants containing such polynucleotides can exhibit improved tolerance to any of a variety of herbicides which target the protox enzyme (also referred to as protox inhibitors). Polynucleotides encoding a glyphosate oxidoreductase and a glyphosate-N-acetyl transferase (GOX described in U.S. Patent 5,393,175 and GAT described in U.S. Patent publication 20030083480, dicamba monooxygenase U.S. Patent publication 20030135879, all of which are incorporated herein by reference); a polynucleotide molecule encoding bromoxynil nitrilase (*Bxn* described in U.S. Patent No. 4,810,648 for Bromoxynil tolerance, which is incorporated herein by reference); a polynucleotide molecule encoding phytoene desaturase (*crtI*) described in Misawa et al, (1993) Plant J. 4:833-840 and Misawa et al, (1994) Plant J. 6:481-489 for norflurazon tolerance; a polynucleotide molecule encoding acetohydroxyacid synthase (AHAS, *aka* ALS) described in Sathasiivan et al. (1990) Nucl. Acids Res. 18:398-2193 for tolerance to sulfonylurea herbicides; and the *bar* gene described in DeBlock, et al. (1987) EMBO J. 6:2513-2519 for glufosinate and bialaphos tolerance. The transgenic coding regions and regulatory elements of the herbicide tolerance genes are targets in which polynucleotide triggers and herbicides can be included in the compositions.

DHPS inhibitor herbicides include but are not limited to carbamates and asulam. Mitosis inhibitor herbicides include but are not limited to dinitroaniline herbicides for example benfluralin, butralin, dinitramine, ethalfluralin, oryzalin, pendimethalin, and trifluralin. Additional mitosis inhibitor herbicides also include but are not limited to Phosphoroamidates, Pyridines, Benzamides, and Benzenedicarboxylic acids.

Numerous herbicides with similar or different modes of action (herein referred to as co-herbicides) are available that can be added to the compositions, for example, members of the herbicide families that include but are not limited to amide herbicides, aromatic acid herbicides, arsenical herbicides, benzothiazole herbicides, benzoylcyclohexanedione herbicides, benzofuranyl alkylsulfonate herbicides, carbamate herbicides, cyclohexene oxime herbicides, cyclopropylisoxazole herbicides, dicarboximide herbicides, dinitroaniline herbicides, dinitrophenol herbicides, diphenyl ether herbicides, dithiocarbamate herbicides, halogenated aliphatic herbicides, imidazolinone herbicides, inorganic herbicides, nitrile herbicides, organophosphorus herbicides, oxadiazolone herbicides, oxazole herbicides, phenoxy herbicides, phenylenediamine herbicides, pyrazole herbicides, pyridazine herbicides, pyridazinone herbicides, pyridine herbicides, pyrimidinediamine herbicides, pyrimidinyloxybenzylamine herbicides, quaternary ammonium herbicides, thiocarbamate herbicides, thiocarbonate herbicides, thiourea herbicides, triazine herbicides, triazinone herbicides, triazole herbicides, triazolone herbicides, triazolopyrimidine herbicides, uracil herbicides, and urea herbicides. In particular, the rates of use of the added herbicides can be reduced in compositions comprising the polynucleotides. Use rate reductions of the additional added herbicides can be 10-25 percent, 26-50 percent, 51-75 percent or more can be achieved that enhance the activity of the polynucleotides and herbicide composition and is contemplated. Representative co-herbicides of the families include but are not limited to acetochlor, acifluorfen, acifluorfen-sodium, aclonifen, acrolein, alachlor, alloxydim, allyl alcohol, ametryn, amicarbazone, amidosulfuron, aminopyralid, amitrole, ammonium sulfamate, anilofos, asulam, atraton, atrazine, azimsulfuron, BCPC, beflubutamid, benazolin, benfluralin, benfuresate, bensulfuron, bensulfuron-methyl, bensulide, bentazone, benzfendizone, benzobicyclon, benzofenap, bifenox, bilanafos, bispyribac, bispyribac-sodium, borax, bromacil, bromobutide, bromoxynil, butachlor, butafenacil, butamifos, butralin, butroxydim, butylate, cacodylic acid, calcium chlorate, cafenstrole, carbetamide, carfentrazone, carfentrazone-ethyl, CDEA, CEPC, chlorflurenol, chlorflurenol-methyl, chloridazon, chlorimuron, chlorimuron-ethyl, chloroacetic acid, chlorotoluron, chlorpropham, chlorsulfuron, chlorthal, chlorthal-dimethyl, cinidon-ethyl, cinmethylin, cinosulfuron, cisanilide, clethodim, clodinafop, clodinafop-propargyl, clomazone, clomeprop, clopyralid, cloransulam, cloransulam-methyl, CMA, 4-CPB, CPMF, 4-CPP, CPPC, cresol, cumyluron, cyanamide, cyanazine, cycloate, cyclosulfamuron, cycloxydim, cyhalofop, cyhalofop-butyl, 2,4-D, 3,4-DA, daimuron, dalapon, dazomet, 2,4-DB, 3,4-DB, 2,4-DEB, desmedipham, dicamba, dichlobenil, ortho-dichlorobenzene, para-dichlorobenzene, dichlorprop, dichlorprop-P, diclofop, diclofop-methyl, diclosulam, difenzoquat, difenzoquat metilsulfate, diflufenican, diflufenzopyr, dimefuron, dimepiperate, dimethachlor, dimethametryn, dimethenamid, dimethenamid-P, dimethipin, dimethylarsinic acid, dinitramine, dinoterb, diphenamid, diquat, diquat dibromide, dithiopyr, diuron, DNOC, 3,4-DP, DSMA, EBEP, endothal, EPTC, esprocarb, ethalfluralin, ethametsulfuron, ethametsulfuron-methyl, ethofumesate, ethoxyfen, ethoxysulfuron, etobenzanid, fenoxaprop-P, fenoxaprop-P-ethyl, fentrazamide, ferrous sulfate, flamprop-M, flazasulfuron, florasulam, fluazifop, fluazifop-butyl, fluazifop-P, fluazifop-P-butyl, flucarbazone, flucarbazone-sodium, flucetosulfuron, fluchloralin, flufenacet, flufenpyr, flufenpyr-ethyl, flumetsulam, flumiclorac, flumiclorac-pentyl, flumioxazin, fluometuron, fluoroglycofen, fluoroglycofen-ethyl, flupropanate, flupyrsulfuron, flupyrsulfuron-methyl-sodium, flurenol, fluridone, fluorochloridone, fluoroxypyr, flurtamone, fluthiacet, fluthiacet-methyl, fomesafen, foramsulfuron, fosamine, glufosinate, glufosinate-ammonium, glyphosate, halosulfuron, halosulfuron-methyl, haloxyfop, haloxyfop-P, HC-252, hexazinone, imazamethabenz, imazamethabenz-methyl, imazamox, imazapic, imazapyr, imazaquin, imazethapyr, imazosulfuron, indanofan, iodomethane, iodosulfuron, iodosulfuron-methyl-sodium, ioxynil, isoproturon, isouron, isoxaben, isoxachlortole, isoxaflutole, karbutilate, lactofen, lenacil, linuron, MAA, MAMA, MCPA, MCPA-thioethyl, MCPB, mecoprop, mecoprop-P, mefenacet, mefluidide, mesosulfuron, mesosulfuron-methyl, mesotrione, metam, metamifop, metamitron, metazachlor, methabenzthiazuron, methylarsonic acid, methyldymron, methyl isothiocyanate, metobenzuron, metolachlor, S-metolachlor, metosulam, metoxuron, metribuzin, metsulfuron, metsulfuron-methyl, MK-66, molinate, monolinuron, MSMA, naproanilide, napropamide, naptalam, neburon, nicosulfuron, nonanoic acid, norflurazon, oleic acid (fatty acids), orbencarb, orthosulfamuron, oryzalin, oxadiargyl, oxadiazon, oxasulfuron, oxaziclomefone, oxyfluorfen, paraquat, paraquat dichloride, pebulate, pendimethalin, penoxsulam, pentachlorophenol, pentanochlor, pentoxazone, pethoxamid, petrolium oils, phenmedipham, phenmedipham-ethyl, picloram, picolinafen, pinoxaden, piperophos, potassium arsenite, potassium azide, pretilachlor, primisulfuron, primisulfuron-methyl, prodiamine, profluazol, profoxydim, prometon, prometryn, propachlor, propanil, propaquizafop, propazine, propham, propisochlor, propoxycarbazone, propoxycarbazone-sodium, propyzamide, prosulfocarb, prosulfuron, pyraclonil, pyraflufen, pyraflufen-ethyl, pyrazolynate, pyrazosulfuron, pyrazosulfuron-ethyl, pyrazoxyfen, pyribenzoxim, pyributicarb, pyridafol, pyridate, pyriftalid, pyriminobac, pyriminobac-methyl, pyrimisulfan, pyrithiobac, pyrithiobac-sodium, quinclorac, quinmerac, quinoclamine, quizalofop, quizalofop-P, rimsulfuron, sethoxydim, siduron, simazine, simetryn, SMA, sodium arsenite, sodium azide, sodium chlorate, sulcotrione, sulfentrazone, sulfometuron, sulfometuron-methyl, sulfosate, sulfosulfuron, sulfuric acid, tar oils, 2,3,6-TBA, TCA, TCA-sodium, tebuthiuron, tepraloxydim, terbacil, terbumeton, terbuthylazine, terbutryn, thenylchlor, thiazopyr, thifensulfuron, thifensulfuron-methyl, thiobencarb, tiocarbazil, topramezone, tralkoxydim, tri-allate, triasulfuron, triaziflam, tribenuron, tribenuron-methyl, tricamba, triclopyr, trietazine, trifloxysulfuron, trifloxysulfuron-sodium, trifluralin, triflusulfuron, triflusulfuron-methyl, trihydroxytriazine, tritosulfuron, [3-[2-chloro-4-fluoro-5-(-methyl-6-trifluoromethyl-2,4-dioxo-,2,3,4-t-etrahydropyrimidin-3-yl)phenoxy]-2-pyridyloxy]acetic acid ethyl ester (CAS RN 353292-3-6), 4-[(4,5-dihydro-3-methoxy-4-methyl-5-oxo)-H-,2,4-triazol--ylcarbonyl-sulfamoyl]-5-methylthiophene-3-carboxylic acid (BAY636), BAY747 (CAS RN 33504-84-2), topramezone (CAS RN 2063-68-8), 4-hydroxy-3-[[2-[(2-methoxyethoxy)methyl]-6-(trifluoromethyl)-3-pyridi-nyl]carbonyl]-bicyclo[3.2.]oct-3-en-2-one (CAS RN 35200-68-5), and 4-hydroxy-3-[[2-(3-methoxypropyl)-6-(difluoromethyl)-3-pyridinyl]carbon-yl]-bicyclo[3.2.]oct-3-en-2-one. Additionally, including herbicidal compounds of unspecified modes of action as described in CN101279950A, CN101279951A, DE10000600A1, DE10116399A1, DE102004054666A1, DE102005014638A1, DE102005014906A1, DE102007012168A1, DE102010042866A1, DE10204951A1, DE10234875A1, DE10234876A1, DE10256353A1, DE10256354A1, DE10256367A1, EP1157991A2, EP1238586A1, EP2147919A1, EP2160098A2, JP03968012B2, JP2001253874A, JP2002080454A, JP2002138075A, JP2002145707A, JP2002220389A, JP2003064059A, JP2003096059A, JP2004051628A, JP2004107228A, JP2005008583A, JP2005239675A, JP2005314407A, JP2006232824A, JP2006282552A, JP2007153847A, JP2007161701A, JP2007182404A, JP2008074840A, JP2008074841A, JP2008133207A, JP2008133218A, JP2008169121A, JP2009067739A, JP2009114128A, JP2009126792A, JP2009137851A, US20060111241A1, US20090036311A1, US20090054240A1, US20090215628A1, US20100099561A1, US20100152443A1, US20110105329A1, US20110201501A1, WO2001055066A2, WO2001056975A1, WO2001056979A1, WO2001090071A2, WO2001090080A1, WO2002002540A1, WO2002028182A1, WO2002040473A1, WO2002044173A2, WO2003000679A2, WO2003006422A1 , WO2003013247A1, WO2003016308A1, WO2003020704A1, WO2003022051A1, WO2003022831A1, WO2003022843A1, WO2003029243A2, WO2003037085A1, WO2003037878A1, WO2003045878A2, WO2003050087A2, WO2003051823A1, WO2003051824A1, WO2003051846A2, WO2003076409A1, WO2003087067A1, WO2003090539A1, WO2003091217A1, WO2003093269A2, WO2003104206A2, WO2004002947A1, WO2004002981A2, WO2004011429A1, WO2004029060A1, WO2004035545A2, WO2004035563A1, WO2004035564A1, WO2004037787A1, WO2004067518A1, WO2004067527A1, WO2004077950A1, WO2005000824A1, WO2005007627A1, WO2005040152A1, WO2005047233A1, WO2005047281A1, WO2005061443A2, WO2005061464A1, WO2005068434A1, WO2005070889A1, WO2005089551A1, WO2005095335A1, WO2006006569A1, WO2006024820A1, WO2006029828A1, WO2006029829A1, WO2006037945A1, WO2006050803A1, WO2006090792A1, WO2006123088A2, WO2006125687A1, WO2006125688A1, WO2007003294A1, WO2007026834A1, WO2007071900A1, WO2007077201A1, WO2007077247A1, WO2007096576A1, WO2007119434A1, WO2007134984A1, WO2008009908A1, WO2008029084A1, WO2008059948A1, WO2008071918A1, WO2008074991A1, WO2008084073A1, WO2008100426A2, WO2008102908A1, WO2008152072A2, WO2008152073A2, WO2009000757A1, WO2009005297A2, WO2009035150A2, WO2009063180A1, WO2009068170A2, WO2009068171A2, WO2009086041A1, WO2009090401A2, WO2009090402A2, WO2009115788A1, WO2009116558A1, WO2009152995A1, WO2009158258A1, WO2010012649A1, WO2010012649A1, WO2010026989A1, WO2010034153A1, WO2010049270A1, WO2010049369A1, WO2010049405A1, WO2010049414A1, WO2010063422A1, WO2010069802A1, WO2010078906A2, WO2010078912A1, WO2010104217A1, WO2010108611A1, WO2010112826A3, WO2010116122A3, WO2010119906A1, WO2010130970A1, WO2011003776A2, WO2011035874A1, WO2011065451A1, all of which are incorporated herein by reference.

An agronomic field in need of plant control is treated by application of the composition directly to the surface of the growing plants, such as by a spray. For example, the method is applied to control weeds in a field of crop plants by spraying the field with the composition.. The composition can be provided as a tank mix, a sequential treatment of components (generally the polynucleotide containing composition followed by the herbicide), or a simultaneous treatment or mixing of one or more of the components of the composition from separate containers. Treatment of the field can occur as often as needed to provide weed control and the components of the composition can be adjusted to target specific weed species or weed families through utilization of specific polynucleotides or polynucleotide compositions capable of selectively targeting the specific species or plant family to be controlled. The composition can be applied at effective use rates according to the time of application to the field, for example, preplant, at planting, post planting, postharvest. DHPS inhibitor herbicides can be applied to a field at rates of 500 to 3000 g ai/ha (active ingredient per hectare) or more. The polynucleotides of the composition can be applied at rates of 1 to 30 grams per acre depending on the number of trigger molecules needed for the scope of weeds in the field.

Crop plants in which weed control is needed include but are not limited to, i) corn, soybean, cotton, canola, sugar beet, alfalfa, sugarcane, rice, and wheat; ii) vegetable plants including, but not limited to, tomato, sweet pepper, hot pepper, melon, watermelon, cucumber, eggplant, cauliflower, broccoli, lettuce, spinach, onion, peas, carrots, sweet corn, Chinese cabbage, leek, fennel, pumpkin, squash or gourd, radish, Brussels sprouts, tomatillo, garden beans, dry beans, or okra; iii) culinary plants including, but not limited to, basil, parsley, coffee, or tea; or, iv) fruit plants including but not limited to apple, pear, cherry, peach, plum, apricot, banana, plantain, table grape, wine grape, citrus, avocado, mango, or berry; v) a tree grown for ornamental or commercial use, including, but not limited to, a fruit or nut tree; or, vi) an ornamental plant (e. g., an ornamental flowering plant or shrub or turf grass). The methods and compositions provided herein can also be applied to plants produced by a cutting, cloning, or grafting process (i. e., a plant not grown from a seed) include fruit trees and plants that include, but are not limited to, citrus, apples, avocados, tomatoes, eggplant, cucumber, melons, watermelons, and grapes as well as various ornamental plants.

### Pesticidal Mixtures

The polynucleotide compositions may also be used as mixtures with various agricultural chemicals and/or insecticides, miticides and fungicides, pesticidal and biopesticidal agents. Examples include but are not limited to azinphos-methyl, acephate, isoxathion, isofenphos, ethion, etrimfos, oxydemeton-methyl, oxydeprofos, quinalphos, chlorpyrifos, chlorpyrifos-methyl, chlorfenvinphos, cyanophos, dioxabenzofos, dichlorvos, disulfoton, dimethylvinphos, dimethoate, sulprofos, diazinon, thiometon, tetrachlorvinphos, temephos, tebupirimfos, terbufos, naled, vamidothion, pyraclofos, pyridafenthion, pirimiphos-methyl, fenitrothion, fenthion, phenthoate, flupyrazophos, prothiofos, propaphos, profenofos, phoxime, phosalone, phosmet, formothion, phorate, malathion, mecarbam, mesulfenfos, methamidophos, methidathion, parathion, methyl parathion, monocrotophos, trichlorphon, EPN, isazophos, isamidofos, cadusafos, diamidaphos, dichlofenthion, thionazin, fenamiphos, fosthiazate, fosthietan, phosphocarb, DSP, ethoprophos, alanycarb, aldicarb, isoprocarb, ethiofencarb, carbaryl, carbosulfan, xylylcarb, thiodicarb, pirimicarb, fenobucarb, furathiocarb, propoxur, bendiocarb, benfuracarb, methomyl, metolcarb, XMC, carbofuran, aldoxycarb, oxamyl, acrinathrin, allethrin, esfenvalerate, empenthrin, cycloprothrin, cyhalothrin, gamma-cyhalothrin, lambda-cyhalothrin, cyfluthrin, beta-cyfluthrin, cypermethrin, alpha-cypermethrin, zeta-cypermethrin, silafluofen, tetramethrin, tefluthrin, deltamethrin, tralomethrin, bifenthrin, phenothrin, fenvalerate, fenpropathrin, furamethrin, prallethrin, flucythrinate, fluvalinate, flubrocythrinate, permethrin, resmethrin, ethofenprox, cartap, thiocyclam, bensultap, acetamiprid, imidacloprid, clothianidin, dinotefuran, thiacloprid, thiamethoxam, nitenpyram, chlorfluazuron, diflubenzuron, teflubenzuron, triflumuron, novaluron, noviflumuron, bistrifluoron, fluazuron, flucycloxuron, flufenoxuron, hexaflumuron, lufenuron, chromafenozide, tebufenozide, halofenozide, methoxyfenozide, diofenolan, cyromazine, pyriproxyfen, buprofezin, methoprene, hydroprene, kinoprene, triazamate, endosulfan, chlorfenson, chlorobenzilate, dicofol, bromopropylate, acetoprole, fipronil, ethiprole, pyrethrin, rotenone, nicotine sulphate, BT (Bacillus Thuringiensis) agent, spinosad, abamectin, acequinocyl, amidoflumet, amitraz, etoxazole, chinomethionat, clofentezine, fenbutatin oxide, dienochlor, cyhexatin, spirodiclofen, spiromesifen, tetradifon, tebufenpyrad, binapacryl, bifenazate, pyridaben, pyrimidifen, fenazaquin, fenothiocarb, fenpyroximate, fluacrypyrim, fluazinam, flufenzin, hexythiazox, propargite, benzomate, polynactin complex, milbemectin, lufenuron, mecarbam, methiocarb, mevinphos, halfenprox, azadirachtin, diafenthiuron, indoxacarb, emamectin benzoate, potassium oleate, sodium oleate, chlorfenapyr, tolfenpyrad, pymetrozine, fenoxycarb, hydramethylnon, hydroxy propyl starch, pyridalyl, flufenerim, flubendiamide, flonicamid, metaflumizole, lepimectin, TPIC, albendazole, oxibendazole, oxfendazole, trichlamide, fensulfothion, fenbendazole, levamisole hydrochloride, morantel tartrate, dazomet, metam-sodium, triadimefon, hexaconazole, propiconazole, ipconazole, prochloraz, triflumizole, tebuconazole, epoxiconazole, difenoconazole, flusilazole, triadimenol, cyproconazole, metconazole, fluquinconazole, bitertanol, tetraconazole, triticonazole, flutriafol, penconazole, diniconazole, fenbuconazole, bromuconazole, imibenconazole, simeconazole, myclobutanil, hymexazole, imazalil, furametpyr, thifluzamide, etridiazole, oxpoconazole, oxpoconazole fumarate, pefurazoate, prothioconazole, pyrifenox, fenarimol, nuarimol, bupirimate, mepanipyrim, cyprodinil, pyrimethanil, metalaxyl, mefenoxam, oxadixyl, benalaxyl, thiophanate, thiophanate-methyl, benomyl, carbendazim, fuberidazole, thiabendazole, manzeb, propineb, zineb, metiram, maneb, ziram, thiuram, chlorothalonil, ethaboxam, oxycarboxin, carboxin, flutolanil, silthiofam, mepronil, dimethomorph, fenpropidin, fenpropimorph, spiroxamine, tridemorph, dodemorph, flumorph, azoxystrobin, kresoxim-methyl, metominostrobin, orysastrobin, fluoxastrobin, trifloxystrobin, dimoxystrobin, pyraclostrobin, picoxystrobin, iprodione, procymidone, vinclozolin, chlozolinate, flusulfamide, dazomet, methyl isothiocyanate, chloropicrin, methasulfocarb, hydroxyisoxazole, potassium hydroxyisoxazole, echlomezol, D-D, carbam, basic copper chloride, basic copper sulfate, copper nonylphenolsulfonate, oxine copper, DBEDC, anhydrous copper sulfate, copper sulfate pentahydrate, cupric hydroxide, inorganic sulfur, wettable sulfur, lime sulfur, zinc sulfate, fentin, sodium hydrogen carbonate, potassium hydrogen carbonate, sodium hypochlorite, silver, edifenphos, tolclofos-methyl, fosetyl, iprobenfos, dinocap, pyrazophos, carpropamid, fthalide, tricyclazole, pyroquilon, diclocymet, fenoxanil, kasugamycin, validamycin, polyoxins, blasticiden S, oxytetracycline, mildiomycin, streptomycin, rape seed oil, machine oil, benthiavalicarbisopropyl, iprovalicarb, propamocarb, diethofencarb, fluoroimide, fludioxanil, fenpiclonil, quinoxyfen, oxolinic acid, chlorothalonil, captan, folpet, probenazole, acibenzolar-S-methyl, tiadinil, cyflufenamid, fenhexamid, diflumetorim, metrafenone, picobenzamide, proquinazid, famoxadone, cyazofamid, fenamidone, zoxamide, boscalid, cymoxanil, dithianon, fluazinam, dichlofluanide, triforine, isoprothiolane, ferimzone, diclomezine, tecloftalam, pencycuron, chinomethionat, iminoctadine acetate, iminoctadine albesilate, ambam, polycarbamate, thiadiazine, chloroneb, nickel dimethyldithiocarbamate, guazatine, dodecylguanidine-acetate, quintozene, tolylfluanid, anilazine, nitrothalisopropyl, fenitropan, dimethirimol, benthiazole, harpin protein, flumetover, mandipropamide and penthiopyrad.

### Polynucleotides

As used herein, the term "DNA", "DNA molecule", "DNA polynucleotide molecule" refers to a single-stranded DNA (ssDNA) or double-stranded DNA (dsDNA) molecule of genomic or synthetic origin, such as, a polymer of deoxyribonucleotide bases or a DNA polynucleotide molecule. As used herein, the term "DNA sequence", "DNA nucleotide sequence" or "DNA polynucleotide sequence" refers to the nucleotide sequence of a DNA molecule. As used herein, the term "RNA", "RNA molecule", "RNA polynucleotide molecule" refers to a single-stranded RNA (ssRNA) or double-stranded RNA (dsRNA) molecule of genomic or synthetic origin, such as, a polymer of ribonucleotide bases that comprise single or double stranded regions. Unless otherwise stated, nucleotide sequences in the text of this specification are given, when read from left to right, in the 5' to 3' direction. The nomenclature used herein is that required by Title 37 of the United States Code of Federal Regulations § 1.822 and set forth in the tables in WIPO Standard ST.25 (1998), Appendix 2, Tables 1 and 3.

As used herein, "polynucleotide" refers to a DNA or RNA molecule containing multiple nucleotides and generally refers both to "oligonucleotides" (a polynucleotide molecule of typically 50 or fewer nucleotides in length) and polynucleotides of 51 or more nucleotides. Embodiments include compositions including oligonucleotides having a length of 18-25 nucleotides (18-mers, 19-mers, 20-mers, 21-mers, 22-mers, 23-mers, 24-mers, or 25-mers), for example, oligonucleotides of Table 3 (SEQ ID NO:907-1175) pr fragments thereof or medium-length polynucleotides having a length of 26 or more nucleotides (polynucleotides of 26, 27, 28, 29, 30, 39, 32, 33, 34, 35, 36, 37, 38, 39, 40, 41, 42, 43, 44, 45, 39, 47, 48, 49, 50, 51, 52, 53, 54, 55, 56, 57, 58, 59, 60, about 65, about 70, about 75, about 80, about 85, about 90, about 95, about 100, about 110, about 120, about 130, about 140, about 150, about 160, about 170, about 180, about 190, about 200, about 210, about 220, about 230, about 240, about 250, about 260, about 270, about 280, about 290, or about 300 nucleotides), for example, polynucleotides of Table 2 (SEQ ID NO: 55-906) or fragments thereof or long polynucleotides having a length greater than about 300 nucleotides (for example, polynucleotides of between about 300 to about 400 nucleotides, between about 400 to about 500 nucleotides, between about 500 to about 600 nucleotides, between about 600 to about 700 nucleotides, between about 700 to about 800 nucleotides, between about 800 to about 900 nucleotides, between about 900 to about 1000 nucleotides, between about 300 to about 500 nucleotides, between about 300 to about 600 nucleotides, between about 300 to about 700 nucleotides, between about 300 to about 800 nucleotides, between about 300 to about 900 nucleotides, or about 1000 nucleotides in length, or even greater than about 1000 nucleotides in length, for example up to the entire length of a target gene including coding or non-coding or both coding and non-coding portions of the target gene), for example, polynucleotides of Table 1 (SEQ ID NO:1-54), wherein the selected polynucleotides or fragments thereof homologous or complementary to SEQ ID NO: 1-54 suppresses, represses or otherwise delay the expression of the target DHPS gene. A target gene comprises any polynucleotide molecule in a plant cell or fragment thereof for which the modulation of the expression of the target gene is provided by the methods and compositions. Where a polynucleotide is double-stranded, its length can be similarly described in terms of base pairs. Oligonucleotides and polynucleotides can be made that are essentially identical or essentially complementary to adjacent genetic elements of a gene, for example, spanning the junction region of an intron and exon, the junction region of a promoter and a transcribed region, the junction region of a 5' leader and a coding sequence, the junction of a 3' untranslated region and a coding sequence.

Polynucleotide compositions used in the various embodiments include compositions including oligonucleotides or polynucleotides or a mixture of both, including RNA or DNA or RNA/DNA hybrids or chemically modified oligonucleotides or polynucleotides or a mixture thereof. In some embodiments, the polynucleotide may be a combination of ribonucleotides and deoxyribonucleotides, for example, synthetic polynucleotides consisting mainly of ribonucleotides but with one or more terminal deoxyribonucleotides or synthetic polynucleotides consisting mainly of deoxyribonucleotides but with one or more terminal dideoxyribonucleotides. In some embodiments, the polynucleotide includes non-canonical nucleotides such as inosine, thiouridine, or pseudouridine. In some embodiments, the polynucleotide includes chemically modified nucleotides. Examples of chemically modified oligonucleotides or polynucleotides are well known in the art; see, for example, US Patent Publication 20110171287, US Patent Publication 20110171176, and US Patent Publication 20110152353, US Patent Publication, 20110152339, US Patent Publication 20110160082, herein incorporated by reference. For example, including but not limited to the naturally occurring phosphodiester backbone of an oligonucleotide or polynucleotide can be partially or completely modified with phosphorothioate, phosphorodithioate, or methylphosphonate internucleotide linkage modifications, modified nucleoside bases or modified sugars can be used in oligonucleotide or polynucleotide synthesis, and oligonucleotides or polynucleotides can be labeled with a fluorescent moiety (for example, fluorescein or rhodamine) or other label (for example, biotin).

The polynucleotides can be single- or double-stranded RNA or single- or double-stranded DNA or double-stranded DNA/RNA hybrids or modified analogues thereof, and can be of oligonucleotide lengths or longer. In more specific embodiments, the polynucleotides that provide single-stranded RNA in the plant cell are selected from the group consisting of (a) a single-stranded RNA molecule (ssRNA), (b) a single-stranded RNA molecule that self-hybridizes to form a double-stranded RNA molecule, (c) a double-stranded RNA molecule (dsRNA), (d) a single-stranded DNA molecule (ssDNA), (e) a single-stranded DNA molecule that self-hybridizes to form a double-stranded DNA molecule, and (f) a single-stranded DNA molecule including a modified Pol III gene that is transcribed to an RNA molecule, (g) a double-stranded DNA molecule (dsDNA), (h) a double-stranded DNA molecule including a modified Pol III gene that is transcribed to an RNA molecule, (i) a double-stranded, hybridized RNA/DNA molecule, or combinations thereof. In some embodiments these polynucleotides include chemically modified nucleotides or non-canonical nucleotides. In some embodiments, the oligonucleotides may be blunt-ended or may comprise a 3' overhang of from 1-5 nucleotides of at least one or both of the strands. Other configurations of the oligonucleotide are known in the field and are contemplated herein. In embodiments of the method the polynucleotides include double-stranded DNA formed by intramolecular hybridization, double-stranded DNA formed by intermolecular hybridization, double-stranded RNA formed by intramolecular hybridization, or double-stranded RNA formed by intermolecular hybridization. In one embodiment the polynucleotides include single-stranded DNA or single-stranded RNA that self-hybridizes to form a hairpin structure having an at least partially double-stranded structure including at least one segment that will hybridize to RNA transcribed from the gene targeted for suppression. Not intending to be bound by any mechanism, it is believed that such polynucleotides are or will produce single-stranded RNA with at least one segment that will hybridize to RNA transcribed from the gene targeted for suppression. In certain other embodiments the polynucleotides further includes a promoter, generally a promoter functional in a plant, for example, a pol II promoter, a pol III promoter, a pol IV promoter, or a pol V promoter.

The term "gene" refers to components that comprise chromosomal DNA, plasmid DNA, cDNA, intron and exon DNA, artificial DNA polynucleotide, or other DNA that encodes a peptide, polypeptide, protein, or RNA transcript molecule, and the genetic elements flanking the coding sequence that are involved in the regulation of expression, such as, promoter regions, 5' leader regions, 3' untranslated region that may exist as native genes or transgenes in a plant genome. The gene or a fragment thereof is isolated and subjected to polynucleotide sequencing methods that determines the order of the nucleotides that comprise the gene. Any of the components of the gene are potential targets for a trigger oligonucleotide and polynucleotides.

The trigger polynucleotide molecules are designed to modulate expression by inducing regulation or suppression of an endogenous DHPS gene in a plant and are designed to have a nucleotide sequence essentially identical or essentially complementary to the nucleotide sequence of an endogenous DHPS gene of a plant or to the sequence of RNA transcribed from an endogenous DHPS gene of a plant, including a transgene in a plant that provides for a herbicide resistant DHPS enzyme, which can be coding sequence or non-coding sequence. Effective molecules that modulate expression are referred to as "a trigger molecule, or trigger polynucleotides". By "essentially identical" or "essentially complementary" is meant that the trigger polynucleotides (or at least one strand of a double-stranded polynucleotide or portion thereof, or a portion of a single strand polynucleotide) are designed to hybridize to the endogenous gene noncoding sequence or to RNA transcribed (known as messenger RNA or an RNA transcript) from the endogenous gene to effect regulation or suppression of expression of the endogenous gene. Trigger molecules are identified by "tiling" the gene targets with partially overlapping probes or non-overlapping probes of antisense or sense polynucleotides that are essentially identical or essentially complementary to the nucleotide sequence of an endogenous gene. Multiple target sequences can be aligned and sequence regions with homology in common are identified as potential trigger molecules for the multiple targets. Multiple trigger molecules of various lengths, for example 18-25 nucleotides, 26-50 nucleotides, 51-100 nucleotides, 101-200 nucleotides, 201-300 nucleotides or more can be pooled into a few treatments in order to investigate polynucleotide molecules that cover a portion of a gene sequence (for example, a portion of a coding versus a portion of a noncoding region, or a 5' versus a 3' portion of a gene) or an entire gene sequence including coding and noncoding regions of a target gene. Polynucleotide molecules of the pooled trigger molecules can be divided into smaller pools or single molecules inorder to identify trigger molecules that provide the desired effect.

The target gene RNA and DNA polynucleotide molecules (Table 1, SEQ ID NO:1-54) are sequenced by any number of available methods and equipment. Some of the sequencing technologies are available commercially, such as the sequencing-by-hybridization platform from Affymetrix Inc. (Sunnyvale, Calif.) and the sequencing-by-synthesis platforms from 454 Life Sciences (Bradford, Conn.), Illumina/Solexa (Hayward, Calif.) and Helicos Biosciences (Cambridge, Mass.), and the sequencing-by-ligation platform from Applied Biosystems (Foster City, Calif.), as described below. In addition to the single molecule sequencing performed using sequencing-by-synthesis of Helicos Biosciences, other single molecule sequencing technologies are encompassed by the method and include the SMRT™ technology of Pacific Biosciences, the Ion Torrent™. technology, and nanopore sequencing being developed for example, by Oxford Nanopore Technologies. A DHPS target gene comprising DNA or RNA can be isolated using primers or probes essentially complementary or essentially homologous to SEQ ID NO:1-54 or a fragment thereof. A polymerase chain reaction (PCR) gene fragment can be produced using primers essentially complementary or essentially homologous to SEQ ID NO:1-54 or a fragment thereof.that is useful to isolate a DHPS gene from a plant genome.

Embodiments of functional single-stranded polynucleotides functional have sequence complementarity that need not be 100 percent, but is at least sufficient to permit hybridization to RNA transcribed from the target gene or DNA of the target gene to form a duplex to permit a gene silencing mechanism. Thus, in embodiments, a polynucleotide fragment is designed to be essentially identical to, or essentially complementary to, a sequence of 18 or more contiguous nucleotides in either the target DHPS gene sequence or messenger RNA transcribed from the target gene. By "essentially identical" is meant having 100 percent sequence identity or at least about 83, 84, 85, 86, 87, 88, 89, 90, 91, 92, 93, 94, 95, 96, 97, 98, or 99 percent sequence identity when compared to the sequence of 18 or more contiguous nucleotides in either the target gene or RNA transcribed from the target gene; by "essentially complementary" is meant having 100 percent sequence complementarity or at least about 83, 84, 85, 86, 87, 88, 89, 90, 91, 92, 93, 94, 95, 96, 97, 98, or 99 percent sequence complementarity when compared to the sequence of 18 or more contiguous nucleotides in either the target gene or RNA transcribed from the target gene. In some embodiments, polynucleotide molecules are designed to have 100 percent sequence identity with or complementarity to one allele or one family member of a given target gene (coding or non-coding sequence of a gene); in other embodiments the polynucleotide molecules are designed to have 100 percent sequence identity with or complementarity to multiple alleles or family members of a given target gene. The trigger polynucleotide sequences in the sequence listing SEQ ID NO: 1-1222 or table 1, 2 or 3 maybe complementary or homologous to a portion of the DHPS target gene sequence.

In certain embodiments, the polynucleotides used in the compositions that are essentially identical or essentially complementary to the target gene or transcript will comprise the predominant nucleic acid in the composition. Thus in certain embodiments, the polynucleotides that are essentially identical or essentially complementary to the target gene or transcript will comprise at least about 50%, 75%, 95%, 98% or 100% of the nucleic acids provided in the composition by either mass or molar concentration. However, in certain embodiments, the polynucleotides that are essentially identical or essentially complementary to the target gene or transcript can comprise at least about 1% to about 50%, about 10% to about 50%, about 20% to about 50%, or about 30% to about 50% of the nucleic acids provided in the composition by either mass or molar concentration. Also provided are compositions where the polynucleotides that are essentially identical or essentially complementary to the target gene or transcript can comprise at least about 1% to 100%, about 10% to 100%, about 20% to about 100%, about 30% to about 50%, or about 50% to a 100% of the nucleic acids provided in the composition by either mass or molar concentration.

"Identity" refers to the degree of similarity between two polynucleic acid or protein sequences. An alignment of the two sequences is performed by a suitable computer program. A widely used and accepted computer program for performing sequence alignments is CLUSTALW v1.6 (Thompson, et al. Nucl. Acids Res., 22: 3973-3980, 1994). The number of matching bases or amino acids is divided by the total number of bases or amino acids, and multiplied by 100 to obtain a percent identity. For example, if two 580 base pair sequences had 145 matched bases, they would be 25 percent identical. If the two compared sequences are of different lengths, the number of matches is divided by the shorter of the two lengths. For example, if there are 100 matched amino acids between a 200 and a 400 amino acid protein, they are 50 percent identical with respect to the shorter sequence. If the shorter sequence is less than 150 bases or 50 amino acids in length, the number of matches are divided by 150 (for nucleic acid bases) or 50 (for amino acids), and multiplied by 100 to obtain a percent identity.

Trigger molecules for specific gene family members can be identified from coding and/or non-coding sequences of gene families of a plant or multiple plants, by aligning and selecting 200-300 polynucleotide fragments from the least homologous regions amongst the aligned sequences and evaluated using topically applied polynucleotides (as sense or anti-sense ssDNA or ssRNA, dsRNA, or dsDNA) to determine their relative effectiveness in inducing the herbicidal phenotype. The effective segments are further subdivided into 50-60 polynucleotide fragments, prioritized by least homology, and reevaluated using topically applied polynucleotides. The effective 50-60 polynucleotide fragments are subdivided into 19-30 polynucleotide fragments, prioritized by least homology, and again evaluated for induction of the yield/quality phenotype. Once relative effectiveness is determined, the fragments are utilized singly, or again evaluated in combination with one or more other fragments to determine the trigger composition or mixture of trigger polynucleotides for providing the yield/quality phenotype.

Trigger molecules for broad activity can be identified from coding and/or non-coding sequences of gene families of a plant or multiple plants, by aligning and selecting 200-300 polynucleotide fragments from the most homologous regions amongst the aligned sequences and evaluated using topically applied polynucleotides (as sense or anti-sense ssDNA or ssRNA, dsRNA, or dsDNA) to determine their relative effectiveness in inducing the yield/quality phenotype. The effective segments are subdivided into 50-60 polynucleotide fragments, prioritized by most homology, and reevaluated using topically applied polynucleotides. The effective 50-60 polynucleotide fragments are subdivided into 19-30 polynucleotide fragments, prioritized by most homology, and again evaluated for induction of the yield/quality phenotype. Once relative effectiveness is determined, the fragments may be utilized singly, or in combination with one or more other fragments to determine the trigger composition or mixture of trigger polynucleotides for providing the yield/quality phenotype.

Methods of making polynucleotides are well known in the art. Chemical synthesis, in vivo synthesis and in vitro synthesis methods and compositions are known in the art and include various viral elements, microbial cells, modified polymerases, and modified nucleotides. Commercial preparation of oligonucleotides often provides two deoxyribonucleotides on the 3' end of the sense strand. Long polynucleotide molecules can be synthesized from commercially available kits, for example, kits from Applied Biosystems/Ambion (Austin, TX) have DNA ligated on the 5' end in a microbial expression cassette that includes a bacterial T7 polymerase promoter that makes RNA strands that can be assembled into a dsRNA and kits provided by various manufacturers that include T7 RiboMax Express (Promega, Madison, WI), AmpliScribe T7-Flash (Epicentre, Madison, WI), and TranscriptAid T7 High Yield (Fermentas, Glen Burnie, MD). dsRNA molecules can be produced from microbial expression cassettes in bacterial cells (Ongvarrasopone et al. ScienceAsia 33:35-39; Yin, Appl. Microbiol. Biotechnol 84:323-333, 2009; Liu et al., BMC Biotechnology 10:85, 2010) that have regulated or deficient RNase III enzyme activity or the use of various viral vectors to produce sufficient quantities of dsRNA. DHPS gene fragments are inserted into the microbial expression cassettes in a position in which the fragments are express to produce ssRNA or dsRNA useful in the methods described herein to regulate expression on a target DHPS gene. Long polynucleotide molecules can also be assembled from multiple RNA or DNA fragments. In some embodiments design parameters such as Reynolds score (Reynolds et al. Nature Biotechnology 22, 326 - 330 (2004),Tuschl rules (Pei and Tuschl, Nature Methods 3(9): 670-676, 2006), i-score (Nucleic Acids Res 35: e123, 2007), i-Score Designer tool and associated algorithms (Nucleic Acids Res 32: 936-948, 2004. Biochem Biophys Res Commun 316: 1050-1058, 2004, Nucleic Acids Res 32: 893-901, 2004, Cell Cycle 3: 790-5, 2004, Nat Biotechnol 23: 995-1001, 2005, Nucleic Acids Res 35: e27, 2007, BMC Bioinformatics 7: 520, 2006, Nucleic Acids Res 35: e123, 2007, Nat Biotechnol 22: 326-330, 2004) are known in the art and may be used in selecting polynucleotide sequences effective in gene silencing. In some embodiments random design or empirical selection of polynucleotide sequences is used in selecting polynucleotide sequences effective in gene silencing. In some embodiments the sequence of a polynucleotide is screened against the genomic DNA of the intended plant to minimize unintentional silencing of other genes.

The polynucleotide compositions are useful in compositions, such as solutions of polynucleotide molecules, at low concentrations, alone or in combination with other components either in the same solution or in separately applied solutions that provide a permeability-enhancing agent. While there is no upper limit on the concentrations and dosages of polynucleotide molecules that can useful in the methods, lower effective concentrations and dosages will generally be sought for efficiency. The concentrations can be adjusted in consideration of the volume of spray or treatment applied to plant leaves or other plant part surfaces, such as flower petals, stems, tubers, fruit, anthers, pollen, or seed. In one embodiment, a useful treatment for herbaceous plants using 25-mer oligonucleotide molecules is about 1 nanomole (nmol) of oligonucleotide molecules per plant, for example, from about 0.05 to 1 nmol per plant. Other embodiments for herbaceous plants include useful ranges of about 0.05 to about 100 nmol , or about 0.1 to about 20 nmol, or about 1 nmol to about 10 nmol of polynucleotides per plant. Very large plants, trees, or vines may require correspondingly larger amounts of polynucleotides. When using long dsRNA molecules that can be processed into multiple oligonucleotides, lower concentrations can be used. To illustrate embodiments, the factor 1X, when applied to oligonucleotide molecules is arbitrarily used to denote a treatment of 0.8 nmol of polynucleotide molecule per plant; 10X, 8 nmol of polynucleotide molecule per plant; and 100X, 80 nmol of polynucleotide molecule per plant.

The trigger polynucleotide and oligonucleotide molecule compositions are useful in compositions, such as liquids that comprise these polynucleotide molecules, alone or in combination with other components, for example one or more herbicide molecules, either in the same liquid or in separately applied liquids that also provide a transfer agent. As used herein, a transfer agent is an agent that, when combined with a polynucleotide in a composition that is topically applied to a target plant surface, enables the polynucleotide to enter a plant cell. In certain embodiments, a transfer agent is an agent that conditions the surface of plant tissue, e. g., leaves, stems, roots, flowers, or fruits, to permeation by the polynucleotide molecules into plant cells. The transfer of polynucleotides into plant cells can be facilitated by the prior or contemporaneous application of a polynucleotide-transferring agent to the plant tissue. In some embodiments the transferring agent is applied subsequent to the application of the polynucleotide composition. The polynucleotide transfer agent enables a pathway for polynucleotides through cuticle wax barriers, stomata and/or cell wall or membrane barriers into plant cells. Suitable transfer agents to facilitate transfer of the polynucleotide into a plant cell include agents that increase permeability of the exterior of the plant or that increase permeability of plant cells to oligonucleotides or polynucleotides. Such agents to facilitate transfer of the composition into a plant cell include a chemical agent, or a physical agent, or combinations thereof. Chemical agents for conditioning or transfer include (a) surfactants, (b) an organic solvent or an aqueous solution or aqueous mixtures of organic solvents, (c) oxidizing agents, (d) acids, (e) bases, (f) oils, (g) enzymes, or combinations thereof. Embodiments of the method can optionally include an incubation step, a neutralization step (e.g., to neutralize an acid, base, or oxidizing agent, or to inactivate an enzyme), a rinsing step, or combinations thereof. Embodiments of agents or treatments for conditioning of a plant to permeation by polynucleotides include emulsions, reverse emulsions, liposomes, and other micellar-like compositions. Embodiments of agents or treatments for conditioning of a plant to permeation by polynucleotides include counter-ions or other molecules that are known to associate with nucleic acid molecules, *e. g.,* inorganic ammonium ions, alkyl ammonium ions, lithium ions, polyamines such as spermine, spermidine, or putrescine, and other cations. Organic solvents useful in conditioning a plant to permeation by polynucleotides include DMSO, DMF, pyridine, N-pyrrolidine, hexamethylphosphoramide, acetonitrile, dioxane, polypropylene glycol, other solvents miscible with water or that will dissolve phosphonucleotides in non-aqueous systems (such as is used in synthetic reactions). Naturally derived or synthetic oils with or without surfactants or emulsifiers can be used, *e. g.,* plant-sourced oils, crop oils (such as those listed in the 9^{th} Compendium of Herbicide Adjuvants, publicly available on the worldwide web (internet) at herbicide.adjuvants.com can be used, *e. g.,* paraffinic oils, polyol fatty acid esters, or oils with short-chain molecules modified with amides or polyamines such as polyethyleneimine or N-pyrrolidine. Transfer agents include, but are not limited to, organosilicone preparations.

Ligands can be tethered to a polynucleotide, for example a dsRNA, ssRNA, dsDNA or ssDNA. Ligands in general can include modifiers, e.g., for enhancing uptake; diagnostic compounds or reporter groups e.g., for monitoring distribution; cross-linking agents; nuclease-resistance conferring moieties; and natural or unusual nucleobases. General examples include lipophiles, lipids (e.g., cholesterol, a bile acid, or a fatty acid (e.g., lithocholic-oleyl, lauroyl, docosnyl, stearoyl, palmitoyl, myristoyl oleoyl, linoleoyl), steroids (e.g., uvaol, hecigenin, diosgenin), terpenes (e.g., triterpenes, e.g., sarsasapogenin, Friedelin, epifriedelanol derivatized lithocholic acid), vitamins (e.g., folic acid, vitamin A, biotin, pyridoxal), carbohydrates, proteins, protein binding agents, integrin targeting molecules, polycationics, peptides, polyamines, and peptide mimics. The ligand may also be a recombinant or synthetic molecule, such as a synthetic polymer, e.g., polyethylene glycol (PEG), PEG-40K, PEG-20K and PEG-5K. Other examples of ligands include lipophilic molecules, e.g, cholesterol, cholic acid, adamantane acetic acid, 1-pyrene butyric acid, dihydrotestosterone, glycerol (e.g., esters and ethers thereof, e.g., C.sub. 10, C.sub.11, C.sub.12, C.sub.13, C.sub.14, C.sub.15, C.sub.16, C.sub.17, C.sub.18, C.sub.19, or C.sub.20 alkyl; e.g., lauroyl, docosnyl, stearoyl, oleoyl, linoleoyl 1,3-bis-O(hexadecyl)glycerol, 1,3-bis-O(octaadecyl)glycerol), geranyloxyhexyl group, hexadecylglycerol, borneol, menthol, 1,3-propanediol, heptadecyl group, palmitic acid, myristic acid, O3-(oleoyl)lithocholic acid, O3-(oleoyl)cholenic acid, dodecanoyl, lithocholyl, 5.beta.-cholanyl, N,N-distearyl-lithocholamide, 1,2-di-O-stearoylglyceride, dimethoxytrityl, or phenoxazine) and PEG (e.g., PEG-5K, PEG-20K, PEG-40K). Preferred lipophilic moieties include lipid, cholesterols, oleyl, retinyl, or cholesteryl residues.

Conjugating a ligand to a dsRNA can enhance its cellular absorption, lipophilic compounds that have been conjugated to oligonucleotides include 1-pyrene butyric acid, 1,3-bis-O-(hexadecyl)glycerol, and menthol. One example of a ligand for receptor-mediated endocytosis is folic acid. Folic acid enters the cell by folate-receptor-radiated endocytosis. dsRNA compounds bearing folic acid would be efficiently transported into the cell via the folate-receptor-mediated endocytosis. Other ligands that have been conjugated to oligonucleotides include polyethylene glycols, carbohydrate clusters, cross-linking agents, porphyrin conjugates, delivery peptides and lipids such as cholesterol. In certain instances, conjugation of a cationic ligand to oligonucleotides results in improved resistance to nucleases. Representative examples of cationic ligands are propylammonium and dimethylpropylammonium. Interestingly, antisense oligonucleotides were reported to retain their high binding affinity to mRNA when the cationic ligand was dispersed, throughout the oligonucleotide. See M. Manoharan Antisense & Nucleic Acid Drug Development 2002, 12, 103 and references therein.

A biologic delivery can be accomplished by a variety of methods including, without limitation, (1) loading liposomes with a dsRNA acid molecule provided herein and (2) complexing a dsRNA molecule with lipids or liposomes to form nucleic acid-lipid or nucleic acid-liposome complexes. The liposome can be composed of cationic and neutral lipids commonly used to transfect cells in vitro. Cationic lipids can complex (e.g., charge-associate) with negatively charged, nucleic acids to form liposomes. Examples of cationic liposomes include, without limitation, lipofectin, lipofectamine, lipofectace, and DOTAP. Procedures for forming liposomes are well known in the art. Liposome compositions can be formed, for example, from phosphatidylcholine, dimyristoyl phosphatidylcholine, dipalmitoyl phosphatidylcholine, dimyristoyl phosphatidyl glycerol, dioleoyl phosphatidylethanolamine or liposomes comprising dihydrosphingomyelin (DHSM) Numerous lipophilic agents are commercially available, including Lipofectin.RTM. (Invitrogen/Life Technologies, Carlsbad, Calif.) and Effectene™ (Qiagen, Valencia, Calif.), In addition, systemic delivery methods can be optimized using commercially available cationic lipids such as DDAB or DOTAP, each of which can be mixed with a neutral lipid such as DOPE or cholesterol. In some eases, liposomes such as those described by Templeton et al. Nature Biotechnology, 15:647-652 (1997) can be used. In other embodiments, polycations such as polyethyleneimine can be used to achieve delivery in vivo and ex vivo (Boletta et al., J. Am Soc. Nephrol. 7:1728, 1996). Additional information regarding the use of liposomes to deliver nucleic acids can be found in U.S. Pat. No. 6,271,359, PCT Publication WO 96/40964 and Morrissey, D. et al., 2005. Nature Biotechnol. 23(8):1002-7.

In certain embodiments, an organosilicone preparation that is commercially available as Silwet® L-77 surfactant having CAS Number 27306-78-1 and EPA Number: CAL.REG.NO. 5905-50073-AA, and currently available from Momentive Performance Materials, Albany, New York can be used to prepare a polynucleotide composition. In certain embodiments where a Silwet L-77 organosilicone preparation is used as a pre-spray treatment of plant leaves or other plant surfaces, freshly made concentrations in the range of about 0.015 to about 2 percent by weight (wt percent) (*e. g.,* about 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.5 wt percent) are efficacious in preparing a leaf or other plant surface for transfer of polynucleotide molecules into plant cells from a topical application on the surface. In certain embodiments of the methods and compositions provided herein, a composition that comprises a polynucleotide molecule and an organosilicone preparation comprising Silwet L-77 in the range of about 0.015 to about 2 percent by weight (wt percent) *(e. g.,* about 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.5 wt percent) is used or provided.

In certain embodiments, any of the commercially available organosilicone preparations provided such as the following Breakthru S 321, Breakthru S 200 Cat# 67674-67-3, Breakthru OE 441 Cat#68937-55-3, Breakthru S 278 Cat #27306-78-1, Breakthru S 243, Breakthru S 233 Cat#134180-76-0, available from manufacturer Evonik Goldschmidt (Germany), Silwet® HS 429, Silwet® HS 312, Silwet® HS 508, Silwet® HS 604 (Momentive Performance Materials, Albany, New York) can be used as transfer agents in a polynucleotide composition. In certain embodiments where an organosilicone preparation is used as a pre-spray treatment of plant leaves or other surfaces, freshly made concentrations in the range of about 0.015 to about 2 percent by weight (wt percent) *(e. g.,* about 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.5 wt percent) are efficacious in preparing a leaf or other plant surface for transfer of polynucleotide molecules into plant cells from a topical application on the surface. In certain embodiments of the methods and compositions provided herein, a composition that comprises a polynucleotide molecule and an organosilicone preparation in the range of about 0.015 to about 2 percent by weight (wt percent) (*e. g.,* about 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.5 wt percent) is used or provided.

Organosilicone preparations used in the methods and compositions provided herein can comprise one or more effective organosilicone compounds. As used herein, the phrase "effective organosilicone compound" is used to describe any organosilicone compound that is found in an organosilicone preparation that enables a polynucleotide to enter a plant cell. In certain embodiments, an effective organosilicone compound can enable a polynucleotide to enter a plant cell in a manner permitting a polynucleotide mediated suppression of a target gene expression in the plant cell. In general, effective organosilicone compounds include, but are not limited to, compounds that can comprise: i) a trisiloxane head group that is covalently linked to, ii) an alkyl linker including, but not limited to, an n-propyl linker, that is covalently linked to, iii) a poly glycol chain, that is covalently linked to, iv) a terminal group. Trisiloxane head groups of such effective organosilicone compounds include, but are not limited to, heptamethyltrisiloxane. Alkyl linkers can include, but are not limited to, an n-propyl linker. Poly glycol chains include, but are not limited to, polyethylene glycol or polypropylene glycol. Poly glycol chains can comprise a mixture that provides an average chain length "n" of about "7.5". In certain embodiments, the average chain length "n" can vary from about 5 to about 14. Terminal groups can include, but are not limited to, alkyl groups such as a methyl group. Effective organosilicone compounds are believed to include, but are not limited to, trisiloxane ethoxylate surfactants or polyalkylene oxide modified heptamethyl trisiloxane. 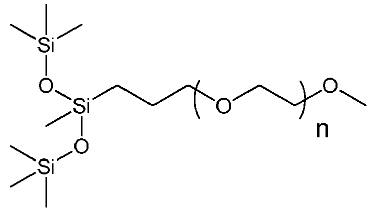 (Compound I: polyalkyleneoxide heptamethyltrisiloxane, average n=7.5).

In certain embodiments, an organosilicone preparation that comprises an organosilicone compound comprising a trisiloxane head group is used in the methods and compositions provided herein. In certain embodiments, an organosilicone preparation that comprises an organosilicone compound comprising a heptamethyltrisiloxane head group is used in the methods and compositions provided herein. In certain embodiments, an organosilicone composition that comprises Compound I is used in the methods and compositions provided herein. In certain embodiments, an organosilicone composition that comprises Compound I is used in the methods and compositions provided herein. In certain embodiments of the methods and compositions provided herein, a composition that comprises a polynucleotide molecule and one or more effective organosilicone compound in the range of about 0.015 to about 2 percent by weight (wt percent) (*e. g.,* about 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.5 wt percent) is used or provided.

Compositions include but are not limited components that are one or more polynucleotides essentially identical to, or essentially complementary to a DHPS gene sequence (promoter, intron, exon, 5' untranslated region, 3' untranslated region), a transfer agent that provides for the polynucleotide to enter a plant cell, a herbicide that complements the action of the polynucleotide, one or more additional herbicides that further enhance the herbicide activity of the composition or provide an additional mode of action different from the complementing herbicide, various salts and stabilizing agents that enhance the utility of the composition as an admixture of the components of the composition.

The methods include one or more applications of a polynucleotide composition and one or more applications of a permeability-enhancing agent for conditioning of a plant to permeation by polynucleotides. When the agent for conditioning to permeation is an organosilicone composition or compound contained therein, embodiments of the polynucleotide molecules are double-stranded RNA oligonucleotides, single-stranded RNA oligonucleotides, double-stranded RNA polynucleotides, single-stranded RNA polynucleotides, double-stranded DNA oligonucleotides, single-stranded DNA oligonucleotides, double-stranded DNA polynucleotides, single-stranded DNA polynucleotides, chemically modified RNA or DNA oligonucleotides or polynucleotides or mixtures thereof.

Compositions and methods are useful for modulating the expression of an endogenous DHPS gene or transgenic DHPS gene (for example US Patent No. 6,121,513) in a plant cell. In various embodiments, a DHPS gene includes coding (protein-coding or translatable) sequence, non-coding (non-translatable) sequence, or both coding and non-coding sequence. Compositions can include polynucleotides and oligonucleotides designed to target multiple genes, or multiple segments of one or more genes. The target gene can include multiple consecutive segments of a target gene, multiple non-consecutive segments of a target gene, multiple alleles of a target gene, or multiple target genes from one or more species.

An aspect provides a method for modulating expression of a DHPS gene in a plant including (a) conditioning of a plant to permeation by polynucleotides and (b) treatment of the plant with the polynucleotide molecules, wherein the polynucleotide molecules include at least one segment of 18 or more contiguous nucleotides cloned from or otherwise identified from the target DHPS gene in either anti-sense or sense orientation, whereby the polynucleotide molecules permeate the interior of the plant and induce modulation of the target gene. The conditioning and polynucleotide application can be performed separately or in a single step. When the conditioning and polynucleotide application are performed in separate steps, the conditioning can precede or can follow the polynucleotide application within minutes, hours, or days. In some embodiments more than one conditioning step or more than one polynucleotide molecule application can be performed on the same plant. In embodiments of the method, the segment can be cloned or identified from (a) coding (protein-encoding), (b) non-coding (promoter and other gene related molecules), or (c) both coding and non-coding parts of the target gene. Non-coding parts include DNA, such as promoter regions or the RNA transcribed by the DNA that provide RNA regulatory molecules, including but not limited to: introns, 5' or 3' untranslated regions, and microRNAs (miRNA), *trans*-acting siRNAs, natural anti-sense siRNAs, and other small RNAs with regulatory function or RNAs having structural or enzymatic function including but not limited to: ribozymes, ribosomal RNAs, t-RNAs, aptamers, and riboswitches.

All publications, patents and patent applications are herein incorporated by reference to the same extent as if each individual publication or patent application was specifically and individually indicated to be incorporated by reference.

The following examples are included to demonstrate examples of certain preferred embodiments. It should be appreciated by those of skill in the art that the techniques disclosed in the examples that follow represent approaches the inventors have found function well in the practice, and thus can be considered to constitute examples of preferred modes for its practice. However, those of skill in the art should, in light of the present disclosure, appreciate that many changes can be made in the specific embodiments that are disclosed and still obtain a like or similar result without departing from the spirit and scope.

### EXAMPLES

### Example 1. Polynucleotides related to the DHPS gene sequences.

The target DHPS polynucleotide molecule naturally occurs in the genome of plants that include but are not limited to *Amaranthus palmeri, Amaranthus rudis, Amaranthus hybridus, Amaranthus lividus, Amaranthus viridis, Ambrosia trifida, Conyza candensis, Digitaria sanguinalis, Euphorbia heterophylla, Kochia scoparia, Lolium multiflorum,* and include molecules related to the expression of a polypeptide identified as a DHPS, that include genomic DNA (gDNA) and coding cDNAs comprising coding and noncoding regions of a DHPS gene and fragments thereof as shown in Table 1.

Polynucleotide molecules were extracted from these plant species by methods standard in the field, for example, total RNA was extracted using Trizol Reagent (Invitrogen Corp, Carlsbad, CA Cat. No. 15596-018), following the manufacturer's protocol or modifications thereof by those skilled in the art of polynucleotide extraction that may enhance recover or purity of the extracted RNA. Briefly, start with 1 gram of ground plant tissue for extraction. Prealiquot 10 milliliters (mL) Trizol reagent to 15 mL conical tubes, add ground powder to tubes and shake to homogenize. Incubate the homogenized samples for 5 minutes (min) at room temperature (RT) and then add 3 mL of chloroform. Shakes tubes vigorously by hand for 15-30 seconds (sec) and incubate at RT for 3 min. Centrifuge the tubes at 7,000 revolutions per minute (rpm) for 10 min at 4 degrees C. Transfer the aqueous phase to a new 1.5 mL tube and add 1 volume of cold isopropanol. Incubate the samples for 20-30 min at RT and centrifuge at 10,000 rpm for 10 min at 4 degrees C. Wash pellet with Sigma-grade 80 percent ethanol. Remove the supernatant and briefly air-dry the pellet. Dissolve the RNA pellet in approximately 200 microliters of DEPC treated water. Heat briefly at 65C to dissolve pellet and vortex or pipet to resuspend RNA pellet and then adjust RNA concentration to 1-2 microgram/microliter.

DNA was extracted using EZNA SP Plant DNA Mini kit (Omega Biotek, Norcross GA, Cat#D5511) and Lysing Matrix E tubes (Q-Biogen, Cat#6914), following the manufacturer's protocol or modifications thereof by those skilled in the art of polynucleotide extraction that may enhance recover or purity of the extracted DNA. Briefly, aliquot ground tissue to a Lysing Matrix E tube on dry ice, add 800µl Buffer SP1 to each sample, homogenize in a bead beater for 35-45sec, incubate on ice for 45-60 sec, centrifuge at ≥14000 rpm for 1min at RT, add 10 microliter RNase A to the lysate, incubate at 65°C for 10min, centrifuge for 1min at RT, add 280µl Buffer SP2 and vortex to mix, incubate the samples on ice for 5min, centrifuge at ≥10,000g for 10 min at RT, transfer the supernatant to a homogenizer column in a 2ml collection tube, centrifuge at 10,000g for 2 min at RT, transfer the cleared lysate into a 1.5ml microfuge tube, add 1.5 volumes Buffer SP3 to the cleared lysate, vortex immediately to obtain a homogeneous mixture, transfer up to 650µl supernatant to the Hi-Bind column, centrifuge at 10,000g for 1min, repeat, apply 100µl 65°C Elution Buffer to the column, centrifuge at 10,000g for 5min at RT.

Next-generation DNA sequencers, such as the 454-FLX (Roche, Branford, CT), the SOLiD (Applied Biosystems,), and the Genome Analyzer (HiSeq2000, Illumina, San Diego, CA) were used to provide polynucleotide sequence from the DNA and RNA extracted from the plant tissues. Raw sequence data was assembled into contigs. The contig sequence was used to identify trigger molecules that can be applied to a plant to enable regulation of the gene expression.

The target DNA sequence isolated from genomic (gDNA) and coding DNA (cDNA) from the various weedy plant species for the DHPS gene and the assembled contigs as set forth in SEQ ID NOs 1-54 and Table 1 (See supplemental attachment 40_21(58641)Btable1.doxc)

### Example 2. Polynucleotides related to the trigger molecules

The gene sequences and fragments of Table 1 were divided into 200 polynucleotide (200-mer) lengths with 25 polynucleotide overlapping regions and are shown in Table 2, SEQ ID NO:55-906. These polynucleotides are tested to select the most efficacious trigger regions across the length of any target sequence. The trigger polynucleotides are constructed as sense or anti-sense ssDNA or ssRNA, dsRNA, or dsDNA, or dsDNA/RNA hybrids and combined with an organosilicone based transfer agent to provide a polynucleotide preparation. The polynucleotides are combined into sets of two to three polynucleotides per set, using 4-8 nmol of each polynucleotide. Each polynucleotide set is prepared with the transfer agent and applied to a plant or a field of plants in combination with a DHPS inhibitor containing herbicide, or followed by a DHPS inhibitor treatment one to three days after the polynucleotide application, to determine the effect on the plant's susceptibility to an DHPS inhibitor. The effect is measured as stunting the growth and/or killing of the plant and is measured 8-14 days after treatment with the polynucleotide set and DHPS inhibitor. The most efficacious sets are identified and the individual polynucleotides are tested in the same methods as the sets are and the most efficacious single 200-mer identified. The 200-mer sequence is divided into smaller sequences of 50-70-mer regions with 10-15 polynucleotide overlapping regions and the polynucleotides tested individually. The most efficacious 50-70-mer is further divided into smaller sequences of 25-mer regions with a 12 to 13 polynucleotide overlapping region and tested for efficacy in combination with DHPS inhibitor treatment. By this method it is possible to identify an oligonucleotide or several oligonucleotides that are the most efficacious trigger molecule to effect plant sensitivity to a DHPS inhibitor or modulation of an DHPS gene expression. The modulation of DHPS gene expression is determined by the detection of DHPS siRNA moleclules specific to a DHPS gene or by an observation of a reduction in the amount of DHPS RNA transcript produced relative to an untreated plant or by merely observing the anticipated phenotype of the application of the trigger with the DHPS inhibitor containing herbicide. Detection of siRNA can be accomplished, for example, using kits such as mirVana (Ambion, Austin TX) and mirPremier (Sigma-Aldrich, St Louis, MO).

The target DNA sequence isolated from genomic (gDNA) and coding DNA (cDNA) from the various weedy plant species for the DHPS gene and the assembled contigs as set forth in SEQ ID NOs 1-54 were divided into polynucleotide fragments as shown in Table 2 (See supplemental attachment 40_21(58641)Btable2.doxc) and as set forth in SEQ ID NOs 55-906.

The gene sequences and fragments of Table 1 were compared and 21-mers of contiguous polynucleotides were identified that had homology across the various DHPS gene sequences. The purpose is to identify trigger molecules that are useful as herbicidal molecules or in combination with a DHPS inhibitor herbicide across a broad range of weed species. The sequences shown in Table 3 represent the 21-mers that were present in the DHPS gene of at least eight of the weed species of Table 1. It is contemplated that additional 21-mers can be selected from the sequences of Table 1 that are specific for a single weed species or a few weeds species within a genus or trigger molecules that are at least 18 contiguous nucleotides, at least 19 contiguous nucleotides, at least 20 contiguous nucleotides or at least 21 contiguous nucleotides in length and at least 85 percent identical to an DHPS gene sequence selected from the group consisting of SEQ ID NO:1-54 or fragment thereof.

By this method it is possible to identify an oligonucleotide or several oligonucleotides that are the most efficacious trigger molecule to effect plant sensitivity to DHPS inhibitor or modulation of DHPS gene expression. The modulation of DHPS gene expression is determined by the detection of DHPS siRNA moleclules specific to DHPS gene or by an observation of a reduction in the amount of DHPS RNA transcript produced relative to an untreated plant. Detection of siRNA can be accomplished, for example, using kits such as mirVana (Ambion, Austin TX) and mirPremier (Sigma-Aldrich, St Louis, MO).

The target DNA sequence isolated from genomic (gDNA) and coding DNA (cDNA) from the various weedy plant species for the DHPS gene and the assembled contigs as set forth in SEQ ID NOs 1-54 were divided into fragments as shown in Table 3 (See supplemental attachment 40_21(58641)Btable3.doxc) and as set forth in SEQ ID NOs 907-1175.

### Example 3. Methods related to treating plants or plant parts with a topical mixture of the trigger molecules.

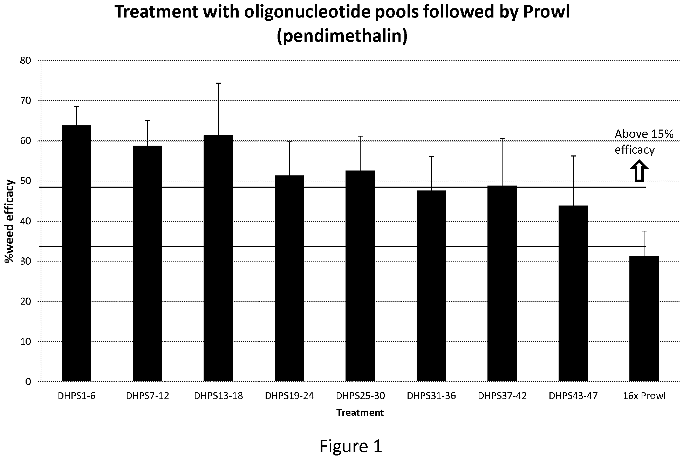

Glyphosate-sensitive Palmer amaranth (*A. palmeri* R-22) plants were grown in the greenhouse (30/20 C day/night T; 14 hour photoperiod) in 4 inch square pots containing Sun Gro® Redi-Earth and 3.5 kg/cubic meter Osmocote® 14-14-14 fertilizer. Palmer amaranth plants at 5 to 10 cm in height were pre-treated with a mixture of short (25mer) dsDNA trigger oligonucleotides targeting DHPS coding or noncoding regions using 4 nmol of each oligonucleotids and pooling 5-6 oligonucleotides in each treatment, formulated in 20 millimolar sodium phosphate buffer (pH 6.8) containing 2% ammonium sulfate and 1% Silwet L-77. Plants were treated manually by pipetting 10 µL of polynucleotide solution on four fully expanded mature leaves, for a total of 40 microliters of solution per plant. There were eight treatment pools, DHPS1-6, DHPS7-12, DHPS13-18, DHPS19-24, DHPS25-30, DHPS31-36, DHPS37-42, and DHPS43-47 (Table 4). Twenty-four hours later, the plants were treated with pendimethalin (Prowl®, BASF, this herbicide functions as a mitosis inhibitor similar to asulam a known DHPS inhibitor, other dinitroaniline herbicides that function to inhibit mitosis include but are not limited to Sonalan® (ethalfluralin), Squadron1®, Steel1®, Treflan®/Trilin®/Tri-4) at a rate of 121b/ac. Four replications of each treatment was conducted. Plant height was determined just before polynucleotide treatment and at intervals upto fourteen days after herbicide treatments to determine effect of the oligonucleotide and herbicide treatments. The results were expressed as percent reduction in height relative to the untreated control (no formulation and no trigger molecules), another treatment was the formulation control which is herbicide plus buffer plus ammonium plus Silwet. Figure 1 illustrates the results of this test. Three of the pooled oligonucleotides demonstrated an enhancement of the herbicide activity, these are DHPS 1-6, DHPS7-12, and DHPS 13-18. Further testing of single oligonucleotides from DHPS1-6 and DHPS7-12 demonstrated that DHPS1 (SEQ ID NO:1176) and DHPS 11(SEQ ID NO:1186) had the highest activity among the oligonucleotides in those respective pools with these 2 oligonucleotides providing greater than 15 percent increase in herbicide injury.

**Table 4. DHPS dsDNA oligonucleotides.**

| trigger name | SEQ ID NO: | Sense sequence | Antisense sequence |
|---|---|---|---|
| DHPS1 | 1176 | TTTTATTCTAAAGTTGCttcGGAGG | CCTCCGAAGCAACTTTAGAATAAAA |
| DHPS2 | 1177 | CCGTCAAGAAGGGGGCACACATTGT | ACAATGTGTGCCCCCTTCTTGACGG |
| DHPS3 | 1178 | GAATGATGTCTCtaGTGGgAAACTC | GAGTTTCCCACTAGAGACATCATTC |
| DHPS4 | 1179 | GATTCCGAGATGTTTAATGTTGTTG | CAACAACATTAAACATCTCGGAATC |
| DHPS5 | 1180 | CGGACCTTAAAGTTCCTTATATAGC | GCTATATAAGGAACTTTAAGGTCCG |
| DHPS6 | 1181 | AATGCACATGCGAGGAGATCCGACT | AGTCGGATCTCCTCGCATGTGCATT |
| DHPS7 | 1182 | TCAATGCAAAACTCTGAGAACTTGA | TCAAGTTCTCAGAGTTTTGCATTGA |
| DHPS8 | 1183 | CCTACAATGATGTTTGTAAGCAAGT | ACTTGCTTACAAACATCATTGTAGG |
| DHPS9 | 1184 | GGCTTCGGAGTTGAGTTCTAGGGTc | GACCCTAGAACTCAACTCCGAAGCC |
| DHPS10 | 1185 | ATAGATGCAGAATTATCGGGAATTC | GAATTCCCGATAATTCTGCATCTAT |
| DHPS11 | 1186 | CTGCTTGGAGGATAGTTATTGATCC | GGATCAATAACTATCCTCCAAGCAG |
| DHPS12 | 1187 | CGGCATCGGATTTTCTAAGAATACG | CGTATTCTTAGAAAATCCGATGCCG |
| DHPS13 | 1188 | AATCAAAATTTGGAAATTCTTAGTG | CACTAAGAATTTCCAAATTTTGATT |
| DHPS14 | 1189 | GTTTACAAAAGATACGGGAAGAGAT | ATCTCTTCCCGTATCTTTTGTAAAC |
| DHPS15 | 1190 | AGCTAAGAAGAGTTTGGCGGTGGCT | AGCCACCGCCAAACTCTTCTTAGCT |
| DHPS16 | 1191 | CATTGCCCCTTGCTAATTGGACCTT | AAGGTCCAATTAGCAAGGGGCAATG |
| DHPS17 | 1192 | CAAGAAAGAGGTTTCTGGGCGAGAT | ATCTCGCCCAGAAACCTCTTTCTTG |
| DHPS18 | 1193 | TTGTGAGCGCCCTGTAGCAGCTGAC | GTCAGCTGCTACAGGGCGCTCACAA |
| DHPS19 | 1194 | AGGGATCCTGCTACCATTGCTTCTA | TAGAAGCAATGGTAGCAGGATCCCT |
| DHPS20 | 1195 | TAACTGCTGGAGTTTTAGGTGGTGC | GCACCACCTAAAACTCCAGCAGTTA |
| DHPS21 | 1196 | AAACATTGTAAGAGTACATAATGTt | AACATTATGTACTCTTACAATGTTT |
| DHPS22 | 1197 | AGGGATAACCTTGATGCTGTCAAGT | ACTTGACAGCATCAAGGTTATCCCT |
| DHPS23 | 1198 | TATGTGATGCCATACTCGGAAAAAC | GTTTTTCCGAGTATGGCATCACATA |
| DHPS24 | 1199 | TGATTAACTGCTTGTTTGTACCACC | GGTGGTACAAACAAGCAGTTAATCA |
| DHPS25 | 1200 | TTGTGAATGATGTCTAGTGGAAGGT | ACCTTCCACTAGACATCATTCACAA |
| DHPS26 | 1201 | TCGATTGGGATTATGATGAAGTCCG | CGGACTTCATCATAATCCCAATCGA |
| DHPS27 | 1202 | TTAGTTTGCTCGAAAGtaGAGCTTT | AAAGCTCTACTTTCGAGCAAACTAA |
| DHPS28 | 1203 | AGCCCGTAAGGctATAGTTCTTACA | TGTAAGAACTATAGCCTTACGGGCT |
| DHPS29 | 1204 | GATGTAGAATCATTGGCTATTTGCC | GGCAAATAGCCAATGATTCTACATC |
| DHPS30 | 1205 | TATTTCTGCTTAGAAGATCATAGCA | TGCTATGATCTTCTAAGCAGAAATA |
| DHPS31 | 1206 | TTGATCCAGACTTTGGATTCCATGT | ACATGGAATCCAAAGTCTGGATCAA |
| DHPS32 | 1207 | AGAATTATGTTGCGACTCATGGTCG | CGACCATGAGTCGCAACATAATTCT |
| DHPS33 | 1208 | AATTGGCCCTTTGAGGAATTGTTGA | TCAACAATTCCTCAAAGGGCCAATT |
| DHPS34 | 1209 | AGTTCCTTGAGTCTATGGTTCAATC | GATTGAACCATAGACTCAAGGAACT |
| DHPS35 | 1210 | GGCTCGGTTCGATGAAGGTTGGACT | AGTCCAACCTTCATCGAACCGAGCC |
| DHPS36 | 1211 | GTGTCGAGACAAGATGGGTCAAAGA | TCTTTGACCCATCTTGTCTCGACAC |
| DHPS37 | 1212 | GACCAATGGACAAGAAGTCGACCTT | AAGGTCGACTTCTTGTCCATTGGTC |
| DHPS38 | 1213 | GGGCAAGCTTGATCgaGTTACTTGG | CCAAGTAACTCGATCAAGCTTGCCC |
| DHPS39 | 1214 | ACAaGCAGATAACAGCAGGCTCTGG | CCAGAGCCTGCTGTTATCTGCTTGT |
| DHPS40 | 1215 | CGACcATTAGACATACATATCATTt | AAATGATATGTATGTCTAATGGTCG |
| DHPS41 | 1216 | GTTTTTtGAAGTCAGATCTGCAATC | GATTGCAGATCTGACTTCAAAAAAC |
| DHPS42 | 1217 | AATCAGTTGCAATGGACAAaCCATA | TATGGTTTGTCCATTGCAACTGATT |
| DHPS43 | 1218 | TACGGTTaTTagTTGTTCCTGTCAC | GTGACAGGAACAACTAATAACCGTA |
| DHPS44 | 1219 | TgTTTGAGTTGAATTAGATCATGCA | TGCATGATCTAATTCAACTCAAACA |
| DHPS45 | 1220 | AACATTGTAAGAATCTTGATCATGT | ACATGATCAAGATTCTTACAATGTT |
| DHPS46 | 1221 | GAAGCTATGTGATACTGTACTTCTC | GAGAAGTACAGTATCACATAGCTTC |
| DHPS47 | 1222 | AAAATGAAATCTTTGATATGATGTT | AACATCATATCAAAGATTTCATTTT |

### Example 4. A method to control weeds in a field.

A method to control weeds in a field comprises the use of trigger polynucleotides that can modulate the expression of a DHPS gene in one or more target weed plant species. In Table 3, an analysis of DHPS gene sequences from multiple plant species provided a collection of 21-mer polynucleotides that were common to at least 4 of the species and can be used in compositions to affect the growth or develop or sensitivity to DHPS inhibitor herbicide to control multiple weed species in a field. Other oligonucleotide segments can be selected from the disclosed gene sequences that are more specific to a particular weed species, for example, an oligonucleotide that has a sequence that is homologous or complementary to a DHPS gene of three weeds species, or of two weed species, or of only one weed species. A composition containing 1 or 2 or 3 or 4 or more of the polynucleotides of Table 3 would enable broad activity of the composition against the multiple weed species that occur in a field environment.

The method includes creating a composition that comprises components that include at least one polynucleotide of Table 3 (SEQ ID NO:907-1175) or any other effective gene expression modulating polynucleotide essentially identical or essentially complementary to SEQ ID NO:1-54 or fragment thereof, a transfer agent that mobilizes the polynucleotide into a plant cell and a DHPS inhibiting herbicide and optionally a polynucleotide that modulates the expression of an essential gene and optionally a co-herbicide that has a different mode of action relative to a DHPS inhibitor. The polynucleotide of the composition includes a dsRNA, ssDNA or dsDNA or a combination thereof. A composition containing a polynucleotide can have a use rate of about 1 to 30 grams or more per acre depending on the size of the polynucleotide and the number of polynucleotides in the composition. The composition may include one or more additional co-herbicides as needed to provide effective multi-species weed control. Crop safety can be enhanced by reducing the amount of effective herbicide needed to control weeds in the field. A field of crop plants or a turf grass environment in need of weedy plant control is treated by spray application of the composition. The composition can be provided as a tank mix, a sequential treatment of components (generally the polynucleotide followed by the herbicide), a simultaneous treatment or mixing of one or more of the components of the composition from separate containers. Treatment of the field can occur as often as needed to provide weed control and the components of the composition can be adjusted to target specific weed species or weed families.

### Example 5. Herbicidal Compositions comprising pesticidal agents

A method of controlling weeds and plant pest and pathogens in a field of DHPS inhibitor tolerant crop plants is provided, wherein the method comprises applying a composition comprising a DHPS trigger oligonucleotide, a DHPS inhibitor composition and an admixture of a pest control agent. For example, the admixture comprises insecticides, fungicides, nematocides, bactericides, acaricides, growth regulators, chemosterilants, semiochemicals, repellents, attractants, pheromones, feeding stimulants or other biologically active compounds or biological agents, such as, microorganisms.

For example, the admixture comprises a fungicide compound for use on a DHPS inhibitor tolerant crop plant to prevent or control plant disease caused by a plant fungal pathogen, The fungicide compound of the admixture may be a systemic or contact fungicide or mixtures of each. More particularly the fungicide compound includes, but is not limited to members of the chemical groups strobilurins, triazoles, chloronitriles, carboxamides and mixtures thereof. The composition may additional have an admixture comprises an insecticidal compound or agent.

The DHPS trigger oligonucleotides and DHPS inhibitor or mitosis inhibitor herbicide (for example, asulam, or other dinitroaniline herbicides) tank mixes with fungicides, insecticides or both are tested for use in soybean and corn for control of foliar diseases and pests. Testing is conducted to develop a method for use of mixtures of the trigger oligonucleotides and asulam formulation and various commercially available fungicides for weed control and pest control. The field plots are planted with soybeans or corn. All plots receive a post plant application of the DHPS trigger + asulam about 3 weeks after planting. The mixtures of trigger + asulam or trigger + asulam + fungicide + insecticides are used to treat the plots at the R1 stage of soybean development (first flowering) or tassel stage of corn. Data is taken for percent weed control at 7 and 21 days after R1 treatment, soybean safety (% necrosis, chlorosis, growth rate): 5 days after treatment, disease rating, pest ratings and yield (bushels/Acre). These mixtures and treatments are designed to provide simultaneous weed and pest control, such as fungal pest control, for example, leaf rust disease; and insect pest control, for example, aphids, armyworms, loopers, beetles, stinkbugs, and leaf hoppers.

Agricultural chemicals are provided in containers suitable for safe storage, transportation and distribution, stability of the chemical compositions, mixing with solvents and instructions for use. A container of a mixture of a trigger oligonucleotide + herbicice + fungicide compound, or a mixture of a trigger oligonucleotide + herbicide compound and an insecticide compound, or a trigger oligonucleotide + a herbicide compound and a fungicide compound and an insecticide compound (for example, lambda-cyhalothrin, Warrier®). The container may further provide instructions on the effective use of the mixture. Containers of the present invention can be of any material that is suitable for the storage of the chemical mixture. Containers of the present invention can be of any material that is suitable for the shipment of the chemical mixture. The material can be of cardboard, plastic, metal, or a composite of these materials. The container can have a volume of 0.5 liter, 1 liter, 2 liter, 3-5 liter, 5-10 liter, 10-20 liter, 20-50 liter or more depending upon the need. A tank mix of a trigger oligonucleotide + herbicide compound and a fungicide compound is provided, methods of application to the crop to achieve an effective dose of each compound are known to those skilled in the art and can be refined and further developed depending on the crop, weather conditions, and application equipment used.

Insecticides, fungicides, nematocides, bactericides, acaricides, growth regulators, chemosterilants, semiochemicals, repellents, attractants, pheromones, feeding stimulants or other biologically active compounds can be added to the trigger oligonucleotide to form a multi-component pesticide giving an even broader spectrum of agricultural protection. Examples of such agricultural protectants with which compounds of this invention can be formulated are: insecticides such as abamectin, acephate, azinphos-methyl, bifenthrin, buprofezin, carbofuran, chlorfenapyr, chlorpyrifos, chlorpyrifos-methyl, cyfluthrin, beta-cyfluthrin, cyhalothrin, lambda-cyhalothrin, deltamethrin, diafenthiuron, diazinon, diflubenzuron, dimethoate, esfenvalerate, fenoxycarb, fenpropathrin, fenvalerate, fipronil, flucythrinate, tau-fluvalinate, fonophos, imidacloprid, isofenphos, malathion, metaldehyde, methamidophos, methidathion, methomyl, methoprene, methoxychlor, methyl 7-chloro-2,5-dihydro-2-[[N-(methoxycarbonyl)-N-[4-(trifluoromethoxy)phenyl]amino]carbonyl]indeno[1,2-e][1,3,4]oxadiazine-4a(3H)-carboxylate (DPX-JW062), monocrotophos, oxamyl, parathion, parathion-methyl, permethrin, phorate, phosalone, phosmet, phosphamidon, pirimicarb, profenofos, rotenone, sulprofos, tebufenozide, tefluthrin, terbufos, tetrachlorvinphos, thiodicarb, tralomethrin, trichlorfon and triflumuron; most preferably a DHPS inhibitor compound is formulated with a fungicide compound or combinations of fungicides, such as azoxystrobin, benomyl, blasticidin-S, Bordeaux mixture (tribasic copper sulfate), bromuconazole, captafol, captan, carbendazim, chloroneb, chlorothalonil, copper oxychloride, copper salts, cymoxanil, cyproconazole, cyprodinil (CGA 219417), diclomezine, dicloran, difenoconazole, dimethomorph, diniconazole, diniconazole-M, dodine, edifenphos, epoxiconazole (BAS 480F), famoxadone, fenarimol, fenbuconazole, fenpiclonil, fenpropidin, fenpropimorph, fluazinam, fluquinconazole, flusilazole, flutolanil, flutriafol, folpet, fosetyl-aluminum, furalaxyl, hexaconazole, ipconazole, iprobenfos, iprodione, isoprothiolane, kasugamycin, kresoxim-methyl, mancozeb, maneb, mepronil, metalaxyl, metconazole, S-methyl 7-benzothiazolecarbothioate (CGA 245704), myclobutanil, neo-asozin

(ferric methanearsonate), oxadixyl, penconazole, pencycuron, probenazole, prochloraz, propiconazole, pyrifenox, pyroquilon, quinoxyfen, spiroxamine (KWG4168), sulfur, tebuconazole, tetraconazole, thiabendazole, thiophanate-methyl, thiram, triadimefon, triadimenol, tricyclazole, trifloxystrobin, triticonazole, validamycin and vinclozolin; combinations of fungicides are common for example, cyproconazole and azoxystrobin, difenoconazole, and metalaxyl-M, fludioxonil and metalaxyl-M, mancozeb and metalaxyl-M, copper hydroxide and metalaxyl-M, cyprodinil and fludioxonil, cyproconazole and propiconazole; commercially available fungicide formulations for control of Asian soybean rust disease include, but are not limited to Quadris® (Syngenta Corp), Bravo® (Syngenta Corp), Echo 720® (Sipcam Agro Inc), Headline® 2.09EC (BASF Corp), Tilt® 3.6EC (Syngenta Corp), PropiMax™ 3.6EC (Dow AgroSciences), Bumper® 41.8EC (MakhteshimAgan), Folicur® 3.6F (Bayer CropScience), Laredo® 25EC (Dow AgroSciences), Laredo™ 25EW (Dow AgroSciences), Stratego® 2.08F (Bayer Corp), Domark™ 125SL (Sipcam Agro USA), and Pristine®38%WDG (BASF Corp) these can be combined with DHPS inhibitor compositions as described in the present invention to provide enhanced protection from fungal disease; nematocides such as aldoxycarb and fenamiphos; bactericides such as streptomycin; acaricides such as amitraz, chinomethionat, chlorobenzilate, cyhexatin, dicofol, dienochlor, etoxazole, fenazaquin, fenbutatin oxide, fenpropathrin, fenpyroximate, hexythiazox, propargite, pyridaben and tebufenpyrad; and biological agents such as *Bacillus thuringiensis, Bacillus thuringiensis* delta endotoxin, baculovirus, and entomopathogenic bacteria, virus and fungi.

**Table 1**

| SEQ ID NO | SPECIES | TYPE | LENGTH |
|---|---|---|---|
| 1 | Amaranthus hybridus | cDNAContig | 672 |
| 2 | Amaranthus lividus | cDNAContig | 458 |
| 3 | Amaranthus palmeri | gDNAContig | 5590 |
| 4 | Amaranthus palmeri | gDNAContig | 5515 |
| 5 | Amaranthus palmeri | cDNAContig | 1431 |
| 6 | Amaranthus palmeri | cDNAContig | 1431 |
| 7 | Amaranthus palmeri | cDNAContig | 1163 |
| 8 | Amaranthus rudis | cDNAContig | 1431 |
| 9 | Amaranthus rudis | gDNAContig | 1059 |
| 10 | Amaranthus rudis | gDNAContig | 792 |
| 11 | Amaranthus rudis | gDNAContig | 764 |
| 12 | Amaranthus rudis | gDNAContig | 755 |
| 13 | Amaranthus rudis | cDNAContig | 602 |
| 14 | Amaranthus rudis | gDNAContig | 584 |
| 15 | Amaranthus rudis | gDNAContig | 570 |
| 16 | Amaranthus rudis | cDNAContig | 411 |
| 17 | Amaranthus rudis | gDNAContig | 260 |
| 18 | Amaranthus viridis | cDNAContig | 1504 |
| 19 | Amaranthus viridis | cDNAContig | 655 |
| 20 | Ambrosia trifida | cDNAContig | 1413 |
| 21 | Ambrosia trifida | gDNAContig | 1328 |
| 22 | Ambrosia trifida | gDNAContig | 486 |
| 23 | Ambrosia trifida | cDNAContig | 486 |
| 24 | Ambrosia trifida | cDNAContig | 410 |
| 25 | Ambrosia trifida | gDNAContig | 381 |
| 26 | Ambrosia trifida | gDNAContig | 304 |
| 27 | Ambrosia trifida | gDNAContig | 255 |
| 28 | Ambrosia trifida | gDNAContig | 201 |
| 29 | Ambrosia trifida | cDNAContig | 101 |
| 30 | Conyza canadensis | gDNAContig | 5446 |
| 31 | Conyza canadensis | gDNAContig | 5446 |
| 32 | Conyza canadensis | cDNAContig | 1401 |
| 33 | Conyza canadensis | cDNAContig | 1262 |
| 34 | Euphorbia heterophylla | gDNAContig | 4643 |
| 35 | Euphorbia heterophylla | gDNAContig | 4556 |
| 36 | Euphorbia heterophylla | cDNAContig | 1425 |
| 37 | Euphorbia heterophylla | cDNAContig | 1425 |
| 38 | Euphorbia heterophylla | cDNAContig | 681 |
| 39 | Euphorbia heterophylla | gDNAContig | 444 |
| 40 | Euphorbia heterophylla | gDNAContig | 379 |
| 41 | Digitaria sanguinalis | gDNAContig | 4031 |
| 42 | Digitaria sanguinalis | cDNAContig | 1428 |
| 43 | Kochia scoparia | gDNAContig | 4007 |
| 44 | Kochia scoparia | gDNAContig | 3505 |
| 45 | Kochia scoparia | cDNAContig | 1446 |
| 47 | Lolium multiflorum | gDNAContig | 3920 |
| 48 | Lolium multiflorum | cDNAContig | 1467 |
| 49 | Lolium multiflorum | gDNAContig | 834 |
| 50 | Lolium multiflorum | cDNAContig | 834 |
| 51 | Lolium multiflorum | gDNAContig | 212 |
| 52 | Lolium multiflorum | cDNAContig | 210 |
| 53 | Lolium multiflorum | gDNAContig | 201 |
| 54 | Lolium multiflorum | cDNAContig | 150 |

**Table 2**

| SEQ ID NO | Species | Name \| Reference | Type | Start | End |
|---|---|---|---|---|---|
| 55 | Amaranthus hybridus | DHP_A1_1 | cDNAContig | 1 | 200 |
| 56 | Amaranthus hybridus | DHP_A1_1 | cDNAContig | 1 | 200 |
| 57 | Amaranthus hybridus | DHP_A1_2 | cDNAContig | 176 | 375 |
| 58 | Amaranthus hybridus | DHP_A1_2 | cDNAContig | 176 | 375 |
| 59 | Amaranthus hybridus | DHP_A1_3 | cDNAContig | 351 | 550 |
| 60 | Amaranthus hybridus | DHP_A1_3 | cDNAContig | 351 | 550 |
| 61 | Amaranthus lividus | DHP_A2_1 | cDNAContig | 1 | 200 |
| 62 | Amaranthus lividus | DHP_A2_1 | cDNAContig | 1 | 200 |
| 63 | Amaranthus lividus | DHP_A2_2 | cDNAContig | 176 | 375 |
| 64 | Amaranthus lividus | DHP_A2_2 | cDNAContig | 176 | 375 |
| 65 | Amaranthus palmeri | DHP_A4_1 | gDNAContig | 1 | 200 |
| 66 | Amaranthus palmeri | DHP_A4_1 | gDNAContig | 1 | 200 |
| 67 | Amaranthus palmeri | DHP_A4_2 | gDNAContig | 176 | 375 |
| 68 | Amaranthus palmeri | DHP_A4_2 | gDNAContig | 176 | 375 |
| 69 | Amaranthus palmeri | DHP_A4_3 | gDNAContig | 351 | 550 |
| 70 | Amaranthus palmeri | DHP_A4_3 | gDNAContig | 351 | 550 |
| 71 | Amaranthus palmeri | DHP_A4_4 | gDNAContig | 526 | 725 |
| 72 | Amaranthus palmeri | DHP_A4_4 | gDNAContig | 526 | 725 |
| 73 | Amaranthus palmeri | DHP_A4_5 | gDNAContig | 701 | 900 |
| 74 | Amaranthus palmeri | DHP_A4_5 | gDNAContig | 701 | 900 |
| 75 | Amaranthus palmeri | DHP_A4_6 | gDNAContig | 876 | 1075 |
| 76 | Amaranthus palmeri | DHP_A4_6 | gDNAContig | 876 | 1075 |
| 77 | Amaranthus palmeri | DHP_A4_7 | gDNAContig | 1051 | 1250 |
| 78 | Amaranthus palmeri | DHP_A4_7 | gDNAContig | 1051 | 1250 |
| 79 | Amaranthus palmeri | DHP_A4_8 | gDNAContig | 1226 | 1425 |
| 80 | Amaranthus palmeri | DHP_A4_8 | gDNAContig | 1226 | 1425 |
| 81 | Amaranthus palmeri | DHP_A4_9 | gDNAContig | 1401 | 1600 |
| 82 | Amaranthus palmeri | DHP_A4_9 | gDNAContig | 1401 | 1600 |
| 83 | Amaranthus palmeri | DHP_A4_10 | gDNAContig | 1576 | 1775 |
| 84 | Amaranthus palmeri | DHP_A4_10 | gDNAContig | 1576 | 1775 |
| 85 | Amaranthus palmeri | DHP_A4_11 | gDNAContig | 1751 | 1950 |
| 86 | Amaranthus palmeri | DHP_A4_11 | gDNAContig | 1751 | 1950 |
| 87 | Amaranthus palmeri | DHP_A4_12 | gDNAContig | 1926 | 2125 |
| 88 | Amaranthus palmeri | DHP_A4_12 | gDNAContig | 1926 | 2125 |
| 89 | Amaranthus palmeri | DHP_A4_13 | gDNAContig | 2101 | 2300 |
| 90 | Amaranthus palmeri | DHP_A4_13 | gDNAContig | 2101 | 2300 |
| 91 | Amaranthus palmeri | DHP_A4_14 | gDNAContig | 2276 | 2475 |
| 92 | Amaranthus palmeri | DHP_A4_14 | gDNAContig | 2276 | 2475 |
| 93 | Amaranthus palmeri | DHP_A4_15 | gDNAContig | 2451 | 2650 |
| 94 | Amaranthus palmeri | DHP_A4_15 | gDNAContig | 2451 | 2650 |
| 95 | Amaranthus palmeri | DHP_A4_16 | gDNAContig | 2626 | 2825 |
| 96 | Amaranthus palmeri | DHP_A4_16 | gDNAContig | 2626 | 2825 |
| 97 | Amaranthus palmeri | DHP_A4_17 | gDNAContig | 2801 | 3000 |
| 98 | Amaranthus palmeri | DHP_A4_17 | gDNAContig | 2801 | 3000 |
| 99 | Amaranthus palmeri | DHP_A4_18 | gDNAContig | 2976 | 3175 |
| 100 | Amaranthus palmeri | DHP_A4_18 | gDNAContig | 2976 | 3175 |
| 101 | Amaranthus palmeri | DHP_A4_19 | gDNAContig | 3151 | 3350 |
| 102 | Amaranthus palmeri | DHP_A4_19 | gDNAContig | 3151 | 3350 |
| 103 | Amaranthus palmeri | DHP_A4_20 | gDNAContig | 3326 | 3525 |
| 104 | Amaranthus palmeri | DHP_A4_20 | gDNAContig | 3326 | 3525 |
| 105 | Amaranthus palmeri | DHP_A4_21 | gDNAContig | 3501 | 3700 |
| 106 | Amaranthus palmeri | DHP_A4_21 | gDNAContig | 3501 | 3700 |
| 107 | Amaranthus palmeri | DHP_A4_22 | gDNAContig | 3676 | 3875 |
| 108 | Amaranthus palmeri | DHP_A4_22 | gDNAContig | 3676 | 3875 |
| 109 | Amaranthus palmeri | DHP_A4_23 | gDNAContig | 3851 | 4050 |
| 110 | Amaranthus palmeri | DHP_A4_23 | gDNAContig | 3851 | 4050 |
| 111 | Amaranthus palmeri | DHP_A4_24 | gDNAContig | 4026 | 4225 |
| 112 | Amaranthus palmeri | DHP_A4_24 | gDNAContig | 4026 | 4225 |
| 113 | Amaranthus palmeri | DHP_A4_25 | gDNAContig | 4201 | 4400 |
| 114 | Amaranthus palmeri | DHP_A4_25 | gDNAContig | 4201 | 4400 |
| 115 | Amaranthus palmeri | DHP_A4_26 | gDNAContig | 4376 | 4575 |
| 116 | Amaranthus palmeri | DHP_A4_26 | gDNAContig | 4376 | 4575 |
| 117 | Amaranthus palmeri | DHP_A4_27 | gDNAContig | 4551 | 4750 |
| 118 | Amaranthus palmeri | DHP_A4_27 | gDNAContig | 4551 | 4750 |
| 119 | Amaranthus palmeri | DHP_A4_28 | gDNAContig | 4726 | 4925 |
| 120 | Amaranthus palmeri | DHP_A4_28 | gDNAContig | 4726 | 4925 |
| 121 | Amaranthus palmeri | DHP_A4_29 | gDNAContig | 4901 | 5100 |
| 122 | Amaranthus palmeri | DHP_A4_29 | gDNAContig | 4901 | 5100 |
| 123 | Amaranthus palmeri | DHP_A4_30 | gDNAContig | 5076 | 5275 |
| 124 | Amaranthus palmeri | DHP_A4_30 | gDNAContig | 5076 | 5275 |
| 125 | Amaranthus palmeri | DHP_A4_31 | gDNAContig | 5251 | 5450 |
| 126 | Amaranthus palmeri | DHP_A4_31 | gDNAContig | 5251 | 5450 |
| 127 | Amaranthus palmeri | DHP_A7_1 | cDNAContig | 1 | 200 |
| 128 | Amaranthus palmeri | DHP_A7_1 | cDNAContig | 1 | 200 |
| 129 | Amaranthus palmeri | DHP_A7_2 | cDNAContig | 176 | 375 |
| 130 | Amaranthus palmeri | DHP_A7_2 | cDNAContig | 176 | 375 |
| 131 | Amaranthus palmeri | DHP_A7_3 | cDNAContig | 351 | 550 |
| 132 | Amaranthus palmeri | DHP_A7_3 | cDNAContig | 351 | 550 |
| 133 | Amaranthus palmeri | DHP_A7_4 | cDNAContig | 526 | 725 |
| 134 | Amaranthus palmeri | DHP_A7_4 | cDNAContig | 526 | 725 |
| 135 | Amaranthus palmeri | DHP_A7_5 | cDNAContig | 701 | 900 |
| 136 | Amaranthus palmeri | DHP_A7_5 | cDNAContig | 701 | 900 |
| 137 | Amaranthus palmeri | DHP_A7_6 | cDNAContig | 876 | 1075 |
| 138 | Amaranthus palmeri | DHP_A7_6 | cDNAContig | 876 | 1075 |
| 139 | Amaranthus palmeri | DHP_A5_1 | cDNAContig | 1 | 200 |
| 140 | Amaranthus palmeri | DHP_A5_1 | cDNAContig | 1 | 200 |
| 141 | Amaranthus palmeri | DHP_A5_2 | cDNAContig | 176 | 375 |
| 142 | Amaranthus palmeri | DHP_A5_2 | cDNAContig | 176 | 375 |
| 143 | Amaranthus palmeri | DHP_A5_3 | cDNAContig | 351 | 550 |
| 144 | Amaranthus palmeri | DHP_A5_3 | cDNAContig | 351 | 550 |
| 145 | Amaranthus palmeri | DHP_A5_4 | cDNAContig | 526 | 725 |
| 146 | Amaranthus palmeri | DHP_A5_4 | cDNAContig | 526 | 725 |
| 147 | Amaranthus palmeri | DHP_A5_5 | cDNAContig | 701 | 900 |
| 148 | Amaranthus palmeri | DHP_A5_5 | cDNAContig | 701 | 900 |
| 149 | Amaranthus palmeri | DHP_A5_6 | cDNAContig | 876 | 1075 |
| 150 | Amaranthus palmeri | DHP_A5_6 | cDNAContig | 876 | 1075 |
| 151 | Amaranthus palmeri | DHP_A5_7 | cDNAContig | 1051 | 1250 |
| 152 | Amaranthus palmeri | DHP_A5_7 | cDNAContig | 1051 | 1250 |
| 153 | Amaranthus palmeri | DHP_A5_8 | cDNAContig | 1226 | 1425 |
| 154 | Amaranthus palmeri | DHP_A5_8 | cDNAContig | 1226 | 1425 |
| 155 | Amaranthus palmeri | DHP_A6_1 | cDNAContig | 1 | 200 |
| 156 | Amaranthus palmeri | DHP_A6_1 | cDNAContig | 1 | 200 |
| 157 | Amaranthus palmeri | DHP_A6_2 | cDNAContig | 176 | 375 |
| 158 | Amaranthus palmeri | DHP_A6_2 | cDNAContig | 176 | 375 |
| 159 | Amaranthus palmeri | DHP_A6_3 | cDNAContig | 351 | 550 |
| 160 | Amaranthus palmeri | DHP_A6_3 | cDNAContig | 351 | 550 |
| 161 | Amaranthus palmeri | DHP_A6_4 | cDNAContig | 526 | 725 |
| 162 | Amaranthus palmeri | DHP_A6_4 | cDNAContig | 526 | 725 |
| 163 | Amaranthus palmeri | DHP_A6_5 | cDNAContig | 701 | 900 |
| 164 | Amaranthus palmeri | DHP_A6_5 | cDNAContig | 701 | 900 |
| 165 | Amaranthus palmeri | DHP_A6_6 | cDNAContig | 876 | 1075 |
| 166 | Amaranthus palmeri | DHP_A6_6 | cDNAContig | 876 | 1075 |
| 167 | Amaranthus palmeri | DHP_A6_7 | cDNAContig | 1051 | 1250 |
| 168 | Amaranthus palmeri | DHP_A6_7 | cDNAContig | 1051 | 1250 |
| 169 | Amaranthus palmeri | DHP_A6_8 | cDNAContig | 1226 | 1425 |
| 170 | Amaranthus palmeri | DHP_A6_8 | cDNAContig | 1226 | 1425 |
| 171 | Amaranthus palmeri | DHP_A3_1 | gDNAContig | 1 | 200 |
| 172 | Amaranthus palmeri | DHP_A3_1 | gDNAContig | 1 | 200 |
| 173 | Amaranthus palmeri | DHP_A3_2 | gDNAContig | 176 | 375 |
| 174 | Amaranthus palmeri | DHP_A3_2 | gDNAContig | 176 | 375 |
| 175 | Amaranthus palmeri | DHP_A3_3 | gDNAContig | 351 | 550 |
| 176 | Amaranthus palmeri | DHP_A3_3 | gDNAContig | 351 | 550 |
| 177 | Amaranthus palmeri | DHP_A3_4 | gDNAContig | 526 | 725 |
| 178 | Amaranthus palmeri | DHP_A3_4 | gDNAContig | 526 | 725 |
| 179 | Amaranthus palmeri | DHP_A3_5 | gDNAContig | 701 | 900 |
| 180 | Amaranthus palmeri | DHP_A3_5 | gDNAContig | 701 | 900 |
| 181 | Amaranthus palmeri | DHP_A3_6 | gDNAContig | 876 | 1075 |
| 182 | Amaranthus palmeri | DHP_A3_6 | gDNAContig | 876 | 1075 |
| 183 | Amaranthus palmeri | DHP_A3_7 | gDNAContig | 1051 | 1250 |
| 184 | Amaranthus palmeri | DHP_A3_7 | gDNAContig | 1051 | 1250 |
| 185 | Amaranthus palmeri | DHP_A3_8 | gDNAContig | 1226 | 1425 |
| 186 | Amaranthus palmeri | DHP_A3_8 | gDNAContig | 1226 | 1425 |
| 187 | Amaranthus palmeri | DHP_A3_9 | gDNAContig | 1401 | 1600 |
| 188 | Amaranthus palmeri | DHP_A3_9 | gDNAContig | 1401 | 1600 |
| 189 | Amaranthus palmeri | DHP_A3_10 | gDNAContig | 1576 | 1775 |
| 190 | Amaranthus palmeri | DHP_A3_10 | gDNAContig | 1576 | 1775 |
| 191 | Amaranthus palmeri | DHP_A3_11 | gDNAContig | 1751 | 1950 |
| 192 | Amaranthus palmeri | DHP_A3_11 | gDNAContig | 1751 | 1950 |
| 193 | Amaranthus palmeri | DHP_A3_12 | gDNAContig | 1926 | 2125 |
| 194 | Amaranthus palmeri | DHP_A3_12 | gDNAContig | 1926 | 2125 |
| 195 | Amaranthus palmeri | DHP_A3_13 | gDNAContig | 2101 | 2300 |
| 196 | Amaranthus palmeri | DHP_A3_13 | gDNAContig | 2101 | 2300 |
| 197 | Amaranthus palmeri | DHP_A3_14 | gDNAContig | 2276 | 2475 |
| 198 | Amaranthus palmeri | DHP_A3_14 | gDNAContig | 2276 | 2475 |
| 199 | Amaranthus palmeri | DHP_A3_15 | gDNAContig | 2451 | 2650 |
| 200 | Amaranthus palmeri | DHP_A3_15 | gDNAContig | 2451 | 2650 |
| 201 | Amaranthus palmeri | DHP_A3_16 | gDNAContig | 2626 | 2825 |
| 202 | Amaranthus palmeri | DHP_A3_16 | gDNAContig | 2626 | 2825 |
| 203 | Amaranthus palmeri | DHP_A3_17 | gDNAContig | 2801 | 3000 |
| 204 | Amaranthus palmeri | DHP_A3_17 | gDNAContig | 2801 | 3000 |
| 205 | Amaranthus palmeri | DHP_A3_18 | gDNAContig | 2976 | 3175 |
| 206 | Amaranthus palmeri | DHP_A3_18 | gDNAContig | 2976 | 3175 |
| 207 | Amaranthus palmeri | DHP_A3_19 | gDNAContig | 3151 | 3350 |
| 208 | Amaranthus palmeri | DHP_A3_19 | gDNAContig | 3151 | 3350 |
| 209 | Amaranthus palmeri | DHP_A3_20 | gDNAContig | 3326 | 3525 |
| 210 | Amaranthus palmeri | DHP_A3_20 | gDNAContig | 3326 | 3525 |
| 211 | Amaranthus palmeri | DHP_A3_21 | gDNAContig | 3501 | 3700 |
| 212 | Amaranthus palmeri | DHP_A3_21 | gDNAContig | 3501 | 3700 |
| 213 | Amaranthus palmeri | DHP_A3_22 | gDNAContig | 3676 | 3875 |
| 214 | Amaranthus palmeri | DHP_A3_22 | gDNAContig | 3676 | 3875 |
| 215 | Amaranthus palmeri | DHP_A3_23 | gDNAContig | 3851 | 4050 |
| 216 | Amaranthus palmeri | DHP_A3_23 | gDNAContig | 3851 | 4050 |
| 217 | Amaranthus palmeri | DHP_A3_24 | gDNAContig | 4026 | 4225 |
| 218 | Amaranthus palmeri | DHP_A3_24 | gDNAContig | 4026 | 4225 |
| 219 | Amaranthus palmeri | DHP_A3_25 | gDNAContig | 4201 | 4400 |
| 220 | Amaranthus palmeri | DHP_A3_25 | gDNAContig | 4201 | 4400 |
| 221 | Amaranthus palmeri | DHP_A3_26 | gDNAContig | 4376 | 4575 |
| 222 | Amaranthus palmeri | DHP_A3_26 | gDNAContig | 4376 | 4575 |
| 223 | Amaranthus palmeri | DHP_A3_27 | gDNAContig | 4551 | 4750 |
| 224 | Amaranthus palmeri | DHP_A3_27 | gDNAContig | 4551 | 4750 |
| 225 | Amaranthus palmeri | DHP_A3_28 | gDNAContig | 4726 | 4925 |
| 226 | Amaranthus palmeri | DHP_A3_28 | gDNAContig | 4726 | 4925 |
| 227 | Amaranthus palmeri | DHP_A3_29 | gDNAContig | 4901 | 5100 |
| 228 | Amaranthus palmeri | DHP_A3_29 | gDNAContig | 4901 | 5100 |
| 229 | Amaranthus palmeri | DHP_A3_30 | gDNAContig | 5076 | 5275 |
| 230 | Amaranthus palmeri | DHP_A3_30 | gDNAContig | 5076 | 5275 |
| 231 | Amaranthus palmeri | DHP_A3_31 | gDNAContig | 5251 | 5450 |
| 232 | Amaranthus palmeri | DHP_A3_31 | gDNAContig | 5251 | 5450 |
| 233 | Amaranthus rudis | DHP_A10_1 | gDNAContig | 1 | 200 |
| 234 | Amaranthus rudis | DHP_A10_1 | gDNAContig | 1 | 200 |
| 235 | Amaranthus rudis | DHP_A10_2 | gDNAContig | 176 | 375 |
| 236 | Amaranthus rudis | DHP_A10_2 | gDNAContig | 176 | 375 |
| 237 | Amaranthus rudis | DHP_A10_3 | gDNAContig | 351 | 550 |
| 238 | Amaranthus rudis | DHP_A10_3 | gDNAContig | 351 | 550 |
| 239 | Amaranthus rudis | DHP_A10_4 | gDNAContig | 526 | 725 |
| 240 | Amaranthus rudis | DHP_A10_4 | gDNAContig | 526 | 725 |
| 241 | Amaranthus rudis | DHP_A13_1 | cDNAContig | 1 | 200 |
| 242 | Amaranthus rudis | DHP_A13_1 | cDNAContig | 1 | 200 |
| 243 | Amaranthus rudis | DHP_A13_2 | cDNAContig | 176 | 375 |
| 244 | Amaranthus rudis | DHP_A13_2 | cDNAContig | 176 | 375 |
| 245 | Amaranthus rudis | DHP_A13_3 | cDNAContig | 351 | 550 |
| 246 | Amaranthus rudis | DHP_A13_3 | cDNAContig | 351 | 550 |
| 247 | Amaranthus rudis | DHP_A14_1 | gDNAContig | 1 | 200 |
| 248 | Amaranthus rudis | DHP_A14_1 | gDNAContig | 1 | 200 |
| 249 | Amaranthus rudis | DHP_A14_2 | gDNAContig | 176 | 375 |
| 250 | Amaranthus rudis | DHP_A14_2 | gDNAContig | 176 | 375 |
| 251 | Amaranthus rudis | DHP_A14_3 | gDNAContig | 351 | 550 |
| 252 | Amaranthus rudis | DHP_A14_3 | gDNAContig | 351 | 550 |
| 253 | Amaranthus rudis | DHP_A17_1 | gDNAContig | 1 | 200 |
| 254 | Amaranthus rudis | DHP_A17_1 | gDNAContig | 1 | 200 |
| 255 | Amaranthus rudis | DHP_A16_1 | cDNAContig | 1 | 200 |
| 256 | Amaranthus rudis | DHP_A16_1 | cDNAContig | 1 | 200 |
| 257 | Amaranthus rudis | DHP_A16_2 | cDNAContig | 176 | 375 |
| 258 | Amaranthus rudis | DHP_A16_2 | cDNAContig | 176 | 375 |
| 259 | Amaranthus rudis | DHP_A8_1 | cDNAContig | 1 | 200 |
| 260 | Amaranthus rudis | DHP_A8_1 | cDNAContig | 1 | 200 |
| 261 | Amaranthus rudis | DHP_A8_2 | cDNAContig | 176 | 375 |
| 262 | Amaranthus rudis | DHP_A8_2 | cDNAContig | 176 | 375 |
| 263 | Amaranthus rudis | DHP_A8_3 | cDNAContig | 351 | 550 |
| 264 | Amaranthus rudis | DHP_A8_3 | cDNAContig | 351 | 550 |
| 265 | Amaranthus rudis | DHP_A8_4 | cDNAContig | 526 | 725 |
| 266 | Amaranthus rudis | DHP_A8_4 | cDNAContig | 526 | 725 |
| 267 | Amaranthus rudis | DHP_A8_5 | cDNAContig | 701 | 900 |
| 268 | Amaranthus rudis | DHP_A8_5 | cDNAContig | 701 | 900 |
| 269 | Amaranthus rudis | DHP_A8_6 | cDNAContig | 876 | 1075 |
| 270 | Amaranthus rudis | DHP_A8_6 | cDNAContig | 876 | 1075 |
| 271 | Amaranthus rudis | DHP_A8_7 | cDNAContig | 1051 | 1250 |
| 272 | Amaranthus rudis | DHP_A8_7 | cDNAContig | 1051 | 1250 |
| 273 | Amaranthus rudis | DHP_A8_8 | cDNAContig | 1226 | 1425 |
| 274 | Amaranthus rudis | DHP_A8_8 | cDNAContig | 1226 | 1425 |
| 275 | Amaranthus rudis | DHP_A12_1 | gDNAContig | 1 | 200 |
| 276 | Amaranthus rudis | DHP_A12_1 | gDNAContig | 1 | 200 |
| 277 | Amaranthus rudis | DHP_A12_2 | gDNAContig | 176 | 375 |
| 278 | Amaranthus rudis | DHP_A12_2 | gDNAContig | 176 | 375 |
| 279 | Amaranthus rudis | DHP_A12_3 | gDNAContig | 351 | 550 |
| 280 | Amaranthus rudis | DHP_A12_3 | gDNAContig | 351 | 550 |
| 281 | Amaranthus rudis | DHP_A12_4 | gDNAContig | 526 | 725 |
| 282 | Amaranthus rudis | DHP_A12_4 | gDNAContig | 526 | 725 |
| 283 | Amaranthus rudis | DHP_A15_1 | gDNAContig | 1 | 200 |
| 284 | Amaranthus rudis | DHP_A15_1 | gDNAContig | 1 | 200 |
| 285 | Amaranthus rudis | DHP_A15_2 | gDNAContig | 176 | 375 |
| 286 | Amaranthus rudis | DHP_A15_2 | gDNAContig | 176 | 375 |
| 287 | Amaranthus rudis | DHP_A15_3 | gDNAContig | 351 | 550 |
| 288 | Amaranthus rudis | DHP_A15_3 | gDNAContig | 351 | 550 |
| 289 | Amaranthus rudis | DHP_A11_1 | gDNAContig | 1 | 200 |
| 290 | Amaranthus rudis | DHP_A11_1 | gDNAContig | 1 | 200 |
| 291 | Amaranthus rudis | DHP_A11_2 | gDNAContig | 176 | 375 |
| 292 | Amaranthus rudis | DHP_A11_2 | gDNAContig | 176 | 375 |
| 293 | Amaranthus rudis | DHP_A11_3 | gDNAContig | 351 | 550 |
| 294 | Amaranthus rudis | DHP_A11_3 | gDNAContig | 351 | 550 |
| 295 | Amaranthus rudis | DHP_A11_4 | gDNAContig | 526 | 725 |
| 296 | Amaranthus rudis | DHP_A11_4 | gDNAContig | 526 | 725 |
| 297 | Amaranthus rudis | DHP_A9_1 | gDNAContig | 1 | 200 |
| 298 | Amaranthus rudis | DHP_A9_1 | gDNAContig | 1 | 200 |
| 299 | Amaranthus rudis | DHP_A9_2 | gDNAContig | 176 | 375 |
| 300 | Amaranthus rudis | DHP_A9_2 | gDNAContig | 176 | 375 |
| 301 | Amaranthus rudis | DHP_A9_3 | gDNAContig | 351 | 550 |
| 302 | Amaranthus rudis | DHP_A9_3 | gDNAContig | 351 | 550 |
| 303 | Amaranthus rudis | DHP_A9_4 | gDNAContig | 526 | 725 |
| 304 | Amaranthus rudis | DHP_A9_4 | gDNAContig | 526 | 725 |
| 305 | Amaranthus rudis | DHP_A9_5 | gDNAContig | 701 | 900 |
| 306 | Amaranthus rudis | DHP_A9_5 | gDNAContig | 701 | 900 |
| 307 | Amaranthus viridis | DHP_A19_1 | cDNAContig | 1 | 200 |
| 308 | Amaranthus viridis | DHP_A19_1 | cDNAContig | 1 | 200 |
| 309 | Amaranthus viridis | DHP_A19_2 | cDNAContig | 176 | 375 |
| 310 | Amaranthus viridis | DHP_A19_2 | cDNAContig | 176 | 375 |
| 311 | Amaranthus viridis | DHP_A19_3 | cDNAContig | 351 | 550 |
| 312 | Amaranthus viridis | DHP_A19_3 | cDNAContig | 351 | 550 |
| 313 | Amaranthus viridis | DHP_A18_1 | cDNAContig | 1 | 200 |
| 314 | Amaranthus viridis | DHP_A18_1 | cDNAContig | 1 | 200 |
| 315 | Amaranthus viridis | DHP_A18_2 | cDNAContig | 176 | 375 |
| 316 | Amaranthus viridis | DHP_A18_2 | cDNAContig | 176 | 375 |
| 317 | Amaranthus viridis | DHP_A18_3 | cDNAContig | 351 | 550 |
| 318 | Amaranthus viridis | DHP_A18_3 | cDNAContig | 351 | 550 |
| 319 | Amaranthus viridis | DHP_A18_4 | cDNAContig | 526 | 725 |
| 320 | Amaranthus viridis | DHP_A18_4 | cDNAContig | 526 | 725 |
| 321 | Amaranthus viridis | DHP_A18_5 | cDNAContig | 701 | 900 |
| 322 | Amaranthus viridis | DHP_A18_5 | cDNAContig | 701 | 900 |
| 323 | Amaranthus viridis | DHP_A18_6 | cDNAContig | 876 | 1075 |
| 324 | Amaranthus viridis | DHP_A18_6 | cDNAContig | 876 | 1075 |
| 325 | Amaranthus viridis | DHP_A18_7 | cDNAContig | 1051 | 1250 |
| 326 | Amaranthus viridis | DHP_A18_7 | cDNAContig | 1051 | 1250 |
| 327 | Amaranthus viridis | DHP_A18_8 | cDNAContig | 1226 | 1425 |
| 328 | Amaranthus viridis | DHP_A18_8 | cDNAContig | 1226 | 1425 |
| 329 | Ambrosia trifida | DHP_A21_1 | gDNAContig | 1 | 200 |
| 330 | Ambrosia trifida | DHP_A21_1 | gDNAContig | 1 | 200 |
| 331 | Ambrosia trifida | DHP_A21_2 | gDNAContig | 176 | 375 |
| 332 | Ambrosia trifida | DHP_A21_2 | gDNAContig | 176 | 375 |
| 333 | Ambrosia trifida | DHP_A21_3 | gDNAContig | 351 | 550 |
| 334 | Ambrosia trifida | DHP_A21_3 | gDNAContig | 351 | 550 |
| 335 | Ambrosia trifida | DHP_A21_4 | gDNAContig | 526 | 725 |
| 336 | Ambrosia trifida | DHP_A21_4 | gDNAContig | 526 | 725 |
| 337 | Ambrosia trifida | DHP_A21_5 | gDNAContig | 701 | 900 |
| 338 | Ambrosia trifida | DHP_A21_5 | gDNAContig | 701 | 900 |
| 339 | Ambrosia trifida | DHP_A21_6 | gDNAContig | 876 | 1075 |
| 340 | Ambrosia trifida | DHP_A21_6 | gDNAContig | 876 | 1075 |
| 341 | Ambrosia trifida | DHP_A21_7 | gDNAContig | 1051 | 1250 |
| 342 | Ambrosia trifida | DHP_A21_7 | gDNAContig | 1051 | 1250 |
| 343 | Ambrosia trifida | DHP_A28_1 | gDNAContig | 1 | 200 |
| 344 | Ambrosia trifida | DHP_A28_1 | gDNAContig | 1 | 200 |
| 345 | Ambrosia trifida | DHP_A26_1 | gDNAContig | 1 | 200 |
| 346 | Ambrosia trifida | DHP_A26_1 | gDNAContig | 1 | 200 |
| 347 | Ambrosia trifida | DHP_A24_1 | cDNAContig | 1 | 200 |
| 348 | Ambrosia trifida | DHP_A24_1 | cDNAContig | 1 | 200 |
| 349 | Ambrosia trifida | DHP_A24_2 | cDNAContig | 176 | 375 |
| 350 | Ambrosia trifida | DHP_A24_2 | cDNAContig | 176 | 375 |
| 351 | Ambrosia trifida | DHP_A27_1 | gDNAContig | 1 | 200 |
| 352 | Ambrosia trifida | DHP_A27_1 | gDNAContig | 1 | 200 |
| 353 | Ambrosia trifida | DHP_A23_1 | cDNAContig | 1 | 200 |
| 354 | Ambrosia trifida | DHP_A23_1 | cDNAContig | 1 | 200 |
| 355 | Ambrosia trifida | DHP_A23_2 | cDNAContig | 176 | 375 |
| 356 | Ambrosia trifida | DHP_A23_2 | cDNAContig | 176 | 375 |
| 357 | Ambrosia trifida | DHP_A29_1 | cDNAContig | 1 | 101 |
| 358 | Ambrosia trifida | DHP_A29_1 | cDNAContig | 1 | 101 |
| 359 | Ambrosia trifida | DHP_A20_1 | cDNAContig | 1 | 200 |
| 360 | Ambrosia trifida | DHP_A20_1 | cDNAContig | 1 | 200 |
| 361 | Ambrosia trifida | DHP_A20_2 | cDNAContig | 176 | 375 |
| 362 | Ambrosia trifida | DHP_A20_2 | cDNAContig | 176 | 375 |
| 363 | Ambrosia trifida | DHP_A20_3 | cDNAContig | 351 | 550 |
| 364 | Ambrosia trifida | DHP_A20_3 | cDNAContig | 351 | 550 |
| 365 | Ambrosia trifida | DHP_A20_4 | cDNAContig | 526 | 725 |
| 366 | Ambrosia trifida | DHP_A20_4 | cDNAContig | 526 | 725 |
| 367 | Ambrosia trifida | DHP_A20_5 | cDNAContig | 701 | 900 |
| 368 | Ambrosia trifida | DHP_A20_5 | cDNAContig | 701 | 900 |
| 369 | Ambrosia trifida | DHP_A20_6 | cDNAContig | 876 | 1075 |
| 370 | Ambrosia trifida | DHP_A20_6 | cDNAContig | 876 | 1075 |
| 371 | Ambrosia trifida | DHP_A20_7 | cDNAContig | 1051 | 1250 |
| 372 | Ambrosia trifida | DHP_A20_7 | cDNAContig | 1051 | 1250 |
| 373 | Ambrosia trifida | DHP_A25_1 | gDNAContig | 1 | 200 |
| 374 | Ambrosia trifida | DHP_A25_1 | gDNAContig | 1 | 200 |
| 375 | Ambrosia trifida | DHP_A25_2 | gDNAContig | 176 | 375 |
| 376 | Ambrosia trifida | DHP_A25_2 | gDNAContig | 176 | 375 |
| 377 | Conyza canadensis | DHP_A33_1 | cDNAContig | 1 | 200 |
| 378 | Conyza canadensis | DHP_A33_1 | cDNAContig | 1 | 200 |
| 379 | Conyza canadensis | DHP_A33_2 | cDNAContig | 176 | 375 |
| 380 | Conyza canadensis | DHP_A33_2 | cDNAContig | 176 | 375 |
| 381 | Conyza canadensis | DHP_A33_3 | cDNAContig | 351 | 550 |
| 382 | Conyza canadensis | DHP_A33_3 | cDNAContig | 351 | 550 |
| 383 | Conyza canadensis | DHP_A33_4 | cDNAContig | 526 | 725 |
| 384 | Conyza canadensis | DHP_A33_4 | cDNAContig | 526 | 725 |
| 385 | Conyza canadensis | DHP_A33_5 | cDNAContig | 701 | 900 |
| 386 | Conyza canadensis | DHP_A33_5 | cDNAContig | 701 | 900 |
| 387 | Conyza canadensis | DHP_A33_6 | cDNAContig | 876 | 1075 |
| 388 | Conyza canadensis | DHP_A33_6 | cDNAContig | 876 | 1075 |
| 389 | Conyza canadensis | DHP_A33_7 | cDNAContig | 1051 | 1250 |
| 390 | Conyza canadensis | DHP_A33_7 | cDNAContig | 1051 | 1250 |
| 391 | Conyza canadensis | DHP_A32_1 | cDNAContig | 1 | 200 |
| 392 | Conyza canadensis | DHP_A32_1 | cDNAContig | 1 | 200 |
| 393 | Conyza canadensis | DHP_A32_2 | cDNAContig | 176 | 375 |
| 394 | Conyza canadensis | DHP_A32_2 | cDNAContig | 176 | 375 |
| 395 | Conyza canadensis | DHP_A32_3 | cDNAContig | 351 | 550 |
| 396 | Conyza canadensis | DHP_A32_3 | cDNAContig | 351 | 550 |
| 397 | Conyza canadensis | DHP_A32_4 | cDNAContig | 526 | 725 |
| 398 | Conyza canadensis | DHP_A32_4 | cDNAContig | 526 | 725 |
| 399 | Conyza canadensis | DHP_A32_5 | cDNAContig | 701 | 900 |
| 400 | Conyza canadensis | DHP_A32_5 | cDNAContig | 701 | 900 |
| 401 | Conyza canadensis | DHP_A32_6 | cDNAContig | 876 | 1075 |
| 402 | Conyza canadensis | DHP_A32_6 | cDNAContig | 876 | 1075 |
| 403 | Conyza canadensis | DHP_A32_7 | cDNAContig | 1051 | 1250 |
| 404 | Conyza canadensis | DHP_A32_7 | cDNAContig | 1051 | 1250 |
| 405 | Conyza canadensis | DHP_A30_1 | gDNAContig | 1 | 200 |
| 406 | Conyza canadensis | DHP_A30_1 | gDNAContig | 1 | 200 |
| 407 | Conyza canadensis | DHP_A30_2 | gDNAContig | 176 | 375 |
| 408 | Conyza canadensis | DHP_A30_2 | gDNAContig | 176 | 375 |
| 409 | Conyza canadensis | DHP_A30_3 | gDNAContig | 351 | 550 |
| 410 | Conyza canadensis | DHP_A30_3 | gDNAContig | 351 | 550 |
| 411 | Conyza canadensis | DHP_A30_4 | gDNAContig | 526 | 725 |
| 412 | Conyza canadensis | DHP_A30_4 | gDNAContig | 526 | 725 |
| 413 | Conyza canadensis | DHP_A30_5 | gDNAContig | 701 | 900 |
| 414 | Conyza canadensis | DHP_A30_5 | gDNAContig | 701 | 900 |
| 415 | Conyza canadensis | DHP_A30_6 | gDNAContig | 876 | 1075 |
| 416 | Conyza canadensis | DHP_A30_6 | gDNAContig | 876 | 1075 |
| 417 | Conyza canadensis | DHP_A30_7 | gDNAContig | 1051 | 1250 |
| 418 | Conyza canadensis | DHP_A30_7 | gDNAContig | 1051 | 1250 |
| 419 | Conyza canadensis | DHP_A30_8 | gDNAContig | 1226 | 1425 |
| 420 | Conyza canadensis | DHP_A30_8 | gDNAContig | 1226 | 1425 |
| 421 | Conyza canadensis | DHP_A30_9 | gDNAContig | 1401 | 1600 |
| 422 | Conyza canadensis | DHP_A30_9 | gDNAContig | 1401 | 1600 |
| 423 | Conyza canadensis | DHP_A30_10 | gDNAContig | 1576 | 1775 |
| 424 | Conyza canadensis | DHP_A30_10 | gDNAContig | 1576 | 1775 |
| 425 | Conyza canadensis | DHP_A30_11 | gDNAContig | 1751 | 1950 |
| 426 | Conyza canadensis | DHP_A30_11 | gDNAContig | 1751 | 1950 |
| 427 | Conyza canadensis | DHP_A30_12 | gDNAContig | 1926 | 2125 |
| 428 | Conyza canadensis | DHP_A30_12 | gDNAContig | 1926 | 2125 |
| 429 | Conyza canadensis | DHP_A30_13 | gDNAContig | 2101 | 2300 |
| 430 | Conyza canadensis | DHP_A30_13 | gDNAContig | 2101 | 2300 |
| 431 | Conyza canadensis | DHP_A30_14 | gDNAContig | 2276 | 2475 |
| 432 | Conyza canadensis | DHP_A30_14 | gDNAContig | 2276 | 2475 |
| 433 | Conyza canadensis | DHP_A30_15 | gDNAContig | 2451 | 2650 |
| 434 | Conyza canadensis | DHP_A30_15 | gDNAContig | 2451 | 2650 |
| 435 | Conyza canadensis | DHP_A30_16 | gDNAContig | 2626 | 2825 |
| 436 | Conyza canadensis | DHP_A30_16 | gDNAContig | 2626 | 2825 |
| 437 | Conyza canadensis | DHP_A30_17 | gDNAContig | 2801 | 3000 |
| 438 | Conyza canadensis | DHP_A30_17 | gDNAContig | 2801 | 3000 |
| 439 | Conyza canadensis | DHP_A30_18 | gDNAContig | 2976 | 3175 |
| 440 | Conyza canadensis | DHP_A30_18 | gDNAContig | 2976 | 3175 |
| 441 | Conyza canadensis | DHP_A30_19 | gDNAContig | 3151 | 3350 |
| 442 | Conyza canadensis | DHP_A30_19 | gDNAContig | 3151 | 3350 |
| 443 | Conyza canadensis | DHP_A30_20 | gDNAContig | 3326 | 3525 |
| 444 | Conyza canadensis | DHP_A30_20 | gDNAContig | 3326 | 3525 |
| 445 | Conyza canadensis | DHP_A30_21 | gDNAContig | 3501 | 3700 |
| 446 | Conyza canadensis | DHP_A30_21 | gDNAContig | 3501 | 3700 |
| 447 | Conyza canadensis | DHP_A30_22 | gDNAContig | 3676 | 3875 |
| 448 | Conyza canadensis | DHP_A30_22 | gDNAContig | 3676 | 3875 |
| 449 | Conyza canadensis | DHP_A30_23 | gDNAContig | 3851 | 4050 |
| 450 | Conyza canadensis | DHP_A30_23 | gDNAContig | 3851 | 4050 |
| 451 | Conyza canadensis | DHP_A30_24 | gDNAContig | 4026 | 4225 |
| 452 | Conyza canadensis | DHP_A30_24 | gDNAContig | 4026 | 4225 |
| 453 | Conyza canadensis | DHP_A30_25 | gDNAContig | 4201 | 4400 |
| 454 | Conyza canadensis | DHP_A30_25 | gDNAContig | 4201 | 4400 |
| 455 | Conyza canadensis | DHP_A30_26 | gDNAContig | 4376 | 4575 |
| 456 | Conyza canadensis | DHP_A30_26 | gDNAContig | 4376 | 4575 |
| 457 | Conyza canadensis | DHP_A30_27 | gDNAContig | 4551 | 4750 |
| 458 | Conyza canadensis | DHP_A30_27 | gDNAContig | 4551 | 4750 |
| 459 | Conyza canadensis | DHP_A30_28 | gDNAContig | 4726 | 4925 |
| 460 | Conyza canadensis | DHP_A30_28 | gDNAContig | 4726 | 4925 |
| 461 | Conyza canadensis | DHP_A30_29 | gDNAContig | 4901 | 5100 |
| 462 | Conyza canadensis | DHP_A30_29 | gDNAContig | 4901 | 5100 |
| 463 | Conyza canadensis | DHP_A30_30 | gDNAContig | 5076 | 5275 |
| 464 | Conyza canadensis | DHP_A30_30 | gDNAContig | 5076 | 5275 |
| 465 | Conyza canadensis | DHP_A31_1 | gDNAContig | 1 | 200 |
| 466 | Conyza canadensis | DHP_A31_1 | gDNAContig | 1 | 200 |
| 467 | Conyza canadensis | DHP_A31_2 | gDNAContig | 176 | 375 |
| 468 | Conyza canadensis | DHP_A31_2 | gDNAContig | 176 | 375 |
| 469 | Conyza canadensis | DHP_A31_3 | gDNAContig | 351 | 550 |
| 470 | Conyza canadensis | DHP_A31_3 | gDNAContig | 351 | 550 |
| 471 | Conyza canadensis | DHP_A31_4 | gDNAContig | 526 | 725 |
| 472 | Conyza canadensis | DHP_A31_4 | gDNAContig | 526 | 725 |
| 473 | Conyza canadensis | DHP_A31_5 | gDNAContig | 701 | 900 |
| 474 | Conyza canadensis | DHP_A31_5 | gDNAContig | 701 | 900 |
| 475 | Conyza canadensis | DHP_A31_6 | gDNAContig | 876 | 1075 |
| 476 | Conyza canadensis | DHP_A31_6 | gDNAContig | 876 | 1075 |
| 477 | Conyza canadensis | DHP_A31_7 | gDNAContig | 1051 | 1250 |
| 478 | Conyza canadensis | DHP_A31_7 | gDNAContig | 1051 | 1250 |
| 479 | Conyza canadensis | DHP_A31_8 | gDNAContig | 1226 | 1425 |
| 480 | Conyza canadensis | DHP_A31_8 | gDNAContig | 1226 | 1425 |
| 481 | Conyza canadensis | DHP_A31_9 | gDNAContig | 1401 | 1600 |
| 482 | Conyza canadensis | DHP_A31_9 | gDNAContig | 1401 | 1600 |
| 483 | Conyza canadensis | DHP_A31_10 | gDNAContig | 1576 | 1775 |
| 484 | Conyza canadensis | DHP_A31_10 | gDNAContig | 1576 | 1775 |
| 485 | Conyza canadensis | DHP_A31_11 | gDNAContig | 1751 | 1950 |
| 486 | Conyza canadensis | DHP_A31_11 | gDNAContig | 1751 | 1950 |
| 487 | Conyza canadensis | DHP_A31_12 | gDNAContig | 1926 | 2125 |
| 488 | Conyza canadensis | DHP_A31_12 | gDNAContig | 1926 | 2125 |
| 489 | Conyza canadensis | DHP_A31_13 | gDNAContig | 2101 | 2300 |
| 490 | Conyza canadensis | DHP_A31_13 | gDNAContig | 2101 | 2300 |
| 491 | Conyza canadensis | DHP_A31_14 | gDNAContig | 2276 | 2475 |
| 492 | Conyza canadensis | DHP_A31_14 | gDNAContig | 2276 | 2475 |
| 493 | Conyza canadensis | DHP_A31_15 | gDNAContig | 2451 | 2650 |
| 494 | Conyza canadensis | DHP_A31_15 | gDNAContig | 2451 | 2650 |
| 495 | Conyza canadensis | DHP_A31_16 | gDNAContig | 2626 | 2825 |
| 496 | Conyza canadensis | DHP_A31_16 | gDNAContig | 2626 | 2825 |
| 497 | Conyza canadensis | DHP_A31_17 | gDNAContig | 2801 | 3000 |
| 498 | Conyza canadensis | DHP_A31_17 | gDNAContig | 2801 | 3000 |
| 499 | Conyza canadensis | DHP_A31_18 | gDNAContig | 2976 | 3175 |
| 500 | Conyza canadensis | DHP_A31_18 | gDNAContig | 2976 | 3175 |
| 501 | Conyza canadensis | DHP_A31_19 | gDNAContig | 3151 | 3350 |
| 502 | Conyza canadensis | DHP_A31_19 | gDNAContig | 3151 | 3350 |
| 503 | Conyza canadensis | DHP_A31_20 | gDNAContig | 3326 | 3525 |
| 504 | Conyza canadensis | DHP_A31_20 | gDNAContig | 3326 | 3525 |
| 505 | Conyza canadensis | DHP_A31_21 | gDNAContig | 3501 | 3700 |
| 506 | Conyza canadensis | DHP_A31_21 | gDNAContig | 3501 | 3700 |
| 507 | Conyza canadensis | DHP_A31_22 | gDNAContig | 3676 | 3875 |
| 508 | Conyza canadensis | DHP_A31_22 | gDNAContig | 3676 | 3875 |
| 509 | Conyza canadensis | DHP_A31_23 | gDNAContig | 3851 | 4050 |
| 510 | Conyza canadensis | DHP_A31_23 | gDNAContig | 3851 | 4050 |
| 511 | Conyza canadensis | DHP_A31_24 | gDNAContig | 4026 | 4225 |
| 512 | Conyza canadensis | DHP_A31_24 | gDNAContig | 4026 | 4225 |
| 513 | Conyza canadensis | DHP_A31_25 | gDNAContig | 4201 | 4400 |
| 514 | Conyza canadensis | DHP_A31_25 | gDNAContig | 4201 | 4400 |
| 515 | Conyza canadensis | DHP_A31_26 | gDNAContig | 4376 | 4575 |
| 516 | Conyza canadensis | DHP_A31_26 | gDNAContig | 4376 | 4575 |
| 517 | Conyza canadensis | DHP_A31_27 | gDNAContig | 4551 | 4750 |
| 518 | Conyza canadensis | DHP_A31_27 | gDNAContig | 4551 | 4750 |
| 519 | Conyza canadensis | DHP_A31_28 | gDNAContig | 4726 | 4925 |
| 520 | Conyza canadensis | DHP_A31_28 | gDNAContig | 4726 | 4925 |
| 521 | Conyza canadensis | DHP_A31_29 | gDNAContig | 4901 | 5100 |
| 522 | Conyza canadensis | DHP_A31_29 | gDNAContig | 4901 | 5100 |
| 523 | Conyza canadensis | DHP_A31_30 | gDNAContig | 5076 | 5275 |
| 524 | Conyza canadensis | DHP_A31_30 | gDNAContig | 5076 | 5275 |
| 525 | Digitaria sanguinalis | DHP_A41_1 | gDNAContig | 1 | 200 |
| 526 | Digitaria sanguinalis | DHP_A41_1 | gDNAContig | 1 | 200 |
| 527 | Digitaria sanguinalis | DHP_A41_2 | gDNAContig | 176 | 375 |
| 528 | Digitaria sanguinalis | DHP_A41_2 | gDNAContig | 176 | 375 |
| 529 | Digitaria sanguinalis | DHP_A41_3 | gDNAContig | 351 | 550 |
| 530 | Digitaria sanguinalis | DHP_A41_3 | gDNAContig | 351 | 550 |
| 531 | Digitaria sanguinalis | DHP_A41_4 | gDNAContig | 526 | 725 |
| 532 | Digitaria sanguinalis | DHP_A41_4 | gDNAContig | 526 | 725 |
| 533 | Digitaria sanguinalis | DHP_A41_5 | gDNAContig | 701 | 900 |
| 534 | Digitaria sanguinalis | DHP_A41_5 | gDNAContig | 701 | 900 |
| 535 | Digitaria sanguinalis | DHP_A41_6 | gDNAContig | 876 | 1075 |
| 536 | Digitaria sanguinalis | DHP_A41_6 | gDNAContig | 876 | 1075 |
| 537 | Digitaria sanguinalis | DHP_A41_7 | gDNAContig | 1051 | 1250 |
| 538 | Digitaria sanguinalis | DHP_A41_7 | gDNAContig | 1051 | 1250 |
| 539 | Digitaria sanguinalis | DHP_A41_8 | gDNAContig | 1226 | 1425 |
| 540 | Digitaria sanguinalis | DHP_A41_8 | gDNAContig | 1226 | 1425 |
| 541 | Digitaria sanguinalis | DHP_A41_9 | gDNAContig | 1401 | 1600 |
| 542 | Digitaria sanguinalis | DHP_A41_9 | gDNAContig | 1401 | 1600 |
| 543 | Digitaria sanguinalis | DHP_A41_10 | gDNAContig | 1576 | 1775 |
| 544 | Digitaria sanguinalis | DHP_A41_10 | gDNAContig | 1576 | 1775 |
| 545 | Digitaria sanguinalis | DHP_A41_11 | gDNAContig | 1751 | 1950 |
| 546 | Digitaria sanguinalis | DHP_A41_11 | gDNAContig | 1751 | 1950 |
| 547 | Digitaria sanguinalis | DHP_A41_12 | gDNAContig | 1926 | 2125 |
| 548 | Digitaria sanguinalis | DHP_A41_12 | gDNAContig | 1926 | 2125 |
| 549 | Digitaria sanguinalis | DHP_A41_13 | gDNAContig | 2101 | 2300 |
| 550 | Digitaria sanguinalis | DHP_A41_13 | gDNAContig | 2101 | 2300 |
| 551 | Digitaria sanguinalis | DHP_A41_14 | gDNAContig | 2276 | 2475 |
| 552 | Digitaria sanguinalis | DHP_A41_14 | gDNAContig | 2276 | 2475 |
| 553 | Digitaria sanguinalis | DHP_A41_15 | gDNAContig | 2451 | 2650 |
| 554 | Digitaria sanguinalis | DHP_A41_15 | gDNAContig | 2451 | 2650 |
| 555 | Digitaria sanguinalis | DHP_A41_16 | gDNAContig | 2626 | 2825 |
| 556 | Digitaria sanguinalis | DHP_A41_16 | gDNAContig | 2626 | 2825 |
| 557 | Digitaria sanguinalis | DHP_A41_17 | gDNAContig | 2801 | 3000 |
| 558 | Digitaria sanguinalis | DHP_A41_17 | gDNAContig | 2801 | 3000 |
| 559 | Digitaria sanguinalis | DHP_A41_18 | gDNAContig | 2976 | 3175 |
| 560 | Digitaria sanguinalis | DHP_A41_18 | gDNAContig | 2976 | 3175 |
| 561 | Digitaria sanguinalis | DHP_A41_19 | gDNAContig | 3151 | 3350 |
| 562 | Digitaria sanguinalis | DHP_A41_19 | gDNAContig | 3151 | 3350 |
| 563 | Digitaria sanguinalis | DHP_A41_20 | gDNAContig | 3326 | 3525 |
| 564 | Digitaria sanguinalis | DHP_A41_20 | gDNAContig | 3326 | 3525 |
| 565 | Digitaria sanguinalis | DHP_A41_21 | gDNAContig | 3501 | 3700 |
| 566 | Digitaria sanguinalis | DHP_A41_21 | gDNAContig | 3501 | 3700 |
| 567 | Digitaria sanguinalis | DHP_A41_22 | gDNAContig | 3676 | 3875 |
| 568 | Digitaria sanguinalis | DHP_A41_22 | gDNAContig | 3676 | 3875 |
| 569 | Digitaria sanguinalis | DHP_A42_1 | cDNAContig | 1 | 200 |
| 570 | Digitaria sanguinalis | DHP_A42_1 | cDNAContig | 1 | 200 |
| 571 | Digitaria sanguinalis | DHP_A42_2 | cDNAContig | 176 | 375 |
| 572 | Digitaria sanguinalis | DHP_A42_2 | cDNAContig | 176 | 375 |
| 573 | Digitaria sanguinalis | DHP_A42_3 | cDNAContig | 351 | 550 |
| 574 | Digitaria sanguinalis | DHP_A42_3 | cDNAContig | 351 | 550 |
| 575 | Digitaria sanguinalis | DHP_A42_4 | cDNAContig | 526 | 725 |
| 576 | Digitaria sanguinalis | DHP_A42_4 | cDNAContig | 526 | 725 |
| 577 | Digitaria sanguinalis | DHP_A42_5 | cDNAContig | 701 | 900 |
| 578 | Digitaria sanguinalis | DHP_A42_5 | cDNAContig | 701 | 900 |
| 579 | Digitaria sanguinalis | DHP_A42_6 | cDNAContig | 876 | 1075 |
| 580 | Digitaria sanguinalis | DHP_A42_6 | cDNAContig | 876 | 1075 |
| 581 | Digitaria sanguinalis | DHP_A42_7 | cDNAContig | 1051 | 1250 |
| 582 | Digitaria sanguinalis | DHP_A42_7 | cDNAContig | 1051 | 1250 |
| 583 | Digitaria sanguinalis | DHP_A42_8 | cDNAContig | 1226 | 1425 |
| 584 | Digitaria sanguinalis | DHP_A42_8 | cDNAContig | 1226 | 1425 |
| 585 | Euphorbia heterophylla | DHP_A35_1 | gDNAContig | 1 | 200 |
| 586 | Euphorbia heterophylla | DHP_A35_1 | gDNAContig | 1 | 200 |
| 587 | Euphorbia heterophylla | DHP_A35_2 | gDNAContig | 176 | 375 |
| 588 | Euphorbia heterophylla | DHP_A35_2 | gDNAContig | 176 | 375 |
| 589 | Euphorbia heterophylla | DHP_A35_3 | gDNAContig | 351 | 550 |
| 590 | Euphorbia heterophylla | DHP_A35_3 | gDNAContig | 351 | 550 |
| 591 | Euphorbia heterophylla | DHP_A35_4 | gDNAContig | 526 | 725 |
| 592 | Euphorbia heterophylla | DHP_A35_4 | gDNAContig | 526 | 725 |
| 593 | Euphorbia heterophylla | DHP_A35_5 | gDNAContig | 701 | 900 |
| 594 | Euphorbia heterophylla | DHP_A35_5 | gDNAContig | 701 | 900 |
| 595 | Euphorbia heterophylla | DHP_A35_6 | gDNAContig | 876 | 1075 |
| 596 | Euphorbia heterophylla | DHP_A35_6 | gDNAContig | 876 | 1075 |
| 597 | Euphorbia heterophylla | DHP_A35_7 | gDNAContig | 1051 | 1250 |
| 598 | Euphorbia heterophylla | DHP_A35_7 | gDNAContig | 1051 | 1250 |
| 599 | Euphorbia heterophylla | DHP_A35_8 | gDNAContig | 1226 | 1425 |
| 600 | Euphorbia heterophylla | DHP_A35_8 | gDNAContig | 1226 | 1425 |
| 601 | Euphorbia heterophylla | DHP_A35_9 | gDNAContig | 1401 | 1600 |
| 602 | Euphorbia heterophylla | DHP_A35_9 | gDNAContig | 1401 | 1600 |
| 603 | Euphorbia heterophylla | DHP_A35_10 | gDNAContig | 1576 | 1775 |
| 604 | Euphorbia heterophylla | DHP_A35_10 | gDNAContig | 1576 | 1775 |
| 605 | Euphorbia heterophylla | DHP_A35_11 | gDNAContig | 1751 | 1950 |
| 606 | Euphorbia heterophylla | DHP_A35_11 | gDNAContig | 1751 | 1950 |
| 607 | Euphorbia heterophylla | DHP_A35_12 | gDNAContig | 1926 | 2125 |
| 608 | Euphorbia heterophylla | DHP_A35_12 | gDNAContig | 1926 | 2125 |
| 609 | Euphorbia heterophylla | DHP_A35_13 | gDNAContig | 2101 | 2300 |
| 610 | Euphorbia heterophylla | DHP_A35_13 | gDNAContig | 2101 | 2300 |
| 611 | Euphorbia heterophylla | DHP_A35_14 | gDNAContig | 2276 | 2475 |
| 612 | Euphorbia heterophylla | DHP_A35_14 | gDNAContig | 2276 | 2475 |
| 613 | Euphorbia heterophylla | DHP_A35_15 | gDNAContig | 2451 | 2650 |
| 614 | Euphorbia heterophylla | DHP_A35_15 | gDNAContig | 2451 | 2650 |
| 615 | Euphorbia heterophylla | DHP_A35_16 | gDNAContig | 2626 | 2825 |
| 616 | Euphorbia heterophylla | DHP_A35_16 | gDNAContig | 2626 | 2825 |
| 617 | Euphorbia heterophylla | DHP_A35_17 | gDNAContig | 2801 | 3000 |
| 618 | Euphorbia heterophylla | DHP_A35_17 | gDNAContig | 2801 | 3000 |
| 619 | Euphorbia heterophylla | DHP_A35_18 | gDNAContig | 2976 | 3175 |
| 620 | Euphorbia heterophylla | DHP_A35_18 | gDNAContig | 2976 | 3175 |
| 621 | Euphorbia heterophylla | DHP_A35_19 | gDNAContig | 3151 | 3350 |
| 622 | Euphorbia heterophylla | DHP_A35_19 | gDNAContig | 3151 | 3350 |
| 623 | Euphorbia heterophylla | DHP_A35_20 | gDNAContig | 3326 | 3525 |
| 624 | Euphorbia heterophylla | DHP_A35_20 | gDNAContig | 3326 | 3525 |
| 625 | Euphorbia heterophylla | DHP_A35_21 | gDNAContig | 3501 | 3700 |
| 626 | Euphorbia heterophylla | DHP_A35_21 | gDNAContig | 3501 | 3700 |
| 627 | Euphorbia heterophylla | DHP_A35_22 | gDNAContig | 3676 | 3875 |
| 628 | Euphorbia heterophylla | DHP_A35_22 | gDNAContig | 3676 | 3875 |
| 629 | Euphorbia heterophylla | DHP_A35_23 | gDNAContig | 3851 | 4050 |
| 630 | Euphorbia heterophylla | DHP_A35_23 | gDNAContig | 3851 | 4050 |
| 631 | Euphorbia heterophylla | DHP_A35_24 | gDNAContig | 4026 | 4225 |
| 632 | Euphorbia heterophylla | DHP_A35_24 | gDNAContig | 4026 | 4225 |
| 633 | Euphorbia heterophylla | DHP_A35_25 | gDNAContig | 4201 | 4400 |
| 634 | Euphorbia heterophylla | DHP_A35_25 | gDNAContig | 4201 | 4400 |
| 635 | Euphorbia heterophylla | DHP_A37_1 | cDNAContig | 1 | 200 |
| 636 | Euphorbia heterophylla | DHP_A37_1 | cDNAContig | 1 | 200 |
| 637 | Euphorbia heterophylla | DHP_A37_2 | cDNAContig | 176 | 375 |
| 638 | Euphorbia heterophylla | DHP_A37_2 | cDNAContig | 176 | 375 |
| 639 | Euphorbia heterophylla | DHP_A37_3 | cDNAContig | 351 | 550 |
| 640 | Euphorbia heterophylla | DHP_A37_3 | cDNAContig | 351 | 550 |
| 641 | Euphorbia heterophylla | DHP_A37_4 | cDNAContig | 526 | 725 |
| 642 | Euphorbia heterophylla | DHP_A37_4 | cDNAContig | 526 | 725 |
| 643 | Euphorbia heterophylla | DHP_A37_5 | cDNAContig | 701 | 900 |
| 644 | Euphorbia heterophylla | DHP_A37_5 | cDNAContig | 701 | 900 |
| 645 | Euphorbia heterophylla | DHP_A37_6 | cDNAContig | 876 | 1075 |
| 646 | Euphorbia heterophylla | DHP_A37_6 | cDNAContig | 876 | 1075 |
| 647 | Euphorbia heterophylla | DHP_A37_7 | cDNAContig | 1051 | 1250 |
| 648 | Euphorbia heterophylla | DHP_A37_7 | cDNAContig | 1051 | 1250 |
| 649 | Euphorbia heterophylla | DHP_A37_8 | cDNAContig | 1226 | 1425 |
| 650 | Euphorbia heterophylla | DHP_A37_8 | cDNAContig | 1226 | 1425 |
| 651 | Euphorbia heterophylla | DHP_A36_1 | cDNAContig | 1 | 200 |
| 652 | Euphorbia heterophylla | DHP_A36_1 | cDNAContig | 1 | 200 |
| 653 | Euphorbia heterophylla | DHP_A36_2 | cDNAContig | 176 | 375 |
| 654 | Euphorbia heterophylla | DHP_A36_2 | cDNAContig | 176 | 375 |
| 655 | Euphorbia heterophylla | DHP_A36_3 | cDNAContig | 351 | 550 |
| 656 | Euphorbia heterophylla | DHP_A36_3 | cDNAContig | 351 | 550 |
| 657 | Euphorbia heterophylla | DHP_A36_4 | cDNAContig | 526 | 725 |
| 658 | Euphorbia heterophylla | DHP_A36_4 | cDNAContig | 526 | 725 |
| 659 | Euphorbia heterophylla | DHP_A36_5 | cDNAContig | 701 | 900 |
| 660 | Euphorbia heterophylla | DHP_A36_5 | cDNAContig | 701 | 900 |
| 661 | Euphorbia heterophylla | DHP_A36_6 | cDNAContig | 876 | 1075 |
| 662 | Euphorbia heterophylla | DHP_A36_6 | cDNAContig | 876 | 1075 |
| 663 | Euphorbia heterophylla | DHP_A36_7 | cDNAContig | 1051 | 1250 |
| 664 | Euphorbia heterophylla | DHP_A36_7 | cDNAContig | 1051 | 1250 |
| 665 | Euphorbia heterophylla | DHP_A36_8 | cDNAContig | 1226 | 1425 |
| 666 | Euphorbia heterophylla | DHP_A36_8 | cDNAContig | 1226 | 1425 |
| 667 | Euphorbia heterophylla | DHP_A40_1 | gDNAContig | 1 | 200 |
| 668 | Euphorbia heterophylla | DHP_A40_1 | gDNAContig | 1 | 200 |
| 669 | Euphorbia heterophylla | DHP_A40_2 | gDNAContig | 176 | 375 |
| 670 | Euphorbia heterophylla | DHP_A40_2 | gDNAContig | 176 | 375 |
| 671 | Euphorbia heterophylla | DHP_A39_1 | gDNAContig | 1 | 200 |
| 672 | Euphorbia heterophylla | DHP_A39_1 | gDNAContig | 1 | 200 |
| 673 | Euphorbia heterophylla | DHP_A39_2 | gDNAContig | 176 | 375 |
| 674 | Euphorbia heterophylla | DHP_A39_2 | gDNAContig | 176 | 375 |
| 675 | Euphorbia heterophylla | DHP_A38_1 | cDNAContig | 1 | 200 |
| 676 | Euphorbia heterophylla | DHP_A38_1 | cDNAContig | 1 | 200 |
| 677 | Euphorbia heterophylla | DHP_A38_2 | cDNAContig | 176 | 375 |
| 678 | Euphorbia heterophylla | DHP_A38_2 | cDNAContig | 176 | 375 |
| 679 | Euphorbia heterophylla | DHP_A38_3 | cDNAContig | 351 | 550 |
| 680 | Euphorbia heterophylla | DHP_A38_3 | cDNAContig | 351 | 550 |
| 681 | Euphorbia heterophylla | DHP_A34_1 | gDNAContig | 1 | 200 |
| 682 | Euphorbia heterophylla | DHP_A34_1 | gDNAContig | 1 | 200 |
| 683 | Euphorbia heterophylla | DHP_A34_2 | gDNAContig | 176 | 375 |
| 684 | Euphorbia heterophylla | DHP_A34_2 | gDNAContig | 176 | 375 |
| 685 | Euphorbia heterophylla | DHP_A34_3 | gDNAContig | 351 | 550 |
| 686 | Euphorbia heterophylla | DHP_A34_3 | gDNAContig | 351 | 550 |
| 687 | Euphorbia heterophylla | DHP_A34_4 | gDNAContig | 526 | 725 |
| 688 | Euphorbia heterophylla | DHP_A34_4 | gDNAContig | 526 | 725 |
| 689 | Euphorbia heterophylla | DHP_A34_5 | gDNAContig | 701 | 900 |
| 690 | Euphorbia heterophylla | DHP_A34_5 | gDNAContig | 701 | 900 |
| 691 | Euphorbia heterophylla | DHP_A34_6 | gDNAContig | 876 | 1075 |
| 692 | Euphorbia heterophylla | DHP_A34_6 | gDNAContig | 876 | 1075 |
| 693 | Euphorbia heterophylla | DHP_A34_7 | gDNAContig | 1051 | 1250 |
| 694 | Euphorbia heterophylla | DHP_A34_7 | gDNAContig | 1051 | 1250 |
| 695 | Euphorbia heterophylla | DHP_A34_8 | gDNAContig | 1226 | 1425 |
| 696 | Euphorbia heterophylla | DHP_A34_8 | gDNAContig | 1226 | 1425 |
| 697 | Euphorbia heterophylla | DHP_A34_9 | gDNAContig | 1401 | 1600 |
| 698 | Euphorbia heterophylla | DHP_A34_9 | gDNAContig | 1401 | 1600 |
| 699 | Euphorbia heterophylla | DHP_A34_10 | gDNAContig | 1576 | 1775 |
| 700 | Euphorbia heterophylla | DHP_A34_10 | gDNAContig | 1576 | 1775 |
| 701 | Euphorbia heterophylla | DHP_A34_11 | gDNAContig | 1751 | 1950 |
| 702 | Euphorbia heterophylla | DHP_A34_11 | gDNAContig | 1751 | 1950 |
| 703 | Euphorbia heterophylla | DHP_A34_12 | gDNAContig | 1926 | 2125 |
| 704 | Euphorbia heterophylla | DHP_A34_12 | gDNAContig | 1926 | 2125 |
| 705 | Euphorbia heterophylla | DHP_A34_13 | gDNAContig | 2101 | 2300 |
| 706 | Euphorbia heterophylla | DHP_A34_13 | gDNAContig | 2101 | 2300 |
| 707 | Euphorbia heterophylla | DHP_A34_14 | gDNAContig | 2276 | 2475 |
| 708 | Euphorbia heterophylla | DHP_A34_14 | gDNAContig | 2276 | 2475 |
| 709 | Euphorbia heterophylla | DHP_A34_15 | gDNAContig | 2451 | 2650 |
| 710 | Euphorbia heterophylla | DHP_A34_15 | gDNAContig | 2451 | 2650 |
| 711 | Euphorbia heterophylla | DHP_A34_16 | gDNAContig | 2626 | 2825 |
| 712 | Euphorbia heterophylla | DHP_A34_16 | gDNAContig | 2626 | 2825 |
| 713 | Euphorbia heterophylla | DHP_A34_17 | gDNAContig | 2801 | 3000 |
| 714 | Euphorbia heterophylla | DHP_A34_17 | gDNAContig | 2801 | 3000 |
| 715 | Euphorbia heterophylla | DHP_A34_18 | gDNAContig | 2976 | 3175 |
| 716 | Euphorbia heterophylla | DHP_A34_18 | gDNAContig | 2976 | 3175 |
| 717 | Euphorbia heterophylla | DHP_A34_19 | gDNAContig | 3151 | 3350 |
| 718 | Euphorbia heterophylla | DHP_A34_19 | gDNAContig | 3151 | 3350 |
| 719 | Euphorbia heterophylla | DHP_A34_20 | gDNAContig | 3326 | 3525 |
| 720 | Euphorbia heterophylla | DHP_A34_20 | gDNAContig | 3326 | 3525 |
| 721 | Euphorbia heterophylla | DHP_A34_21 | gDNAContig | 3501 | 3700 |
| 722 | Euphorbia heterophylla | DHP_A34_21 | gDNAContig | 3501 | 3700 |
| 723 | Euphorbia heterophylla | DHP_A34_22 | gDNAContig | 3676 | 3875 |
| 724 | Euphorbia heterophylla | DHP_A34_22 | gDNAContig | 3676 | 3875 |
| 725 | Euphorbia heterophylla | DHP_A34_23 | gDNAContig | 3851 | 4050 |
| 726 | Euphorbia heterophylla | DHP_A34_23 | gDNAContig | 3851 | 4050 |
| 727 | Euphorbia heterophylla | DHP_A34_24 | gDNAContig | 4026 | 4225 |
| 728 | Euphorbia heterophylla | DHP_A34_24 | gDNAContig | 4026 | 4225 |
| 729 | Euphorbia heterophylla | DHP_A34_25 | gDNAContig | 4201 | 4400 |
| 730 | Euphorbia heterophylla | DHP_A34_25 | gDNAContig | 4201 | 4400 |
| 731 | Euphorbia heterophylla | DHP_A34_26 | gDNAContig | 4376 | 4575 |
| 732 | Euphorbia heterophylla | DHP_A34_26 | gDNAContig | 4376 | 4575 |
| 733 | Kochia scoparia | DHP_A43_1 | gDNAContig | 1 | 200 |
| 734 | Kochia scoparia | DHP_A43_1 | gDNAContig | 1 | 200 |
| 735 | Kochia scoparia | DHP_A43_2 | gDNAContig | 176 | 375 |
| 736 | Kochia scoparia | DHP_A43_2 | gDNAContig | 176 | 375 |
| 737 | Kochia scoparia | DHP_A43_3 | gDNAContig | 351 | 550 |
| 738 | Kochia scoparia | DHP_A43_3 | gDNAContig | 351 | 550 |
| 739 | Kochia scoparia | DHP_A43_4 | gDNAContig | 526 | 725 |
| 740 | Kochia scoparia | DHP_A43_4 | gDNAContig | 526 | 725 |
| 741 | Kochia scoparia | DHP_A43_5 | gDNAContig | 701 | 900 |
| 742 | Kochia scoparia | DHP_A43_5 | gDNAContig | 701 | 900 |
| 743 | Kochia scoparia | DHP_A43_6 | gDNAContig | 876 | 1075 |
| 744 | Kochia scoparia | DHP_A43_6 | gDNAContig | 876 | 1075 |
| 745 | Kochia scoparia | DHP_A43_7 | gDNAContig | 1051 | 1250 |
| 746 | Kochia scoparia | DHP_A43_7 | gDNAContig | 1051 | 1250 |
| 747 | Kochia scoparia | DHP_A43_8 | gDNAContig | 1226 | 1425 |
| 748 | Kochia scoparia | DHP_A43_8 | gDNAContig | 1226 | 1425 |
| 749 | Kochia scoparia | DHP_A43_9 | gDNAContig | 1401 | 1600 |
| 750 | Kochia scoparia | DHP_A43_9 | gDNAContig | 1401 | 1600 |
| 751 | Kochia scoparia | DHP_A43_10 | gDNAContig | 1576 | 1775 |
| 752 | Kochia scoparia | DHP_A43_10 | gDNAContig | 1576 | 1775 |
| 753 | Kochia scoparia | DHP_A43_11 | gDNAContig | 1751 | 1950 |
| 754 | Kochia scoparia | DHP_A43_11 | gDNAContig | 1751 | 1950 |
| 755 | Kochia scoparia | DHP_A43_12 | gDNAContig | 1926 | 2125 |
| 756 | Kochia scoparia | DHP_A43_12 | gDNAContig | 1926 | 2125 |
| 757 | Kochia scoparia | DHP_A43_13 | gDNAContig | 2101 | 2300 |
| 758 | Kochia scoparia | DHP_A43_13 | gDNAContig | 2101 | 2300 |
| 759 | Kochia scoparia | DHP_A43_14 | gDNAContig | 2276 | 2475 |
| 760 | Kochia scoparia | DHP_A43_14 | gDNAContig | 2276 | 2475 |
| 761 | Kochia scoparia | DHP_A43_15 | gDNAContig | 2451 | 2650 |
| 762 | Kochia scoparia | DHP_A43_15 | gDNAContig | 2451 | 2650 |
| 763 | Kochia scoparia | DHP_A43_16 | gDNAContig | 2626 | 2825 |
| 764 | Kochia scoparia | DHP_A43_16 | gDNAContig | 2626 | 2825 |
| 765 | Kochia scoparia | DHP_A43_17 | gDNAContig | 2801 | 3000 |
| 766 | Kochia scoparia | DHP_A43_17 | gDNAContig | 2801 | 3000 |
| 767 | Kochia scoparia | DHP_A43_18 | gDNAContig | 2976 | 3175 |
| 768 | Kochia scoparia | DHP_A43_18 | gDNAContig | 2976 | 3175 |
| 769 | Kochia scoparia | DHP_A43_19 | gDNAContig | 3151 | 3350 |
| 770 | Kochia scoparia | DHP_A43_19 | gDNAContig | 3151 | 3350 |
| 771 | Kochia scoparia | DHP_A43_20 | gDNAContig | 3326 | 3525 |
| 772 | Kochia scoparia | DHP_A43_20 | gDNAContig | 3326 | 3525 |
| 773 | Kochia scoparia | DHP_A43_21 | gDNAContig | 3501 | 3700 |
| 774 | Kochia scoparia | DHP_A43_21 | gDNAContig | 3501 | 3700 |
| 775 | Kochia scoparia | DHP_A43_22 | gDNAContig | 3676 | 3875 |
| 776 | Kochia scoparia | DHP_A43_22 | gDNAContig | 3676 | 3875 |
| 777 | Kochia scoparia | DHP_A46_1 | cDNAContig | 1 | 200 |
| 778 | Kochia scoparia | DHP_A46_1 | cDNAContig | 1 | 200 |
| 779 | Kochia scoparia | DHP_A46_2 | cDNAContig | 176 | 375 |
| 780 | Kochia scoparia | DHP_A46_2 | cDNAContig | 176 | 375 |
| 781 | Kochia scoparia | DHP_A46_3 | cDNAContig | 351 | 550 |
| 782 | Kochia scoparia | DHP_A46_3 | cDNAContig | 351 | 550 |
| 783 | Kochia scoparia | DHP_A46_4 | cDNAContig | 526 | 725 |
| 784 | Kochia scoparia | DHP_A46_4 | cDNAContig | 526 | 725 |
| 785 | Kochia scoparia | DHP_A46_5 | cDNAContig | 701 | 900 |
| 786 | Kochia scoparia | DHP_A46_5 | cDNAContig | 701 | 900 |
| 787 | Kochia scoparia | DHP_A46_6 | cDNAContig | 876 | 1075 |
| 788 | Kochia scoparia | DHP_A46_6 | cDNAContig | 876 | 1075 |
| 789 | Kochia scoparia | DHP_A46_7 | cDNAContig | 1051 | 1250 |
| 790 | Kochia scoparia | DHP_A46_7 | cDNAContig | 1051 | 1250 |
| 791 | Kochia scoparia | DHP_A46_8 | cDNAContig | 1226 | 1425 |
| 792 | Kochia scoparia | DHP_A46_8 | cDNAContig | 1226 | 1425 |
| 793 | Kochia scoparia | DHP_A44_1 | gDNAContig | 1 | 200 |
| 794 | Kochia scoparia | DHP_A44_1 | gDNAContig | 1 | 200 |
| 795 | Kochia scoparia | DHP_A44_2 | gDNAContig | 176 | 375 |
| 796 | Kochia scoparia | DHP_A44_2 | gDNAContig | 176 | 375 |
| 797 | Kochia scoparia | DHP_A44_3 | gDNAContig | 351 | 550 |
| 798 | Kochia scoparia | DHP_A44_3 | gDNAContig | 351 | 550 |
| 799 | Kochia scoparia | DHP_A44_4 | gDNAContig | 526 | 725 |
| 800 | Kochia scoparia | DHP_A44_4 | gDNAContig | 526 | 725 |
| 801 | Kochia scoparia | DHP_A44_5 | gDNAContig | 701 | 900 |
| 802 | Kochia scoparia | DHP_A44_5 | gDNAContig | 701 | 900 |
| 803 | Kochia scoparia | DHP_A44_6 | gDNAContig | 876 | 1075 |
| 804 | Kochia scoparia | DHP_A44_6 | gDNAContig | 876 | 1075 |
| 805 | Kochia scoparia | DHP_A44_7 | gDNAContig | 1051 | 1250 |
| 806 | Kochia scoparia | DHP_A44_7 | gDNAContig | 1051 | 1250 |
| 807 | Kochia scoparia | DHP_A44_8 | gDNAContig | 1226 | 1425 |
| 808 | Kochia scoparia | DHP_A44_8 | gDNAContig | 1226 | 1425 |
| 809 | Kochia scoparia | DHP_A44_9 | gDNAContig | 1401 | 1600 |
| 810 | Kochia scoparia | DHP_A44_9 | gDNAContig | 1401 | 1600 |
| 811 | Kochia scoparia | DHP_A44_10 | gDNAContig | 1576 | 1775 |
| 812 | Kochia scoparia | DHP_A44_10 | gDNAContig | 1576 | 1775 |
| 813 | Kochia scoparia | DHP_A44_11 | gDNAContig | 1751 | 1950 |
| 814 | Kochia scoparia | DHP_A44_11 | gDNAContig | 1751 | 1950 |
| 815 | Kochia scoparia | DHP_A44_12 | gDNAContig | 1926 | 2125 |
| 816 | Kochia scoparia | DHP_A44_12 | gDNAContig | 1926 | 2125 |
| 817 | Kochia scoparia | DHP_A44_13 | gDNAContig | 2101 | 2300 |
| 818 | Kochia scoparia | DHP_A44_13 | gDNAContig | 2101 | 2300 |
| 819 | Kochia scoparia | DHP_A44_14 | gDNAContig | 2276 | 2475 |
| 820 | Kochia scoparia | DHP_A44_14 | gDNAContig | 2276 | 2475 |
| 821 | Kochia scoparia | DHP_A44_15 | gDNAContig | 2451 | 2650 |
| 822 | Kochia scoparia | DHP_A44_15 | gDNAContig | 2451 | 2650 |
| 823 | Kochia scoparia | DHP_A44_16 | gDNAContig | 2626 | 2825 |
| 824 | Kochia scoparia | DHP_A44_16 | gDNAContig | 2626 | 2825 |
| 825 | Kochia scoparia | DHP_A44_17 | gDNAContig | 2801 | 3000 |
| 826 | Kochia scoparia | DHP_A44_17 | gDNAContig | 2801 | 3000 |
| 827 | Kochia scoparia | DHP_A44_18 | gDNAContig | 2976 | 3175 |
| 828 | Kochia scoparia | DHP_A44_18 | gDNAContig | 2976 | 3175 |
| 829 | Kochia scoparia | DHP_A44_19 | gDNAContig | 3151 | 3350 |
| 830 | Kochia scoparia | DHP_A44_19 | gDNAContig | 3151 | 3350 |
| 831 | Lolium multiflorum | DHP_A47_1 | gDNAContig | 1 | 200 |
| 832 | Lolium multiflorum | DHP_A47_1 | gDNAContig | 1 | 200 |
| 833 | Lolium multiflorum | DHP_A47_2 | gDNAContig | 176 | 375 |
| 834 | Lolium multiflorum | DHP_A47_2 | gDNAContig | 176 | 375 |
| 835 | Lolium multiflorum | DHP_A47_3 | gDNAContig | 351 | 550 |
| 836 | Lolium multiflorum | DHP_A47_3 | gDNAContig | 351 | 550 |
| 837 | Lolium multiflorum | DHP_A47_4 | gDNAContig | 526 | 725 |
| 838 | Lolium multiflorum | DHP_A47_4 | gDNAContig | 526 | 725 |
| 839 | Lolium multiflorum | DHP_A47_5 | gDNAContig | 701 | 900 |
| 840 | Lolium multiflorum | DHP_A47_5 | gDNAContig | 701 | 900 |
| 841 | Lolium multiflorum | DHP_A47_6 | gDNAContig | 876 | 1075 |
| 842 | Lolium multiflorum | DHP_A47_6 | gDNAContig | 876 | 1075 |
| 843 | Lolium multiflorum | DHP_A47_7 | gDNAContig | 1051 | 1250 |
| 844 | Lolium multiflorum | DHP_A47_7 | gDNAContig | 1051 | 1250 |
| 845 | Lolium multiflorum | DHP_A47_8 | gDNAContig | 1226 | 1425 |
| 846 | Lolium multiflorum | DHP_A47_8 | gDNAContig | 1226 | 1425 |
| 847 | Lolium multiflorum | DHP_A47_9 | gDNAContig | 1401 | 1600 |
| 848 | Lolium multiflorum | DHP_A47_9 | gDNAContig | 1401 | 1600 |
| 849 | Lolium multiflorum | DHP_A47_10 | gDNAContig | 1576 | 1775 |
| 850 | Lolium multiflorum | DHP_A47_10 | gDNAContig | 1576 | 1775 |
| 851 | Lolium multiflorum | DHP_A47_11 | gDNAContig | 1751 | 1950 |
| 852 | Lolium multiflorum | DHP_A47_11 | gDNAContig | 1751 | 1950 |
| 853 | Lolium multiflorum | DHP_A47_12 | gDNAContig | 1926 | 2125 |
| 854 | Lolium multiflorum | DHP_A47_12 | gDNAContig | 1926 | 2125 |
| 855 | Lolium multiflorum | DHP_A47_13 | gDNAContig | 2101 | 2300 |
| 856 | Lolium multiflorum | DHP_A47_13 | gDNAContig | 2101 | 2300 |
| 857 | Lolium multiflorum | DHP_A47_14 | gDNAContig | 2276 | 2475 |
| 858 | Lolium multiflorum | DHP_A47_14 | gDNAContig | 2276 | 2475 |
| 859 | Lolium multiflorum | DHP_A47_15 | gDNAContig | 2451 | 2650 |
| 860 | Lolium multiflorum | DHP_A47_15 | gDNAContig | 2451 | 2650 |
| 861 | Lolium multiflorum | DHP_A47_16 | gDNAContig | 2626 | 2825 |
| 862 | Lolium multiflorum | DHP_A47_16 | gDNAContig | 2626 | 2825 |
| 863 | Lolium multiflorum | DHP_A47_17 | gDNAContig | 2801 | 3000 |
| 864 | Lolium multiflorum | DHP_A47_17 | gDNAContig | 2801 | 3000 |
| 865 | Lolium multiflorum | DHP_A47_18 | gDNAContig | 2976 | 3175 |
| 866 | Lolium multiflorum | DHP_A47_18 | gDNAContig | 2976 | 3175 |
| 867 | Lolium multiflorum | DHP_A47_19 | gDNAContig | 3151 | 3350 |
| 868 | Lolium multiflorum | DHP_A47_19 | gDNAContig | 3151 | 3350 |
| 869 | Lolium multiflorum | DHP_A47_20 | gDNAContig | 3326 | 3525 |
| 870 | Lolium multiflorum | DHP_A47_20 | gDNAContig | 3326 | 3525 |
| 871 | Lolium multiflorum | DHP_A47_21 | gDNAContig | 3501 | 3700 |
| 872 | Lolium multiflorum | DHP_A47_21 | gDNAContig | 3501 | 3700 |
| 873 | Lolium multiflorum | DHP_A47_22 | gDNAContig | 3676 | 3875 |
| 874 | Lolium multiflorum | DHP_A47_22 | gDNAContig | 3676 | 3875 |
| 875 | Lolium multiflorum | DHP_A48_1 | cDNAContig | 1 | 200 |
| 876 | Lolium multiflorum | DHP_A48_1 | cDNAContig | 1 | 200 |
| 877 | Lolium multiflorum | DHP_A48_2 | cDNAContig | 176 | 375 |
| 878 | Lolium multiflorum | DHP_A48_2 | cDNAContig | 176 | 375 |
| 879 | Lolium multiflorum | DHP_A48_3 | cDNAContig | 351 | 550 |
| 880 | Lolium multiflorum | DHP_A48_3 | cDNAContig | 351 | 550 |
| 881 | Lolium multiflorum | DHP_A48_4 | cDNAContig | 526 | 725 |
| 882 | Lolium multiflorum | DHP_A48_4 | cDNAContig | 526 | 725 |
| 883 | Lolium multiflorum | DHP_A48_5 | cDNAContig | 701 | 900 |
| 884 | Lolium multiflorum | DHP_A48_5 | cDNAContig | 701 | 900 |
| 885 | Lolium multiflorum | DHP_A48_6 | cDNAContig | 876 | 1075 |
| 886 | Lolium multiflorum | DHP_A48_6 | cDNAContig | 876 | 1075 |
| 887 | Lolium multiflorum | DHP_A48_7 | cDNAContig | 1051 | 1250 |
| 888 | Lolium multiflorum | DHP_A48_7 | cDNAContig | 1051 | 1250 |
| 889 | Lolium multiflorum | DHP_A48_8 | cDNAContig | 1226 | 1425 |
| 890 | Lolium multiflorum | DHP_A48_8 | cDNAContig | 1226 | 1425 |
| 891 | Lolium multiflorum | DHP_A51_1 | gDNAContig | 1 | 200 |
| 892 | Lolium multiflorum | DHP_A51_1 | gDNAContig | 1 | 200 |
| 893 | Lolium multiflorum | DHP_A50_1 | cDNAContig | 1 | 200 |
| 894 | Lolium multiflorum | DHP_A50_1 | cDNAContig | 1 | 200 |
| 895 | Lolium multiflorum | DHP_A50_2 | cDNAContig | 176 | 375 |
| 896 | Lolium multiflorum | DHP_A50_2 | cDNAContig | 176 | 375 |
| 897 | Lolium multiflorum | DHP_A50_3 | cDNAContig | 351 | 550 |
| 898 | Lolium multiflorum | DHP_A50_3 | cDNAContig | 351 | 550 |
| 899 | Lolium multiflorum | DHP_A50_4 | cDNAContig | 526 | 725 |
| 900 | Lolium multiflorum | DHP_A50_4 | cDNAContig | 526 | 725 |
| 901 | Lolium multiflorum | DHP_A53_1 | gDNAContig | 1 | 200 |
| 902 | Lolium multiflorum | DHP_A53_1 | gDNAContig | 1 | 200 |
| 903 | Lolium multiflorum | DHP_A52_1 | cDNAContig | 1 | 200 |
| 904 | Lolium multiflorum | DHP_A52_1 | cDNAContig | 1 | 200 |
| 905 | Lolium multiflorum | DHP_A54_1 | cDNAContig | 1 | 150 |
| 906 | Lolium multiflorum | DHP_A54_1 | cDNAContig | 1 | 150 |

**Table 3**

| SEQ ID NO | Gene | # Species | Species |
|---|---|---|---|
| 907 | DHP | 5 | Amaranthus hybridus,Amaranthus palmeri,Amaranthus rudis,Amaranthus viridis,Kochia scoparia |
| 908 | DHP | 5 | Amaranthus hybridus,Amaranthus palmeri,Amaranthus rudis,Amaranthus viridis,Kochia scoparia |
| 909 | DHP | 5 | Amaranthus hybridus,Amaranthus palmeri,Amaranthus rudis,Amaranthus viridis,Kochia scoparia |
| 910 | DHP | 5 | Amaranthus lividus,Amaranthus palmeri,Amaranthus rudis,Amaranthus viridis,Kochia scoparia |
| 911 | DHP | 5 | Amaranthus lividus,Amaranthus palmeri,Amaranthus rudis,Amaranthus viridis,Kochia scoparia |
| 912 | DHP | 5 | Amaranthus hybridus,Amaranthus palmeri,Amaranthus rudis,Amaranthus viridis,Kochia scoparia |
| 913 | DHP | 5 | Amaranthus hybridus,Amaranthus palmeri,Amaranthus rudis,Amaranthus viridis,Kochia scoparia |
| 914 | DHP | 5 | Amaranthus hybridus,Amaranthus palmeri,Amaranthus rudis,Amaranthus viridis,Kochia scoparia |
| 915 | DHP | 5 | Amaranthus hybridus,Amaranthus palmeri,Amaranthus rudis,Amaranthus viridis,Kochia scoparia |
| 916 | DHP | 5 | Amaranthus hybridus,Amaranthus palmeri,Amaranthus rudis,Amaranthus viridis,Kochia scoparia |
| 917 | DHP | 5 | Amaranthus lividus,Amaranthus palmeri,Amaranthus rudis,Amaranthus viridis,Kochia scoparia |
| 918 | DHP | 5 | Amaranthus hybridus,Amaranthus palmeri,Amaranthus rudis,Amaranthus viridis,Kochia scoparia |
| 919 | DHP | 5 | Amaranthus hybridus,Amaranthus palmeri,Amaranthus rudis,Amaranthus viridis,Kochia scoparia |
| 920 | DHP | 5 | Amaranthus hybridus,Amaranthus palmeri,Amaranthus rudis,Amaranthus viridis,Kochia scoparia |
| 921 | DHP | 5 | Amaranthus hybridus,Amaranthus palmeri,Amaranthus rudis,Amaranthus viridis,Kochia scoparia |
| 922 | DHP | 4 | Amaranthus lividus,Amaranthus palmeri,Amaranthus rudis,Amaranthus viridis |
| 923 | DHP | 4 | Amaranthus lividus,Amaranthus palmeri,Amaranthus rudis,Amaranthus viridis |
| 924 | DHP | 4 | Amaranthus lividus,Amaranthus palmeri,Amaranthus rudis,Amaranthus viridis |
| 925 | DHP | 4 | Amaranthus lividus,Amaranthus palmeri,Amaranthus rudis,Amaranthus viridis |
| 926 | DHP | 4 | Amaranthus hybridus,Amaranthus palmeri,Amaranthus rudis,Amaranthus viridis |
| 927 | DHP | 4 | Amaranthus hybridus,Amaranthus palmeri,Amaranthus rudis,Amaranthus viridis |
| 928 | DHP | 4 | Amaranthus lividus,Amaranthus palmeri,Amaranthus rudis,Amaranthus viridis |
| 929 | DHP | 4 | Amaranthus lividus,Amaranthus palmeri,Amaranthus rudis,Amaranthus viridis |
| 930 | DHP | 4 | Amaranthus lividus,Amaranthus palmeri,Amaranthus rudis,Amaranthus viridis |
| 931 | DHP | 4 | Amaranthus lividus,Amaranthus palmeri,Amaranthus rudis,Amaranthus viridis |
| 932 | DHP | 4 | Amaranthus lividus,Amaranthus palmeri,Amaranthus rudis,Amaranthus viridis |
| 933 | DHP | 4 | Amaranthus hybridus,Amaranthus palmeri,Amaranthus rudis,Amaranthus viridis |
| 934 | DHP | 4 | Amaranthus lividus,Amaranthus palmeri,Amaranthus rudis,Amaranthus viridis |
| 935 | DHP | 4 | Amaranthus lividus,Amaranthus palmeri,Amaranthus rudis,Amaranthus viridis |
| 936 | DHP | 4 | Amaranthus hybridus,Amaranthus palmeri,Amaranthus rudis,Amaranthus viridis |
| 937 | DHP | 4 | Amaranthus lividus,Amaranthus palmeri,Amaranthus rudis,Amaranthus viridis |
| 938 | DHP | 4 | Amaranthus lividus,Amaranthus palmeri,Amaranthus rudis,Amaranthus viridis |
| 939 | DHP | 4 | Amaranthus lividus,Amaranthus palmeri,Amaranthus rudis,Amaranthus viridis |
| 940 | DHP | 4 | Amaranthus lividus,Amaranthus palmeri,Amaranthus rudis,Amaranthus viridis |
| 941 | DHP | 4 | Amaranthus lividus,Amaranthus palmeri,Amaranthus rudis,Amaranthus viridis |
| 942 | DHP | 4 | Amaranthus hybridus,Amaranthus palmeri,Amaranthus rudis,Amaranthus viridis |
| 943 | DHP | 4 | Amaranthus lividus,Amaranthus palmeri,Amaranthus rudis,Amaranthus viridis |
| 944 | DHP | 4 | Amaranthus lividus,Amaranthus palmeri,Amaranthus rudis,Amaranthus viridis |
| 945 | DHP | 4 | Amaranthus lividus,Amaranthus palmeri,Amaranthus rudis,Amaranthus viridis |
| 946 | DHP | 4 | Amaranthus lividus,Amaranthus palmeri,Amaranthus rudis,Amaranthus viridis |
| 947 | DHP | 4 | Amaranthus lividus,Amaranthus palmeri,Amaranthus rudis,Amaranthus viridis |
| 948 | DHP | 4 | Amaranthus lividus,Amaranthus palmeri,Amaranthus rudis,Amaranthus viridis |
| 949 | DHP | 4 | Amaranthus lividus,Amaranthus palmeri,Amaranthus rudis,Amaranthus viridis |
| 950 | DHP | 4 | Amaranthus lividus,Amaranthus palmeri,Amaranthus rudis,Amaranthus viridis |
| 951 | DHP | 4 | Amaranthus lividus,Amaranthus palmeri,Amaranthus rudis,Amaranthus viridis |
| 952 | DHP | 4 | Amaranthus lividus,Amaranthus palmeri,Amaranthus rudis,Amaranthus viridis |
| 953 | DHP | 4 | Amaranthus lividus,Amaranthus palmeri,Amaranthus rudis,Amaranthus viridis |
| 954 | DHP | 4 | Amaranthus lividus,Amaranthus palmeri,Amaranthus rudis,Amaranthus viridis |
| 955 | DHP | 4 | Amaranthus lividus,Amaranthus palmeri,Amaranthus rudis,Amaranthus viridis |
| 956 | DHP | 4 | Amaranthus lividus,Amaranthus rudis,Amaranthus viridis,Kochia scoparia |
| 957 | DHP | 4 | Amaranthus lividus,Amaranthus palmeri,Amaranthus rudis,Amaranthus viridis |
| 958 | DHP | 4 | Amaranthus hybridus,Amaranthus palmeri,Amaranthus rudis,Amaranthus viridis |
| 959 | DHP | 4 | Amaranthus palmeri,Amaranthus rudis,Amaranthus viridis,Kochia scoparia |
| 960 | DHP | 4 | Amaranthus hybridus,Amaranthus palmeri,Amaranthus rudis,Amaranthus viridis |
| 961 | DHP | 4 | Amaranthus lividus,Amaranthus palmeri,Amaranthus rudis,Amaranthus viridis |
| 962 | DHP | 4 | Amaranthus lividus,Amaranthus palmeri,Amaranthus rudis,Amaranthus viridis |
| 963 | DHP | 4 | Amaranthus lividus,Amaranthus palmeri,Amaranthus rudis,Amaranthus viridis |
| 964 | DHP | 4 | Amaranthus hybridus,Amaranthus palmeri,Amaranthus rudis,Amaranthus viridis |
| 965 | DHP | 4 | Amaranthus hybridus,Amaranthus palmeri,Amaranthus rudis,Amaranthus viridis |
| 966 | DHP | 4 | Amaranthus hybridus,Amaranthus palmeri,Amaranthus rudis,Amaranthus viridis |
| 967 | DHP | 4 | Amaranthus lividus,Amaranthus palmeri,Amaranthus rudis,Amaranthus viridis |
| 968 | DHP | 4 | Amaranthus lividus,Amaranthus palmeri,Amaranthus rudis,Amaranthus viridis |
| 969 | DHP | 4 | Amaranthus lividus,Amaranthus palmeri,Amaranthus rudis,Amaranthus viridis |
| 970 | DHP | 4 | Amaranthus hybridus,Amaranthus palmeri,Amaranthus rudis,Amaranthus viridis |
| 971 | DHP | 4 | Amaranthus lividus,Amaranthus palmeri,Amaranthus rudis,Amaranthus viridis |
| 972 | DHP | 4 | Amaranthus lividus,Amaranthus palmeri,Amaranthus rudis,Amaranthus viridis |
| 973 | DHP | 4 | Amaranthus lividus,Amaranthus palmeri,Amaranthus rudis,Amaranthus viridis |
| 974 | DHP | 4 | Amaranthus lividus,Amaranthus palmeri,Amaranthus rudis,Amaranthus viridis |
| 975 | DHP | 4 | Amaranthus lividus,Amaranthus palmeri,Amaranthus rudis,Amaranthus viridis |
| 976 | DHP | 4 | Amaranthus lividus,Amaranthus palmeri,Amaranthus rudis,Amaranthus viridis |
| 977 | DHP | 4 | Amaranthus lividus,Amaranthus palmeri,Amaranthus rudis,Amaranthus viridis |
| 978 | DHP | 4 | Amaranthus lividus,Amaranthus palmeri,Amaranthus rudis,Amaranthus viridis |
| 979 | DHP | 4 | Amaranthus lividus,Amaranthus palmeri,Amaranthus rudis,Amaranthus viridis |
| 980 | DHP | 4 | Amaranthus lividus,Amaranthus palmeri,Amaranthus rudis,Amaranthus viridis |
| 981 | DHP | 4 | Amaranthus hybridus,Amaranthus palmeri,Amaranthus rudis,Amaranthus viridis |
| 982 | DHP | 4 | Amaranthus lividus,Amaranthus palmeri,Amaranthus rudis,Amaranthus viridis |
| 983 | DHP | 4 | Amaranthus lividus,Amaranthus palmeri,Amaranthus rudis,Amaranthus viridis |
| 984 | DHP | 4 | Amaranthus lividus,Amaranthus palmeri,Amaranthus rudis,Amaranthus viridis |
| 985 | DHP | 4 | Amaranthus hybridus,Amaranthus palmeri,Amaranthus rudis,Amaranthus viridis |
| 986 | DHP | 4 | Amaranthus lividus,Amaranthus palmeri,Amaranthus rudis,Amaranthus viridis |
| 987 | DHP | 4 | Amaranthus lividus,Amaranthus palmeri,Amaranthus rudis,Amaranthus viridis |
| 988 | DHP | 4 | Amaranthus lividus,Amaranthus palmeri,Amaranthus rudis,Amaranthus viridis |
| 989 | DHP | 4 | Amaranthus lividus,Amaranthus palmeri,Amaranthus rudis,Amaranthus viridis |
| 990 | DHP | 4 | Amaranthus lividus,Amaranthus palmeri,Amaranthus rudis,Amaranthus viridis |
| 991 | DHP | 4 | Amaranthus lividus,Amaranthus palmeri,Amaranthus rudis,Amaranthus viridis |
| 992 | DHP | 4 | Amaranthus lividus,Amaranthus palmeri,Amaranthus rudis,Amaranthus viridis |
| 993 | DHP | 4 | Amaranthus lividus,Amaranthus palmeri,Amaranthus rudis,Amaranthus viridis |
| 994 | DHP | 4 | Amaranthus lividus,Amaranthus palmeri,Amaranthus rudis,Amaranthus viridis |
| 995 | DHP | 4 | Amaranthus lividus,Amaranthus palmeri,Amaranthus rudis,Amaranthus viridis |
| 996 | DHP | 4 | Amaranthus lividus,Amaranthus palmeri,Amaranthus rudis,Amaranthus viridis |
| 997 | DHP | 4 | Amaranthus lividus,Amaranthus palmeri,Amaranthus rudis,Amaranthus viridis |
| 998 | DHP | 4 | Amaranthus lividus,Amaranthus palmeri,Amaranthus rudis,Amaranthus viridis |
| 999 | DHP | 4 | Amaranthus lividus,Amaranthus palmeri,Amaranthus rudis,Amaranthus viridis |
| 1000 | DHP | 4 | Amaranthus palmeri,Amaranthus rudis,Amaranthus viridis,Kochia scoparia |
| 1001 | DHP | 4 | Amaranthus lividus,Amaranthus palmeri,Amaranthus rudis,Amaranthus viridis |
| 1002 | DHP | 4 | Amaranthus lividus,Amaranthus palmeri,Amaranthus rudis,Amaranthus viridis |
| 1003 | DHP | 4 | Amaranthus lividus,Amaranthus palmeri,Amaranthus rudis,Amaranthus viridis |
| 1004 | DHP | 4 | Amaranthus lividus,Amaranthus palmeri,Amaranthus rudis,Amaranthus viridis |
| 1005 | DHP | 4 | Amaranthus lividus,Amaranthus palmeri,Amaranthus rudis,Amaranthus viridis |
| 1006 | DHP | 4 | Amaranthus hybridus,Amaranthus palmeri,Amaranthus rudis,Amaranthus viridis |
| 1007 | DHP | 4 | Amaranthus lividus,Amaranthus palmeri,Amaranthus rudis,Amaranthus viridis |
| 1008 | DHP | 4 | Amaranthus lividus,Amaranthus palmeri,Amaranthus rudis,Amaranthus viridis |
| 1009 | DHP | 4 | Amaranthus lividus,Amaranthus palmeri,Amaranthus rudis,Amaranthus viridis |
| 1010 | DHP | 4 | Amaranthus lividus,Amaranthus palmeri,Amaranthus rudis,Amaranthus viridis |
| 1011 | DHP | 4 | Amaranthus lividus,Amaranthus palmeri,Amaranthus rudis,Amaranthus viridis |
| 1012 | DHP | 4 | Amaranthus lividus,Amaranthus palmeri,Amaranthus rudis,Amaranthus viridis |
| 1013 | DHP | 4 | Amaranthus lividus,Amaranthus palmeri,Amaranthus rudis,Amaranthus viridis |
| 1014 | DHP | 4 | Amaranthus lividus,Amaranthus palmeri,Amaranthus rudis,Amaranthus viridis |
| 1015 | DHP | 4 | Amaranthus hybridus,Amaranthus palmeri,Amaranthus rudis,Amaranthus viridis |
| 1016 | DHP | 4 | Amaranthus hybridus,Amaranthus palmeri,Amaranthus rudis,Amaranthus viridis |
| 1017 | DHP | 4 | Amaranthus lividus,Amaranthus rudis,Amaranthus viridis,Kochia scoparia |
| 1018 | DHP | 4 | Amaranthus lividus,Amaranthus palmeri,Amaranthus rudis,Amaranthus viridis |
| 1019 | DHP | 4 | Amaranthus lividus,Amaranthus palmeri,Amaranthus rudis,Amaranthus viridis |
| 1020 | DHP | 4 | Amaranthus hybridus,Amaranthus palmeri,Amaranthus rudis,Amaranthus viridis |
| 1021 | DHP | 4 | Amaranthus lividus,Amaranthus palmeri,Amaranthus rudis,Amaranthus viridis |
| 1022 | DHP | 4 | Amaranthus lividus,Amaranthus palmeri,Amaranthus rudis,Amaranthus viridis |
| 1023 | DHP | 4 | Amaranthus hybridus,Amaranthus palmeri,Amaranthus rudis,Amaranthus viridis |
| 1024 | DHP | 4 | Amaranthus palmeri,Amaranthus rudis,Amaranthus viridis,Kochia scoparia |
| 1025 | DHP | 4 | Amaranthus lividus,Amaranthus palmeri,Amaranthus rudis,Amaranthus viridis |
| 1026 | DHP | 4 | Amaranthus lividus,Amaranthus palmeri,Amaranthus rudis,Amaranthus viridis |
| 1027 | DHP | 4 | Amaranthus lividus,Amaranthus palmeri,Amaranthus rudis,Amaranthus viridis |
| 1028 | DHP | 4 | Amaranthus lividus,Amaranthus palmeri,Amaranthus rudis,Amaranthus viridis |
| 1029 | DHP | 4 | Amaranthus lividus,Amaranthus palmeri,Amaranthus rudis,Amaranthus viridis |
| 1030 | DHP | 4 | Amaranthus lividus,Amaranthus palmeri,Amaranthus rudis,Amaranthus viridis |
| 1031 | DHP | 4 | Amaranthus lividus,Amaranthus palmeri,Amaranthus rudis,Amaranthus viridis |
| 1032 | DHP | 4 | Amaranthus lividus,Amaranthus palmeri,Amaranthus rudis,Amaranthus viridis |
| 1033 | DHP | 4 | Amaranthus lividus,Amaranthus palmeri,Amaranthus rudis,Amaranthus viridis |
| 1034 | DHP | 4 | Amaranthus lividus,Amaranthus palmeri,Amaranthus rudis,Amaranthus viridis |
| 1035 | DHP | 4 | Amaranthus lividus,Amaranthus palmeri,Amaranthus rudis,Amaranthus viridis |
| 1036 | DHP | 4 | Amaranthus hybridus,Amaranthus palmeri,Amaranthus rudis,Amaranthus viridis |
| 1037 | DHP | 4 | Amaranthus lividus,Amaranthus palmeri,Amaranthus rudis,Amaranthus viridis |
| 1038 | DHP | 4 | Amaranthus hybridus,Amaranthus palmeri,Amaranthus rudis,Amaranthus viridis |
| 1039 | DHP | 4 | Amaranthus lividus,Amaranthus palmeri,Amaranthus rudis,Amaranthus viridis |
| 1040 | DHP | 4 | Amaranthus lividus,Amaranthus palmeri,Amaranthus rudis,Amaranthus viridis |
| 1041 | DHP | 4 | Amaranthus lividus,Amaranthus palmeri,Amaranthus rudis,Amaranthus viridis |
| 1042 | DHP | 4 | Amaranthus lividus,Amaranthus palmeri,Amaranthus rudis,Amaranthus viridis |
| 1043 | DHP | 4 | Amaranthus hybridus,Amaranthus palmeri,Amaranthus rudis,Amaranthus viridis |
| 1044 | DHP | 4 | Amaranthus hybridus,Amaranthus palmeri,Amaranthus rudis,Amaranthus viridis |
| 1045 | DHP | 4 | Amaranthus lividus,Amaranthus palmeri,Amaranthus rudis,Amaranthus viridis |
| 1046 | DHP | 4 | Amaranthus lividus,Amaranthus palmeri,Amaranthus rudis,Amaranthus viridis |
| 1047 | DHP | 4 | Amaranthus hybridus,Amaranthus palmeri,Amaranthus rudis,Amaranthus viridis |
| 1048 | DHP | 4 | Amaranthus lividus,Amaranthus palmeri,Amaranthus rudis,Amaranthus viridis |
| 1049 | DHP | 4 | Amaranthus lividus,Amaranthus palmeri,Amaranthus rudis,Amaranthus viridis |
| 1050 | DHP | 4 | Amaranthus lividus,Amaranthus palmeri,Amaranthus rudis,Amaranthus viridis |
| 1051 | DHP | 4 | Amaranthus lividus,Amaranthus palmeri,Amaranthus rudis,Amaranthus viridis |
| 1052 | DHP | 4 | Amaranthus lividus,Amaranthus palmeri,Amaranthus rudis,Amaranthus viridis |
| 1053 | DHP | 4 | Amaranthus lividus,Amaranthus palmeri,Amaranthus rudis,Amaranthus viridis |
| 1054 | DHP | 4 | Amaranthus lividus,Amaranthus palmeri,Amaranthus rudis,Amaranthus viridis |
| 1055 | DHP | 4 | Amaranthus lividus,Amaranthus palmeri,Amaranthus rudis,Amaranthus viridis |
| 1056 | DHP | 4 | Amaranthus lividus,Amaranthus palmeri,Amaranthus rudis,Amaranthus viridis |
| 1057 | DHP | 4 | Amaranthus lividus,Amaranthus palmeri,Amaranthus rudis,Amaranthus viridis |
| 1058 | DHP | 4 | Amaranthus lividus,Amaranthus palmeri,Amaranthus rudis,Amaranthus viridis |
| 1059 | DHP | 4 | Amaranthus lividus,Amaranthus palmeri,Amaranthus rudis,Amaranthus viridis |
| 1060 | DHP | 4 | Amaranthus lividus,Amaranthus palmeri,Amaranthus rudis,Amaranthus viridis |
| 1061 | DHP | 4 | Amaranthus lividus,Amaranthus palmeri,Amaranthus rudis,Amaranthus viridis |
| 1062 | DHP | 4 | Amaranthus hybridus,Amaranthus palmeri,Amaranthus rudis,Amaranthus viridis |
| 1063 | DHP | 4 | Amaranthus lividus,Amaranthus palmeri,Amaranthus rudis,Amaranthus viridis |
| 1064 | DHP | 4 | Amaranthus lividus,Amaranthus palmeri,Amaranthus rudis,Amaranthus viridis |
| 1065 | DHP | 4 | Amaranthus lividus,Amaranthus palmeri,Amaranthus rudis,Amaranthus viridis |
| 1066 | DHP | 4 | Amaranthus lividus,Amaranthus palmeri,Amaranthus rudis,Amaranthus viridis |
| 1067 | DHP | 4 | Amaranthus lividus,Amaranthus palmeri,Amaranthus rudis,Amaranthus viridis |
| 1068 | DHP | 4 | Amaranthus lividus,Amaranthus palmeri,Amaranthus rudis,Amaranthus viridis |
| 1069 | DHP | 4 | Amaranthus lividus,Amaranthus palmeri,Amaranthus rudis,Amaranthus viridis |
| 1070 | DHP | 4 | Amaranthus lividus,Amaranthus palmeri,Amaranthus rudis,Amaranthus viridis |
| 1071 | DHP | 4 | Amaranthus lividus,Amaranthus palmeri,Amaranthus rudis,Amaranthus viridis |
| 1072 | DHP | 4 | Amaranthus lividus,Amaranthus palmeri,Amaranthus rudis,Amaranthus viridis |
| 1073 | DHP | 4 | Amaranthus lividus,Amaranthus palmeri,Amaranthus rudis,Amaranthus viridis |
| 1074 | DHP | 4 | Amaranthus lividus,Amaranthus palmeri,Amaranthus rudis,Amaranthus viridis |
| 1075 | DHP | 4 | Amaranthus lividus,Amaranthus palmeri,Amaranthus rudis,Amaranthus viridis |
| 1076 | DHP | 4 | Amaranthus lividus,Amaranthus palmeri,Amaranthus rudis,Amaranthus viridis |
| 1077 | DHP | 4 | Amaranthus lividus,Amaranthus palmeri,Amaranthus rudis,Amaranthus viridis |
| 1078 | DHP | 4 | Amaranthus lividus,Amaranthus palmeri,Amaranthus rudis,Amaranthus viridis |
| 1079 | DHP | 4 | Amaranthus lividus,Amaranthus palmeri,Amaranthus rudis,Amaranthus viridis |
| 1080 | DHP | 4 | Amaranthus lividus,Amaranthus palmeri,Amaranthus rudis,Amaranthus viridis |
| 1081 | DHP | 4 | Amaranthus lividus,Amaranthus palmeri,Amaranthus rudis,Amaranthus viridis |
| 1082 | DHP | 4 | Amaranthus lividus,Amaranthus palmeri,Amaranthus rudis,Amaranthus viridis |
| 1083 | DHP | 4 | Amaranthus lividus,Amaranthus palmeri,Amaranthus rudis,Amaranthus viridis |
| 1084 | DHP | 4 | Amaranthus lividus,Amaranthus palmeri,Amaranthus rudis,Amaranthus viridis |
| 1085 | DHP | 4 | Amaranthus lividus,Amaranthus palmeri,Amaranthus rudis,Amaranthus viridis |
| 1086 | DHP | 4 | Amaranthus lividus,Amaranthus palmeri,Amaranthus rudis,Amaranthus viridis |
| 1087 | DHP | 4 | Amaranthus lividus,Amaranthus palmeri,Amaranthus rudis,Amaranthus viridis |
| 1088 | DHP | 4 | Amaranthus lividus,Amaranthus palmeri,Amaranthus rudis,Amaranthus viridis |
| 1089 | DHP | 4 | Amaranthus lividus,Amaranthus palmeri,Amaranthus rudis,Amaranthus viridis |
| 1090 | DHP | 4 | Amaranthus lividus,Amaranthus palmeri,Amaranthus rudis,Amaranthus viridis |
| 1091 | DHP | 4 | Amaranthus palmeri,Amaranthus rudis,Amaranthus viridis,Kochia scoparia |
| 1092 | DHP | 4 | Amaranthus lividus,Amaranthus palmeri,Amaranthus rudis,Amaranthus viridis |
| 1093 | DHP | 4 | Amaranthus lividus,Amaranthus palmeri,Amaranthus rudis,Amaranthus viridis |
| 1094 | DHP | 4 | Amaranthus lividus,Amaranthus palmeri,Amaranthus rudis,Amaranthus viridis |
| 1095 | DHP | 4 | Amaranthus lividus,Amaranthus palmeri,Amaranthus rudis,Amaranthus viridis |
| 1096 | DHP | 4 | Amaranthus lividus,Amaranthus palmeri,Amaranthus rudis,Amaranthus viridis |
| 1097 | DHP | 4 | Amaranthus lividus,Amaranthus palmeri,Amaranthus rudis,Amaranthus viridis |
| 1098 | DHP | 4 | Amaranthus lividus,Amaranthus palmeri,Amaranthus rudis,Amaranthus viridis |
| 1099 | DHP | 4 | Amaranthus lividus,Amaranthus palmeri,Amaranthus rudis,Amaranthus viridis |
| 1100 | DHP | 4 | Amaranthus lividus,Amaranthus palmeri,Amaranthus rudis,Amaranthus viridis |
| 1101 | DHP | 4 | Amaranthus hybridus,Amaranthus palmeri,Amaranthus rudis,Amaranthus viridis |
| 1102 | DHP | 4 | Amaranthus lividus,Amaranthus palmeri,Amaranthus rudis,Amaranthus viridis |
| 1103 | DHP | 4 | Amaranthus lividus,Amaranthus palmeri,Amaranthus rudis,Amaranthus viridis |
| 1104 | DHP | 4 | Amaranthus lividus,Amaranthus palmeri,Amaranthus rudis,Amaranthus viridis |
| 1105 | DHP | 4 | Amaranthus lividus,Amaranthus rudis,Amaranthus viridis,Kochia scoparia |
| 1106 | DHP | 4 | Amaranthus lividus,Amaranthus palmeri,Amaranthus rudis,Amaranthus viridis |
| 1107 | DHP | 4 | Amaranthus lividus,Amaranthus palmeri,Amaranthus rudis,Amaranthus viridis |
| 1108 | DHP | 4 | Amaranthus lividus,Amaranthus palmeri,Amaranthus rudis,Amaranthus viridis |
| 1109 | DHP | 4 | Amaranthus lividus,Amaranthus palmeri,Amaranthus rudis,Amaranthus viridis |
| 1110 | DHP | 4 | Amaranthus lividus,Amaranthus palmeri,Amaranthus rudis,Amaranthus viridis |
| 1111 | DHP | 4 | Amaranthus lividus,Amaranthus palmeri,Amaranthus rudis,Amaranthus viridis |
| 1112 | DHP | 4 | Amaranthus lividus,Amaranthus palmeri,Amaranthus rudis,Amaranthus viridis |
| 1113 | DHP | 4 | Amaranthus lividus,Amaranthus palmeri,Amaranthus rudis,Amaranthus viridis |
| 1114 | DHP | 4 | Amaranthus lividus,Amaranthus palmeri,Amaranthus rudis,Amaranthus viridis |
| 1115 | DHP | 4 | Amaranthus lividus,Amaranthus palmeri,Amaranthus rudis,Amaranthus viridis |
| 1116 | DHP | 4 | Amaranthus lividus,Amaranthus palmeri,Amaranthus rudis,Amaranthus viridis |
| 1117 | DHP | 4 | Amaranthus lividus,Amaranthus palmeri,Amaranthus rudis,Amaranthus viridis |
| 1118 | DHP | 4 | Amaranthus lividus,Amaranthus palmeri,Amaranthus rudis,Amaranthus viridis |
| 1119 | DHP | 4 | Amaranthus hybridus,Amaranthus palmeri,Amaranthus rudis,Amaranthus viridis |
| 1120 | DHP | 4 | Amaranthus hybridus,Amaranthus palmeri,Amaranthus rudis,Amaranthus viridis |
| 1121 | DHP | 4 | Amaranthus lividus,Amaranthus palmeri,Amaranthus rudis,Amaranthus viridis |
| 1122 | DHP | 4 | Amaranthus palmeri,Amaranthus rudis,Amaranthus viridis,Kochia scoparia |
| 1123 | DHP | 4 | Amaranthus lividus,Amaranthus palmeri,Amaranthus rudis,Amaranthus viridis |
| 1124 | DHP | 4 | Amaranthus lividus,Amaranthus palmeri,Amaranthus rudis,Amaranthus viridis |
| 1125 | DHP | 4 | Amaranthus lividus,Amaranthus palmeri,Amaranthus rudis,Amaranthus viridis |
| 1126 | DHP | 4 | Amaranthus lividus,Amaranthus palmeri,Amaranthus rudis,Amaranthus viridis |
| 1127 | DHP | 4 | Amaranthus lividus,Amaranthus palmeri,Amaranthus rudis,Amaranthus viridis |
| 1128 | DHP | 4 | Amaranthus lividus,Amaranthus palmeri,Amaranthus rudis,Amaranthus viridis |
| 1129 | DHP | 4 | Amaranthus lividus,Amaranthus palmeri,Amaranthus rudis,Amaranthus viridis |
| 1130 | DHP | 4 | Amaranthus palmeri,Amaranthus rudis,Amaranthus viridis,Kochia scoparia |
| 1131 | DHP | 4 | Amaranthus lividus,Amaranthus palmeri,Amaranthus rudis,Amaranthus viridis |
| 1132 | DHP | 4 | Amaranthus hybridus,Amaranthus palmeri,Amaranthus rudis,Amaranthus viridis |
| 1133 | DHP | 4 | Amaranthus hybridus,Amaranthus palmeri,Amaranthus rudis,Amaranthus viridis |
| 1134 | DHP | 4 | Amaranthus lividus,Amaranthus palmeri,Amaranthus rudis,Amaranthus viridis |
| 1135 | DHP | 4 | Amaranthus lividus,Amaranthus palmeri,Amaranthus rudis,Amaranthus viridis |
| 1136 | DHP | 4 | Amaranthus hybridus,Amaranthus palmeri,Amaranthus rudis,Amaranthus viridis |
| 1137 | DHP | 4 | Amaranthus lividus,Amaranthus palmeri,Amaranthus rudis,Amaranthus viridis |
| 1138 | DHP | 4 | Amaranthus lividus,Amaranthus palmeri,Amaranthus rudis,Amaranthus viridis |
| 1139 | DHP | 4 | Amaranthus lividus,Amaranthus palmeri,Amaranthus rudis,Amaranthus viridis |
| 1140 | DHP | 4 | Amaranthus hybridus,Amaranthus palmeri,Amaranthus rudis,Amaranthus viridis |
| 1141 | DHP | 4 | Amaranthus hybridus,Amaranthus palmeri,Amaranthus rudis,Amaranthus viridis |
| 1142 | DHP | 4 | Amaranthus lividus,Amaranthus rudis,Amaranthus viridis,Kochia scoparia |
| 1143 | DHP | 4 | Amaranthus lividus,Amaranthus palmeri,Amaranthus rudis,Amaranthus viridis |
| 1144 | DHP | 4 | Amaranthus lividus,Amaranthus palmeri,Amaranthus rudis,Amaranthus viridis |
| 1145 | DHP | 4 | Amaranthus lividus,Amaranthus palmeri,Amaranthus rudis,Amaranthus viridis |
| 1146 | DHP | 4 | Amaranthus lividus,Amaranthus palmeri,Amaranthus rudis,Amaranthus viridis |
| 1147 | DHP | 4 | Amaranthus lividus,Amaranthus palmeri,Amaranthus rudis,Amaranthus viridis |
| 1148 | DHP | 4 | Amaranthus lividus,Amaranthus palmeri,Amaranthus rudis,Amaranthus viridis |
| 1149 | DHP | 4 | Amaranthus hybridus,Amaranthus palmeri,Amaranthus rudis,Amaranthus viridis |
| 1150 | DHP | 4 | Amaranthus lividus,Amaranthus palmeri,Amaranthus rudis,Amaranthus viridis |
| 1151 | DHP | 4 | Amaranthus lividus,Amaranthus palmeri,Amaranthus rudis,Amaranthus viridis |
| 1152 | DHP | 4 | Amaranthus lividus,Amaranthus palmeri,Amaranthus rudis,Amaranthus viridis |
| 1153 | DHP | 4 | Amaranthus lividus,Amaranthus palmeri,Amaranthus rudis,Amaranthus viridis |
| 1154 | DHP | 4 | Amaranthus lividus,Amaranthus palmeri,Amaranthus rudis,Amaranthus viridis |
| 1155 | DHP | 4 | Amaranthus lividus,Amaranthus palmeri,Amaranthus rudis,Amaranthus viridis |
| 1156 | DHP | 4 | Amaranthus lividus,Amaranthus palmeri,Amaranthus rudis,Amaranthus viridis |
| 1157 | DHP | 4 | Amaranthus hybridus,Amaranthus palmeri,Amaranthus rudis,Amaranthus viridis |
| 1158 | DHP | 4 | Amaranthus lividus,Amaranthus palmeri,Amaranthus rudis,Amaranthus viridis |
| 1159 | DHP | 4 | Amaranthus hybridus,Amaranthus palmeri,Amaranthus rudis,Amaranthus viridis |
| 1160 | DHP | 4 | Amaranthus lividus,Amaranthus palmeri,Amaranthus rudis,Amaranthus viridis |
| 1161 | DHP | 4 | Amaranthus hybridus,Amaranthus palmeri,Amaranthus rudis,Amaranthus viridis |
| 1162 | DHP | 4 | Amaranthus lividus,Amaranthus palmeri,Amaranthus rudis,Amaranthus viridis |
| 1163 | DHP | 4 | Amaranthus lividus,Amaranthus palmeri,Amaranthus rudis,Amaranthus viridis |
| 1164 | DHP | 4 | Amaranthus lividus,Amaranthus palmeri,Amaranthus rudis,Amaranthus viridis |
| 1165 | DHP | 4 | Amaranthus lividus,Amaranthus palmeri,Amaranthus rudis,Amaranthus viridis |
| 1166 | DHP | 4 | Amaranthus lividus,Amaranthus rudis,Amaranthus viridis,Kochia scoparia |
| 1167 | DHP | 4 | Amaranthus hybridus,Amaranthus palmeri,Amaranthus rudis,Amaranthus viridis |
| 1168 | DHP | 4 | Amaranthus lividus,Amaranthus palmeri,Amaranthus rudis,Amaranthus viridis |
| 1169 | DHP | 4 | Amaranthus lividus,Amaranthus palmeri,Amaranthus rudis,Amaranthus viridis |
| 1170 | DHP | 4 | Amaranthus lividus,Amaranthus palmeri,Amaranthus rudis,Amaranthus viridis |
| 1171 | DHP | 4 | Amaranthus lividus,Amaranthus palmeri,Amaranthus rudis,Amaranthus viridis |
| 1172 | DHP | 4 | Amaranthus lividus,Amaranthus palmeri,Amaranthus rudis,Amaranthus viridis |
| 1173 | DHP | 4 | Amaranthus lividus,Amaranthus palmeri,Amaranthus rudis,Amaranthus viridis |
| 1174 | DHP | 4 | Amaranthus lividus,Amaranthus rudis,Amaranthus viridis,Kochia scoparia |
| 1175 | DHP | 4 | Amaranthus lividus,Amaranthus palmeri,Amaranthus rudis,Amaranthus viridis |

The following represent further embodiments of the present invention:
1. A method of plant control comprising: treating a plant with a composition comprising a polynucleotide and a transfer agent, wherein said polynucleotide is essentially identical or essentially complementary to a DHPS gene sequence or fragment thereof, or to an RNA transcript of said DHPS gene sequence or fragment thereof, wherein said DHPS gene sequence is selected from the group consisting of SEQ ID NO: 1-54 or a polynucleotide fragment thereof, whereby said plant growth or development or reproductive ability is regulated, suppressed or delayed or said plant is more sensitive to a DHPS inhibitor herbicide or mitosis inhibitor herbicide as a result of said polynucleotide containing composition relative to a plant not treated with said composition.
2. The method of embodiment 1, wherein said transfer agent comprises an organosilicone surfactant composition or compound contained therein.
3. The method of embodiment 1, wherein said polynucleotide fragment is 18 contiguous, 19 contiguous nucleotides, 20 contiguous nucleotides, or at least 21 contiguous nucleotides in length and at least 85 percent identical to an DHPS gene sequence selected from the group consisting of SEQ ID NO:1-54.
4. The method of embodiment 3, wherein said polynucleotide fragment is selected from the group consisting of sense or anti-sense ssDNA or ssRNA, dsRNA, or dsDNA, or dsDNA/RNA hybrids.
5. The method of embodiment 1, wherein said plant is selected from the group consisting of *Amaranthus palmeri, Amaranthus rudis, Amaranthus hybridus, Amaranthus lividus, Amaranthus viridis, Ambrosia trifida, Conyza candensis, Digitaria sanguinalis, Euphorbia heterophylla, Kochia scoparia, Lolium multiflorum.*
6. The method of embodiment 1, wherein said composition further comprises said DHPS inhibitor or mitosis inhibitor herbicide and external application to a plant with said composition.
7. The method of embodiment 6, wherein said composition further comprises one or more co-herbicides different from said DHPS inhibitor or mitosis inhibitor herbicide.
8. The method of embodiment 3, wherein said composition comprises any combination of two or more of said polynucleotide fragments and external application to a plant with said composition.
9. A composition comprising a polynucleotide and a transfer agent, wherein said polynucleotide is essentially identical or essentially complementary to an DHPS gene sequence, or to an RNA transcript of said DHPS gene sequence, wherein said DHPS gene sequence is selected from the group consisting of SEQ ID NO: 1-54 or a polynucleotide fragment thereof and whereby a plant treated with said composition has its growth or development or reproductive ability regulated, suppressed or delayed or said plant is more sensitive to a DHPS inhibitor herbicide or mitosis inhibitor herbicide as a result of said polynucleotide containing composition relative to a plant not treated with said composition.
10. The composition of embodiment 9, wherein said transfer agent is an organosilicone composition.
11. The composition of embodiment 9, wherein said polynucleotide fragment is 18 contiguous, 19 contiguous nucleotides, 20 contiguous nucleotides or at least 21 contiguous nucleotides in length and at least 85 percent identical to an DHPS gene sequence selected from the group consisting of SEQ ID NO:1-54.
12. The composition of embodiment 9, wherein said polynucleotide is selected from the group consisting of SEQ ID NO:55-906.
13. The composition of embodiment 9, wherein said polynucleotide is selected from the group consisting of SEQ ID NO:907-1222.
14. The composition of embodiment 9, further comprising a DHPS inhibitor herbicide or mitosis inhibitor herbicide.
15. The composition of embodiment 14, wherein said DHPS inhibitor molecule is selected from the group consisting of carbamates and asulam.
16. The composition of embodiment 14, wherein said mitosis inhibitor molecule is selected from the group consisting of dinitroaniline herbicides.
17. The composition of embodiment 14, further comprising a co-herbicide.
18. A method of reducing expression of an DHPS gene in a plant comprising: external application to a plant of a composition comprising a polynucleotide and a transfer agent, wherein said polynucleotide is essentially identical or essentially complementary to an DHPS gene sequence, or to the RNA transcript of said DHPS gene sequence, wherein said DHPS gene sequence is selected from the group consisting of SEQ ID NO: 1-54 or a polynucleotide fragment thereof, whereby said expression of said DHPS gene is reduced relative to a plant in which the composition was not applied.
19. The method of embodiment 18, wherein said transfer agent comprises an organosilicone compound.
20. The method of embodiment 18, wherein said polynucleotide fragment is 19 contiguous nucleotides, 20 contiguous nucleotides or at least 21 contiguous nucleotides in length and at least 85 percent identical to an DHPS gene sequence selected from the group consisting of SEQ ID NO:1-54.
21. The method of embodiment 18, wherein said polynucleotide molecule is selected from the group consisting of sense or anti-sense ssDNA or ssRNA, dsRNA, or dsDNA, or dsDNA/RNA hybrids.
22. A microbial expression cassette comprising a polynucleotide fragment of 18 contiguous, 19 contiguous nucleotides, 20 contiguous nucleotides or at least 21 contiguous nucleotides in length and at least 85 percent identical to an DHPS gene sequence selected from the group consisting of SEQ ID NO:1-54.
23. A method of making a polynucleotide comprising a) transforming the microbial expression cassette of embodiment 21 into a microbe; b) growing said microbe; c) harvesting a polynucleotide from said microbe, wherein said polynucleotide is at least 18 contiguous, at least 19 contiguous nucleotides, at least 20 contiguous nucleotides or at least 21 contiguous nucleotides in length and at least 85 percent identical to an DHPS gene sequence selected from the group consisting of SEQ ID NO:1-54.
24. A method of identifying polynucleotides useful in modulating DHPS gene expression when externally treating a plant comprising: a) providing a plurality of polynucleotides that comprise a region essentially identical or essentially complementary to a polynucleotide fragment of 18 contiguous, 19 contiguous nucleotides, 20 contiguous nucleotides or at least 21 contiguous nucleotides in length and at least 85 percent identical to an DHPS gene sequence selected from the group consisting of SEQ ID NO:1-54; b) externally treating said plant with one or more of said polynucleotides and a transfer agent; c) analyzing said plant or extract for modulation of DHPS gene expression, and whereby a plant treated with said composition has its growth or development or reproductive ability regulated, suppressed or delayed or said plant is more sensitive to a DHPS inhibitor herbicide or mitosis inhibitor herbicide as a result of said polynucleotide containing composition relative to a plant not treated with said composition.
25. The method of embodiment 24, wherein said plant is selected from the group consisting of *Amaranthus palmeri, Amaranthus rudis, Amaranthus hybridus, Amaranthus lividus, Amaranthus viridis, Ambrosia trifida, Conyza candensis, Digitaria sanguinalis, Euphorbia heterophylla, Kochia scoparia, Lolium multiflorum.*
26. The method of embodiment 24, wherein said DHPS gene expression is reduced relative to a plant not treated with said polynucleotide fragment and a transfer agent.
27. The method of embodiment 24, wherein said transfer agent is an organosilicone compound.
28. An agricultural chemical composition comprising an admixture of a polynucleotide and a DHPS inhibitor or mitosis inhibitor herbicide and a co-herbicide, wherein said polynucleotide is essentially identical or essentially complementary to a portion of a DHPS gene sequence, or to a portion of an RNA transcript of said DHPS gene sequence, wherein said DHPS gene sequence is selected from the group consisting of SEQ ID NO: 1-54 or a polynucleotide fragment thereof, and whereby a plant treated with said composition has its growth or development or reproductive ability regulated, suppressed or delayed or said plant is more sensitive to a DHPS inhibitor herbicide or mitosis inhibitor herbicide as a result of said polynucleotide containing composition relative to a plant not treated with said composition.
29. The agricultural chemical composition of embodiment 28, wherein said co-herbicide is selected from the group consisting of amide herbicides, arsenical herbicides, benzothiazole herbicides, benzoylcyclohexanedione herbicides, benzofuranyl alkylsulfonate herbicides, cyclohexene oxime herbicides, cyclopropylisoxazole herbicides, dicarboximide herbicides, dinitroaniline herbicides, dinitrophenol herbicides, diphenyl ether herbicides, dithiocarbamate herbicides, glycine herbicides, halogenated aliphatic herbicides, imidazolinone herbicides, inorganic herbicides, nitrile herbicides, organophosphorus herbicides, oxadiazolone herbicides, oxazole herbicides, phenoxy herbicides, phenylenediamine herbicides, pyrazole herbicides, pyridazine herbicides, pyridazinone herbicides, pyridine herbicides, pyrimidinediamine herbicides, pyrimidinyloxybenzylamine herbicides, quaternary ammonium herbicides, thiocarbamate herbicides, thiocarbonate herbicides, thiourea herbicides, triazine herbicides, triazinone herbicides, triazole herbicides, triazolone herbicides, triazolopyrimidine herbicides, uracil herbicides, and urea herbicides.
30. An agricultural chemical composition comprising an admixture of a polynucleotide and a DHPS inhibitor herbicide and a pesticide, wherein said polynucleotide is essentially identical or essentially complementary to a portion of a DHPS gene sequence, or to a portion of an RNA transcript of said DHPS gene sequence, wherein said DHPS gene sequence is selected from the group consisting of SEQ ID NO:1-54 or a polynucleotide fragment thereof, whereby a field of crop plants in need of weed and pest control are treated with said composition and whereby a plant treated with said composition has its growth or development or reproductive ability regulated, suppressed or delayed or said plant is more sensitive to a DHPS inhibitor herbicide or mitosis inhibitor herbicide as a result of said polynucleotide containing composition relative to a plant not treated with said composition.
31. The agricultural chemical composition of embodiment 30, wherein said pesticide is selected from the group consisting of insecticides, fungicides, nematocides, bactericides, acaricides, growth regulators, chemosterilants, semiochemicals, repellents, attractants, pheromones, feeding stimulants, and biopesticides.
32. A composition comprising a polynucleotide and a transfer agent, wherein said polynucleotide is essentially identical or essentially complementary to a DHPS gene sequence, or to an RNA transcript of said DHPS gene sequence, wherein said polynucleotide is selected from the group consisting of SEQ ID NO:1176 and 1186 or a complement or polynucleotide fragment thereof, and whereby a plant treated with said composition has its growth or development or reproductive ability regulated, suppressed or delayed or said plant is more sensitive to a DHPS inhibitor herbicide or mitosis inhibitor herbicide as a result of said polynucleotide containing composition relative to a plant not treated with said composition.

## Claims

1. A method for potentiating the activity of a 7,8-dihydropteroate synthase (DHPS) inhibitor herbicide or a mitosis inhibitor herbicide in a plant comprising:
(a) topically applying to a surface of the plant a composition comprising a doublestranded RNA (dsRNA) and a transfer agent,
wherein the dsRNA is identical or complementary to an RNA sequence of a DHPS gene sequence, wherein the DHPS gene sequence is selected from the group consisting of SEQ ID NOs: 1-54 and a polynucleotide fragment thereof with at least 18 contiguous nucleotides from the DHPS gene sequence, wherein the transfer agent conditions the surface of the plant for permeation by the dsRNA;
(b) providing a suitable period of time for the dsRNA to permeate the interior of the plant and induce suppression of a DHPS gene; and
(c) topically applying the DHPS inhibitor herbicide or the mitosis inhibitor herbicide to the surface of the plant, wherein the activity of the DHPS inhibitor herbicide or the mitosis inhibitor herbicide in the plant is increased relative to a control plant not treated with the composition.

2. The method of claim 1, wherein the period of time is at least one day before the treating with the DHPS inhibitor herbicide or the mitosis inhibitor herbicide.

3. The method of claim 1, wherein the period of time is at least two days before the treating with the DHPS inhibitor herbicide or the mitosis inhibitor herbicide.

4. The method of claim 1, wherein the period of time is at least three days before the treating with the DHPS inhibitor herbicide or the mitosis inhibitor herbicide.

5. The method of claim 1, wherein the transfer agent is a chemical agent.

6. The method of claim 5, wherein the chemical agent is selected from the group consisting of a surfactant, an organic solvent or an aqueous solution or aqueous mixture thereof, an oxidizing agent, an acid, a base, an oil, an enzyme, and combinations thereof.

7. The method of claim 6, wherein the chemical agent is an organosilicone composition or an organosilicone compound contained therein.

8. The method of claim 7, wherein the organosilicone compound is Silwet L-77.

9. The method of claim 1, wherein the DHPS inhibitor herbicide is carbamates or asulam.

10. The method of claim 1, wherein the mitosis inhibitor herbicide is selected from the group consisting of benfluralin, butralin, dinitramine, ethalfluralin, oryzalin, pendimethalin, trifluralin, phosphoroamidates, pyridines, benzamides, benzenedicarboxylic acids, and combinations thereof.

11. The method of claim 1, wherein the plant is treated with a co-herbicide in combination with the DHPS inhibitor herbicide or the mitosis inhibitor herbicide.

12. The method of claim 11, wherein the co-herbicide is selected from the group consisting of amide herbicides, arsenical herbicides, benzothiazole herbicides, benzoylcyclohexanedione herbicides, benzofuranyl alkylsulfonate herbicides, cyclohexene oxime herbicides, cyclopropylisoxazole herbicides, dicarboximide herbicides, dinitroaniline herbicides, dinitrophenol herbicides, diphenyl ether herbicides, dithiocarbamate herbicides, glycine herbicides, halogenated aliphatic herbicides, imidazolinone herbicides, inorganic herbicides, nitrile herbicides, organophosphorus herbicides, oxadiazolone herbicides, oxazole herbicides, phenoxy herbicides, phenylenediamine herbicides, pyrazole herbicides, pyridazine herbicides, pyridazinone herbicides, pyridine herbicides, pyrimidinediamine herbicides, pyrimidinyloxybenzylamine herbicides, quaternary ammonium herbicides, thiocarbamate herbicides, thiocarbonate herbicides, thiourea herbicides, triazine herbicides, triazinone herbicides, triazole herbicides, triazolone herbicides, triazolopyrimidine herbicides, uracil herbicides, and urea herbicides.

13. The method of claim 1, wherein the plant is treated with a pesticide in combination with the DHPS inhibitor herbicide or the mitosis inhibitor herbicide.

14. The method of claim 13, wherein the pesticide is selected from the group consisting of insecticides, fungicides, nematicides, bactericides, acaricides, growth regulators, chemosterilants, semiochemicals, repellents, attractants, pheromones, feeding stimulants, biopesticides, and combinations thereof.

15. The method of claim 1, wherein the plant is selected from the group consisting of Amaranthus palmeri, Amaranthus rudis, Amaranthus hybridus, Amaranthus lividus, Amaranthus viridis, Ambrosia trifida, Conyza canadensis, Digitaria sanguinalis, Euphorbia heterophylla, Kochia scoparia, and Lolium multiflorum.
